# EUROPEAN PATENT APPLICATION

(11) **EP 4 831 176 A1**
(43) Date of publication of application: **28.10.2026**
(21) Application number: 24906581.4
(22) Date of filing: 23.12.2024
(51) Int. Cl.: C07D 487/04, C07D 513/04, C07D 401/14, A61K 31/4985, A61K 31/519, A61P 35/00, A61P 35/02

(54) **BICYCLIC FUSED HETEROAROMATIC RING DERIVATIVE, AND PHARMACEUTICAL COMPOSITION AND USE THEREOF**

(30) Priority: 22.12.2023 CN 202311789109; 06.06.2024 CN 202410735064
(71) Applicant: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: LI, Xin, Shanghai 201203 (CN); ZHU, Weixing, Shanghai 201203 (CN); JIANG, Bin, Shanghai 201203 (CN); WANG, Ruwei, Shanghai 201203 (CN); XU, Feng, Shanghai 201203 (CN); WANG, Hailong, Shanghai 201203 (CN); HUA, Mojia, Shanghai 201203 (CN); XI, Baoxin, Shanghai 201203 (CN)
(74) Representative: Buzzi, Notaro & Antonielli d'Oulx S.p.A.
(86) International application number: PCT/CN2024/141290
(87) International publication number: WO 2025/131111

(57) **Abstract**

Provided in the present application is a bifunctional compound as shown in formula (I). In addition, further disclosed in the present application are a pharmaceutically acceptable salt or a stereoisomer, a pharmaceutical composition and the use of the compound. The compound of the present application has a good degradation effect on EED, has a good inhibition effect on cell proliferation of tumor cells, and has a potential treatment effect on tumors. POI-(L)ₙ₀-ULM (I)

## Description

This application claims priorities to Chinese Patent Application No. 202311789109.3, titled "BICYCLIC FUSED HETEROAROMATIC RING DERIVATIVE, AND PHARMACEUTICAL COMPOSITION AND USE THEREOF" filed before the China National Intellectual Property Administration on December 22, 2023, and Chinese Patent Application No. 202410735064.X, titled "BICYCLIC FUSED HETEROAROMATIC RING DERIVATIVE, AND PHARMACEUTICAL COMPOSITION AND USE THEREOF" filed before the China National Intellectual Property Administration on June 6, 2024, which are incorporated herein by reference in their entireties.

### TECHNICAL FIELD

The present application relates to the field of pharmaceutical technology, and in particular, to a bicyclic fused heteroaromatic ring derivative, a pharmaceutically acceptable salt thereof, a stereoisomer thereof, a pharmaceutical composition thereof, and medical uses thereof.

### BACKGROUND

The Polycomb group (PcG) protein Polycomb repressive complex 2 (PRC2) performs a core function of transcriptional repression in an organism, achieving gene silencing by catalyzing trimethylation of histone H3 at lysine 27 (H3K27me3). It can promote tumor initiation and progression by inhibiting the expression of tumor suppressor genes in tumors. The catalytic subunit of PRC2, EZH2, is an important representative of third-generation epigenetic regulation precision therapeutic targets. Compared to first-generation and second-generation pan-epigenetic regulation targets, current therapeutic targets can target tumors with specific mutation types, offering significantly improved efficacy and safety. Although EZH2 is an ideal target for directly shutting down aberrant PRC2 activity, PRC2 functions as a protein complex, whose activity and functionality are highly dependent on the regulatory role of the scaffold and another core subunit, EED. Interfering with the protein-protein interaction (PPI) between EZH2 and EED can also inhibit the methyltransferase activity of the PRC2 complex. Novartis AG pioneered the demonstration through high-throughput screening that the H3K27me3 recognition pocket of EED is "druggable", and discovered that targeting EED can allosterically inhibit the catalytic activity of EZH2.

The rise of targeted protein degradation (TPD) technology has provided a new pathway for small molecule drug development. Among these, proteolysis targeting chimeras (PROTACs) are the most well-developed system. Their mechanism of action involves linking a small molecule inhibitor and a ligand for an E3 ubiquitin ligase via a linker to form a targeted protein degradation chimera. In vivo, the inhibitor portion of this bifunctional molecule recognizes the target protein, while the E3 ligase ligand portion recognizes the ubiquitin ligase, ultimately degrading the target protein through the ubiquitin-proteasome pathway. Protein degradation drugs can target "undruggable" targets, improve selectivity and inhibitory activity against targets, and prolong drug duration of action and resistance to mutations. They are particularly suitable for drug development against traditionally undruggable targets (such as transcription factors and scaffold proteins), targets prone to acquired resistance mutations during tumor targeted therapy, targets with gene amplification and/or protein overexpression, targets with different protein isoforms, scaffold proteins, protein aggregates, etc. Currently, small molecule inhibitors targeting EZH2 have been approved for marketing, clinically validating the feasibility of PRC2 as an anti-tumor drug target. However, the efficacy of EZH2 small molecule inhibitors remains limited in solid tumors, and multiple small molecule inhibitors of EZH2 and EED are still under clinical investigation. Considering the structural and functional characteristics of PRC2 and the advantages of protein degradation drugs, utilizing protein degradation technology for drug development against this target may bring new directions and breakthroughs to the drug development for this target.

Although a protein degrader of EED has been reported in the literature (Cell Chemical Biology 2020, 27: 41-46.), its degradation activity and druggability still have significant room for improvement. Therefore, there remains a need to develop highly active EED degraders for clinical research.

### SUMMARY

The purpose of the present application is to provide a PROTAC compound. The compound of the present application is able to degrade and/or inhibit EED proteins, exhibits an excellent degradation/inhibition effect on EED protein and an excellent inhibition effect on tumor cell proliferation, has excellent pharmacokinetic properties, low toxic side effects, good safety, and is more suitable for treating diseases or conditions associated with aberrant EED protein activity (such as proliferative diseases like cancer).

A first aspect of the present application provides a compound of formula (I), or a pharmaceutically acceptable salt thereof, or a stereoisomer thereof:

POI-(L)ₙ₀-ULM (I),

wherein POI is a ligand that binds to an EED protein; L is a linker linking POI and ULM; ULM is a group that binds to an E3 ligase; and n0 is 0 or 1.

In some embodiments, n0 is 0.

In some embodiments, n0 is 1.

In some embodiments, POI is a structure of formula (A-1) or an isomer thereof: 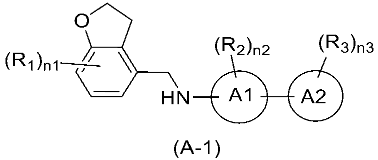
wherein, ring A1 is a 5- to 20-membered heterocycloalkyl ring (preferably a 5- to 6-membered heterocycloalkyl ring, preferably a 15- to 20-membered heterocycloalkyl ring, more preferably a 15- to 18-membered heterocycloalkyl ring, further preferably an 18-membered heterocycloalkyl ring) or a 5-to 10-membered heteroaryl ring (preferably an 8- to 10-membered heteroaryl ring, more preferably a 9-membered heteroaryl ring, more preferably a 10-membered heteroaryl ring);
ring A2 is absent, or is a 3- to 15-membered heterocycloalkyl ring, a C₃₋₁₀ cycloalkylring, a 5- to 15-membered heteroaryl ring (preferably a 5- to 12-membered heteroaryl ring, more preferably a 5- to 10-membered heteroaryl ring, further preferably a 5- to 6-membered heteroaryl ring) or a C₆₋₁₀ aryl ring (preferably a benzene ring or a naphthalene ring);
(R₁)ₙ₁ represents substitution of hydrogen on 2,3-dihydrobenzofuran ring by n1 R₁, n1 is 0, 1 or 2, each R₁ is identical or different, and each R₁ is independently selected from the group consisting of hydrogen, deuterium, halogen (preferably fluorine, chlorine or bromine), oxo, C₁₋₈ alkyl (preferably C₁₋₆ alkyl, more preferably C₁₋₃ alkyl), C₁₋₈ haloalkyl (preferably C₁₋₆ haloalkyl, more preferably C₁₋₃ haloalkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy), -COC₁₋₈ alkyl (preferably -COC₁₋₆ alkyl, more preferably -COC₁₋₃ alkyl), -OCOC₁₋₈ alkyl (preferably -OCOC₁₋₆ alkyl, more preferably -OCOC₁₋₃ alkyl), -COOC₁₋₈ alkyl (preferably -COOC₁₋₆ alkyl, more preferably -COOC₁₋₃ alkyl), -CONH₂, -CONHC₁₋₈ alkyl (preferably -CONHC₁₋₆ alkyl, more preferably -CONHC₁₋₃ alkyl), -CON(C₁₋₈ alkyl)₂ (preferably -CON(C₁₋₆ alkyl)₂, more preferably -CON(C₁₋₃ alkyl)₂), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), -OSO₂C₁₋₈ alkyl (preferably -OSO₂C₁₋₆ alkyl, more preferably -OSO₂C₁₋₃ alkyl), -SO₂NH₂, -SO₂NHC₁₋₈ alkyl (preferably -SO₂NHC₁₋₆ alkyl, more preferably -SO₂NHC₁₋₃ alkyl), -SO₂N(C₁₋₈ alkyl)₂ (preferably -SO₂N(C₁₋₆ alkyl)₂, more preferably -SO₂N(C₁₋₃ alkyl)₂), C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl); and/or two R₁ and carbon atom linked to the two R₁ together form a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring) or a 3- to 15-membered heterocycloalkyl ring (preferably a 3- to 10-membered heterocycloalkyl ring, more preferably a 3- to 8-membered heterocycloalkyl ring, further preferably a 3- to 6-membered heterocycloalkyl ring); the C₁₋₈ alkyl, C₁₋₈ haloalkyl, C₁₋₈ alkoxy, C₁₋₈ haloalkoxy, -COC₁₋₈ alkyl, -COOC₁₋₈ alkyl, -OCOC₁₋₈ alkyl, -CONH₂, -CONHC₁₋₈ alkyl, -CON(C₁₋₈ alkyl)₂, -SOC₁₋₈ alkyl, -SO₂C₁₋₈ alkyl, -OSO₂C₁₋₈ alkyl, -SO₂NH₂, -SO₂NHC₁₋₈ alkyl, -SO₂N(C₁₋₈ alkyl)₂, C₃₋₈ cycloalkyl, 3- to 12-membered heterocycloalkyl, 5- to 10-membered heteroaryl, C₆₋₁₄ aryl, C₃₋₁₅ cycloalkyl ring and 3- to 15-membered heterocycloalkyl ring are unsubstituted or substituted with 1, 2 or 3 substituents selected from the group consisting of: halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl) and 5- to 10-membered heteroaryl (preferably 5- to 6-membered heteroaryl);
(R₂)ₙ₂ represents substitution of hydrogen on ring A1 by n2 R₂, n2 is 0, 1 or 2, each R₂ is identical or different, and each R₂ is independently selected from the group consisting of X₁, hydrogen, deuterium, halogen, cyano, carboxyl, hydroxyl, amino, oxo, C₁₋₈ alkyl (preferably C₁₋₆ alkyl, more preferably C₁₋₃ alkyl), C₁₋₈ haloalkyl (preferably C₁₋₆ haloalkyl, more preferably C₁₋₃ haloalkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy), -NHC₁₋₈ alkyl (preferably -NHC₁₋₆ alkyl, more preferably -NHC₁₋₃ alkyl), -N(C₁₋₈ alkyl)₂ (preferably -N(C₁₋₆ alkyl)₂, more preferably -N(C₁₋₃ alkyl)₂), -COC₁₋₈ alkyl (preferably -COC₁₋₆ alkyl, more preferably -COC₁₋₃ alkyl), -OCOC₁₋₈ alkyl (preferably -OCOC₁₋₆ alkyl, more preferably -OCOC₁₋₃ alkyl), -COOC₁₋₈ alkyl (preferably -COOC₁₋₆ alkyl, more preferably -COOC₁₋₃ alkyl), -CONH₂, -CONHC₁₋₈ alkyl (preferably -CONHC₁₋₆ alkyl, more preferably -CONHC₁₋₃ alkyl), -CON(C₁₋₈ alkyl)₂ (preferably -CON(C₁₋₆ alkyl)₂, more preferably -CON(C₁₋₃ alkyl)₂), -PO(C₁₋₈ alkyl)₂ (preferably -PO(C₁₋₆ alkyl)₂, more preferably -PO(C₁₋₃ alkyl)₂), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), -OSO₂C₁₋₈ alkyl (preferably -OSO₂C₁₋₆ alkyl, more preferably -OSO₂C₁₋₃ alkyl), -SC₁₋₆ alkyl, -SO₂NH₂, -SO₂NHC₁₋₈ alkyl (preferably -SO₂NHC₁₋₆ alkyl, more preferably -SO₂NHC₁₋₃ alkyl), -SO₂N(C₁₋₈ alkyl)₂ (preferably -SO₂N(C₁₋₆ alkyl)₂, more preferably -SO₂N(C₁₋₃ alkyl)₂), C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl); the C₁₋₈ alkyl, C₁₋₈ haloalkyl, C₁₋₈ alkoxy, C₁₋₈ haloalkoxy, -NHC₁₋₈ alkyl, -N(C₁₋₈ alkyl)₂, -COC₁₋₈ alkyl, -OCOC₁₋₈ alkyl, -COOC₁₋₈ alkyl, -CONH₂, -CONHC₁₋₈ alkyl, -CON(C₁₋₈ alkyl)₂, -PO(C₁₋₈ alkyl)₂, -SOC₁₋₈ alkyl, -SO₂C₁₋₈ alkyl, -OSO₂C₁₋₈ alkyl, -SO₂NH₂, -SO₂NHC₁₋₈ alkyl, -SO₂N(C₁₋₈ alkyl)₂, C₃₋₈ cycloalkyl, 3- to 12-membered heterocycloalkyl, 5- to 10-membered heteroaryl, C₆₋₁₄ aryl are unsubstituted or substituted with 1, 2 or 3 substituents selected from the group consisting of: halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, NHC₁₋₆ alkyl, -N(C₁₋₆ alkyl)₂, -COC₁₋₆alkyl, -OCOC₁₋₆ alkyl, -COOC₁₋₆ alkyl, -CONH₂, -CONHC₁₋₆ alkyl, -CON(C₁₋₆ alkyl)₂, -PO(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl);
or R₁, R₂ and carbon atom linked to R₁ and R₂ together form a 6- to 15-membered heterocycloalkyl ring; the 6- to 15-membered heterocycloalkyl ring is unsubstituted or substituted with 1, 2, 3 or 4 substituents selected from the group consisting of: halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, -NHC₁₋₆ alkyl, -N(C₁₋₆ alkyl)₂, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -PO(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl);
(R₃)ₙ₃ represents substitution of hydrogen on ring A2 by n3 R₃, n3 is 0, 1, 2 or 3, each R₃ is identical or different, and each R₃ is independently selected from the group consisting of X₁, hydrogen, deuterium, cyano, carboxyl, nitro, formyl, sulfo, halogen, oxo, C₁₋₁₀ alkyl (preferably C₁₋₈ alkyl, more preferably C₁₋₆ alkyl, further preferably C₁₋₃ alkyl), C₁₋₁₀ haloalkyl (preferably C₁₋₈ haloalkyl, more preferably C₁₋₆ haloalkyl, further preferably C₁₋₃ haloalkyl), C₁₋₁₀ alkoxy (preferably C₁₋₈ alkoxy, more preferably C₁₋₆ alkoxy, further preferably C₁₋₃ alkoxy), C₁₋₁₀ haloalkoxy (preferably C₁₋₈ haloalkoxy, more preferably C₁₋₆ haloalkoxy, further preferably C₁₋₃ haloalkoxy), -COC₁₋₁₀ alkyl (preferably -COC₁₋₈ alkyl, more preferably -COC₁₋₆alkyl, further preferably -COC₁₋₃ alkyl), -OCOC₁₋₁₀ alkyl (preferably -OCOC₁₋₈ alkyl, more preferably -OCOC₁₋₆ alkyl, further preferably -OCOC₁₋₃ alkyl), -COOC₁₋₁₀ alkyl (preferably -COOC₁₋₈ alkyl, more preferably -COOC₁₋₆ alkyl, further preferably -COOC₁₋₃ alkyl), -CONH₂, -CONHC₁₋₁₀ alkyl (preferably -CONHC₁₋₈ alkyl, more preferably -CONHC₁₋₆ alkyl, further preferably -CONHC₁₋₃ alkyl), -CON(C₁₋₁₀ alkyl)₂ (preferably -CON(C₁₋₈ alkyl)₂, more preferably -CON(C₁₋₆ alkyl)₂, further preferably -CON(C₁₋₃ alkyl)₂), -SOC₁₋₁₀ alkyl (preferably -SOC₁₋₈ alkyl, more preferably -SOC₁₋₆ alkyl, further preferably -SOC₁₋₃ alkyl), -SO₂C₁₋₁₀ alkyl (preferably -SO₂C₁₋₈ alkyl, more preferably -SO₂C₁₋₆ alkyl, further preferably -SO₂C₁₋₃ alkyl), -OSO₂C₁₋₁₀ alkyl (preferably -OSO₂C₁₋₈ alkyl, more preferably -OSO₂C₁₋₆ alkyl, further preferably -OSO₂C₁₋₃ alkyl), -SO₂NH₂, -SO₂NHC₁₋₁₀ alkyl (preferably -SO₂NHC₁₋₈ alkyl, more preferably -SO₂NHC₁₋₆ alkyl, further preferably -SO₂NHC₁₋₃ alkyl), -SO₂N(C₁₋₁₀ alkyl)₂ (preferably -SO₂N(C₁₋₈ alkyl)₂, more preferably -SO₂N(C₁₋₆ alkyl)₂, further preferably -SO₂N(C₁₋₃ alkyl)₂), C₃₋₈ cycloalkyl (preferably C₃₋₆ cycloalkyl), 3- to 15-membered heterocycloalkyl (preferably 4- to 12-membered heterocycloalkyl, more preferably 4- to 8-membered heterocycloalkyl, further preferably 4- to 6-membered heterocycloalkyl), 5- to 10-membered heteroaryl (preferably 5- to 6-membered heteroaryl) and C₆₋₁₄ aryl; the C₁₋₁₀ alkyl, C₁₋₁₀ haloalkyl, C₁₋₁₀ alkoxy, C₁₋₁₀ haloalkoxy, -COC₁₋₁₀ alkyl, -OCOC₁₋₁₀ alkyl, -COOC₁₋₁₀ alkyl, -CONH₂, -CONHC₁₋₁₀ alkyl, -CON(C₁₋₁₀ alkyl)₂, -SOC₁₋₁₀ alkyl, -SO₂C₁₋₁₀ alkyl, -OSO₂C₁₋₁₀ alkyl, -SO₂NH₂, -SO₂NHC₁₋₁₀ alkyl, -SO₂N(C₁₋₁₀ alkyl)₂, C₃₋₈ cycloalkyl, 3- to 15-membered heterocycloalkyl, 5- to 10-membered heteroaryl, C₆₋₁₄ aryl are unsubstituted or substituted with 1, 2 or 3 substituents selected from the group consisting of: cyano, hydroxyl, carboxyl, nitro, formyl, sulfo, halogen, C₁₋₆ alkyl, C₁₋₆ haloalkyl, C₁₋₆ alkoxy, C₁₋₆ haloalkoxy, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₆ cycloalkyl, 3- to 15-membered heterocycloalkyl (preferably 4- to 12-membered heterocycloalkyl, more preferably 4- to 8-membered heterocycloalkyl, further preferably 4- to 6-membered heterocycloalkyl), 5- to 10-membered heteroaryl (preferably 5- to 6-membered heteroaryl), phenyl and naphthyl;
X₁ in R₂ and R₃ is a point of attachment of POI to L or ULM, and at least one of R₂ and R₃ is X₁.

In some embodiments, ring A1 is an 8- to 10-membered heteroaryl ring.

In some embodiments, ring A1 is a 9-membered heteroaryl ring or a 10-membered heteroaryl ring.

In some embodiments, ring A1 is a 9-membered nitrogen-containing heteroaryl ring or a 10-membered nitrogen-containing heteroaryl ring.

In some embodiments, ring A1 is selected from the group consisting of an indolizine ring, a pyrazolopyridine ring, an imidazopyridine ring, a triazolopyridine ring, a tetrazolopyridine ring, a pyrrolopyridazine ring, a pyrazolopyridazine ring, an imidazopyridazine ring, a triazolopyridazine ring, a tetrazolopyridazine ring, a pyrrolopyrimidine ring, a pyrazolopyrimidine ring, an imidazopyrimidine ring, a triazolopyrimidine ring, a tetrazolopyrimidine ring, a pyrrolopyrazine ring, a pyrazolopyrazine ring, an imidazopyrazine ring, a triazolopyrazine ring, a pyridopyridine ring, a pyridopyrazine ring, a pyridopyridazine ring, a pyridotriazine ring, a pyridopyrimidine ring and a tetrazolopyrazine ring.

In some embodiments, ring A1 is selected from the group consisting of [1,2,4]triazolo[4,3-c]pyrimidine, imidazo[1,5-c]pyrimidine, imidazo[1,2-c]pyrimidine, [1,2,4]triazolo[4,3-a]pyridine and pyrido[3,4-d]pyridazine.

In some embodiments, ring A1 is a 5- to 6-membered heterocycloalkyl ring.

In some embodiments, ring A1 is selected from the group consisting of pyrimidin-4(3H)-one, 1,6-dihydropyrimidine, pyridin-2(1H)-one, 1,2-dihydropyridine and 3,8-diazabicyclo[3.2.1]octane.

In some embodiments, ring A1 is an 18-membered heterocycloalkyl ring.

In some embodiments, ring A1 is a partially unsaturated 18-membered heterocycloalkyl ring.

In some embodiments, ring A1 is selected from the group consisting of 2,3-dihydro-7'H-spiro[indene-1,8'-pyrido[3,4-d]pyridazin]-7'-one and 2,3-dihydro-7'H-spiro[indene-1,8'-pyrido[4,3-c]pyridazin]-7'-one.

In some embodiments, 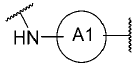 is selected from the group consisting of the following structures or isomers thereof:

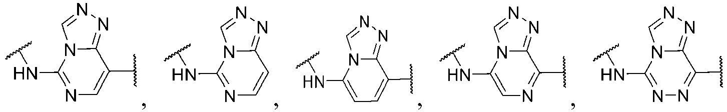

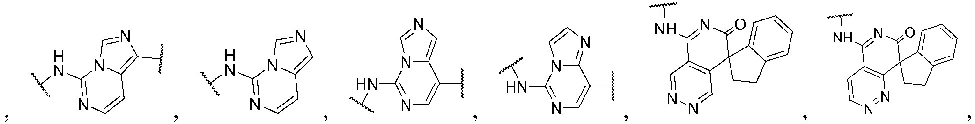

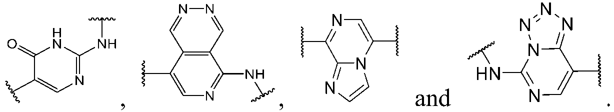

In some embodiments, 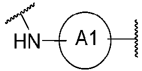 is selected from the group consisting of the following structures or isomers thereof:

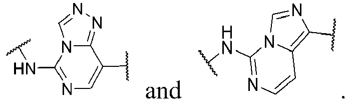

In some embodiments, 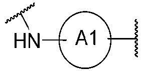 is selected from the group consisting of the following structures or isomers thereof:

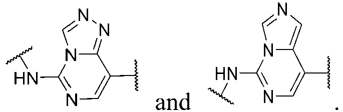

In some embodiments, 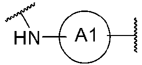 is selected from the group consisting of the following structures or isomers thereof:

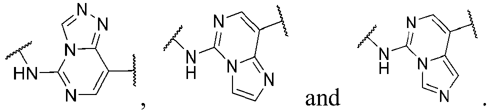

In some embodiments, POI is a structure of formula (A-2-1) or an isomer thereof: 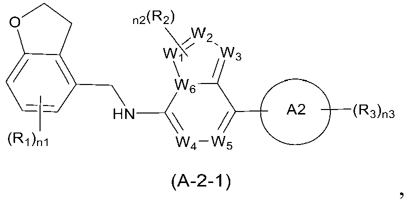 wherein, W₁, W₂, W₃, W₄, W₅ and W₆ are each independently CH or N; ring A2, R₁, n1, R₂, n2, R₃ and n3 are as defined in the specification, and at least one of R₂ and R₃ is X₁.

In some embodiments, POI is a structure of formula (A-2-2) or an isomer thereof: 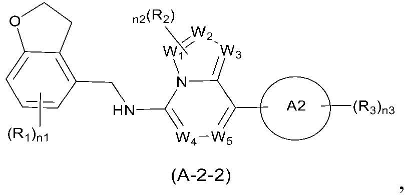 wherein, W₁, W₂, W₃, W₄ and W₅ are each independently CH or N; ring A2, R₁, n1, R₂, n2, R₃ and n3 are as defined in the specification, and at least one of R₂ and R₃ is X₁.

In some embodiments, POI is a structure of formula (A-3) or an isomer thereof: 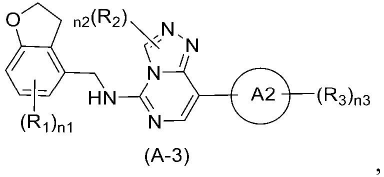 wherein, ring A2, R₁, n1, R₂, n2, R₃ and n3 are as defined in the specification, and at least one of R₂ and R₃ is X₁.

In some embodiments, POI is a structure of formula (A-4) or an isomer thereof: 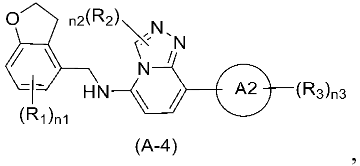 wherein, ring A2, R₁, n1, R₂, n2, R₃ and n3 are as defined in the specification, and at least one of R₂ and R₃ is X₁.

In some embodiments, POI is a structure of formula (A-5) or an isomer thereof: 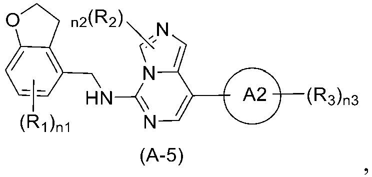 wherein, ring A2, R₁, n1, R₂, n2, R₃ and n3 are as defined in the specification, and at least one of R₂ and R₃ is X₁.

In some embodiments, POI is a structure of formula (A-6) or an isomer thereof: 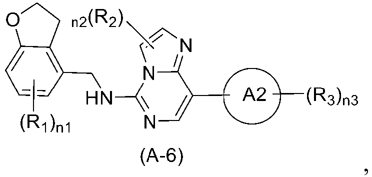 wherein, ring A2, R₁, n1, R₂, n2, R₃ and n3 are as defined in the specification, and at least one of R₂ and R₃ is X₁.

In some embodiments, POI is a structure of formula (A-7) or an isomer thereof: 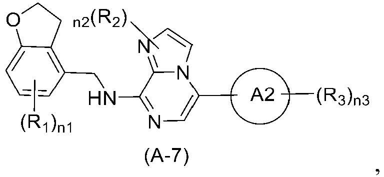 wherein, ring A2, R₁, n1, R₂, n2, R₃ and n3 are as defined in the specification, and at least one of R₂ and R₃ is X₁.

In some embodiments, POI is a structure of formula (A-8) or an isomer thereof: 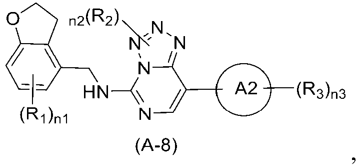 wherein, ring A2, R₁, n1, R₂, n2, R₃ and n3 are as defined in the specification, and at least one of R₂ and R₃ is X₁.

In some embodiments, ring A2 is absent or is selected from the group consisting of a 4- to 8-membered heterocycloalkyl ring, a C₄₋₈ cycloalkyl ring, a 5- to 10-membered heteroaryl ring, a 10-to 15-membered heterocycloalkyl ring and a benzene ring.

In some embodiments, ring A2 is absent or is selected from the group consisting of a 4- to 8-membered heterocycloalkyl ring, a saturated C₄₋₈ cycloalkyl ring, a 5- to 10-membered heteroaryl ring, a partially unsaturated 10- to 15-membered heterocycloalkyl ring and a benzene ring.

In some embodiments, ring A2 is absent or is selected from the group consisting of a cyclobutane ring, a cyclopentane ring, a cyclohexane ring, an azetidine ring, a tetrahydropyrrole ring, a pyrazolidine ring, an imidazolidine ring, a piperidine ring, a hexahydropyridazine ring, a hexahydropyrimidine ring, a piperazine ring, a benzene ring, a pyrazole ring, an oxazole ring, an imidazole ring, a triazole ring, a tetrazole ring, a pyridine ring, a pyrimidine ring, a pyrazine ring, a pyridazine ring, an indolizine ring, pyrazolopyridine, imidazopyridine, triazolopyridine, tetrazolopyridine, pyrrolopyridazine, pyrazolopyridazine, imidazopyridazine, triazolopyridazine, tetrazolopyridazine, pyrrolopyrimidine, pyrazolopyrimidine, imidazopyrimidine, triazolopyrimidine, tetrazolopyrimidine, pyrrolopyrazine, pyrazolopyrazine, imidazopyrazine, triazolopyrazine, tetrazolopyrazine, pyridopyridine, pyridopyrazine, pyridopyridazine, pyridotriazine, pyridopyrimidine, 3,8-diazabicyclo[3.2.1]octane and 2,3-dihydrospiro[indene-1,4'-piperidine].

In some embodiments, ring A2 is absent or is selected from the group consisting of a saturated 6-membered heterocycloalkyl ring, a partially unsaturated 12-membered heterocycloalkyl ring, a cyclohexane ring, a 5- to 6-membered heteroaryl ring, a 9-membered heteroaryl ring and a benzene ring.

In some embodiments, ring A2 is absent or is selected from the group consisting of a cyclohexane ring, a piperidine ring, a piperazine ring, a benzene ring, a pyridine ring, a pyrazole ring, a triazolopyridine ring and 2,3-dihydrospiro[indene-1,4'-piperidine].

In some embodiments, ring A2 is selected from the group consisting of a benzene ring, a pyridine ring and a pyrazole ring.

In some embodiments, 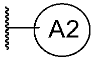 is selected from the group consisting of the following structures or isomers thereof:

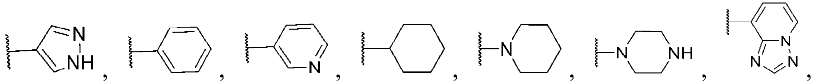

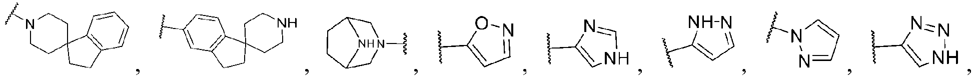

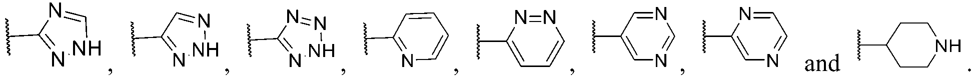

In some embodiments, 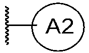 is selected from the group consisting of the following structures:

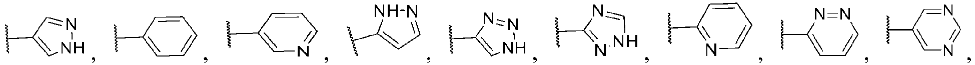

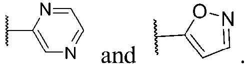

In some embodiments, 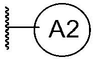 is

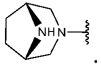

In some embodiments, 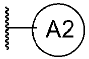 is selected from the group consisting of the following structures or isomers thereof:

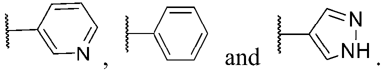

In some embodiments, 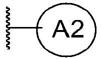 is selected from the group consisting of the following structures or isomers thereof:

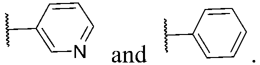

In some embodiments, n1 is 0.

In some embodiments, n1 is 1 or 2.

In some embodiments, n1 is 1.

In some embodiments, R₁ is selected from the group consisting of hydrogen, deuterium, fluorine, chlorine, bromine, iodine, methyl, ethyl, propyl, isopropyl, monofluoromethyl, difluoromethyl, trifluoromethyl, monofluoroethyl, difluoroethyl, trifluoroethyl, methoxy, ethoxy, propoxy, isopropoxy, monofluoromethoxy, difluoromethoxy, trifluoromethoxy, monofluoroethoxy, difluoroethoxy, trifluoroethoxy, cyclopropyl, cyclobutyl, cyclopentyl, cyclohexyl, cyclohexenyl, pyrrolyl, pyrazolyl, pyridinyl, phenyl, pyrimidinyl, -CH₂-cyclopropyl, -CH₂-tetrahydropyrrolyl, -CH₂-pyrrolyl, -CH₂-phenyl, -CH₂-pyridinyl, -CH₂-cyclohexenyl, -CH₂-azetidine, -CH₂-piperidine, -CH₂-piperazine, tetrahydro-2H-pyran and -CH₂-(tetrahydro-2H-pyran).

In some embodiments, R₁ is selected from the group consisting of hydrogen, deuterium, fluorine, chlorine, bromine, iodine, -CH₃, -CH₂CH₃, -CH(CH₃)₂, -CH₂CH₂CH₃, -CH₂F, -CHF₂, -CF₃, -OCH₃, -OCH₂CH₃, -OCH₂CH₂CH₃, -OCH(CH₃)₂, -OCH₂F, -OCHF₂, -OCF₃, -CH₂CH₂F, -CH₂CHF₂, -CH₂CF₃, -OCH₂CH₂F, -OCH₂CHF₂ and -OCH₂CF₃.

In some embodiments, n1 is 1, and R₁ is fluorine.

In some embodiments, 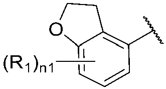 is

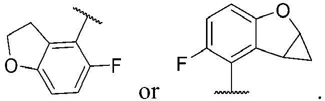

In some embodiments, 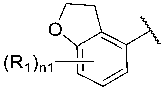 is

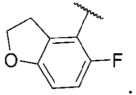

In some embodiments, two R₁ and carbon atom linked to the two R₁ together form a cyclopropyl ring, a cyclobutyl ring, a cyclopentyl ring, a cyclohexyl ring, a cyclobutene ring, a cyclopentene ring, a cyclohexene ring, an azetidine ring, a tetrahydropyrrole ring, a piperidine ring or a piperazine ring.

In some embodiments, two R₁ and carbon atom linked to the two R₁ together form a cyclopropyl ring.

In some embodiments, n3 is 3, one R₃ is fluorine, and the other two R₃ and carbon atom linked to the other two R₃ together form a cyclopropane ring.

In some embodiments, 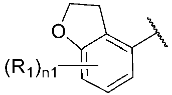 is

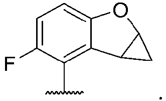

In some embodiments, 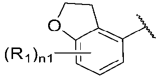 is

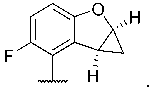

In some embodiments, R₂ is selected from the group consisting of X₁, hydrogen, deuterium, halogen, cyano, carboxyl, hydroxyl, amino, oxo, -NHC₁₋₆ alkyl, -N(C₁₋₆ alkyl)₂, -CONH₂, -CONHC₁₋₆ alkyl, -CON(C₁₋₆ alkyl)₂, -COC₁₋₆ alkyl, -COOC₁₋₆ alkyl, -OCOC₁₋₆ alkyl, -PO(C₁₋₆ alkyl)₂, -SOC₁₋₆ alkyl, -SO₂C₁₋₆ alkyl, -OSO₂C₁₋₆ alkyl, -SC₁₋₆ alkyl, C₁₋₆ alkyl, C₁₋₆ alkoxy, C₁₋₆ haloalkyl and C₁₋₆ haloalkoxy.

In some embodiments, R₂ is selected from the group consisting of X₁, hydrogen, deuterium, halogen, cyano, carboxyl, hydroxyl, amino, oxo, -NHC₁₋₆ alkyl, -N(C₁₋₆ alkyl)₂, -CONH₂, -CONHC₁₋₆ alkyl, -CON(C₁₋₆ alkyl)₂, -COC₁₋₃ alkyl, -COOC₁₋₃ alkyl, -OCOC₁₋₃ alkyl, -PO(C₁₋₃ alkyl)₂, -SOC₁₋₃ alkyl, -SO₂C₁₋₃ alkyl, -OSO₂C₁₋₃ alkyl, -SC₁₋₃ alkyl, C₁₋₃ alkyl, C₁₋₃ alkoxy, C₁₋₃ haloalkyl and C₁₋₃ haloalkoxy.

In some embodiments, R₂ is selected from the group consisting of X₁, hydrogen, deuterium, halogen, cyano, carboxyl, hydroxyl, amino, oxo, -NHCH₃, -N(CH₃)₂, -CONH₂, -CONHCH₃, -CON(CH₃)₂, -COCH₃, -COC(CH₃)₃, -COOCH₃, -COOCH₂CH₃, -COOC(CH₃)₃, -OCOCH₃, -OCOCH₂CH₃, -OCOC(CH₃)₃, -PO(CH₃)₂, -PO(CH₂CH₃)₂, -SOCH₃, -SOCH₂CH₃, -SOC(CH₃)₃, -SO₂CH₃, -SO₂CH₂CH₃, -SO₂C(CH₃)₃, -OSO₂CH₃, -OSO₂CH₂CH₃, -OSO₂C(CH₃)₃, -SCH₃, -SCH₂CH₃, methyl, ethyl, propyl, isopropyl, tert-butyl, methoxy, ethoxy, propoxy, isopropoxy, tert-butoxy, monofluoromethyl, difluoromethyl, trifluoromethyl, monofluoroethyl, difluoroethyl, trifluoroethyl, monofluoropropyl, difluoropropyl, trifluoropropyl, monofluoroisopropyl, difluoroisopropyl, trifluoroisopropyl, monofluoro-tert-butyl, difluoro-tert-butyl, trifluoro-tert-butyl, monofluoromethoxy, difluoromethoxy, trifluoromethoxy, monofluoroethoxy, difluoroethoxy, trifluoroethoxy, monofluoropropoxy, difluoropropoxy, trifluoropropoxy, monofluoroisopropoxy, difluoroisopropoxy, trifluoroisopropoxy, monofluoro-tert-butoxy, difluoro-tert-butoxy and trifluoro-tert-butoxy.

In some embodiments, R₂ is selected from the group consisting of X₁, hydrogen, halogen, cyano, -SO₂C₁₋₆ alkyl and -OSO₂C₁₋₆ alkyl.

In some embodiments, R₂ is selected from the group consisting of X₁, hydrogen, halogen, cyano, -SO₂C₁₋₃ alkyl,and -OSO₂C₁₋₃ alkyl.

In some embodiments, R₂ is selected from the group consisting of X₁, hydrogen, fluorine, chlorine, bromine, iodine, cyano, -SO₂CH₃ and -OSO₂CH₃.

In some embodiments, R₂ is selected from the group consisting of cyano, fluorine, chlorine, bromine, iodine, -CH₃, -CF₃, -SO₂CH₃, hydrogen and -PO(CH₃)₂.

In some embodiments, R₂ is selected from the group consisting of cyano, chlorine, bromine, -SO₂CH₃ and hydrogen.

In some embodiments, n2 is 1, and R₂ is selected from the group consisting of X₁, hydrogen, fluorine, chlorine, bromine, iodine, -CH₃, cyano, -SO₂CH₃, -OSO₂CH₃ and -PO(CH₃)₂.

In some embodiments, n2 is 1, and R₂ is selected from the group consisting of cyano, fluorine, chlorine, bromine, iodine, -CH₃, -CF₃, -SO₂CH₃, hydrogen and -PO(CH₃)₂.

In some embodiments, n2 is 1, and R₂ is selected from the group consisting of cyano, chlorine, bromine, -SO₂CH₃ and hydrogen.

In some embodiments, 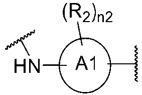 is selected from the group consisting of the following structures or isomers thereof: 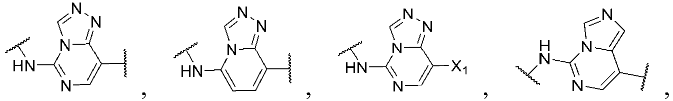 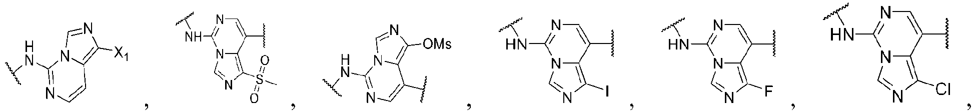 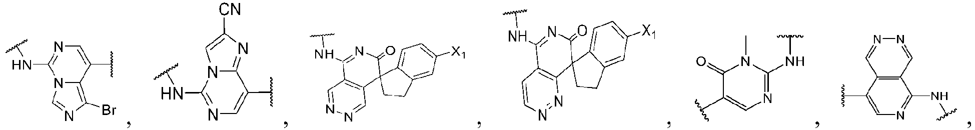 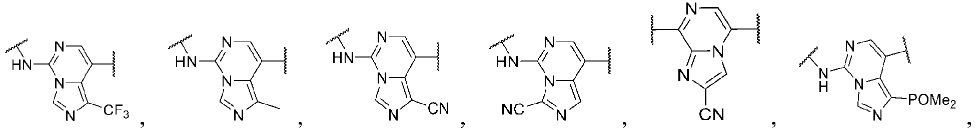 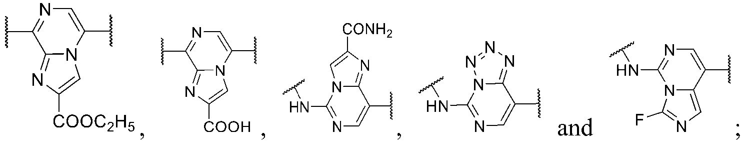 wherein X₁ is the point of attachment to L or ULM.

In some embodiments, 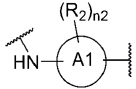 is selected from the group consisting of the following structures or isomers thereof: 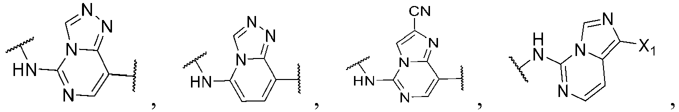 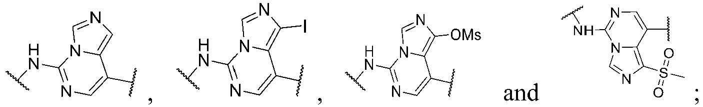 wherein X₁ is the point of attachment to L or ULM.

In some embodiments, 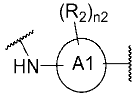 is selected from the group consisting of the following structures or isomers thereof:

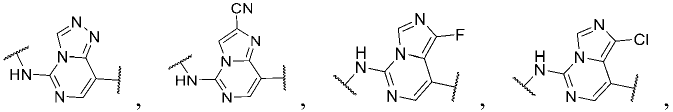

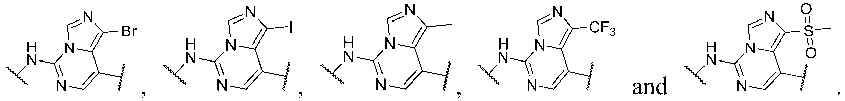

In some embodiments, 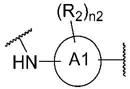 is selected from the group consisting of the following structures or isomers thereof:

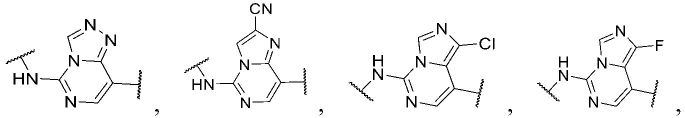

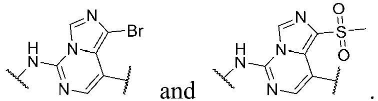

In some embodiments, 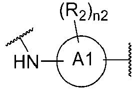 is selected from the group consisting of the following structures or isomers thereof:

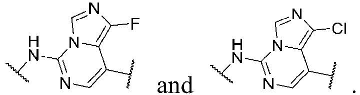

In some embodiments, R₁, R₂ and carbon atom linked to R₁, R₂ together form a 6- to 15-membered heterocycloalkyl ring, the 6- to 15-membered heterocycloalkyl ring is selected from the group consisting of 5,8-dihydro-4H-1,4-oxazocine, 4,5,8,9-tetrahydro-1,4-oxazepine, 5,8,9,10-tetrahydro-4H-1,4-oxazonine, and 1-oxa-4-azacycloundeca-2,6-diene; the 6- to 15-membered heterocycloalkyl ring is unsubstituted or substituted with 1, 2, 3 or 4 substituents selected from the group consisting of: fluorine, chlorine, bromine, iodine, cyano, hydroxyl, carboxyl, nitro, formyl, sulfo, methyl, ethyl, isopropyl, tert-butyl, monofluoromethyl, difluoromethyl, trifluoromethyl, monofluoroethyl, difluoroethyl, trifluoroethyl, methoxy, ethoxy, isopropoxy, tert-butoxy, monofluoromethoxy, difluoromethoxy, trifluoromethoxy, monofluoroethoxy, difluoroethoxy, trifluoroethoxy, -COCH₃, -COCH₂CH₃, -COCH(CH₃)₂, -COC(CH₃)₃, -OCOCH₃, -OCOCH₂CH₃, -OCOCH(CH₃)₂, -OCOC(CH₃)₃, -CONH₂, -CONHCH₃, -CONHCH₂CH₃, -CONHCH(CH₃)₂, -CONHC(CH₃)₃, -CON(CH₃)₂, -CON(CH₂CH₃)₂, -SOCH₃, -SO₂CH₃, -SO₂CH₂CH₃, -SO₂CH(CH₃)₂, -SO₂C(CH₃)₃, -OSO₂CH₃, -OSO₂CH₂CH₃, -OSO₂CH(CH₃)₂, -OSO₂C(CH₃)₃, -SO₂NH₂, -SO₂NHCH₃, -SO₂NHCH₂CH₃, -SO₂NHCH(CH₃)₂, -SO₂NHC(CH₃)₃, -SO₂N(CH₃)₂, -SO₂N(CH₂CH₃)₂, cyclopropyl, cyclobutyl, cyclopentyl, cyclohexyl, cyclohexenyl, azetidinyl, tetrahydropyrrolyl, piperidinyl, piperazinyl, pyrrolyl, pyrazolyl, pyridinyl, phenyl and pyrimidinyl.

In some embodiments, R₁, R₂ and carbon atom linked to R₁, R₂ together form 4,5,8,9-tetrahydro-1,4-oxazepine, 5,8-dihydro-4H-1,4-oxazocine, 5,8,9,10-tetrahydro-4H-1,4-oxazonine, or 1-oxa-4-azacycloundeca-2,6-diene.

In some embodiments, R₁, R₂ and carbon atom linked to R₁, R₂ together form (2Z,6Z)-4,5,8,9-tetrahydro-1,4-oxazepine, (2E,6Z)-4,5,8,9-tetrahydro-1,4-oxazepine, (2E,6E)-4,5,8,9-tetrahydro-1,4-oxazepine, (2Z,6E)-4,5,8,9-tetrahydro-1,4-oxazepine, (2Z,6Z)-5,8-dihydro-4H-1,4-oxazocine, (2E,6Z)-5,8-dihydro-4H-1,4-oxazocine, (2Z,6E)-5,8-dihydro-4H-1,4-oxazocine, (2E,6E)-5,8-dihydro-4H-1,4-oxazocine, (2Z,6Z)-5,8,9,10-tetrahydro-4H-1,4-oxazonine, (2E,6Z)-5,8,9,10-tetrahydro-4H-1,4-oxazonine, (2Z,6E)-5,8,9,10-tetrahydro-4H-1,4-oxazonine, (2E,6E)-5,8,9,10-tetrahydro-4H-1,4-oxazonine, (2Z,6Z)-1-oxa-4-azacycloundeca-2,6-diene, (2E,6Z)-1-oxa-4-azacycloundeca-2,6-diene, (2Z,6E)-1-oxa-4-azacycloundeca-2,6-diene or (2E,6E)-1-oxa-4-azacycloundeca-2,6-diene.

In some embodiments, R₁, R₂ and carbon atom linked to R₁, R₂ together form (2Z,6Z)-4,5,8,9-tetrahydro-1,4-oxazepine, (2Z,6Z)-5,8-dihydro-4H-1,4-oxazocine, (2Z,6Z)-5,8,9,10-tetrahydro-4H-1,4-oxazonine or (2Z,6Z)-1-oxa-4-azacycloundeca-2,6-diene.

In some embodiments, 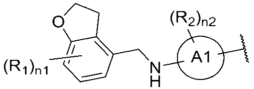 is selected from the group consisting of 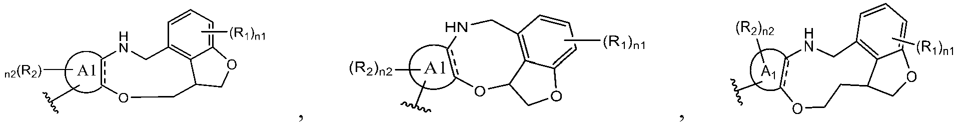 and 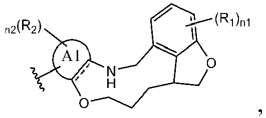 wherein -̅ -̅ -̅ -̅ -̅ -̅ represents 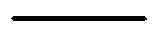 (single bond) or 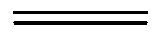 (double bond); n1 is 0 or 1, and n2 is 0 or 1.

In some embodiments, 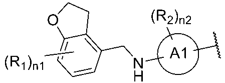 is selected from the group consisting of 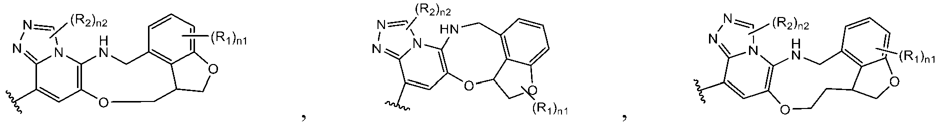 and 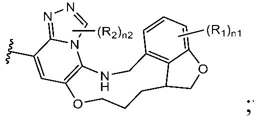 wherein n1 is 0 or 1, and n2 is 0 or 1.

In some embodiments, 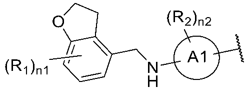 is selected from the group consisting of

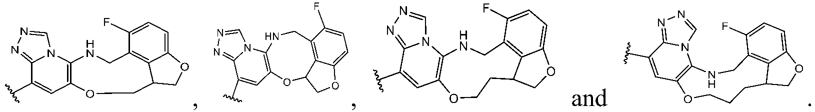

In some embodiments, 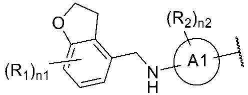 is selected from the group consisting of

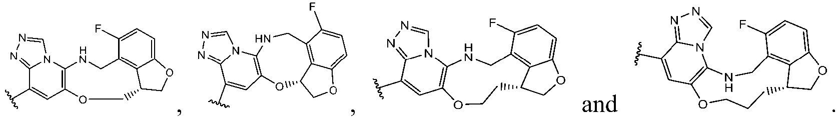

In some embodiments, 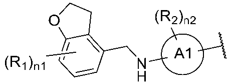 is

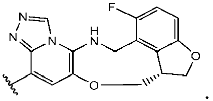

In some embodiments, R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl; the C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl are unsubstituted or substituted with 1, 2 or 3 substituents selected from the group consisting of: cyano, hydroxyl, carboxyl, nitro, formyl, sulfo, halogen, C₁₋₃ alkyl, C₁₋₃ haloalkyl, C₁₋₃ alkoxy, C₁₋₃ haloalkoxy, -COC₁₋₃ alkyl, -COOC₁₋₃ alkyl, -CONH₂, -CONHC₁₋₃ alkyl, -CON(C₁₋₃ alkyl)₂, -SOC₁₋₃ alkyl, -SO₂C₁₋₃ alkyl, -OSO₂C₁₋₃ alkyl, -SO₂NH₂, -SO₂NHC₁₋₃ alkyl, -SO₂N(C₁₋₃ alkyl)₂, C₃₋₆ cycloalkyl, 4- to 6-membered heterocycloalkyl, 5- to 6-membered heteroaryl and phenyl.

In some embodiments, R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl; the C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl are unsubstituted or substituted with 1, 2 or 3 substituents selected from the group consisting of: cyano, hydroxyl, carboxyl, nitro, formyl, sulfo, halogen, methyl, trifluoromethyl, methoxy, trifluoromethoxy, -COCH₃, -OCOCH₃, -COOCH₃, -CONH₂, -CONHCH₃, -CON(CH₃)₂, -SOCH₃, -SO₂CH₃, -SO₂NH₂, -SO₂NHCH₃, -SO₂N(CH₃)₂, cyclopropyl, cyclobutyl, cyclopentyl, cyclohexyl, cyclobutenyl, cyclopentenyl, cyclohexenyl, azetidinyl, tetrahydropyrrolyl, piperidinyl, piperazinyl, pyrrolyl, pyrazolyl, imidazolyl, triazolyl, pyridinyl, pyrimidinyl, pyridazinyl and phenyl.

In some embodiments, R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CO₂NH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl.

In some embodiments, R₃ is selected from the group consisting of X₁, fluorine, chlorine, bromine, iodine, methyl, ethyl, n-propyl, isopropyl, tert-butyl, methoxy, ethoxy, isopropoxy, tert-butoxy, monofluoromethyl, difluoromethyl, trifluoromethyl, monofluoroethyl, difluoroethyl, trifluoroethyl, monofluoromethoxy, difluoromethoxy, trifluoromethoxy, monofluoroethoxy, difluoroethoxy, trifluoroethoxy, -COCH₃, -COCH₂CH₃, -COC(CH₃)₃, -OCOCH₃, -OCOCH₂CH₃, -OCOC(CH₃)₃, -COOCH₃, -COOCH₂CH₃, -COOC(CH₃)₃, -CONH₂, -CONHCH₃, -CON(CH₃)₂, -SOCH₃, -SO₂CH₃, -SO₂C(CH₃)₃, -OSO₂CH₃, -OSO₂C(CH₃)₃, -SO₂NH₂, -SO₂NHCH₃, -SO₂N(CH₃)₂, cyclopropyl, cyclobutyl, cyclobutenyl, cyclopentyl, cyclopentenyl, cyclohexyl, cyclohexenyl, azetidinyl, piperazinyl, piperidinyl, 1,2,3,6-tetrahydropyridinyl, pyrrolyl, pyrazolyl, imidazolyl, pyridinyl, pyrimidinyl and phenyl.

In some embodiments, R₃ is selected from the group consisting of X₁, C₁₋₆ alkyl, C₃₋₈ cycloalkyl, C₁₋₆ haloalkyl and -COC₁₋₆ alkyl.

In some embodiments, R₃ is selected from the group consisting of X₁, C₁₋₃ alkyl, C₃₋₆ cycloalkyl, C₁₋₃ haloalkyl and -COC₁₋₃ alkyl.

In some embodiments, R₃ is selected from the group consisting of X₁, methyl, cyclopropyl, trifluoromethyl and -COCH₃.

In some embodiments, R₃ is selected from the group consisting of X₁, fluorine and methyl.

In some embodiments, n3 is 0.

In some embodiments, n3 is 1, and R₃ is selected from the group consisting of X₁, methyl, cyclopropyl, trifluoromethyl and -COCH₃.

In some embodiments, n3 is 1, and R₃ is methyl.

In some embodiments, n3 is 2, and R₃ are X₁ and methyl, respectively.

In some embodiments, 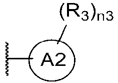 is selected from the group consisting of the following structures or isomers thereof: 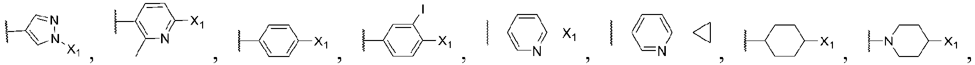 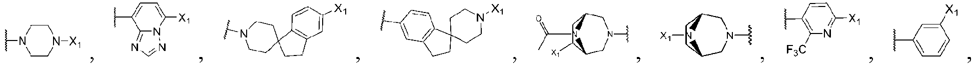 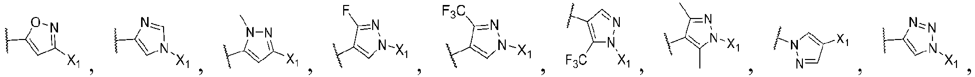 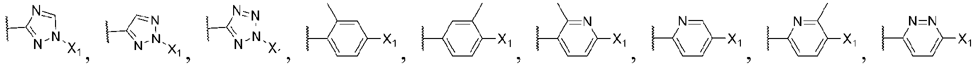 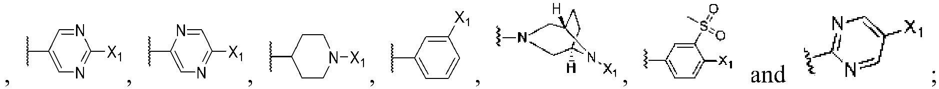 X₁ is the point of attachment to L or ULM.

In some embodiments, 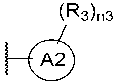 is selected from the group consisting of the following structures or isomers thereof: 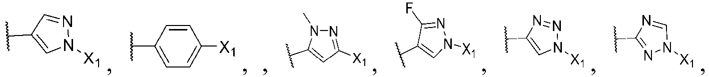 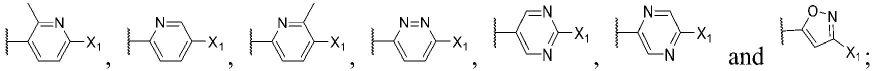 X₁ is the point of attachment to L or ULM.

In some embodiments, 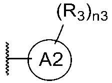 is selected from the group consisting of the following structures or isomers thereof: 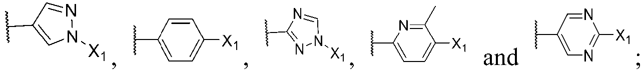 X₁ is the point of attachment to L or ULM.

In some embodiments, 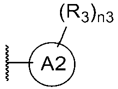 is selected from the group consisting of the following structures or isomers thereof: 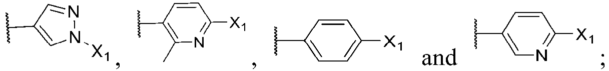 X₁ is the point of attachment to L or ULM.

In some embodiments, 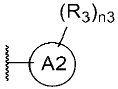 is selected from the group consisting of the following structures or isomers thereof: 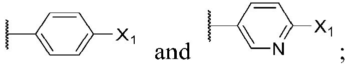 X₁ is the point of attachment to L or ULM.

In some embodiments, POI is selected from the group consisting of the following structures or isomers thereof: 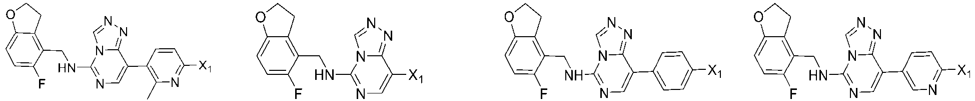 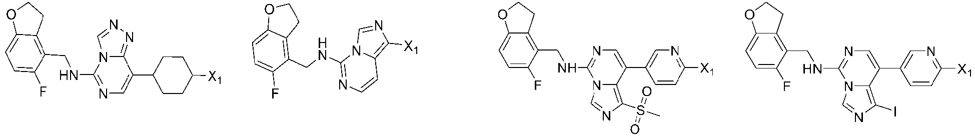 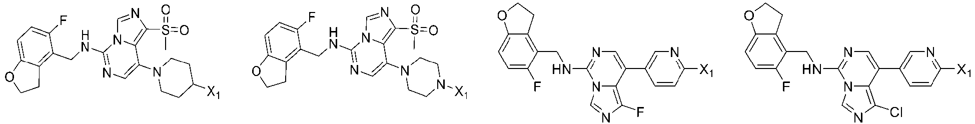 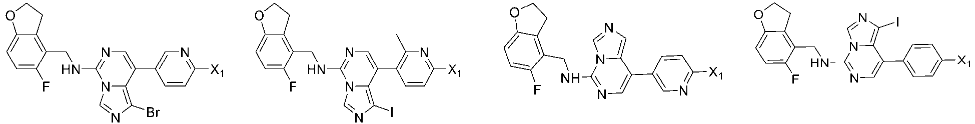 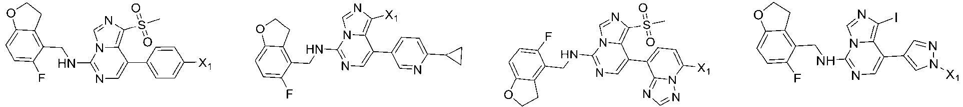 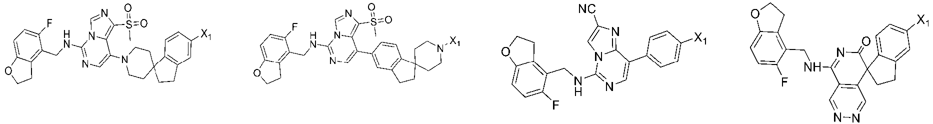 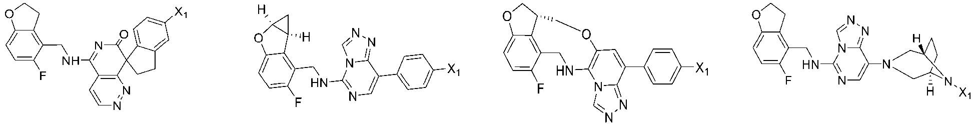 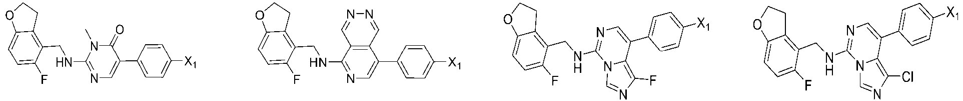 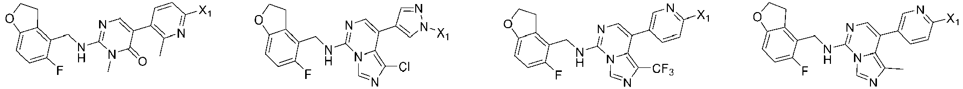 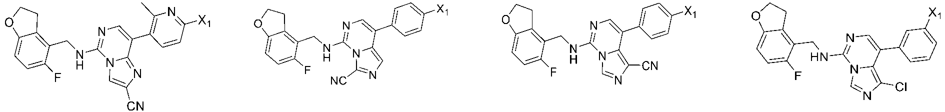 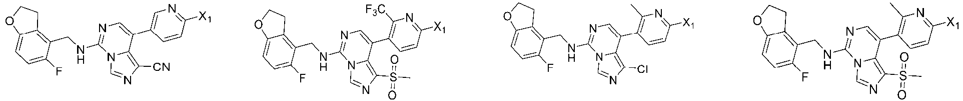 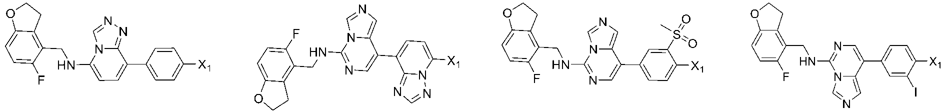 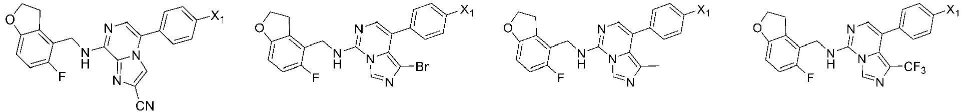 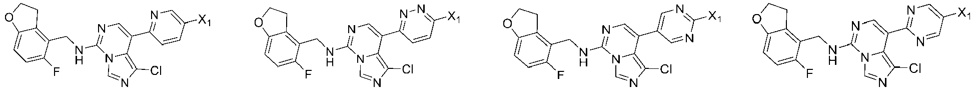 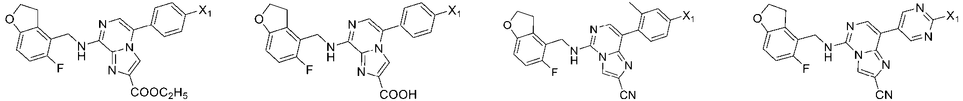 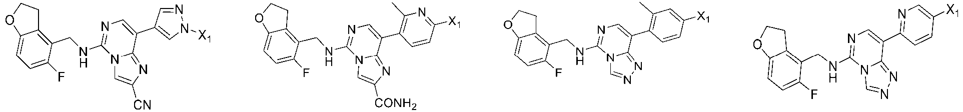 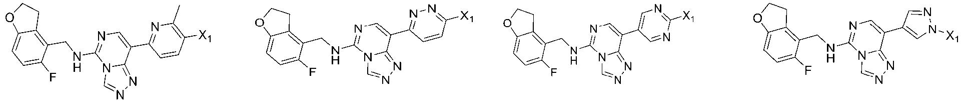 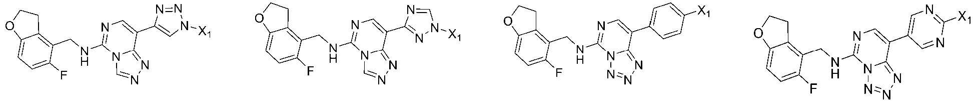 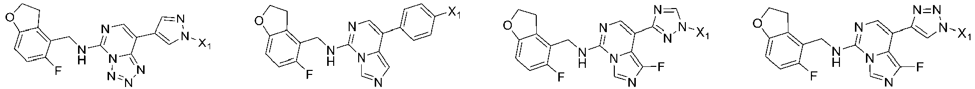 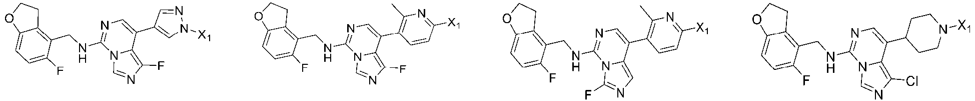 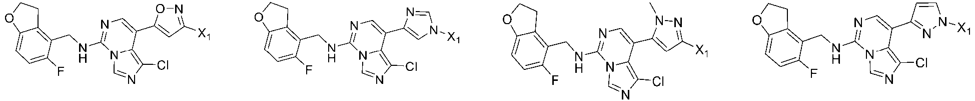 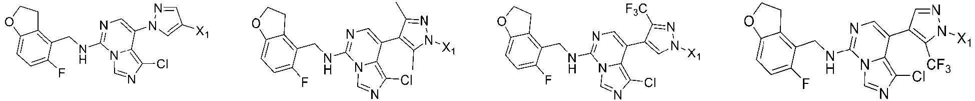 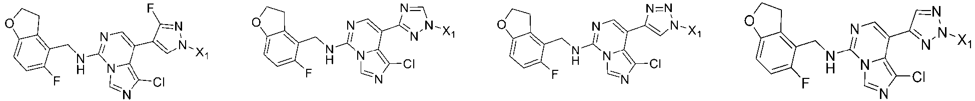 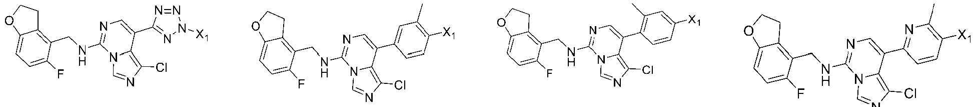 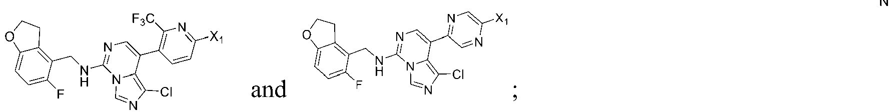 wherein X₁ is the point of attachment to L or ULM.

In some embodiments, POI is selected from the group consisting of the following structures or isomers thereof: 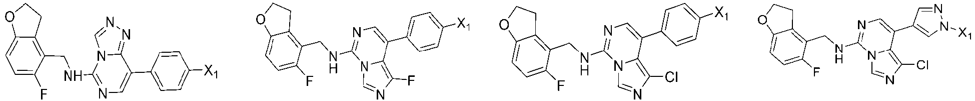 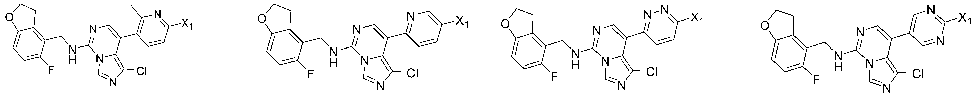 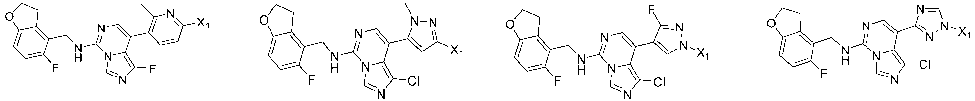 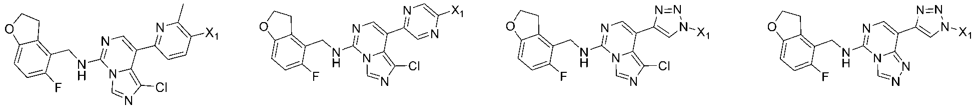 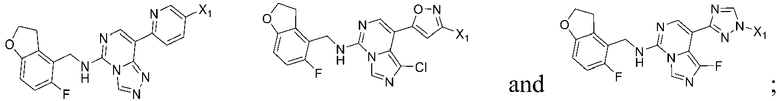 wherein X₁ is the point of attachment to L or ULM.

In some embodiments, POI is selected from the group consisting of the following structures or isomers thereof: 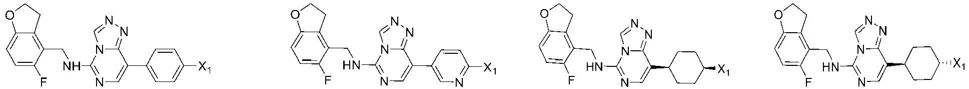 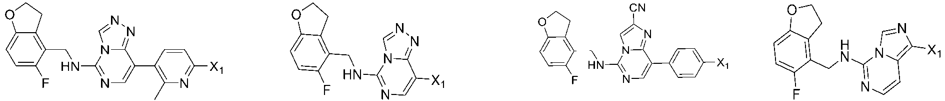 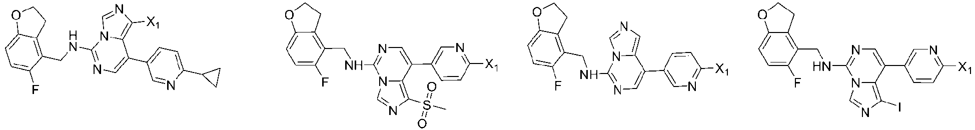 and 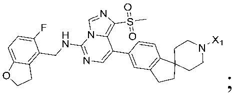 wherein X₁ is the point of attachment to L or ULM.

In some embodiments, POI is selected from the group consisting of the following structures or isomers thereof: 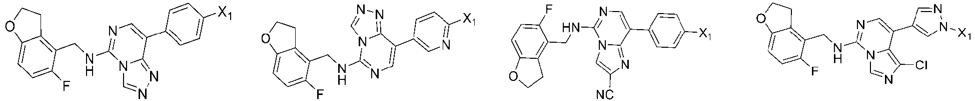 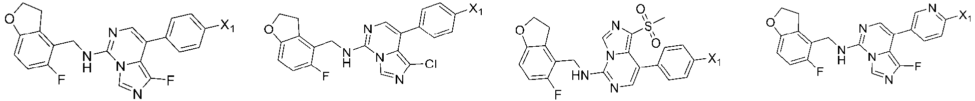 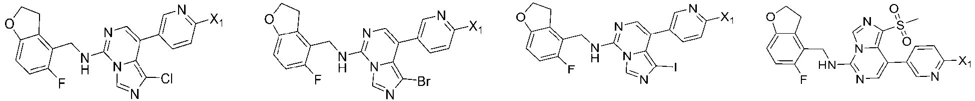 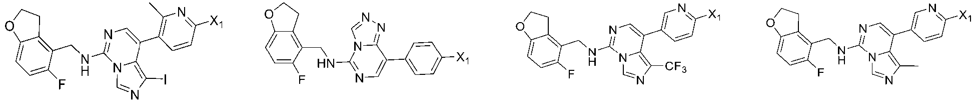 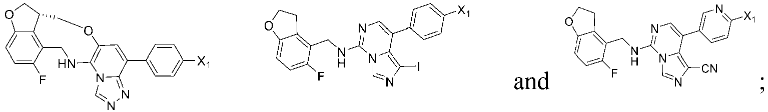 wherein X₁ is the point of attachment to L or ULM.

In some embodiments, POI is selected from the group consisting of the following structures or isomers thereof: 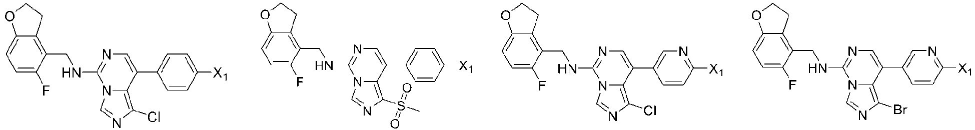 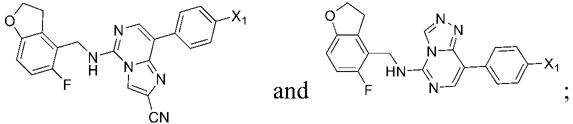 wherein X₁ is the point of attachment to L or ULM.

In some embodiments, L is a structure of formula (L-1) or an isomer thereof,

-(Lₐ)ₘ₁- (L-1),
wherein m1 is independently 0, 1, 2, 3, 4, 5, 6, 7, 8, 9 or 10;
each Lₐ at each occurrence is independently selected from the group consisting of a chemical bond, -C(O)-, -C(O)NR_{L1}-, -NR_{L1}-, -O-, -S-, C₁₋₁₀ alkylene (preferably C₁₋₈ alkylene, more preferably C₁₋₆ alkylene, further preferably C₁₋₃ alkylene), C₁₋₁₀ alkyleneoxy (preferably C₁₋₈ alkyleneoxy, more preferably C₁₋₆ alkyleneoxy, further preferably C₁₋₃ alkyleneoxy), C₂₋₁₀ alkenylene (preferably C₂₋₈ alkenylene, more preferably C₂₋₆ alkenylene, further preferably C₂₋₄ alkenylene), C₂₋₁₀ alkynylene (preferably C₂₋₈ alkynylene, more preferably C₂₋₆ alkynylene, further preferably C₂₋₄ alkynylene), a C₃₋₁₅ cycloalkylene ring (preferably a C₃₋₁₀ cycloalkylene ring, more preferably a C₃₋₈ cycloalkylene ring, further preferably a C₃₋₆ cycloalkylene ring), a 3- to 15-membered heterocycloalkylene ring (preferably a 4- to 12-membered heterocycloalkylene ring, more preferably a 4- to 10-membered heterocycloalkylene ring, further preferably a 4- to 8-membered heterocycloalkylene ring, further preferably a 4- to 6-membered heterocycloalkylene ring), a 5- to 15-membered heteroarylene ring (preferably a 5- to 14-membered heteroarylene ring, more preferably a 5- to 12-membered heteroarylene ring, further preferably a 5- to 10-membered heteroarylene ring, further preferably a 5-to 6-membered heteroarylene ring) and a C₆₋₁₄ arylene ring (preferably a phenylene ring or a naphthylene ring); the C₁₋₁₀ alkylene, C₁₋₁₀ alkyleneoxy, C₂₋₁₀ alkenylene, C₂₋₁₀ alkynylene, C₃₋₁₅ cycloalkylene ring, 3- to 15-membered heterocycloalkylene ring, 5- to 15-membered heteroarylene ring and C₆₋₁₄ arylene ring are unsubstituted or substituted with 1, 2, 3 or 4 R_{L2}, the each R_{L2} is independently selected from the group consisting of deuterium, halogen (preferably fluorine, chlorine or bromine), hydroxyl, cyano, amino, carboxyl, formyl, oxo, sulfo, C₁₋₆ alkyl, C₁₋₆ alkoxy, C₁₋₆ haloalkyl, C₁₋₆ haloalkoxy, hydroxy-substituted C₁₋₆ alkyl, cyano-substituted C₁₋₆ alkyl, amino-substituted C₁₋₆ alkyl, C₁₋₆ alkoxy C₁₋₆ alkyl, C₃₋₆ cycloalkyl, C₃₋₆ cycloalkyl C₁₋₆ alkyl, 3- to 6-membered heterocycloalkyl, 3- to 6-membered heterocycloalkyl C₁₋₆ alkyl, 5- to 6-membered heteroaryl, 5- to 6-membered heteroaryl C₁₋₆ alkyl, phenyl, phenyl C₁₋₆ alkyl, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -SO₂NH₂, -SO₂NHC₁₋₆ alkyl and -SO₂N(C₁₋₆ alkyl)₂;
each R_{L1} at each occurrence is independently selected from the group consisting of hydrogen, deuterium, C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy) and C₁₋₈ haloalkyl (preferably C₁₋₆ haloalkyl, more preferably C₁₋₃ haloalkyl).

In some embodiments, each R_{L1} at each occurrence is independently selected from the group consisting of hydrogen, methyl, ethyl, isopropyl, tert-butyl, methoxy, ethoxy, isopropoxy, tert-butoxy, trifluoromethyl, difluoromethyl, monofluoromethyl, trifluoroethyl, difluoroethyl, monofluoroethyl, trifluoromethoxy, difluoromethoxy, monofluoromethoxy, trifluoroethoxy, difluoroethoxy and monofluoroethoxy.

In some embodiments, each R_{L1} at each occurrence is independently selected from the group consisting of hydrogen, methyl, ethyl, difluoromethyl and monofluoromethyl.

In some embodiments, R_{L1} is hydrogen.

In some embodiments, each R_{L2} is independently selected from the group consisting of deuterium, halogen (preferably fluorine, chlorine or bromine), hydroxyl, cyano, amino, carboxyl, hydroxymethyl, hydroxyethyl, methyl, ethyl, difluoromethyl, monofluoromethyl, trifluoromethyl, monofluoroethyl, difluoroethyl, trifluoroethyl, cyclopropyl, cyclobutyl, cyclopentyl, cyclohexyl, cyclohexenyl, cyclopentenyl, tetrahydropyrrolyl, tetrahydrofuranyl, phenyl, pyrrolyl, furanyl, thienyl, thiazolyl, oxazolyl, pyrazolyl, imidazolyl, triazolyl, tetrazolyl, pyridinyl, pyrazinyl, pyrimidinyl, pyridazinyl, triazinyl, -CH₂-cyclopropyl, -CH₂-cyclobutyl, -CH₂-cyclopentyl, -CH₂-cyclohexyl, -CH₂-cyclohexenyl, -CH₂-cyclopentenyl, -CH₂-tetrahydropyrrolyl, -CH₂-tetrahydrofuranyl, -CH₂-phenyl, -CH₂-pyrrolyl, -CH₂-triazolyl, -CH₂-tetrazolyl, -CH₂-pyridinyl, -CH₂-pyrazinyl, -CH₂-triazinyl, methoxy, ethoxy, difluoromethoxy, monofluoromethoxy, trifluoromethoxy, acetyl, acetylamino and sulfonamido.

In some embodiments, each R_{L2} is independently selected from the group consisting of deuterium, -F, -Cl, -Br, -OH, -CN, -CHO, -COOH, -NH₂, -CH₂OH, -CH₂CH₂OH, -CH₃, -CH₂CH₃, -CH₂F, -CHF₂, -CF₃, -CH₂CH₂F, -CH₂CHF₂, -CH₂CF₃, -OCH₃, -OCH₂CH₃, -OCH₂F, -OCHF₂, -OCF₃, -OCH₂CH₂F, -OCH₂CHF₂, -OCH₂CF₃, -COCH₃, -CH₂-cyclopropyl, cyclopropyl, -CONH₂, -COOCH₃, -OCOCH₃, -CONHCH₃, -CON(CH₃)₂, -SOCH₃, -SO₂CH₃, -SO₂NH₂, -SO₂NHCH₃ and -SO₂N(CH₃)₂.

In some embodiments, each R_{L2} is independently selected from the group consisting of deuterium, -F, -Cl, -Br, -OH, -CN, -COOH, -NH₂, -CH₂OH, -CH₂CH₂OH, -CH₃, -CH₂CH₃, -CH₂F, -CHF₂, -CF₃, -CH₂CH₂F, -CH₂CHF₂, -CH₂CF₃, -OCH₃, -OCH₂CH₃, -OCH₂F, -OCHF₂, -OCF₃, -OCH₂CH₂F, -OCH₂CHF₂, -OCH₂CF₃, -COCH₃, -CH₂-cyclopropyl, cyclopropyl and -CONH₂.

In some embodiments, each R_{L2} is independently selected from the group consisting of fluorine, chlorine, bromine, hydroxy and hydroxymethyl.

In some embodiments, each Lₐ is independently selected from the group consisting of the following structures or isomers thereof: -C(O)-, -C(O)NH-, -O-, -S-, -NH-, C₁₋₁₀ alkylene, C₁₋₁₀ alkyleneoxy, a C₃₋₁₂ cycloalkylene ring, a 4- to 12-membered heterocycloalkylene ring, a 5- to 6-membered heteroarylene ring and a phenylene ring; the C₃₋₁₂ cycloalkylene ring, 4- to 12-membered heterocycloalkylene ring, 5- to 6-membered heteroarylene ring and phenylene ring are unsubstituted or substituted with 1, 2, 3 or 4 R_{L2}, and R_{L2} is selected from the group consisting of fluorine, hydroxy and hydroxymethyl.

In some embodiments, each Lₐ is independently selected from the group consisting of the following structures or isomers thereof: -O-, -S-, -NH-, -C(O)-, -C(O)NH-, -(CH₂)ₘ₂-, -O(CH₂)ₘ₂-, -(CH₂)ₘ₂O-, and a divalent form of a cyclic group selected from the group consisting of: a cyclopropane ring, a cyclobutane ring, a bicyclopentane ring, a cyclopentane ring, a cyclohexane ring, an azetidine ring, a tetrahydropyrrole ring, a piperidine ring, a piperazine ring, a hydroxy-substituted piperidine ring, a hydroxy-substituted piperazine ring, a hydroxymethyl-substituted piperidine ring, a hydroxymethyl-substituted piperazine ring, 3,3,5,5-tetramethylpiperidine, 3,3-difluoropiperidine, a 2-azaspiro[3.3]heptane ring, a 6-azaspiro[3.4]octane ring, a 7-azaspiro[3.5]nonane ring, a 2,6-diazaspiro[3.3]heptane ring, a 2,6-diazaspiro[3.4]octane ring, a 2,7-diazaspiro[3.5]nonane ring, a 2-azaspiro[3.5]nonane ring, a spiro[3.3]heptane ring, spiro[3.4]octane ring, a spiro[3.5]nonane ring, a 3,9-diazaspiro[5.5]undecane, a benzene ring, a pyridine ring, a pyrimidine ring, a pyrazine ring, a pyridazine ring, a triazine ring, a thiophene ring, a furan ring, a pyrrole ring, a thiazole ring, an oxazole ring, a pyrazole ring, an imidazole ring and a triazole ring; wherein each m2 at each occurrence is independently 1, 2, 3, 4, 5, 6, 7, 8, 9 or 10.

In some embodiments, each Lₐ is independently selected from the group consisting of the following structures or isomers thereof: -(CH₂)-, -(CH₂)₂-, -(CH₂)₅-, -C(O)-, -(CH₂)₃-, -C(O)NH-, -(CH₂)₈-, -O-, -NH-,

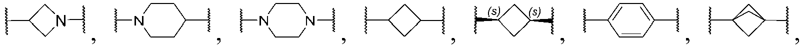

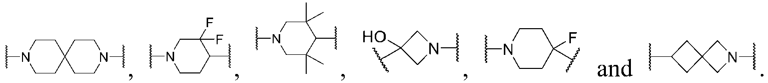

In some embodiments, each Lₐ is independently selected from the group consisting of the following structures or isomers thereof: -(CH₂)-,

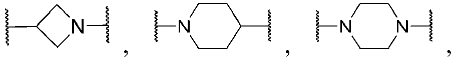

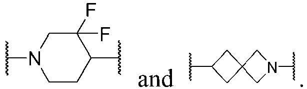

In some embodiments, L is selected from the group consisting of the following structures or isomers thereof: -(CH₂)ₘ₃-, -C(O)-(CH₂)ₘ₃-, -C(O)NH-(CH₂)ₘ₃-, -C(O)NH-(CH₂)ₘ₃-O-, -Cy₀-, -NH-Cy₀-, -Cy₀-NH-(CH₂)ₘ₃-, -Cy₀-(CH₂)ₘ₃-NH-, -Cy₀-(CH₂)ₘ₃-O-, -C(O)NH-Cy₀-, -(CH₂)ₘ₃-C(O)NH-(CH₂)ₘ₃-O-Cy₀-O-(CH₂)ₘ₃-, -Cy₀-Cy₀-, -(CH₂)ₘ₃-Cy₀-O-Cy₀-O-(CH₂)ₘ₃-, -Cy₀-C(O)NH-Cy₀-, -Cy₀-(CH₂)ₘ₃-Cy₀-, -Cy₀-O-Cy₀-, -Cy₀-C(O)-Cy₀-, -(CH₂)ₘ₃-Cy₀-Cy₀ and -C(O)NH-(CH₂)ₘ₃-Cy₀-(CH₂)ₘ₃-Cy₀; wherein each m3 at each occurrence is independently 0, 1, 2, 3, 4, 5, 6, 7, 8, 9 or 10; each Cy₀ at each occurrence is independently selected from the group consisting of a C₃₋₁₂ cycloalkyl ring (preferably a C₃₋₁₀ cycloalkyl ring, more preferably a C₃₋₈ cycloalkyl ring, further preferably a C₃₋₆ cycloalkyl ring), a 3- to 12-membered heterocycloalkyl ring (preferably a 3- to 10-membered heterocycloalkyl ring, more preferably a 3- to 8-membered heterocycloalkyl ring, further preferably a 3- to 6-membered heterocycloalkyl ring), a 5- to 6-membered heteroaryl ring and a benzene ring; the C₃₋₁₂ cycloalkyl ring, 3- to 12-membered heterocycloalkyl ring, 5- to 6-membered heteroaryl ring, benzene ring unsubstituted or substituted with 1, 2, 3 or 4 R_{L2}, each R_{L2} at each occurrence is independently selected from the group consisting of deuterium, halogen, hydroxyl, cyano, amino, carboxyl, C₁₋₃ alkyl, C₁₋₃ alkoxy, C₁₋₃ haloalkyl, C₁₋₃ haloalkoxy, C₁₋₃ hydroxy alkyl, cyano-substituted C₁₋₃ alkyl, amino-substituted C₁₋₃ alkyl, C₁₋₃ alkoxy C₁₋₃ alkyl, C₃₋₆ cycloalkyl, C₃₋₆ cycloalkyl C₁₋₃ alkyl, -COC₁₋₃ alkyl, -COOC₁₋₃ alkyl, -OCOC₁₋₃ alkyl, -CONH₂, -CONHC₁₋₃ alkyl and -CON(C₁₋₃ alkyl)₂.

In some embodiments, each Cy₀ is independently selected from the group consisting of a cyclopropane ring, a cyclobutane ring, a cyclopentane ring, a bicyclopentane ring, a cyclohexane ring, an azetidine ring, a hydroxy-substituted azetidine ring, a tetrahydropyrrole ring, a piperidine ring, a hydroxy-substituted piperidine ring, a hydroxymethyl-substituted piperidine ring, a 4-fluoropiperidine ring, 3,3-difluoropiperidine, 3,3,5,5-tetramethylpiperidine, a piperazine ring, a 2-azaspiro[3.3]heptane ring, a 6-azaspiro[3.4]octane ring, a 7-azaspiro[3.5]nonane ring, a 2,6-diazaspiro[3.3]heptane ring, a 2,6-diazaspiro[3.4]octane ring, a 2,7-diazaspiro[3.5]nonane ring, a 2-azaspiro[3.5]nonane ring, a spiro[3.3]heptane ring, a spiro[3.4]octane ring, a spiro[3.5]nonane ring, 3,9-diazaspiro[5.5]undecane, a benzene ring, a pyridine ring, a pyrimidine ring, a pyrazine ring, a pyridazine ring, a triazine ring, a thiophene ring, a furan ring, a pyrrole ring, a thiazole ring, an oxazole ring, a pyrazole ring, an imidazole ring and a triazole ring.

In some embodiments, each Cy₀ is independently selected from the group consisting of a cyclobutane ring, a bicyclopentane ring, a benzene ring, an azetidine ring, a piperidine ring, a piperazine ring, and 3,9-diazaspiro[5.5]undecane.

In some embodiments, each Cy₀ is independently selected from the group consisting of a cyclobutane ring, an azetidine ring, a benzene ring, and a piperidine ring.

In some embodiments, each Cy₀ is independently selected from the group consisting of the following structures or isomers thereof:

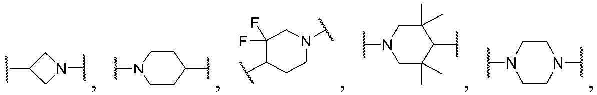

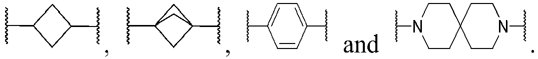

In some embodiments, each Cy₀ is independently selected from the group consisting of the following structures or isomers thereof:

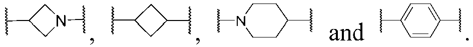

In some embodiments, L is selected from the group consisting of the following structures or isomers thereof: 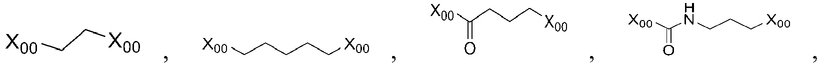 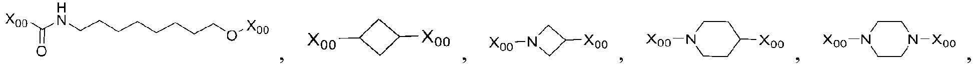 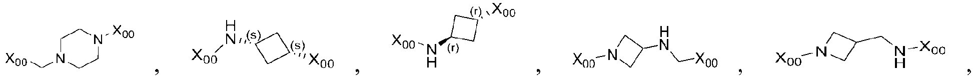 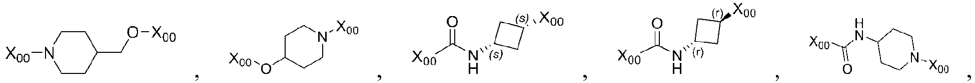 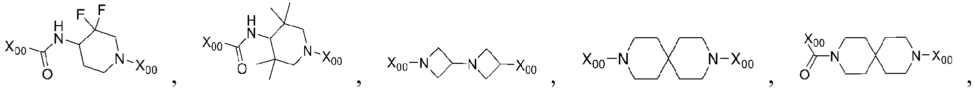 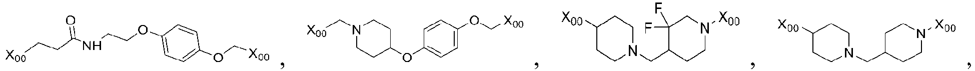 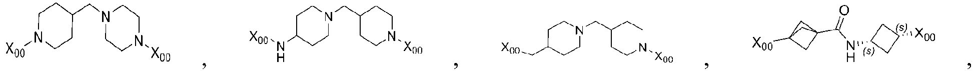 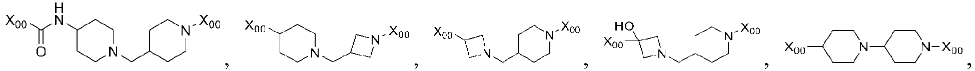 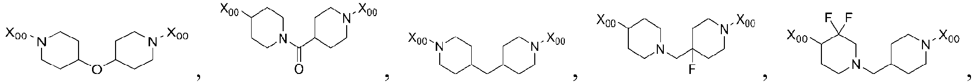 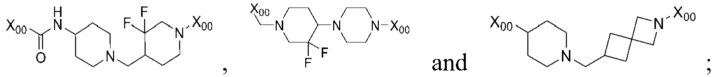 wherein X₀₀ is a point of attachment of L to ULM or POI.

In some embodiments, L is selected from the group consisting of the following structures or isomers thereof: 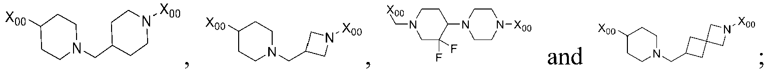 wherein X₀₀ is the point of attachment of L to ULM or POI.

In some embodiments, L is selected from the group consisting of the following structures or isomers thereof: 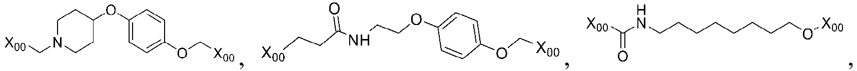 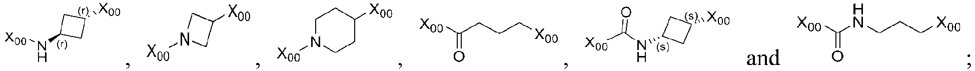 wherein X₀₀ is the point of attachment of L to ULM or POI.

In some embodiments, L is selected from the group consisting of the following structures or isomers thereof: 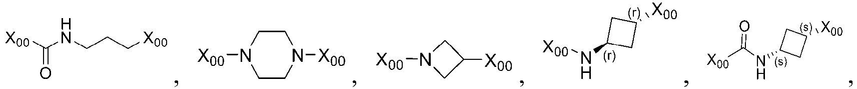 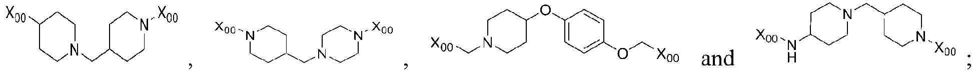 wherein X₀₀ is the point of attachment of L to ULM or POI.

In some embodiments, L is selected from the group consisting of the following structures or isomers thereof: 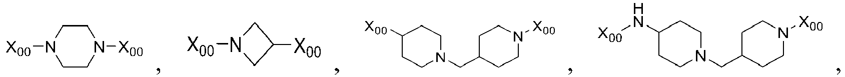 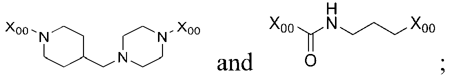 wherein X₀₀ is the point of attachment of L to ULM or POI.

In some embodiments, L is selected from the group consisting of the following structures or isomers thereof: 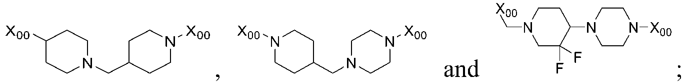 wherein X₀₀ is the point of attachment of L to ULM or POI.

In some embodiments, L is selected from the group consisting of the following structures or isomers thereof: 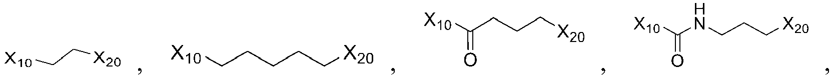 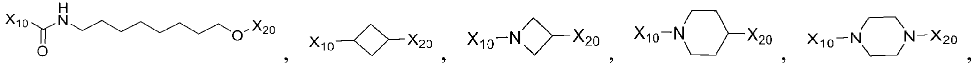 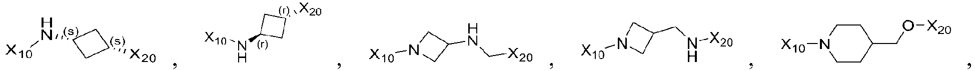 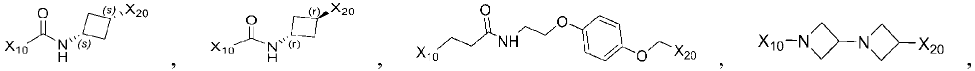 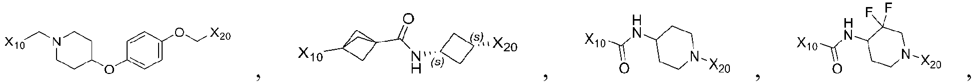 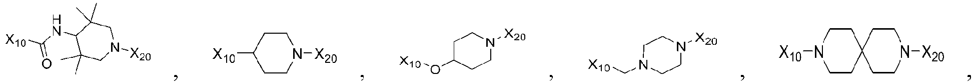 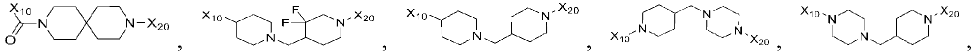 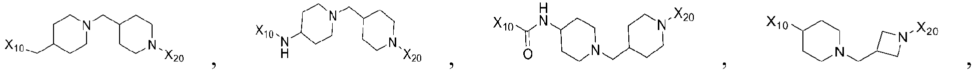 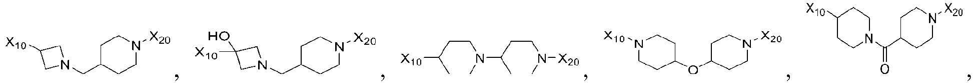 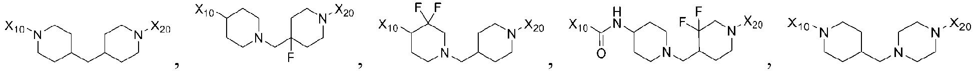 , 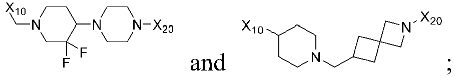 wherein X₁₀ is a point of attachment of L to POI, and X₂₀ is a point of attachment of L to ULM.

In some embodiments, L is selected from the group consisting of the following structures or isomers thereof: 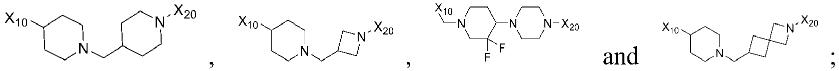 wherein X₁₀ is the point of attachment of L to POI, and X₂₀ is the point of attachment of L to ULM.

In some embodiments, L is selected from the group consisting of the following structures or isomers thereof: 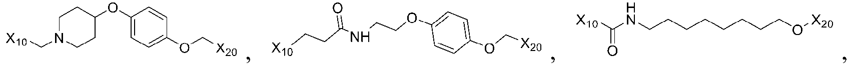 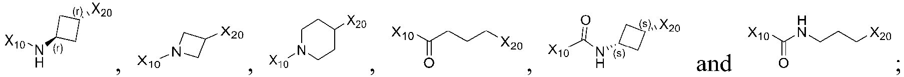 wherein X₁₀ is the point of attachment of L to POI, and X₂₀ is the point of attachment of L to ULM.

In some embodiments, L is selected from the group consisting of the following structures or isomers thereof: 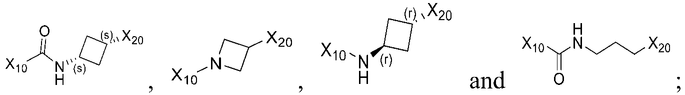 wherein X₁₀ is the point of attachment of L to POI, and X₂₀ is the point of attachment of L to ULM.

In some embodiments, L is selected from the group consisting of the following structures or isomers thereof: 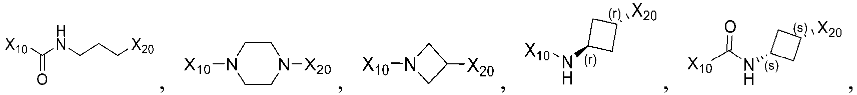 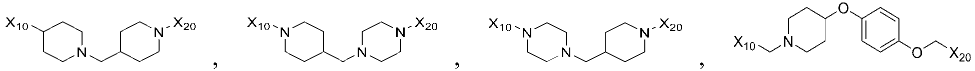 and 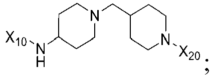 wherein X₁₀ is the point of attachment of L to POI, and X₂₀ is the point of attachment of L to ULM.

In some embodiments, L is selected from the group consisting of the following structures or isomers thereof: 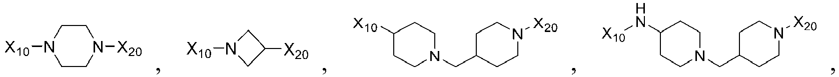 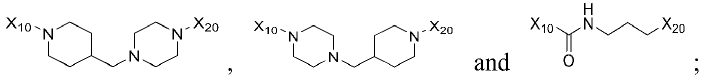 wherein X₁₀ is the point of attachment of L to POI, and X₂₀ is the point of attachment of L to ULM.

In some embodiments, L is selected from the group consisting of the following structures or isomers thereof: 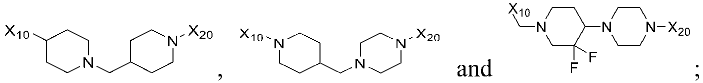 wherein X₁₀ is the point of attachment of L to POI, and X₂₀ is the point of attachment of L to ULM.

In some embodiments, ULM is a compound of formula (U-1) or an isomer thereof: 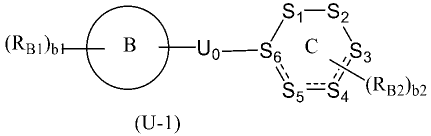 wherein,
-̅-̅-̅-̅-̅-̅ represents 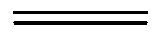 (double bond) or 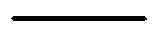 (single bond);
U₀ is a chemical bond, -N(R_{U0})-, -CON(R_{U0})-, -CH₂- or -(CH₂)₂-;
each R_{U0} at each occurrence is independently hydrogen or C₁₋₃ alkyl;
ring B is absent or is selected from the group consisting of a 5- to 15-membered heteroaryl ring (preferably a 6- to 12-membered heteroaryl ring, more preferably a 6- to 10-membered heteroaryl ring), a 3- to 15-membered heterocycloalkyl ring (preferably a 5- to 12-membered heterocycloalkyl ring, more preferably a 5- to 10-membered heterocycloalkyl ring), a C₃₋₁₅ cycloalkyl ring and a C₆₋₁₀ aryl ring (preferably a benzene ring);
S₁, S₃ and S₅ are each independently selected from the group consisting of a chemical bond, -O-, -NH-, -N-, -CH₂-, -CH-, -C(O)-, -C(O)O-, -C(O)S-, -CH₂C(O)-, -CH₂C(S)-, -C(S)-, -CONH-, -CH=N-, -N=N-, -CH=CH-, -SO- and -SO₂-;
S₂ and S₄ are each independently selected from the group consisting of -N-, -NH-, -CH- and -CH₂-;
S₆ is selected from the group consisting of C, -CH- and N;
(R_{B1})_{b1} represents substitution of hydrogen on ring B by b1 R_{B1}, b1 is 0, 1, 2 or 3, each R_{B1} is identical or different, and each R_{B1} is independently selected from the group consisting of X₂, deuterium, halogen (preferably fluorine, chlorine or bromine), cyano, carboxyl, hydroxyl, nitro, -NRₐ₁R_{b1}, C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), -SC₁₋₈ alkyl (preferably -SC₁₋₆ alkyl, more preferably -SC₁₋₃ alkyl), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), C₁₋₈ haloalkyl (preferably C₁₋₆ haloalkyl, more preferably C₁₋₃ haloalkyl), C₁₋₈ haloalkoxy (preferably C₁₋₆ haloalkoxy, more preferably C₁₋₃ haloalkoxy), amino-substituted C₁₋₈ alkyl (preferably amino-substituted C₁₋₆ alkyl, more preferably amino-substituted C₁₋₃ alkyl), cyano-substituted C₁₋₈ alkyl (preferably cyano-substituted C₁₋₆ alkyl, more preferably cyano-substituted C₁₋₃ alkyl), hydroxy-substituted C₁₋₈ alkyl (preferably hydroxy-substituted C₁₋₆ alkyl, more preferably hydroxy-substituted C₁₋₃ alkyl), carboxy-substituted C₁₋₈ alkyl (preferably carboxy-substituted C₁₋₆ alkyl, more preferably carboxy-substituted C₁₋₃ alkyl), -COC₁₋₈ alkyl (preferably -COC₁₋₆ alkyl, more preferably -COC₁₋₃ alkyl), -COOC₁₋₈ alkyl-CONRₐ₂R_{b2} (preferably -COOC₁₋₆ alkyl-CONRₐ₂R_{b2}, more preferably -COOC₁₋₃ alkyl-CONRₐ₂R_{b2}), -SO₂NRₐ₂R_{b2}, a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring), a 3- to 15-membered heterocycloalkyl ring (preferably a 4- to 12-membered heterocycloalkyl ring, more preferably a 4- to 10-membered heterocycloalkyl ring, further preferably a 4- to 8-membered heterocycloalkyl ring, further preferably a 4- to 6-membered heterocycloalkyl ring), a 5- to 10-membered heteroaryl ring (preferably a 5- to 6-membered heteroaryl ring) and a C₆₋₁₀ aryl ring (preferably a benzene ring or a naphthalene ring); or two adjacent R_{B1} and carbon atom linked to the two adjacent R_{B1} together form a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring), a 3-to 15-membered heterocycloalkyl ring (preferably a 4- to 12-membered heterocycloalkyl ring, more preferably a 4- to 10-membered heterocycloalkyl ring, further preferably a 4- to 8-membered heterocycloalkyl ring, further preferably a 4- to 6-membered heterocycloalkyl ring), a 5- to 6-membered heteroaryl ring or a benzene ring; the C₃₋₁₅ cycloalkyl ring, 3- to 15-membered heterocycloalkyl ring, 5- to 6-membered heteroaryl ring or benzene ring is unsubstituted or substituted with 1, 2, 3 or 4 substituents selected from the group consisting of: X₂, deuterium, halogen (preferably fluorine, chlorine or bromine), cyano, carboxyl, hydroxyl, nitro, -NRₐ₁R_{b1}, C₁₋₆ alkyl, C₁₋₆ alkoxy, -SC₁₋₆ alkyl, -SOC₁₋₆ alkyl, -SO₂C₁₋₆ alkyl, C₁₋₆ haloalkyl, C₁₋₆ haloalkoxy, -COC₁₋₆ alkyl, -COOC₁₋₆ alkyl, -CONRₐ₂R_{b2} and -SO₂NRₐ₂R_{b2};
(R_{B2})_{b2} represents substitution of hydrogen on ring C by b2 R_{B2}, b2 is 0, 1, 2, 3 or 4, each R_{B2} is identical or different, and each R_{B2} is independently selected from the group consisting of X₂, deuterium, halogen (preferably fluorine, chlorine or bromine), cyano, carboxyl, hydroxyl, -NRₐ₁R_{b1}, C₁₋₆ alkyl, C₁₋₆ alkoxy, C₁₋₆ haloalkyl, C₁₋₆ haloalkoxy, -COC₁₋₆ alkyl, -COOC₁₋₆ alkyl, -OCOC₁₋₆ alkyl, -CONRₐ₂R_{b2}, -OC(O)C₁₋₆ alkyl-substituted C₁₋₆ alkyl and -COOC₁₋₆ alkyl-substituted C₁₋₆ alkyl;
Rₐ₁, R_{b1}, Rₐ₂ and R_{b2} are each independently selected from the group consisting of hydrogen, C₁₋₆ alkyl, C₁₋₆ haloalkyl, hydroxy-substituted C₁₋₆ alkyl, cyano-substituted C₁₋₆ alkyl, carboxy-substituted C₁₋₆ alkyl, amino-substituted C₁₋₆ alkyl, -COC₁₋₆ alkyl and -COOC₁₋₆ alkyl;
X₂ in R_{B1} and R_{B2} is a point of attachment of ULM to L or POI, and at least one of R_{B1} and R_{B2} is X₂.

In some embodiments, R_{B1} and R_{B2} are not both X₂.

In some embodiments, ring B is absent.

In some embodiments, the 5- to 15-membered heteroaryl ring in ring B is selected from the group consisting of a pyrrole ring, a furan ring, a thiophene ring, a pyrazole ring, an imidazole ring, a triazole ring, a tetrazole ring, an oxazole ring, a thiazole ring, an oxadiazole ring, a thiadiazole ring, a pyridine ring, a pyridazine ring, a pyrimidine ring, a pyrazine ring, a triazine ring, a benzopyrrole ring, a benzofuran ring, a benzothiophene ring, a benzopyrazole ring, a benzimidazole ring, a benzothiazole ring, a benzoxazole ring, a pyridopyrrole ring, a pyridofuran ring, a pyridothiophene ring, a pyridopyrazole ring, a pyridoimidazole ring, a pyridothiazole ring, a pyridooxazole ring, a pyrimidopyrrole ring, a pyridazopyrrole ring, a pyrazinopyrrole ring, a pyrimidopyrazole ring, a pyridazopyrazole ring, a pyrazinopyrazole ring, a pyrimidoimidazole ring, a pyridazoimidazole ring, a pyrazinoimidazole ring, a quinoline ring, an isoquinoline ring and a 9H-pyrido[2,3-b]indole ring.

In some embodiments, the 5- to 15-membered heteroaryl ring in ring B is a benzopyrazole ring.

In some embodiments, the 3- to 15-membered heterocycloalkyl ring in ring B is 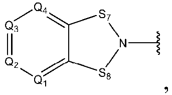
wherein Q₁, Q₂, Q₃ and Q₄ are each independently selected from the group consisting of -CH-, N and N-O;
S₇ and S₈ are each independently selected from the group consisting of a chemical bond, -O-, -NH-, -CH₂-, -C(O)-, -C(O)O-, -C(O)S-, -CH₂C(O)-, -CH₂C(S)-, -C(S)-, -CONH-, -CH=N-, -N=N-, -CH=CH-, -SO- and -SO₂-;
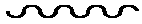 represents a covalent connection to U₀.

In some embodiments, Q₁, Q₂, Q₃ and Q₄ are each independently -CH-.

In some embodiments, S₇ and S₈ are each independently selected from the group consisting of -CH₂- and -C(O)-.

In some embodiments, S₇ is -CH₂- and S₈ is -C(O)-.

In some embodiments, S₇ is -C(O)- and S₈ is -C(O)-.

In some embodiments, 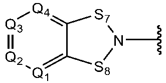 is selected from the group consisting of the following structures or isomers thereof: 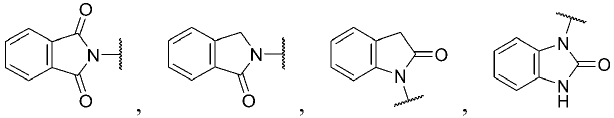 and 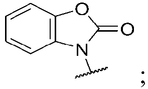 wherein 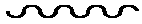 represents a covalent connection to U₀.

In some embodiments, 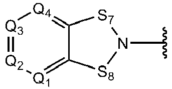 is selected from the group consisting of the following structures or isomers thereof: 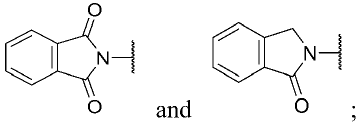 wherein 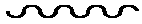 represents a covalent connection to U₀.

In some embodiments, the 3- to 15-membered heterocycloalkyl ring in ring B is 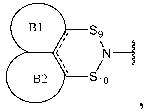
wherein ring B1 and ring B are each independently selected from the group consisting of a C₄₋₈ cycloalkyl ring, a 4- to 8-membered heterocycloalkyl ring, a 5- to 6-membered heteroaryl ring and a benzene ring;
S₉ and S₁₀ are each independently selected from the group consisting of a bond, -CH₂- and -C(O)-;
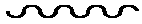 represents a covalent connection to U₀.

In some embodiments, 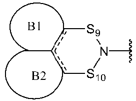 is selected from the group consisting of 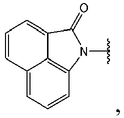 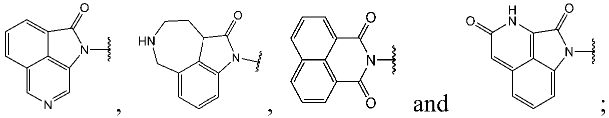 wherein 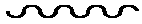 represents a covalent connection to U₀.

In some embodiments, the 3- to 15-membered heterocycloalkyl ring in ring B is 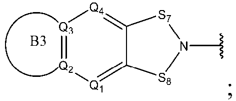 wherein ring B3 is a 3- to 7-membered heterocycloalkyl, 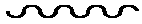 represents a covalent connection to U₀.

In some embodiments, ring B3 is a 5- to 7-membered nitrogen-containing heterocycloalkyl ring.

In some embodiments, ring B3 is a partially unsaturated 5- to 7-membered nitrogen-containing heterocycloalkyl ring.

In some embodiments, ring B3 is selected from the group consisting of 2,5-dihydro-1H-pyrrole, 2,3-dihydro-1H-pyrrole, 1,2,3,6-tetrahydropyridine, 1,2,3,4-tetrahydropyridine, 2,3,6,7-tetrahydro-1H-azepine, 2,3,4,7-tetrahydro-1H-azepine and 2,3,4,5-tetrahydro-1H-azepine.

In some embodiments, 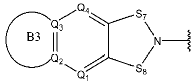 is selected from the group consisting of 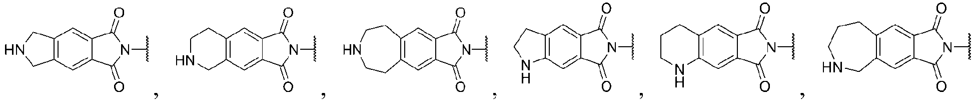 and 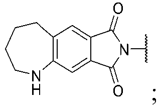 wherein 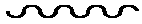 represents a covalent connection to U₀.

In some embodiments, 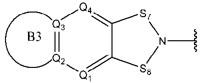 is 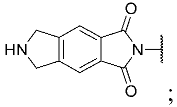 wherein 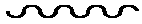 represents a covalent connection to U₀.

In some embodiments, ring B is selected from the group consisting of: a benzene ring, 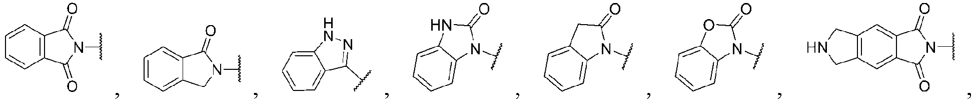 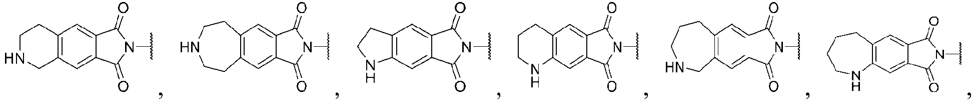 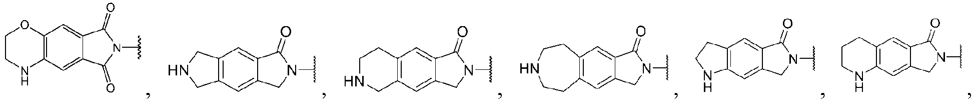 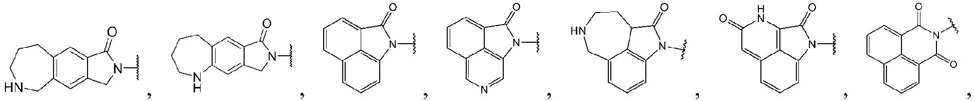 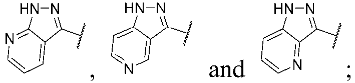 wherein 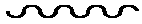 represents a covalent connection to U₀.

In some embodiments, ring B is selected from the group consisting of: a benzene ring, 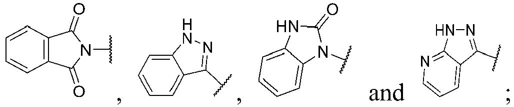 wherein 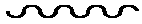 represents a covalent connection to U₀.

In some embodiments, ring B is selected from the group consisting of: 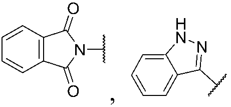 and 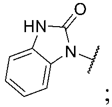 wherein 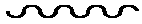 represents a covalent connection to U₀.

In some embodiments, each R_{B1} is independently X₂, deuterium, fluorine, chlorine, bromine, cyano, carboxyl, hydroxyl, nitro, -NH₂, -N(CH₃)₂, -NHCH₃, -NHCOCH₃, methyl, ethyl, propyl, isopropyl, methoxy, ethoxy, isopropoxy, trifluoromethyl, difluoromethyl, monofluoromethyl, trifluoromethoxy, difluoromethoxy, monofluoromethoxy, -SCH₃, -SOCH₃, -SO₂CH₃, -CH₂NH₂, -(CH₂)₂NH₂, -(CH₂)₃NH₂, -CH₂CN, -(CH₂)₂CN, -(CH₂)₃CN, -CH₂OH, -(CH₂)₂OH, -(CH₂)₃OH, -CH₂COOH, -(CH₂)₂COOH, -(CH₂)₃COOH, -COCH₃, -COCH₂CH₃, -COOCH₃, -COOCH₂CH₃, -CONH₂ or -SO₂NH₂; or two adjacent R_{B1} and carbon atom linked to the two adjacent R_{B1} together form a cyclobutyl ring, a cyclopentyl ring, a cyclohexyl ring, a cycloheptyl ring, a cyclopentene ring, a cyclohexene ring, a cycloheptene ring, a tetrahydropyrrole ring, a tetrahydrofuran ring, a tetrahydrothiophene ring, a piperidine ring, a pyrazine ring, a 1,2,3,4-tetrahydropyridine ring, a 1,2,3,4-tetrahydropyran ring, a 3,4-dihydro-2H-1,4-oxazine ring, a 2,3,4,5-tetrahydro-1H-azepine ring, a pyrrole ring, a pyrazole ring, an oxazole ring, a thiazole ring, a pyran ring, a pyridine ring, a pyridazine ring, a pyrimidine ring or a benzene ring; wherein the cyclobutyl ring, cyclopentyl ring, cyclohexyl ring, cycloheptyl ring, cyclopentene ring, cyclohexene ring, cycloheptene ring, tetrahydropyrrole ring, tetrahydrofuran ring, tetrahydrothiophene ring, piperidine ring, pyrazine ring, 1,2,3,4-tetrahydropyridine ring, 1,2,3,4-tetrahydropyran ring, 3,4-dihydro-2H-1,4-oxazine ring, 2,3,4,5-tetrahydro-1H-azepine ring, pyrrole ring, pyrazole ring, oxazole ring, thiazole ring, pyran ring, pyridine ring, pyridazine ring, pyrimidine ring or benzene ring is unsubstituted or substituted with 1, 2, 3 or 4 substituents selected from the group consisting of: X₂, deuterium, fluorine, chlorine, bromine, cyano, carboxyl, hydroxyl, nitro, amino, methyl, trifluoromethyl, methoxy, trifluoromethoxy, -SCH₃, -SOCH₃, -SO₂CH₃, -COCH₃, -COOCH₃, -CONH₂ and -SO₂NH₂.

In some embodiments, R_{B1} is selected from the group consisting of X₂, fluorine, chlorine, hydroxyl, methyl, trifluoromethyl and methoxy.

In some embodiments, b1 is 1, and R_{B1} is fluorine.

In some embodiments, b1 is 1, and R_{B1} is methyl.

In some embodiments, 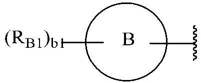 is selected from the group consisting of: 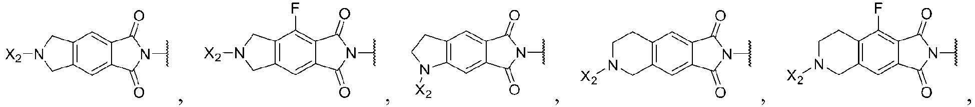 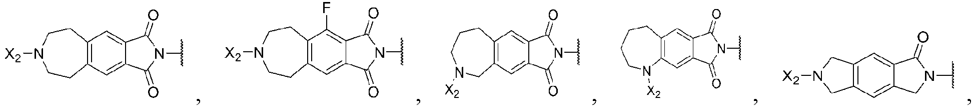 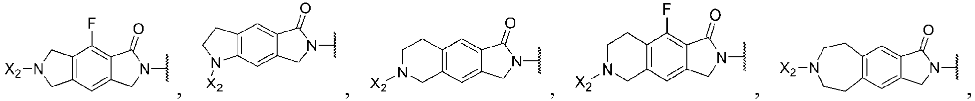 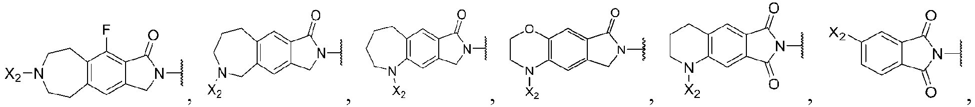 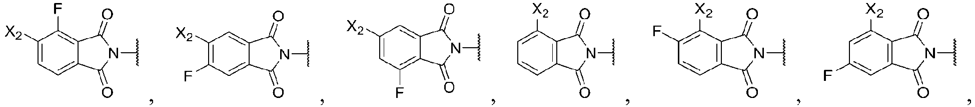 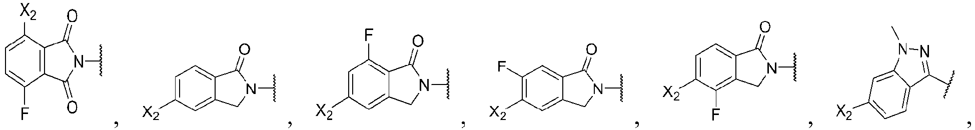 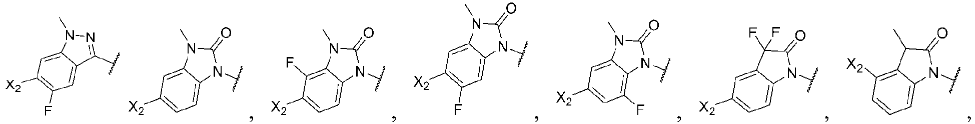 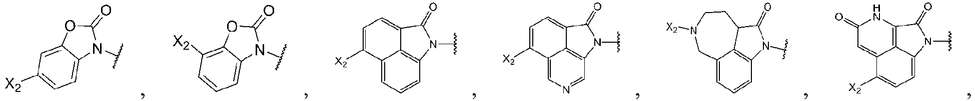 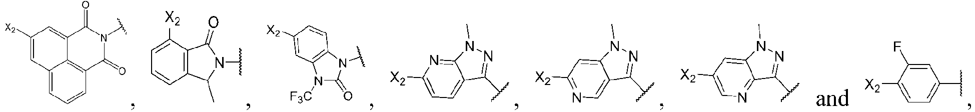 wherein X₂ is the point of attachment of ULM to L or POI, 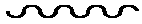 represents a covalent connection to U₀.

In some embodiments, 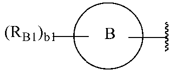 is selected from the group consisting of: 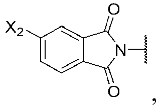 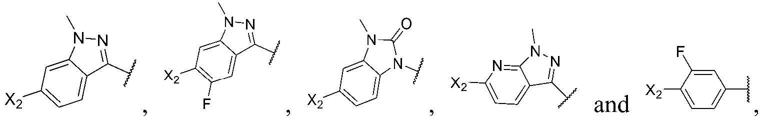 wherein X₂ is the point of attachment of ULM to L or POI, 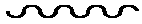 represents a covalent connection to U₀.

In some embodiments, 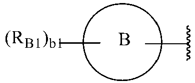 is selected from the group consisting of: 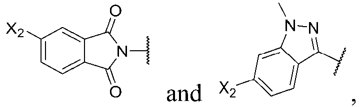 wherein X₂ is the point of attachment of ULM to L or POI, 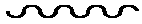 represents a covalent connection to U₀.

In some embodiments, U₀ is a chemical bond.

In some embodiments, U₀ is -NH-.

In some embodiments, U₀ is -CONH-.

In some embodiments, U₀ is -CH₂-.

In some embodiments, S₁ and S₃ are -C(O)-, S₂ is -NH-, and S₄ and S₃ are -CH₂-.

In some embodiments, S₆ is -CH-.

In some embodiments, S₆ is N.

In some embodiments, b2 is 0.

In some embodiments, 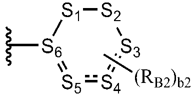 is selected from the group consisting of the following structure or an isomer thereof: 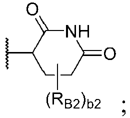 wherein 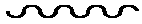 represents a covalent connection to U₀.

In some embodiments, 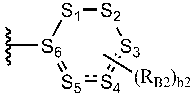 is selected from the group consisting of the following structures or isomers thereof: 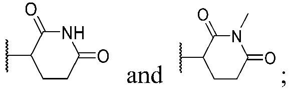 wherein 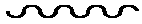 represents a covalent connection to U₀.

In some embodiments, ULM is selected from the group consisting of the following structures or isomers thereof: 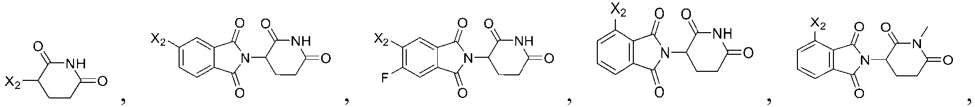 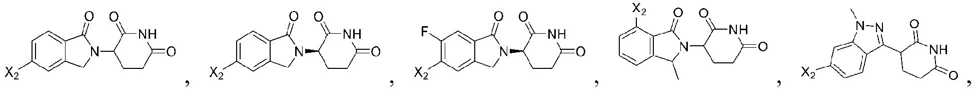 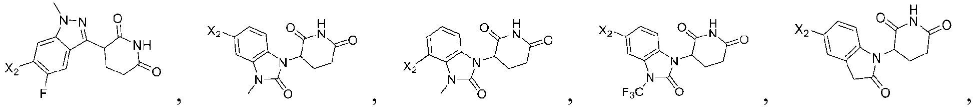 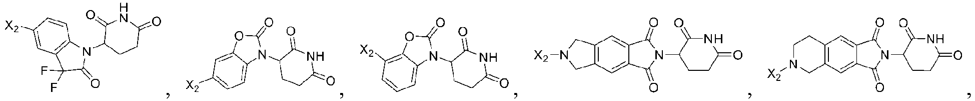 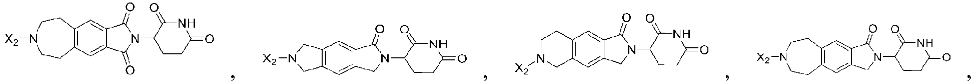 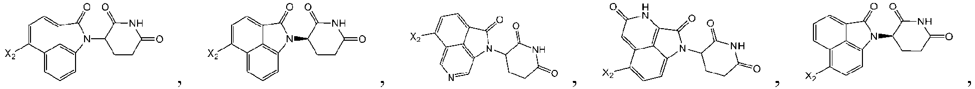 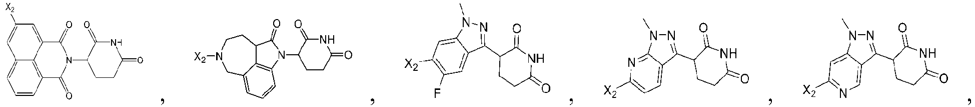 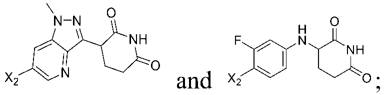 wherein X₂ is the point of attachment of ULM to L or POI.

In some embodiments, ULM is selected from the group consisting of the following structures or isomers thereof: 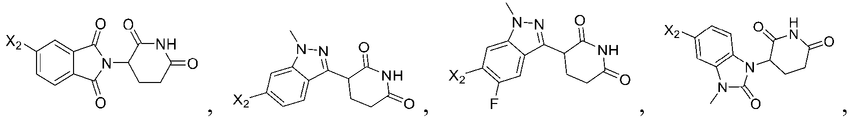 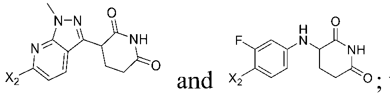 wherein X₂ is the point of attachment of ULM to L or POI.

In some embodiments, ULM is selected from the group consisting of the following structure or an isomer thereof: 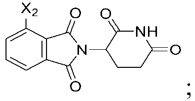 wherein X₂ is the point of attachment of ULM to L or POI.

In some embodiments, ULM is selected from the group consisting of the following structures or isomers thereof: 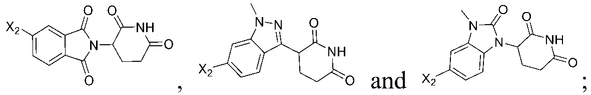 wherein X₂ is the point of attachment of ULM to L or POI.

In some embodiments, ULM is a structure of formula (U-2) or an isomer thereof: 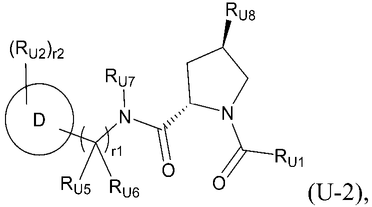 wherein,
r1 is 0, 1 or 2;
ring D is selected from the group consisting of a benzene ring, a 5- to 6-membered heteroaryl ring, a C₃₋₁₀ cycloalkyl ring (preferably a C₃₋₈ cycloalkyl ring, more preferably a C₃₋₆ cycloalkyl ring) and a 3- to 10-membered heterocycloalkyl ring (preferably a 3- to 8-membered heterocycloalkyl ring, more preferably a 3- to 6-membered heterocycloalkyl ring);
(R_{U2})ᵣ₂ represents substitution of hydrogen on ring D by r2 R_{U2}, r2 is 0, 1, 2 or 3, each R_{U2} is identical or different, and each R_{U2} is independently selected from the group consisting of X₂, hydrogen, deuterium, halogen (preferably fluorine, chlorine), nitro, cyano, carboxyl, hydroxyl, C₁₋₆ alkyl, C₁₋₆ alkoxy, C₁₋₆ alkoxy C₁₋₆ alkyl, hydroxy-substituted C₁₋₆ alkyl, C₁₋₆ haloalkyl, C₁₋₆ haloalkyl, -NRₐ₃R_{b3}, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -SC₁₋₆ alkyl, 5- to 6-membered heteroaryl and phenyl; the 5- to 6-membered heteroaryl and phenyl are unsubstituted or substituted with 1, 2 or 3 substituents selected from the group consisting of: hydrogen, deuterium, halogen, cyano, carboxyl, hydroxyl, C₁₋₆ alkyl (preferably C₁₋₃ alkyl), hydroxy-substituted C₁₋₆ alkyl (preferably hydroxy-substituted C₁₋₃ alkyl), C₁₋₆ alkoxy C₁₋₆ alkyl (preferably C₁₋₃ alkoxy C₁₋₃ alkyl), C₁₋₆ alkoxy (preferably C₁₋₃ alkoxy), C₁₋₆ haloalkyl (preferably C₁₋₃ haloalkyl), C₁₋₆ haloalkoxy (preferably C₁₋₃ haloalkoxy), -NH₂, -NHCOC₁₋₆ alkyl (preferably -NHCOC₁₋₃ alkyl), -COC₁₋₆ alkyl (preferably -COC₁₋₃ alkyl), -COOC₁₋₆ alkyl (preferably -COOC₁₋₃ alkyl), -OCOC₁₋₆ alkyl (preferably -OCOC₁₋₃ alkyl), -CONH₂, -NHCONH₂, -CONHC₁₋₆ alkyl (preferably -CONHC₁₋₃ alkyl), -NHCONHC₁₋₆ alkyl (preferably -NHCONHC₁₋₃ alkyl), -SOC₁₋₆ alkyl (preferably -SOC₁₋₃ alkyl), -SO₂C₁₋₆ alkyl (preferably -SO₂C₁₋₃ alkyl) and -SC₁₋₆ alkyl (preferably -SC₁₋₃ alkyl);
R_{U1} is -C(R_{U3}R_{U4})-U₁;
U₁ is selected from the group consisting of the following structures or isomers thereof: X₂, -NHCO-X₂, -NHCOCH₃, 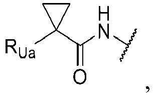 a 5- to 6-membered heteroaryl ring, 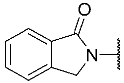 and 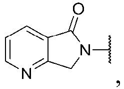 the 5- to 6-membered heteroaryl ring, 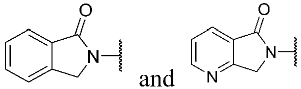 are unsubstituted or substituted with 1, 2 or 3 substituents selected from the group consisting of: X₂, halogen, hydroxyl, cyano, amino, carboxyl, C₁₋₆ alkyl (preferably methyl, ethyl, isopropyl), C₁₋₆ alkoxy (preferably methoxy, ethoxy, isopropoxy), C₁₋₆ haloalkyl (preferably trifluoromethyl), C₁₋₆ haloalkoxy (preferably trifluoromethoxy), -COC₁₋₆ alkyl (preferably -COCH₃), -COOC₁₋₆ alkyl (preferably -COOCH₃), -CONH₂, -CONHC₁₋₆ alkyl (preferably -CONHCH₃), -CON(C₁₋₆ alkyl)₂ (preferably -CON(CH₃)₂) and hydroxy-substituted C₁₋₆ alkyl (preferably -CH₂OH);
R_{Ua} is selected from the group consisting of hydrogen, halogen (preferably fluorine, chlorine or bromine), cyano, hydroxyl, carboxyl, amino, C₁₋₆ alkyl, C₁₋₆ alkoxy, C₁₋₆ haloalkyl, C₁₋₆ haloalkoxy, -COC₁₋₆ alkyl, -NHCOC₁₋₆ alkyl, -N(C₁₋₆ alkyl)COC₁₋₆ alkyl, -NHC₁₋₆ alkyl and -N(C₁₋₆ alkyl)₂;
R_{U3} and R_{U4} are each independently selected from the group consisting of hydrogen, deuterium, halogen, cyano, carboxyl, hydroxyl, C₁₋₆ alkyl (preferably C₁₋₃ alkyl), C₁₋₆ alkoxy (preferably C₁₋₃ alkoxy), C₁₋₆ haloalkyl (preferably C₁₋₃ alkoxy), C₁₋₆ haloalkoxy (preferably C₁₋₃ haloalkoxy) and -SC₁₋₆ alkyl (preferably -SC₁₋₃ alkyl); or R_{U3}, R_{U4} and carbon atom linked to R_{U3} and R_{U4} together form a C₃₋₇ cycloalkyl (preferably C₃₋₆ cycloalkyl) and a 3- to 7-membered heterocycloalkyl (preferably a 4-to 6-membered heterocycloalkyl); wherein the C₁₋₆ alkyl, C₁₋₆ alkoxy, C₁₋₆ haloalkyl, C₁₋₆ haloalkoxy, -SC₁₋₆ alkyl, C₃₋₇ cycloalkyl and 3- to 7-membered heterocycloalkyl are unsubstituted or substituted with 1, 2 or 3 substituents selected from the group consisting of: X₂, halogen (preferably fluorine, chlorine or bromine), cyano, carboxyl and hydroxy;
R_{U5} and R_{U6} are each independently selected from the group consisting of X₂, hydrogen, deuterium, halogen, amino, cyano, carboxyl, hydroxyl, C₁₋₆ alkyl (preferably C₁₋₃ alkyl), C₁₋₆ alkoxy (preferably C₁₋₃ alkoxy), C₁₋₆ haloalkyl (preferably C₁₋₃ haloalkyl), C₁₋₆ haloalkoxy (C₁₋₃ haloalkoxy), -SC₁₋₆ alkyl (preferably -SC₁₋₃ alkyl), CONHC₁₋₆ alkyl-substituted C₁₋₆ alkyl, CON(C₁₋₆ alkyl)₂-substituted C₁₋₆ alkyl, carboxyl-substituted C₁₋₆ alkyl and COOC₁₋₆ alkyl-substituted C₁₋₆ alkyl;
R_{U7} is selected from the group consisting of hydrogen, C₁₋₆ alkyl, and C₁₋₆ haloalkyl; or R_{U7}, nitrogen atom linked to R_{U7}, R_{U6} and carbon atom linked to R_{U6} together form a 3- to 8-membered heterocycloalkyl ring or a 5- to 6-membered heteroaryl ring;
R_{U8} is selected from the group consisting of hydroxyl, amino, -NH(C₁₋₆ alkyl), -N(C₁₋₆ alkyl)₂, C₁₋₆ alkoxy, C₁₋₆ haloalkoxy and -OCOC₁₋₆ alkyl;
Rₐ₃ and R_{b3} are each independently selected from the group consisting of hydrogen, C₁₋₆ alkyl (preferably C₁₋₃ alkyl), C₁₋₆ alkoxy (preferably C₁₋₃ alkoxy), -SC₁₋₆ alkyl (preferably -SC₁₋₃ alkyl), C₁₋₆ haloalkyl (preferably C₁₋₃ haloalkyl), C₁₋₆ haloalkoxy (preferably C₁₋₃ haloalkoxy), -COC₁₋₆ alkyl (preferably -COC₁₋₃ alkyl), -CONH₂, -CONHC₁₋₆ alkyl (preferably -CONHC₁₋₃ alkyl), -CON(C₁₋₆ alkyl)₂ (preferably -CON(C₁₋₃ alkyl)₂), 5- to 6-membered heteroaryl and phenyl; wherein the 5- to 6-membered heteroaryl and phenyl are each independently unsubstituted or substituted with 1, 2 or 3 substituents selected from the group consisting of: hydrogen, C₁₋₆ alkyl (preferably C₁₋₃ alkyl), C₁₋₆ alkoxy (preferably C₁₋₃ alkoxy), -SC₁₋₆ alkyl (preferably -SC₁₋₃ alkyl), C₁₋₆ haloalkyl (preferably C₁₋₃ haloalkyl), C₁₋₆ haloalkoxy (preferably C₁₋₃ haloalkoxy), -COC₁₋₆ alkyl (preferably -COC₁₋₃ alkyl), -CONH₂, -CONHC₁₋₆ alkyl (preferably -CONHC₁₋₃ alkyl) and -CON(C₁₋₆ alkyl)₂ (preferably -CON(C₁₋₃ alkyl)₂);
X₂ in R_{U2}, R_{U1}, R_{U5} and R_{U6} is the point of attachment of ULM to L or POI, and at least one of R_{U1}, R_{U5}, R_{U6} and R_{U2} is X₂.

In some embodiments, r1 is 0.

In some embodiments, r1 is 1.

In some embodiments, r1 is 2.

In some embodiments, ring D is selected from the group consisting of a benzene ring, a 5- to 6-membered heteroaryl ring, a C₅₋₉ cycloalkyl ring and a 5- to 9-membered heterocycloalkyl ring.

In some embodiments, ring D is selected from the group consisting of a benzene ring and 2,3-dihydro-1H-indene.

In some embodiments, ring D is a benzene ring.

In some embodiments, ring D is 2,3-dihydro-1H-indene.

In some embodiments, ring D is selected from the group consisting of a benzene ring and a 5- to 6-membered heteroaryl ring.

In some embodiments, ring D is selected from the group consisting of a benzene ring, a pyrrole ring, a thiophene ring, a furan ring, a pyrazole ring, an imidazole ring, a triazole ring, a thiazole ring, an oxazole ring, a pyridine ring, a pyrimidine ring, a pyrazine ring, a pyridazine ring, a cyclopropane ring, a cyclobutane ring, a cyclopentane ring, a cyclohexane ring, a piperidine ring, a piperazine ring and a tetrahydropyrrole ring.

In some embodiments, ring D is selected from the group consisting of a benzene ring and a pyridine ring.

In some embodiments, r1 is 0, and ring D is 2,3-dihydro-1H-indene.

In some embodiments, r1 is 1, and ring D is a benzene ring.

In some embodiments, r2 is 0.

In some embodiments, r2 is 1.

In some embodiments, r2 is 2.

In some embodiments, R_{U2} is 5- to 6-membered heteroaryl or phenyl, the 5- to 6-membered heteroaryl or phenyl is unsubstituted or substituted with 1, 2 or 3 substituents selected from the group consisting of: deuterium, halogen (preferably fluorine, chlorine), cyano, carboxyl, hydroxyl, C₁₋₃ alkyl, C₁₋₃ alkoxy, C₁₋₃ haloalkyl, C₁₋₃ haloalkoxy, -NH₂, -NHCOC₁₋₃ alkyl, -COC₁₋₃ alkyl, -COOC₁₋₃ alkyl, -OCOC₁₋₃ alkyl, -CONH₂, -NHCONH₂, -CONHC₁₋₃ alkyl, -NHCONHC₁₋₃ alkyl, -SOC₁₋₃ alkyl, -SO₂C₁₋₃ alkyl and -SC₁₋₃ alkyl.

In some embodiments, the 5- to 6-membered heteroaryl is selected from the group consisting of thiazolyl, oxazolyl, pyrazolyl, imidazolyl, pyrrolyl, pyridinyl, pyrimidinyl, pyridazinyl, pyrazinyl, tetrazolyl and triazolyl.

In some embodiments, the 5- to 6-membered heteroaryl is selected from the group consisting of

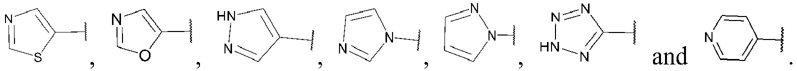

In some embodiments, the 5- to 6-membered heteroaryl is

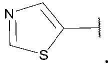

In some embodiments, the 5- to 6-membered heteroaryl ring is selected from the group consisting of a thiazole ring, an oxazole ring, a pyrazole ring, an imidazole ring, a pyrrole ring, a pyridine ring, a pyrimidine ring, a pyridazine ring, a pyrazine ring, a tetrazole ring and a triazole ring.

In some embodiments, R_{U2} is cyano.

In some embodiments, Rₐ₃ and R_{b3} are each independently selected from the group consisting of hydrogen, 5- to 6-membered heteroaryl and phenyl; the 5- to 6-membered heteroaryl is selected from the group consisting of thiazolyl, oxazolyl, pyrazolyl, imidazolyl, thienyl, furyl, pyrrolyl, triazolyl and tetrazolyl; the 5- to 6-membered heteroaryl and phenyl are unsubstituted or substituted with 1 or 2 substituents selected from the group consisting of: hydrogen, methyl, ethyl, isopropyl, trifluoromethyl, trifluoromethoxy, -COCH₃ and -CONH₂.

In some embodiments, R_{U2} is -NHRₐ₃, wherein Rₐ₃ is 5- to 6-membered heteroaryl or phenyl, and the 5- to 6-membered heteroaryl is selected from the group consisting of thiazolyl, imidazolyl, pyrazolyl, oxazolyl, pyridinyl and pyrimidinyl; wherein the 5- to 6-membered heteroaryl or phenyl is unsubstituted or substituted with 1, 2 or 3 substituents selected from the group consisting of: C₁₋₃ alkoxy, C₁₋₃ haloalkoxy, C₁₋₃ alkyl, C₁₋₃ haloalkyl, -SC₁₋₃ alkyl and -OCOC₁₋₃ alkyl.

In some embodiments, R_{U2} is -NHRₐ₃, wherein Rₐ₃ is thiazolyl; and the thiazolyl is substituted with 1, 2 or 3 substituents selected from the group consisting of: methyl, ethyl, propyl, isopropyl, methoxy, ethoxy, propoxy, isopropoxy, monofluoromethyl, difluoromethyl, trifluoromethyl, monofluoroethyl, difluoroethyl, trifluoroethyl, monofluoromethoxy, difluoromethoxy, trifluoromethoxy, monofluoroethoxy, difluoroethoxy and trifluoroethoxy.

In some embodiments, R_{U2} is -NHRₐ₃, wherein Rₐ₃ is thiazolyl; and the thiazolyl is substituted with 1, 2 or 3 substituents selected from the group consisting of: methyl, ethyl, propyl and isopropyl.

In some embodiments, R_{U2} is selected from the group consisting of following structure: cyano,

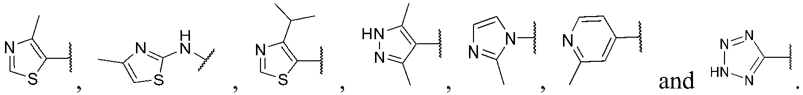

In some embodiments, r2 is 1, and R_{U2} is selected from the group consisting of cyano,

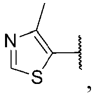

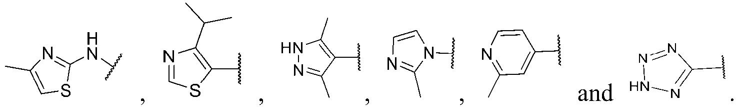

In some embodiments, r2 is 1, and R_{U2} is

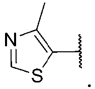

In some embodiments, r2 is 2, and R_{U2} are X₂ and 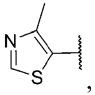 respectively.

In some embodiments, 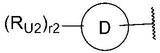 is selected from the group consisting of the following structures or isomers thereof: 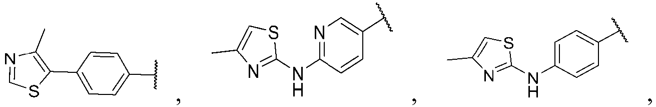 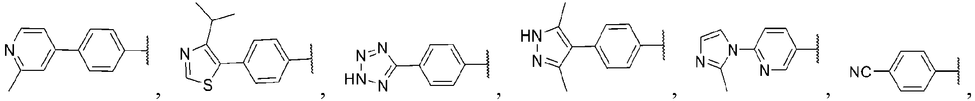 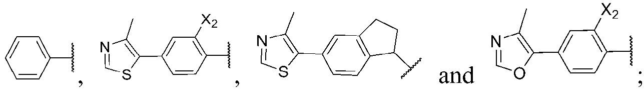 wherein X₂ is the point of attachment of ULM to L or POI.

In some embodiments, 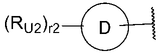 is selected from the group consisting of the following structure or an isomer thereof:

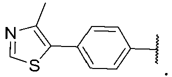

In some embodiments, one of R_{U1}, R_{U5}, R_{U6} and R_{U2} is X₂.

In some embodiments, R_{Ua} is selected from the group consisting of fluorine, cyano, methyl, ethyl, trifluoromethyl and trifluoromethoxy.

In some embodiments, R_{Ua} is selected from the group consisting of fluorine and cyano.

In some embodiments, U₁ is selected from the group consisting of X₂, -NHCO-X₂, -NHCOCH₃, 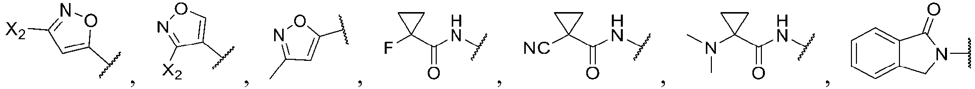 and 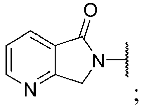 wherein X₂ is the point of attachment of ULM to L or POI.

In some embodiments, U₁ is selected from the group consisting of -NHCO-X₂ and 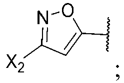 wherein X₂ is the point of attachment of ULM to L or POI.

In some embodiments, R_{U3} and R_{U4} are each independently hydrogen, C₁₋₆ alkyl, C₁₋₆ haloalkyl, C₁₋₆ alkoxy or C₁₋₆ haloalkoxy; or R_{U3}, R_{U4} and carbon atom linked to R_{U3} and R_{U4} form a C₃₋₆ cycloalkyl ring.

In some embodiments, R_{U3} and R_{U4} are each independently hydrogen, -CH₃, -CH₂CH₃, -CH(CH₃)₂, -C(CH₃)₃, -CF₃, -CHF₂, -CH₂F, -OCH₃, -OCH(CH₃)₂, -OC(CH₃)₃, -OCF₃, -OCHF₂, -OCH₂F, fluoro-substituted isopropyl or fluoro-substituted tert-butyl; or R_{U3}, R_{U4} and carbon atom linked to R_{U3} and R_{U4} form a cyclopropyl ring, a cyclobutyl ring, a cyclopentyl ring or a cyclohexyl ring.

In some embodiments, R_{U3} and R_{U4} are each independently hydrogen, -CH₃, -CH₂CH₃, -CH₂CH₂CH₃, -CH(CH₃)₂ or -C(CH₃)₃; or R_{U3}, R_{U4} and carbon atom linked to R_{U3} and R_{U4} form a cyclopropyl ring.

In some embodiments, R_{U3} and R_{U4} are each independently hydrogen, -CH(CH₃)₂ or -C(CH₃)₃.

In some embodiments, R_{U1} is selected from the group consisting of the following structures or isomers thereof: 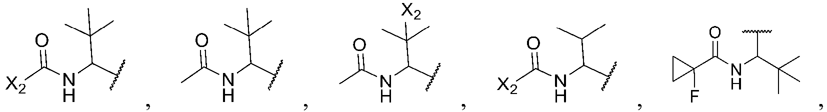 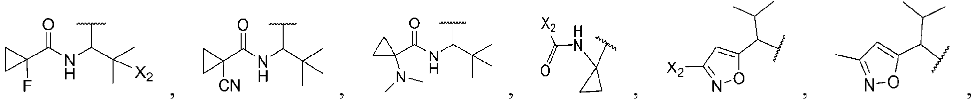 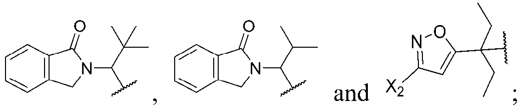 wherein X₂ is the point of attachment of ULM to L or POI.

In some embodiments, R_{U1} is selected from the group consisting of the following structures: 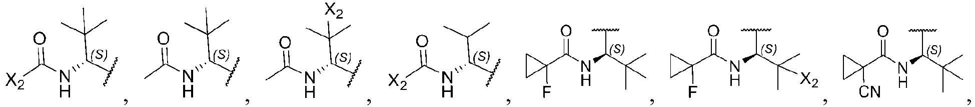 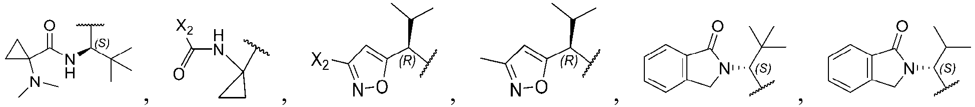 and 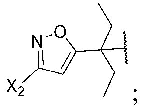 wherein X₂ is the point of attachment of ULM to L or POI.

In some embodiments, R_{U1} is selected from the group consisting of the following structure: 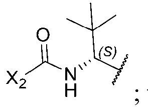 wherein X₂ is the point of attachment of ULM to L or POI.

In some embodiments, R_{U5} and R_{U6} are each independently X₂, hydrogen, deuterium, halogen, hydroxyl, carboxyl, cyano, amino, C₁₋₃ alkyl, C₁₋₃ alkoxy, C₁₋₃ haloalkyl or C₁₋₃ haloalkoxy; or R_{U5} and carbon atom on ring D together form a C₃₋₆ cycloalkyl ring; wherein the C₃₋₆ cycloalkyl ring is unsubstituted or substituted with 1, 2, 3 or 4 substituents selected from the group consisting of: deuterium, halogen (preferably fluorine, chlorine or bromine), cyano, carboxyl, hydroxyl, nitro, formyl, sulfo, -NH₂, -NH(C₁₋₆ alkyl), -N(C₁₋₆ alkyl)₂, C₁₋₆ alkyl, C₁₋₆ alkoxy, -SC₁₋₆ alkyl, -SOC₁₋₆ alkyl, -SO₂C₁₋₆ alkyl, C₁₋₆ haloalkyl, C₁₋₆ haloalkoxy, amino-substituted C₁₋₆ alkyl, cyano-substituted C₁₋₆ alkyl, hydroxy-substituted C₁₋₆ alkyl, carboxyl-substituted C₁₋₆ alkyl, -COC₁₋₆ alkyl, -CO₂NH₂, -CO₂NH(C₁₋₆ alkyl), -CO₂N(C₁₋₆ alkyl)₂, -SO₂NH₂, -SO₂NH(C₁₋₆ alkyl), -SO₂N(C₁₋₆ alkyl)₂, a C₃₋₈ cycloalkyl ring, a 4- to 10-membered heterocycloalkyl ring, a 5- to 6-membered heteroaryl ring and a benzene ring.

In some embodiments, R_{U5} and R_{U6} are each independently hydrogen, C₁₋₃ alkyl or C₁₋₃ haloalkyl.

In some embodiments, R_{U5} and R_{U6} are each independently hydrogen, -CH₃, -OCH₃, -CF₃, -OCF₃, -CHF₂, -CH₂F, -OCHF₂ or -OCH₂F.

In some embodiments, R_{U5} and R_{U6} are each independently hydrogen or -CH₃.

In some embodiments, R_{U7} is selected from the group consisting of hydrogen, methyl, ethyl, monofluoromethyl, difluoromethyl and trifluoromethyl.

In some embodiments, R_{U7} is hydrogen.

In some embodiments, R_{U7}, nitrogen atom linked to R_{U7}, R_{U6} and carbon atom linked to R_{U6} together form a partially unsaturated 3- to 8-membered heterocycloalkyl ring, a 5- to 6-membered heteroaryl ring or a benzene ring.

In some embodiments, R_{U7}, nitrogen atom linked to R_{U7}, R_{U6} and carbon atom linked to R_{U6} together form a tetrahydropyrrole ring, a pyrazolidine ring, an imidazolidine ring, a piperazine ring, a piperidine ring, a 2,3-dihydro-1H-pyrrole ring, a 2,3-dihydro-1H-pyrazole ring, a pyrrole ring, a pyrazole ring, an imidazole ring, a pyridine ring, a pyrimidine ring or a benzene ring.

In some embodiments, R_{U7}, nitrogen atom linked to R_{U7}, R_{U6} and carbon atom linked to R_{U6} together form a 4- to 6-membered heterocycloalkyl ring.

In some embodiments, R_{U7}, nitrogen atom linked to R_{U7}, R_{U6} and carbon atom linked to R_{U6} together form a 5-membered heterocycloalkyl ring.

In some embodiments, R_{U7}, nitrogen atom linked to R_{U7}, R_{U6} and carbon atom linked to R_{U6} together form a tetrahydropyrrole ring.

In some embodiments, 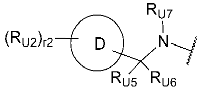 is selected from the group consisting of the following structures or isomers thereof: 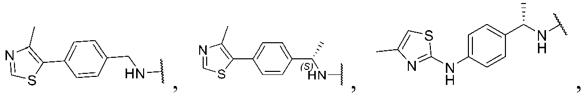 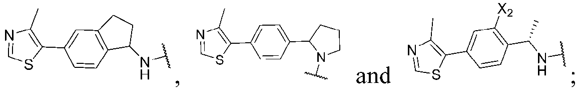 wherein X₂ is the point of attachment of ULM to L or POI.

In some embodiments, 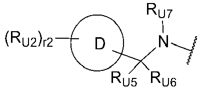 is selected from the group consisting of the following structures or isomers thereof:

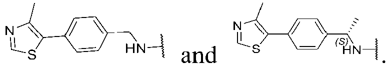

In some embodiments, R_{U8} is selected from the group consisting of hydroxyl, amino, -NH(C₁₋₃ alkyl), -N(C₁₋₃ alkyl)₂, C₁₋₃ alkoxy, fluoro C₁₋₃ alkoxy and -OCOC₁₋₃ alkyl.

In some embodiments, R_{U8} is selected from the group consisting of hydroxyl, amino, -NH(CH₃), -N(CH₃)₂, -OCH₃, -OCF₃ and -OCOCH₃.

In some embodiments, R_{U8} is hydroxyl.

In some embodiments, ULM is selected from the group consisting of the following structures or isomers thereof: 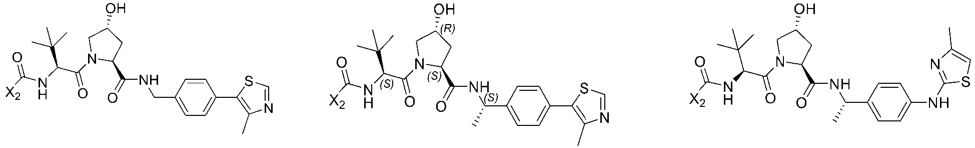 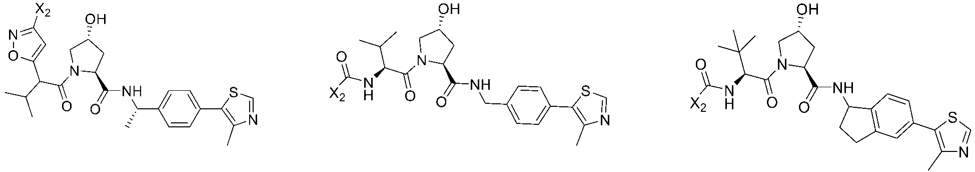 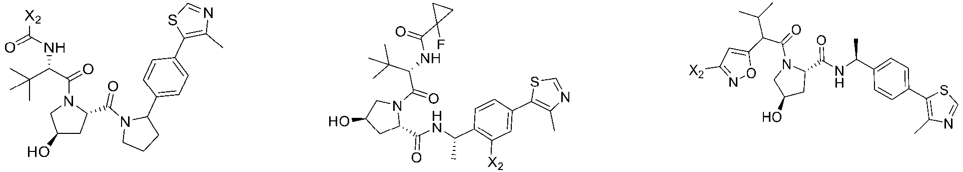 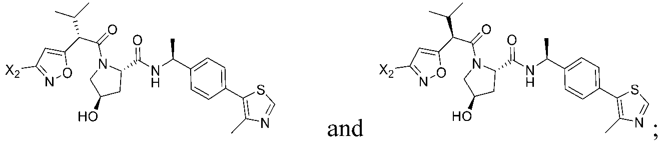 wherein X₂ is the point of attachment of ULM to L or POI.

In some embodiments, ULM is selected from the group consisting of the following structures or isomers thereof: 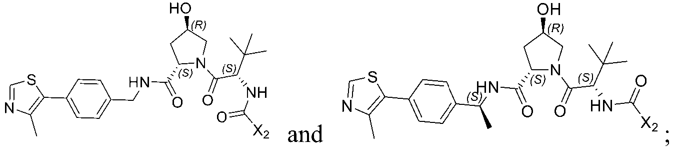 wherein X₂ is the point of attachment of ULM to L or POI.

In some embodiments, ULM is selected from the group consisting of the following structures or isomers thereof: 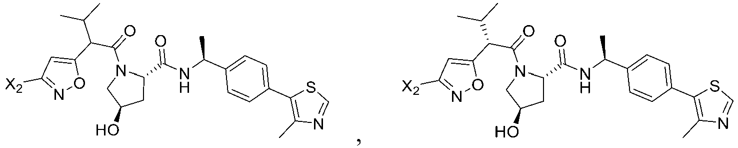 and 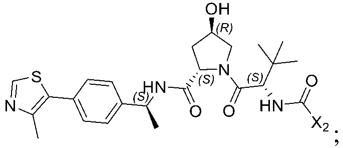 wherein X₂ is the point of attachment of ULM to L or POI.

In some embodiments, the compound of formula (I) is a structure of formula (I-A), 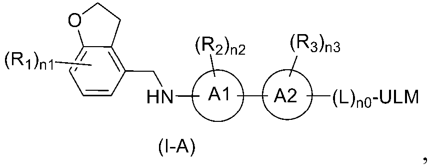 wherein, ring A1, ring A2, R₁, n1, R₂, n2, R₃, n3, L, n0 and ULM are as defined in the specification, and both R₂ and R₃ are not X₁.

In some embodiments, the compound of formula (I) is a structure of formula (I-B), 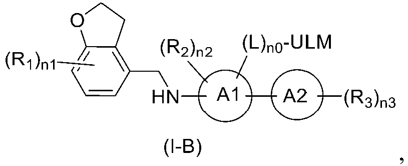 wherein, ring A1, ring A2, R₁, n1, R₂, n2, R₃, n3, L, n0 and ULM are as defined in the specification, and both R₂ and R₃ are not X₁.

In some embodiments, the compound of formula (I) is selected from the group consisting of the following structures or isomers thereof: 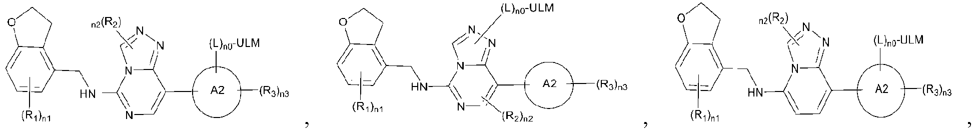 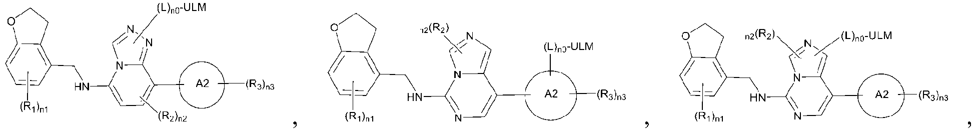 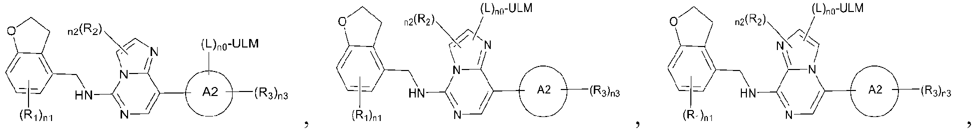 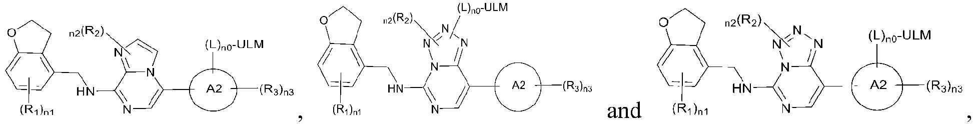 wherein, ring A2, R₁, n1, R₂, n2, R₃, n3, L, n0 and ULM are as defined in the specification, and both R₂ and R₃ are not X₁.

In some embodiments, the compound of formula (I) is selected from the group consisting of the following structures or isomers thereof: 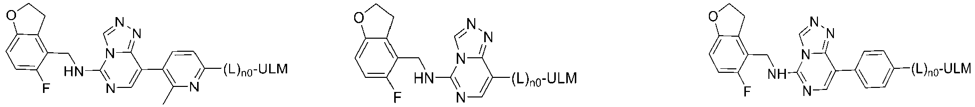 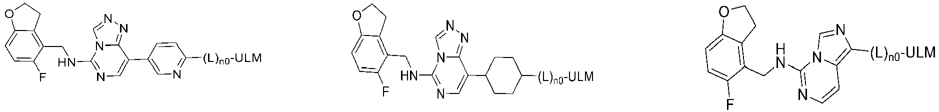 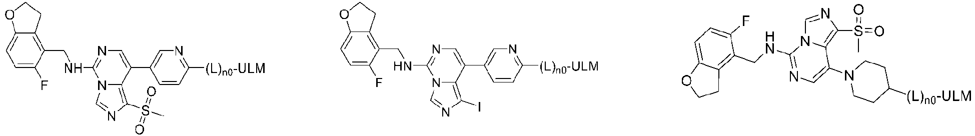 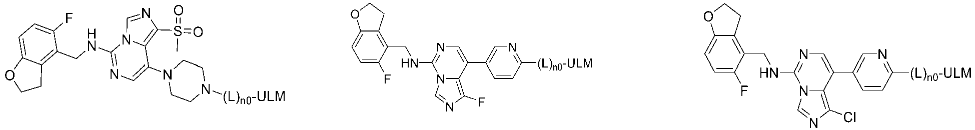 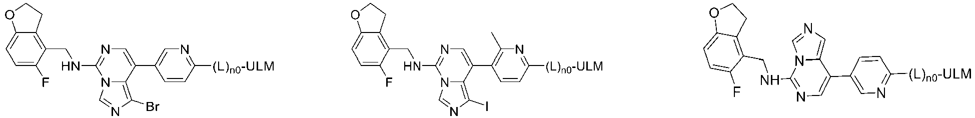 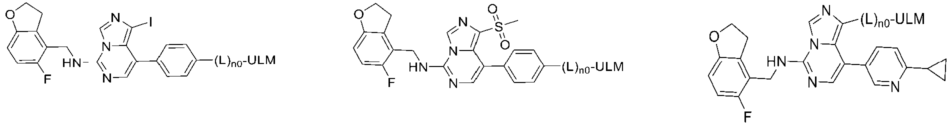 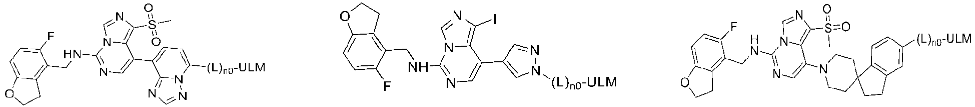 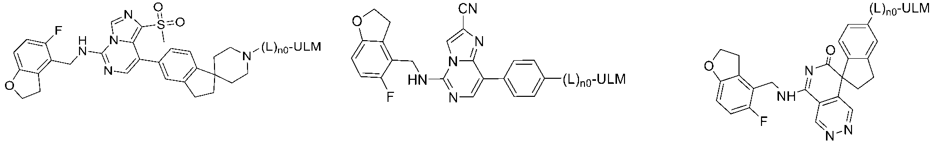 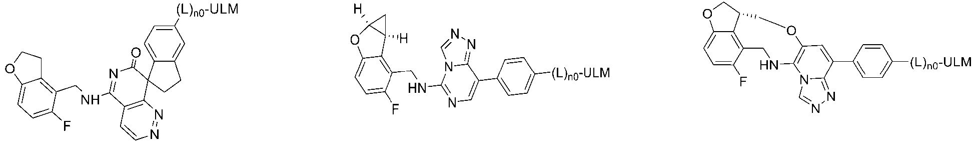 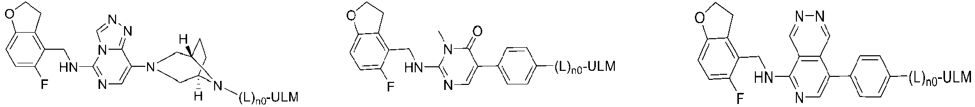 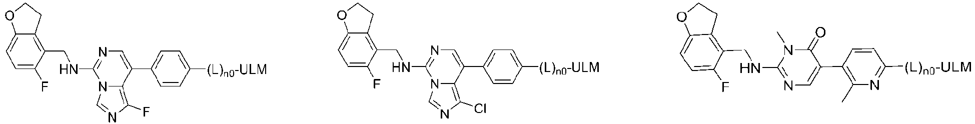 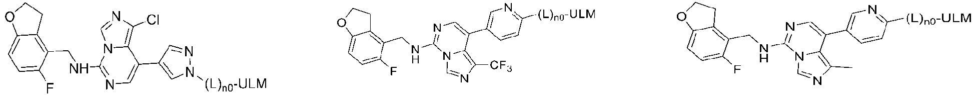 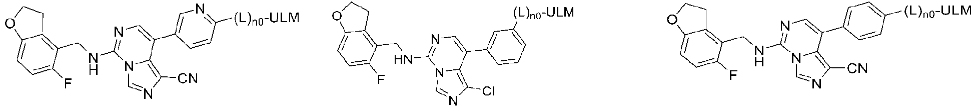 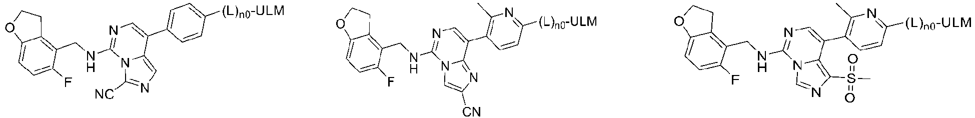 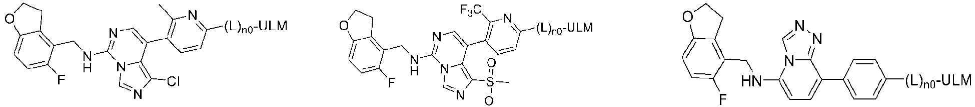 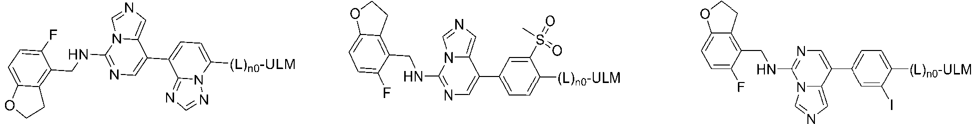 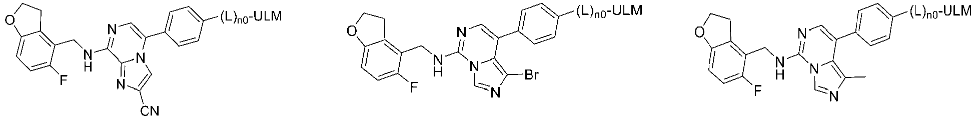 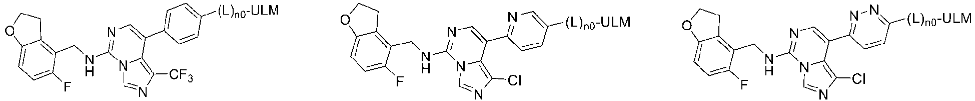 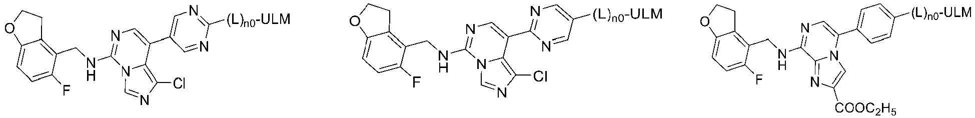 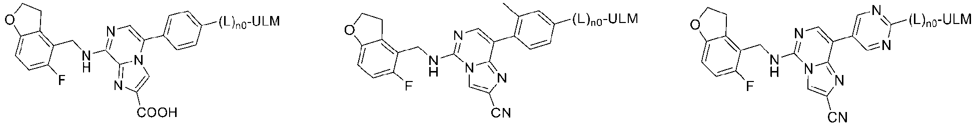 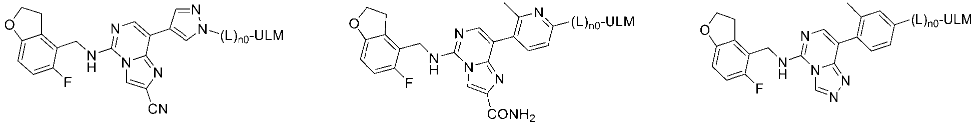 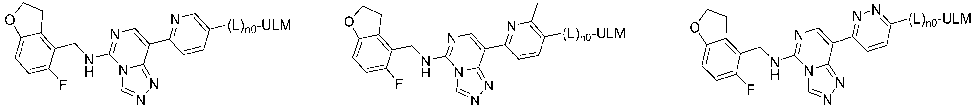 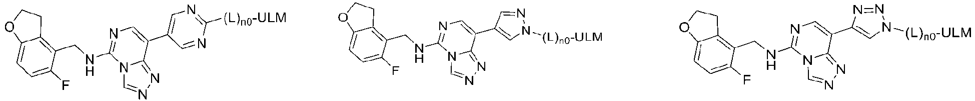 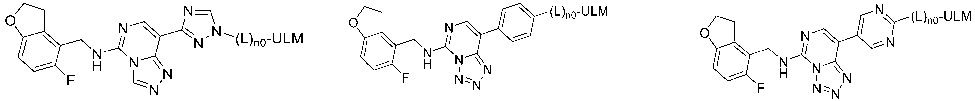 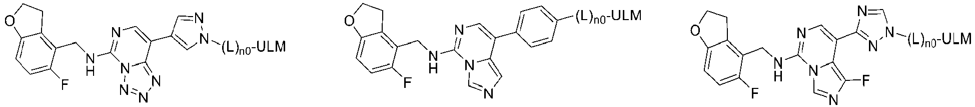 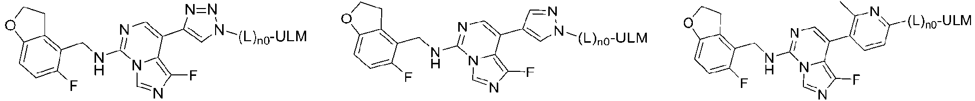 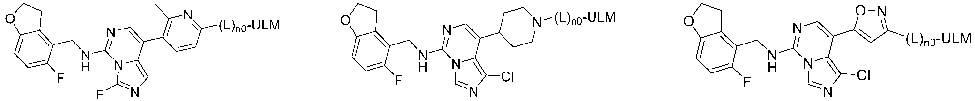 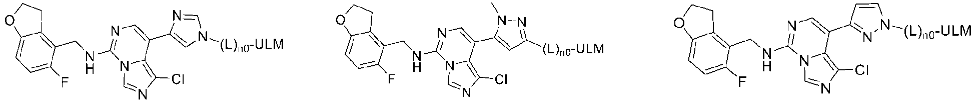 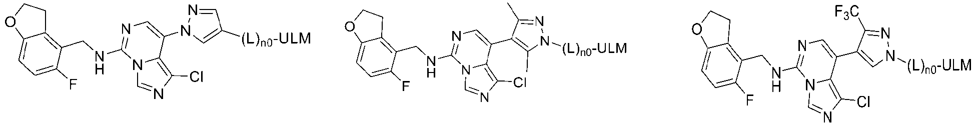 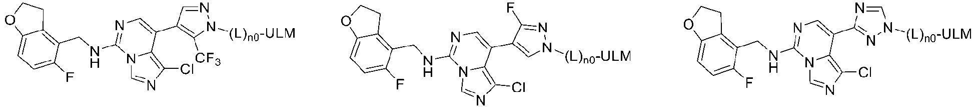 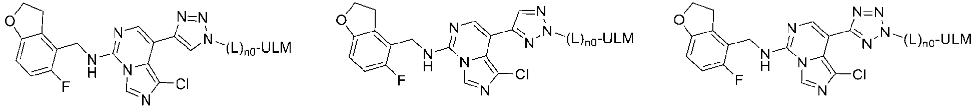 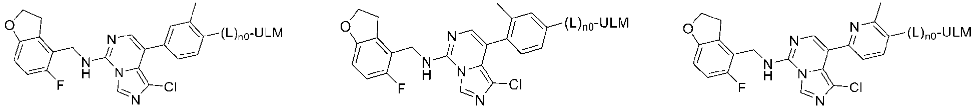 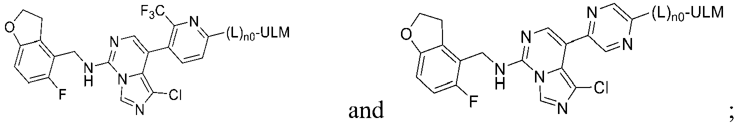 wherein, L, n0 and ULM are as defined in the specification.

In some embodiments, the compound of formula (I) is selected from the group consisting of the following structures or isomers thereof: 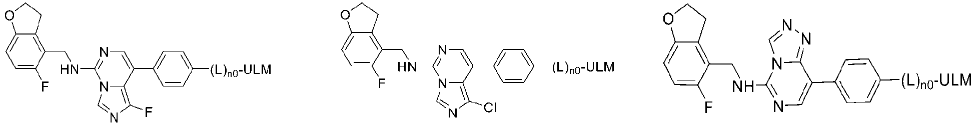 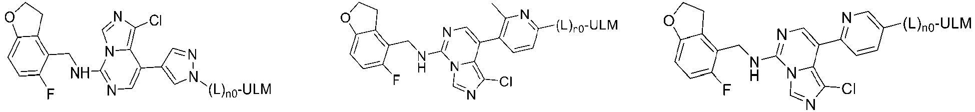 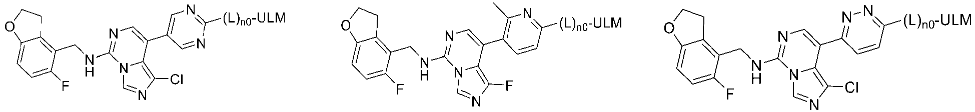 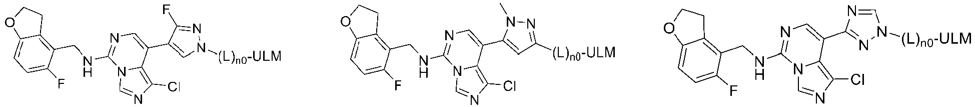 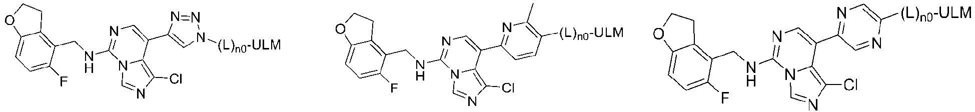 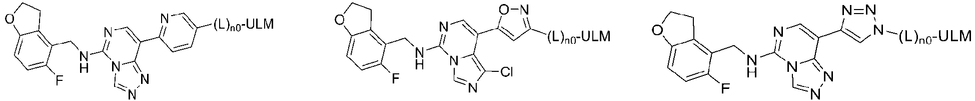 and 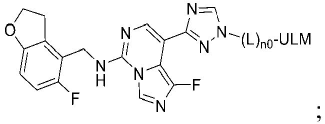 wherein, L, n0 and ULM are as defined in the specification.

In some embodiments, the compound of formula (I) is selected from the group consisting of the following structures or isomers thereof: 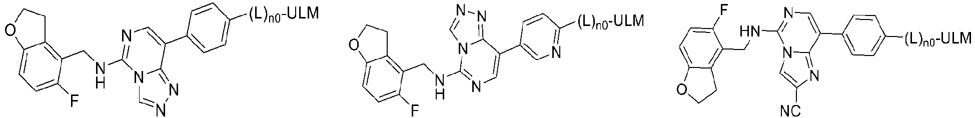 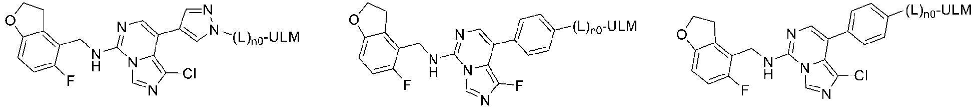 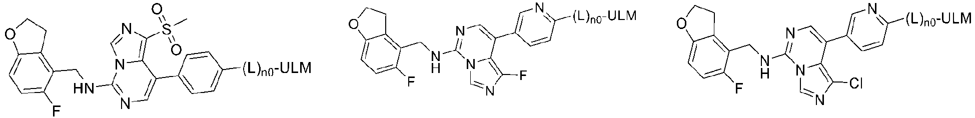 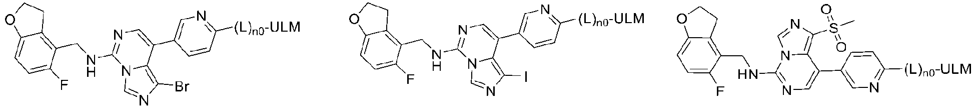 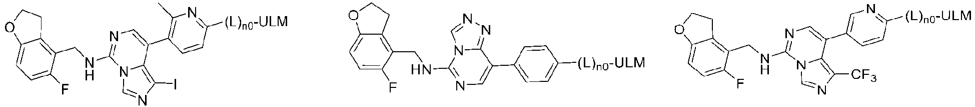 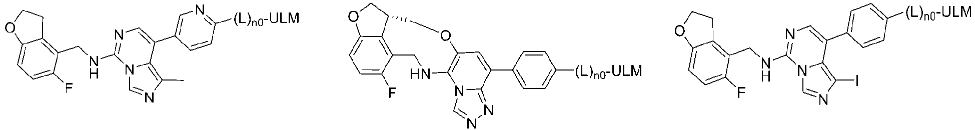 and 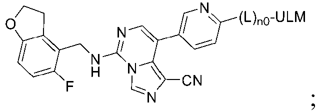 wherein, L, n0 and ULM are as defined in the specification.

In some embodiments, the compound of formula (I) is selected from the group consisting of the following structures or isomers thereof: 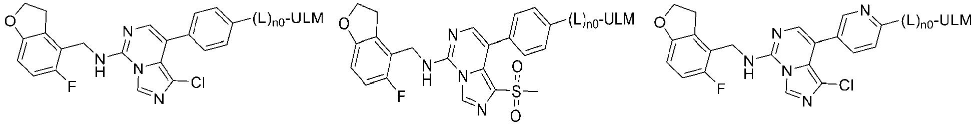 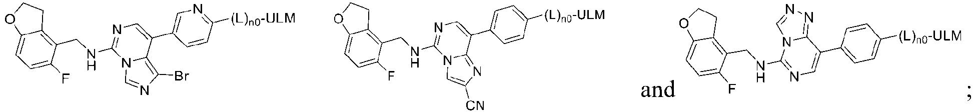 wherein, L, n0, and ULM are as defined in the specification.

In some embodiments, the compound of formula (I) is a specific compound selected from the Examples.

In some embodiments, the compound of formula (I) is a compound selected from Table A or a stereoisomer thereof:

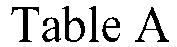

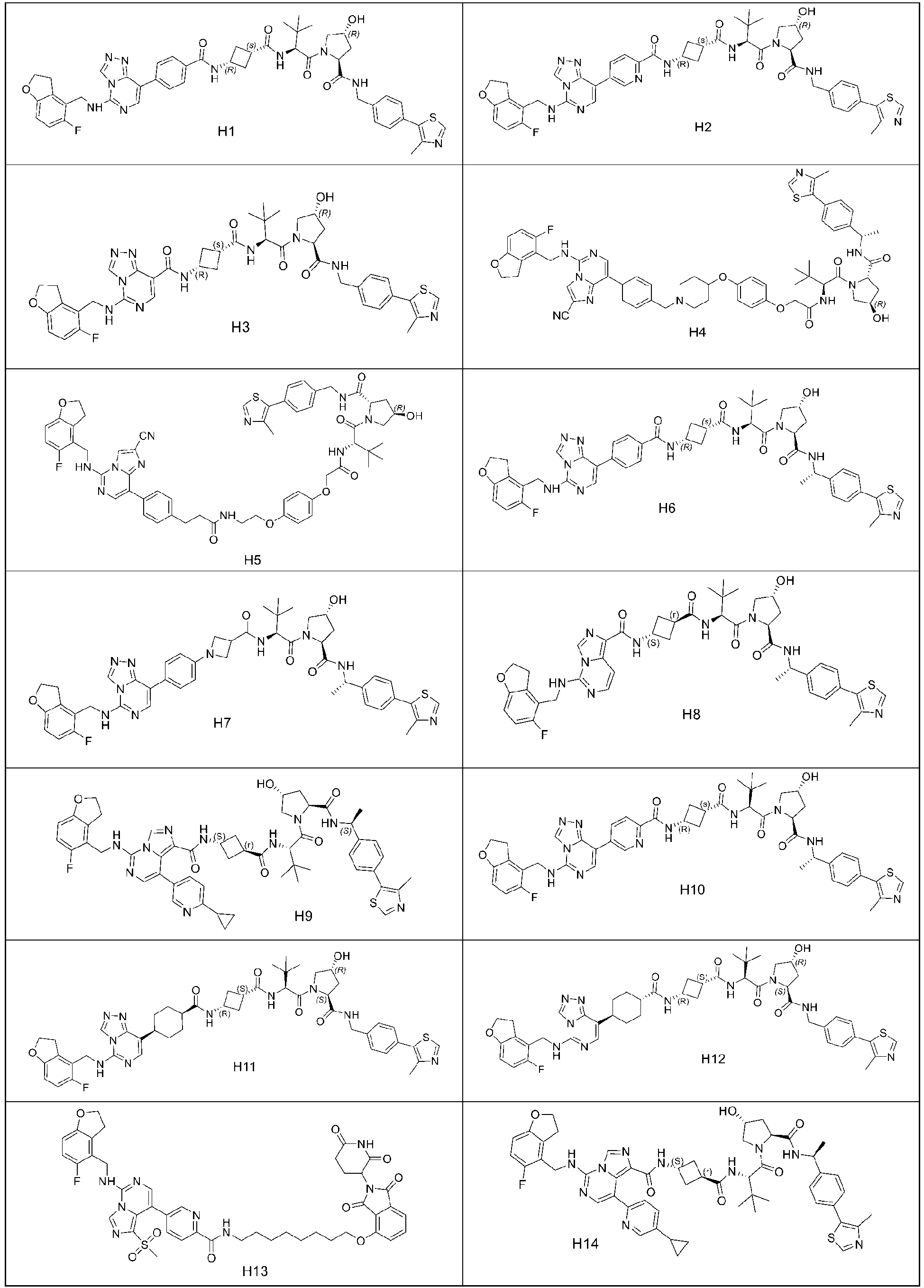

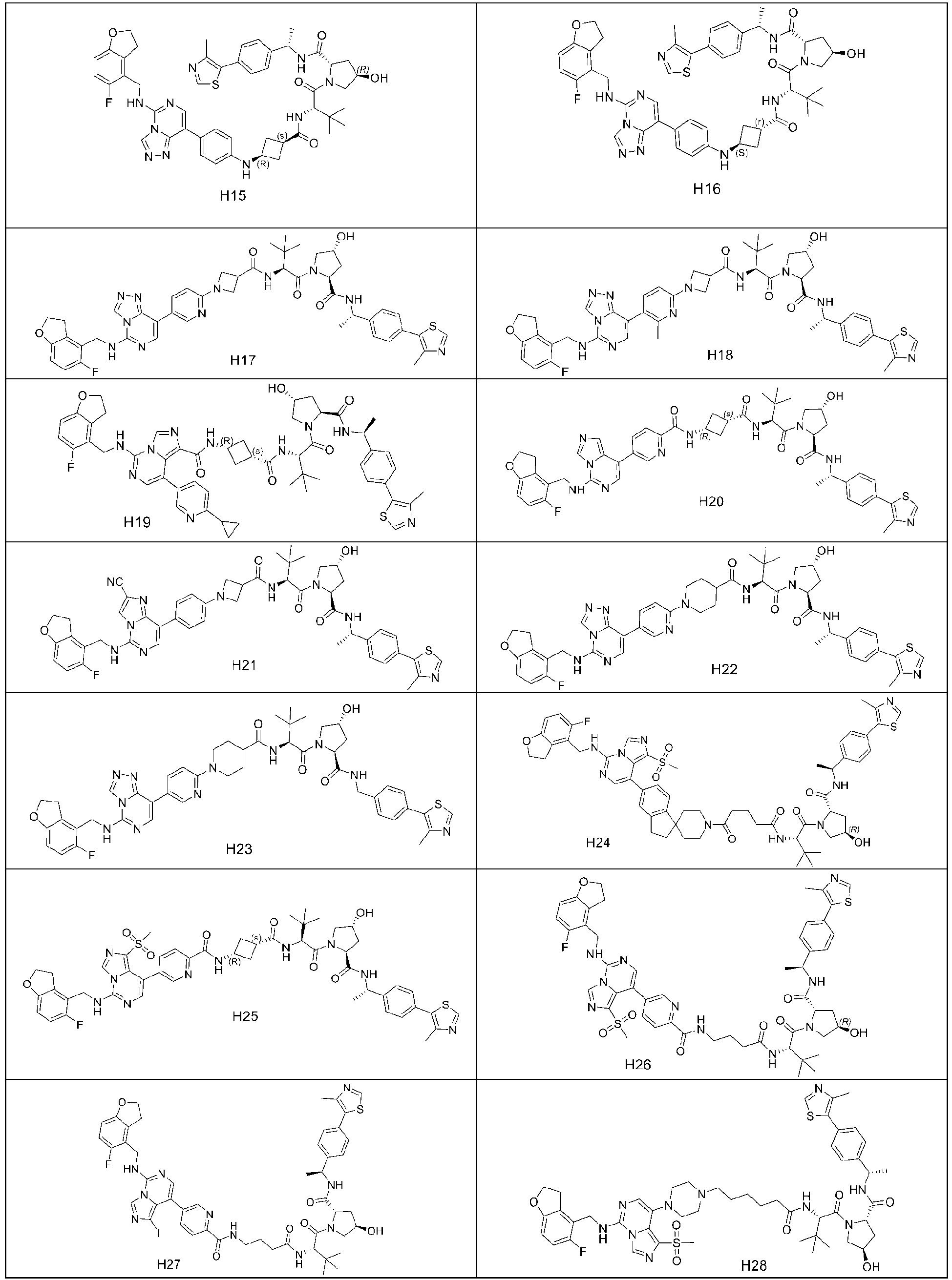

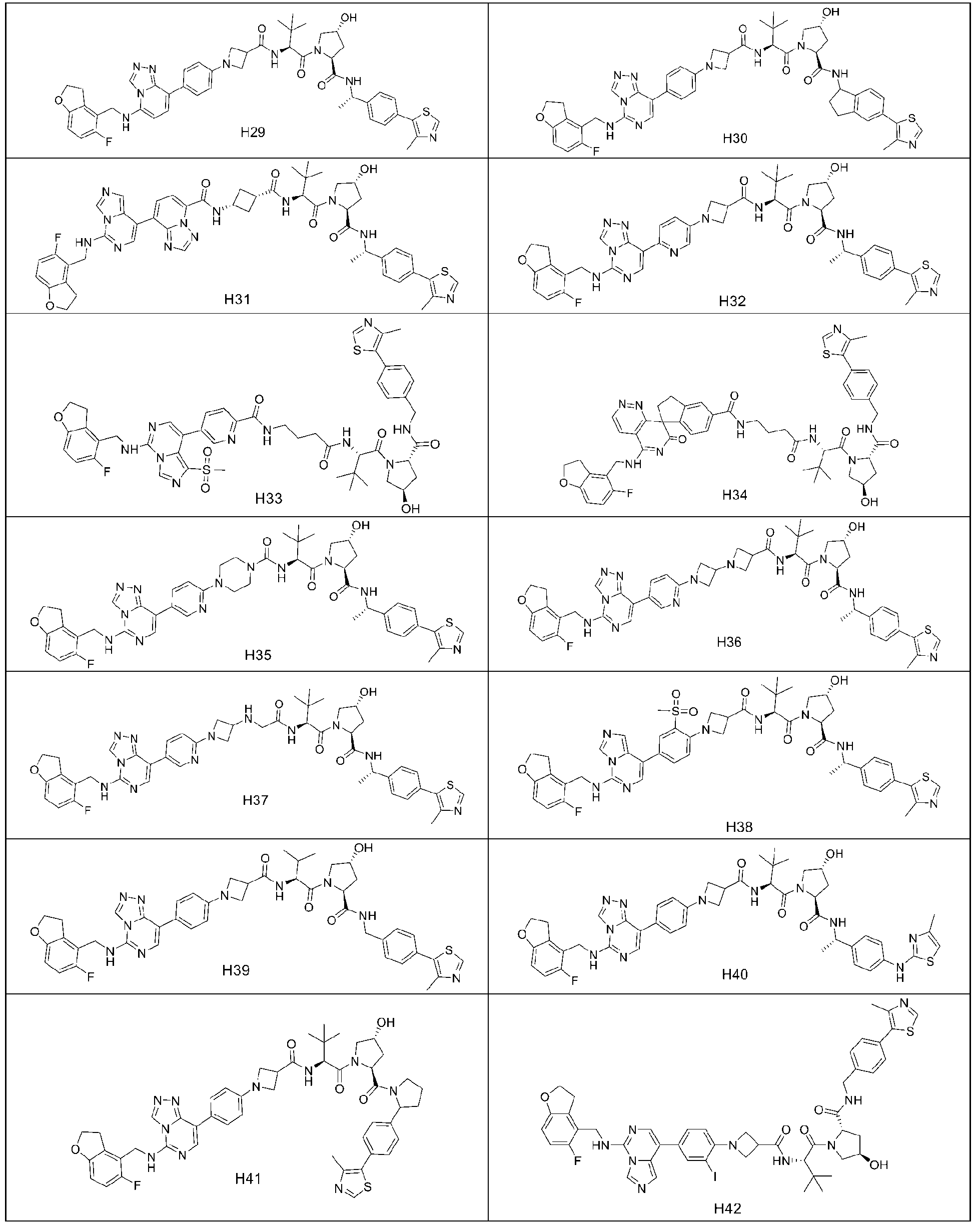

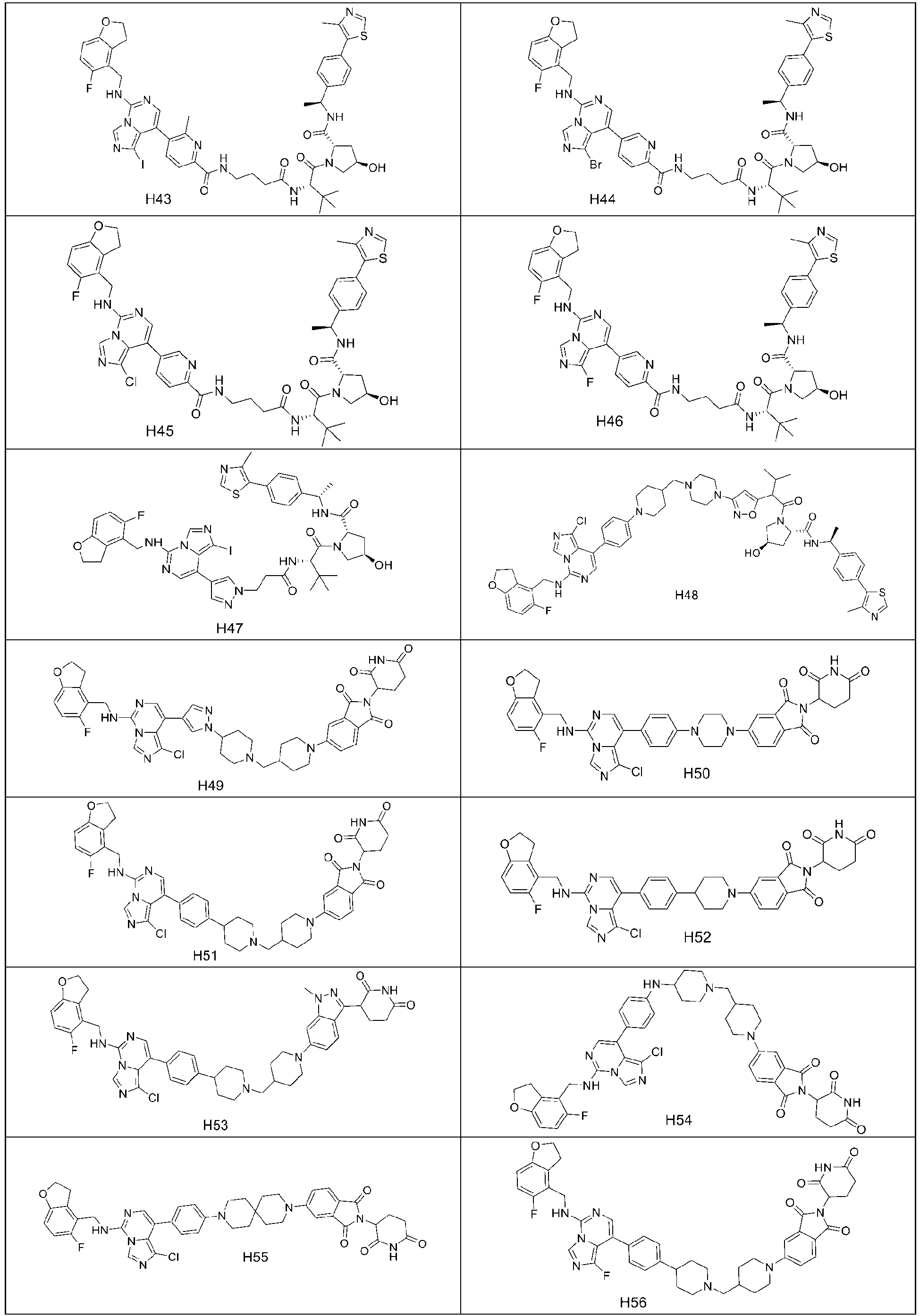

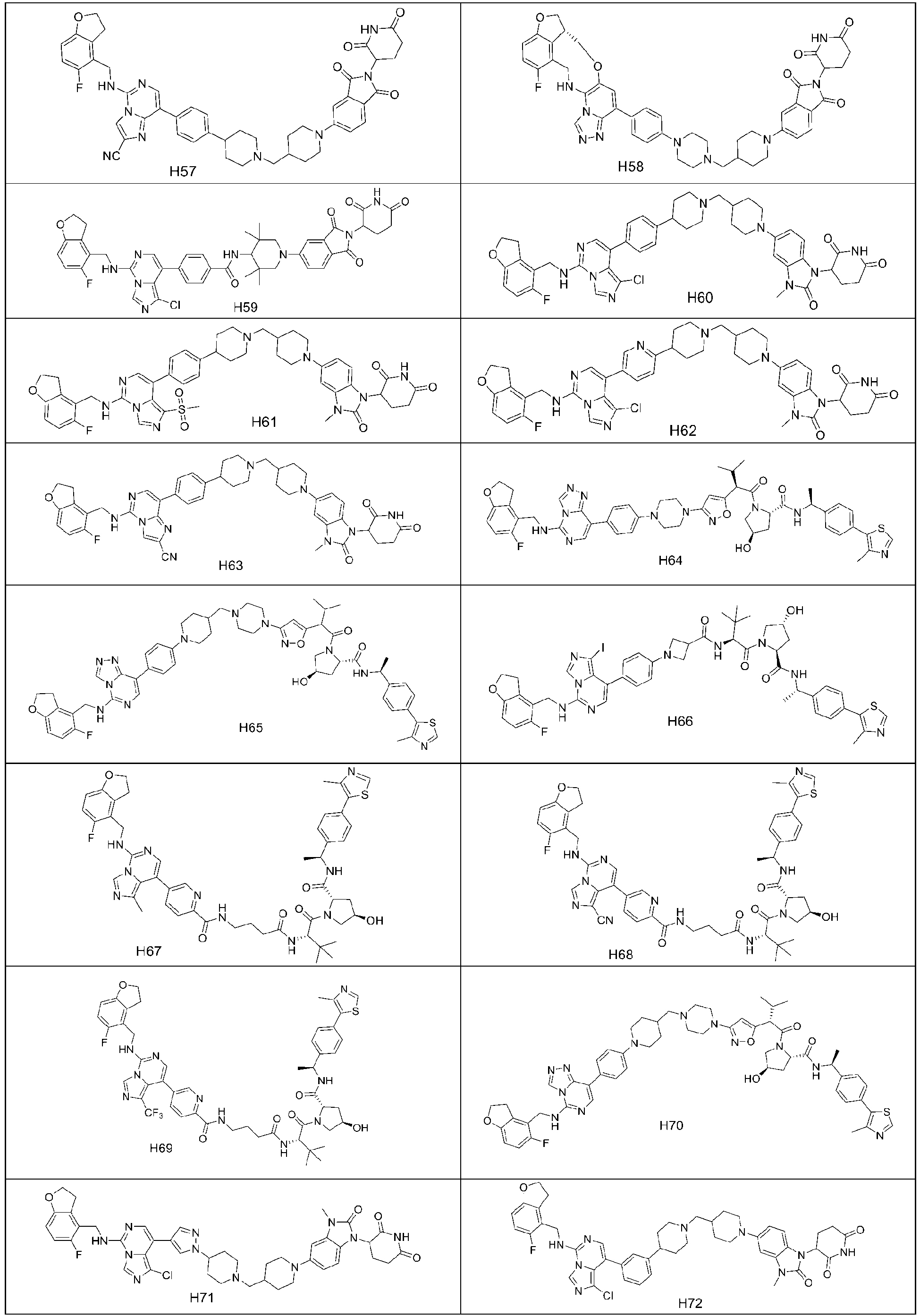

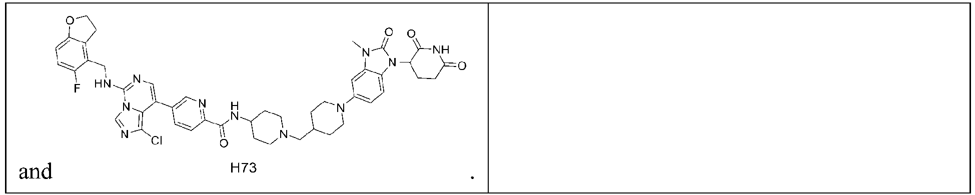

In some embodiments, the compound of formula (I) is a compound selected from Table B or a stereoisomer thereof:

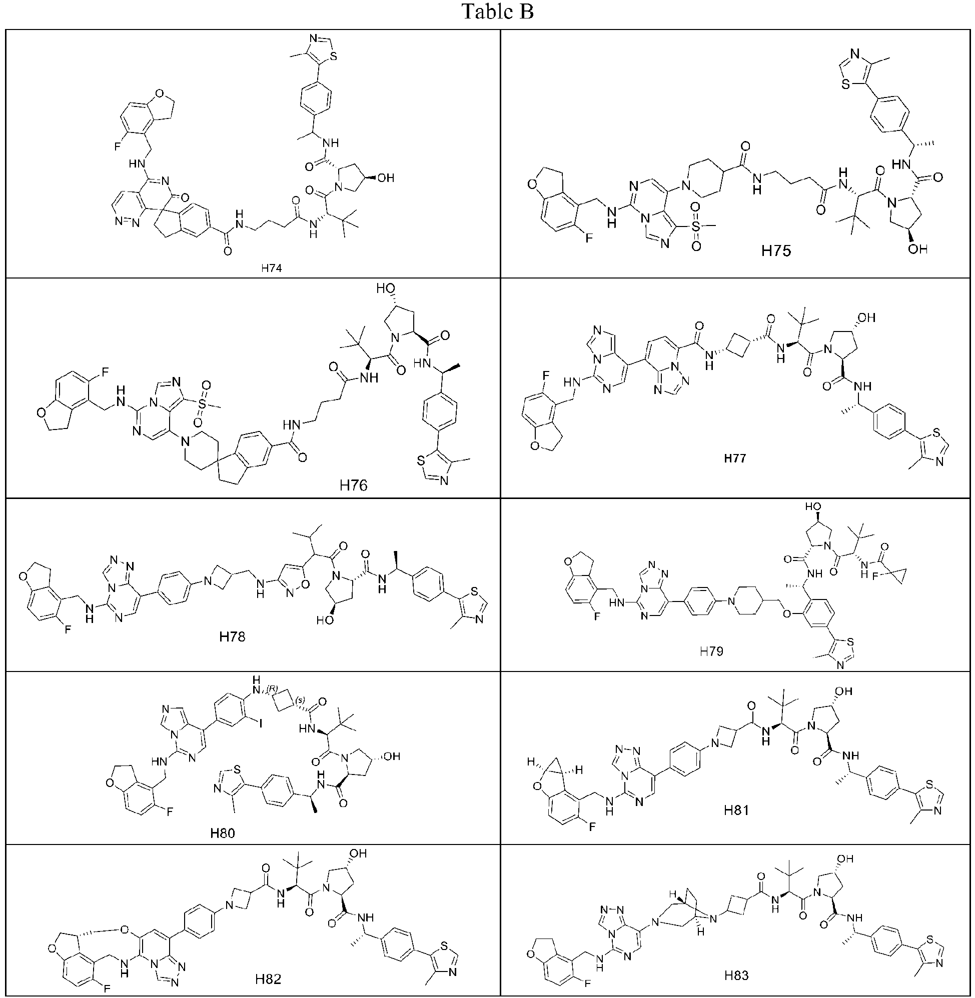

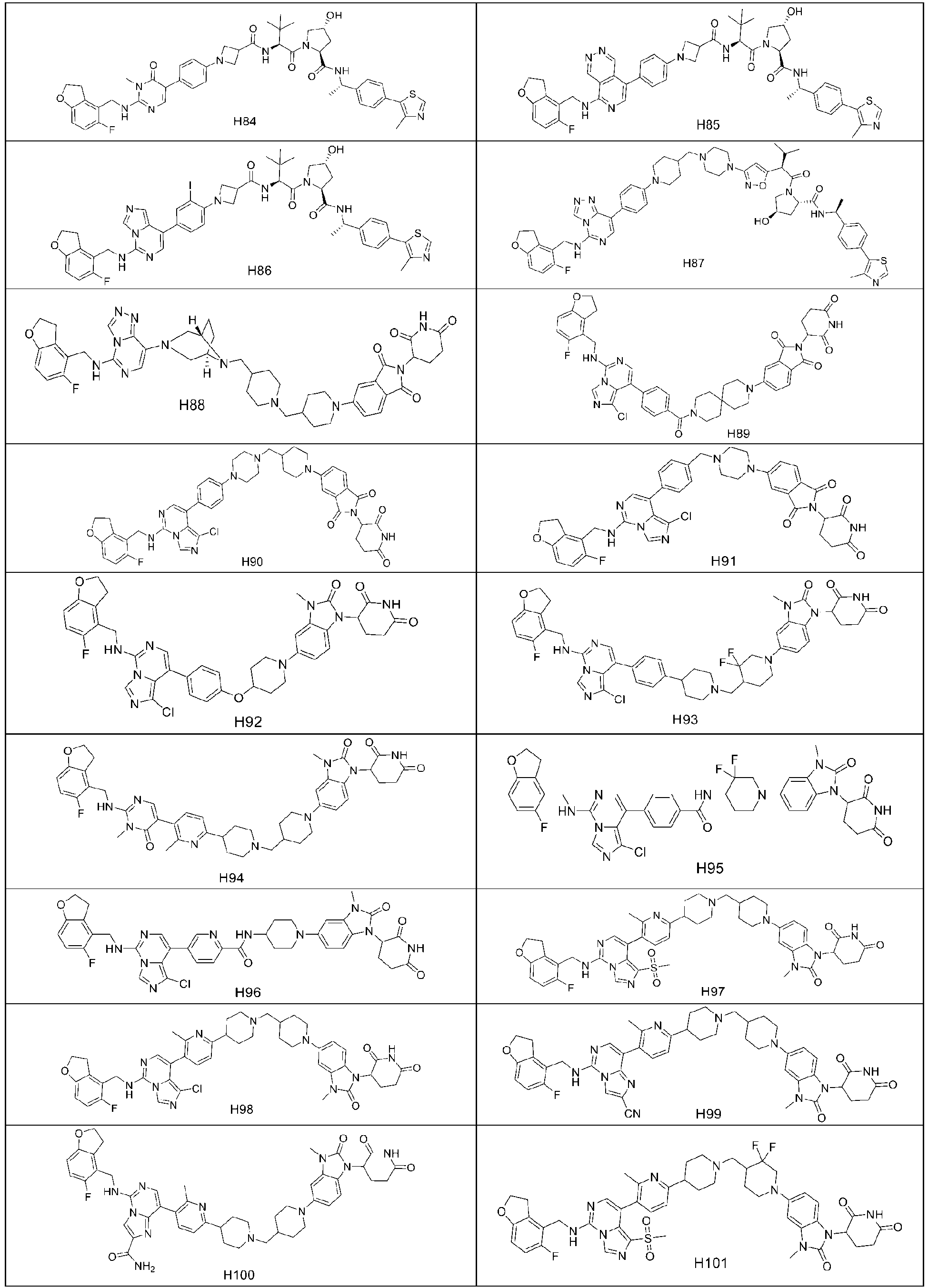

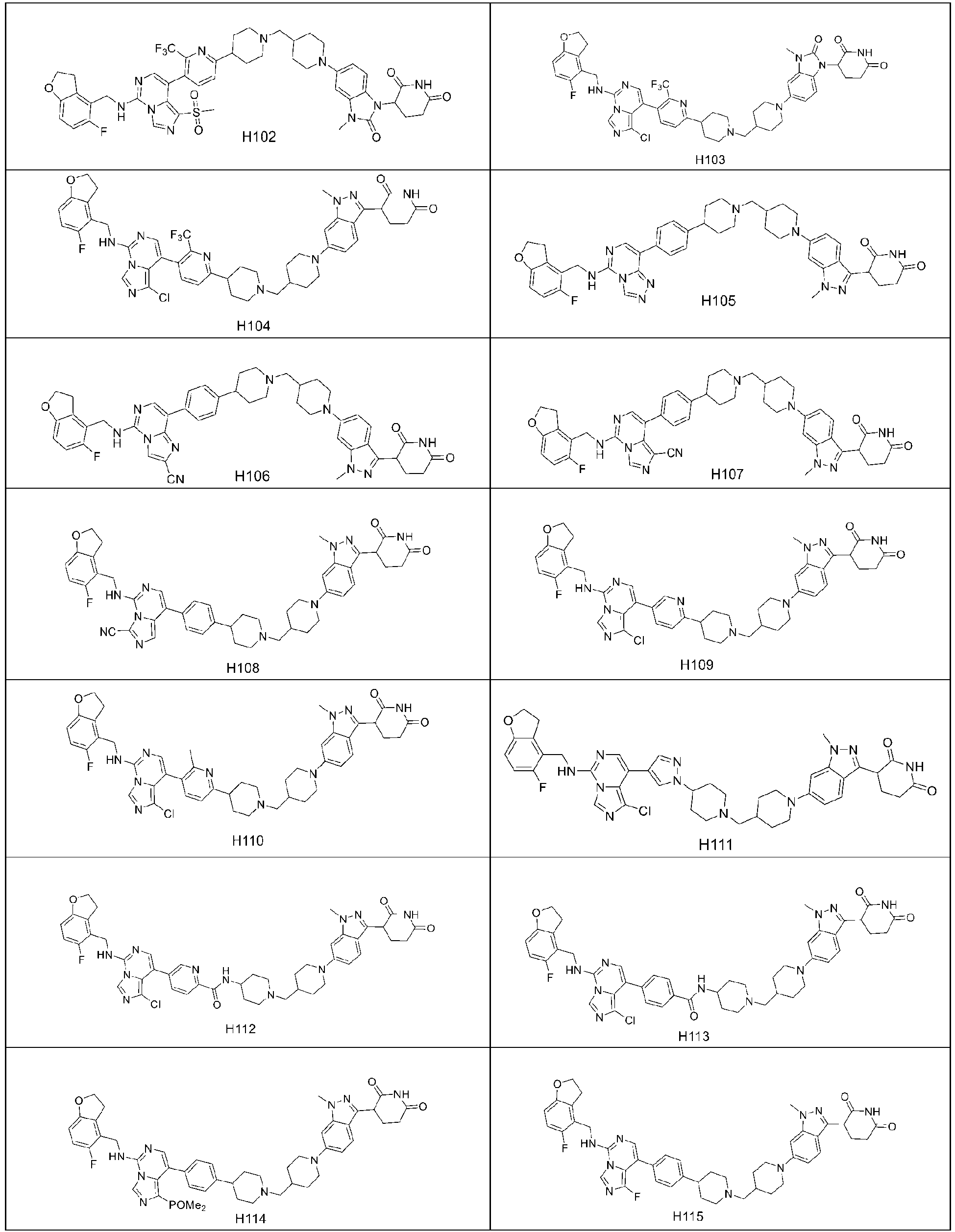

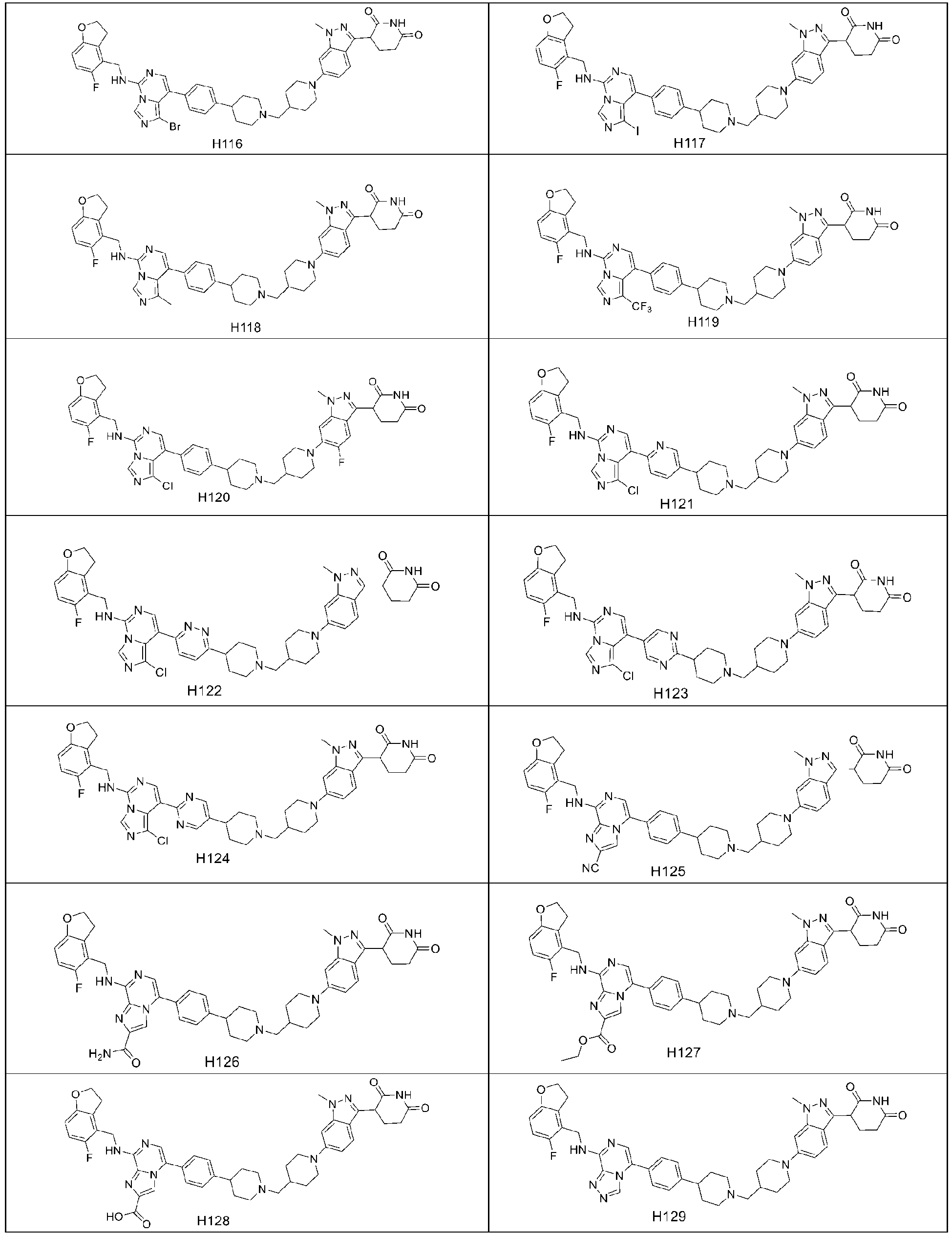

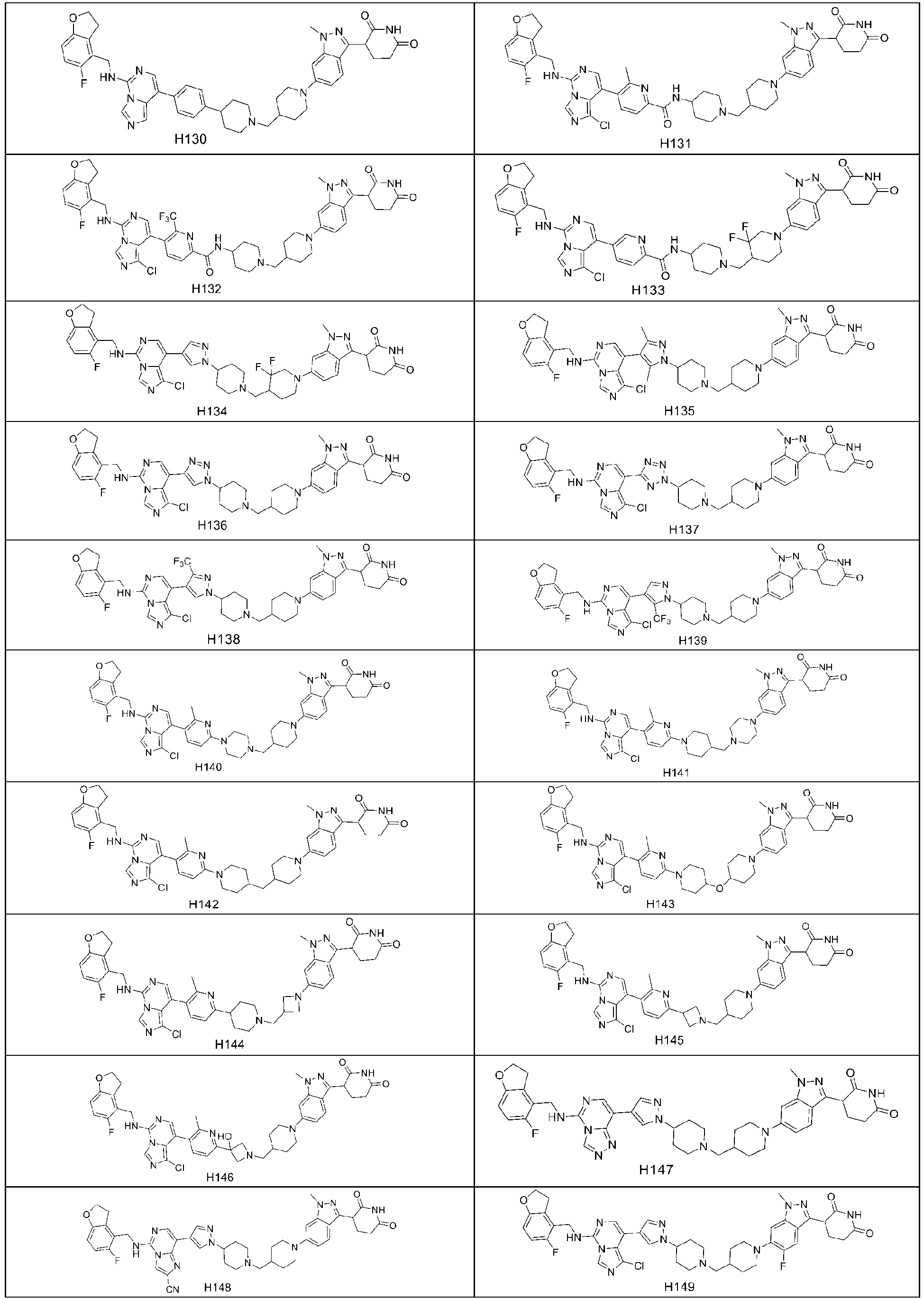

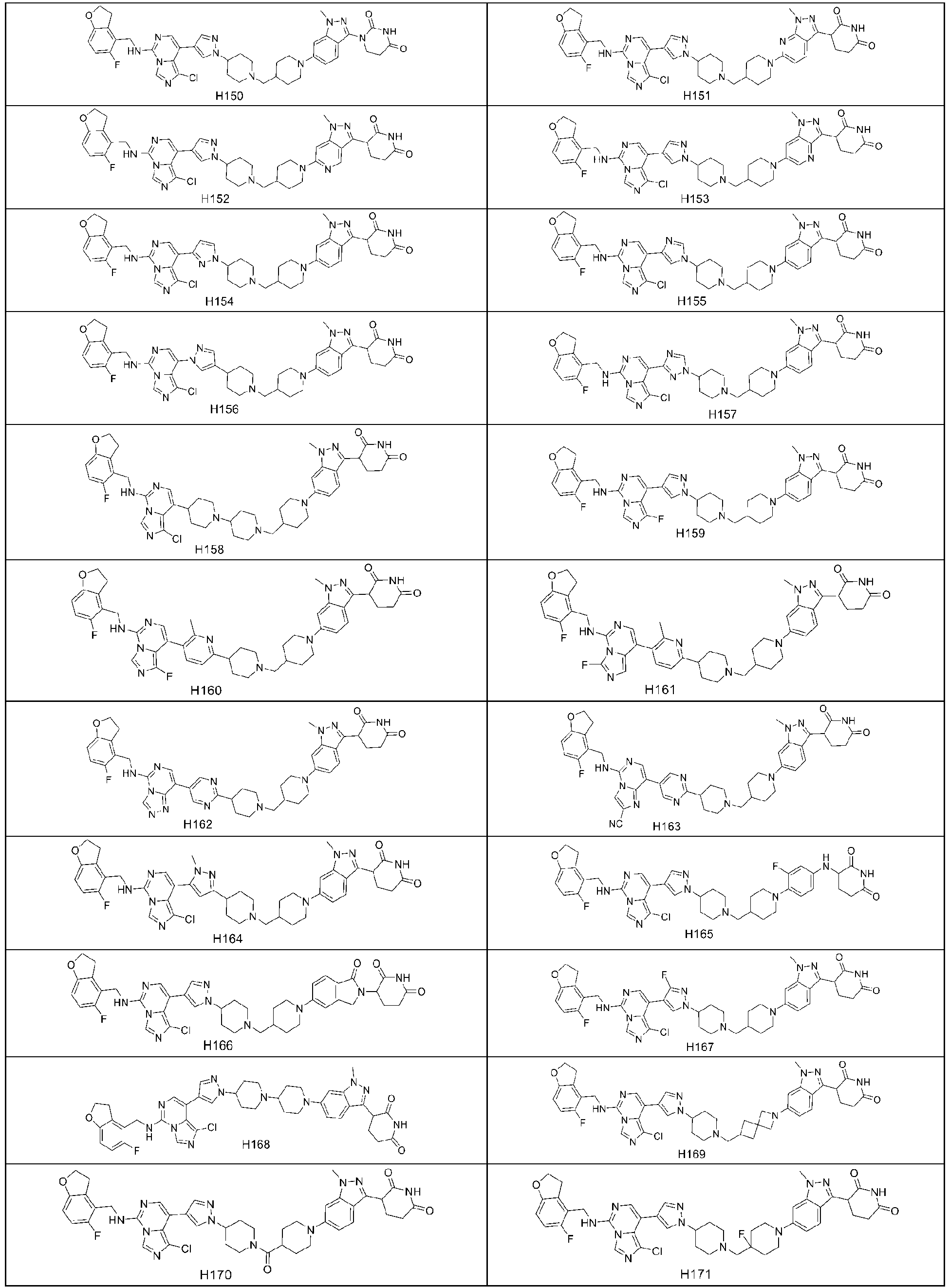

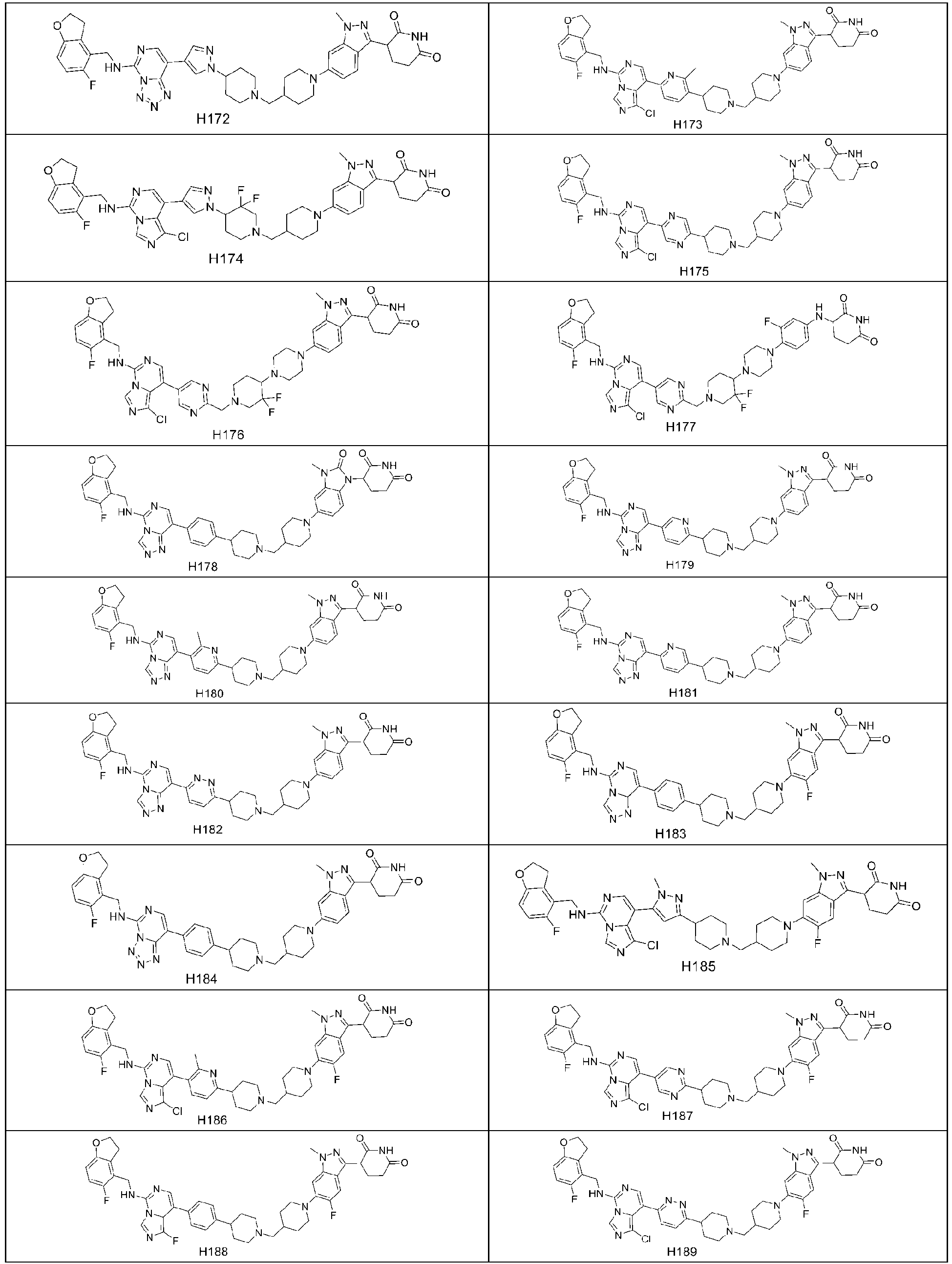

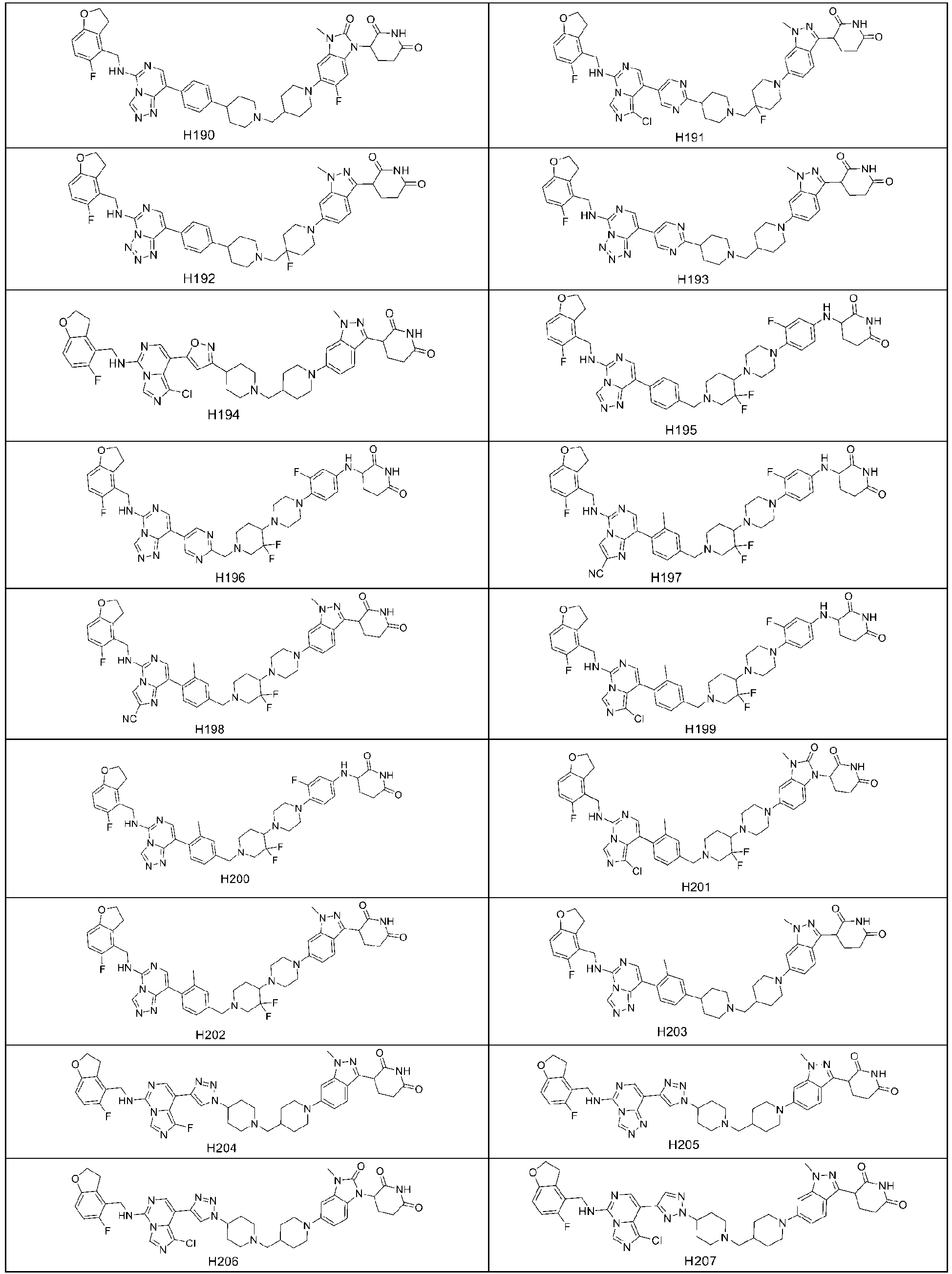

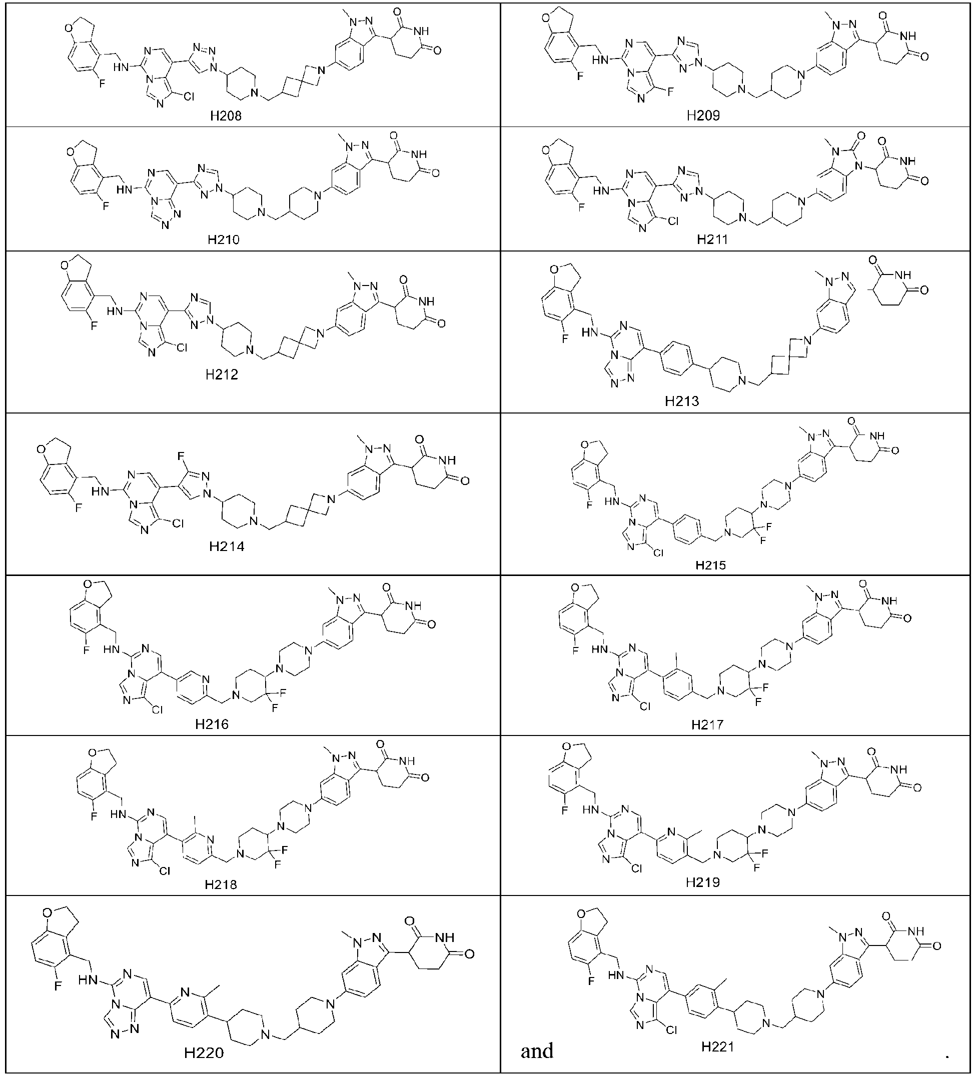

A second aspect of the present application provides a pharmaceutical composition, comprising the compound of formula (I) according to the first aspect of the present application, or a pharmaceutically acceptable salt thereof, or a stereoisomer thereof; and a pharmaceutically acceptable carrier.

A third aspect of the present application provides use of the compound of formula (I) according to the first aspect of the present application, or a pharmaceutically acceptable salt thereof, or a stereoisomer thereof, or the pharmaceutical composition according to the second aspect of the present application, in the manufacture of a medicament for preventing and/or treating an EED-mediated disease.

In some embodiments, the EED-mediated disease is a tumor or an autoimmune disease.

In some embodiments, the EED-mediated disease is cancer.

In some embodiments, the cancer is selected from the group consisting of multiple myeloma, leukemia, non-small cell lung cancer, colon cancer, central nervous system cancer, melanoma, ovarian cancer, renal cancer, prostate cancer and breast cancer.

A fourth aspect of the present application provides a method for preventing and/or treating an EED-mediated disease, comprising administering a therapeutically effective amount of the compound of formula (I) according to the first aspect of the present application, or a pharmaceutically acceptable salt thereof, or a stereoisomer thereof, or the pharmaceutical composition according to the second aspect of the present application to a subject.

In some embodiments, the EED-mediated disease is a tumor or an autoimmune disease.

In some embodiments, the EED-mediated disease is cancer.

In some embodiments, the cancer is selected from the group consisting of multiple myeloma, leukemia, non-small cell lung cancer, colon cancer, central nervous system cancer, melanoma, ovarian cancer, renal cancer, prostate cancer and breast cancer.

### BRIEF DESCRIPTION OF THE DRAWINGS

The accompanying drawings described herein are intended to provide a further understanding of the present application and constitute a part of the present application. The schematic embodiments and descriptions thereof in the present application are intended to explain the present application and do not constitute an undue limitation on the present application.
FIG. 1 is a graph showing the in vivo antitumor activity results of Compound D1 and Compound H105;
FIG. 2 is a graph showing the effect of Compound D1 and Compound H105 on mouse body weight.

### DETAILED DESCRIPTION

After extensive and in-depth research, the present inventors have unexpectedly discovered a bifunctional compound targeting degradation and/or inhibition of EED protein. This compound exhibits excellent EED protein degradation activity, excellent tumor cell (such as wsuDLCL-2 cells, Pfeiffer cells, etc.) proliferation inhibitory activity, excellent pharmacokinetic properties, excellent CYP450 profile, and good safety, making it more suitable for the treatment of diseases or conditions associated with abnormal EED protein activity (such as proliferative diseases like cancer). Based on this, the inventors have completed the present application.

### Definition of Terms

In order to more clearly understand the technical content of the present application, the terms of the present application are further described below.

The present application provides a bifunctional compound or PROTAC compound, which has a structure of POI-ULM or a structure of POI-L-ULM, wherein POI is a ligand targeting EED protein (or a ligand that binds to EED protein), ULM is an E3 ligase binding group (or a binding group), and L is a linker connecting POI and ULM. The PROTAC compound can bind to EED protein through the POI moiety, thereby bringing EED protein into proximity with an E3 ligase, thus inducing degradation (and/or inhibition) of EED protein. Commonly used E3 ligase ligands include VHL (Von Hippel-Lindau) E3 ubiquitin ligase binding group (abbreviated as VLM), CRBN (cereblon) E3 ubiquitin ligase binding group (abbreviated as CLM), MDM2 (mouse double minute 2 homologue) E3 ubiquitin ligase binding group (abbreviated as MLM), cIAP (cellular inhibitor of apoptosis) E3 ubiquitin ligase binding group (abbreviated as ILM), and the like. POI is a ligand targeting EED protein and can bind to EED protein.

"Alkyl" refers to linear and branched saturated aliphatic hydrocarbon groups. "C₁₋₁₀ alkyl" refers to an alkyl group having 1 to 10 carbon atoms, preferably C₁₋₈ alkyl; more preferably C₁₋₆ alkyl; further preferably C₁₋₃ alkyl. Non-limiting examples of alkyl include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof.

"Alkenyl" refers to linear or branched unsaturated aliphatic hydrocarbon groups having one or more carbon-carbon double bonds (C=C). "C₂₋₁₀ alkenyl" refers to an alkenyl group having 2 to 10 carbon atoms, preferably C₂₋₈ alkenyl, more preferably C₂₋₆ alkenyl, further preferably C₂₋₄ alkenyl, with a similar definition. Non-limiting examples of alkenyl include vinyl, propenyl, isopropenyl, n-butenyl, isobutenyl, pentenyl, hexenyl, and the like.

"Alkynyl" refers to linear and branched unsaturated aliphatic hydrocarbon groups having one or more carbon-carbon triple bonds. "C₂₋₁₀ alkynyl" refers to an alkynyl group having 2 to 10 carbon atoms, preferably C₂₋₈ alkynyl, more preferably C₂₋₆ alkynyl, further preferably C₂₋₄ alkynyl, with a similar definition. Non-limiting examples of alkynyl include ethynyl, propynyl, n-butynyl, isobutynyl, pentynyl, hexynyl, and the like.

"Alkylene" is divalent and requires two binding partners. Formally, the second valence is generated by removing a hydrogen atom from an alkyl group, such as -CH₃ and -CH₂-, -CH₂CH₃ and -CH₂CH₂- or -CH(CH₃)-, and the like. In certain embodiments, C₁₋₁₀ alkylene is preferred, more preferably C₁₋₈ alkylene, further preferably C₁₋₆ alkylene, most preferably C₁₋₃ alkylene. For example, C₁₋₃ alkylene includes -CH₂-, -(CH₂)₂-, -CH(CH₃)-, -(CH₂)₃-, -CH(CH₂CH₃)-, -CH₂CH(CH₃)- and -C(CH₃)₂-. In certain embodiments, alkylene can be methylene, ethylene, propylene, 1-methylethylene, butylene, 1-methylpropylene1,1-dimethylethylene, 1,2-dimethylethylene, pentylene, 1,1-dimethylpropylene, 2,2-dimethylpropylene, 1,2-dimethylpropylene, 1,3-dimethylpropylene, and the like. In the absence of any further definition, the generic terms propylene, butylene, pentylene, hexylene, etc., refer to all conceivable isomeric forms having the corresponding number of carbon atoms, i.e., propylene includes 1-methylethylene, and butylene includes 1-methylpropylene, 2-methylpropylene, 1,1-dimethylethylene and 1,2-dimethylethylene.

"Alkenylene" consists of at least two carbon atoms, wherein at least two adjacent carbon atoms are connected by a C-C double bond, and the carbon atoms can be part of only one C-C double bond. Formally, in the alkylene defined above, if two hydrogen atoms on adjacent carbon atoms are formally removed and the free valences are saturated to form a second bond, the corresponding alkenylene is formed. In certain embodiments, C₂₋₁₀ alkenylene is preferred, more preferably C₂₋₈ alkenylene, further preferably C₂₋₆ alkenylene, most preferably C₂₋₄ alkenylene. In certain embodiments, alkenylene can be ethenylene, propenylene, 1-methylethenylene, butenylene, 1-methylpropenylene, 1,1-dimethylethenylene, 1,2-dimethylethenylene, pentenylene, 1,1-dimethylpropenylene, 2,2-dimethylpropenylene, 1,2-dimethylpropenylene, 1,3-dimethylpropenylene, hexenylene, and the like. In the absence of any further definition, the generic terms propenylene, butenylene, pentenylene, hexenylene, etc., refer to all conceivable isomeric forms having the corresponding number of carbon atoms, i.e., propenylene includes 1-methylpropenylene, and butenylene includes 1-methylpropenylene, 2-methylpropenylene, 1,1-dimethylethenylene and 1,2-dimethylethenylene. For one or more double bonds, alkenylene may optionally exist in cis or trans, or E or Z forms.

"Alkynylene" consists of at least two carbon atoms, wherein at least two adjacent carbon atoms are connected by a C-C triple bond. Formally, in the alkylene defined above, if two hydrogen atoms are removed from two adjacent carbon atoms and the free valences are saturated to form two additional bonds, the corresponding alkynylene is formed. In certain embodiments, C₂₋₁₀ alkynylene is preferred, more preferably C₂₋₈ alkynylene, further preferably C₂₋₆ alkynylene, most preferably C₂₋₄ alkynylene. In certain embodiments, alkynylene can be ethynylene, propynylene, 1-methylethynylene, butynylene, 1-methylpropynylene, 1,1-dimethylethynylene, 1,2-dimethylethynylene, pentynylene, 1,1-dimethylpropynylene, 2,2-dimethylpropynylene, 1,2-dimethylpropynylene, 1,3-dimethylpropynylene, hexynylene, and the like. In the absence of any further definition, the generic terms propynylene, butynylene, pentynylene, hexynylene, etc., refer to all conceivable isomeric forms having the corresponding number of carbon atoms, i.e., propynylene includes 1-methylethynylene, and butynylene includes 1-methylpropynylene, 2-methylpropynylene, 1,1-dimethylethynylene and 1,2-dimethylethynylene.

"Alkyleneoxy" refers to a divalent alkoxy group. In certain embodiments, C₁₋₁₀ alkyleneoxy is preferred, more preferably C₁₋₈ alkyleneoxy, further preferably C₁₋₆ alkyleneoxy, most preferably C₁₋₃ alkyleneoxy. In certain embodiments, alkyleneoxy can be -OCH₂-, -OCH(CH₃)CH₂-, -OCH₂CH₂O-, -CH₂CH₂O-, and the like. In the absence of any further definition, the generic terms propyleneoxy, butyleneoxy, pentyleneoxy, hexyleneoxy, etc., refer to all conceivable isomeric forms having the corresponding number of carbon atoms, i.e., propyleneoxy includes -O(CH₂)₃O-, -O(CH₂)₃-, -OCH₂CH(CH₃)-, -OC(CH₃)₂-, -OCH(CH₃)CH₂-, -OCH₂CH(CH₃)O-, -OC(CH₃)₂O- and -OCH(CH₃)CH₂O-.

"Cycloalkyl" and "cycloalkyl ring" are used interchangeably and both refer to a monocyclic or polycyclic cyclic hydrocarbon group, which may be fused to an aryl or heteroaryl group. The cycloalkyl ring may be optionally substituted. In certain embodiments, the cycloalkyl ring includes one or more of spiro rings and bridged rings. In certain embodiments, the cycloalkyl ring contains one or more carbonyl groups, such as oxo groups. "C₃₋₁₅ cycloalkyl" refers to a monocyclic or polycyclic cycloalkyl group having 3 to 15 carbon atoms, such as spiro[4.5]decane, spiro[3.3]heptane, spiro[5.5]undecane, dispiro[5.2.59.26]hexadecane, decahydroazulene, 1,2-diethylcyclopent-l-ene, bicyclo[3.3.2]decane. Preferred are C₃₋₈ cycloalkyl such as cyclopropyl, cyclobutyl, cyclopentyl, cyclohexyl, cycloheptyl, cyclooctyl, cyclobutanone, cyclopentanone, cyclopentane-1,3-dione, and the like. Preferred are C₃₋₇ cycloalkyl, such as cycloheptane, spiro[3.3]heptane, etc., more preferably C₃₋₆ cycloalkyl, including cyclopropyl, cyclobutyl, cyclopentyl and cyclohexyl. It can be a saturated cycloalkyl, such as cyclohexyl, cyclopropyl, etc. It can be a partially unsaturated cycloalkyl, such as cyclohexene, 1,2-diethylcyclopent-1-ene, etc. "Spiro ring" refers to a polycyclic group in which single rings share one carbon atom (called the spiro atom), which may contain one or more double bonds, but no ring has a fully conjugated π-electron system. Spiro rings are classified as dispiro rings or polyspiro rings based on the number of rings, with dispiro rings being preferred. More preferred are 4-membered/5-membered, 5-membered/5-membered or 5-membered/6-membered dispiro rings. For example:

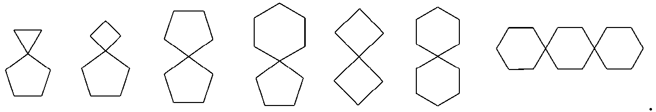

"Cycloalkylene" and "cycloalkylene ring" are used interchangeably, both are divalent and require two binding partners. Formally, the second valence is generated by removing a hydrogen atom from a cycloalkyl group, for example

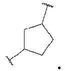

"Spiro-heterocycle" refers to a polycyclic hydrocarbon in which single rings share one atom (referred to as the spiro atom), wherein one or two ring atoms are heteroatoms selected from nitrogen, oxygen or S(O)n (wherein n is an integer from 0 to 2), and the remaining ring atoms are carbon, which may contain one or more double bonds, but no ring has a fully conjugated π-electron system. Spiroheterocycles are classified as dispiroheterocycles or polyspiroheterocycles based on the number of rings, with dispiroheterocycles being preferred. More preferred are 4-membered/5-membered, 5-membered/5-membered or 5-membered/6-membered dispiroheterocycles. For example:

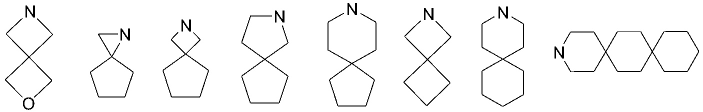

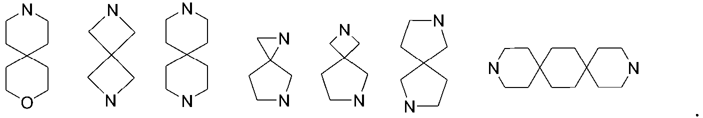

"Bridged ring" refers to a polycyclic group in which two or more carbon atoms are shared. The shared carbon atoms are referred to as bridgehead carbons, and between two bridgehead carbons there may be a carbon chain or a single bond, which is referred to as a bridge. These may contain one or more double bonds, but no ring has a fully conjugated π-electron system. Preferred are bicyclic or tricyclic bridged rings. For example:

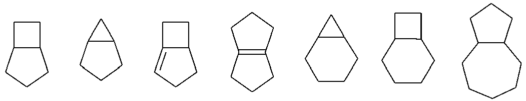

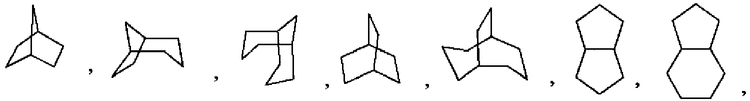

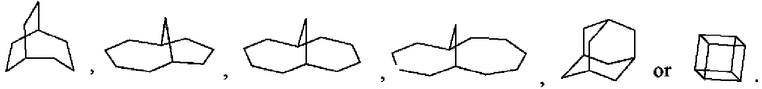

"Bridged heterocycle" refers to a polycyclic group in which two or more atoms are shared, wherein one or more ring atoms are heteroatoms selected from nitrogen, oxygen or S(O)n (wherein n is an integer from 0 to 2), and the remaining ring atoms are carbon. These may contain one or more double bonds, but no ring has a fully conjugated π-electron system. Preferred are bicyclic or tricyclic bridged heterocycles. For example:

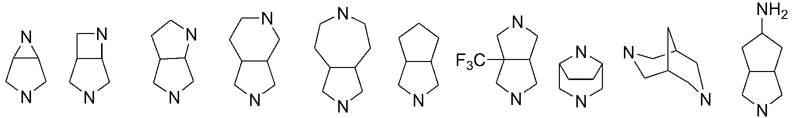

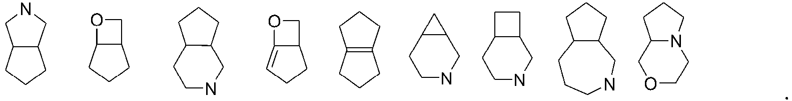

"Heterocycloalkyl" and "heterocycloalkyl ring" are used interchangeably, both refer to a cycloalkyl group containing at least one heteroatom selected from nitrogen, oxygen and sulfur, and the group may be fused to an aryl or heteroaryl group. The heterocycloalkyl ring may be a saturated heterocycloalkyl ring or a partially unsaturated heterocycloalkyl ring. The heterocycloalkyl ring may be optionally substituted. In certain embodiments, the heterocycloalkyl ring includes one or more of spiro-heterocycles and bridged heterocycles. In certain embodiments, the heterocycloalkyl ring contains one or more carbonyl or thiocarbonyl groups, such as groups containing oxo and thio. "3- to 15-membered heterocycloalkyl" refers to a heterocycloalkyl group having 3 to 15 ring atoms, wherein 1, 2 or 3 ring atoms are heteroatoms selected from nitrogen, oxygen and sulfur. Preferred are 3- to 10-membered heterocycloalkyl, more preferably 3- to 7-membered heterocycloalkyl, further more preferably 3- to 7-membered heterocycloalkyl, further preferably 3- to 6-membered heterocycloalkyl. Non-limiting examples of heterocycloalkyl include aziridinyl, oxiranyl, azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholine-1,1-dioxide, tetrahydropyranyl, azetidin-2-onyl, oxetan-2-onyl, dihydrofuran-2(3H)-onyl, pyrrolidin-2-onyl, pyrrolidine-2,5-dionyl, dihydrofuran-2,5-dionyl, piperidin-2-onyl, tetrahydro-2H-pyran-2-onyl, piperazin-2-onyl, morpholin-3-onyl, 2,3-dihydrofuran, 2,5-dihydrofuran, 2,5-dihydro-1H-pyrrole, 1,2,3,4-tetrahydropyridine, 1,2,3,6-tetrahydropyridine, and the like.

"Heterocycloalkylene" and "heterocycloalkylene ring" are used interchangeably, both are divalent and require two binding partners. Formally, the second valence is generated by removing a hydrogen atom from a cycloalkyl group, for example

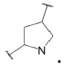

"Aryl" and "aryl ring" are used interchangeably, both refer to a group of a monocyclic, bicyclic or polycyclic 4n+2 aromatic ring system having ring carbon atoms (e.g., having 6 or 10, or 14 π electrons shared in a cyclic arrangement). In the present application, the aryl ring may be optionally substituted. "C₆₋₁₄ aryl" refers to an aryl group having 6 to 14 ring carbon atoms. "C₆₋₁₀ aryl" refers to an aryl group having 6 to 10 ring carbon atoms. Non-limiting examples include phenyl, naphthyl and anthracenyl.

"Arylene" and "arylene ring" are used interchangeably, both are divalent and require two binding partners. Formally, the second valence is generated by removing a hydrogen atom from a cycloalkyl group, for example

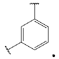

"Heteroaryl" and "heteroaryl ring" are used interchangeably, both refer to a group of a monocyclic, bicyclic or polycyclic 4n+2 aromatic ring system having ring carbon atoms and ring heteroatoms (e.g., having 6 or 10 or 14 π electrons shared in a cyclic arrangement), wherein each heteroatom is independently selected from nitrogen, oxygen and sulfur. In the present application, heteroaryl also includes ring systems in which the aforementioned heteroaryl ring is fused to one or more cycloalkyl rings, heterocycloalkyl rings, cycloalkenyl rings, heterocycloalkenyl rings or aryl rings. The heteroaryl ring may be optionally substituted. "5- to 15-membered heteroaryl" refers to a monocyclic heteroaryl having 5 to 15 ring atoms, wherein 1, 2, 3 or 4 ring atoms are heteroatoms. "5-to 6-membered heteroaryl" refers to a monocyclic heteroaryl having 5 to 6 ring atoms, wherein 1, 2, 3 or 4 ring atoms are heteroatoms. Non-limiting examples include thienyl, furyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridinyl, pyridazinyl, pyrimidinyl, pyrazinyl, triazinyl, tetrazinyl, quinolinyl, isoquinolinyl, benzopyrrolyl, benzofuranyl, benzothienyl, benzopyrazolyl, benzimidazolyl, benzotriazolyl, benzotetrazolyl, benzoxazolyl, benzisoxazolyl, benzoxadiazolyl, benzothiazolyl, benzothiadiazolyl, pyridopyrrolyl, pyridofuranyl, pyridothienyl, pyridopyrazolyl, pyridoimidazolyl, pyridotriazolyl, pyridotetrazolyl, pyridooxazolyl, pyridoisoxazolyl, pyridooxadiazolyl, pyridothiazolyl, pyridothiadiazolyl, pyrimidopyrrolyl, pyrimidofuranyl, pyrimidothienyl, pyrimidopyrazolyl, pyrimidoimidazolyl, pyrimidotriazolyl, pyrimidotetrazolyl, pyrimidooxazolyl, pyrimidoisoxazolyl, pyrimidooxadiazolyl, pyrimidothiazolyl, pyrimidothiadiazolyl, pyridazopyrrolyl, pyridazofuranyl, pyridazothienyl, pyridazopyrazolyl, pyridazoimidazolyl, pyridazotriazolyl, pyridazotetrazolyl, pyridazooxazolyl, pyridazoisoxazolyl, pyridazooxadiazolyl, pyridazothiazolyl, pyridazothiadiazolyl, pyrazinopyrrolyl, pyrazinofuranyl, pyrazinothienyl, pyrazinopyrazolyl, pyrazinoimidazolyl, pyrazinotriazolyl, pyrazinotetrazolyl, pyrazinooxazolyl, pyrazinoisoxazolyl, pyrazinooxadiazolyl, pyrazinothiazolyl, pyrazinothiadiazolyl, triazinopyrrolyl, triazinofuranyl, triazinothienyl, triazinopyrazolyl, triazinoimidazolyl, triazinotriazolyl, triazinotetrazolyl, triazinooxazolyl, triazinoisoxazolyl, triazinooxadiazolyl, triazinothiazolyl, triazinothiadiazolyl, benzoquinolinyl, benzoisoquinolinyl and carbazolyl. "Heteroatom" refers to nitrogen, oxygen or sulfur. In a heteroaryl containing one or more nitrogen atoms, the point of attachment may be a carbon or nitrogen atom, provided that the valence allows. A heteroaryl bicyclic system may include one or more heteroatoms in one or two rings.

"Heteroarylene" and "heteroarylene ring" are used interchangeably, both are divalent and require two binding partners. Formally, the second valence is generated by removing a hydrogen atom from a cycloalkyl group, for example

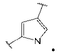

"Halogen" refers to fluorine (F), chlorine (Cl), bromine (Br) or iodine (I).

"Halo" refers to the substitution of one or more (e.g., 1, 2, 3 or all) hydrogen atoms in a group with halogen.

"Haloalkyl" refers to an alkyl group substituted with one or more (e.g., 1, 2, 3 or all) halogen atoms, wherein alkyl is as defined above. Preferred are C₁₋₈ haloalkyl, more preferably C₁₋₆ haloalkyl, even more preferably C₁₋₃ haloalkyl. Examples of haloalkyl include, but are not limited to, chloromethyl, dichloromethyl, trichloromethyl, chloroethyl, 1,2-dichloroethyl, trichloroethyl, bromoethyl, fluoromethyl, difluoromethyl, trifluoromethyl, fluoroethyl, difluoroethyl, trifluoroethyl, and the like.

"Alkoxy" refers to -O-alkyl, wherein alkyl is as defined above. Preferred are C₁₋₈ alkoxy, more preferably C₁₋₆ alkoxy, most preferably C₁₋₃ alkoxy. Non-limiting examples of alkoxy include methoxy, ethoxy, n-propoxy, isopropoxy, butoxy, tert-butoxy, isobutoxy, pentyloxy, and the like.

"Alkoxyalkyl" refers to an alkyl group substituted with one or more alkoxy groups, wherein alkyl and alkoxy are as defined above. Preferred are C₁₋₆ alkoxy C₁₋₆ alkyl, more preferably C₁₋₃ alkoxy C₁₋₃ alkyl. Non-limiting examples of alkoxyalkyl include -CH₂OCH₃, -CH₂OCH₂CH₃, -CH₂CH₂OCH₃, and the like.

"Cycloalkylalkyl" refers to an alkyl group substituted with one or more cycloalkyl groups, wherein alkyl and cycloalkyl are as defined above. Preferred are C₃₋₆ cycloalkyl C₁₋₆ alkyl, more preferably C₃₋₆ cycloalkyl C₁₋₃ alkyl. Non-limiting examples of cycloalkylalkyl include -CH₂-cyclopropyl, -CH₂-cyclobutyl, -CH₂-cyclobutyl, and the like.

"Alkenylalkyl" refers to an alkyl group substituted with one or more alkenyl groups, wherein alkyl and alkenyl are as defined above. Preferred are C₂₋₈ alkenyl C₁₋₁₀ alkyl, more preferably C₂₋₈ alkenyl C₁₋₈ alkyl, further preferably C₂₋₈ alkenyl C₁₋₆ alkyl, further preferably C₂₋₈ alkenyl C₁₋₃ alkyl. Non-limiting examples of cycloalkylalkyl include -H₂C-HC=CH₂, -CH₂CH₂-HC=CH₂, 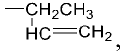 and the like.

"Alkynylalkyl" refers to an alkyl group substituted with one or more alkynyl groups, wherein alkyl and alkynyl are as defined above. Preferred are C₂₋₈ alkynyl C₁₋₁₀ alkyl, more preferably C₂₋₈ alkynyl C₁₋₈ alkyl, further preferably C₂₋₈ alkynyl C₁₋₆ alkyl, further preferably C₂₋₈ alkynyl C₁₋₃ alkyl. Non-limiting examples of cycloalkylalkyl include , -H₂C-C≡CH, -CH₂CH₂-C≡CH, 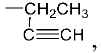 and the like.

"Heterocycloalkylalkyl" refers to an alkyl group substituted with one or more heterocycloalkyl groups, wherein alkyl and heterocycloalkyl are as defined above. Preferred are 4- to 10-membered heterocycloalkyl C₁₋₆ alkyl, more preferably 4- to 8-membered heterocycloalkyl C₁₋₃ alkyl, further preferably 3- to 6-membered heterocycloalkyl C₁₋₃ alkyl, further preferably 4- to 6-membered heterocycloalkyl C₁₋₃ alkyl. Non-limiting examples of heterocycloalkylalkyl include -CH₂-tetrahydropyrrolyl, -CH₂-azetidinyl, -CH₂-piperidinyl, -CH₂-piperazinyl, and the like.

"Hydroxy-substituted alkyl" refers to an alkyl group substituted with one or more hydroxyl groups, wherein alkyl is as defined above. Preferred are hydroxy-substituted C₁₋₁₀ alkyl, more preferably hydroxy-substituted C₁₋₈ alkyl, further preferably hydroxy-substituted C₁₋₆ alkyl, even more preferably hydroxy-substituted C₁₋₃ alkyl. Non-limiting examples of "hydroxy-substituted alkyl" include -CH₂OH, -CH₂CH₂OH, -CH(OH)CH₃, and the like.

"Cyano-substituted alkyl" refers to an alkyl group substituted with one or more cyano groups, wherein alkyl is as defined above. Preferred are cyano-substituted C₁₋₁₀ alkyl, more preferably cyano-substituted C₁₋₈ alkyl, further preferably cyano-substituted C₁₋₆ alkyl, further preferably cyano-substituted C₁₋₃ alkyl. Non-limiting examples of "cyano-substituted alkyl" include -CH₂CN, -CH₂CH₂CN, -CH(CN)CH₃, and the like.

"Carboxy-substituted alkyl" refers to an alkyl group substituted with one or more carboxy groups, wherein alkyl is as defined above. Preferred are carboxy-substituted C₁₋₁₀ alkyl, more preferably carboxy-substituted C₁₋₈ alkyl, further preferably carboxy-substituted C₁₋₆ alkyl, further preferably carboxy-substituted C₁₋₃ alkyl. Non-limiting examples of "carboxy-substituted alkyl" include -CH₂COOH, -CH₂CH₂COOH, -CH(COOH)CH₃, and the like.

"Amino-substituted alkyl" refers to an alkyl group substituted with one or more amino groups, wherein alkyl is as defined above. Preferred are amino-substituted C₁₋₁₀ alkyl, more preferably amino-substituted C₁₋₈ alkyl, further preferably amino-substituted C₁₋₆ alkyl, further preferably amino-substituted C₁₋₃ alkyl. Non-limiting examples of "amino-substituted alkyl" include -CH₂NH₂, -CH₂CH₂NH₂, -CH(NH₂)CH₃, and the like.

"Haloalkoxy" refers to an alkoxy group substituted with one or more (e.g., 1, 2, 3, 4 or 5) halogen atoms, wherein alkoxy is as defined above. Preferred are C₁₋₁₀ haloalkoxy, more preferably C₁₋₈ haloalkoxy, further preferably C₁₋₆ haloalkoxy, further preferably C₁₋₃ haloalkoxy. Haloalkoxy includes, but is not limited to, trifluoromethoxy, trifluoroethoxy, monofluoromethoxy, monofluoroethoxy, difluoromethoxy, difluoroethoxy, and the like.

"Amino" refers to -NH₂, "cyano" refers to -CN, "nitro" refers to -NO₂, "benzyl" refers to -CH₂-phenyl, "oxo" refers to =O, "carboxy" refers to -C(O)OH, "acetyl" refers to -C(O)CH₃, "acetamido" refers to -C(O)NH₂, "hydroxymethyl" refers to -CH₂OH, "hydroxyethyl" refers to -CH₂CH₂OH or -CHOHCH₃, "hydroxyl" refers to -OH, "thiol" refers to -SH; "formyl" refers to -CHO; "sulfo" refers to -SO₃H.

As defined in the present application, "when R₂ or R₃ is X₁, X₁ is the point of attachment of POI to L or ULM" means that POI is connected to L or ULM via a covalent connection, and X₁ is the point of attachment to L or ULM. For the compound of formula (I), when POI is structure 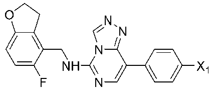 and X₁ represents the point of attachment to L or ULM, its meaning is the same as that expressed by 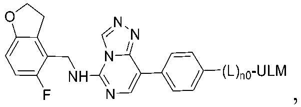 when n₀ is 0 or 1. Similarly, for the compound of formula (I), when ULM is 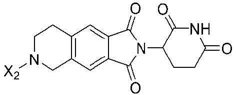 wherein X₂ is the point of attachment of ULM to L or POI, its meaning is the same as that expressed by 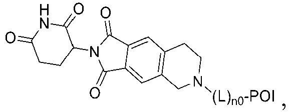 when n₀ is 0 or 1.

"Substituted" means that one or more hydrogen atoms in a group, preferably 1 to 5 hydrogen atoms, are independently replaced by a corresponding number of substituents, more preferably 1 to 3 hydrogen atoms are independently replaced by a corresponding number of substituents. It goes without saying that substituents are only present at their chemically possible positions, and a person skilled in the art can determine (through experimentation or theory) possible or impossible substitutions without undue effort. For example, an amino or hydroxyl group having a free hydrogen may be unstable when bonded to a carbon atom having an unsaturated (e.g., ethylenic) bond.

When the number of substituents is not indicated in this application, it means that substitution can be carried out with an optional substitutable number of substituents.

Unless otherwise defined, "substituents each independently selected from ..." in this application means that when more than one hydrogen on a group is replaced by substituents, the types of said substituents can be the same or different, and the selected substituents are each independent types.

Unless otherwise defined, any group herein can be substituted or unsubstituted. When the above group is substituted, the substituents are preferably 1 to 5 groups independently selected from the group consisting of deuterium, halogen (preferably fluorine, chlorine), cyano, hydroxyl, carboxyl, C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), C₂₋₄ alkenyl, C₂₋₄ alkynyl, C₁₋₈ haloalkyl (preferably C₁₋₆ haloalkyl, more preferably C₁₋₃ haloalkyl), cyano-substituted C₁₋₈ alkyl (preferably cyano-substituted C₁₋₆ alkyl, more preferably cyano-substituted C₁₋₃ alkyl), C₁₋₈ haloalkoxy (preferably C₁₋₆ haloalkoxy, more preferably C₁₋₃ haloalkoxy), NR_{A0}R_{B0}, -SO₂C₁₋₃ alkyl, -S(O)C₁₋₃ alkyl, -C(O)NR_{A10}R_{B10}, -C(O)C₁₋₈ alkyl (preferably -C(O)C₁₋₆ alkyl, more preferably -C(O)C₁₋₃ alkyl), -C(O)OC₁₋₈ alkyl (preferably -C(O)OC₁₋₆ alkyl, more preferably -C(O)OC₁₋₃ alkyl), -OC(O)C₁₋₈ alkyl (preferably -OC(O)C₁₋₆ alkyl, more preferably -OC(O)C₁₋₃ alkyl), C₃₋₆ cycloalkyl, C₃₋₆ cycloalkyloxy, 3- to 6-membered heterocycloalkyl, phenyl and 5- to 6-membered heteroaryl; wherein the 3- to 6-membered heterocycloalkyl, phenyl and 5- to 6-membered heteroaryl in the substituents are unsubstituted or substituted with 1, 2 or 3 groups each independently selected from the group consisting of halogen, cyano, hydroxyl, carboxyl, C₁₋₃ alkyl, C₁₋₃ alkoxy, C₂₋₄ alkenyl, C₂₋₄ alkynyl, C₁₋₃ haloalkyl, C₁₋₃ haloalkoxy, NR_{A0}R_{B0}, -SO₂C₁₋₃ alkyl, -S(O)C₁₋₃ alkyl, -C(O)NR_{A10}R_{B10}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl.

R_{A10} and R_{B10} are each independently hydrogen or C₁₋₃ alkyl; or R_{A10}, R_{B10} and nitrogen atom linked to R_{A10} and R_{B10} together form a 4- to 6-membered saturated monocyclic heterocycle; the 4- to 6-membered saturated monocyclic heterocycle is unsubstituted or substituted with 1, 2 or 3 substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, C₁₋₃ haloalkyl, C₁₋₃ haloalkoxy, -SO₂C₁₋₃ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl;

R_{A0} and R_{B0} are each independently hydrogen, C₁₋₃ alkyl or acetyl; or R_{A0}, and R_{B0} and nitrogen atom linked to R_{A0} and R_{B0} together form a 4- to 6-membered saturated monocyclic heterocycle; the 4-to 6-membered saturated monocyclic heterocycle is unsubstituted or substituted with 1, 2 or 3 substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, C₁₋₃ haloalkyl, C₁₋₃ haloalkoxy, -SO₂C₁₋₃ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

In this application, when two or more "preferably" appear in one scheme, any two "preferably" can be independent of each other.

In this application, when the number of substituents is greater than 1, any two substituents can be the same or different. For example, it can be two identical or different halogen substitutions, or one halogen and one hydroxy substitution.

Each of the various substituent groups described above can itself be substituted with the groups described herein.

### Pharmaceutical Composition

Generally, the compound of the present application or a pharmaceutically acceptable salt thereof, or a stereoisomer thereof, can be formulated into a suitable dosage form with one or more pharmaceutical carriers for administration. These dosage forms are suitable for oral, rectal, topical, buccal administration, and other parenteral administration (e.g., subcutaneous, intramuscular, intravenous, etc.). For example, dosage forms suitable for oral administration include capsules, tablets, granules, syrups, and the like.

"Pharmaceutically acceptable carrier" refers to a non-toxic, inert, solid, semi-solid substance or liquid filler, diluent, encapsulating material, or auxiliary formulation, or any type of excipient, which is compatible with the patient, preferably a mammal, more preferably a human, and is suitable for delivering the active agent to the target site without terminating the activity of the agent.

The compositions of the present application are formulated, dosed, and administered in a manner consistent with good medical practice. A "therapeutically effective amount" of the compound to be administered is determined by factors such as the specific condition being treated, the individual being treated, the cause of the condition, the target of the drug, and the mode of administration.

"Therapeutically effective amount" refers to an amount of the compound of the present application that will elicit a biological or medical response in an individual, for example, reducing or inhibiting enzyme or protein activity, or ameliorating symptoms, alleviating conditions, slowing or delaying disease progression, or preventing disease, etc.

"Patient" refers to an animal, preferably a mammal, more preferably a human. The term "mammal" refers to warm-blooded vertebrate mammals, including, for example, cats, dogs, rabbits, bears, foxes, wolves, monkeys, deer, mice, pigs, and humans.

"Treatment" refers to alleviating, delaying progression, attenuating, preventing, or maintaining an existing disease or condition (e.g., cancer). Treatment also includes curing one or more symptoms of a disease or condition, preventing its development, or alleviating it to some extent.

"Pharmaceutically acceptable salt" includes pharmaceutically acceptable acid addition salts and pharmaceutically acceptable base addition salts.

"Pharmaceutically acceptable acid addition salt" refers to a salt formed with an inorganic or organic acid that retains the biological effectiveness of the free base without other side effects.

"Pharmaceutically acceptable base addition salt" includes, but is not limited to, salts of inorganic bases and salts of organic bases.

The compounds of the present application may contain one or more chiral centers and exist in different optically active forms. When the compound contains one chiral center, the compound includes enantiomers. When the compound contains more than one chiral center, diastereomers may exist. The present application includes both of these stereoisomers as well as mixtures of these two stereoisomers, such as racemates, diastereomeric mixtures, and the like. Unless otherwise indicated, a wedge bond 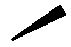 or 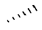 is used to indicate the absolute configuration of a stereocenter. When the compounds described herein contain olefinic double bonds or other geometric asymmetry centers, unless otherwise specified, they include E and Z geometric isomers. Likewise, all tautomeric forms are included within the scope of the present application.

The enantiomers, diastereomers, and mixtures of these isomers of the compounds of the present application are within the protection scope of the present invention. Enantiomers and diastereomers can be resolved by methods known in the art, such as crystallization and chiral chromatography.

### Preparation Methods

The present application provides methods for preparing the compounds of formula (I). The compounds of formula (I) can be synthesized using standard synthetic techniques known to those skilled in the art, or using methods known in the art in combination with the methods described herein. The solvents, temperatures, and other reaction conditions given herein can be varied according to the knowledge in the art. The reactions can be used sequentially to provide the compounds of the present application, or they can be used to synthesize fragments that are subsequently added by methods described herein and/or known in the art.

For the reactions in the above schemes, a person skilled in the art can make adaptive adjustments with reference to the specific examples described in this application or based on existing literature, according to the properties of the participating reactants, without causing difficulty to those skilled in the art.

The compounds described in this application can be synthesized using methods analogous to those described below or the exemplary methods in the Examples, or relevant published literature used by those skilled in the art, by using appropriate alternative starting materials. Starting materials or intermediates used to synthesize the compounds described in this application can be synthesized or obtained from commercial sources. If not reported in existing literature or not available commercially, they can be prepared using similar existing preparation methods for analogous compounds or similar preparation methods described in this application. The compounds described in this application and other related compounds with different substituents can be synthesized using techniques and starting materials known to those skilled in the art. General methods for preparing the compounds disclosed in this application can be derived from reactions known in the art, and the reactions can be modified by reagents and conditions deemed appropriate by those skilled in the art to introduce various moieties into the molecules provided herein.

Compared with the prior art, the main advantages of the present application are: the compound of the present application have excellent EED protein degradation effects and tumor cell proliferation inhibitory effects, and have excellent pharmacokinetic properties, excellent effects on cytochrome P450, good safety, and are more suitable for treating diseases or conditions with abnormal EED protein activity (such as proliferative diseases like cancer) .

The present application is further described below in conjunction with specific examples. It should be understood that these examples are only intended to illustrate the present application and are not intended to limit the scope of the present application. In the following examples, where specific conditions are not indicated for the experimental methods, they were usually carried out under conventional conditions such as those described in Sambrook et al., Molecular Cloning: A Laboratory Manual (New York: Cold Spring Harbor Laboratory Press, 1989), or according to the conditions recommended by the manufacturer. Unless otherwise indicated, percentages and parts are calculated by weight. Unless otherwise defined, terms used herein have the same meanings as those familiar to those skilled in the art. In addition, any methods and materials similar or equivalent to those described can be applied in the present application.

### Reagents and Instruments

¹H NMR: Bruker AVANCE-400 nuclear magnetic resonance instrument, with tetramethylsilane (TMS) as internal standard.

LC-MS: Agilent 1290 HPLC System/6130/6150 MS LC-MS mass spectrometer (manufacturer: Agilent), column Waters BEH/CHS, 50 × 2.1 mm, 1.7 µm.

Preparative High-Performance Liquid Chromatography (pre-HPLC): GX-281 (manufacturer: Gilson).

ISCO Combiflash-Rf75 or Rf200 automatic column chromatography instrument was used, with Agela 4 g, 12 g, 20 g, 40 g, 80 g, 120 g disposable silica gel columns.

Known starting materials can be synthesized using or according to methods known in the art, or can be purchased from companies such as ABCR GmbH & Co. KG, Acros Organics, Aldrich Chemical Company, Accela ChemBio Inc., and Darui Chemicals.

In the examples, the progress of reactions can be monitored using thin layer chromatography (TLC), and compound purification can be performed using column chromatography. The developing solvent systems used for column chromatography or TLC can be selected from dichloromethane and methanol systems, n-hexane and ethyl acetate systems, petroleum ether and ethyl acetate systems, and acetone systems, etc. The volume ratio of solvents is adjusted according to the polarity of the compound.

As used herein, PE: petroleum ether, EA: ethyl acetate, THF: tetrahydrofuran, H₂O: water, DMF: N,N-dimethylformamide, DCM: dichloromethane, MeOH: methanol, EtOH: ethanol, DMSO: dimethyl sulfoxide, DCE: 1,2-dichloroethane, NMP: N-methylpyrrolidone, DME: dimethyl ether, DMAC: N,N-dimethylacetamide, TFA: trifluoroacetic acid, FA: formic acid, AcOH: acetic acid, CH₃COOH: acetic acid, SOCl₂: thionyl chloride, POCl₃: phosphorus oxychloride, PCy₃: tricyclohexylphosphine, Et₃N: triethylamine, LiOH: lithium hydroxide, LiAlH₄: lithium aluminum hydride, NaOH: sodium hydroxide, NaHCO₃: sodium bicarbonate, NaH: sodium hydride, KOAc: potassium acetate, K₂CO₃: potassium carbonate, Cs₂CO₃: cesium carbonate, NH₄HCO₃: ammonium bicarbonate, TCFH: N,N,N',N'-tetramethylchloroformamidinium hexafluorophosphate, HATU: 2-(7-azabenzotriazol-1-yl)-N,N,N',N'-tetramethyluronium hexafluorophosphate, HOBT: 1-hydroxybenzotriazole, EDCI: 1-(3-dimethylaminopropyl)-3-ethylcarbodiimide, Pd(dppf)Cl₂: [1,1'-bis(diphenylphosphino)ferrocene] dichloropalladium(II); DIPEA: N,N-diisopropylethylamine, mCPBA: meta-chloroperoxybenzoic acid, Pd(PPh₃)₄: tetrakis(triphenylphosphine)palladium(0), Pd₂(dba)₃: tris(dibenzylideneacetone) dipalladium(0), Pd(dba)₂: bis(dibenzylideneacetone)palladium(0), Pd(OAc)₂: palladium(II) acetate, Xantphos: 4,5-bis(diphenylphosphino)-9,9-dimethylxanthene, NIS: N-iodosuccinimide, NBS: N-bromosuccinimide, CuI: copper(I) iodide, PPh₃: triphenylphosphine, DEAD: diethyl azodicarboxylate, NaBH₃CN: sodium cyanoborohydride, NaBH(OAc)₃: sodium triacetoxyborohydride, B₂Pin₂: bis(pinacolato)diboron, X-Phos: 2-dicyclohexylphosphino-2',4',6'-triisopropylbiphenyl, Selectfluor: 1-chloromethyl-4-fluoro-1,4-diazoniabicyclo[2.2.2]octane bis(tetrafluoroborate), Ruphos-Pd-G₃: methanesulfonato(2-dicyclohexylphosphino-2',6'-diisopropoxy-1,1'-biphenyl)(2'-amino-1,1'-biphenyl-2-yl)palladium(II), DIBAL-H: diisobutylaluminum hydride, DIEA: N,N-diisopropylethylamine, DMAP: 4-dimethylaminopyridine, Ruphos: 2-dicyclohexylphosphino-2',6'-diisopropoxy-1,1'-biphenyl, LiHMDS: lithium bis(trimethylsilyl)amide, TEA: triethylamine, MeCN: acetonitrile, Boc₂O: di-tert-butyl dicarbonate, Dess-Martin periodinane: Dess-Martin periodinane, (Pin)₂B₂: bis(pinacolato)diboron, n-BuLi: n-butyllithium, Pd(PPh₃)₂Cl₂ bis(triphenylphosphine)palladium(II) dichloride, t-BuOH: tert-butanol, DIAD: diisopropyl azodicarboxylate, TBSCl: tert-butyldimethylchlorosilane, CataCXium A: n-butylbis(1-adamantyl)phosphine, RhCl(PPh₃)₃: chlorotris(triphenylphosphine)rhodium(I), TsOH: p-toluenesulfonic acid, PtO₂: platinum(IV) oxide, Pd(aMphos)Cl₂: dichlorobis(di-tert-butyl(4-dimethylaminophenyl)phosphine)palladium(II).

Regarding the percentage contents involved herein, unless otherwise specified, for solid-liquid mixtures and solid-solid mixtures, they refer to mass percentages; for liquid-liquid mixtures, they refer to volume percentages. Unless otherwise specified, the solvent is water.

As used herein, r.t. indicates room temperature, which refers to approximately 20°C to 30°C.

As used herein, "overnight" means approximately 10 h to 16 h.

### Preparation of Intermediate Z1

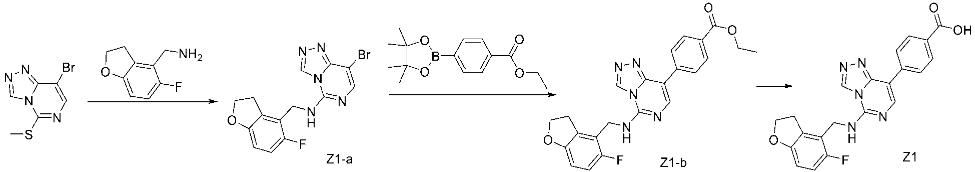

Step 1: 8-Bromo-5-(methylthio)-[1,2,4]triazolo[4,3-c]pyrimidine (5 g, 20.40 mmol) and (5-fluoro-2,3-dihydrobenzofuran-4-yl)methanamine (5 g, 29.91 mmol) were mixed, pre-sonicated for 5 minutes, and then stirred at 85°C for 3 hours under microwave irradiation. After the reaction was complete, DCM (100 mL) was added, followed by sonication and filtration. The filter cake was dried naturally to give Z1-a (5.5 g), yield: 50.50%. MS m/z (ESI): 364 [M+H]⁺, 366 [M+H]⁺.

Step 2: Z1-a (300 mg, 823.8 µmol) and ethyl 4-(4,4,5,5-tetramethyl-1,3,2-dioxaborolan-2-yl)benzoate (455 mg, 1.65 mmol) were mixed and added to an aqueous 1,4-dioxane solution (5 mL, 20%). Then K₂CO₃ (342 mg, 2.47 mmol) and catalyst Pd(dppf)Cl₂ (73.1 mg, 0.1 mmol) were added. Under a nitrogen atmosphere, the mixture was heated to 100°C and stirred for 12 hours. After the reaction was complete, the reaction mixture was purified by column chromatography (PE/EA = 15% to 100%) to give Z1-b (200 mg), yield: 56.10%. MS m/z (ESI): 434 [M+H]⁺.

Step 3: Z1-b (200 mg, 0.46 mmol) was added to a THF/H₂O solution (2 mL, 50%). Then LiOH (40 mg) was added, and the mixture was stirred at room temperature for 12 hours. After the reaction was complete, the pH was adjusted to neutrality with dilute hydrochloric acid (2 mol/L), followed by purification by column chromatography (DCM/MeOH = 0% to 10%) to give intermediate Z1 (160 mg), yield: 86.9%. MS m/z (ESI): 406 [M+H]⁺.

### Preparation of Intermediate Z2

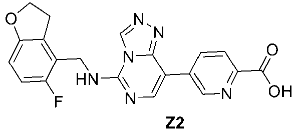

Referring to the method in Preparation of Intermediate Z1, ethyl 4-(4,4,5,5-tetramethyl-1,3,2-dioxaborolan-2-yl)benzoate was replaced with methyl 5-(4,4,5,5-tetramethyl-1,3,2-dioxaborolan-2-yl)picolinate to prepare Intermediate Z2, MS m/z (ESI): 407 [M+H]⁺.

### Preparation of Intermediate Z3

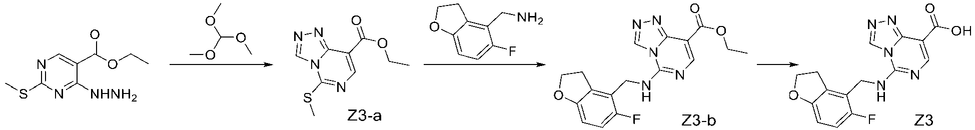

Step 1: Ethyl 4-hydrazinyl-2-(methylthio)pyrimidine-5-carboxylate (2.5 g, 10.96 mmol) was added to trimethyl orthoformate (5 mL). One drop of TFA was added, and the mixture was then heated to reflux and stirred for 2 hours. After the reaction was complete, the mixture was concentrated to give Z3-a (2.2 g), yield: 84.3%. MS m/z (ESI): 239 [M+H]⁺.

Step 2: Z3-a (2.2 g, 9.24 mmol) and (5-fluoro-2,3-dihydrobenzofuran-4-yl)methanamine (2.2 g) were added to a microwave tube. The reaction was carried out under microwave irradiation at 60°C for 30 min. DCM was added, and after uniform dispersion by sonication, the mixture was filtered to give Z3-b (1 g), yield: 30.3%. MS m/z (ESI): 358 [M+H]⁺.

Step 3: Z3-b (1 g, 2.8 mmol) was added to an aqueous tetrahydrofuran solution (5 mL, 50%). Then LiOH (150 mg) was added, and the mixture was stirred at room temperature for 3 hours. After the reaction was complete, a hydrochloric acid solution (2 mol/L) was added to adjust the pH to neutrality, followed by purification by column chromatography (DCM/MeOH = 2% to 10%) to give Z3 (500 mg), yield: 54.2%. MS m/z (ESI): 330 [M+H]⁺.

### Preparation of Intermediate Z4

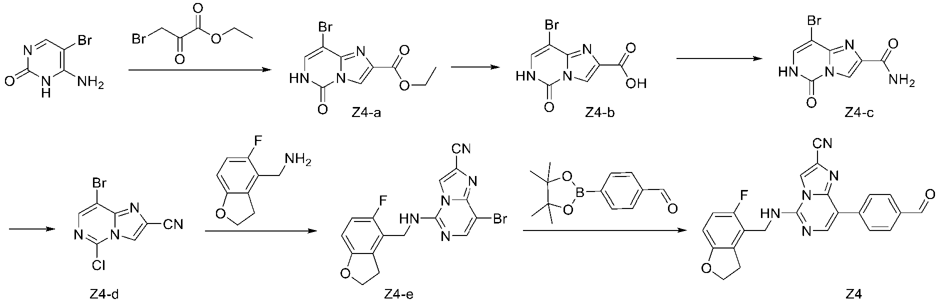

Step 1: 6-Amino-5-bromopyrimidin-2(1H)-one (50 g, 263.16 mmol) and ethyl 3-bromopyruvate (128.30 g, 657.90 mmol) were dissolved in AcOH (500 mL). The reaction mixture was stirred at 120°C for 3 h. After the reaction was complete, the mixture was cooled to room temperature, concentrated to remove most of the acetic acid, and added with water. The resultant was extracted with ethyl acetate four times. The combined organic phases were dried over anhydrous sodium sulfate, concentrated, and purified by CombiFlash (120 g, 0% to 10% MeOH/DCM) to give Z4-a (13 g, yellow solid), yield: 17.27%. MS m/z (ESI): 286.0 [M+H]⁺.

Step 2: Z4-a (13 g, 45.44 mmol) was dissolved in MeOH (130 mL), and then an aqueous NaOH solution (6.36 g, 159.05 mmol, 50 mL) was added thereto. The reaction mixture was stirred at 50°C for 4 hours. After the reaction was complete, the resultant was cooled to room temperature, and concentrated to remove MeOH. The aqueous phase was cooled to 0°C, and adjusted to pH=2-3 with dilute hydrochloric acid (2 mol/L). A solid precipitated, which was filtered. The filter cake was washed three times with water and dried under vacuum to give Z4-b (4.8 g, brown solid, crude), yield: 40.94%. MS m/z (ESI): 258.0 [M+H]⁺.

Step 3: DMF (67.99 mg, 930.13 µmol, 72.02 µL) was added to a suspension of Z4-b (4.8 g, 18.60 mmol) in SOCl₂ (65.52 g, 550.73 mmol, 40 mL). The mixture was stirred at 80°C for 2 h. The reaction mixture was then concentrated, dissolved in DCM (29.93 mL), and aqueous ammonia (13.04 g, 372.05 mmol) was added dropwise to the resulting mixture at 0°C. The mixture was then stirred at 25°C for 1 h. After the reaction was complete, the mixture was filtered, and the filter cake was washed with MeOH. The filter cake was dried under vacuum to give Z4-c (3.5 g, brown solid), yield: 73.20%. MS m/z (ESI): 257.0 [M+H]⁺.

Step 4: Z4-c (3.5 g, 13.62 mmol) was dissolved in POCl₃ (67.85 g, 442.53 mmol, 40 mL), and then DIPEA (8.80 g, 68.08 mmol, 11.86 mL) was added dropwise thereto. The reaction mixture was stirred at 120°C for 14 h. After the reaction was complete, the mixture was cooled to room temperature, concentrated, and purified by CombiFlash (80 g, 0% to 25% EA/PE) to give Z4-d (1.2 g, light yellow solid), yield: 34.23%. MS m/z (ESI): 256.9 [M+H]⁺.

Step 5: Z4-d (1.2 g, 4.66 mmol) and (5-fluoro-2,3-dihydrobenzofuran-4-yl)methanamine (935.00 mg, 5.59 mmol) were dissolved in DCM (15 mL), and then DIPEA (1.20 g, 9.32 mmol, 1.62 mL) was added. The reaction mixture was stirred at room temperature for 1 h. After the reaction was complete, the reaction mixture was concentrated and purified by CombiFlash (24 g, 0% to 100% EA/PE) to give Z4-e (1.78 g, light yellow solid), yield: 98.38%. MS m/z (ESI): 388.0 [M+H]⁺.

Step 6: Z4-e (300 mg, 772.81 µmol) and 4-formylphenylboronic acid pinacol ester (269.03 mg, 1.16 mmol) were dissolved in a mixture of water (1.5 mL)/1,4-dioxane (7.5 mL). Then Pd(dppf)Cl₂ (56.55 mg, 77.28 µmol) and NaHCO₃ (324.61 mg, 3.86 mmol) were added. The reaction mixture was stirred at 100°C for 1.5 h. After the reaction was complete, the reaction mixture was filtered. The filtrate was concentrated and purified by CombiFlash (12 g, 0% to 5% MeOH/DCM) to give Intermediate Z4 (201 mg, light yellow solid), yield: 62.91%. MS m/z (ESI): 414.1 [M+H]⁺.

### Preparation of Intermediate Z5

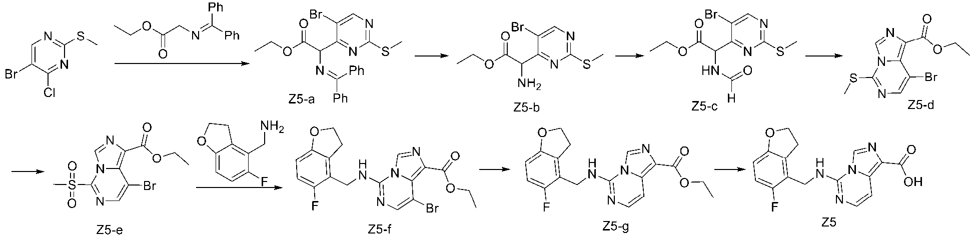

Step 1: Under nitrogen protection, NaH (1.67 g, 41.75 mmol) was dissolved in DMSO (16 mL). The temperature was then lowered to 0°C, and a solution (16 mL) of ethyl diphenylmethyleneglycinate (6.14 g, 22.96 mmol) in DMSO was added. The mixture was stirred at 0°C for 5 minutes, and the reaction mixture turned orange-yellow. 2-Methylthio-4-chloro-5-bromopyrimidine (5 g, 20.88 mmol) was dissolved in DMSO (16 mL) and added dropwise to the above reaction mixture at 0°C. The reaction mixture was then slowly warmed to 25°C and stirred under nitrogen protection for 2 h, and the reaction mixture turned dark red. After the reaction was complete, the reaction mixture was quenched with saturated ammonium chloride. The aqueous phase was extracted with ethyl acetate (100 mL×2). The combined organic phases were dried over anhydrous sodium sulfate, concentrated, and purified by silica gel column chromatography (EA/PE=9% to 11%) to give Z5-a (9.8 g, yellow oil), yield: 99.80%. MS m/z (ESI): 470.2, 472.2 [M+H]⁺.

Step 2: Z5-a (5 g, 10.63 mmol) was dissolved in a mixed solution of THF (50 mL)/water (30 mL). After the solution was cooled to 0°C, concentrated hydrochloric acid (10 mL, 120.00 mmol) was slowly added. The reaction mixture was stirred at 0°C for 10 minutes, then slowly warmed to 25°C and reacted for another 1 hour. After the reaction was complete, the pH of the reaction mixture was adjusted to 8 to 9 with aqueous NaOH solution (20 wt%). The aqueous phase was then extracted with ethyl acetate (30 mL×2). The combined organic phases were dried over anhydrous sodium sulfate, concentrated, and purified by silica gel column chromatography (EA/PE=50% to 55%) to give Z5-b (3.2 g, yellow oil), yield: 98.32%. MS m/z (ESI): 306.0, 308.0 [M+H]⁺.

Step 3: Formic acid (6 mL, 159.06 mmol) and acetic anhydride (6 mL, 64.06 mmol) were mixed and the reaction mixture was stirred at 50°C for 1 hour. The reaction mixture was then cooled to 25°C. Z5-b (3.2 g, 10.45 mmol) was dissolved in DCM (30 mL), and then the above reaction mixture was added and stirred at 25°C for 2 h. After the reaction was complete, the pH of the reaction mixture was adjusted to 8 to 9 with aqueous NaOH solution (20 wt%). The aqueous phase was then extracted with DCM (100 mL×2). The combined organic phases were dried over anhydrous sodium sulfate, concentrated, and purified by silica gel column chromatography (EA/PE=55% to 60%) to give Z5-c (3.25 g, white solid), yield: 93.05%. MS m/z (ESI): 334.0 [M+H]⁺, 336.0 [M+H]⁺.

Step 4: Z5-c (3.2 g, 9.58 mmol) was dissolved in 1,4-dioxane (50 mL), POCl₃ (2.67 mL, 28.73 mmol) was added, and the reaction mixture was stirred at 110°C for 1 h. After the reaction was complete, the reaction mixture was concentrated, and adjusted to pH=8-9 with saturated sodium bicarbonate solution. The aqueous phase was extracted with ethyl acetate (100 mL×2). The combined organic phases were dried over anhydrous sodium sulfate, concentrated, and purified by silica gel column chromatography (EA/PE=55% to 60%) to give Z5-d (3 g, light yellow solid), yield: 99.09%. MS m/z (ESI): 316.0 [M+H]⁺, 318.0 [M+H]⁺.

Step 5: At room temperature, Z5-d (1 g, 3.16 mmol) was dissolved in DCM (15 mL). mCPBA (2.18 g, 12.64 mmol) was then added, and the reaction mixture was stirred at 25°C for 3 h. After the reaction was complete, the reaction mixture was concentrated and purified by column chromatography (PE/EA=40% to 50%) to give Z5-e (900 mg, yellow solid), yield: 81.73%. MS m/z (ESI): 348.0 [M+H]⁺, 350.0 [M+H]⁺.

Step 6: At room temperature, (5-fluoro-2,3-dihydro-1-benzofuran-4-yl)methanamine (247.77 mg, 1.48 mmol) and Z5-e (430 mg, 1.24 mmol) were dissolved in DCM (10 mL), and stirred in an ice bath for 15 minutes. Then, triethylamine (342.39 µL, 2.47 mmol) was added, and the reaction mixture was stirred at 25°C for 2 h. After the reaction was complete, the reaction mixture was concentrated and purified by column chromatography (PE/EA=40% to 50%) to give Z5-f (300 mg, yellow solid), yield: 55.81%. MS m/z (ESI): 434.8 [M+H]⁺, 436.8 [M+H]⁺.

Step 7: Z5-f (60 mg, 0.14 mmol) was dissolved in a mixed solution of MeOH (1 mL)/THF (1 mL). 10% wet palladium on carbon (14.67 mg, 0.01 mmol) was then added, and the reaction mixture was stirred at 25°C for 4 h. After the reaction was complete, the mixture was filtered, the filter cake was washed with MeOH (10 mL) and EA (10 mL), and the filtrates were combined and concentrated to give Z5-g (40 mg, white solid, crude), yield: 81.43%. MS m/z (ESI): 356.9 [M+H]⁺.

Step 8: Z5-g (40 mg, 0.11 mmol) was dissolved in a mixed solution of MeOH (1 mL), water (1 mL), and THF (1 mL), and then LiOH (23.07 mg, 0.55 mmol) was added. The reaction mixture was stirred at 60°C for 18 h. After the reaction was complete, the pH of the reaction mixture was adjusted to 3 to 4 with hydrochloric acid (2 mol/L). The aqueous phase was then extracted with ethyl acetate (20 mL×2), and the combined organic phases were concentrated to give Intermediate Z5 (30 mg, white solid), yield: 81.41%. MS m/z (ESI): 329.0 [M+H]⁺.

### Preparation of Intermediate Z6

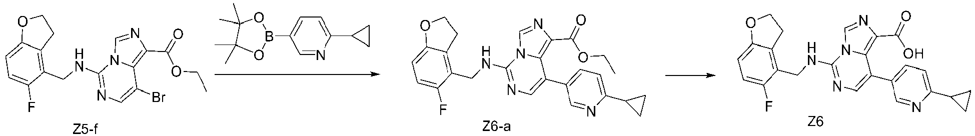

Step 1: Under nitrogen protection, Z5-f (200 mg, 0.46 mmol) and 6-cyclopropylpyridine-3-boronic acid pinacol ester (168.96 mg, 0.69 mmol) were dissolved in a mixed solution of 1,4-dioxane (3 mL) and water (1 mL). K₂CO₃ (190.51 mg, 1.38 mmol) and Pd(dppf)Cl₂ (34.09 mg, 0.05 mmol) were then added, and the reaction mixture was stirred at 100°C for 2 h under nitrogen protection. After the reaction was complete, the reaction mixture was poured into water (10 mL), and the aqueous phase was extracted with ethyl acetate (100 mL×2). The combined organic phases were dried over anhydrous sodium sulfate, concentrated, and purified by silica gel column chromatography (EA/PE=50% to 80%) to give Z6-a (200 mg, white solid), yield: 91.92%. MS m/z (ESI): 474.2 [M+H]⁺.

Step 2: Z6-a (200 mg, 0.42 mmol) was dissolved in a mixed solution of THF (3 mL)/methanol (1 mL)/water (1 mL). LiOH (88.61 mg, 2.11 mmol) was then added, and the temperature was raised to 60°C and the reaction mixture was stirred for 18 h. After the reaction was complete, the reaction mixture was concentrated to give Intermediate Z6 (180 mg, white solid, crude), yield: 95.67%. MS m/z (ESI): 445.9 [M+H]⁺.

### Preparation of Intermediate Z7

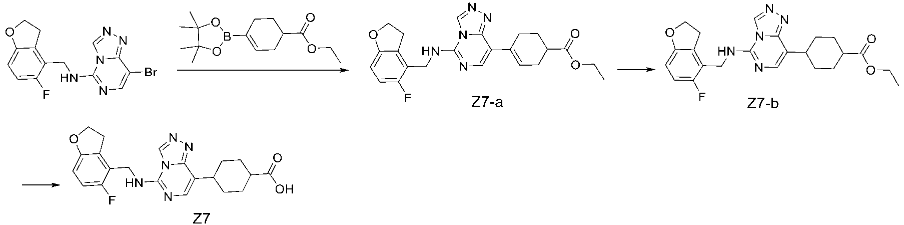

Step 1: Compound 8-bromo-N-((5-fluoro-2,3-dihydrobenzofuran-4-yl)methyl)-[1,2,4] triazolo[4,3-c]pyrimidin-5-amine (300 mg, 823.8 µmol) and compound ethyl 4-(4,4,5,5-tetramethyl-1,3,2-dioxaborolan-2-yl)cyclohex-3-enecarboxylate (462 mg, 1.65 mmol) were mixed and added to an aqueous dioxane solution (5 mL, v/v = 20%). K₂CO₃ (342 mg, 2.47 mmol) and Pd(dppf)Cl₂ (73.1 mg, 0.1 mmol) were added, and the mixture was heated to 100°C under nitrogen protection and stirred for 12 hours. After the reaction was complete, the reaction mixture was purified by column chromatography (15% to 100% PE/EA) to give Z7-a (200 mg), yield: 56.10%. MS m/z (ESI): 438 [M+H]⁺.

Step 2: Z7-a (200 mg, 0.46 mmol) was added to MeOH, and 10% palladium on carbon (20 mg) was added. The mixture was heated to 40°C under hydrogen protection and stirred for 5 hours. After the reaction was complete, the resultant was filtered, and the filtrate was concentrated to give Z7-b (200 mg), yield: 100%. MS m/z (ESI): 440 [M+H]⁺.

Step 3: Z7-b (200 mg, 0.46 mmol) was added to a THF/H₂O solution (2 mL, v/v = 50%). Lithium hydroxide (40 mg) was then added, and the mixture was stirred at room temperature for 3 hours. After the reaction was complete, an aqueous hydrochloric acid solution (2 mol/L) was added to adjust the pH to neutrality, and the mixture was purified by column chromatography (0% to 10% DCM/MeOH) to give Intermediate Z7 (150 mg), yield: 79.3%. MS m/z (ESI): 412 [M+H]⁺.

### Preparation of Intermediate Z8

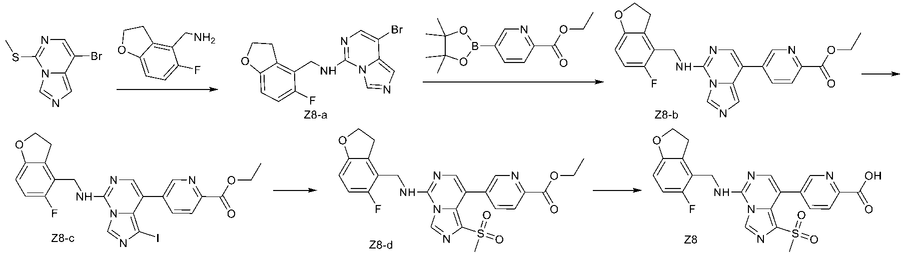

Step 1: To a solution of 8-bromo-5-(methylthio)imidazo[1,5-c]pyrimidine (1 g, 4.10 mmol) in DCM (20 mL) was added m-chloroperoxybenzoic acid (1.08 g, 5.33 mmol), and the mixture was stirred at 0°C under a nitrogen atmosphere for 5 hours. Then, at 0°C, triethylamine (2.84 mL, 20.5 mmol) and (5-fluoro-2,3-dihydrobenzofuran-4-yl)methanamine (0.68 g, 4.10 mmol) were added to the mixture, and the mixture was stirred at 25°C under a nitrogen atmosphere for 18 hours. After the reaction was complete, the mixture was concentrated and purified by silica gel chromatography (DCM:MeOH = 100:1 to 40:1) to give Z8-a (1 g, yellow solid), yield: 67.1%. MS m/z (ESI): 365.0 [M+H]⁺.

Step 2: To a solution of Z8-a (500 mg, 1.38 mmol) in 1,4-dioxane (20 mL) and water (4 mL) were added ethyl 5-(4,4,5,5-tetramethyl-1,3,2-dioxaborolan-2-yl)picolinate (763 mg, 2.75 mmol), K₂CO₃ (380 mg, 2.75 mmol), and Pd(PPh₃)₄ (318 mg, 0.28 mmol), and the mixture was stirred at 90°C under a nitrogen atmosphere for 7 hours. After the reaction was complete, the reaction mixture was poured into water (20 mL) and extracted with ethyl acetate (30 mL×3). The combined organic phases were washed with saturated brine (10 mL), dried over anhydrous sodium sulfate, concentrated, and purified by silica gel column chromatography (DCM:MeOH = 100:1 to 20:1) to give Z8-b (260 mg, yellow solid), yield: 43.6%. MS m/z (ESI): 434.1 [M+H]⁺.

Step 3: At 0°C under a nitrogen atmosphere, NIS (141 mg, 0.63 mmol) was slowly added to a solution of Z8-b (160 mg, 0.37 mmol) in DMF (6 mL), and the reaction mixture was stirred at 25°C under a nitrogen atmosphere for 2 hours. After the reaction was complete, the reaction mixture was poured into ice water (20 mL) and extracted with ethyl acetate (30 mL×3). The combined organic phases were washed with saturated brine (10 mL×3), dried over anhydrous sodium sulfate, concentrated, and purified by silica gel column chromatography (DCM:MeOH = 500:1 to 40:1) to give Z8-c (88 mg, red solid), yield: 42.6%. MS m/z (ESI): 559.8 [M+H]⁺.

Step 4: To a solution of Z8-c (59 mg, 0.11 mmol) in DMSO (2.5 mL) were added sodium methanesulfinate (32 mg, 0.32 mmol) and CuI (60 mg, 0.32 mmol). Under a nitrogen atmosphere, the mixture was heated under microwave irradiation at 120°C for 20 min and then reacted under microwave at 100°C for 3 hours. After the reaction was complete, the reaction mixture was poured into water (20 mL) and extracted with ethyl acetate (30 mL×3). The combined organic phases were washed with saturated brine (10 mL×3), dried over anhydrous sodium sulfate, concentrated, and purified by silica gel column chromatography (DCM:MeOH = 100:1 to 30:1) to give Z8-d (50 mg, yellow solid), yield: 92.7%. MS m/z (ESI): 511.9 [M+H]⁺.

Step 5: To a solution of Z8-d (40 mg, 0.08 mmol) in methanol (4 mL) and water (0.4 mL) was added lithium hydroxide monohydrate (40 mg, 0.95 mmol), and the mixture was stirred at 25°C for 5 h. After the reaction was complete, the mixture was concentrated and diluted with water (5 mL). The pH was adjusted to 7 with aqueous hydrochloric acid solution (2 mol/L). The resultant was extracted with ethyl acetate (30 mL×3), and the combined organic phases were dried over anhydrous sodium sulfate and concentrated to give Intermediate Z8 (35 mg, yellow solid), yield: 92.5%. MS m/z (ESI): 483.9 [M+H]⁺.

### Preparation of Intermediate Z9

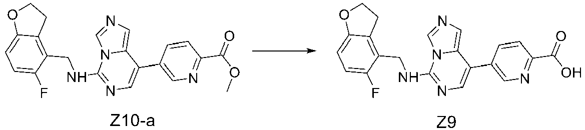

Step 1: To a single-neck flask were added Z10-a (80.0 mg, 0.180 mmol), methanol (5 mL), and water (0.5 mL) at room temperature. Lithium hydroxide monohydrate (80.0 mg, 1.91 mmol) was added with stirring, and the reaction was carried out at 25°C for 3 hours. After the reaction was complete, the reaction mixture was poured into water (20 mL), and filtered. The filter cake was washed with water (5 mL). The filter cake was dried to give Intermediate Z9 (68 mg, light yellow solid), yield: 90.88%. MS m/z (ESI): 406.2 [M+H]⁺.

### Preparation of Intermediate Z10

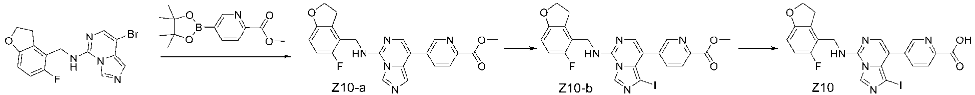

Step 1: 8-Bromo-N-((5-fluoro-2,3-dihydrobenzofuran-4-yl)methyl)imidazo[1,5-c]pyrimidin-5-amine (400 mg, 1.10 mmol) and methyl 5-(4,4,5,5-tetramethyl-1,3,2- dioxaborolan-2-yl)picolinate (580 mg, 2.20 mmol) were dissolved in 1,4-dioxane (15 mL) and water (2 mL). Pd(PPh₃)₄ (255 mg, 0.22 mmol) and K₂CO₃ (304.43 mg, 2.20 mmol) were added, and the mixture was stirred at 90°C under argon protection for 20 hours. After the reaction was complete, the reaction mixture was poured into water, and extracted with ethyl acetate (30 mL×2). The combined organic phases were dried over anhydrous sodium sulfate, concentrated, and purified by CombiFlash (PE/EA = 0% to 90%) to give Z10-a (78 mg, yellow solid), yield: 16.89%. MS m/z (ESI): 420.1 [M+H]⁺.

Step 2: Z10-a (78 mg, 0.19 mmol) was dissolved in DMF (5 mL), and cooled to 0°C. NIS (42 mg, 0.19 mmol) was added, and the mixture was stirred at room temperature for 6s. After the reaction was complete, the reaction mixture was poured into water, and extracted with ethyl acetate (30 mL×2). The combined organic phases were dried over anhydrous sodium sulfate, concentrated, and then purified by CombiFlash (PE/EA = 0% to 100%) to give Z10-b (70 mg, yellow solid), yield: 69%. MS m/z (ESI): 546.0 [M+H]⁺.

Step 3: Z10-b (50 mg, 0.092 mmol) was dissolved in methanol (12 mL) and water (3 mL), and lithium hydroxide monohydrate (39 mg, 0.92 mmol) was added. The mixture was stirred at room temperature for 20 hours, adjusted to pH=5 with dilute hydrochloric acid (1 mol/L), and extracted with dichloromethane (30 mL×3). The combined organic phases were dried over anhydrous sodium sulfate and concentrated to give Intermediate Z10 (35 mg, yellow solid, crude), yield: 71.8%. MS m/z (ESI): 532.0 [M+H]⁺.

### Preparation of Intermediate Z11

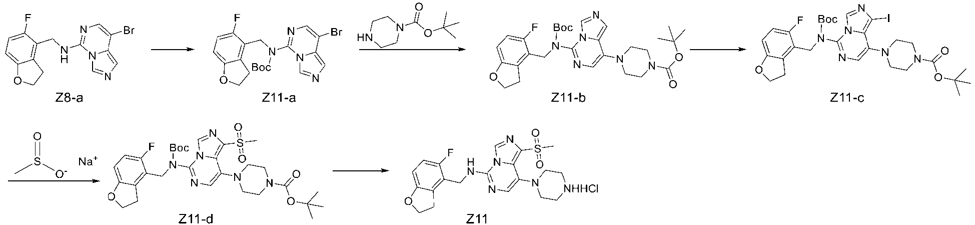

Step 1: Z8-a (1 g, 2.75 mmol), di-tert-butyl dicarbonate (1.20 g, 5.51 mmol), triethylamine (0.83 g, 8.25 mmol) and 4-dimethylaminopyridine (0.34 g, 2.75 mmol) were dissolved in anhydrous DCM (20 mL), and the mixture was stirred at 35°C under nitrogen protection for 2 hours. After the reaction was complete, the reaction mixture was poured into water, and extracted twice with dichloromethane. The combined organic phases were washed with saturated brine, dried over anhydrous sodium sulfate, concentrated, and purified by silica gel column chromatography (PE:EA = 5:1) to give Z11-a (1.13 g, light yellow solid), yield: 88.6%. MS m/z (ESI): 463.2 [M+H]⁺.

Step 2: Z11-a (460 mg, 0.99 mmol), tert-butyl piperazine-1-carboxylate (50.90 mg, 0.32 mmol), cesium carbonate (970.48 mg, 2.98 mmol), Xantphos (229.80 mg, 0.40 mmol) and Pd₂(dba)₃ (181.83 mg, 0.20 mmol) were dissolved in 1,4-dioxane (5 mL). The mixture was purged with nitrogen three times, heated to 90°C, and stirred at 90°C for 18 hours. After the reaction was complete, the reaction mixture was poured into water, and extracted twice with dichloromethane. The combined organic phases were washed with saturated brine, dried over anhydrous sodium sulfate, concentrated, and purified by silica gel column chromatography (PE:EA = 3:1) to give Z11-b (440 mg, light yellow solid), yield: 77.9%. MS m/z (ESI): 569.5 [M+H]⁺.

Step 3: Z11-b (200 mg, 0.35 mmol) was dissolved in DMF (8 mL), and cooled to 0°C. NIS (134.52 mg, 0.60 mmol) was then added, and the mixture was slowly warmed to 25°C and stirred for 18 hours. After the reaction was complete, the reaction mixture was poured into water, and extracted twice with ethyl acetate. The combined organic phases were washed with saturated brine, dried over anhydrous sodium sulfate, concentrated, and purified by silica gel column chromatography (PE:EA = 2:1) to give Z11-c (50 mg, light yellow solid), yield: 20.47%. MS m/z (ESI): 695.6 [M+H]⁺. ¹H NMR (400 MHz, CDCl₃) δ 7.77 (s, 1H), 6.90 (s, 1H), 6.64 - 6.53 (m, 1H), 6.48 (dd, J = 8.7, 4.0 Hz, 1H), 4.92 (s, 2H), 4.47 (t, J = 8.7 Hz, 2H), 3.60-3.72 (m, 4H), 3.15-3.25 (m, 2H), 2.90-2.99 (m, 4H), 1.42 (s, 9H), 1.28 (s, 9H).

Step 4: Z11-c (25 mg, 0.05 mmol) was dissolved in DMSO (2 mL). CuI (17.14 mg, 0.09 mmol) and sodium methanesulfinate (8.82 mg, 0.09 mmol) were then added. The mixture was reacted under microwave at 120°C for 20 minutes, and then reacted under microwave at 100°C for 3 hours. After the reaction was complete, the reaction mixture was poured into water, extracted twice with ethyl acetate. The combined organic phases were washed with saturated brine, dried over anhydrous sodium sulfate, and concentrated to give Z11-d (18 mg, light yellow solid, crude), which was used in the next step without further purification. MS m/z (ESI): 647.3 [M+H]⁺.

Step 5: Z11-d (50 mg, 0.08 mmol) was dissolved in ethyl acetate (2 mL). A solution of hydrogen chloride in ethyl acetate (4 mol/L, 2 mL) was added, and the mixture was stirred at 25°C for 18 hours. After the reaction was complete, the mixture was filtered, and the filter cake was washed twice with ethyl acetate. The filter cake was dried to give Intermediate Z11 (36 mg, white solid), yield: 96.4%. MS m/z (ESI): 447.2 [M+H]⁺.

### Preparation of Intermediate Z12

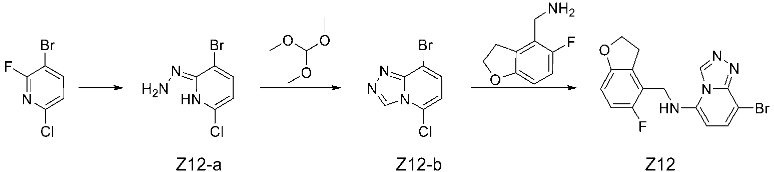

Step 1: 3-Bromo-6-chloro-2-fluoropyridine (8.8 g, 41.82 mmol) was dissolved in ethanol (40 mL). Hydrazine hydrate (2.96 g, 50.18 mmol, 85% purity) was added, and the mixture was heated to 80°C and stirred at 80°C overnight. After the reaction was complete, the reaction mixture was concentrated to give Z12-a (9.3 g), yield: 99.96%. It was used directly in the next reaction. MS m/z (ESI): 221.9 [M+H]⁺.

Step 2: Z12-a (9.3 g, 41.80 mmol) was dissolved in trimethyl orthoformate (90 mL), and the mixture was stirred at 100°C under argon protection for 4 hours. After the reaction was complete, the reaction mixture was filtered, and the filter cake was dried to give Z12-b (8.5 g), yield: 87.47%. MS m/z (ESI): 231.9 [M+H]⁺.

Step 3: Z12-b (2 g, 8.60 mmol), (5-fluoro-2,3-dihydrobenzofuran-4-yl)methanamine (3.31 g, 19.79 mmol) and ethanol (1 mL) were mixed and stirred at 85°C for 20 hours. After the reaction was complete, the reaction mixture was concentrated and purified by CombiFlash (0% to 10% MeOH/DCM) to give Intermediate Z12 (1.2 g, yellow solid), yield: 38.40%. MS m/z (ESI): 365.0 [M+2H]²⁺.

### Preparation of Intermediate Z13

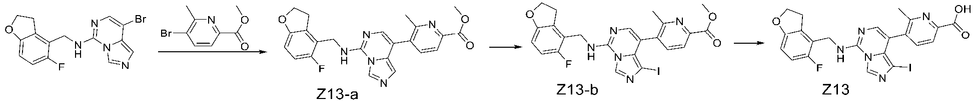

Step 1: To 8-bromo-5-(((5-fluoro-2,3-dihydro-1-benzofuran-4-yl)methyl)amino)imidazo[4,3-flpyrimidine (100 mg, 0.28 mmol) in dimethoxyethane (2 mL) and water (0.2 mL) were added methyl 5-bromo-6-methylpyridine-2-carboxylate (126 mg, 0.55 mmol), 4,4,5,5-tetramethyl-2-(4,4,5,5-tetramethyl-1,3,2-dioxaborolan-2-yl)-1,3,2-dioxaborolane (174 mg, 0.69 mmol), n-butyldi(1-adamantyl)phosphine (39 mg, 0.11 mmol), K₂CO₃ (152 mg, 1.10 mmol) and Pd(OAc)₂ (12 mg, 0.06 mmol). The mixture was stirred at 70°C under a nitrogen atmosphere for 18 hours. The reaction mixture was concentrated and purified by silica gel chromatography (eluent: DCM:MeOH = 100:1 to 20:1) to give Z13-a (45 mg, black solid), yield: 37.1%. MS m/z (ESI): 434.1 [M+H]⁺.

Step 2: At 0°C under a nitrogen atmosphere, NIS (22 mg, 0.10 mmol) was added to a solution of Z13-a (25 mg, 0.06 mmol) in DMF (1.5 mL). The mixture was stirred at 0°C under a nitrogen atmosphere for 30 minutes. The reaction mixture was poured into water (10 mL) and extracted with ethyl acetate (30 mL×3). The combined organic phases were washed with brine (10 mL), dried over anhydrous sodium sulfate, and concentrated to give Z13-b (20 mg, yellow liquid, crude), yield: 43.3%. MS m/z (ESI): 560.1 [M+H]⁺.

Step 3: To Z13-b (20 mg, 0.03 mmol) in methanol (1 mL) and water (0.2 mL) was added lithium hydroxide monohydrate (10 mg, 0.43 mmol). The reaction mixture was stirred at 25°C for 2 hours. The reaction mixture was concentrated to give Intermediate Z13 (22 mg, yellow solid), yield: 96.7%. MS m/z (ESI): 546.0 [M+H]⁺.

### Preparation of Intermediate Z15

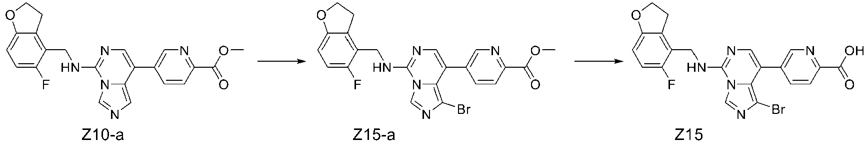

Step 1: Z10-a (190 mg, 453.02 µmol) was dissolved in DCM (50 mL). The temperature was lowered to 0°C under argon protection. NBS (80.63 mg, 453.02 µmol) was added, and the mixture was stirred at 0°C for 1 hour. After the reaction was complete, the reaction mixture was concentrated under reduced pressure and purified by silica gel column chromatography (MeOH:DCM = 0% to 10%) to give Z15-a (45 mg, 90.31 µmol, yellow solid), yield: 19.93%. MS m/z (ESI): 498 [M+H]⁺.

Step 2: Z15-a (45 mg, 90.31 µmol) was dissolved in MeOH (5 mL). A solution of NaOH (3.61 mg, 90.31 µmol) in methanol/water (5 mL/0.5 mL) was added with stirring at room temperature, and the mixture was stirred at room temperature for 3 hours. After the reaction was complete, the pH of the reaction mixture was adjusted to 4-5 with concentrated hydrochloric acid in an ice bath. The mixture was concentrated to give Intermediate Z15 (43.73 mg, yellow solid, crude), yield: 100.00%. MS m/z (ESI): 484 [M+H]⁺.

### Preparation of Intermediate Z16

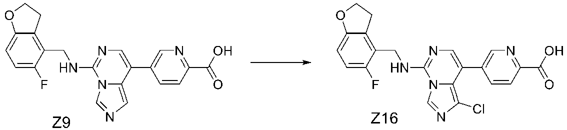

Step 1: Intermediate Z9 (170 mg, 419.36 µmol) was dissolved in DMF (10 mL). Under ice bath, NCS (56.00 mg, 419.36 µmol) was slowly added portionwise. After the reaction was complete, water was added to the reaction mixture, and the mixture was extracted twice with ethyl acetate. The combined organic phases were washed twice with saturated brine, dried over anhydrous sodium sulfate, and concentrated to give Intermediate Z16 (150 mg, yellow solid), yield: 81.3%. MS m/z (ESI): 440.1 [M+H]⁺.

### Preparation of Intermediate Z17

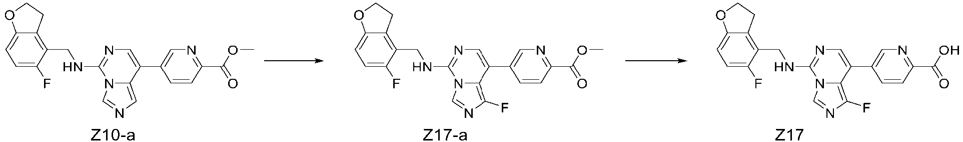

Step 1: Z10-a (323 mg, 0.77 mmol) was dissolved in super dry DCM (15 mL), and selectfluor (409 mg, 1.16 mmol) was added. The mixture was stirred at room temperature for 20 hours. After the reaction was complete, the reaction mixture was poured into water, and extracted with dichloromethane (30 mL×2). The combined organic phases were dried over anhydrous sodium sulfate, concentrated, and purified by CombiFlash (0% to 10% MeOH/DCM) to give Z17-a (35 mg, yellow solid), yield: 10.39%. MS m/z (ESI): 438.1 [M+H]⁺.

Step 2: Z17-a (35 mg, 0.08 mmol) was dissolved in a mixed solution of MeOH/water (6 mL/1 mL). Lithium hydroxide monohydrate (20 mg, 0.48 mmol) was added, and the reaction mixture was stirred at room temperature for 20 hours. After the reaction was complete, the reaction mixture was neutralized to pH=5 with HCl solution (6 mol/L) and concentrated to give Intermediate Z17 (33 mg, crude), yield: 97.41%. MS m/z (ESI): 424.1 [M+H]⁺.

### Preparation of Intermediate Z18

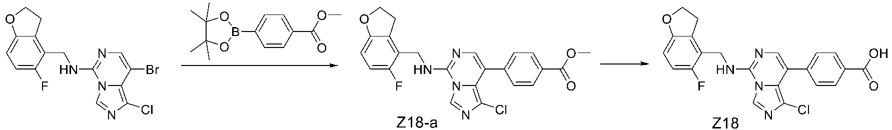

Referring to the preparation method of Intermediate Z6, Z5-f was replaced with 8-bromo-1-chloro-N-((5-fluoro-2,3-dihydrobenzofuran-4-yl)methyl)imidazo[1,5-c]pyrimidin-5- amine to prepare Intermediate Z18, MS m/z (ESI): 439.1 [M+H]⁺.

### Preparation of Intermediate Z19

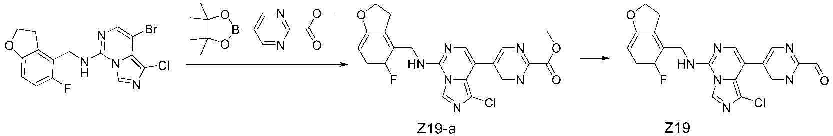

Step 1: 8-Bromo-1-chloro-N-((5-fluoro-2,3-dihydrobenzofuran-4-yl)methyl)imidazo[1,5-c] pyrimidin-5-amine (0.9 g, 2.26 mmol) and methyl 5-(4,4,5,5-tetramethyl-1,3,2-dioxaborolan-2-yl)pyrimidine-2-carboxylate (896.60 mg, 3.40 mmol) were dissolved in 1,4-dioxane (20 mL) and water (2 mL). Ruphos-Pd-G₃ (379.06 mg, 452.68 µmol) and K₃PO₄ (672.63 mg, 3.17 mmol) were added, and the reaction mixture was stirred at 100°C under argon protection for 20 hours. After the reaction was complete, the reaction mixture was poured into water, and extracted with DCM. The combined organic phases were dried over anhydrous sodium sulfate, concentrated, and purified by CombiFlash (0% to 100% EA/PE) to give Z19-a (415 mg, yellow solid), yield: 40.31%. MS m/z (ESI): 455.1 [M+H]⁺.

Step 2: Z19-a (415 mg, 912.41 µmol) was dissolved in THF (8.81 mL), and cooled to -78°C. DIBAL-H (1 mol/L, 3.01 mL) was added, and the reaction mixture was stirred at -78°C for 1 hour. After the reaction was complete, the reaction mixture was quenched with saturated aqueous NH₄Cl solution, and filtered. The filtrate was extracted with ethyl acetate. The combined organic phases were dried over anhydrous sodium sulfate and concentrated to give Intermediate Z19 (360 mg, crude), yield: 92.88%. MS m/z (ESI): 425.1 [M+H]⁺.

### Preparation of Intermediate Z20

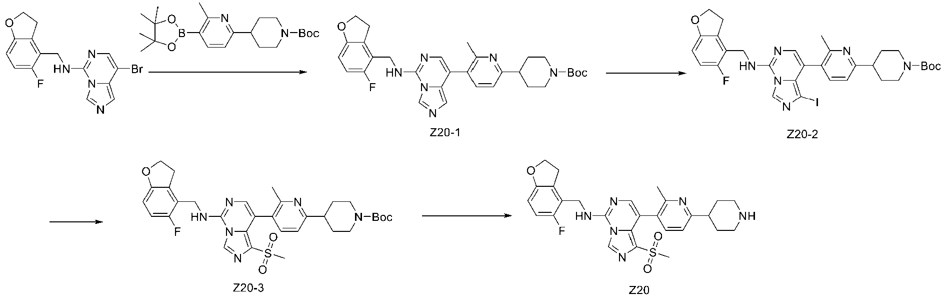

Step 1: 8-Bromo-N-((5-fluoro-2,3-dihydrobenzofuran-4-yl)methyl)imidazo[1,5-c]pyrimidin-5-amine (1 g, 2.75 mmol) was dissolved in water (4 mL) and 1,4-dioxane. [1,1'-Bis(diphenylphosphino)ferrocene]dichloropalladium(II) (302.20 mg, 413.01 µmol), potassium carbonate (761.08 mg, 5.51 mmol), and tert-butyl 4-(6-methyl-5-(4,4,5,5-tetramethyl-1,3,2-dioxaborolan-2-yl)pyridin-2-yl)piperidine-1-carboxylate (1.33 g, 3.30 mmol) were added. The reaction was carried out at 90°C for 16 h. LCMS showed the reaction was complete. The reaction mixture was filtered and concentrated to dryness under reduced pressure. The residue was purified by combiflash with PE:EA = 1:2 to give compound Z20-1 (1.11 g, 1.99 mmol, 72.16% yield) as a yellow solid. MS m/z (ESI): 559.3 [M+H]⁺.

Step 2: Compound Z20-1 (200 mg, 358.01 µmol) was dissolved in DMF (2 mL). Under ice bath conditions, N-iodosuccinimide (80.54 mg, 358.01 µmol) was added, and the mixture was stirred at room temperature for 15 minutes. LC-MS showed the reaction was complete. The reaction mixture was concentrated to dryness under reduced pressure, and purified by silica gel column chromatography (EA:PE = 0% to 100%) to give compound Z20-2 (180 mg, 262.95 µmol, 73.45% yield) as a yellow solid. MS m/z (ESI): 685.1 [M+H]⁺.

Step 3: Compound Z20-2 (200 mg, 292.17 µmol) was dissolved in DMSO (4 mL). Under argon protection, sodium bisulfite (89.40 mg, 876.50 µmol) and copper monoiodide (166.93 mg, 876.50 µmol) were added. The temperature was raised to 100°C, and the mixture was stirred for 1 hour. LCMS showed the reaction was complete. The reaction mixture was filtered and concentrated to dryness under reduced pressure. The residue was purified by combi-flash with DCM:MeOH = 10:1 to give compound Z20-3 (150 mg, 235.58 µmol, 80.63% yield) as a yellow solid. MS m/z (ESI): 637.3 [M+H]⁺.

Step 4: Compound Z20-3 (150 mg, 235.58 µmol) was dissolved in trifluoroacetic acid (1 mL) in dichloromethane (5 mL). The reaction was carried out at room temperature for 1 h. LCMS showed the reaction was complete. The reaction mixture was concentrated to dryness under reduced pressure. The residue was purified by combiflash with DCM:MeOH = 10:1 to give compound Z20 (110 mg, 204.99 µmol, 87.1% yield) as a yellow solid. MS m/z (ESI): 537.2 [M+H]⁺.

### Preparation of Intermediate Z21

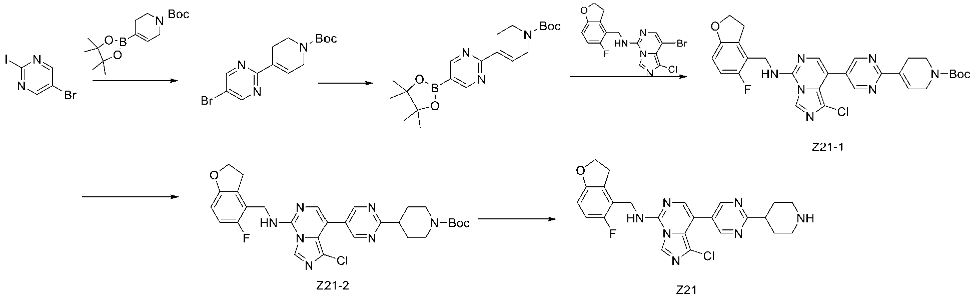

Step 1: 5-Bromo-2-iodopyrimidine (5 g, 17.55 mmol) and tert-butyl 4-(4,4,5,5-tetramethyl-1,3,2-dioxaborolan-2-yl)-3,6-dihydropyridine-1(2H)-carboxylate (5.97 g, 19.31 mmol) were dissolved in water (15 mL) and 1,4-dioxane (100 mL). Under argon protection, [1,1'-bis(diphenylphosphino)ferrocene]dichloropalladium(II) (1.92 g, 2.63 mmol) and potassium carbonate (7.28 g, 52.65 mmol) were added. The temperature was raised to 80°C and stirred for 16 hours. LCMS showed the reaction was complete. The reaction mixture was filtered and concentrated to dryness under reduced pressure. The residue was purified by combiflash with PE:EA = 2:1 to give tert-butyl 4-(5-bromopyrimidin-2-yl)-3,6-dihydropyridine-1(2H)- carboxylate (5 g, 14.70 mmol, 83.74% yield) as a yellow solid. MS m/z (ESI): 342.1 [M+H]⁺.

Step 2: tert-Butyl 4-(5-bromopyrimidin-2-yl)-3,6-dihydropyridine-1(2H)-carboxylate (1 g, 2.94 mmol) and bis(pinacolato)diboron (1.12 g, 4.41 mmol) were dissolved in 1,4-dioxane (20 mL). [1,1'-Bis(diphenylphosphino)ferrocene]dichloropalladium (II) (322.30 mg, 440.90 µmol) and potassium acetate (865.39 mg, 8.82 mmol) were added. The mixture was purged with argon three times, heated to 95°C, and stirred overnight. LCMS showed product formation. The solid was filtered off, and the mother liquor was concentrated under reduced pressure to give a crude product. The residue was separated by CombiFlash column chromatography (20 g, 0% to 20% EA/PE) to give tert-butyl 4-(5-(4,4,5,5-tetramethyl-1,3,2-dioxaborolan-2-yl)pyrimidin-2-yl)-3,6-dihydropyridine-1(2H)-carboxylate (800 mg, 2.07 mmol, 70.28% yield). MS m/z (ESI): 330.3 [M-56+H]⁺.

Step 3: Tert-butyl 4-(5-(4,4,5,5-tetramethyl-1,3,2-dioxaborolan-2-yl)pyrimidin-2-yl)-3,6-dihydropyridine-1(2H)-carboxylate (1 g, 2.58 mmol) and 8-bromo-1-chloro-N-((5-fluoro-2,3-dihydrobenzofuran-4-yl)methyl)imidazo[1,5-c]pyrimidin-5-amine (1.03 g, 2.58 mmol) were dissolved in water (3 mL) and 1,4-dioxane (20 mL). Under argon protection, [1,1'-bis(diphenylphosphino)ferrocene]dichloropalladium (II) (283.40 mg, 387.32 µmol) and potassium carbonate (713.72 mg, 5.16 mmol) were added. The temperature was raised to 80°C and stirred for 16 hours. LCMS showed the reaction was complete. The reaction mixture was filtered and concentrated to dryness. The residue was purified by combiflash (PE:EA = 2:1) to give compound Z21-1 (1 g, 1.73 mmol, 67.00% yield) as a yellow solid. MS m/z (ESI): 522.2 [M-56+H]⁺.

Step 4: Compound Z21-1 (200 mg, 346.00 µmol) was dissolved in ethyl acetate (10 mL). Pd/C (42.02 mg, 346.00 µmol) was added, and the mixture was stirred at room temperature for 2 hours under hydrogen protection. Reaction progress was monitored by LC-MS. The reaction mixture was filtered and concentrated to dryness under reduced pressure to give compound Z21-2 (190 mg, 327.56 µmol, 94.67% yield) as a colorless oil. MS m/z (ESI): 524.2 [M-56+H]⁺.

Step 5: Compound Z21-2 (200 mg, 344.80 µmol) was dissolved in trifluoroacetic acid (3 mL) and dichloromethane (6 mL) was added. The reaction was carried out at room temperature for 2 h. LCMS showed the reaction was complete. The reaction mixture was concentrated to dryness under reduced pressure, adjusted to pH=8 with aqueous sodium bicarbonate solution, extracted twice with DCM, washed twice with saturated brine, dried over anhydrous sodium sulfate, filtered, and concentrated to dryness under reduced pressure. The residue was purified by combiflash with DCM:MeOH = 5:1 to give compound Z21 (75 mg, 156.27 µmol, 45.32% yield) as a light yellow solid. MS m/z (ESI): 480.2 [M+H]⁺.

### Preparation of Intermediate Z22

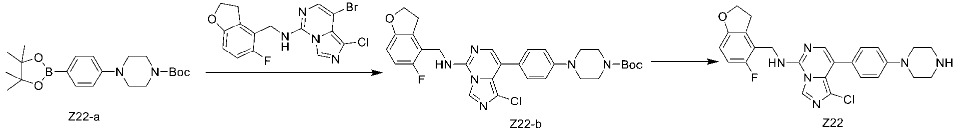

Step 1: 8-Bromo-1-chloro-N-((5-fluoro-2,3-dihydrobenzofuran-4-yl)methyl)imidazo[1,5-c]pyrimidin-5-amine (320 mg, 804.77 µmol) and Z22-a (312.50 mg, 804.77 µmol) were dissolved in dioxane (10 mL), dimethyl sulfoxide (1 mL), and water (1 mL). Under argon protection, Pd(dppf)Cl₂ (58.89 mg, 80.48 µmol) and K₂CO₃ (333.67 mg, 2.41 mmol) were added. The temperature was raised to 90°C and the reaction mixture was stirred for 3 hours. Water and ethyl acetate were added, and the mixture was extracted three times with ethyl acetate. The combined organic phases were washed with brine, dried over anhydrous sodium sulfate, and concentrated under reduced pressure to give a crude product. The residue was separated by CombiFlash column chromatography (12 g, 0% to 40% EA/PE) to give compound Z22-b (350 mg, 604.42 µmol, 75.11% yield) as a light yellow solid. MS m/z (ESI): 579.3 [M+H]⁺.

Step 2: Compound Z22-b (350 mg, 604.42 µmol) was dissolved in DCM (6 mL), and TFA (2 mL) was added. The mixture was stirred at room temperature for 1 hour. The resultant was concentrated under reduced pressure to remove the solvent. The residue was then dissolved in dichloromethane, and the pH was adjusted to greater than 8 with aqueous sodium bicarbonate solution. The resultant was extracted three times with dichloromethane and methanol (10:1). The combined organic phases were dried over anhydrous sodium sulfate and concentrated under reduced pressure to give crude product Z22 (240 mg, 501.10 µmol, 82.91% yield) as a light yellow solid, which was used in the next step without purification. MS m/z (ESI): 479.2 [M+H]⁺.

### Preparation of Intermediate Z23-d

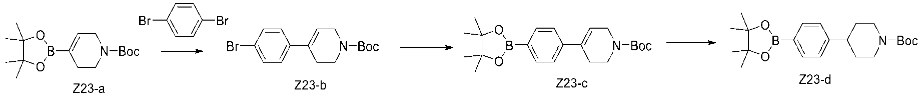

Step 1: Under argon protection, 1,4-dibromobenzene (1 g, 4.24 mmol), compound Z23-a (786.44 mg, 2.54 mmol), [1,1'-bis(diphenylphosphino)ferrocene]dichloropalladium(II) (310.17 mg, 423.90 µmol), potassium carbonate (1.76 g, 12.72 mmol), 1,4-dioxane (20 mL), and water (5 mL) were mixed and then heated to 85°C for reaction for 2 hours. The reaction mixture was filtered, water was added to the filtrate, and extraction was performed with ethyl acetate (20 mL×2). The combined organic layers were washed with saturated brine and concentrated to give a crude product. The crude product was purified by column chromatography (petroleum ether:ethyl acetate = 75%:25%) to give compound Z23-b (540 mg, 1.60 mmol, yield 37.66%) as a colorless liquid. The crude product was used directly in the next step. MS m/z (ESI): 282.0 [M+H-56]⁺.

Step 2: Under argon protection, compound Z23-b (490 mg, 1.45 mmol), bis(pinacolato)diboron (919.69 mg, 3.62 mmol), [1,1'-bis(diphenylphosphino)ferrocene]dichloropalladium(II) (106.00 mg, 144.87 µmol), potassium acetate (426.53 mg, 4.35 mmol), and 1,4-dioxane (35 mL) were mixed and then heated to 90°C for reaction for 16 hours. The reaction mixture was filtered, and the filtrate was concentrated to give a crude product. The crude product was purified by column chromatography (petroleum ether:ethyl acetate = 85%:15%) to give compound Z23-c (420 mg, 1.09 mmol, yield 75.24%) as a yellow solid. MS m/z (ESI): 330.2 [M+H-56]⁺.

Step 3: Under hydrogen atmosphere, palladium on carbon (11.05 mg, 103.81 µmol) was added to a solution of compound Z23-c (400 mg, 1.04 mmol) in methanol (10 mL). After addition, the mixture was reacted at room temperature for 1 hour. The reaction mixture was filtered, and the filtrate was concentrated to dryness to give compound Z23-d (380 mg, 981.10 µmol, yield 94.51%) as a colorless oil. The crude product was used directly in the next step. MS m/z (ESI): 332.2 [M+H-56]⁺.

### Preparation of Intermediate Z24

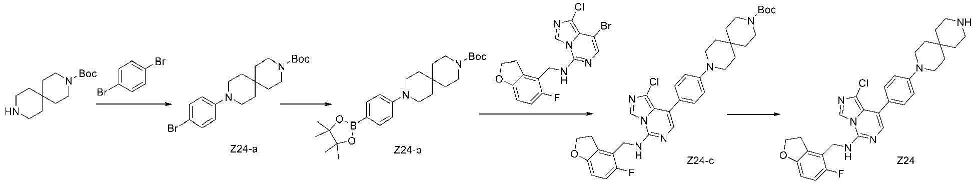

Step 1: Tert-butyl 3,9-diazaspiro[5.5]undecane-3-carboxylate (2 g, 7.86 mmol) was dissolved in toluene (30 mL). 1,4-Dibromobenzene (5.56 g, 23.59 mmol), cesium carbonate (7.67 g, 23.59 mmol), bis(dibenzylideneacetone)palladium (719.99 mg, 786.26 µmol), and 4,5-bis(diphenylphosphino)-9,9-dimethylxanthene (908.92 mg, 1.57 mmol) were added. The reaction was carried out at 110°C overnight. LCMS monitoring showed the starting material disappeared. Water was added to the reaction mixture, which was extracted twice with ethyl acetate, washed twice with saturated brine, dried over anhydrous sodium sulfate, filtered, and concentrated to dryness under reduced pressure. Purification by CombiFlash (40 g, 0% to 40% EA/PE) successfully gave compound Z24-a (1.3 g, 3.18 mmol, yield: 40.39%) as a yellow solid product. MS m/z (ESI): 409.2 [M+H]⁺.

Step 2: Compound Z24-a (1.3 g, 3.18 mmol) was dissolved in 1,4-dioxane (15 mL). [1,1'-Bis(diphenylphosphinoferrocene]dichloropalladium (II) (464.29 mg, 635.14 µmol) and bis(pinacolato)diboron (1.61 g, 6.35 mmol) were added. The mixture was purged with argon three times and reacted at 100°C for 16 h. LCMS monitoring showed the starting material disappeared and product was formed. Water was added to the reaction mixture, which was extracted twice with ethyl acetate, washed twice with saturated brine, dried over anhydrous sodium sulfate, filtered, and concentrated to dryness under reduced pressure. Purification by CombiFlash (20 g, 0% to 40% EA/PE) successfully gave compound Z24-b (1 g, 2.19 mmol, yield: 68.99%) as a yellow solid product. MS m/z (ESI): 457.3 [M+H]⁺.

Step 3: Compound Z24-b (250 mg, 628.73 µmol) and 8-bromo-1-chloro-N-((5-fluoro-2,3-dihydrobenzofuran-4-yl)methyl)imidazo[1,5-c]pyrimidin-5-amine were dissolved in 1,4-dioxane (10 mL). [1,1'-Bis(diphenylphosphinoferrocene]dichloropalladium (II) (68.94 mg, 94.31 µmol) and potassium carbonate (260.68 mg, 1.89 mmol) were added. The mixture was purged with argon three times and reacted at 100°C for 16 h. LCMS monitoring showed the starting material disappeared and product was formed. The reaction mixture was filtered and concentrated to dryness under reduced pressure. The residue was purified by CombiFlash (12 g, 0% to 25% EA/PE) to successfully give compound Z24-c (400 mg, 618.07 µmol, yield: 98.30%) as a yellow solid product. MS m/z (ESI): 647.1 [M+H]⁺.

Step 4: Compound Z24-c (300 mg, 463.55 µmol) was dissolved in DCM (5 mL), and trifluoroacetic acid (2 mL) was added. The reaction was carried out at room temperature overnight. LCMS monitoring showed product was formed and starting material disappeared. The reaction mixture was concentrated to dryness under reduced pressure. The residue was dissolved in TEA and DCM until clear, which was then stirred for sample loading. Purification by CombiFlash (12 g, 0% to 50% MeOH/DCM) successfully gave compound Z24 (250 mg, 456.98 µmol, yield: 98.58%) as a yellow solid product. MS m/z (ESI): 547.3 [M+H]⁺.

### Preparation of Intermediate Z25

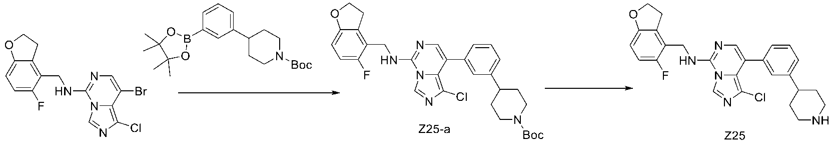

Step 1: Compound 8-bromo-1-chloro-N-[(5-fluoro-2,3-dihydrobenzofuran-4-yl)methyl]imidazo[1,5-c]pyrimidin-5-amine (200 mg, 502.98 µmol) and tert-butyl 4-[3-(4,4,5,5-tetramethyl-1,3,2-dioxaborolan-2-yl)phenyl]piperidine-1-carboxylate (194.81 mg, 502.98 µmol) were dissolved in dioxane (5 mL) and water (0.5 mL). Under argon protection, [1,1'-bis(diphenylphosphino)ferrocene]dichloropalladium(II) (73.61 mg, 100.60 µmol) and potassium carbonate (139.03 mg, 1.01 mmol) were added sequentially. The temperature was raised to 95°C and the mixture was stirred for 16 hours. The reaction mixture was concentrated to dryness under reduced pressure, and purified by silica gel column chromatography (DCM:EA = 0-80%) to give Z25-a (200 mg, 345.98 µmol, 68.78% yield) as a yellow solid. MS m/z (ESI): 578 [M+H]⁺.

Step 2: Compound Z25-a (150 mg, 259.48 µmol) was dissolved in dichloromethane (8.53 mL) and methanol (3.41 mL). A solution of hydrogen chloride in ethyl acetate (4.0 M, 64.87 µL) was added with stirring at room temperature, and the mixture was stirred at room temperature for 2 hours. The reaction mixture was concentrated to dryness under reduced pressure to give Z25 (124.02 mg, 259.48 µmol, 100.00% yield) as a yellow solid. MS m/z (ESI): 478 [M+H]⁺.

### Preparation of Intermediate Z26

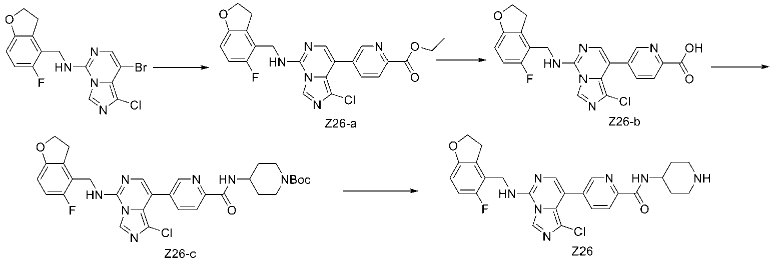

Step 1: Ethyl 5-(4,4,5,5-tetramethyl-1,3,2-dioxaborolan-2-yl)picolinate (554 mg, 2 mmol), 8-bromo-1-chloro-N-((5-fluoro-2,3-dihydrobenzofuran-4-yl)methyl)imidazo[1,5-c]pyrimidin-5-amine (792 mg, 2 mmol), potassium carbonate (414 mg, 3 mmol), and catalyst [1,1'-bis(diphenylphosphinoferrocene]dichloropalladium(II) (146 mg, 0.2 mmol) were added to a mixed solvent of 5 ml of 1,4-dioxane and 1 ml of water. After purging with nitrogen three times, the mixture was heated to 100°C and reacted for 5 hours. After the reaction was complete, silica gel was directly added to the mixture, which was stirred for sample loading. Column chromatography was performed using 30% to 100% PE/EA. The target product was concentrated to give compound Z26-a (467 mg, 1 mmol). Yield: 50%. MS m/z (ESI): 468 [M+H]⁺.

Step 2: Compound Z26-a (467 mg, 1 mmol) and 100 mg of lithium hydroxide were added to a mixed solvent of 5 ml of tetrahydrofuran and 2 ml of water. After stirring at room temperature for 2 hours, 2M aqueous hydrochloric acid solution was added dropwise to adjust the pH to weakly acidic. Extraction was performed three times with ethyl acetate (20 ml×3), and the combined organic phases were concentrated to give compound Z26-b (330 mg, 0.75 mmol). Yield: 75%. MS m/z (ESI): 440 [M+H]⁺.

Step 3: Compound Z26-b (330 mg, 0.75 mmol), 1 ml of DIEA, and HATU (285 mg, 0.75 mmol) were sequentially added to 5 ml of DMF. After stirring at room temperature for 10 minutes, tert-butyl 4-aminopiperidine-1-carboxylate (200 mg, 1 mmol) was added, and the reaction was allowed to proceed for 1 hour. Silica gel was added to the resulting mixture solution which was stirred for sample loading. Column chromatography was performed using PE/EA (10% to 100%) to give Z26-c (156 mg, 0.25 mmol). Yield: 33%. MS m/z (ESI): 622 [M+H]⁺.

Step 4: Compound Z26-c (156 mg, 0.25 mmol) was added to a mixed solution of 5 ml of dichloromethane and 1 ml of TFA, stirred at room temperature for 1 hour, and then concentrated and dried under oil pump vacuum to give compound Z26 (120 mg, 0.23 mmol). Yield: 92%. MS m/z (ESI): 522 [M+H]⁺.

### Preparation of Intermediate Z27

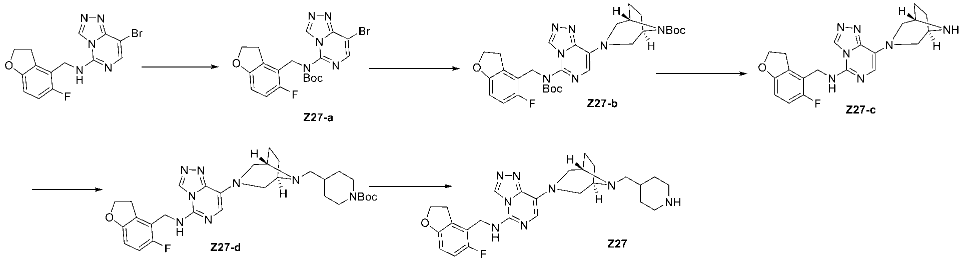

Step 1: Compound 8-bromo-N-((5-fluoro-2,3-dihydrobenzofuran-4-yl)methyl)[1,2,4] triazolo[4,3-c]pyrimidin-5-amine (1090 mg, 3 mmol) was added to 3 ml of pyridine. Then, 100 mg of DMAP and di-tert-butyl dicarbonate (1090 mg, 5 mmol) were added. The reaction mixture was stirred at room temperature for 5 hours. After the reaction was complete, silica gel was added, and the mixture was stirred for sample loading. Column chromatography was performed using PE/EA (25% to 100%) to give Z27-a (926 mg, 2 mmol). Yield: 66%. MS m/z (ESI): 464 [M+H]⁺.

Step 2: Compound Z27-a (926 mg, 2 mmol), tert-butyl (1R,5S)-3,8-diazabicyclo[3.2.1]octane-8-carboxylate (500 mg, 2.36 mmol), 100 mg of Xantphos, 100 mg of Pd₂(dba)₃, and cesium carbonate were sequentially added to 10 ml of 1,4-dioxane. After purging with nitrogen three times, the mixture was heated to 110°C and stirred for 10 hours. After the reaction was complete, silica gel was added, and the mixture was stirred for sample loading. Column chromatography was performed using PE/EA (10% to 100%) to give compound Z27-b (595 mg, 1 mmol). Yield: 50%. MS m/z (ESI): 596 [M+H]⁺.

Step 3: Compound Z27-b (595 mg, 1 mmol) was added to 5 ml of dichloromethane. Then, 2 ml of trifluoroacetic acid was added, and the reaction mixture was stirred at room temperature for 1 hour. After the reaction was complete, the mixture was concentrated to give compound Z27-c (394 mg, 1 mmol). Yield: 100%. MS m/z (ESI): 396 [M+H]⁺.

Step 4: Compound Z27-c (394 mg, 1 mmol) and tert-butyl 4-formylpiperidine-1-carboxylate (213 mg, 1 mmol) were added to 5 ml of DMSO. Then, 3 drops of acetic acid were added, and the mixture was heated under microwave at 50°C and reacted for 30 min. Then, 80 mg of sodium triacetoxyborohydride was added, and the reaction was continued under microwave heating at 50°C with stirring for 1 hour. After the reaction was complete, silica gel was added, and the mixture was stirred for sample loading. Column chromatography was performed using PE/EA (10% to 100%) to give compound Z27-d (355 mg, 0.6 mmol). Yield: 60%. MS m/z (ESI): 593 [M+H]⁺.

Step 5: Compound Z27-d (355 mg, 0.6 mmol) was added to 5 ml of dichloromethane. Then, 2 ml of trifluoroacetic acid was added, and the reaction mixture was stirred at room temperature for 1 hour. After the reaction was complete, the mixture was concentrated to give compound Z27 (295 mg, 0.6 mmol). Yield: 60%. MS m/z (ESI): 493 [M+H]⁺.

### Preparation of Intermediate Z28

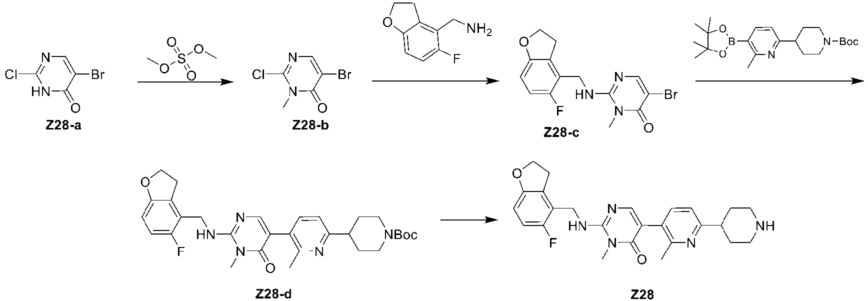

Step 1: Compound Z28-a (3 g, 14.32 mmol) and KOH (1.61 g, 28.65 mmol) were dissolved in THF (30 mL). Dimethyl sulfate (3.61 g, 28.65 mmol, 2.71 mL) was added dropwise, and the mixture was stirred at room temperature for 1 hour. The solid was filtered off, and the filtrate was concentrated under reduced pressure to give a crude product. The residue was separated by CombiFlash column chromatography (40 g, 0% to 20% EA/PE) to give Z28-b (0.7 g, 3.13 mmol, 21.87% yield) as a white solid. MS m/z (ESI): 223.0 [M+H]⁺.

Step 2: Z28-b (0.5 g, 2.24 mmol) and NaHCO₃ (375.94 mg, 4.48 mmol) were dissolved in ethanol (15 mL). (5-Fluoro-2,3-dihydrobenzofuran-4-yl)methanamine (486.30 mg, 2.91 mmol) was added. The temperature was raised to 60°C and the mixture was stirred for 1 hour. Water and dichloromethane were added, and extraction was performed three times with DCM:MeOH ~ 10:1. The combined organic phases were dried over anhydrous sodium sulfate and concentrated under reduced pressure to give a crude product. The crude product was then slurried with ethyl acetate, and filtered to obtain a filter cake. The filter cake was oven-dried to give product Z28-c (0.6 g, 1.69 mmol, 75.71% yield) as a white solid. It was used directly in the next step without purification. MS m/z (ESI): 354.0 [M+H]⁺.

Step 3: Z28-c (80 mg, 225.88 µmol) and tert-butyl 4-(6-methyl-5-(4,4,5,5-tetramethyl-1,3,2-dioxaborolan-2-yl)pyridin-2-yl)piperidine-1-carboxylate (99.97 mg, 248.47 µmol) were dissolved in dioxane (5 mL), DMSO (0.5 mL), and water (1 mL). Under argon protection, Pd(dppf)Cl₂ (16.53 mg, 22.59 µmol) and K₂CO₃ (62.44 mg, 451.76 µmol) were added. The temperature was raised to 100°C and the mixture was stirred for 2 hours. The resultant was concentrated under reduced pressure to remove the solvent. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (4 g, 0% to 80% EA/PE) to give Z28-d (30 mg, 54.58 µmol, 24.16% yield) as a brown oil. MS m/z (ESI): 550.3 [M+H]⁺.

Step 4: Z28-d (30 mg, 54.58 µmol) was dissolved in DCM (2 mL). TFA (0.8 mL) was added, and the mixture was stirred at room temperature for 1 hour. The resultant was concentrated under reduced pressure to remove the solvent. Aqueous sodium bicarbonate solution and ethyl acetate were added, and extraction was performed three times with ethyl acetate. The combined organic phases were washed with brine, dried over anhydrous sodium sulfate, and concentrated under reduced pressure to give crude product Z28 (24 mg, 53.39 µmol, 97.82% yield) as a light yellow solid. It was used directly in the next step without purification. MS m/z (ESI): 450.2 [M+H]⁺.

### Preparation of Intermediate Z29

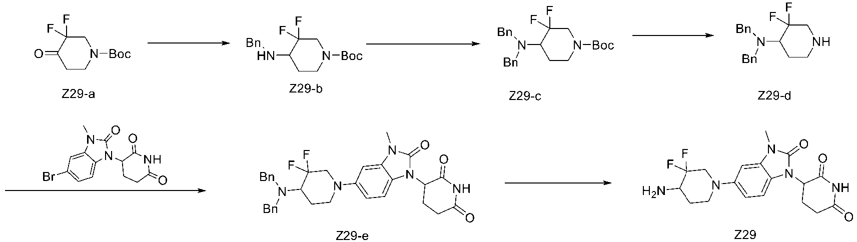

Step 1: Z29-a (12 g, 51.01 mmol) and benzylamine (5.47 g, 51.01 mmol) were dissolved in DCM (100 mL). Sodium triacetoxyborohydride (21.62 g, 102.03 mmol) was then added, and the reaction mixture was stirred at room temperature for 18 hours. After the reaction was complete, the mixture was quenched with saturated aqueous sodium bicarbonate solution, and extracted with dichloromethane (200 mL×3). The combined organic phases were washed with saturated brine (60 mL), dried over anhydrous sodium sulfate, and filtered. The filtrate was concentrated to dryness under reduced pressure to successfully give product Z29-b (16.65 g, 51.01 mmol, 100.00% yield) as an oil. MS m/z (ESI): 327.2 [M+H]⁺.

Step 2: Z29-b (16.65 g, 51.01 mmol) and benzaldehyde (8.12 g, 76.52 mmol) were dissolved in DCM (100 mL). Sodium triacetoxyborohydride (21.62 g, 102.03 mmol) was then added, and the reaction mixture was stirred at room temperature for 18 hours. After the reaction was complete, the mixture was quenched with saturated aqueous sodium bicarbonate solution, and extracted with dichloromethane (200 mL×3). The combined organic phases were washed with saturated brine (100 mL), dried over anhydrous sodium sulfate, and filtered. The filtrate was concentrated under reduced pressure. Purification by CombiFlash column chromatography (120 g, 0% to 45% PE/DCM) successfully gave product Z29-c (6.4 g, 15.37 mmol, 30.12% yield) as a white solid. MS m/z (ESI): 417.3 [M+H]⁺.

Step 3: Z29-c (6.4 g, 15.37 mmol) was dissolved in DCM (35 mL). HCl (4 M in dioxane, 25 mL) was then added thereto. The reaction mixture was stirred at room temperature for 18 hours. After the reaction was complete, the reaction mixture was concentrated, then neutralized with saturated aqueous sodium bicarbonate solution, and extracted with ethyl acetate (150 mL×3). The combined organic phases were dried over anhydrous sodium sulfate, and filtered. The filtrate was concentrated under reduced pressure to successfully give product Z29-d (4.6 g, 14.54 mmol, 94.62% yield) as a white solid. MS m/z (ESI): 317.2 [M+H]⁺.

Step 4: 3-(5-Bromo-3-methyl-2-oxo-2,3-dihydro-1H-benzo[d]imidazol-1-yl)piperidine-2,6- dione (1 g, 2.96 mmol) and Z29-d (1.50 g, 4.73 mmol) were dissolved in toluene (60 mL). Ruphos (275.99 mg, 591.44 µmol) and Ruphos-Pd-G₃ (495.26 mg, 591.44 µmol) were then added thereto. LiHMDS (1 M in THF, 14.79 mL) was added under a nitrogen atmosphere, and the reaction mixture was stirred at 90°C for 2 hours. After the reaction was complete, the mixture was cooled to room temperature, poured into water (60 mL), and extracted with ethyl acetate (100 mL×3). The combined organic phases were dried over anhydrous sodium sulfate, and filtered. The filtrate was concentrated under reduced pressure. Purification by CombiFlash column chromatography (24 g, 0% to 100% EA/PE) successfully gave product Z29-e (1.1 g, 1.92 mmol, 64.85% yield) as a yellow solid. MS m/z (ESI): 574.2 [M+H]⁺.

Step 5: Z29-e (1.1 g, 1.92 mmol) and Pd/C (300 mg, 10% purity and 50% water) were dissolved in THF (30 mL). The reaction was then stirred at 50°C under a hydrogen balloon condition for 6 hours. The mixture was filtered through Celite. The filter cake was washed with a small amount of methanol, and the filtrate was concentrated to successfully give product Z29 (690 mg, 1.75 mmol, 91.47% yield) as a light yellow solid. MS m/z (ESI): 394.1 [M+H]⁺.

### Preparation of Intermediate Z30

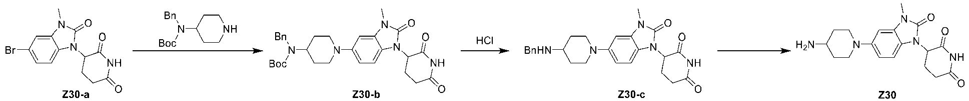

Step 1: Z30-a (0.5 g, 1.48 mmol) and 4-N-Boc-4-N-benzylpiperidine (515.26 mg, 1.77 mmol) were dissolved in toluene (10 mL). Ruphos-Pd-G₃ (123.81 mg, 147.86 µmol) and Ruphos (137.99 mg, 295.72 µmol) were added. The mixture was purged with argon three times, then LiHMDS (1 M, 7.39 mL) was added, and the mixture was purged with argon three more times. The temperature was raised to 80°C and stirred for 2 hours. After cooling to room temperature, water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (12 g, 0% to 60% EA/PE) to give Z30-b (0.5 g, 913.00 µmol, 61.75% yield) as a light yellow solid. MS m/z (ESI): 548.3 [M+H]⁺.

Step 2: Z30-b (0.4 g, 730.40 µmol) was dissolved in HCl/dioxane (4 M) (10 mL) and stirred at room temperature for 1 hour. The resultant was concentrated under reduced pressure to remove the solvent and to give a crude product. The crude product was then dissolved in dichloromethane:methanol (10:1), and washed with aqueous sodium bicarbonate solution. The organic phase was dried and concentrated under reduced pressure to remove the solvent to give crude product Z30-c (0.3 g, 670.35 µmol, 91.78% yield) as a white solid. It was used directly in the next step without purification. MS m/z (ESI): 448.2 [M+H]⁺.

Step 3: Z30-c (0.3 g, 670.35 µmol) was dissolved in THF (5 mL). Pd/C (713.39 mg, 670.35 µmol, 10% purity) was added, and the mixture was purged with a hydrogen balloon three times and stirred at room temperature overnight. The catalyst was filtered off, and the mother liquor was concentrated under reduced pressure to give crude product Z30 (0.1 g, 279.79 µmol, 41.74% yield) as an off-white solid, which was used directly in the next step without purification. MS m/z (ESI): 358.2 [M+H]⁺.

### Preparation of Intermediate Z31

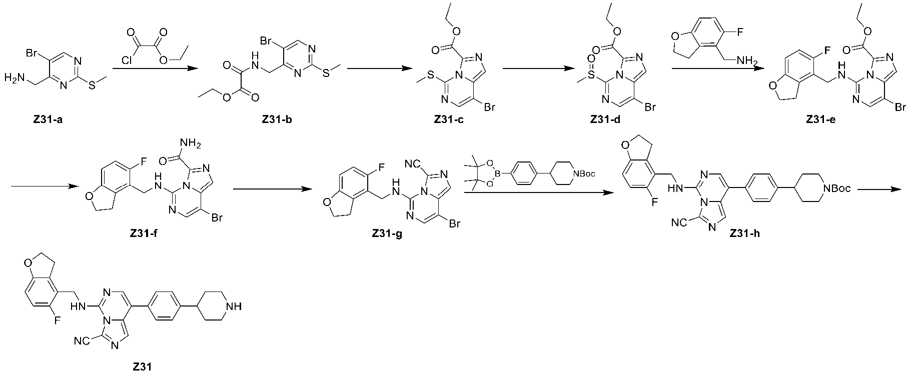

Step 1: Z31-a (7 g, 29.90 mmol) and TEA (9.08 g, 89.70 mmol, 12.51 mL) were dissolved in DCM (946.01 µL). Ethyl oxalyl chloride (4.08 g, 29.90 mmol, 3.34 mL) was added at 0°C. The mixture was stirred at room temperature for 1 hour. Water was added, and extraction was performed three times with dichloromethane. The combined organic phases were dried over anhydrous sodium sulfate and concentrated under reduced pressure to give a crude product. The crude product was slurried with PE:EA ~ 1:1, and filtered to obtain a filter cake. The filter cake was oven-dried to give product Z31-b (3.5 g, 10.47 mmol, 35.03% yield) as a white solid. MS m/z (ESI): 334.0 [M+H]⁺.

Step 2: Phosphorus oxychloride (4.11 g, 26.82 mmol, 2.50 mL) was added dropwise to a solution of Z31-b (3.5 g, 10.47 mmol) in dioxane (40 mL). The temperature was raised to 110°C and stirred for 8 hours. The mixture was cooled to room temperature and concentrated. The residue was slowly added to saturated aqueous NaHCO₃ solution. The precipitated solid was filtered, and the filter cake was washed twice with water. The solid was dried under vacuum to give a crude product. The crude product was then slurried with petroleum ether to give crude product Z31-c (3.3 g, 10.44 mmol, 99.66% yield) as a light brown solid. It was used directly in the next step without purification. MS m/z (ESI): 315.9 [M+H]⁺.

Step 3: Z31-c (3.3 g, 10.44 mmol) was dissolved in DCM (50 mL). m-Chloroperoxybenzoic acid (4.24 g, 20.87 mmol, 85% purity) was added at 0°C, and the mixture was stirred at low temperature for 1 hour. The solid was filtered off, and the mother liquor containing Z31-d (3.4 g, 10.24 mmol, 98.07% yield) was obtained as a light yellow solution, which was used directly in the next step without purification. MS m/z (ESI): 332.0 [M+H]⁺.

Step 4: (5-Fluoro-2,3-dihydrobenzofuran-4-yl)methanamine (3.42 g, 20.47 mmol) and DIEA (3.97 g, 30.71 mmol, 5.35 mL) were dissolved in DCM (1.03 mL). Z31-d (3.4 g, 10.24 mmol) was added at 0°C, and the mixture was stirred at room temperature for 1 hour. Water was added, and extraction was performed three times with dichloromethane. The combined organic phases were dried over anhydrous sodium sulfate and concentrated under reduced pressure to give a crude product. The residue was separated by CombiFlash column chromatography (40 g, 0% to 40% EA/PE) to give Z31-e (1.8 g, 4.14 mmol, 40.40% yield) as a light yellow solid. MS m/z (ESI): 435.0 [M+H]⁺.

Step 5: Z31-e (1.8 g, 4.14 mmol) was dissolved in NH₃/MeOH (7 M) (30 mL) and stirred at room temperature overnight. The reaction mixture was filtered directly, and the filter cake was dried to give product Z31-f (1.2 g, 2.95 mmol, 71.43% yield) as a white solid, which was used directly in the next step without purification. MS m/z (ESI): 406.0 [M+H]⁺.

Step 6: Z31-f (0.5 g, 1.23 mmol) was dissolved in phosphorus oxychloride (10 mL). The temperature was raised to 120°C under argon protection and stirred for 4 hours. The resultant was concentrated under reduced pressure to remove the solvent. The pH was then adjusted to greater than 8 with saturated aqueous sodium bicarbonate solution, and extraction was performed three times with ethyl acetate. The combined organic phases were dried over anhydrous sodium sulfate and concentrated under reduced pressure to give a crude product. The residue was separated by CombiFlash column chromatography (12 g, 0% to 10% MeOH/DCM) to give Z31-g (0.3 g, 772.81 µmol, 62.78% yield) as a dark green solid. MS m/z (ESI): 388.0 [M+H]⁺.

Step 7: Z31-g (0.15 g, 386.41 µmol) and pinacol 4-(1-Boc-4-piperidinyl)phenylboronate (179.59 mg, 463.69 µmol) were dissolved in dioxane (5 mL) and water (0.5 mL). Under argon protection, Pd(dppf)Cl₂ (56.55 mg, 77.28 µmol) and K₂CO₃ (160.21 mg, 1.16 mmol) were added. The temperature was raised to 100°C and stirred for 2 hours. After cooling to room temperature, water and ethyl acetate were added, and extraction was performed three times with ethyl acetate. The combined organic phases were dried over anhydrous sodium sulfate and concentrated under reduced pressure to give a crude product. The residue was separated by CombiFlash column chromatography (4 g, 0% to 10% MeOH/DCM) to give Z31-h (0.1 g, 175.86 µmol, 45.51% yield) as a light yellow solid. MS m/z (ESI): 569.1 [M+H]⁺.

Step 8: Z31-h (0.15 g, 263.79 µmol) was dissolved in DCM (3 mL). TFA (1 mL) was added, and the mixture was stirred at room temperature for 0.5 hours. The resultant was concentrated under reduced pressure. The pH was adjusted to greater than 7 with saturated sodium bicarbonate, and extraction was performed three times with dichloromethane:methanol ~ 10:1. The combined organic phases were dried over anhydrous sodium sulfate and concentrated under reduced pressure to give a crude product. The residue was separated by CombiFlash column chromatography (4 g, 0% to 10% MeOH/DCM) to give a crude product. The crude product was purified by preparative liquid chromatography (preparative column: 21.2X250 mm C18 column; system: 10 mM NH₄HCO₃ H₂O/acetonitrile; wavelength: 254/214 nm; gradient: 5% to 95% acetonitrile change) to give Z31 (15 mg, 32.02 µmol, 12.14% yield) as a light yellow solid. MS m/z (ESI): 469.2 [M+H]⁺.

### Preparation of Intermediate Z32

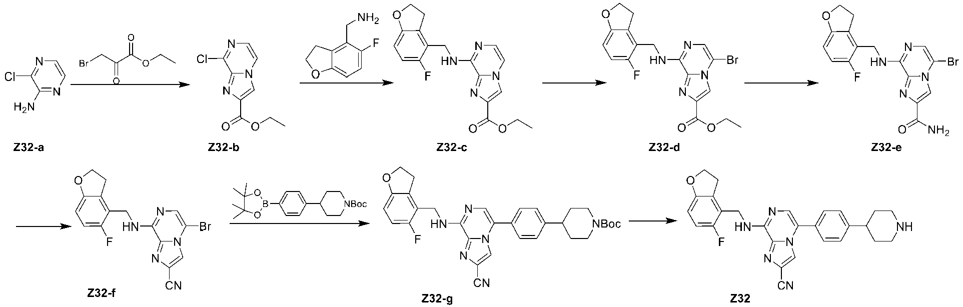

Step 1: Z32-a (3.5 g, 27.02 mmol) and NaHCO₃ (6.81 g, 81.05 mmol) were dissolved in MeCN (80 mL). Under argon protection, ethyl 3-bromopyruvate (9.88 g, 40.53 mmol, 6.37 mL) was added. The temperature was raised to 80°C, and the mixture was stirred for 16 hours. The resultant was concentrated under reduced pressure to remove the solvent. Water and ethyl acetate were added, and extraction was performed three times with ethyl acetate. The combined organic phases were dried over anhydrous sodium sulfate and concentrated under reduced pressure to give a crude product. The residue was separated by CombiFlash column chromatography (20 g, 0% to 10% MeOH/DCM) to give a crude product, which was then slurried with petroleum ether and ethyl acetate, and filtered to give product Z32-b (4.5 g, 19.94 mmol, 73.82% yield) as a light yellow solid. MS m/z (ESI): 226.0 [M+H]⁺.

Step 2: Z32-b (4.5 g, 19.94 mmol) and TEA (6.05 g, 59.83 mmol, 8.35 mL) were dissolved in ethanol (100 mL), and (5-fluoro-2,3-dihydrobenzofuran-4-yl)methanamine (6.67 g, 39.89 mmol, 5.33 mL) was added. The temperature was raised to 90°C, and the mixture was stirred for 16 hours. The resultant was concentrated under reduced pressure to remove the solvent and to give a crude product. The residue was separated by CombiFlash column chromatography (80 g, 0% to 40% EA/PE) to give a product, which was then slurried with petroleum ether, and filtered. The filter cake was oven-dried to give product Z32-c (4.5 g, 12.63 mmol, 63.32% yield) as an off-white solid. MS m/z (ESI): 357.1 [M+H]⁺.

Step 3: Z32-c (4 g, 11.22 mmol) was dissolved in DCM (80 mL). Pyridinium tribromide (3.59 g, 11.22 mmol) was added at 0°C, and the mixture was stirred at 0°C for 1 hour. Water was added, and extraction was performed three times with dichloromethane. The combined organic phases were washed with saturated aqueous sodium sulfite solution, dried over anhydrous sodium sulfate, and concentrated under reduced pressure to give a crude product. The residue was separated by CombiFlash column chromatography (40 g, 0% to 10% EA/DCM) to give Z32-d (4 g, 9.19 mmol, 81.87% yield) as a white solid. MS m/z (ESI): 435.0 [M+H]⁺.

Step 4: Z32-d (2 g, 4.60 mmol) was dissolved in NH₃/MeOH (50 mL). The temperature was raised to 50°C in a sealed vessel, and the mixture was stirred for 20 hours. The resultant was concentrated under reduced pressure to remove the solvent and to give crude product Z32-e (1.5 g, 3.69 mmol, 80.36% yield) as a white solid, which was used directly in the next step without purification. MS m/z (ESI): 406.0 [M+H]⁺.

Step 5: Z32-e (1.3 g, 3.20 mmol) was dissolved in phosphorus oxychloride (15 mL). The temperature was raised to 120°C under argon protection, and the mixture was stirred for 4 hours. The resultant was concentrated under reduced pressure to remove the solvent. The pH was then adjusted to greater than 8 with saturated aqueous sodium bicarbonate solution, and extraction was performed three times with ethyl acetate. The combined organic phases were dried over anhydrous sodium sulfate and concentrated under reduced pressure to give a crude product. The crude product was slurried with petroleum ether and ethyl acetate, and filtered. The filter cake was concentrated to dryness to give product Z32-f (1 g, 2.58 mmol, 80.49% yield) as a white solid. MS m/z (ESI): 388.0 [M+H]⁺.

Step 6: Z32-f (0.5 g, 1.29 mmol) and pinacol 4-(1-Boc-4-piperidinyl)phenylboronate (748.31 mg, 1.93 mmol) were dissolved in dioxane (5 mL) and water (1 mL). Under argon protection, Pd(dppf)Cl₂ (94.24 mg, 128.80 µmol) and K₂CO₃ (534.03 mg, 3.86 mmol) were added. The temperature was raised to 100°C and stirred for 2 hours. The mixture was concentrated under reduced pressure to give a crude product. Water and ethyl acetate were added, and extraction was performed three times with ethyl acetate. The combined organic phases were dried over anhydrous sodium sulfate, and concentrated under reduced pressure to give a crude product. The residue was separated by CombiFlash column chromatography (12 g, 0% to 50% EA/PE) to give Z32-g (0.6 g, 1.06 mmol, 81.92% yield) as a light yellow solid. MS m/z (ESI): 569.3 [M+H]⁺.

Step 7: Z32-g (0.1 g, 175.86 µmol) was dissolved in DCM (3 mL). TFA (1 mL) was added, and the mixture was stirred at room temperature for 1 hour. The resultant was concentrated under reduced pressure to remove the solvent. Dichloromethane and saturated aqueous sodium bicarbonate solution were added, and extraction was performed three times with dichloromethane. The combined organic phases were dried over anhydrous sodium sulfate, and concentrated under reduced pressure to give crude product Z32 (80 mg, 170.75 µmol, 97.09% yield) as a light yellow solid, which was used directly in the next step without purification. MS m/z (ESI): 469.3 [M+H]⁺.

### Preparation of Intermediate Z33

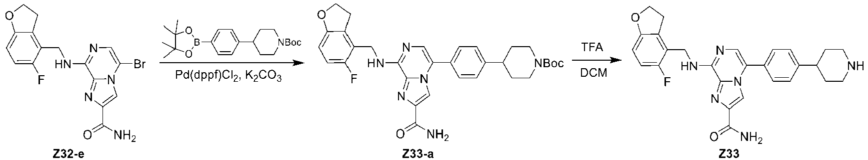

Intermediate Z33 was prepared referring to steps 6-7 of Intermediate Z32. MS m/z (ESI): 487.2 [M+H]⁺.

### Preparation of Intermediate Z34

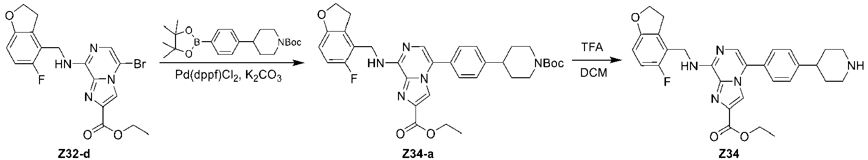

Intermediate Z34 was prepared referring to steps 6-7 of Intermediate Z32. MS m/z (ESI): 516.2 [M+H]⁺.

### Preparation of Intermediate Z35

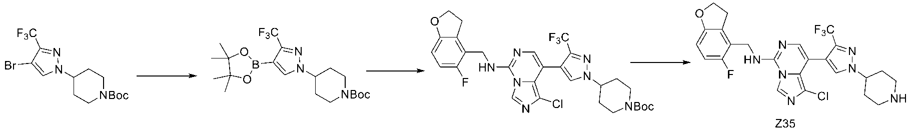

Step 1: Tert-butyl 4-(4-bromo-3-(trifluoromethyl)-1H-pyrazol-1-yl)piperidine-1-carboxylate (762 mg, 3 mmol), bis(pinacolato)diboron (774 mg, 3 mmol), potassium acetate (2.7 g, 9 mmol), and catalyst [1,1'-bis(diphenylphosphinoferrocene]dichloropalladium(II) (146 mg, 0.2 mmol) were added to 5 ml of dioxane. After purging with nitrogen three times, the mixture was heated to 110°C and stirred for 15 hours. After the reaction was complete, silica gel was directly added to the mixture, which was stirred for sample loading. Column chromatography was performed using 30% to 100% PE/EA. The target product was concentrated to give compound tert-butyl 4-(4-(4,4,5,5-tetramethyl-1,3,2-dioxaborolan-2-yl)-3-(trifluoromethyl)-1H-pyrazol-1-yl)piperidine-1-carboxylate (890 mg, 2 mmol). Yield: 66%. MS m/z (ESI): 446 [M+H]⁺.

Step 2: Tert-butyl 4-(4-(4,4,5,5-tetramethyl-1,3,2-dioxaborolan-2-yl)-3-(trifluoromethyl)-1H-pyrazol-1-yl)piperidine-1-carboxylate (890 mg, 2 mmol), 8-bromo-1-chloro-N-((5-fluoro-2,3-dihydrobenzofuran-4-yl)methyl)imidazo[1,5-c]pyrimidin-5-amine (792 mg, 3 mmol), potassium carbonate (828 mg, 6 mmol), catalyst [1,1'-bis(diphenylphosphinoferrocene]dichloropalladium(II) (146 mg, 0.2 mmol), and 1 ml of water were sequentially added to 5 ml of dioxane. After purging with nitrogen three times, the mixture was heated to 100°C and stirred for 15 hours. After the reaction was complete, silica gel was directly added to the mixture, which was stirred for sample loading. Column chromatography was performed using 30% to 100% PE/EA. The target product was concentrated to give compound tert-butyl 4-(4-(1-chloro-5-(((5-fluoro-2,3-dihydrobenzofuran-4-yl)methyl)amino) imidazo[1,5-c]pyrimidin-8-yl)-3-(trifluoromethyl)-1H-pyrazol-1-yl)piperidine-1-carboxylate (952 mg, 1.5 mmol). Yield: 75%. MS m/z (ESI): 636 [M+H]⁺.

Step 3: Tert-butyl 4-(4-(1-chloro-5-(((5-fluoro-2,3-dihydrobenzofuran-4-yl)methyl)amino) imidazo[1,5-c]pyrimidin-8-yl)-3-(trifluoromethyl)-1H-pyrazol-1-yl)piperidine-1-carboxylate (952 mg, 1.5 mmol) was added to a mixed solution of 5 ml of dichloromethane and 1 ml of trifluoroacetic acid. The mixture was stirred at room temperature for 1 hour, and then concentrated and dried under oil pump vacuum to give compound Z35 (802 mg, 0.5 mmol). Yield: 100%. MS m/z (ESI): 536 [M+H]⁺.

### Preparation of Intermediate Z36

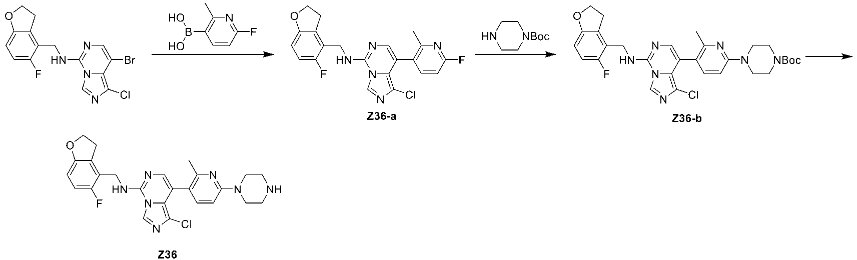

Step 1: 8-Bromo-1-chloro-N-((5-fluoro-2,3-dihydrobenzofuran-4-yl)methyl)imidazo[1,5-c]pyrimidin-5-amine (0.6 g, 1.51 mmol) and 2-fluoro-6-methylpyridine-5-boronic acid (350.68 mg, 2.26 mmol) were dissolved in dioxane (15 mL), water (2 mL), and DMSO (1 mL). Under argon protection, Pd(dppf)Cl₂ (110.41 mg, 150.89 µmol) and K₂CO₃ (625.63 mg, 4.53 mmol) were added. The temperature was raised to 100°C, and the mixture was stirred for 2 hours. After cooling to room temperature, water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (12 g, 0% to 30% EA/PE) to give Z36-a (0.55 g, 1.29 mmol, 85.20% yield) as a light brown solid. MS m/z (ESI): 428.1 [M+H]⁺.

Step 2: Z36-a (100 mg, 233.74 µmol) and tert-butyl piperazine-1-carboxylate (217.67 mg, 1.17 mmol) were dissolved in NMP (3 mL). DIEA (90.63 mg, 701.21 µmol, 122.14 µL) was added, and the mixture was heated under microwave to 200°C and stirred for 2 hours. After cooling to room temperature, Boc₂O (153.04 mg, 701.21 µmol) was added dropwise, and the mixture was stirred at room temperature for 0.5 hours. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (4 g, 0% to 30% EA/DCM) to give Z36-b (80 mg, 134.66 µmol, 57.61% yield) as a light yellow solid. MS m/z (ESI): 594.3 [M+H]⁺.

Step 3: Z36-b (80 mg, 134.66 µmol) was dissolved in DCM (2 mL). TFA (1 mL) was added, and the mixture was stirred at room temperature for 1 hour. The resultant was concentrated under reduced pressure to remove the solvent. Dichloromethane and saturated aqueous sodium bicarbonate solution were added, and extraction was performed three times with dichloromethane. The combined organic phases were dried over anhydrous sodium sulfate, and concentrated under reduced pressure to give crude product Z36 (60 mg, 121.47 µmol, 90.20% yield) as a light yellow solid, which was used directly in the next step without purification. MS m/z (ESI): 494.2 [M+H]⁺.

### Preparation of Intermediate Z37

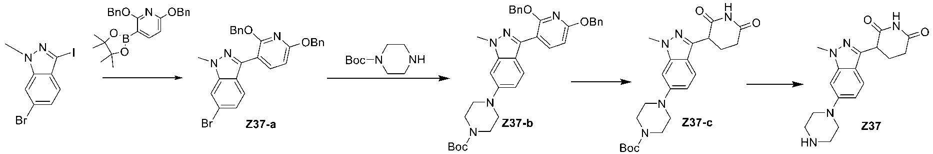

Step 1: 6-Bromo-3-iodo-1-methyl-1H-indazole (5.2 g, 15.43 mmol) and 2,6-bis(benzyloxy)-3-(4,4,5,5-tetramethyl-1,3,2-dioxaborolan-2-yl)pyridine (7.41 g, 17.75 mmol) were dissolved in 1,4-dioxane (80 mL) and water (20 mL). Pd(dppf)Cl₂ (1.13 g, 1.54 mmol) and Cs₂CO₃ (10.06 g, 30.86 mmol) were then added thereto, and the mixture was purged with nitrogen. The reaction mixture was stirred at 100°C for 3 hours. After the reaction was complete, the mixture was cooled to room temperature, quenched with water, and extracted with ethyl acetate (120 mL×3). The combined organic phases were dried over anhydrous sodium sulfate, and filtered. The filtrate was concentrated under reduced pressure. Purification by CombiFlash column chromatography (24 g, 0% to 100% EA/PE) successfully gave product Z37-a (4.9 g, 63.45% yield) as a white solid. MS m/z (ESI): 500.1 [M+H]⁺.

Step 2: Z37-a (1.5 g, 3.00 mmol) and tert-butyl piperazine-1-carboxylate (837.48 mg, 4.50 mmol) were dissolved in dioxane (15 mL). Under argon protection, Pd₂(dba)₃ (274.50 mg, 299.77 µmol), X-Phos (285.81 mg, 599.54 µmol), and Cs₂CO₃ (1.95 g, 6.00 mmol) were added. The temperature was raised to 100°C and stirred overnight. The resultant was concentrated under reduced pressure to remove the solvent. Water and ethyl acetate were then added, and extraction was performed three times with ethyl acetate. The combined organic phases were washed with brine, dried over anhydrous sodium sulfate, and concentrated under reduced pressure to give a crude product. The residue was separated by CombiFlash column chromatography (20 g, 0% to 30% EA/PE) to give Z37-b (1.7 g, 2.81 mmol, 93.62% yield) as a light brown solid. MS m/z (ESI): 606.3 [M+H]⁺.

Step 3: Z37-b (1.7 g, 2.81 mmol) was dissolved in ethanol (30 mL) and THF (30 mL). Pd/C (297.50 mg, 280.66 µmol, 10% purity) was added, and the mixture was purged with a hydrogen balloon three times. The mixture was stirred at room temperature overnight. The catalyst was filtered off, and the mother liquor was concentrated under reduced pressure to give crude product Z37-c (1.1 g, 2.57 mmol, 91.68% yield) as a light yellow solid. It was used directly in the next step without purification. MS m/z (ESI): 428.2 [M+H]⁺.

Step 4: Z37-c (0.5 g, 1.17 mmol) was dissolved in DCM (6 mL). TFA (2 mL) was added, and the mixture was stirred at room temperature for 1 hour. The resultant was concentrated under reduced pressure to remove the solvent. Dichloromethane and saturated aqueous sodium bicarbonate solution were added, and extraction was performed three times with dichloromethane. The combined organic phases were dried over anhydrous sodium sulfate, and concentrated under reduced pressure to give crude product Z37 (0.25 g, 763.64 µmol, 65.29% yield) as a light yellow solid. It was used directly in the next step without purification. MS m/z (ESI): 328.2 [M+H]⁺.

### Preparation of Intermediate Z38

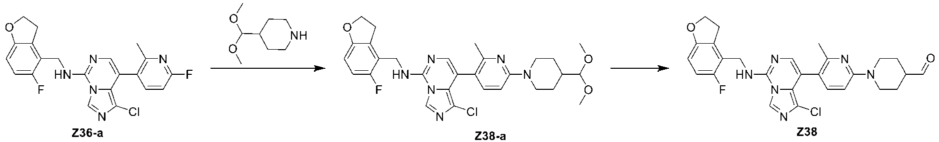

Step 1: Z36-a (150 mg, 350.60 µmol) and 4-(dimethoxymethyl)piperidine (279.12 mg, 1.75 mmol) were dissolved in NMP (3 mL). DIEA (135.94 mg, 1.05 mmol, 183.20 µL) was added, and the mixture was heated under microwave to 200°C and stirred for 1 hour. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (4 g, 0% to 40% EA/PE) to give Z38-a (150 mg, 264.53 µmol, 75.45% yield) as a light yellow solid. MS m/z (ESI): 567.3 [M+H]⁺.

Step 2: Z38-a (150 mg, 264.53 µmol) was dissolved in DCM (3 mL). TFA (1 mL) was added, and the mixture was stirred at room temperature for 1 hour. The resultant was concentrated under reduced pressure to remove the solvent. Dichloromethane and saturated aqueous sodium bicarbonate solution were added, and extraction was performed three times with dichloromethane. The combined organic phases were dried over anhydrous sodium sulfate, and concentrated under reduced pressure to give crude product Z38 (0.1 g, 191.94 µmol, 72.56% yield) as a light yellow solid, which was used directly in the next step without purification. MS m/z (ESI): 521.2 [M+H]⁺.

### Preparation of Intermediate Z39

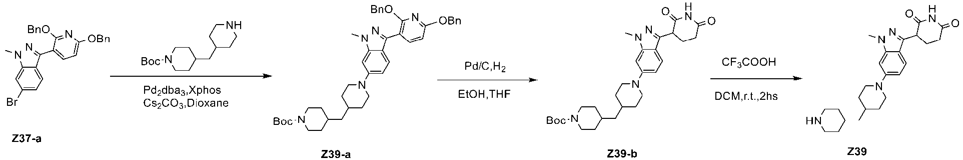

Using Z37-a as starting material and referring to the method of Z37, Z39 was prepared. MS m/z (ESI): 424.3 [M+H]⁺.

### Preparation of Intermediate Z40

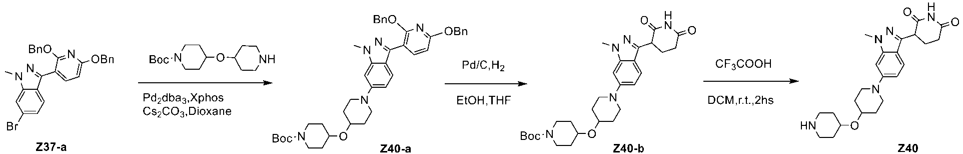

Using Z37-a as starting material and referring to the method of Z37, Z40 was prepared. MS m/z (ESI): 426.3 [M+H]⁺.

### Preparation of Intermediate Z41

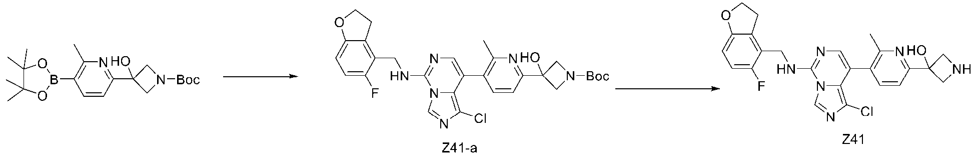

Step 1: Compound 8-bromo-1-chloro-N-[(5-fluoro-2,3-dihydrobenzofuran-4-yl)methyl] imidazo[1,5-c]pyrimidin-5-amine (150 mg, 377.24 µmol) and tert-butyl 3-hydroxy-3-[6-methyl-5-(4,4,5,5-tetramethyl-1,3,2-dioxaborolan-2-yl)-2-pyridyl]azetidine-1-carboxylate (147.23 mg, 377.24 µmol) were dissolved in dioxane (5 mL) and water (0.5 mL). Under argon protection, [1,1'-bis(diphenylphosphino)ferrocene]dichloropalladium(II) (276.03 mg, 377.24 µmol) and potassium carbonate (52.14 mg, 377.24 µmol) were added sequentially. The temperature was raised to 95°C, and the mixture was stirred overnight. Reaction progress was monitored by LC-MS. The reaction mixture was concentrated to dryness under reduced pressure, and purified by silica gel column chromatography (MeOH:DCM = 0% to 10%) to give Z41-a (110 mg, 189.32 µmol, 50.19% yield) as a yellow solid. MS m/z (ESI): 581 [M+H]⁺.

Step 2: Z41-a (113.11 mg, 194.68 µmol) was dissolved in dichloromethane (5 mL). Trifluoroacetic acid (1.83 g, 16.08 mmol, 1 mL) was added with stirring at room temperature, and the mixture was stirred at room temperature for 2 hours with the reaction progress monitored by LC-MS. The reaction mixture was concentrated to dryness under reduced pressure, added with saturated sodium carbonate solution, extracted with EA (30 mL×2), dried over anhydrous sodium sulfate, and concentrated to dryness under reduced pressure to give Z41 (60 mg, 124.76 µmol, 64.09% yield) as a yellow solid. MS m/z (ESI): 481 [M+H]⁺.

### Preparation of Intermediate Z42

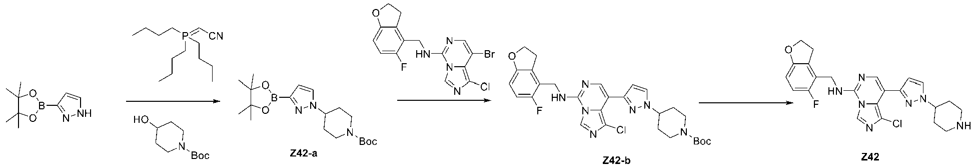

Step 1: 3-(4,4,5,5-Tetramethyl-1,3,2-dioxaborolan-2-yl)-1H-pyrazole (500 mg, 2.58 mmol), tert-butyl 4-hydroxypiperidine-1-carboxylate (1.04 g, 5.15 mmol), and cyanomethylenetributylphosphorane (1.24 g, 5.15 mmol) were dissolved in toluene (10 mL). After purging with argon, the mixture was stirred at 90°C for 16 hours with product generated. The reaction mixture was concentrated to dryness under reduced pressure, and the concentrate was purified by combiflash (EA:PE = 0% to 40%) to give product Z42-a (550 mg, crude) as a yellow oil. MS m/z (ESI): 378.3 [M+H]⁺.

Step 2: 8-Bromo-1-chloro-N-[(5-fluoro-2,3-dihydrobenzofuran-4-yl)methyl]imidazo[1,5-c]pyrimidin-5-amine (150 mg, 377.24 µmol) and Z42-a (426.98 mg, 1.13 mmol) were dissolved in a mixed solution of 1,4-dioxane (10 mL), H₂O (2 mL), and DMSO (0.5 mL). PdCl₂(dppf) (82.81 mg, 113.17 µmol) and potassium carbonate (260.69 mg, 1.89 mmol) were added. After purging with argon, the mixture was stirred at 95°C for 3 hours, and the starting material was completely reacted. The reaction mixture was concentrated to dryness under reduced pressure to give a crude product, which was purified by combiflash (EA:PE = 0% to 40%) to give product Z42-b (105 mg, yield: 49.00%) as a yellow oil. MS m/z (ESI): 512.2 [M-56+H]⁺.

Step 3: Z42-b (105 mg, 184.85 µmol) was dissolved in DCM (5 mL). Trifluoroacetic acid (21.08 mg, 184.85 µmol, 1 mL) was added, and the mixture was stirred at room temperature (25°C) for 2 hours, and the starting material was completely reacted. The reaction mixture was concentrated to dryness under reduced pressure. Triethylamine (1 ml) was added to basify the product, and the resultant was further concentrated to dryness under reduced pressure to give crude product Z42 (86 mg, crude) as a yellow oil, which was used directly in the next step without further purification. MS m/z (ESI): 468.1 [M+H]⁺.

### Preparation of Intermediate Z43

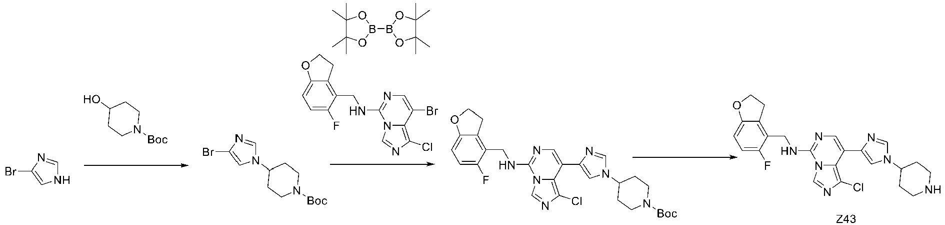

Following the above route and referring to the preparation method of Z42, Z43 was obtained. MS m/z (ESI): 468.1 [M+H]⁺.

### Preparation of Intermediate Z44

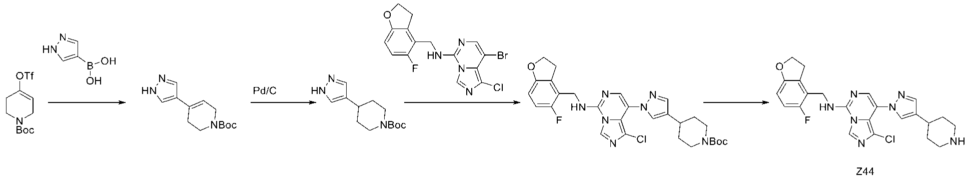

Following the above route and referring to the preparation method of Z42, Z44 was obtained. MS m/z (ESI): 468.1 [M+H]⁺.

### Preparation of Intermediate Z45

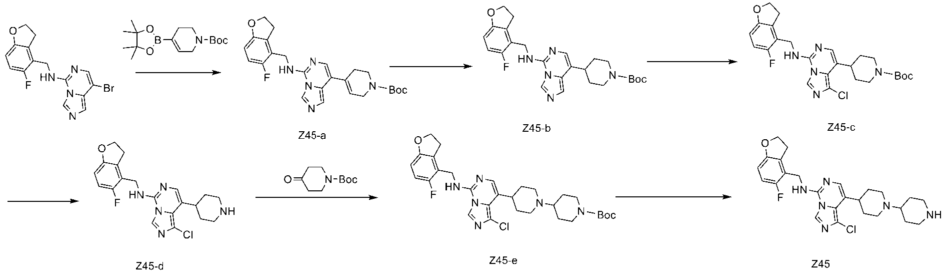

Step 1: 8-Bromo-N-((5-fluoro-2,3-dihydrobenzofuran-4-yl)methyl)imidazo[1,5-c]pyrimidin-5-amine (2 g, 5.51 mmol) and tert-butyl 4-(4,4,5,5-tetramethyl-1,3,2-dioxaborolan-2-yl)-3,6-dihydro-2H-pyridine-1-carboxylate (2.04 g, 6.61 mmol) were dissolved in 1,4-dioxane (30 mL) and water (6 mL). Pd(dppf)Cl₂ (805.88 mg, 1.10 mmol) and K₂CO₃ (2.28 g, 16.52 mmol) were then added. The reaction mixture was stirred at 100°C for 3 hours. After the reaction was complete, the resultant was concentrated under reduced pressure to remove the solvent. The residue was separated by CombiFlash column chromatography (12 g, 0% to 100% EA/PE) to successfully give product Z45-a (2.2 g, 85.82% yield) as a light yellow solid. MS m/z (ESI): 466.2 [M+H]⁺.

Step 2: Z45-a (2.2 g, 4.73 mmol) was dissolved in MeOH (40 mL). Pd(OH)₂/C (774.70 mg, 1.26 mmol, 20% purity) was then added, and the reaction mixture was stirred at room temperature under hydrogen atmosphere for 18 hours. After the reaction was complete, the mixture was filtered, and the filtrate was concentrated to give crude product Z45-b (2.1 g, 95.04% yield) as a light yellow solid. MS m/z (ESI): 468.2 [M+H]⁺.

Step 3: Compound Z45-b (2.1 g, 4.49 mmol) was dissolved in DMF (40 mL). NCS (599.78 mg, 4.49 mmol) was added under ice bath, and the reaction was allowed to warm to room temperature under ice bath with stirring. The reaction mixture was stirred overnight. After the starting material was completely reacted, the reaction mixture was directly concentrated to dryness under reduced pressure, and purified by CombiFlash column chromatography (12 g, 0% to 40% EA/DCM) to give product Z45-c (2.1 g, 93.14% yield) as an off-white solid. MS m/z (ESI): 502.2 [M+H]⁺.

Step 4: Z45-c (2.1 g, 4.18 mmol) was dissolved in DCM (30 mL). HCl (4 M in dioxane, 10 mL) was then added thereto, and the reaction mixture was stirred at room temperature for 2 hours. After the reaction was complete, the reaction mixture was concentrated, and then neutralized with saturated aqueous sodium bicarbonate solution with a solid precipitated. The solid was filtered, washed twice with water, and then washed twice with a small amount of methanol. The filter cake was dried under vacuum to give crude product Z45-d (1.3 g, 77.33% yield) as a light yellow solid. MS m/z (ESI): 402.1 [M+H]⁺.

Step 5: Z45-d (500 mg, 1.24 mmol) and tert-butyl 4-oxopiperidine-1-carboxylate (1.49 g, 7.47 mmol) were dissolved in DMSO (9 mL) and EtOH (3 mL). NaBH₃CN (781.88 mg, 12.44 mmol) and AcOH (373.58 mg, 6.22 mmol) were then added, and the reaction mixture was stirred under microwave at 85°C for 0.7 hours. After the reaction was complete, the reaction mixture was poured into water, and extracted with dichloromethane (50 mL×3). The combined organic phases were dried over anhydrous sodium sulfate, and filtered. The filtrate was concentrated to dryness under reduced pressure. Purification by CombiFlash column chromatography (24 g, 0% to 100% THF/PE) successfully gave product Z45-e (620 mg, 85.17% yield) as a light yellow solid. MS m/z (ESI): 585.3 [M+H]⁺.

Step 6: Z45-e (620 mg, 1.06 mmol) was dissolved in DCM (20 mL). CF₃COOH (120.82 mg, 1.06 mmol, 6 mL) was then added, and the reaction mixture was stirred at room temperature for 2 hours. After the reaction was complete, the mixture was concentrated, and neutralized with aqueous sodium bicarbonate solution with a solid precipitated. The solid was filtered, and the filter cake was washed with water and dichloromethane. The filter cake was dried under vacuum to give product Z45 (420 mg, 81.73% yield) as a light yellow solid. MS m/z (ESI): 485.2 [M+H]⁺.

### Preparation of Intermediate Z46

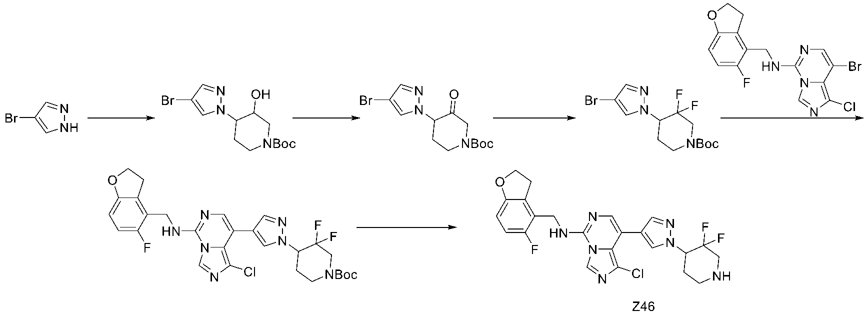

Step 1: 4-Bromo-1H-pyrazole (7 g, 47.63 mmol) and tert-butyl 7-oxa-3-azabicyclo[4.1.0]heptane-3-carboxylate (9.96 g, 50.01 mmol) were dissolved in DMSO (100 mL), and Cs₂CO₃ (31.04 g, 95.26 mmol) was added. The reaction mixture was stirred at 80°C for 4 hours. The reaction mixture was filtered, and washed. The filtrate was diluted with EA, washed three times with saturated brine, and concentrated under reduced pressure. The crude product was separated by CombiFlash column chromatography (0% to 70% EA/PE) to obtain tert-butyl 4-(4-bromopyrazol-1-yl)-3-hydroxy-piperidine-1-carboxylate (15.46 g, 93.76% yield) as a yellow solid. MS m/z (ESI): 290.0 [M+H-56]⁺.

Step 2: Tert-butyl 4-(4-bromopyrazol-1-yl)-3-hydroxy-piperidine-1-carboxylate (2.9 g, 8.38 mmol) was dissolved in DCM (20 mL), and Dess-Martin oxidant (5.33 g, 12.56 mmol) was added. The reaction mixture was stirred at room temperature for 20 hours. The reaction mixture was poured into water, neutralized by adding saturated NaHCO₃ solution, filtered, washed, extracted, dried over anhydrous sodium sulfate, and concentrated under reduced pressure. The crude product was separated by CombiFlash column chromatography (0% to 70% EA/PE) to obtain tert-butyl 4-(4-bromopyrazol-1-yl)-3-oxo-piperidine-1-carboxylate (2.4 g, 83.24% yield) as a yellow solid. MS m/z (ESI): 288.0 [M+H-56]⁺.

Step 3: Tert-butyl 4-(4-bromopyrazol-1-yl)-3-oxo-piperidine-1-carboxylate (3.95 g, 11.48 mmol) was dissolved in DCM (40 mL), cooled to -78°C, and N-ethyl-N-(trifluoro-sulfanyl)ethanamine (5.55 g, 34.43 mmol, 4.55 mL) was added. The reaction mixture was stirred at room temperature for 20 hours. The reaction mixture was poured into water, extracted with DCM, dried over anhydrous sodium sulfate, and concentrated under reduced pressure. The crude product was separated by CombiFlash column chromatography (0% to 70% EA/PE) to obtain tert-butyl 4-(4-bromopyrazol-1-yl)-3,3-difluoro-piperidine-1-carboxylate (2.5 g, 59.49% yield) as a yellow solid. MS m/z (ESI): 310.0 [M+H-56]⁺.

Step 4: 8-Bromo-1-chloro-N-[(5-fluoro-2,3-dihydrobenzofuran-4-yl)methyl]imidazo[1,5-c]pyrimidin-5-amine (360 mg, 905.37 µmol) and tert-butyl 4-(4-bromopyrazol-1-yl)-3,3-difluoro-piperidine-1-carboxylate (663.09 mg, 1.81 mmol) were dissolved in DME (15 mL) and water (1.5 mL), and palladium acetate (40.65 mg, 181.07 µmol), di(1-adamantyl)butylphosphine (129.84 mg, 362.15 µmol), K₂CO₃ (501 mg, 3.62 mmol), and (Pin)₂B₂ (919.63 mg, 3.62 mmol) were added. The reaction mixture was reacted at 70°C under argon atmosphere for 6 hours. The reaction mixture was poured into water, extracted with ethyl acetate, dried over anhydrous sodium sulfate, concentrated under reduced pressure, and the crude product was separated by CombiFlash column chromatography (0% to 100% EA/PE) to obtain tert-butyl 4-[4-[1-chloro-5-[(5-fluoro-2,3-dihydrobenzofuran-4-yl)methylamino]imidazo[1,5-c]pyrimidin-8-yl]pyrazol-1-yl]-3,3-difluoro-piperidine-1-carboxylate (177 mg, 32.37% yield) as a yellow solid. MS m/z (ESI): 548.2 [M+H-56]⁺.

Step 5: Tert-butyl 4-[4-[1-chloro-5-[(5-fluoro-2,3-dihydrobenzofuran-4-yl)methylamino] imidazo[1,5-c]pyrimidin-8-yl]pyrazol-1-yl]-3,3-difluoro-piperidine-1-carboxylate (177 mg, 293.04 µmol) was dissolved in DCM (15.00 mL), and trifluoroacetic acid (3 mL) was added. The reaction mixture was stirred at room temperature for 4 hours. The reaction mixture was concentrated under reduced pressure, neutralized with NaHCO₃ solution, extracted with DCM, dried over anhydrous sodium sulfate, and concentrated under reduced pressure. The crude product, 1-chloro-8-[1-(3,3-difluoropiperidin-4-yl)pyrazol-4-yl]-N-[(5-fluoro-2,3-dihydrobenzofuran-4-yl)meth yl]imidazo[1,5-c]pyrimidin-5-amine (130 mg, 88.04% yield), was directly used in the next step. MS m/z (ESI): 504.2 [M+H]⁺.

### Preparation of Intermediate Z47

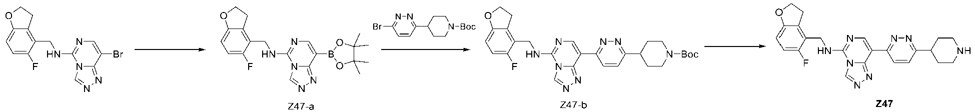

Step 1: 8-Bromo-N-((5-fluoro-2,3-dihydrobenzofuran-4-yl)methyl)-[1,2,4]triazolo[4,3-c] pyrimidin-5-amine (0.8 g, 2.20 mmol) was dissolved in THF (30 mL), and n-BuLi (2.5 M, 4.39 mL) was added dropwise at -78°C. The reaction mixture was stirred for 1 hour. Triisopropyl borate (4.96 g, 26.36 mmol, 6.08 mL) was slowly added dropwise, and the reaction mixture was stirred for 1 hour. The reaction mixture was quenched with 1N HCl, stirred at room temperature for 0.5 hours, then extracted with ethyl acetate twice, and concentrated under reduced pressure. The residue was purified by combiflash with DCM:MeOH=10:1 to obtain Z47-a (22 mg, 66.85 µmol, 3.04% yield) as a yellow oil. MS m/z (ESI): 412.1 [M+H]⁺.

Step 2: Under argon atmosphere, tert-butyl 4-(6-bromopyridazin-3-yl)piperidine-1- carboxylate (100 mg, 292.20 µmol), Z47-a (400.67 mg, 438.30 µmol), Pd(dppf)Cl₂ (21.38 mg, 29.22 µmol), potassium carbonate (121.15 mg, 876.60 µmol), 1,4-dioxane (4 mL), and water (1 mL) were heated to 115°C and reacted for 2 hours. The reaction mixture was filtered, the filtrate was separated to obtain the aqueous layer, which was then concentrated to give a crude product. The crude product was purified by column chromatography (petroleum ether:ethyl acetate=30%:70%) to obtain crude Z47-b (135 mg, 29.64 µmol, 10.14% yield) as a yellow solid. MS m/z (ESI): 547.3 [M+H]⁺.

Step 3: At room temperature, Z47-b (125 mg, 228.69 µmol), TFA (0.5 mL), and DCM (0.5 mL) were mixed and reacted for 1 hour. The reaction mixture was concentrated to give a crude product. The crude product was dissolved in DCM:MeOH=10:1, and adjusted to pH=7 to 8 by adding saturated sodium bicarbonate solution. The organic layer was collected, dried over anhydrous sodium sulfate, and concentrated to give Z47 (100 mg, 223.97 µmol, 97.94% yield) as a yellow oil. The crude product was directly used in the next step. MS m/z (ESI): 447.2 [M+H]⁺.

### Preparation of Intermediate Z48

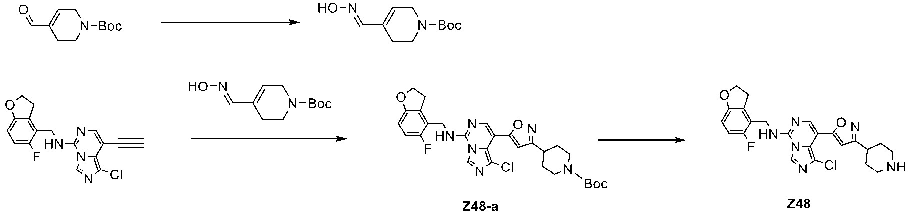

Step 1: Tert-butyl 4-formyl-3,6-dihydropyridine-1(2H)-carboxylate (5 g, 23.44 mmol) was dissolved in ethanol (50 mL). Hydroxylamine hydrochloride (2.45 g, 35.28 mmol) and pyridine (2.45 g, 31.03 mmol, 2.50 mL) were then added thereto, and the reaction mixture was stirred at room temperature for 6 hours. After the reaction was complete, water and ethyl acetate were added, which was extracted with ethyl acetate (150 mL×3), and washed with saturated brine (100 mL). The combined organic phases were dried over anhydrous sodium sulfate, and filtered. The filtrate was concentrated to give a crude product. Purification by CombiFlash column chromatography (40 g, 0% to 10% DCM/MeOH) successfully gave product tert-butyl (E)-4-((hydroxyimino)methyl)-3,6-dihydropyridine-1(2H)-carboxylate (1.2 g, 22.42% yield) as a white solid. MS m/z (ESI): 129.2 [M-100+H]⁺.

Step 2: Tert-butyl (E)-4-((hydroxyimino)methyl)-3,6-dihydropyridine-1(2H)-carboxylate (559.47 mg, 2.45 mmol) and 1-chloro-8-ethyl-N-((5-fluoro-2,3-dihydrobenzofuran-4-yl)methyl)imidazo[1,5-c]pyrimidin-5-amine (280 mg, 816.91 µmol) were dissolved in methanol (60 mL) and THF (15 mL). [Bis(trifluoroacetoxy)iodo]benzene (1.23 g, 2.86 mmol) was then added thereto, and the reaction mixture was stirred at room temperature for 20 hours. After the reaction was complete, the reaction was quenched with water, and the resultant was extracted with ethyl acetate (70 mL×3). The combined organic phases were dried over anhydrous sodium sulfate, and filtered. The filtrate was concentrated to dryness under reduced pressure. Purification by CombiFlash column chromatography (12 g, 0% to 50% EA/PE) successfully gave product Z48-a (87 mg, 18.72% yield) as a light yellow solid. MS m/z (ESI): 569.2 [M+H]⁺.

Step 3: Z48-a (87 mg, 152.89 µmol) was dissolved in DCM (4 mL). CF₃COOH (1.05 g, 9.17 mmol, 1 mL) was then added thereto. The reaction mixture was stirred at room temperature for 1 hour. After the reaction was complete, the mixture was neutralized with saturated sodium bicarbonate, and extracted with dichloromethane (70 mL×3). The combined organic phases were dried over anhydrous sodium sulfate, and filtered. The filtrate was concentrated to dryness under reduced pressure to successfully give product Z48 (65 mg, 90.66% yield) as a light yellow solid. MS m/z (ESI): 469.2 [M+H]⁺.

### Preparation of Intermediate Z49

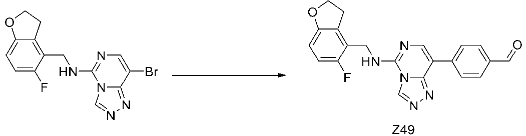

Compound 8-bromo-N-((5-fluoro-2,3-dihydrobenzofuran-4-yl)methyl)-[1,2,4]triazolo[4,3-c]pyrimidin-5-amine (726 mg, 2 mmol), 4-(4,4,5,5-tetramethyl-1,3,2-dioxaborolan-2- yl)benzaldehyde (464 mg, 2 mmol), potassium carbonate (414 mg, 3 mmol), and catalyst [1,1'-bis(diphenylphosphinoferrocene]dichloropalladium(II) (146 mg, 0.2 mmol) were added to a mixed solution of 5 ml of 1,4-dioxane and 1 ml of water. After purging with nitrogen three times, the mixture was heated to 100°C and stirred for 5 hours. After the reaction was complete, silica gel was directly added to the mixture, which was stirred for sample loading. Column chromatography was performed using 30% to 100% PE/EA. The target product was concentrated to give Z49 (391 mg, 1 mmol). Yield: 50%. MS m/z (ESI): 390 [M+H]⁺.

### Preparation of Intermediate Z50

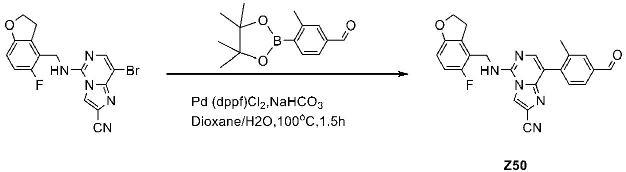

Intermediate Z50 was obtained referring to the preparation method of Z49. MS m/z (ESI): 428.1 [M+H]⁺.

### Preparation of Intermediate Z51

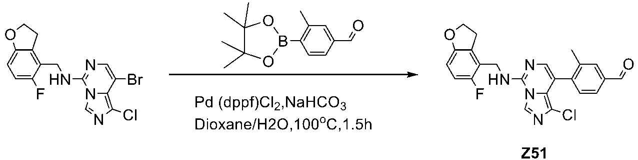

Intermediate Z51 was obtained referring to the preparation method of Z49. MS m/z (ESI): 437.1 [M+H]⁺.

### Preparation of Intermediate Z52

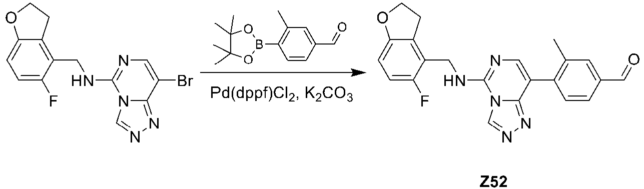

Intermediate Z52 was obtained referring to the preparation method of Z49. MS m/z (ESI): 404.1 [M+H]⁺.

### Preparation of Intermediate Z53

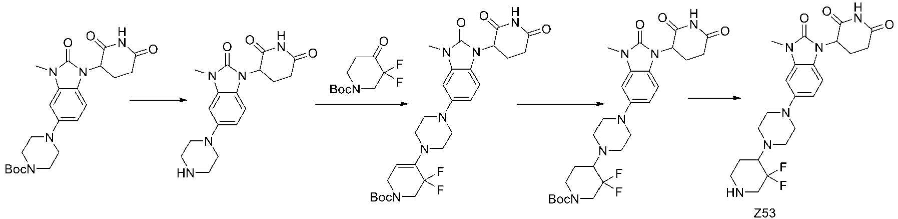

Step 1: Tert-butyl 4-[1-(2,6-dioxopiperidin-3-yl)-3-methyl-2-oxo-benzimidazol-5-yl] piperazine-1-carboxylate (240 mg, 541.16 µmol) was dissolved in DCM (15 mL), and trifluoroacetic acid (3 mL) was added. The reaction mixture was stirred at room temperature for 4 hours. The reaction mixture was concentrated under reduced pressure, neutralized to basicity with NaHCO₃ solution, extracted with dichloromethane (30 mL×3), dried over anhydrous sodium sulfate, and concentrated under reduced pressure. The obtained crude product, 3-(3-methyl-2-oxo-5-piperazin-1-yl-benzimidazol-1-yl)piperidine-2,6-dione (180 mg, 96.87% yield), was used directly in the next step. MS m/z (ESI): 344.2 [M+H]⁺.

Step 2: 3-(3-Methyl-2-oxo-5-piperazin-1-yl-benzimidazol-1-yl)piperidine-2,6-dione (180 mg, 524.20 µmol) and tert-butyl 3,3-difluoro-4-oxopiperidine-1-carboxylate (185 mg, 786.30 µmol) were dissolved in toluene (6 mL) and acetonitrile (3 mL). Sodium acetate (175.40 mg, 1.31 mmol) and acetic acid (293.72 mg, 4.89 mmol) were added. The reaction mixture was stirred at 120°C for 20 hours. The reaction mixture was poured into water, neutralized to basicity with saturated NaHCO₃ solution, extracted with dichloromethane (30 mL×3), dried over anhydrous sodium sulfate, and concentrated under reduced pressure. The obtained crude product, tert-butyl 4-[4-[1-(2,6-dioxo-3-piperidinyl)-3-methyl-2-oxo-benzimidazol-5-yl]piperazin-1-yl]-3,3-difluoropiperidine-1-carboxylate (280 mg, 94.94% yield), was used directly in the next step. MS m/z (ESI): 561.3 [M+H]⁺.

Step 3: Tert-butyl 4-[4-[1-(2,6-dioxo-3-piperidinyl)-3-methyl-2-oxo-benzimidazol-5-yl]piperazin-1-yl]-3,3-difluoropiperidine-1-carboxylate (280 mg, 499.47 µmol) was dissolved in dichloroethane (10 mL) and methanol (10 mL). AcOH (73.32 mg, 1.22 mmol) and NaBH₃CN (156.94 mg, 2.50 mmol) were added. The reaction mixture was stirred at room temperature overnight. The reaction mixture was poured into water, neutralized with saturated NaHCO₃, extracted with dichloromethane (30 mL×3), dried over anhydrous sodium sulfate, and concentrated under reduced pressure. The obtained crude product, tert-butyl 4-[4-[1-(2,6-dioxo-3-piperidinyl)-3-methyl-2-oxo-benzimidazol-5-yl]piperazin-1-yl]-3,3-difluoropiperidine-1-carboxylate (100 mg, 35.59% yield), was used directly in the next step. MS m/z (ESI): 563.3 [M+H]⁺.

Step 4: Tert-butyl 4-[4-[1-(2,6-dioxo-3-piperidinyl)-3-methyl-2-oxo-benzimidazol-5-yl]piperazin-1-yl]-3,3-difluoropiperidine-1-carboxylate (100 mg, 177.74 µmol) was dissolved in DCM (15 mL), and trifluoroacetic acid (3 mL) was added. The reaction mixture was stirred at room temperature for 2 hours. The reaction mixture was concentrated under reduced pressure, neutralized to basicity with NaHCO₃ solution, extracted with dichloromethane (30 mL×3), dried over anhydrous sodium sulfate, and concentrated under reduced pressure. The obtained crude product Z53 (80 mg, 97.32% yield) was used directly in the next step. MS m/z (ESI): 463.2 [M+H]⁺.

### Preparation of Intermediate Z54

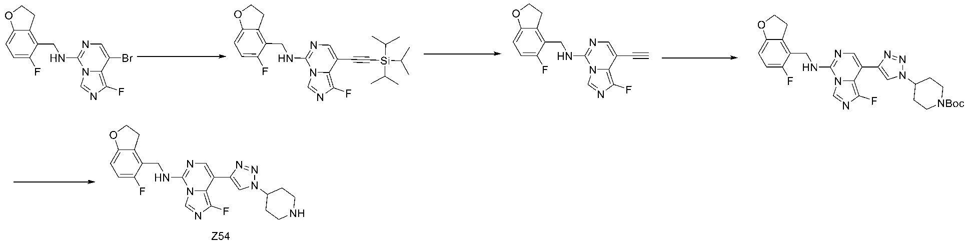

Step 1: Compound 8-bromo-1-fluoro-N-((5-fluoro-2,3-dihydrobenzofuran-4-yl)methyl)imidazo[1,5-c]pyrimidin-5-amine (191 mg, 0.5 mmol) and ethynyl(triisopropyl)silane (0.46 g, 2.52 mmol) were dissolved in THF (5 mL). Copper(I) iodide (29 mg, 0.15 mmol), Pd(PPh₃)₂Cl₂ (71 mg, 0.1 mmol), and diisopropylamine (0.51 g, 5.03 mmol, 0.69 mL) were then added. After purging with nitrogen three times, the reaction mixture was reacted at 75°C under argon protection for 48 hours. After the reaction was complete, silica gel was directly added to the mixture, which was stirred for sample loading. Column chromatography was performed using 30% to 100% PE/EA. The target product was concentrated to give compound 1-fluoro-N-((5-fluoro-2,3-dihydrobenzofuran-4-yl)methyl)-8-((triisopropylsilyl)ethynyl)imidazo[1,5-c]pyrimidin-5-amine (170 mg, 0.35 mmol). Yield: 70%. MS m/z (ESI): 483 [M+H]⁺.

Step 2: Compound 1-fluoro-N-((5-fluoro-2,3-dihydrobenzofuran-4-yl)methyl)-8-((triisopropylsilyl)ethynyl)imidazo[1,5-c]pyrimidin-5-amine (170 mg, 0.35 mmol) was added to 0.5 M TBAF-THF solution (3 mL) and stirred at room temperature for 2 hours. After the reaction was complete, silica gel was directly added to the mixture, which was stirred for sample loading. Column chromatography was performed using 30% to 100% PE/EA. The target product was concentrated to give compound 8-ethynyl-1-fluoro-N-((5-fluoro-2,3-dihydrobenzofuran-4-yl)methyl)imidazo[1,5-c]pyrimidin-5-amine (65 mg, 0.2 mmol). Yield: 57%. MS m/z (ESI): 327 [M+H]⁺.

Step 3: Compound 8-ethynyl-1-fluoro-N-((5-fluoro-2,3-dihydrobenzofuran-4-yl)methyl) imidazo[1,5-c]pyrimidin-5-amine (65 mg, 0.2 mmol) and tert-butyl 4-azidopiperidine-1- carboxylate (130 mg, 0.58 mmol) were dissolved in t-BuOH (5 mL) and water (1 mL). Sodium ascorbate (12 mg, 70 µmol) and CuSO₄·5H₂O (7 mg, 29 µmol) were added. After purging with argon, the mixture was stirred at 70°C for 12 hours. After the reaction was complete, silica gel was directly added to the mixture, which was stirred for sample loading. Column chromatography was performed using 30% to 100% PE/EA. The target product was concentrated to give compound tert-butyl 4-(4-(1-fluoro-5-(((5-fluoro-2,3-dihydrobenzofuran-4-yl)methyl)amino)imidazo[1,5-c]pyrimidin-8-yl)-1H-1,2,3-triazol-1-yl)piperidine-1-carboxylate (10 mg, 18 µmol). Yield: 9%. MS m/z (ESI): 553 [M+H]⁺.

Step 4: Compound tert-butyl 4-(4-(1-fluoro-5-(((5-fluoro-2,3-dihydrobenzofuran-4-yl) methyl)amino)imidazo[1,5-c]pyrimidin-8-yl)-1H-1,2,3-triazol-1-yl)piperidine-1-carboxylate (10 mg, 18 µmol) was added to a mixed solvent of 2 ml of dichloromethane and 0.5 ml of trifluoroacetic acid. After stirring at room temperature for 1 hour, the solvent was removed by rotary evaporation under oil pump vacuum to give crude compound Z54 (8.1 mg, 18 µmol). Yield: 100%. MS m/z (ESI): 453 [M+H]⁺.

### Preparation of Intermediate Z55

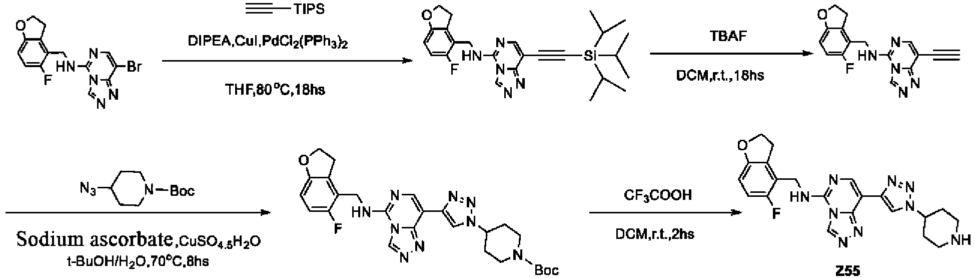

Following the above synthetic route and referring to the preparation method of Z54, Z55 was obtained. MS m/z (ESI): 436.2 [M+H]⁺.

### Preparation of Intermediate Z56

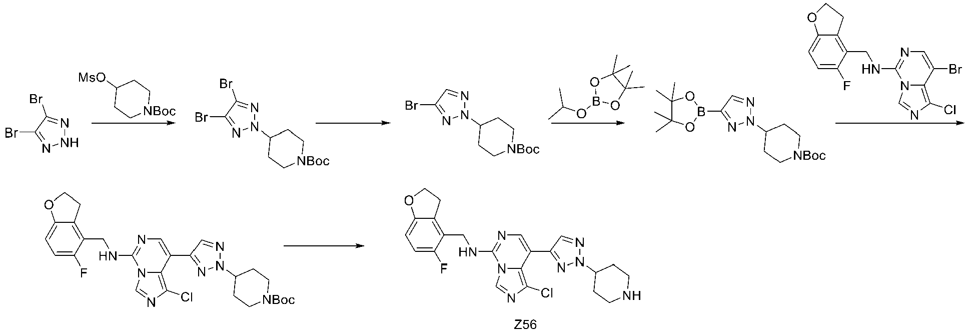

Step 1: 4,5-Dibromo-2H-triazole (5 g, 22.04 mmol) and tert-butyl 4-methylsulfonyloxypiperidine-1-carboxylate (6.46 g, 23.14 mmol) were dissolved in DMF (110 mL). Cs₂CO₃ (21.54 g, 66.12 mmol) was added. The reaction mixture was stirred at 100°C overnight. The reaction mixture was poured into water, extracted with ethyl acetate (30 mL×3), dried over anhydrous sodium sulfate, and concentrated under reduced pressure. The obtained crude product was separated by CombiFlash (0% to 50% EA/PE) to give product tert-butyl 4-(4,5-dibromotriazol-2-yl)piperidine-1-carboxylate (6.27 g, 69.37% yield) as a colorless oil. MS m/z (ESI): 352.9 [M+H-56]⁺.

Step 2: Tert-butyl 4-(4,5-dibromotriazol-2-yl)piperidine-1-carboxylate (6.27 g, 15.29 mmol) was dissolved in tetrahydrofuran (45 mL), cooled to -78°C, and n-BuLi (2.5 M, 6.07 mL) was slowly added dropwise. The reaction mixture was stirred at -78°C for 1 hour. NH₄Cl solution was added to quench the reaction. Extraction was performed with ethyl acetate (30 mL×3), dried over anhydrous sodium sulfate, and concentrated under reduced pressure. The crude product was separated by CombiFlash (0% to 60% EA/PE) to give tert-butyl 4-(4-bromotriazol-2-yl)piperidine-1-carboxylate (980 mg, 19.35% yield) as an anhydrous oil. MS m/z (ESI): 275.0 [M+H-56]⁺.

Step 3: Tert-butyl 4-(4-bromotriazol-2-yl)piperidine-1-carboxylate (490 mg, 1.48 mmol) was dissolved in THF (20 mL), cooled to -78°C, and n-BuLi (2.5 M, 769.30 µL) was slowly added dropwise. The reaction mixture was stirred at -78°C for 0.5 h, then 2-isopropoxy-4,4,5,5-tetramethyl-1,3,2-dioxaborolane (551 mg, 2.96 mmol) was added. The reaction mixture was slowly warmed to room temperature and stirred for another 2 hours. The reaction mixture was cooled to 0°C, and saturated NH₄Cl solution was added dropwise to quench the reaction. The resultant was extracted with ethyl acetate (30 mL×3), dried over anhydrous sodium sulfate, and concentrated under reduced pressure to give crude product tert-butyl 4-[4-(4,4,5,5-tetramethyl-1,3,2-dioxaborolan-2-yl)triazol-2-yl]piperidine-1-carboxylate (550 mg, 98.28% yield), which was used directly in the next step. MS m/z (ESI): 197.1 [M+H-182]⁺.

Step 4: 8-Bromo-1-chloro-N-[(5-fluoro-2,3-dihydrobenzofuran-4-yl)methyl]imidazo[1,5-c]pyrimidin-5-amine (100 mg, 251.49 µmol) and tert-butyl 4-[4-(4,4,5,5-tetramethyl-1,3,2-dioxaborolan-2-yl)triazol-2-yl]piperidine-1-carboxylate (114.16 mg, 301.79 µmol) were dissolved in dioxane (12 mL) and water (2 mL). PdCl₂(dppf) (18.40 mg, 25.15 µmol) and potassium carbonate (69.51 mg, 502.98 µmol) were added. The reaction mixture was stirred at 100°C overnight. The reaction mixture was poured into water, extracted with ethyl acetate (30 mL×3), dried over anhydrous sodium sulfate, and concentrated under reduced pressure. The crude product was separated by CombiFlash column chromatography (0% to 80% EA/PE) to give product tert-butyl 4-[4-[1-chloro-5-[(5-fluoro-2,3-dihydrobenzofuran-4-yl)methylamino] imidazo[1,5-c]pyrimidin-8-yl]triazol-2-yl]piperidine-1-carboxylate (22 mg, 15.37% yield) as a yellow solid. MS m/z (ESI): 513.2 [M+H-56]⁺.

Step 5: Tert-butyl 4-[4-[1-chloro-5-[(5-fluoro-2,3-dihydrobenzofuran-4-yl)methylamino] imidazo[1,5-c]pyrimidin-8-yl]triazol-2-yl]piperidine-1-carboxylate (22 mg, 38.66 µmol) was dissolved in dichloromethane (10 mL), and trifluoroacetic acid (3 mL) was added. The reaction mixture was stirred at room temperature for 2 hours. The reaction mixture was concentrated under reduced pressure, neutralized to basicity with NaHCO₃ solution, extracted with dichloromethane (30 mL×3), dried over anhydrous sodium sulfate, and concentrated under reduced pressure. The obtained crude product Z56 (18 mg, 99.29% yield) was used directly in the next step. MS m/z (ESI): 469.2 [M+H]⁺.

### Preparation of Intermediate Z57

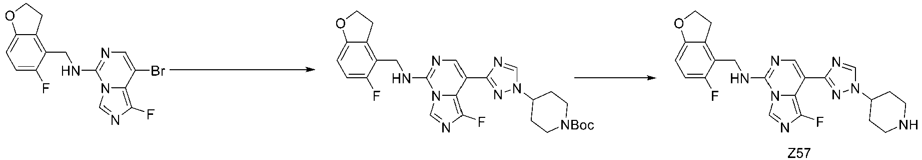

Step 1: Compound 8-bromo-1-fluoro-N-((5-fluoro-2,3-dihydrobenzofuran-4-yl)methyl) imidazo[1,5-c]pyrimidin-5-amine (570 mg, 1.5 mmol), n-butyldi(1-adamantyl)phosphine (214 mg, 598 µmol), diacetoxypalladium (67 mg, 300 µmol), K₂CO₃ (620 mg, 4.48 mmol), and (Pin)₂B₂ (1140 mg, 4.48 mmol) were sequentially added to a mixed solution of DME (100 mL) and water (10 mL). After purging with nitrogen three times, the reaction mixture was reacted at 70°C under argon protection for 20 hours. After the reaction was complete, silica gel was directly added to the mixture, which was stirred for sample loading. Column chromatography was performed using 30% to 100% PE/EA. The target product was concentrated to give compound tert-butyl 4-(3-(1-fluoro-5-(((5-fluoro-2,3-dihydrobenzofuran-4-yl)methyl)amino)imidazo[1,5-c]pyrimidin-8-yl)-1H-1,2,4-triazol-1-yl)piperidine-1-carboxylate (83 mg, 0.15 mmol). Yield: 10%. MS m/z (ESI): 553 [M+H]⁺.

Step 2: Compound tert-butyl 4-(3-(1-fluoro-5-(((5-fluoro-2,3-dihydrobenzofuran-4-yl)methyl)amino)imidazo[1,5-c]pyrimidin-8-yl)-1H-1,2,4-triazol-1-yl)piperidine-1-carboxylate (83 mg, 0.15 mmol) was added to a mixed solution of 5 ml of dichloromethane and 1 ml of trifluoroacetic acid. The mixture was stirred at room temperature for 1 hour, and then concentrated and dried under oil pump vacuum to give compound Z57 (68 mg, 0.15 mmol). Yield: 100%. MS m/z (ESI): 453 [M+H]⁺.

### Preparation of Intermediate Z58

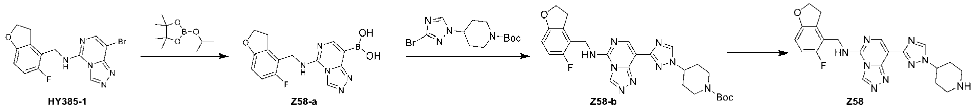

Step 1: 8-Bromo-N-((5-fluoro-2,3-dihydrobenzofuran-4-yl)methyl)-[1,2,4]triazolo[4,3-c] pyrimidin-5-amine (1 g, 2.75 mmol) was dissolved in THF (30 mL). At -78°C, n-BuLi (2.5 M, 5.49 mL) was added dropwise, and the reaction was carried out for 1 hour. Isopropoxy pinacol borate (6.13 g, 32.95 mmol, 6.72 mL) was slowly added dropwise, and the mixture was stirred at low temperature for 1 hour. The reaction mixture was quenched with saturated ammonium chloride solution, stirred at room temperature for 0.5 hours, and then extracted twice with ethyl acetate. The combined organic phases were washed with brine, dried over anhydrous sodium sulfate, and concentrated under reduced pressure to give a crude product. The crude product was purified by preparative liquid chromatography (preparative column: 21.2×250 mm C18 column; system: 10 mM FA H₂O/acetonitrile; wavelength: 254/214 nm; gradient: 5%-95% acetonitrile change) to give Z58-a (0.2 g, 607.73 µmol, 22.13% yield) as a light yellow solid. MS m/z (ESI): 330.1 [M+H]⁺.

Step 2: Z58-a (198.72 mg, 603.85 µmol) and tert-butyl 4-(3-bromo-1H-1,2,4-triazol-1-yl) piperidine-1-carboxylate (200 mg, 603.85 µmol) were dissolved in dioxane (8 mL) and water (1 mL). Under argon protection, Pd(dppf)Cl₂ (44.18 mg, 60.38 µmol) and K₂CO₃ (166.92 mg, 1.21 mmol) were added. The temperature was raised to 120°C, and the mixture was stirred for 2 hours. The resultant was concentrated under reduced pressure to remove the solvent. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (4 g, 0% to 10% MeOH/DCM) to give Z58-b (40 mg, 74.69 µmol, 12.37% yield) as a light yellow solid. MS m/z (ESI): 536.3 [M+H]⁺.

Step 3: Z58-b (40 mg, 74.69 µmol) was dissolved in DCM (3 mL). TFA (1 mL) was added. The mixture was stirred at room temperature for 1 hour. The resultant was concentrated under reduced pressure to remove the solvent and to give crude product Z58 (30 mg, 68.89 µmol, 92.24% yield) as a light yellow solid. It was used directly in the next step without purification. MS m/z (ESI): 436.2 [M+H]⁺.

### Preparation of Intermediate Z61

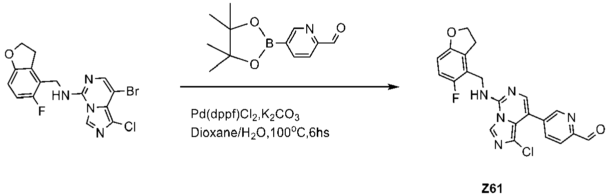

Intermediate Z61 was obtained referring to the preparation method of Z49. MS m/z (ESI): 424.1 [M+H]⁺.

### Preparation of Intermediate Z62

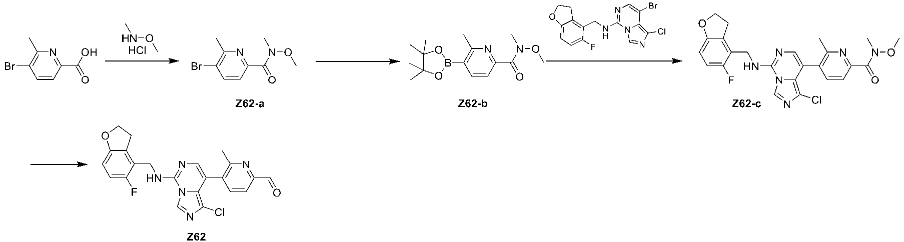

Step 1: 5-Bromo-6-methylpicolinic acid (2 g, 9.26 mmol) and dimethylhydroxylamine hydrochloride (993.35 mg, 10.18 mmol, HCl) were dissolved in THF (30 mL). TEA (3.75 g, 37.03 mmol, 5.16 mL) and HATU (4.19 g, 11.11 mmol) were added, and the mixture was stirred at room temperature for 2 hours. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (20 g, 0% to 30% EA/PE) to give Z62-a (2.2 g, 8.49 mmol, 91.72% yield) as a light yellow oil. MS m/z (ESI): 259.0 [M+H]⁺.

Step 2: Z62-a (2.2 g, 8.49 mmol) and bis(pinacolato)diboron (3.23 g, 12.74 mmol) were dissolved in dioxane (30 mL). Under argon protection, potassium acetate (2.50 g, 25.47 mmol) and Pd(dppf)Cl₂ (621.29 mg, 849.09 µmol) were added. The temperature was raised to 95°C and stirred overnight. The solid was filtered off, and the filtrate was concentrated under reduced pressure to give a crude product. The residue was separated by CombiFlash column chromatography (20 g, 0% to 30% EA/PE) to give Z62-b (2.4 g, 7.84 mmol, 92.32% yield) as a light yellow solid. MS m/z (ESI): 307.2 [M+H]⁺.

Step 3: 8-Bromo-1-chloro-N-((5-fluoro-2,3-dihydrobenzofuran-4-yl)methyl)imidazo[1,5-c] pyrimidin-5-amine (0.5 g, 1.26 mmol) and Z62-b (577.48 mg, 1.89 mmol) were dissolved in dioxane (10 mL) and water (2 mL). Under argon protection, K₂CO₃ (347.57 mg, 2.51 mmol) and Pd(dppf)Cl₂ (92.01 mg, 125.75 µmol) were added. The temperature was raised to 100°C and stirred for 3 hours. Water and ethyl acetate were added, and extraction was performed three times with ethyl acetate. The combined organic phases were washed with brine, dried over anhydrous sodium sulfate, and concentrated under reduced pressure to give a crude product. The residue was separated by CombiFlash column chromatography (12 g, 0% to 30% EA/PE) to give Z62-c (0.4 g, 804.96 µmol, 64.01% yield) as a light yellow solid. MS m/z (ESI): 497.2 [M+H]⁺.

Step 4: Z62-c (0.3 g, 603.72 µmol) was dissolved in THF (10 mL). Under argon protection, the temperature was lowered to -78°C, and DIBAL-H (1 M, 2.41 mL) was slowly added dropwise. The mixture was stirred at low temperature for 3 hours. Saturated aqueous ammonium chloride solution was added dropwise at low temperature to quench the reaction. A large amount of anhydrous sodium sulfate and ethyl acetate were added. The solid was filtered off, and the mother liquor was concentrated under reduced pressure to give a crude product. The residue was separated by CombiFlash column chromatography (4 g, 0% to 60% EA/PE) to give Z62 (0.2 g, 456.77 µmol, 75.66% yield) as a light yellow solid. MS m/z (ESI): 438.1 [M+H]⁺.

### Preparation of Intermediate Z63

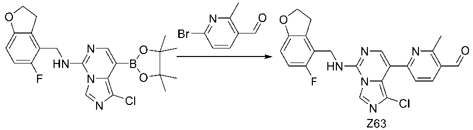

Intermediate Z63 was prepared referring to step 2 of Z47. MS m/z (ESI): 438.1 [M+H]⁺.

### Preparation of Intermediate Z64

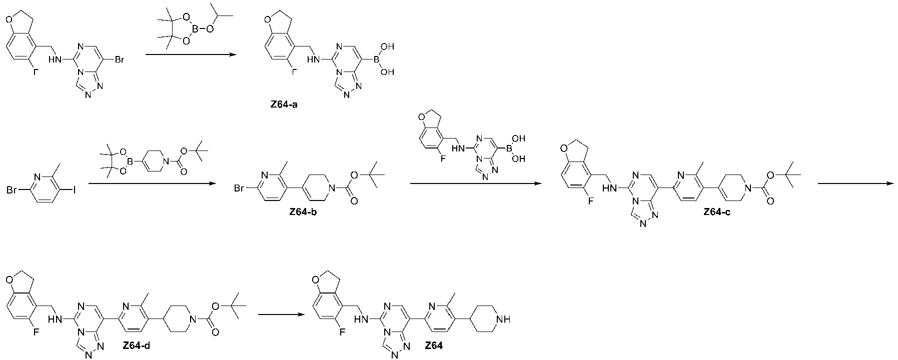

Step 1: Under argon protection, 8-bromo-N-((5-fluoro-2,3-dihydrobenzofuran-4-yl)methyl)-[1,2,4]triazolo[4,3-c]pyrimidin-5-amine (1 g, 2.75 mmol) was dissolved in THF (50 mL), and n-BuLi (2.5 M, 5.49 mL) was added dropwise at -78°C. After reaction at -78°C for 1 hour, 2-isopropoxy-4,4,5,5-tetramethyl-1,3,2-dioxaborolane was slowly added dropwise, and the mixture was reacted at -78°C for 1 hour. The reaction solution was quenched with saturated ammonium chloride solution, stirred at room temperature for 0.5 hour, and then extracted twice with ethyl acetate, and dried by rotary evaporation to give a crude product. The crude product was purified by prep-HPLC to give Z64-a (200 mg, 486.34 µmol, yield 17.71%) as a white solid. MS m/z (ESI): 330.1 [M+H]⁺.

Step 2: 6-Bromo-3-iodo-2-methylpyridine (1 g, 3.36 mmol) and tert-butyl 4-(4,4,5,5-tetramethyl-1,3,2-dioxaborolan-2-yl)-3,6-dihydropyridine-1(2H)-carboxylate (1.04 g, 3.36 mmol) were dissolved in 1,4-dioxane (20 mL), water (4 mL), and DMSO (2 mL). Pd(dppf)Cl₂ (245.60 mg, 335.66 µmol) and K₂CO₃ (927.84 mg, 6.71 mmol) were then added. The mixture was stirred at 65°C for 3 hours. The reaction solution was concentrated, water and ethyl acetate were then added.The mixture was extracted with ethyl acetate, washed three times with saturated brine, dried, filtered, and concentrated. The resultant was purified by CombiFlash column chromatography (petroleum ether:ethyl acetate = 70%:30%) to give Z64-b (1.2 g, 3.16 mmol, yield 94.20%) as a colorless oil. MS m/z (ESI): 353.1 [M+H]⁺.

Step 3: Under argon protection, Z64-b (153.50 mg, 434.53 µmol), Z64-a (110 mg, 334.25 µmol), Pd(dppf)Cl₂ (43.17 mg, 66.85 µmol), K₂CO₃ (115.49 mg, 835.63 µmol), 1,4-dioxane (10 mL), and water (0.5 mL) were mixed and heated to 100°C, and the mixture was reacted for 3 hours. Water and ethyl acetate were added to the reaction solution, the organic layer was separated, washed with saturated brine, and concentrated to give a crude product. The crude product was purified by column chromatography (petroleum ether:ethyl acetate = 20%:80%) to give Z64-c (170 mg, 304.87 µmol, yield 91.21%) as a yellow oil. MS m/z (ESI): 558.3 [M+H]⁺.

Step 4: Under hydrogen atmosphere, Z64-c (70 mg, 125.53 µmol), platinum dioxide (2.85 mg, 12.55 µmol), methanol (2 mL), and EA (2 mL) were stirred at room temperature for 6 hours. The reaction solution was filtered and concentrated to give Z64-d (70 mg, 125.08 µmol, yield 99.64%) as a yellow solid oil. The crude product was used directly in the next step. MS m/z (ESI): 560.3 [M+H]⁺.

Step 5: At room temperature, Z64-d (70 mg, 125.08 µmol), TFA (1 mL), and DCM (1 mL) were stirred for 1 hour. The reaction solution was concentrated to give a crude product. The crude product was dissolved in dichloromethane, saturated sodium bicarbonate was added to adjust the pH to 9-10, then the mixture was extracted with dichloromethane:methanol = 10:1 (10 mL×2). The organic layers were combined, dried over anhydrous sodium sulfate and concentrated to give Z64 (50 mg, 108.81 µmol, yield 86.99%) as a yellow solid. MS m/z (ESI): 460.2 [M+H]⁺.

### Preparation of Intermediate Z65

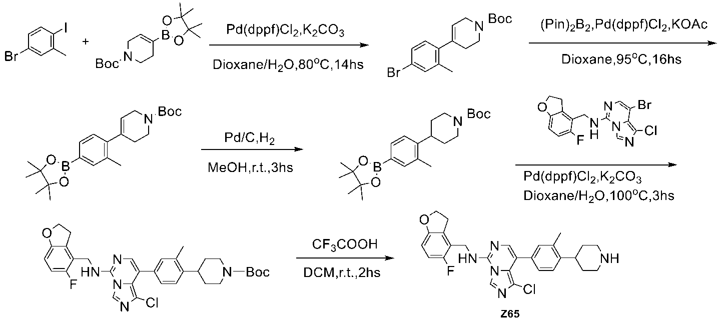

Intermediate Z65 was prepared according to the above synthetic route and by reference to the preparation method of Z56. MS m/z (ESI): 492.2 [M+H]⁺.

### Preparation of Intermediate Z66

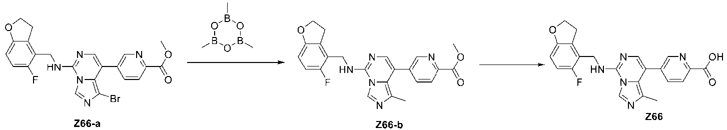

Step 1: Z66-a (200 mg, 401.36 µmol) was dissolved in DMF (4 mL). Trimethylcyclotriboroxane (3.5 M, 1 mL), K₂CO₃ (443.76 mg, 3.21 mmol), and Pd(dppf)Cl₂ (88.10 mg, 120.41 µmol) were then added. The mixture was stirred under microwave at 120°C for 3 hours. After the reaction was complete, the solvent was dried by rotary evaporation under reduced pressure, and the residue was separated by CombiFlash (12 g, 0-100% DCM/EA) to successfully give product Z66-b (127 mg, 293.01 µmol, 73.00% yield) as a light yellow solid. MS m/z (ESI): 434.1 [M+H]⁺.

Step 2: Z66-b (127 mg, 293.01 µmol) was dissolved in THF (6 mL) and water (2 mL). LiOH (70.17 mg, 2.93 mmol) was then added. The mixture was stirred at room temperature for 2 hours, then neutralized with dilute hydrochloric acid (2N), filtered, and the solid was dried under vacuum to successfully give product Z66 (87 mg, 207.44 µmol, 70.79% yield) as a yellow solid. MS m/z (ESI): 420.2 [M+H]⁺.

### Preparation of Intermediate Z67

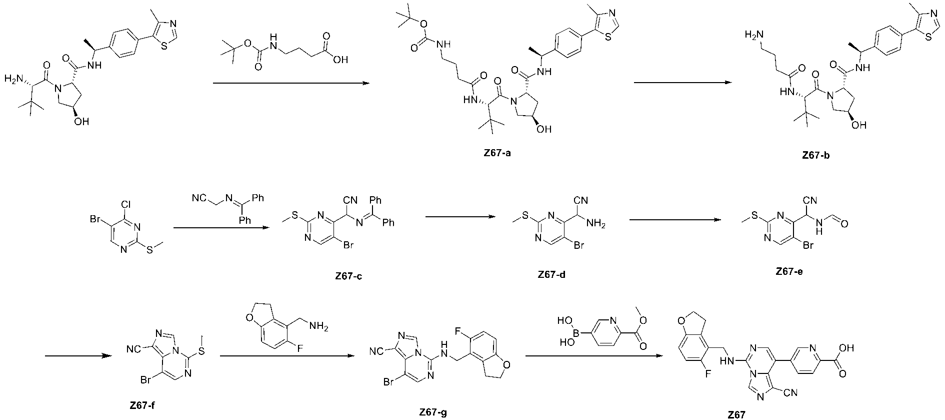

Step 1: 4-((tert-Butoxycarbonyl)amino)butanoic acid (502.84 mg, 2.47 mmol) and (2S,4R)-1-((S)-2-amino-3,3-dimethylbutanoyl)-4-hydroxy-N-((S)-1-(4-(4-methylthiazol-5-yl)phenyl)et hyl)pyrrolidine-2-carboxamide (1 g, 2.25 mmol) were dissolved in DMF (25 mL). DIPEA (872.09 mg, 6.75 mmol, 1.18 mL) and HATU (1.27 g, 3.37 mmol) were then added. The mixture was stirred at room temperature for 1 hour. The reaction solution was then dried by rotary evaporation under reduced pressure and purified by column chromatography CombiFlash (24 g, 0-10% MeOH/DCM) to successfully give product Z67-a (0.8 g, 1.27 mmol, 56.47% yield) as a light yellow solid. MS m/z (ESI): 530.3 [M-100+H]⁺.

Step 2: Z67-a (0.8 g, 1.27 mmol) was dissolved in DCM (20 mL). HCl (4 M in EA, 5 mL) was then added. The mixture was stirred at room temperature for 1 hour. The reaction solution was then concentrated to give product Z67-b (719 mg, 1.27 mmol, 99.98% yield, HCl) as a yellow solid. MS m/z (ESI): 530.3 [M+H]⁺.

Step 3: 5-Bromo-4-chloro-2-(methylthio)pyrimidine (9.66 g, 43.84 mmol) was dissolved in DMF (120 mL). The temperature was lowered to 0°C, and then NaH (3.19 g, 83.34 mmol, 60% purity) was added portionwise. The mixture was stirred at 0°C for 1 hour. Then a solution of 5-bromo-4-chloro-2-(methylamino)pyrimidine (10 g, 41.75 mmol) in DMF (120 mL) was added dropwise to the reaction solution. After the addition, the mixture was warmed to room temperature and stirred for 2 hours. After the reaction was complete, the mixture was quenched with saturated aqueous ammonium chloride solution, extracted with ethyl acetate (250 mL×3), washed with water (100 mL×3), washed with saturated brine (100 mL×2). The organic phases were combined, dried over anhydrous sodium sulfate, filtered, and the filtrate was dried by rotary evaporation under reduced pressure to give crude product Z67-c (15 g, 35.43 mmol, 84.87% yield) as an oil. MS m/z (ESI): 423.0 [M+H]⁺.

Step 4: At 0°C, aqueous HCl (3 M, 30 mL) was added to a solution of Z67-c (15 g, 35.43 mmol) in THF (200 mL). The reaction solution was stirred at room temperature for 1 hour. The reaction solution was then concentrated, basified to pH 8-9 with aqueous Na₂CO₃ solution, extracted with DCM (500 mL×2), washed with saturated brine (100 mL). The organic phases were combined, dried over anhydrous sodium sulfate, filtered, and the filtrate was concentrated under reduced pressure to successfully give product Z67-d (8.5 g, 32.80 mmol, 92.57% yield) as a red oil. MS m/z (ESI): 259.0 [M+H]⁺.

Step 5: A mixture of formic acid (39 g, 720.26 mmol, 31.97 mL, 85% purity) and acetic anhydride (34.8 g, 328.21 mmol, 32.01 mL) was heated at 50°C for 1 hour. The reaction mixture was then cooled to room temperature and added to a solution of Z67-d (8.5 g, 32.80 mmol) in DCM (100 mL). The reaction solution was stirred at room temperature for 2 hours. After the reaction was complete, the mixture was concentrated, water (500 mL) was added, and the mixture was extracted with DCM (500 mL×3). The organic phases were combined, washed sequentially with water (200 mL) and brine (100 mL), dried over anhydrous sodium sulfate, filtered, and the filtrate was concentrated to successfully give product Z67-e (8.5 g, 29.60 mmol, 90.25% yield) as a light yellow solid. MS m/z (ESI): 287.0 [M+H]⁺.

Step 6: POCl₃ (9.05 g, 50.18 mmol, 7.42 mL, 85% purity) was added dropwise to a solution of Z67-e (8.5 g, 29.60 mmol) in dioxane (83.96 mL). The reaction mixture was then heated to reflux for 4 hours. The reaction solution was cooled to room temperature and concentrated, diluted with DCM (100 mL), and adjusted to pH = 8 with saturated aqueous NaHCO₃ solution. The mixture was extracted with DCM (200 mL×3). The organic phases were combined, washed with brine (50 mL), dried over anhydrous sodium sulfate, filtered, and the filtrate was concentrated. The resultant was purified by CombiFlash (40 g, 0-100% PE/EA) successfully to give product Z67-f (6.5 g, 24.15 mmol, 81.59% yield) as a light yellow solid. MS m/z (ESI): 269.0 [M+H]⁺.

Step 7: At 0°C, Z67-f (1.6 g, 5.95 mmol) was dissolved in DCM (35 mL). m-CPBA (3.73 g, 18.39 mmol, 85% purity) was then added. The mixture was allowed to warm to room temperature and stirred for 18 hours. Then (5-fluoro-2,3-dihydrobenzofuran-4-yl)methanamine (1.19 g, 7.13 mmol) and sodium carbonate (3.14 g, 29.60 mmol) were added, and the mixture was stirred at room temperature for 4 hours. After the reaction was complete, the reaction solution was filtered, and the filtrate was dried by rotary evaporation under reduced pressure. The resultant was purified by CombiFlash (12 g, 0-60% DCM/MeOH) successfully to give product Z67-g (600 mg, 1.55 mmol, 26.00% yield) as a light yellow solid. MS m/z (ESI): 388.0 [M+H]⁺.

Step 8: Z67-g (225 mg, 579.61 µmol) and (6-methoxycarbonyl-3-pyridyl)boronic acid (209.76 mg, 1.16 mmol) were dissolved in dioxane (10 mL) and water (3 mL). K₂CO₃ (400.53 mg, 2.90 mmol) and Pd(dppf)Cl₂ (127.23 mg, 173.88 µmol) were then added. The mixture was stirred at 105°C for 2 hours. After the reaction was complete, the mixture was extracted twice with water (25 mL×2). The aqueous phases were combined, neutralized with HCl (2N), and the precipitated solid was filtered out and dried under vacuum to successfully give product Z67 (170 mg, 394.99 µmol, 68.15% yield) as a brown solid. MS m/z (ESI): 431.1 [M+H]⁺.

### Preparation of Intermediate Z68

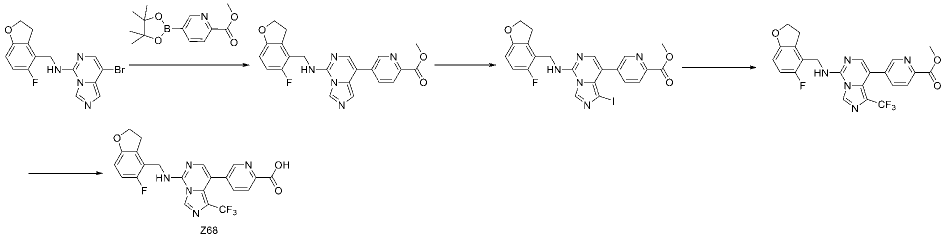

Step 1: 8-Bromo-N-[(5-fluoro-2,3-dihydrobenzofuran-4-yl)methyl]imidazo[1,5-c]pyrimidin-5-amine (300 mg, 0.83 mmol) and methyl 5-(4,4,5,5-tetramethyl-1,3,2-dioxaborolan-2-yl)pyridine-2-carboxylate (652 mg, 2.48 mmol) were dissolved in 1,4-dioxane (20 mL) and water (3 mL). Tetrakis(triphenylphosphine)palladium(0) (191 mg, 0.16 mmol) and potassium carbonate (228 mg, 1.65 mmol) were added. The reaction solution was stirred at 100°C for 20 hours. The solvent was concentrated under reduced pressure, and the crude product was separated by CombiFlash (0-10% MeOH/DCM) to give product methyl 5-[5-[(5-fluoro-2,3-dihydrobenzofuran-4-yl)methylamino] imidazo[1,5-c]pyrimidin-8-yl]pyridine-2-carboxylate (340 mg, 98.14% yield) as a yellow solid. MS m/z (ESI): 420.1 [M+H]⁺.

Step 2: Methyl 5-[5-[(5-fluoro-2,3-dihydrobenzofuran-4-yl)methylamino]imidazo[1,5-c] pyrimidin-8-yl]pyridine-2-carboxylate (323 mg, 0.77 mmol) was dissolved in tetrahydrofuran (10 mL), cooled to 0°C, and N-iodosuccinimide (173 mg, 0.77 mmol) was added. The reaction solution was stirred at 0°C for 1 hour. The reaction solution was poured into water, extracted with DCM (100 mL×3), dried over anhydrous sodium sulfate, and the solvent was concentrated under reduced pressure. The obtained crude product was separated by CombiFlash (0-10% MeOH/DCM) to give product methyl 5-[5-[(5-fluoro-2,3-dihydrobenzofuran-4-yl)methylamino]-1-iodoimidazo[1,5-c]pyrimidin-8-yl]pyridine-2-carboxylate (350 mg, 83.34% yield) as a yellow solid. MS m/z (ESI): 546.1 [M+H]⁺.

Step 3: Methyl 5-[5-[(5-fluoro-2,3-dihydrobenzofuran-4-yl)methylamino]-1-iodoimidazo[1,5-c] pyrimidin-8-yl]pyridine-2-carboxylate (350 mg, 0.64 mmol) was dissolved in DMF (15 mL). CuI (1.22 g, 6.42 mmol), PdCl₂(dppf) (47 mg, 0.064 mmol), and methyl 2,2-difluoro-2-(fluorosulfonyl)acetate (1.23 g, 6.42 mmol) were added. The reaction solution was stirred at 90°C overnight under argon protection. The reaction solution was filtered, washed, and the solvent was concentrated under reduced pressure. The obtained crude product was separated by CombiFlash (0-10% MeOH/DCM) to give product methyl 5-[5-[(5-fluoro-2,3-dihydrobenzofuran-4-yl)methylamino]-1-(trifluoromethyl)imidazo [1,5-c]pyrimidin-8-yl]pyridine-2-carboxylate (200 mg, 63.93% yield) as a yellow solid. MS m/z (ESI): 488.1 [M+H]⁺.

Step 4: Methyl 5-[5-[(5-fluoro-2,3-dihydrobenzofuran-4-yl)methylamino]-1-(trifluoromethyl) imidazo[1,5-c]pyrimidin-8-yl]pyridine-2-carboxylate (100 mg, 0.21 mmol) was dissolved in tetrahydrofuran (15 mL), and potassium trimethylsilanolate (40 mg, 0.31 mmol) was added. The reaction solution was stirred at room temperature for 4 hours. The reaction solution was separated by CombiFlash (0-80% MeOH/DCM) to give product Z68 (50 mg, 51.48% yield) as a yellow solid. MS m/z (ESI): 474.1 [M+H]⁺.

### Preparation of Intermediate H159-b

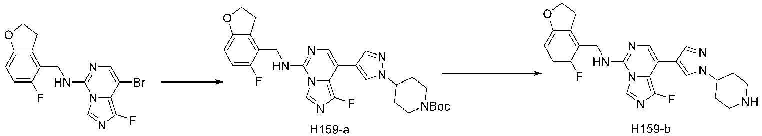

Step 1: Compound 8-bromo-1-fluoro-N-((5-fluoro-2,3-dihydrobenzofuran-4-yl)methyl)imidazo [1,5-c]pyrimidin-5-amine (760 mg, 2 mmol), tert-butyl 4-(4-(4,4,5,5-tetramethyl-1,3,2-dioxaborolan-2-yl)-1H-pyrazol-1-yl)piperidine-1-carboxylate (754 mg, 2 mmol), potassium carbonate (828 mg, 6 mmol), and catalyst [1,1'-bis(diphenylphosphinoferrocene]dichloropalladium(II) (146 mg, 0.2 mmol) were added to a mixed solvent of 5 ml of 1,4-dioxane and 1 ml of water. The mixture was purged with nitrogen three times, the mixture was heated to 100°C and stirred for 5 hours. After the reaction was complete, silica gel was directly added to the mixture for sample loading, and the resultant was purified by column chromatography using 30-100% PE/EA. The target product was concentrated to give compound H159-a (552 mg, 1 mmol). Yield: 50%. MS m/z (ESI): 552 [M+H]⁺.

Step 2: Compound H159-a (552 mg, 1 mmol) was added to a mixed solvent of 5 ml of dichloromethane and 1 ml of trifluoroacetic acid, the mixture was stirred at room temperature for 1 hour, and then concentrated and dried under oil pump vacuum to give compound H159-b (451 mg, 1 mmol). Yield: 100%. MS m/z (ESI): 452 [M+H]⁺.

### Preparation of Intermediate H80-d

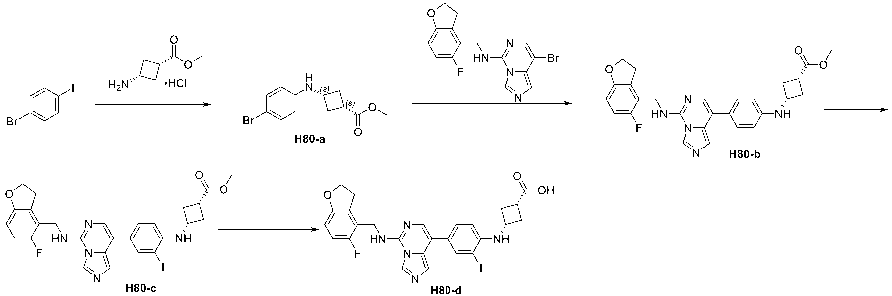

Step 1: Under argon protection, 1-bromo-4-iodobenzene (8.20 g, 28.98 mmol), methyl 3-aminocyclobutanecarboxylate hydrochloride (4 g, 24.15 mmol), L-proline (1.11 g, 9.66 mmol), copper(I) iodide (919.95 mg, 4.83 mmol), potassium carbonate (6.68 g, 48.30 mmol), and DMSO (40 mL) were mixed and heated to 80°C, and the mixture was reacted for 16 hours. The reaction solution was poured into water, extracted with ethyl acetate (20 mL×2), and the organic layers were combined, washed and concentrated to give a crude product. The crude product was purified by column chromatography (petroleum ether:ethyl acetate = 80%:20%) to give H80-a (4.6 g, 16.19 mmol, yield 67.03%) as a yellow solid. MS m/z (ESI): 284.1 [M+H]⁺.

Step 2: Under argon protection, H80-a (730 mg, 2.01 mmol), 8-bromo-N-((5-fluoro-2,3-dihydrobenzofuran-4-yl)methyl)imidazo[1,5-c]pyrimidin-5-amine (628.25 mg, 2.21 mmol), palladium acetate (90.25 mg, 402.00 µmol), n-butyldi(1-adamantyl)phosphine (144.13 mg, 402.00 µmol), potassium carbonate (1.11 g, 8.04 mmol), bis(pinacolato)diboron (1.02 g, 4.02 mmol), DME (20 mL), and water (2 mL) were mixed and heated to 70°C, and the mixture was reacted for 16 hours. The reaction solution was filtered, and the filtrate was extracted with ethyl acetate (20 mL×2). The organic layers were combined, washed with saturated brine, dried over anhydrous sodium sulfate, and concentrated to give a crude product. The crude product was purified by column chromatography (petroleum ether:ethyl acetate = 20%:80%) to give H80-b (540 mg, 1.11 mmol, yield 55.11%) as a yellow solid. MS m/z (ESI): 488.3 [M+H]⁺.

Step 3: H80-b (490 mg, 804.06 µmol), THF (20 mL), and TFA (2 mL) were mixed and stirred at room temperature for 5 minutes, then cooled to -15°C, and N-iodosuccinimide (180.90 mg, 804.06 µmol) was added. After addition, the mixture was reacted at -15°C for 15 minutes. The reaction solution was poured into ice water, saturated sodium bicarbonate solution was added to adjust the pH > 7, and then the mixture was extracted with ethyl acetate (10 mL×2). The organic layers were combined, washed with saturated brine, dried over anhydrous sodium sulfate, and concentrated to give a crude product. The crude product was purified by column chromatography (petroleum ether:ethyl acetate = 50%:50%) to give H80-c (55 mg, 89.66 µmol, yield 11.15%) as a yellow solid. MS m/z (ESI): 614.1 [M+H]⁺.

Step 4: At room temperature, lithium hydroxide (5.86 mg, 244.53 µmol) was added to a solution of H80-c (50 mg, 81.51 µmol) in THF (2 mL) and water (0.5 mL). After addition, the mixture was reacted at room temperature for 1 hour. The reaction solution was concentrated, 1N HCl was added to adjust the pH < 3, and the aqueous phase was lyophilized to give H80-d (48 mg, 80.08 µmol, yield 98.25%) as a white solid. MS m/z (ESI): 600.2 [M+H]⁺.

### Preparation of Intermediate H87-h

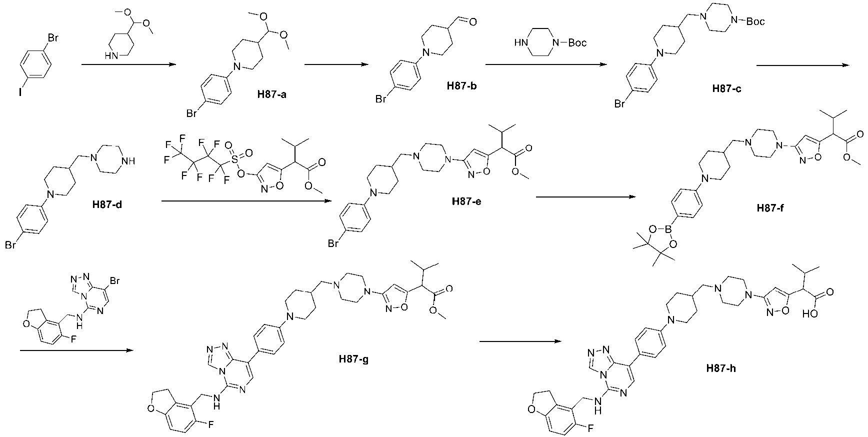

Step 1: Under argon protection, 1-bromo-4-iodobenzene (13.33 g, 47.10 mmol), 4-(dimethoxymethyl)piperidine (5 g, 31.40 mmol), L-proline (1.45 g, 12.56 mmol), copper(I) iodide (1.20 g, 6.28 mmol), potassium carbonate (8.68 g, 62.80 mmol), and DMSO (50 mL) were mixed and heated to 80°C, and the mixture was reacted for 16 hours. The reaction solution was filtered, and the filtrate was added to water, extracted with ethyl acetate (20 mL×2). The organic layers were combined, washed three times with water and once with saturated brine, then concentrated to give a crude product. The crude product was purified by column chromatography (petroleum ether:ethyl acetate = 70%:30%) to give H87-a (6.1 g, 19.41 mmol, yield 61.82%) as a yellow solid. MS m/z (ESI): 314.1 [M+H]⁺.

Step 2: At room temperature, TFA (5 mL) was added to a solution of H87-a (6.1 g, 19.41 mmol) in DCM (10 mL). After addition, the mixture was reacted at room temperature for 1 hour. The reaction solution was concentrated to give a crude product. The crude product was dissolved in dichloromethane, washed once with saturated sodium bicarbonate solution, washed once with saturated brine, dried over anhydrous sodium sulfate, and concentrated to give H87-b (5 g, 18.65 mmol, yield 96.05%) as a yellow liquid. The crude product was used directly in the next step. MS m/z (ESI): 268.1 [M+H]⁺.

Step 3: NaBH(OAc)₃ (13.66 g, 64.43 mmol) was added to a solution of tert-butyl piperazine-1-carboxylate (3 g, 16.11 mmol), H87-b (4.75 g, 17.72 mmol), DCM (50 mL), and AcOH (0.5 mL). After addition, the mixture was reacted at room temperature for 3 hours. The reaction solution was poured into water, extracted with dichloromethane (10 mL×2). The organic layers were combined, washed with saturated brine and concentrated to give a crude product. The crude product was purified by column chromatography (petroleum ether:ethyl acetate = 80%:20%) to give H87-c (4.8 g, 10.95 mmol, yield 67.97%) as a yellow solid. MS m/z (ESI): 438.0 [M+H]⁺.

Step 4: At room temperature, TFA (10 mL) was added to a solution of H87-c (4.8 g, 10.95 mmol) in DCM (20 mL). After addition, the mixture was reacted at room temperature for 1 hour. The reaction solution was concentrated, dichloromethane was added, saturated sodium bicarbonate solution was added to adjust the pH > 8.The organic phase was collected, dried over anhydrous sodium sulfate, and concentrated to give H87-d (4 g, 8.84 mmol, 80.77% yield, TFA) as a yellow solid. The crude product was used directly in the next step. MS m/z (ESI): 338.2 [M+H]⁺.

Step 5: H87-d (4 g, 8.84 mmol), methyl 3-methyl-2-(3-(((perfluorobutyl)sulfonyl)oxy)isoxazol-5-yl)butanoate (6.38 g, 13.27 mmol), DIPEA (3.43 g, 26.53 mmol, 4.62 mL), and DMSO (50 mL) were mixed and heated to 110°C, and the mixture was reacted for 16 hours. The reaction solution was poured into ice water, and extracted with ethyl acetate (30 mL×2). The organic layers were combined, washed three times with water, once with saturated brine, dried over anhydrous sodium sulfate, and concentrated to give a crude product. The crude product was purified by column chromatography (dichloromethane:ammonia/methanol = 95%:5%) to give H87-e (4.3 g, 8.28 mmol, yield 93.60%) as a brown liquid. MS m/z (ESI): 519.2 [M+H]⁺.

Step 6: H87-e (1 g, 1.93 mmol) and bis(pinacolato)diboron (1.47 g, 5.78 mmol) were dissolved in 1,4-dioxane (20 mL). Under argon protection, Pd(dba)₂ (218.28 mg, 385.01 µmol) and PCy3 (259.12 mg, 924.01 µmol) were added, and the mixture was heated to 120°C and stirred for 24 hours. The reaction solution was filtered, and the filtrate was concentrated to give a crude product. The crude product was purified by column chromatography (petroleum ether/triethylamine:dichloromethane = 85%:15%) to give H87-f (700 mg, 1.24 mmol, yield 64.18%) as a yellow solid. MS m/z (ESI): 567.4 [M+H]⁺.

Step 7: Under argon protection, 8-bromo-N-((5-fluoro-2,3-dihydrobenzofuran-4-yl)methyl)-[1,2,4]triazolo[4,3-c]pyrimidin-5-amine (400 mg, 1.10 mmol), H87-f (684.50 mg, 1.21 mmol), [1,1'-bis(diphenylphosphinoferrocene]dichloropalladium(II) (80.37 mg, 109.84 µmol), potassium carbonate (455.41 mg, 3.30 mmol), 1,4-dioxane (20 mL), and DMSO (1 mL) were mixed and heated to 100°C, and the mixture was reacted for 2 hours. The reaction solution was filtered, ethyl acetate and saturated brine were added to the filtrate, the organic layer was separated, dried over anhydrous sodium sulfate, and concentrated to give a crude product. The crude product was purified by column chromatography (petroleum ether:ethyl acetate/triethylamine = 80%:20% to 0:100%) to give H87-g (470 mg, 649.32 µmol, yield 59.12%) as a yellow solid. MS m/z (ESI): 724.4 [M+H]⁺.

Step 8: At room temperature, LiOH (76.10 mg, 3.18 mmol) was added to a solution of H87-g (460 mg, 635.50 µmol) in THF (6 mL), methanol (4 mL), and water (2 mL). After addition, the mixture was reacted at room temperature for 1 hour. The pH of the reaction solution was adjusted to 5-6 with 1N aqueous HCl solution. After adding ethyl acetate, a large amount of yellow solid precipitated. The solid was collected and dried to give H87-h (400 mg, 563.53 µmol, yield 88.67%) as a yellow solid. MS m/z (ESI): 710.4 [M+H]⁺.

### Preparation of Intermediates H66-b and H86-b

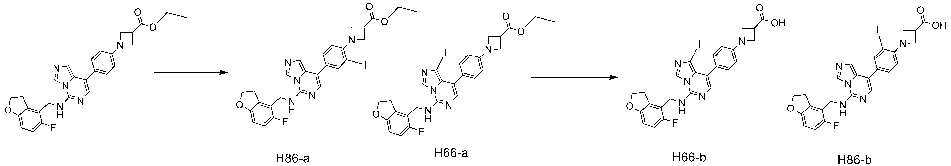

Step 1: Ethyl 1-(4-(5-((5-fluoro-2,3-dihydrobenzofuran-4-yl)methylamino)imidazo[1,5-c]pyrimidin-8-yl)phenyl)azetidine-3-carboxylate (700 mg, 1.44 mmol) was dissolved in THF (10 mL). The temperature was lowered to 0°C, and N-iodosuccinimide (226.13 mg, 1.01 mmol) was then added. The mixture was stirred at 0°C for 0.5 hour. Saturated aqueous sodium bicarbonate solution was added, and the mixture was extracted with ethyl acetate (70 mL×3). The organic phases were combined, dried over anhydrous sodium sulfate, filtered, and the filtrate was concentrated to give a crude product. The resultant was purified by CombiFlash (12 g, 0-50% EA/PE) successfully to give a mixture of H86-a and H66-a (88 mg, 143.46 µmol, 9.99% yield) as a light yellow solid. MS m/z (ESI): 614.2 [M+H]⁺.

Step 2: The mixture of H86-a and H66-a (80 mg, 130.42 µmol) was dissolved in THF (8 mL) and water (2 mL). LiOH (62.46 mg, 2.61 mmol) was then added. The mixture was stirred at room temperature for 1 hour. After the reaction was complete, the mixture was neutralized to pH=3-4 with dilute hydrochloric acid (2N), and then the solvent was dried by rotary evaporation under reduced pressure to successfully give a mixture of H86-b and H66-b (66 mg, 112.75 µmol, 86.45% yield) as a white solid. MS m/z (ESI): 586.1 [M+H]⁺.

### Preparation of Intermediate H64-b

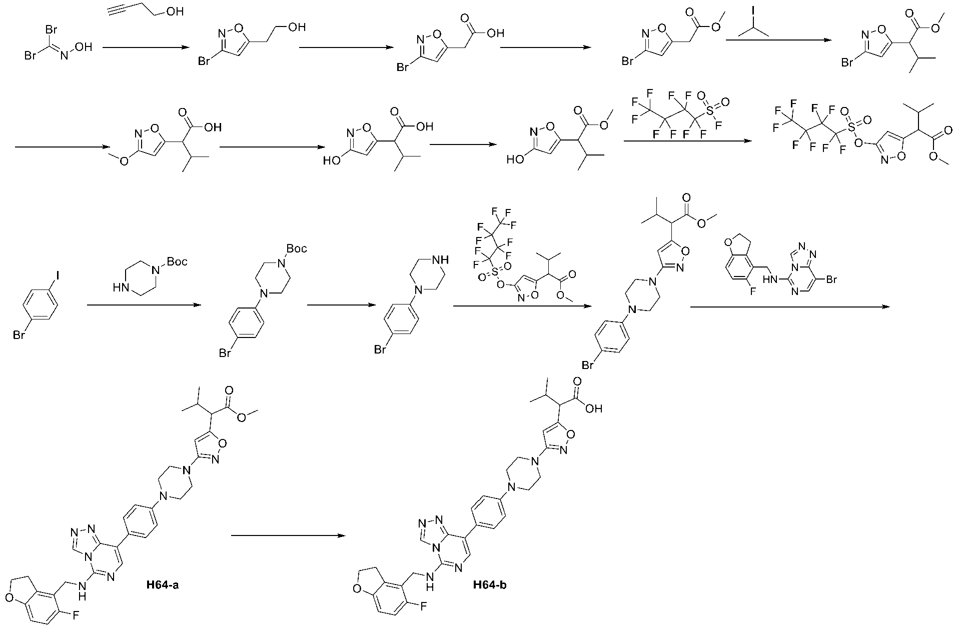

Step 1: Dibromohydroxycarbonate (25 g, 123.25 mmol) was dissolved in EA (250 mL) and water (25 mL). 3-Butyn-1-ol (34.56 g, 493.02 mmol) and NaHCO₃ (31.80 g, 369.76 mmol) were added, and the mixture was reacted at room temperature for 3 hours. The reaction solution was filtered, water was added to the filtrate, the layers were separated, and the mixture was extracted twice with ethyl acetate. The organic phase was washed once with saturated brine, dried over anhydrous sodium sulfate, filtered, and dried by rotary evaporation to give a crude product. The crude product was purified by column chromatography (petroleum ether:dichloromethane = 50%:50% to 0:100%) to give 2-(3-bromoisoxazol-5-yl)ethan-1-ol (18 g, 93.74 mmol, yield 76.06%) as a yellow liquid. MS m/z (ESI): 192.0 [M+H]⁺.

Step 2: At 0°C, 2-(3-bromoisoxazol-5-yl)ethan-1-ol (18 g, 93.74 mmol) was dissolved in acetone (150 mL). Jones reagent (100 mL) was added, and the mixture was reacted at room temperature for 16 hours. The reaction solution was slowly poured into ice water, extracted twice with ethyl acetate. The organic phases were combined, washed twice with saturated brine, dried over anhydrous sodium sulfate, and concentrated to give 2-(3-bromoisoxazol-5-yl)acetic acid (16.8 g, 81.56 mmol, yield 87.00%) as a brown liquid. The crude product was used directly in the next step. MS m/z (ESI): 203.7 [M+H]⁺.

Step 3: 2-(3-Bromoisoxazol-5-yl)acetic acid (16 g, 77.67 mmol) was dissolved in methanol (100 mL). Concentrated sulfuric acid (2 mL) was added, and the mixture was reacted at 70°C for 1 hour. The reaction solution was concentrated to a smaller volume, water was added, the mixture was extracted with dichloromethane (50 mL×2), washed with saturated brine, dried over anhydrous sodium sulfate, and concentrated to give a crude product. The crude product was purified by column chromatography (petroleum ether:dichloromethane = 30%:70%) to give methyl 2-(3-bromoisoxazol-5-yl)acetate (14 g, 63.63 mmol, yield 81.92%) as a white solid. MS m/z (ESI): 220.0 [M+H]⁺.

Step 4: Methyl 2-(3-bromoisoxazol-5-yl)acetate (14 g, 63.63 mmol) was dissolved in THF (150 mL). Under ice bath cooling, potassium tert-butoxide (10.69 g, 95.27 mmol) was added, stirred for 10 min, and 2-iodopropane (14.06 g, 82.72 mmol, 8.26 mL) was slowly added dropwise. After addition, the mixture was reacted at room temperature for 16 hours. The reaction solution was poured into ice water, extracted twice with ethyl acetate, washed twice with saturated brine, dried over anhydrous sodium sulfate, filtered, and dried by rotary evaporation to give crude methyl 2-(3-bromoisoxazol-5-yl)-3-methylbutanoate (8 g, 30.52 mmol, yield 47.97%) as a yellow oil. The crude product was used directly in the next step. MS m/z (ESI): 262.0 [M+H]⁺.

Step 5: Methyl 2-(3-bromoisoxazol-5-yl)-3-methylbutanoate (8 g, 30.52 mmol), potassium hydroxide (8.56 g, 152.61 mmol), methanol (45 mL), and water (5 mL) were added to a pressure vessel and reacted at 110°C for 16 hours. The reaction solution was filtered, the filtrate was concentrated, ice water was added, and the mixture was washed once with ethyl acetate. The aqueous phase was collected, adjusted to pH < 4 with concentrated hydrochloric acid at 0°C, and then extracted with ethyl acetate (50 mL×2). The organic layers were combined, washed with saturated brine, dried over anhydrous sodium sulfate, and concentrated to give 2-(3-methoxyisoxazol-5-yl)-3-methylbutanoic acid (5.1 g, 25.60 mmol, yield 83.88%) as a yellow liquid. The crude product was used directly in the next step. MS m/z (ESI): 200.2 [M+H]⁺.

Step 6: 2-(3-Methoxyisoxazol-5-yl)-3-methylbutanoic acid (5 g, 25.10 mmol) and HBr/AcOH (20 mL) were heated to 80°C and reacted for 0.5 hour. The reaction solution was concentrated to give 2-(3-hydroxyisoxazol-5-yl)-3-methylbutanoic acid (4.6 g, 24.84 mmol, yield 98.97%) as a yellow liquid. The crude product was used directly in the next step. MS m/z (ESI): 186.1 [M+H]⁺.

Step 7: Thionyl chloride (13.30 g, 111.78 mmol, 8.12 mL) was added dropwise to a solution of 2-(3-hydroxyisoxazol-5-yl)-3-methylbutanoic acid (4.6 g, 24.84 mmol) in methanol (50 mL). After addition, the mixture was reacted at room temperature for 2 hours. The reaction solution was concentrated to dryness, water was added, and the mixture was extracted with ethyl acetate (20 mL×2). The organic layers were combined, washed with saturated brine and concentrated to give a crude product. The crude product was purified by column chromatography (petroleum ether:dichloromethane = 50%:50%) to give methyl 2-(3-hydroxyisoxazol-5-yl)-3-methylbutanoate (3.8 g, 19.08 mmol, yield 76.79%) as a brown-red liquid. MS m/z (ESI): 200.1 [M+H]⁺.

Step 8: Methyl 2-(3-hydroxyisoxazol-5-yl)-3-methylbutanoate (3.8 g, 19.08 mmol), potassium carbonate (5.27 g, 38.15 mmol), and acetonitrile (100 mL) were mixed, and perfluorobutanesulfonyl fluoride (6.63 g, 21.94 mmol) was added dropwise. After addition, the mixture was reacted at room temperature for 2 hours. The reaction solution was filtered, and the filtrate was concentrated to give a crude product. The crude product was purified by column chromatography (petroleum ether:dichloromethane = 80%:20%) to give methyl 3-methyl-2-(3-(((perfluorobutyl)sulfonyl)oxy) isoxazol-5-yl)butanoate (3 g, 6.23 mmol, yield 32.68%) as a colorless liquid. MS m/z (ESI): 482.0 [M+H]⁺.

Step 9: Under argon protection, 1-bromo-4-iodobenzene (7.29 g, 25.77 mmol), tert-butyl piperazine-1-carboxylate (4 g, 21.48 mmol, HCl), L-proline (989.03 mg, 8.59 mmol), copper(I) iodide (818.04 mg, 4.30 mmol), potassium carbonate (5.94 g, 42.95 mmol), and DMSO (40 mL) were mixed and heated to 80°C, and the mixture was reacted for 16 hours. The reaction solution was poured into water, extracted with ethyl acetate (20 mL×2). The organic layers were combined, washed and concentrated to give a crude product. The crude product was purified by column chromatography (petroleum ether:ethyl acetate = 80%:20%) to give tert-butyl 4-(4-bromophenyl)piperazine-1-carboxylate (3 g, 8.79 mmol, yield 40.94%) as a yellow solid. MS m/z (ESI): 285.1 [M+H-56]⁺.

Step 10: At room temperature, TFA (2.5 mL) was added to a solution of tert-butyl 4-(4-bromophenyl)piperazine-1-carboxylate (1 g, 2.93 mmol) in DCM (5 mL). After addition, the mixture was reacted at room temperature for 1 hour. The reaction solution was concentrated to dryness to give 1-(4-bromophenyl)piperazine (700 mg, 2.90 mmol, 99.06% yield) as a yellow solid. The crude product was used directly in the next step. MS m/z (ESI): 241.1 [M+H]⁺.

Step 11: 1-(4-Bromophenyl)piperazine (500 mg, 2.07 mmol), methyl 3-methyl-2-(3-(((perfluorobutyl)sulfonyl)oxy)isoxazol-5-yl)butanoate (798.40 mg, 1.66 mmol), DIPEA (803.99 mg, 6.22 mmol, 1.08 mL), and DMSO (15 mL) were heated to 110°C, and the mixture was reacted for 16 hours. The reaction solution was poured into ice water, extracted with ethyl acetate (20 mL×2). The organic layers were combined, washed three times with water, once with saturated brine, and concentrated to give a crude product. The crude product was purified by column chromatography (dichloromethane:ammonia/methanol 4M = 98%:2%) to give methyl 2-(3-(4-(4-bromophenyl)piperazin-1-yl)isoxazol-5-yl)-3-methylbutanoate (268 mg, 634.60 µmol, yield 30.60%) as a brown oil. MS m/z (ESI): 422.1 [M+H]⁺.

Step 12: Under argon protection, methyl 2-(3-(4-(4-bromophenyl)piperazin-1-yl)isoxazol-5-yl)-3-methylbutanoate (255.12 mg, 604.11 µmol), 8-bromo-N-((5-fluoro-2,3-dihydrobenzofuran-4-yl)methyl)-[1,2,4]triazolo[4,3-c]pyrimidin-5-amine (200 mg, 549.19 µmol), palladium acetate (24.66 mg, 109.84 µmol), n-butyldi(1-adamantyl)phosphine (39.38 mg, 109.84 µmol), potassium carbonate (303.60 mg, 2.20 mmol), bis(pinacolato)diboron (278.92 mg, 1.10 mmol), DME (25 mL), and water (5 mL) were heated to 85°C, and the mixture was reacted for 16 hours. The reaction solution was filtered, the filtrate was concentrated, water was added, the mixture was extracted with ethyl acetate (10 mL×2), the organic layers were combined, washed with saturated brine, and concentrated to give a crude product. The crude product was purified by column chromatography (dichloromethane:ammonia/ methanol 4M = 95%:5%) to give H64-a (150 mg, 239.36 µmol, 43.58% yield) as a brown oil. MS m/z (ESI): 627.3 [M+H]⁺.

Step 13: At room temperature, lithium hydroxide (9.36 mg, 390.95 µmol) was added to a solution of H64-a (140 mg, 78.19 µmol) in THF (2 mL), methanol (2 mL), and water (1 mL). After addition, the mixture was reacted at room temperature for 1 hour. The reaction solution was concentrated, washed once with ethyl acetate, then adjusted to pH=6-7 with 1N HCl, extracted with ethyl acetate (5 mL×2), and the organic layers were combined, dried over anhydrous sodium sulfate and concentrated to give H64-b (45 mg, 73.45 µmol, yield 93.94%) as a yellow oil. MS m/z (ESI): 613.3 [M+H]⁺.

### Preparation of Intermediate H64-f

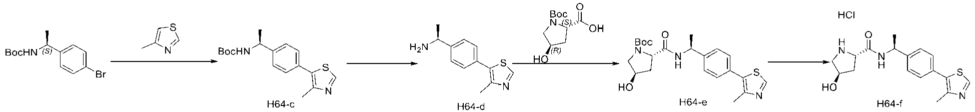

Step 1: Under argon protection, tert-butyl (S)-(1-(4-bromophenyl)ethyl)carbamate (5 g, 16.66 mmol), 4-methylthiazole (3.30 g, 33.31 mmol), palladium acetate (37.39 mg, 166.56 µmol), potassium acetate (3.27 g, 33.31 mmol), and DMA (10 mL) were mixed and heated to 150°C, and the mixture was reacted for 4 hours. The reaction solution was filtered, ice water was added to the filtrate, and the mixture was extracted with ethyl acetate (20 mL×2). The organic layers were combined, washed three times with water, once with saturated brine, dried over anhydrous sodium sulfate, and concentrated to give a crude product. The crude product was purified by column chromatography (petroleum ether:ethyl acetate = 75%:25%) to give H64-c (5.2 g, 16.33 mmol, yield 98.04%) as a yellow solid. MS m/z (ESI): 319.1 [M+H]⁺.

Step 2: At room temperature, HCl/1,4-dioxane (15 mL) was added to a solution of H64-c (5.1 g, 16.02 mmol) in DCM (30 mL). After addition, the mixture was reacted at room temperature for 2 hours. The reaction solution was filtered, and the solid was collected to give H64-d (4 g, 15.70 mmol, 98.03% yield, HCl) as a yellow solid. MS m/z (ESI): 219.1 [M+H]⁺.

Step 3: At room temperature, H64-d (4.34 g, 18.76 mmol), HATU (7.81 g, 20.54 mmol), DIPEA (6.93 g, 53.59 mmol, 9.33 mL), and DMF (50 mL) were mixed, and rac-(1S)-1-[4-(4-methylthiazol-5-yl)phenyl]ethanamine (3.9 g, 17.86 mmol) was added. After addition, the mixture was reacted at room temperature for 2 hours. The reaction solution was poured into water, and the aqueous phase was extracted with ethyl acetate (20 mL×2). The organic phases were combined, dried over anhydrous sodium sulfate and concentrated to give a crude product. The crude product was purified by column chromatography (dichloromethane:methanol = 95%:5%) to give H64-e (6 g, 13.90 mmol, yield 77.83%) as a white solid. MS m/z (ESI): 432.2 [M+H]⁺.

Step 4: H64-e (1 g, 2.32 mmol) was dissolved in HCl/1,4-dioxane (4 M) (20 mL) and stirred at room temperature for 2 hours. The solvent was concentrated under reduced pressure to give crude product H64-f (720 mg, 2.17 mmol, 93.75% yield) as a pink solid. The crude product was used directly in the next step. MS m/z (ESI): 332.2 [M+H]⁺.

### Preparation of Intermediate H65-g

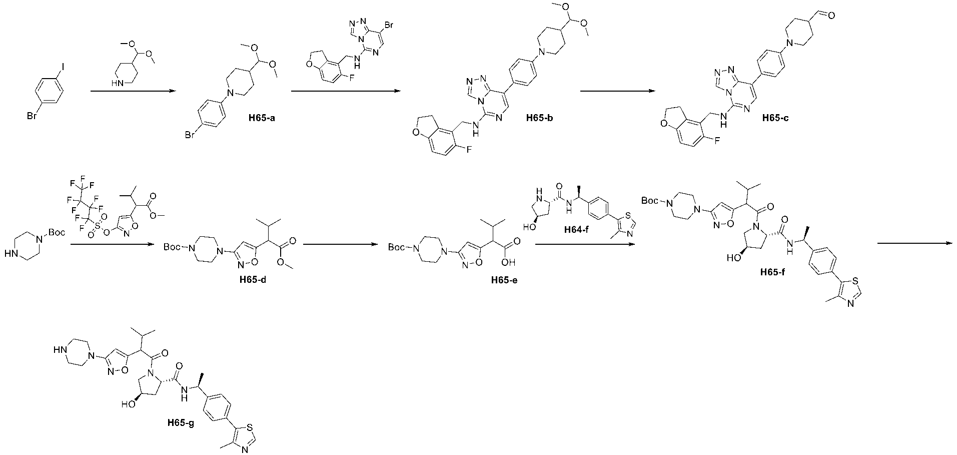

Step 1: Under argon protection, 1-bromo-4-iodobenzene (6.40 g, 22.61 mmol), 4-(dimethoxymethyl)piperidine (3 g, 18.84 mmol, HCl), L-proline (867.68 mg, 7.54 mmol), copper(I) iodide (717.66 mg, 3.77 mmol), potassium carbonate (5.21 g, 37.68 mmol), and DMSO (40 mL) were mixed and heated to 80°C, and the mixture was reacted for 16 hours. The reaction solution was poured into water, extracted with ethyl acetate (20 mL×2). The organic layers were combined, washed with saturated brine and concentrated to give a crude product. The crude product was purified by column chromatography (petroleum ether:ethyl acetate = 100% to 80%:20%) to give H65-a (3.2 g, 10.18 mmol, yield 54.05%) as a yellow solid. MS m/z (ESI): 314.1 [M+H]⁺.

Step 2: Under argon protection, 8-bromo-N-((5-fluoro-2,3-dihydrobenzofuran-4-yl)methyl)-[1,2,4]triazolo[4,3-c]pyrimidin-5-amine (500 mg, 1.37 mmol), H65-a (474.55 mg, 1.51 mmol), palladium acetate (61.65 mg, 274.60 µmol), n-butyldi(1-adamantyl)phosphine (98.45 mg, 274.60 µmol), potassium carbonate (759.01 mg, 5.49 mmol), bis(pinacolato)diboron (697.30 mg, 2.75 mmol), DME (20 mL), and water (2 mL) were mixed and heated to 85°C, and the mixture was reacted for 16 hours. The reaction solution was filtered, the aqueous layer was separated from the filtrate, and the resultant was concentrated to give a crude product. The crude product was purified by column chromatography (petroleum ether:ethyl acetate = 50%:50% to 0%:100%) to give H65-b (120 mg, 231.40 µmol, yield 16.85%) as a yellow solid. MS m/z (ESI): 519.3 [M+H]⁺.

Step 3: At room temperature, TFA (0.5 mL) was added to a solution of H65-b (50 mg, 96.42 µmol) in DCM (1 mL). After addition, the mixture was reacted at room temperature for 1 hour. The reaction solution was concentrated to give H65-c (36 mg, 76.19 µmol, yield 79.02%) as a yellow solid. The crude product was used directly in the next step. MS m/z (ESI): 473.2 [M+H]⁺.

Step 4: tert-Butyl piperazine-1-carboxylate (400 mg, 2.15 mmol), methyl 3-methyl-2-(3-(((perfluorobutyl)sulfonyl)oxy)isoxazol-5-yl)butanoate (930.27 mg, 1.93 mmol), DIPEA (832.68 mg, 6.44 mmol, 1.12 mL), and DMSO (15 mL) were heated to 110°C, and the mixture was reacted for 16 hours. The reaction solution was poured into ice water, extracted with ethyl acetate (20 mL×2). The organic layers were combined, washed three times with water, once with saturated brine, and concentrated to give a crude product. The crude product was purified by column chromatography (dichloromethane:ammonia/methanol 4M = 95%:5%) to give H65-d (700 mg, 1.91 mmol, yield 88.71%) as a brown oil. MS m/z (ESI): 312.1 [M+H-56]⁺.

Step 5: At room temperature, H65-d (200 mg, 544.31 µmol), NaOH (78.21 mg, 1.96 mmol), THF (6 mL), and water (2 mL) were mixed and stirred for reaction for 16 hours. The reaction solution was concentrated, 1N HCl was added to adjust the pH to 4-5, and then the mixture was extracted with ethyl acetate (5 mL×2). The organic layers were combined, washed with saturated brine and concentrated to give H65-e (90 mg, 254.66 µmol, yield 46.79%) as a brown solid. MS m/z (ESI): 298.1 [M+H-56]⁺.

Step 6: At room temperature, H65-e (80 mg, 226.36 µmol), H64-f (75.02 mg, 226.36 µmol), DIPEA (87.77 mg, 679.09 µmol, 118.29 µL), and DMF (5 mL) were mixed, and HATU (102.48 mg, 271.64 µmol) was added. After addition, the mixture was reacted at room temperature for 1 hour. The reaction solution was filtered, ice water was added to the filtrate, and the mixture was extracted with ethyl acetate (15 mL×2). The organic layers were combined, washed with saturated brine and concentrated to give a crude product. The crude product was purified by column chromatography (dichloromethane:ammonia/methanol 4M = 95%:5%) to give H65-f (100 mg, 149.96 µmol, yield 66.25%) as a brown solid. MS m/z (ESI): 611.3 [M+H-56]⁺.

Step 7: At room temperature, TFA (1 mL) was added to a solution of H65-f (90 mg, 134.97 µmol) in DCM (2 mL). After addition, the mixture was reacted at room temperature for 1 hour. The reaction solution was concentrated to dryness, ethyl acetate was added to the residue, saturated sodium bicarbonate solution was added to adjust the pH > 8, the organic layer was separated and concentrated to give H65-g (55 mg, 97.05 µmol, yield 71.91%) as a yellow solid. The crude product was used directly in the next step. MS m/z (ESI): 567.3 [M+H]⁺.

### Preparation of Intermediate 8-(5-(((5-fluoro-2,3-dihydrobenzofuran-4-yl)methyl)amino) imidazo[1,5-c]pyrimidin-8-yl)-[1,2,4]triazolo[1,5-a]pyridine-5-carboxylic acid

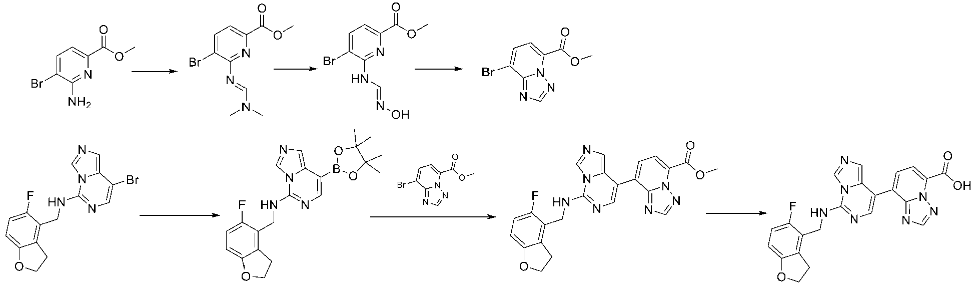

Step 1: Methyl 5-bromo-6-aminopyridine-2-carboxylate (4.0 g, 17.31 mmol) was dissolved in toluene (40 mL). N,N-Dimethylformamide dimethyl acetal (4.63 mL, 34.62 mmol) was then added. The mixture was stirred at 110°C for 4 hours. The crude reaction solution was concentrated to give crude product methyl (E)-5-bromo-6-((dimethylamino)methylene)amino)picolinate (5.1 g, yellow solid, crude), which was used directly in the next step without purification. MS m/z (ESI): 286.0 [M+H]⁺.

Step 2: Methyl (E)-5-bromo-6-((dimethylamino)methylene)amino)picolinate (5.1 g, crude, 17.82 mmol) was dissolved in ethanol (50 mL). Sodium acetate (2.92 g, 35.65 mmol) and hydroxylamine hydrochloride (2.48 g, 35.65 mmol) were then added. The mixture was stirred at 50°C for 4 hours. The crude reaction solution was concentrated to give crude product methyl (E)-5-bromo-6-(N'-hydroxycarbamimidoyl)picolinate (10 g, yellow oil, crude), which was used directly in the next step without purification. MS m/z (ESI): 273.8 [M+H]⁺.

Step 3: Methyl (E)-5-bromo-6-(N'-hydroxycarbamimidoyl)picolinate (10.0 g, crude) was dissolved in tetrahydrofuran (100 mL). Trifluoroacetic anhydride (17.30 g, 35.65 mmol) was then added. The mixture was stirred at 75°C for 3 hours. After cooling to room temperature, the reaction solution was quenched with saturated aqueous sodium bicarbonate solution (30 mL), extracted with ethyl acetate (20 mL×3). The organic phase was washed with saturated brine (50 mL), dried over anhydrous sodium sulfate, and the solvent was concentrated. The resultant was purified by silica gel column chromatography (PE/EA = 50/50) to give methyl 8-bromo-[1,2,4]triazolo[1,5-a]pyridine-5-carboxylate (3.55 g, white solid, 80.0% yield over three steps). MS m/z (ESI): 258.0 [M+H]⁺.

Step 4: At 25°C under a nitrogen atmosphere, 8-bromo-N-((5-fluoro-2,3-dihydrobenzofuran-4-yl)methyl)imidazo[1,5-c]pyrimidin-5-amine (1.50 g, 4.13 mmol), potassium acetate (1.22 g, 12.39 mmol), and bis(pinacolato)diboron (2.10 g, 8.26 mmol) were dissolved in 1,4-dioxane (50 mL). Tetrakis(triphenylphosphine)palladium(0) (0.95 g, 0.83 mmol) was then added in one portion, and the mixture was stirred at 100°C for 18 hours. The crude reaction solution was concentrated to give a crude product. The crude product was purified by silica gel chromatography (eluent: dichloromethane:methanol = 1:0 to 10:1) to give N-((5-fluoro-2,3-dihydrobenzofuran-4-yl)methyl)-8-(4,4,5,5-tetramethyl-1,3,2-dioxaborolan-2-yl)imidazo[1,5-c]pyrimidin-5-amine (400.0 mg, yellow solid). Yield: 23.61%. MS m/z (ESI): 411.2 [M+H]⁺.

Step 5: At 25°C under a nitrogen atmosphere, N-((5-fluoro-2,3-dihydrobenzofuran-4-yl)methyl)-8-(4,4,5,5-tetramethyl-1,3,2-dioxaborolan-2-yl)imidazo[1,5-c]pyrimidin-5-amine (370 mg, 0.90 mmol), methyl 8-bromo-[1,2,4]triazolo[1,5-a]pyridine-5-carboxylate (461.9 mg, 1.80 mmol), and potassium carbonate (373.9 mg, 2.71 mmol) were dissolved in dimethoxyethane (20 mL) and water (2 mL). Tetrakis(triphenylphosphine)palladium(0) (104.2 mg, 0.09 mmol) was then added in one portion, and the mixture was stirred at 80°C for 2 hours. The crude reaction solution was concentrated to give a crude product. The crude product was purified by silica gel chromatography (eluent: dichloromethane:methanol = 1:0 to 10:1) to give methyl 8-(5-(((5-fluoro-2,3-dihydrobenzofuran-4-yl)methyl)amino)imidazo[1,5-c]pyrimidin-8-yl)-[1,2,4]triazolo[1,5-a]pyridine-5-carboxylate (16.0 mg, yellow solid). Yield: 38.61%. MS m/z (ESI): 460.2 [M+H]⁺.

Step 6: At 25°C under a nitrogen atmosphere, methyl 8-(5-(((5-fluoro-2,3-dihydrobenzofuran-4-yl)methyl)amino)imidazo[1,5-c]pyrimidin-8-yl)-[1,2,4]triazolo[1,5-a]pyridine-5-carboxylate (53 mg, 0.12 mmol) and lithium hydroxide (24.2 mg, 0.58 mmol) were dissolved in methanol (2 mL) and water (0.5 mL). The mixture was stirred at 25°C for 2 hours. The crude reaction solution was concentrated to give a crude product. The crude product was used directly in the next step. MS m/z (ESI): 446.2 [M+H]⁺.

### Preparation of Intermediate 5'-[(5-fluoro-2,3-dihydrobenzofuran-4-yl)methylamino]-7'-oxo-spiro[indene-1,8'-pyrido[4,3-c]pyridazine]-5-carboxylic acid

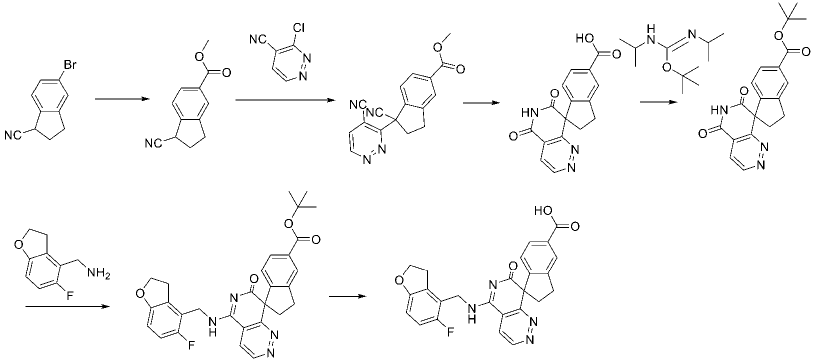

Step 1: Compound 5-bromoindane-1-carbonitrile (3.0 g, 13.51 mmol) was dissolved in methanol (100 mL). Under argon protection, molybdenum hexacarbonyl (5.35 g, 20.26 mmol), triethylamine (5.46 g, 54.03 mmol, 7.52 mL), palladium acetate (303.28 mg, 1.35 mmol), and 1,1'-binaphthyl-2,2'-bis(diphenylphosphine) (1.68 g, 2.70 mmol) were added sequentially. The tube was sealed and heated to 70°C, and the mixture was stirred for 2 hours. The reaction solution was filtered, concentrated to dryness under reduced pressure, and purified by silica gel column chromatography (EA:PE = 0-50%) to give methyl 1-cyanoindane-5-carboxylate (600 mg, 2.98 mmol, 22.07% yield) as a yellow solid. MS m/z (ESI): 202 [M+H]⁺.

Step 2: Methyl 1-cyanoindane-5-carboxylate (1.0 g, 4.97 mmol) was dissolved in tetrahydrofuran (20 mL). Under argon protection, the temperature was lowered to 0°C, and sodium hydride (795.07 mg, 19.88 mmol, 60% purity) was added portionwise. After addition, the mixture was stirred at 0°C for 1 hour. 3-Chloropyridazine-4-carbonitrile (832.17 mg, 5.96 mmol) dissolved in tetrahydrofuran (5 mL) was added dropwise. After addition, the mixture was stirred at 0°C for 2 hours, then naturally warmed to room temperature and stirred overnight. The reaction solution was poured into ice water, extracted with EA (30 mL×2), dried over anhydrous sodium sulfate, concentrated to dryness under reduced pressure, and purified by silica gel column chromatography (EA:PE = 0-100%) to give methyl 1-cyano-1-(4-cyanopyridazin-3-yl)indane-5-carboxylate (300 mg, 985.86 µmol, 19.84% yield) as a yellow solid. MS m/z (ESI): 305 [M+H]⁺.

Step 3: Methyl 1-cyano-1-(4-cyanopyridazin-3-yl)indane-5-carboxylate (300 mg, 985.86 µmol) was dissolved in concentrated hydrochloric acid (97.15 mg, 985.86 µmol, 15 mL, 37% purity). The mixture was heated to 80°C and stirred for 2 hours. The reaction solution was concentrated to dryness under reduced pressure to give 5',7'-dioxospiro[indene-1,8'-pyrido[4,3-c]pyridazine]-5-carboxylic acid (304.90 mg, 985.85 µmol, 100.00% yield) as a gray solid. MS m/z (ESI): 305 [M+H]⁺.

Step 4: 5',7'-Dioxospiro[indene-1,8'-pyrido[4,3-c]pyridazine]-5-carboxylic acid (300 mg, 970.01 µmol) was dissolved in tetrahydrofuran (10 mL). 2-tert-Butyl-1,3-diisopropylisourea (971.56 mg, 4.85 mmol) was added, and the mixture was stirred at room temperature, and the mixture was stirred at room temperature for 16 hours. The reaction solution was concentrated to dryness under reduced pressure and purified by silica gel column chromatography (EA:PE = 0-100%) to give tert-butyl 5',7'-dioxospiro[indene-1,8'-pyrido[4,3-c]pyridazine]-5-carboxylate (320 mg, 875.80 µmol, 90.29% yield) as a yellow solid. MS m/z (ESI): 366 [M+H]⁺.

Step 5: tert-Butyl 5',7'-dioxospiro[indene-1,8'-pyrido[4,3-c]pyridazine]-5-carboxylate (226.03 mg, 618.62 µmol) was dissolved in acetonitrile (10 mL). Under argon protection, phosphorus oxychloride (474.27 mg, 3.09 mmol) and diisopropylethylamine (799.52 mg, 6.19 mmol, 1.08 mL) were added. The mixture was heated to 80°C and stirred for 25 minutes, then cooled to room temperature.

(5-Fluoro-2,3-dihydrobenzofuran-4-yl)methanamine (517.10 mg, 3.09 mmol) was added, and the mixture was stirred at room temperature for 2 hours. The reaction solution was concentrated to dryness under reduced pressure and purified by silica gel column chromatography (MeOH:DCM = 0-10%) to give tert-butyl 5'-[(5-fluoro-2,3-dihydrobenzofuran-4-yl)methylamino]-7'-oxospiro[indene-1,8'-pyrido[4,3-c]pyridazine]-5-carboxylate (60 mg, 116.61 µmol, 18.85% yield) as a yellow solid. MS m/z (ESI): 515 [M+H]⁺.

Step 6: tert-Butyl 5'-[(5-fluoro-2,3-dihydrobenzofuran-4-yl)methylamino]-7'-oxospiro[indene-1,8'-pyrido[4,3-c]pyridazine]-5-carboxylate (50 mg, 97.17 µmol) was dissolved in dichloromethane (10 mL). A solution of hydrogen chloride in ethyl acetate (4.0 M, 10.00 mL) was added, and the mixture was stirred at room temperature, and the mixture was stirred at room temperature for 3 hours. The reaction solution was concentrated to dryness under reduced pressure to give 5'-[(5-fluoro-2,3-dihydrobenzofuran-4-yl)methylamino]-7'-oxo-spiro[indene-1,8'-pyrido[4,3-c]pyridazi ne]-5-carboxylic acid (44.55 mg, 97.18 µmol, 100.00% yield) as a yellow solid. MS m/z (ESI): 459 [M+H]⁺.

### Preparation of Intermediate 2-(2,6-dioxopiperidin-3-yl)-5-(3,9-diazaspiro[5.5]undecan-3-yl)isoindoline-1,3-dione

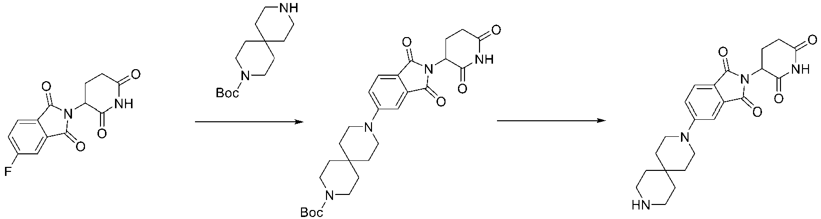

Step 1: 2-(2,6-Dioxopiperidin-3-yl)-5-fluoroisoindoline-1,3-dione (500 mg, 1.81 mmol) and tert-butyl 3,9-diazaspiro[5.5]undecane-3-carboxylate (506.49 mg, 1.99 mmol) were dissolved in DMSO (5 mL). DIPEA (467.90 mg, 3.62 mmol, 630.59 µL) was added. The mixed solution was stirred at 90°C under nitrogen protection for 15 hours, and the starting material was completely reacted. H₂O (50 mL) was added to the reaction solution, the mixture was extracted three times with ethyl acetate (25 mL×3), the organic phases were combined, dried over anhydrous sodium sulfate, filtered, and the filtrate was concentrated to dryness under reduced pressure to give a crude product. The crude product was purified by combiflash (EA:PE = 0-60%) to give tert-butyl 9-(2-(2,6-dioxopiperidin-3-yl)-1,3-dioxoisoindolin-5-yl)-3,9-diazaspiro[5.5]undecane-3-carboxylate (765 mg, yield: 82.77%) as a yellow oil. MS m/z (ESI): 510.9 [M+H]⁺.

Step 2: tert-Butyl 9-(2-(2,6-dioxopiperidin-3-yl)-1,3-dioxoisoindolin-5-yl)-3,9-diazaspiro[5.5]undecane-3-carboxylate (715 mg, 1.40 mmol) was dissolved in dioxane (10 mL). A solution of HCl in dioxane (4 M, 10 mL) was added at room temperature (25°C). The mixed solution was stirred at room temperature (25°C) for 5 hours, and the starting material was completely reacted. The reaction solution was concentrated to dryness under reduced pressure to give crude product 2-(2,6-dioxopiperidin-3-yl)-5-(3,9-diazaspiro[5.5]undecan-3-yl)isoindoline-1,3-dione (570 mg, crude), which was used directly in the next step without further purification. MS m/z (ESI): 411.2 [M+H]⁺.

### Preparation of Intermediate 1-(1-(2,6-dioxopiperidin-3-yl)-3-methyl-2-oxo-2,3-dihydro-1H-benzo[d]imidazol-5-yl)piperidine-4-carbaldehyde

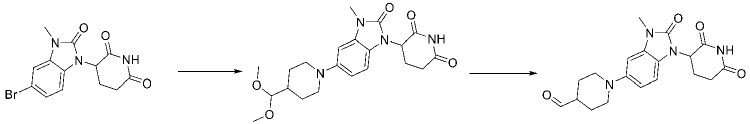

Step 1: 3-(5-Bromo-3-methyl-2-oxo-benzimidazol-1-yl)piperidine-2,6-dione (5.0 g, 14.79 mmol) and 4-(dimethoxymethyl)piperidine (4.71 g, 29.57 mmol) were dissolved in toluene (150 mL). Under argon protection, methanesulfonato(2-dicyclohexylphosphino-2',6'-diisopropoxy-1,1'-biphenyl)(2'-amino-1,1'-biphenyl-2-yl)palladium(II) (2.48 g, 2.96 mmol), 2-dicyclohexylphosphino-2',6'-diisopropoxy-1,1'-biphenyl (1.38 g, 2.96 mmol), and lithium bis(trimethylsilyl)amide (1.0 M, 73.93 mL) were added sequentially. The mixture was heated to 80°C and stirred for 2 hours. The reaction solution was poured into saturated ammonium chloride solution and extracted with ethyl acetate (30 mL×2). The organic phase was dried over anhydrous sodium sulfate, concentrated to dryness under reduced pressure, and purified by silica gel column chromatography (EA:PE = 0-100%) to give 3-[5-[4-(dimethoxymethyl)piperidin-1-yl]-3-methyl-2-oxo-benzimidazol-1-yl]piperidine-2,6-dione as a yellow solid. MS m/z (ESI): 417 [M+H]⁺.

Step 2: 3-[5-[4-(Dimethoxymethyl)piperidin-1-yl]-3-methyl-2-oxo-benzimidazol-1-yl]piperidine-2,6-dione (5.3 g, 12.73 mmol) was dissolved in formic acid (122.00 g, 2.65 mol, 100 mL), and the mixture was stirred at 50°C for 1 hour. After the reaction was complete, the solvent was dried by rotary evaporation under reduced pressure to give 1-(1-(2,6-dioxopiperidin-3-yl)-3-methyl-2-oxo-2,3-dihydro-1H-benzo[d]imidazol-5-yl)piperidine-4-carbaldehyde (4.71 g, 12.72 mmol, 100.00% yield) as a red solid. MS m/z (ESI): 371 [M+H]⁺.

### Preparation of Intermediate 1-(3-(2,6-dioxopiperidin-3-yl)-5-fluoro-1-methyl-1H-indazol-6-yl)piperidine-4-carbaldehyde

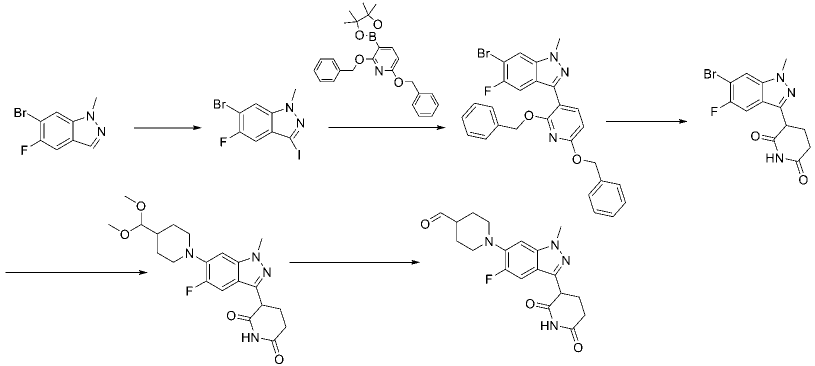

Step 1: Compound 6-bromo-5-fluoro-1-methyl-1H-indazole (1140 mg, 5 mmol) was added to 5 ml of DMSO. N-Iodosuccinimide (1125 mg, 5 mmol) was then added, and the mixture was heated to 120°C and stirred for 3 hours. After the reaction was complete, the mixture was poured into water, extracted three times with 30 ml of ethyl acetate. The organic phases were combined, washed once with 50 ml of saturated sodium chloride, and the organic phase was concentrated to give compound 6-bromo-5-fluoro-3-iodo-1-methyl-1H-indazole (1239 mg, 3.5 mmol). Yield: 70%. MS m/z (ESI): 354.9 [M+H]⁺.

Step 2: Compound 6-bromo-5-fluoro-3-iodo-1-methyl-1H-indazole (1239 mg, 3.5 mmol), 2,6-bis(benzyloxy)-3-(4,4,5,5-tetramethyl-1,3,2-dioxaborolan-2-yl)pyridine (1460 mg, 3.5 mmol), 120 mg of Pd(dppf)Cl₂, and 3.41 g of cesium carbonate were sequentially added to 10 ml of 1,4-dioxane. Then, 2 ml of water was added, and the mixture was purged with nitrogen three times. The mixture was then heated to 110°C and stirred for 15 hours. After the reaction was complete, the mixture was poured into water, extracted three times with 30 ml of ethyl acetate. The organic phases were combined, washed once with 50 ml of saturated sodium chloride. An appropriate amount of silica gel was added to the obtained organic phase for sample loading, and the resultant was purified by column chromatography using PE/EA (0-50%) to give compound 3-(2,6-bis(benzyloxy)pyridin-3-yl)-6-bromo-5-fluoro-1-methyl-1H-indazole (1036 mg, 2 mmol). Yield: 57%. MS m/z (ESI): 518 [M+H]⁺.

Step 3: Compound 3-(2,6-bis(benzyloxy)pyridin-3-yl)-6-bromo-5-fluoro-1-methyl-1H-indazole (1036 mg, 2 mmol) was added to 5 ml of methanol. Then, 200 mg of 10 wt% Pd/C was added, and the mixture was purged with hydrogen three times and then stirred at room temperature for 3 hours. After the reaction was complete, the mixture was filtered, and the filtrate was concentrated to give compound 3-(6-bromo-5-fluoro-1-methyl-1H-indazol-3-yl)piperidine-2,6-dione (680 mg, 2 mmol). Yield: 100%. MS m/z (ESI): 340 [M+H]⁺.

Step 4: Compound 3-(6-bromo-5-fluoro-1-methyl-1H-indazol-3-yl)piperidine-2,6-dione (680 mg, 2 mmol), 4-(dimethoxymethyl)piperidine (477 mg, 3 mmol), 340 mg of Ruphos-Pd-G₃, and cesium carbonate (1952 mg, 6 mmol) were sequentially added to 10 ml of 1,4-dioxane. The mixture was purged with nitrogen three times, the mixture was heated to 120°C and stirred for 15 hours. After the reaction was complete, the mixture was poured into water, then extracted three times with 30 ml of ethyl acetate. The organic phases were combined, washed once with 50 ml of saturated brine. An appropriate amount of silica gel was added to the organic phase for sample loading, and the resultant was purified by column chromatography using PE/EA (0-100%) to give 3-(6-(4-(dimethoxymethyl)piperidin-1-yl)-5-fluoro-1-methyl-1H-indazol-3-yl)piperidine-2,6-dione (84 mg, 0.2 mmol). Yield: 10%. MS m/z (ESI): 419 [M+H]⁺.

Step 5: Compound 3-(6-(4-(dimethoxymethyl)piperidin-1-yl)-5-fluoro-1-methyl-1H-indazol-3-yl)piperidine-2,6-dione (84 mg, 0.2 mmol) was added to 5 ml of dichloromethane. Then, 2 ml of trifluoroacetic acid was added, and the mixture was stirred at room temperature for 1 hour. After the reaction was complete, the mixture was dried under oil pump vacuum to give 1-(3-(2,6-dioxopiperidin-3-yl)-5-fluoro-1-methyl-1H-indazol-6-yl)piperidine-4-carbaldehyde (74.5 mg, 0.2 mmol). Yield: 100%. MS m/z (ESI): 373 [M+H]⁺.

### Preparation of Intermediate 1-(3-(2,6-dioxopiperidin-3-yl)-1-methyl-1H-indazol-6-yl)piperidine-4-carbaldehyde

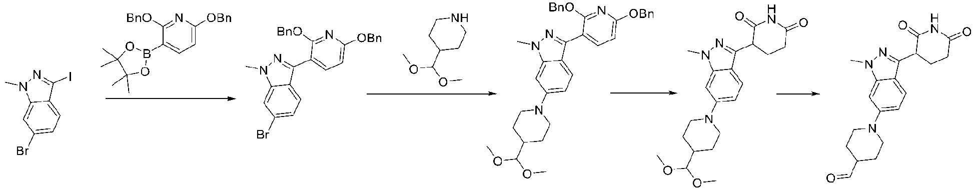

Step 1: 6-Bromo-3-iodo-1-methylindazole (3000 mg, 8.90 mmol) was dissolved in 24 mL of 1,4-dioxane and water (8 mL). Then, 2,6-bis(benzyloxy)-3-(4,4,5,5-tetramethyl-1,3,2-dioxaborolan-2-yl)pyridine (3.72 g, 8.90 mmol), cesium carbonate (5.80 g, 17.81 mmol), and [1,1'-bis(diphenylphosphino)ferrocene]dichloropalladium(II) (0.73 g, 0.98 mmol) were added. The mixture was purged with nitrogen three times and stirred at 95°C for 2 hours under a nitrogen balloon. The reaction solution was poured into 30 mL of water, extracted with ethyl acetate (10 mL×3). The organic phase was washed with saturated brine, dried over anhydrous sodium sulfate, filtered, and dried by rotary evaporation to give a crude product. The crude product was purified by silica gel chromatography (eluent: petroleum ether:ethyl acetate = 10:1 to 1:1) to give 3-[2,6-bis(benzyloxy)pyridin-3-yl]-6-bromo-1-methylindazole (4.00 g, 7.99 mmol) as a white solid, yield: 89.78%.

Step 2: 3-[2,6-Bis(benzyloxy)pyridin-3-yl]-6-bromo-1-methylindazole (1000 mg, 2.00 mmol) was dissolved in 20 mL of 1,4-dioxane. Then, 4-(dimethoxymethyl)piperidine (0.47 mL, 0.20 mmol), cesium carbonate (1953 mg, 6.00 mmol), tris(dibenzylideneacetone)dipalladium(0) (183 mg, 0.20 mmol), and Xphos (95 mg, 0.25 mmol) were added. The mixture was purged with nitrogen three times and stirred at 100°C for 2 hours under a nitrogen balloon. The reaction solution was poured into 50 mL of water, extracted with ethyl acetate (30 mL×3). The organic phase was washed with saturated brine, dried over anhydrous sodium sulfate, filtered, and dried by rotary evaporation to give a crude product. The crude product was purified by silica gel chromatography (eluent: petroleum ether:ethyl acetate = 10:1 to 1:1) to give 3-(2,6-bis(benzyloxy)pyridin-3-yl)-6-(4-(dimethoxymethyl)piperidin-1-yl)-1-methyl-1H-indazole (1000 mg, 2.51 mmol) as a yellow oil, yield: 86.47%. MS m/z (ESI): 579.3 [M+H]⁺.

Step 3: 3-(2,6-bis(benzyloxy)pyridin-3-yl)-6-(4-(dimethoxymethyl)piperidin-1-yl)-1-methyl-1H-indazole (1000 mg, 2.51 mmol) was dissolved in 10 mL of trifluoroethanol. Then, trifluoroacetic acid (0.02 mL, 0.25 mmol) and palladium on carbon (30 mg, 0.25 mmol) were added. The mixture was purged with hydrogen three times and stirred at room temperature for 3 hours under a hydrogen balloon, then transferred to 50°C and reacted for 16 hours. The reaction solution was filtered, and the filtrate was dried by rotary evaporation to give a yellow crude product. The crude product was purified by silica gel chromatography (eluent: dichloromethane:methanol = 20:1 to 10:1) to give 3-(6-(4-(dimethoxymethyl)piperidin-1-yl)-1-methyl-1H-indazol-3-yl)piperidine-2,6-dione (300 mg, yellow oil), yield: 29.85%. MS m/z (ESI): 401.2 [M+H]⁺.

Step 4: 3-(6-(4-(Dimethoxymethyl)piperidin-1-yl)-1-methyl-1H-indazol-3-yl)piperidine-2,6-dione (300 mg, 0.75 mmol) was dissolved in 10 mL of tetrahydrofuran. Then, 5 mL of hydrochloric acid was added. The mixture was reacted at 50°C for 3 hours. The reaction solution was neutralized to pH = 8 with saturated sodium bicarbonate, extracted with ethyl acetate (30 mL×3). The organic phase was washed with saturated brine, dried over anhydrous sodium sulfate, filtered, and dried by rotary evaporation to give a crude product. The crude product was purified by silica gel chromatography (eluent: dichloromethane: methanol = 20:1 to 10:1) to give 1-(3-(2,6-dioxopiperidin-3-yl)-1-methyl-1H-indazol-6-yl)piperidine-4-carbaldehyde (120 mg, yellow oil), yield: 45.20%. MS m/z (ESI): 355.2 [M+H]⁺.

### Preparation of Intermediate 1-(3-(2,6-dioxopiperidin-3-yl)-1-methyl-1H-indazol-6-yl)azetidine-3-carbaldehyde

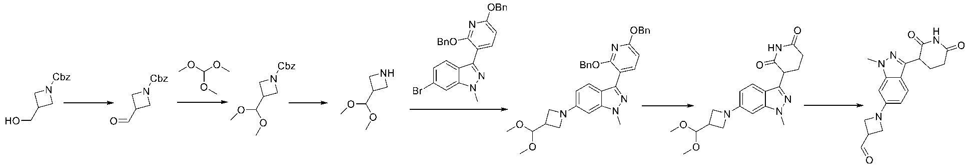

Step 1: Benzyl 3-(hydroxymethyl)azetidine-1-carboxylate (5.0 g, 22.60 mmol) was dissolved in dichloromethane (60 mL). (1,1-Diacetoxy-3-oxo-1,2-benziodoxol-1-yl) acetate (14.38 g, 33.90 mmol) was added, and the mixture was stirred at room temperature, and the mixture was stirred at room temperature for 2 hours. The reaction was monitored by LC-MS. The reaction solution was filtered and concentrated to dryness under reduced pressure at 25°C to give benzyl 3-formylazetidine-1-carboxylate (4.95 g, 22.58 mmol, 100.00% yield) as a yellow solid, which was used directly in the next step. MS m/z (ESI): 220 [M+H]⁺.

Step 2: Benzyl 3-formylazetidine-1-carboxylate (5.0 g, 22.81 mmol) was dissolved in methanol (100 mL). Trimethyl orthoformate (12.10 g, 114.03 mmol) and 4-methylbenzenesulfonic acid (196.37 mg, 1.14 mmol) were added, and the mixture was stirred at room temperature, and the mixture was stirred at room temperature overnight. The reaction was monitored by LC-MS. The reaction solution was concentrated to dryness under reduced pressure, and purified by silica gel column chromatography (EA:PE = 0-50%) to give benzyl 3-(dimethoxymethyl)azetidine-1-carboxylate (4.2 g, 15.83 mmol, 69.41% yield) as a yellow oil. MS m/z (ESI): 266 [M+H]⁺.

Step 3: Benzyl 3-(dimethoxymethyl)azetidine-1-carboxylate (668.62 mg, 2.52 mmol) was dissolved in methanol (20 mL). Palladium on carbon (576.39 mg, 541.62 µmol, 10% purity) was added, and the mixture was stirred at room temperature for 2 hours under hydrogen protection. The reaction was monitored by LC-MS. The reaction solution was filtered and concentrated to dryness under reduced pressure to give 3-(dimethoxymethyl)azetidine (330 mg, 2.52 mmol, 99.82% yield) as a colorless oil. MS m/z (ESI): 132 [M+H]⁺.

Step 4: 6-Bromo-3-(2,6-dibenzyloxypyridin-3-yl)-1-methylindazole (1 g, 2.00 mmol) and 3-(dimethoxymethyl)azetidine (340.79 mg, 2.60 mmol) were dissolved in dioxane (20 mL). Under argon protection, tris(dibenzylideneacetone)dipalladium(0) (183.00 mg, 199.85 µmol), 2-dicyclohexylphosphino-2',4',6'-triisopropylbiphenyl (190.54 mg, 399.69 µmol), and cesium carbonate (1.30 g, 4.00 mmol) were added. The mixture was heated to 100°C and stirred overnight. Product formation was shown by LCMS. The solvent was concentrated under reduced pressure. Water and ethyl acetate were then added, the mixture was extracted three times with ethyl acetate, the organic phases were combined, washed with brine, dried over anhydrous sodium sulfate, and concentrated under reduced pressure to give a crude product. The residue was separated by silica gel column (24 g, 0-40% EA/PE) to give 3-(2,6-dibenzyloxypyridin-3-yl)-6-[3-(dimethoxymethyl)azetidin-1-yl]-1-methylindazole (0.8 g, 1.45 mmol, 72.70% yield). MS m/z (ESI): 551 [M+H]⁺.

Step 5: 3-(2,6-Dibenzyloxypyridin-3-yl)-6-[3-(dimethoxymethyl)azetidin-1-yl]-1-methylindazole (0.8 g, 1.45 mmol) was dissolved in ethanol (20 mL) and tetrahydrofuran (20 mL). Palladium on carbon (154.61 mg, 145.28 µmol, 10% purity) was added. The mixture was purged with hydrogen three times and stirred at room temperature overnight under a hydrogen atmosphere. Hydrogen was replaced again, and the mixture was heated to 40°C and stirred for 4 hours. Product formation was shown by LCMS. The catalyst was filtered off, and the solvent was concentrated under reduced pressure to give crude product 3-[6-[3-(dimethoxymethyl)azetidin-1-yl]-1-methylindazol-3-yl]piperidine-2,6-dione (350 mg, 939.81 µmol, 64.69% yield).

Step 6: Compound 3-[6-[3-(dimethoxymethyl)azetidin-1-yl]-1-methylindazol-3-yl]piperidine-2,6-dione (426.64 mg, 1.15 mmol) was dissolved in formic acid (10 mL), heated to 40°C, and stirred for 2 hours. Product formation was shown by LCMS. The reaction solution was concentrated to dryness under reduced pressure to give 1-(3-(2,6-dioxopiperidin-3-yl)-1-methyl-1H-indazol-6-yl)azetidine-3-carbaldehyde (373.87 mg, 1.15 mmol, 100.00% yield) as a yellow solid. MS m/z (ESI): 327 [M+H]⁺.

### Preparation of Intermediate 1-(4-((2,6-dioxopiperidin-3-yl)amino)-2-fluorophenyl)piperidine-4-carbaldehyde

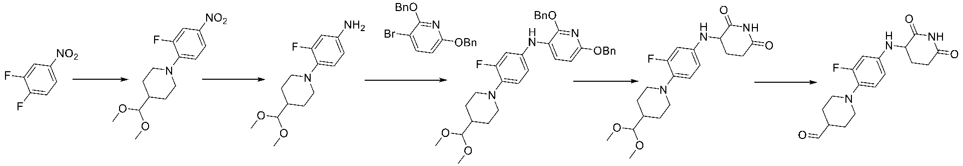

Step 1: At 25°C under a nitrogen atmosphere, 4-(dimethoxymethyl)piperidine (1.0 g, 6.28 mmol), 1,2-difluoro-4-nitrobenzene (1.10 g, 6.91 mmol), and potassium carbonate (1.30 g, 9.42 mmol) were dissolved in N,N-dimethylformamide (60 mL). The mixture was stirred at 25°C for 18 hours. The mixture was diluted with water (500 mL) and extracted with ethyl acetate (100 mL×3). The organic phases were combined, washed with brine (100 mL), dried over anhydrous sodium sulfate, filtered, and concentrated to give a crude product. The crude product was purified by silica gel chromatography (eluent: petroleum ether:ethyl acetate = 1:0 to 10:1) to give [1-(2-fluoro-4-nitrophenyl)piperidin-4-yl]dimethoxymethane (1.8 g, yellow solid). Yield: 96.08%. MS m/z (ESI): 299.1 [M+H]⁺.

Step 2: At 25°C under an air atmosphere, [1-(2-fluoro-4-nitrophenyl)piperidin-4-yl]dimethoxymethane (1.8 g, 6.03 mmol) and palladium on carbon (180 mg) were dissolved in tetrahydrofuran (20 mL). The mixture was purged with hydrogen three times and stirred at 25°C for 18 hours. The mixture was filtered and concentrated to give [1-(4-amino-2-fluorophenyl)piperidin-4-yl]dimethoxymethane (1.6 g, yellow solid). Yield: 86.47%. MS m/z (ESI): 269.2 [M+H]⁺.

Step 3: [1-(4-Amino-2-fluorophenyl)piperidin-4-yl]dimethoxymethane (1.6 g, 5.22 mmol), 2,6-bis(benzyloxy)-3-bromopyridine (1.93 g, 5.22 mmol), 2-(2-aminophenyl)phenylmethanesulfonate palladium, {2-[2,6-bis(propan-2-yloxy)phenyl]phenyl}dicyclohexylphosphine (0.44 g, 0.52 mmol), and cesium carbonate (5.10 g, 15.65 mmol) were dissolved in 1,4-dioxane (50 mL). The mixture was stirred at 100°C under a nitrogen atmosphere for 18 hours. The reaction solution was concentrated to give a crude product. The crude product was purified by silica gel chromatography (eluent: petroleum ether:ethyl acetate = 1:1 to 0:1) to give [1-(4-{[2,6-bis(benzyloxy)pyridin-3-yl]amino}-2-fluorophenyl)piperidin-4-yl]dimethoxymethane (2.0 g, yellow solid). Yield: 68.70%. MS m/z (ESI): 558.6 [M+H]⁺.

Step 4: At 25°C under an air atmosphere, [1-(4-{[2,6-bis(benzyloxy)pyridin-3-yl]amino}-2-fluorophenyl)piperidin-4-yl]dimethoxymethane (2.0 g, 3.59 mmol) and palladium on carbon (2.0 g) were dissolved in tetrahydrofuran (30 mL). The mixture was purged with hydrogen three times and stirred at 25°C for 18 hours. The mixture was filtered and concentrated to give a crude product. The crude product was purified by silica gel chromatography (eluent: petroleum ether:ethyl acetate = 1:1 to 0:1) to give 3-({4-[4-(2,4-dioxapentan-3-yl)piperidin-1-yl]-3-fluorophenyl}amino)piperidine-2,6-dione (400 mg, blue solid). Yield: 29.25%. MS m/z (ESI): 380.2 [M+H]⁺.

Step 5: At room temperature, 3-({4-[4-(2,4-dioxapentan-3-yl)piperidin-1-yl]-3-fluorophenyl} amino)piperidine-2,6-dione (200 mg, 0.530 mmol) and dichloromethane (2.5 mL) were added to a single-neck flask. A 4.0 M solution of hydrogen chloride in dioxane (1.25 mL, 5.00 mmol) was added, and the mixture was stirred at room temperature, and the mixture was reacted at 25°C for 3 hours. After the reaction was complete, the reaction solution was concentrated to give 1-(4-((2,6-dioxopiperidin-3-yl)amino)-2-fluorophenyl)piperidine-4-carbaldehyde (196 mg, green solid). Yield: 100%. MS m/z (ESI): 334.1 [M+H]⁺.

### Preparation of Intermediate 1-(1-(2,6-dioxopiperidin-3-yl)-3-methyl-2-oxo-2,3-dihydro-1H-benzo[d]imidazol-5-yl)-3,3-difluoropiperidine-4-carbaldehyde

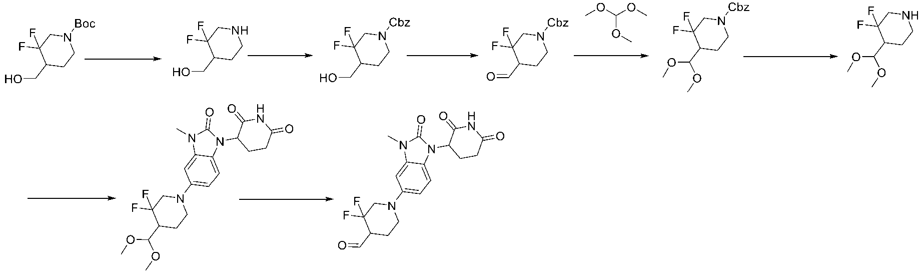

Step 1: tert-Butyl 3,3-difluoro-4-(hydroxymethyl)piperidine-1-carboxylate (1.0 g, 3.98 mmol) was dissolved in dichloromethane (20 mL). A solution of hydrogen chloride in ethyl acetate (4.0 M, 10 mL) was added, and the mixture was stirred at room temperature, and the mixture was stirred at room temperature for 2 hours. The reaction solution was concentrated to dryness under reduced pressure to give (3,3-difluoropiperidin-4-yl)methanol (601.56 mg, 3.98 mmol, 100.00% yield) as a yellow solid. MS m/z (ESI): 152 [M+H]⁺.

Step 2: (3,3-Difluoropiperidin-4-yl)methanol (601.56 mg, 3.98 mmol) was dissolved in dichloromethane (20 mL). Triethylamine (1.21 g, 11.94 mmol) was added, and the mixture was stirred at room temperature, and the mixture was stirred at room temperature for 30 minutes. The temperature was lowered to 0°C, benzyl chloroformate (678.92 mg, 3.98 mmol) was added, and the mixture was stirred at 0°C for 2 hours. The reaction solution was concentrated to dryness under reduced pressure and purified by silica gel column chromatography (EA:PE = 0-50%) to give benzyl 3,3-difluoro-4-(hydroxymethyl)piperidine-1-carboxylate (1.0 g, 3.51 mmol, 88.08% yield) as a yellow solid. MS m/z (ESI): 286 [M+H]⁺.

Step 3: Benzyl 3,3-difluoro-4-(hydroxymethyl)piperidine-1-carboxylate (1.0 g, 3.51 mmol) was dissolved in dichloromethane (20 mL). (1,1-Diacetoxy-3-oxo-1,2-benziodoxol-1-yl) acetate (1.78 g, 4.21 mmol) was added, and the mixture was stirred at room temperature, and the mixture was stirred at room temperature for 2 hours. The reaction solution was filtered, concentrated to dryness under reduced pressure, and purified by silica gel column chromatography (EA:PE = 0-50%) to give benzyl 3,3-difluoro-4-formylpiperidine-1-carboxylate (850 mg, 3.00 mmol, 85.60% yield) as a yellow solid. MS m/z (ESI): 284 [M+H]⁺.

Step 4: Benzyl 3,3-difluoro-4-formylpiperidine-1-carboxylate (850 g, 3.00 mol) was dissolved in methanol (20 mL). Trimethoxymethane (1.59 kg, 15.00 mol) and 4-methylbenzenesulfonic acid (25.84 g, 150.03 mmol) were added, and the mixture was stirred at room temperature, and the mixture was stirred at room temperature overnight. The reaction solution was concentrated to dryness under reduced pressure and purified by silica gel column chromatography (EA:PE = 0-50%) to give benzyl 4-(dimethoxymethyl)-3,3-difluoropiperidine-1-carboxylate (800 mg, 2.43 mmol, 0.081% yield) as a yellow oil. MS m/z (ESI): 330 [M+H]⁺.

Step 5: Benzyl 4-(dimethoxymethyl)-3,3-difluoropiperidine-1-carboxylate (830 mg, 2.52 mmol) was dissolved in methanol (20 mL). Palladium on carbon (576.39 mg, 541.62 µmol, 10% purity) was added, and the mixture was stirred at room temperature for 2 hours under hydrogen protection. The reaction solution was filtered and concentrated to dryness under reduced pressure to give 4-(dimethoxymethyl)-3,3-difluoropiperidine (300 mg, 1.54 mmol, 60.98% yield) as a colorless oil. MS m/z (ESI): 196 [M+H]⁺.

Step 6: 3-(5-Bromo-3-methyl-2-oxobenzimidazol-1-yl)piperidine-2,6-dione (400 mg, 1.18 mmol) and 4-(dimethoxymethyl)-3,3-difluoropiperidine (300.18 mg, 1.54 mmol) were dissolved in dimethyl sulfoxide (10 mL). Under argon protection, methanesulfonato(2-dicyclohexylphosphino-2',6'-diisopropoxy-1,1'-biphenyl)(2'-amino-1,1'-biphenyl-2-yl)palladium(II) (148.58 mg, 177.43 µmol) and cesium carbonate (770.82 mg, 2.37 mmol, 147.86 µL) were added, and the mixture was heated under microwave to 150°C and stirred for 2 hours. The reaction solution was concentrated to dryness under reduced pressure and purified by silica gel column chromatography (MeOH:dichloromethane = 0-10%) to give 3-[5-[4-(dimethoxymethyl)-3,3-difluoropiperidin-1-yl]-3-methyl-2-oxobenzimidazol-1-yl]piperidine-2,6-dione (130 mg, 287.32 µmol, 24.29% yield) as a yellow solid. MS m/z (ESI): 453 [M+H]⁺.

Step 7: 3-[5-[4-(Dimethoxymethyl)-3,3-difluoropiperidin-1-yl]-3-methyl-2-oxobenzimidazol-1-yl]piperidine-2,6-dione (25 mg, 55.25 µmol) was dissolved in dichloromethane (3 mL). TFA (3 mL) was added, and the mixture was stirred at room temperature, and the mixture was stirred at room temperature for 4 hours. The reaction solution was concentrated to dryness under reduced pressure and dissolved in dichloromethane. The resultant was extracted with saturated sodium bicarbonate, dried over anhydrous sodium sulfate, and concentrated to dryness under reduced pressure to give 1-(1-(2,6-dioxopiperidin-3-yl)-3-methyl-2-oxo-2,3-dihydro-1H-benzo[d]imidazol-5-yl)-3,3-difluoropi peridine-4-carbaldehyde (22.45 mg, 55.24 µmol, 100.00% yield) as a yellow solid. MS m/z (ESI): 407 [M+H]⁺.

### Preparation of Intermediate 1-(2-(2,6-dioxopiperidin-3-yl)-1,3-dioxoisoindolin-5-yl)piperidine-4-carbaldehyde

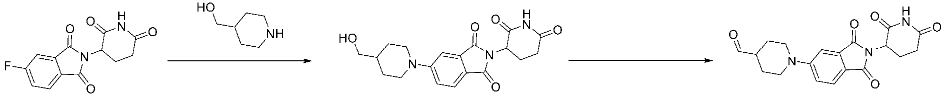

Step 1: 2-(2,6-Dioxopiperidin-3-yl)-5-fluoroisoindoline-1,3-dione (4 g, 14.48 mmol) and piperidin-4-ylmethanol (3.34 g, 28.96 mmol) were dissolved in DMF (40 mL). K₂CO₃ (8.01 g, 57.92 mmol) was then added. The mixture was stirred at 85°C for 20 hours. After the reaction was complete, the reaction solution was filtered, and the filtrate was dried by rotary evaporation under reduced pressure. The resultant was purified by CombiFlash (80 g, 0-12% EA/PE) to successfully give product 2-(2,6-dioxopiperidin-3-yl)-5-(4-(hydroxymethyl)piperidin-1-yl)isoindoline-1,3-dione (1.5 g, 4.04 mmol, 27.89% yield) as a yellow solid. MS m/z (ESI): 372.2 [M+H]⁺.

Step 2: 2-(2,6-Dioxopiperidin-3-yl)-5-(4-(hydroxymethyl)piperidin-1-yl)isoindoline-1,3-dione (1.4 g, 3.77 mmol) was dissolved in DCM (5 mL). Dess-Martin periodinane (2.40 g, 5.65 mmol) was then added. The mixture was stirred at 20°C for 1 hour. After the reaction was complete, the reaction solution was concentrated and purified by CombiFlash (20 g, 0-5% MeOH/DCM). This to successfully give product 1-(2-(2,6-dioxopiperidin-3-yl)-1,3-dioxoisoindolin-5-yl)piperidine-4-carbaldehyde (1.1 g, 2.98 mmol, 79.00% yield) as a yellow solid. MS m/z (ESI): 370.1 [M+H]⁺.

### Preparation of Intermediate 1-(2-(2,6-dioxopiperidin-3-yl)-1-oxoisoindolin-5-yl)piperidine-4-carbaldehyde

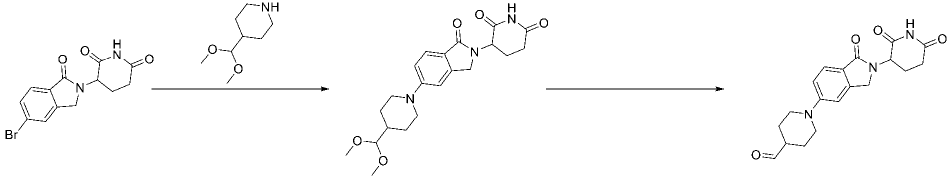

Step 1: At room temperature, 3-(5-bromo-1-oxo-2,3-dihydro-1H-isoindol-2-yl)piperidine-2,6-dione (300 mg, 0.93 mmol) and 4-(dimethoxymethyl)piperidine (221.73 mg, 1.39 mmol) were dissolved in dimethyl sulfoxide (5 ml). Cesium carbonate (605 mg, 1.86 mmol) and (SP-4-1)-[1,3-bis[2,6-bis(1-propylbutyl)phenyl]-4,5-dichloro-1,3-dihydro-2H-imidazol-2-ylidene]dichl oro(3-chloropyridine-N)palladium (90.47 mg, 0.09 mmol) were then added. The reaction solution was purged with nitrogen three times and stirred at 100°C for 1 hour to give a yellow solution. After the reaction was complete, the pH was adjusted to ~7 with formic acid. The mixture was poured into water (20 mL) and extracted with EtOAc (30 mL×3). The organic phases were combined, washed with brine (10 mL), dried over anhydrous Na₂SO₄, filtered, and concentrated to dryness under reduced pressure to give crude 3-{5-[4-(2,4-dioxapentan-3-yl)piperidin-1-yl]-1-oxo-2,3-dihydro-1H-isoindol-2-yl} piperidine-2,6-dione (200 mg, 53.66%) as a yellow oil. MS m/z (ESI): 402.2 [M+H]⁺.

Step 2: At room temperature, 3-{5-[4-(2,4-dioxapentan-3-yl)piperidin-1-yl]-1-oxo-2,3-dihydro-1H-isoindol-2-yl}piperidine-2,6-dione (2.00 ml, 0.25 mmol) was dissolved in tetrahydrofuran (2 ml). Hydrochloric acid (2 ml, 4.00 mmol) was then added, and the resulting mixture was stirred at 70°C for 1 hour to give a yellow solution. After the reaction was complete, the pH was adjusted to ~7 with saturated sodium bicarbonate. The mixture was poured into water (20 mL) and extracted with EtOAc (30 mL×3). The organic phases were combined, washed with brine (10 mL), dried over anhydrous Na₂SO₄, filtered, and concentrated to dryness under reduced pressure to give crude 1-(2-(2,6-dioxopiperidin-3-yl)-1-oxoisoindolin-5-yl)piperidine-4-carbaldehyde (32 mg, 36.15%) as a yellow solid. MS m/z (ESI): 356.1 [M+H]⁺.

### Preparation of Intermediate 8-bromo-1-chloro-N-((5-fluoro-2,3-dihydrobenzofuran-4-yl)methyl)imidazo[1,5-c]pyrimidin-5-amine

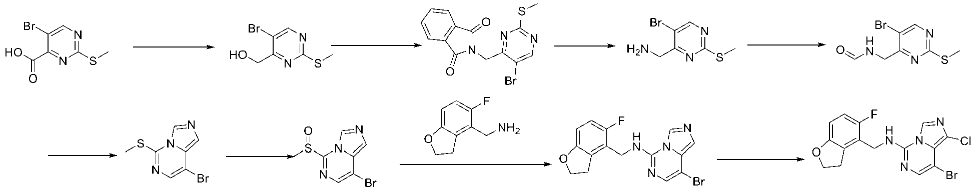

Step 1: 5-Bromo-2-(methylthio)pyrimidine-4-carboxylic acid (130 g, 494.09 mmol) was dissolved in DCM (1000 mL). The mixture was purged with nitrogen, diisobutylaluminum hydride (1 M, 988.17 mL) was added dropwise at -60°C. The mixed solution was stirred at -60°C for 4 hours, then naturally warmed to room temperature and stirred at room temperature (25°C) for 16 hours, and the starting material was completely reacted. Water (80 ml) was slowly added dropwise to the reaction solution at 0°C, and the mixture was stirred at room temperature (25°C) for 0.5 hour. 15% aqueous NaOH solution (80 ml) was then added dropwise at 0°C, and the mixture was stirred for 0.5 hour. The mixture was filtered, and the filter cake was washed three times by slurrying with EA (500 mL×3). The filtrates were combined and concentrated to dryness under reduced pressure to give a crude product. The crude product was purified by combiflash (EA:DCM = 0-5%) to give (5-bromo-2-(methylthio)pyrimidin-4-yl)methanol (41 g, yield: 35.30%) as a white solid. MS m/z (ESI): 235.0 [M+H]⁺.

Step 2: (5-Bromo-2-(methylthio)pyrimidin-4-yl)methanol (40 g, 170.14 mmol) and phthalimide (30.04 g, 204.17 mmol) were dissolved in THF (1500 mL). Triphenylphosphine (66.94 g, 255.21 mmol) was added. The mixture was purged with nitrogen, DIAD (51.61 g, 255.21 mmol) was slowly added dropwise at 0°C. The mixed solution was stirred at 25°C for 2 hours, and the starting material was completely reacted. H₂O (100 ml) was added to the reaction solution, the mixture was extracted with EA (100 mL×3), the organic phases were combined, washed with saturated NaCl (100 ml), dried over anhydrous sodium sulfate, filtered, and the filtrate was concentrated to dryness under reduced pressure to give a crude product. The crude product was purified by combiflash (DCM:MeOH = 0-5%) to give 2-((5-bromo-2-(methylthio)pyrimidin-4-yl)methyl)isoindoline-1,3-dione (60 g, yield: 96.82%) as a yellow solid. MS m/z (ESI): 364.0 [M+H]⁺.

Step 3: 2-((5-Bromo-2-(methylthio)pyrimidin-4-yl)methyl)isoindoline-1,3-dione (60 g, 164.74 mmol) was dissolved in ethanol (1.5 L). Hydrazine hydrate (58.21 g, 988.42 mmol) was added, and the mixed solution was stirred at room temperature (25°C) for 18 hours, and the starting material was completely reacted. The reaction solution was filtered, and the filtrate was concentrated to dryness under reduced pressure. DCM (500 ml) was added, and the mixture was stirred at room temperature for 0.5 hour, filtered, the filtrate was dried over anhydrous sodium sulfate, filtered, and the filtrate was concentrated to dryness under reduced pressure to give crude (5-bromo-2-(methylthio)pyrimidin-4-yl)methanamine (38.5 g, crude) as a yellow oil, which was used directly in the next step without further purification. MS m/z (ESI): 234.0 [M+H]⁺.

Step 4: Formic acid (73.20 g, 1.59 mol, 60.00 mL) and acetic anhydride (67.15 g, 657.79 mmol, 60 mL) were mixed. The mixture was purged with nitrogen, the mixture was stirred at 50°C for 1 hour, then cooled to room temperature (25°C). The reaction solution was slowly added to a solution of (5-bromo-2-(methylthio)pyrimidin-4-yl)methanamine (38.5 g, 164.45 mmol) in DCM (300 ml). The mixture was stirred at room temperature (25°C) for 2 hours, and the starting material was completely reacted. H₂O (500 ml) was added to the reaction solution, the mixture was extracted three times with ethyl acetate (500 mL×3), the organic phases were combined, washed once with saturated sodium chloride solution (500 ml), dried over anhydrous sodium sulfate, filtered, and the filtrate was concentrated to dryness under reduced pressure to give crude N-((5-bromo-2-(methylthio)pyrimidin-4-yl)methyl)formamide (43 g, crude) as a yellow solid, which was used directly in the next step without further purification. MS m/z (ESI): 262.0 [M+H]⁺.

Step 5: N-((5-Bromo-2-(methylthio)pyrimidin-4-yl)methyl)formamide (43 g, 164.04 mmol) was dissolved in dioxane (350 mL). Phosphorus oxychloride (37.73 g, 246.06 mmol) was added. The mixture was purged with nitrogen, the mixed solution was stirred at 110°C for 1.5 hours, and the starting material was completely reacted. The reaction solution was naturally cooled to room temperature, and the reaction solution was concentrated to dryness under reduced pressure. EA (1000 ml) was added, the pH was adjusted to 7-8 with saturated NaHCO₃ solution, the organic phase was collected, dried over anhydrous sodium sulfate, filtered, and the filtrate was concentrated to dryness under reduced pressure to give crude 8-bromo-5-(methylthio)imidazo[1,5-c]pyrimidine (38 g, crude) as a brown solid, which was used directly in the next step without further purification. MS m/z (ESI): 243.9 [M+H]⁺.

Step 6: 8-Bromo-5-(methylthio)imidazo[1,5-c]pyrimidine (36 g, 147.47 mmol) was dissolved in DCM (300 mL). m-CPBA (40.72 g, 235.96 mmol) was added portionwise under ice bath cooling (0°C). The mixed solution was stirred under ice bath cooling (0°C) for 3 hours, and the starting material was completely reacted to give 8-bromo-5-(methylsulfinyl)imidazo[1,5-c]pyrimidine. The reaction solution was used directly in the next step without any workup. MS m/z (ESI): 260 [M+H]⁺.

Step 7: (5-Fluoro-2,3-dihydrobenzofuran-4-yl)methanamine (27.12 g, 162.22 mmol) and triethylamine (29.85 g, 294.95 mmol, 41.14 mL) were added to the reaction solution from Step 6 under ice bath cooling (0°C). The mixed solution was stirred under ice bath cooling (0°C) for 1 hour, then stirred at room temperature for 15 hours, and the starting material was completely reacted. The reaction solution was concentrated to dryness under reduced pressure and purified by combiflash (EA:DCM = 0-40%) to give a yellow crude product. The crude product was slurried twice with acetonitrile (500 mL×2), filtered, and the filter cake was concentrated to dryness under reduced pressure to give 8-bromo-N-((5-fluoro-2,3-dihydrobenzofuran-4-yl)methyl)imidazo[1,5-c]pyrimidin-5-amine (23.2 g, 63.88 mmol, 43.32% yield) as a white solid. MS m/z (ESI): 363.0 [M+H]⁺.

Step 8: 8-Bromo-N-((5-fluoro-2,3-dihydrobenzofuran-4-yl)methyl)imidazo[1,5-c]pyrimidin-5-amine (10 g, 27.53 mmol) was dissolved in N,N-dimethylformamide (200 mL). 1-Chloropyrrolidine-2,5-dione (4.04 g, 30.29 mmol) was added under ice bath cooling, and the mixture was stirred under ice bath cooling, then naturally warmed to room temperature, and stirred overnight. The reaction was monitored by LC-MS. The reaction solution was directly concentrated to dryness under reduced pressure, slurried with dichloromethane, filtered, and the filter cake was collected to give 6.0 g of product. The mother liquor was subjected to column chromatography with DCM:EA = 0-40% to yield 800 mg. A total of 8-bromo-1-chloro-N-((5-fluoro-2,3-dihydrobenzofuran-4-yl)methyl)imidazo[1,5-c]pyrimidin-5-amine (7.5 g, 18.86 mmol, 68.50% yield) was obtained as an off-white solid. MS m/z (ESI): 397 [M+H]⁺.

### Preparation of Intermediate 5-bromo-N-((5-fluoro-2,3-dihydrobenzofuran-4-yl)methyl)-[1,2,4]triazolo[4,3-a]pyrazin-8-amine

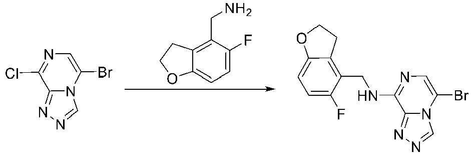

Under argon protection, (5-fluoro-2,3-dihydrobenzofuran-4-yl)methanamine (200 mg, 1.20 mmol), 5-bromo-8-chloro-[1,2,4]triazolo[4,3-a]pyrazine (307.21 mg, 1.32 mmol), TEA (363.17 mg, 3.59 mmol), and ethanol (15 mL) were heated to 90°C and reacted for 2 hours. The reaction solution was filtered, and the filter cake was washed with ethanol, collected, and dried to give 5-bromo-N-((5-fluoro-2,3-dihydrobenzofuran-4-yl)methyl)-[1,2,4]triazolo[4,3-a]pyrazin-8-amine (370 mg, 1.02 mmol, yield 84.93%) as a yellow solid. MS m/z (ESI): 364.0 [M+H]⁺.

### Preparation of Intermediate 8-bromo-1-fluoro-N-((5-fluoro-2,3-dihydrobenzofuran-4-yl)methyl)imidazo[1,5-c]pyrimidin-5-amine

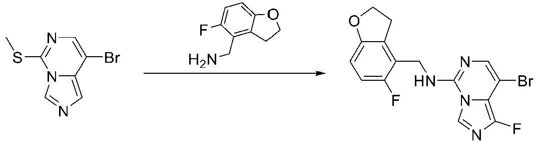

8-Bromo-5-(methylthio)imidazo[1,5-c]pyrimidine (1200 mg, 4.92 mmol) was dissolved in acetonitrile (30 mL). 1-Chloromethyl-4-fluoro-1,4-diazoniabicyclo[2.2.2]octane bis(tetrafluoroborate) (2.09 g, 5.90 mmol) was then added, and the mixture was stirred at room temperature for 18 hours. Et₃N (2.49 g, 24.58 mmol) and (5-fluoro-2,3-dihydrobenzofuran-4-yl)methanamine (986.18 mg, 5.90 mmol) were then added, and the mixture was stirred at room temperature for another 5 hours. The reaction solution was concentrated and purified by basic preparation (preparative column: 21.2×250 mm C18 column; system: 10 mM NH₄HCO₃ H₂O/acetonitrile; wavelength: 254/214 nm; gradient: 0%-70% acetonitrile change) to successfully give product 8-bromo-1-fluoro-N-((5-fluoro-2,3-dihydrobenzofuran-4-yl)methyl)imidazo[1,5-c]pyrimidin-5-amine (130 mg, 341.05 µmol, 6.94% yield) as a light yellow solid. MS m/z (ESI): 381.0 [M+H]⁺. ¹H NMR (400 MHz, DMSO-d₆) δ 8.41 (s, 1H), 8.34 (s, 1H), 7.29 (s, 1H), 6.96 - 6.87 (m, 1H), 6.68 (dd, J = 8.6, 3.9 Hz, 1H), 4.62 (s, 2H), 4.51 (t, J = 8.8 Hz, 2H), 3.24 (t, J = 8.7 Hz, 2H).

### Example 1 Preparation of Compound H1

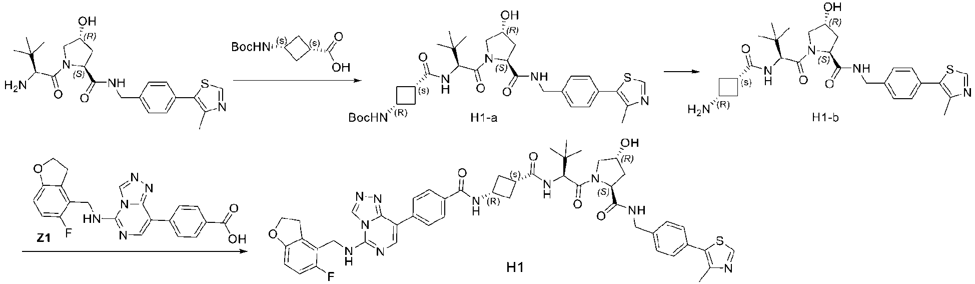

Step 1: (1s,3s)-3-((tert-Butoxycarbonyl)amino)cyclobutanecarboxylic acid (1.2 g, 5.57 mmol) and (2S,4R)-1-((S)-2-amino-3,3-dimethylbutanoyl)-4-hydroxy-N-(4-(4-methylthiazol-5-yl)benzyl)pyrrolidi ne-2-carboxamide (2 g, 4.65 mmol) were added to DMF (2 mL). HATU (2.12 g, 5.57 mmol) and DIPEA (2 mL) were then added, and the mixture was stirred at room temperature for 10 minutes. After the reaction was completed, the reaction solution was purified by column chromatography (DCM/MeOH = 0-10%) to obtain H1-a (2.2 g), yield: 75.44%. MS m/z (ESI): 628 [M+H]+.

Step 2: H1-a (2.2 g, 3.5 mmol) was dissolved in EA (20 mL). A solution of hydrogen chloride in ethyl acetate (4 mol/L, 6 mL) was then slowly added dropwise, and the mixture was reacted at room temperature for 2 hours. After the reaction was completed, the reaction solution was concentrated to obtain H1-b (1.84 g), yield: 100%. MS m/z (ESI): 528 [M+H]⁺.

Step 3: H1-b (156 mg, 296 µmol) and Z1 (100 mg, 246.7 µmol) were dissolved in DMF (2 mL). TCFH (83 mg, 296 µmol) and N-methylimidazole (1 mL) were then added, and the mixture was stirred at room temperature for 1 hour. After the reaction was completed, the reaction solution was concentrated and purified by HPLC (waters-sunfire-10µm-19×150 mm column (mobile phase: 28%-38% (v/v) acetonitrile/aqueous formic acid)) to obtain H1 (34 mg), yield: 15.06%. MS m/z (ESI): 458.5 [M+2H]²⁺/2. ¹H NMR (400 MHz, DMSO-d₆) δ 8.97 (s, 1H), 8.87 (s, 1H), 8.70 (d, J=7.5 Hz, 1H), 8.62 (s, 1H), 8.55 (t, J=6.1 Hz, 1H), 8.42 (s, 1H), 8.18 (d, J=8.5 Hz, 2H), 7.94 (d, J=8.3 Hz, 2H), 7.80 (d, J=9.2 Hz, 1H), 7.45-7.29 (m, 4H), 6.88 (dd, J=10.3, 8.7 Hz, 1H), 6.63 (dd, J=8.6, 3.8 Hz, 1H), 5.11 (d, J=3.6 Hz, 1H), 4.76 (d, J=3.3 Hz, 2H), 4.60-4.15 (m, 8H), 3.76-3.56 (m, 2H), 2.92 (q, J=8.7 Hz, 2H), 2.43 (s, 3H), 2.34-2.13 (m, 4H), 2.02 (t, J=10.5 Hz, 1H), 1.96-1.81 (m, 1H), 1.22 (s, 1H), 0.93 (s, 9H).

### Example 2 Preparation of Compound H2

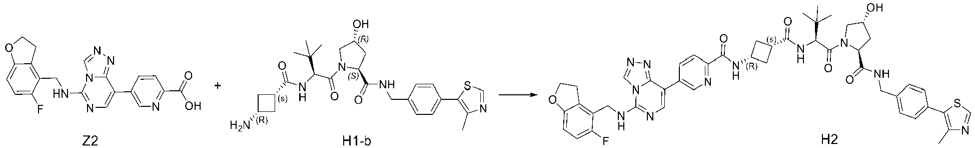

Step 1: H1-b (156 mg, 296 µmol) and Z2 (148 mg, 0.365 mmol) were dissolved in DMF (2 mL). TCFH (83 mg, 296 µmol) and N-methylimidazole (1 mL) were then added, and the mixture was stirred at room temperature for 2 hours. After the reaction was completed, the reaction solution was concentrated and purified by HPLC (waters-sunfire-10µm-19× 150 mm column (mobile phase: 28%-38% (v/v) acetonitrile/aqueous formic acid)) to obtain H2 (14 mg), yield: 5.2%. MS m/z (ESI): 458.7 [M+2]⁺/2. ¹H NMR (400 MHz, DMSO-d₆) δ 9.51 (s, 1H), 9.38 (d, J = 2.2 Hz, 1H), 8.97 (s, 1H), 8.89 (d, J = 8.3 Hz, 2H), 8.72 (dd, J = 8.3, 2.2 Hz, 1H), 8.55 (s, 1H), 8.30 (s, 1H), 8.07 (d, J = 8.3 Hz, 1H), 7.79 (d, J = 9.3 Hz, 1H), 7.51 - 7.31 (m, 4H), 6.95 (dd, J = 10.3, 8.6 Hz, 1H), 6.70 (dd, J = 8.7, 3.8 Hz, 1H), 5.12 (d, J = 3.4 Hz, 1H), 4.75 (s, 2H), 4.63 - 4.48 (m, 3H), 4.46 - 4.26 (m, 4H), 4.21 (dd, J = 15.8, 5.4 Hz, 1H), 3.73 - 3.56 (m, 2H), 3.00 - 2.62 (m, 2H), 2.43 (s, 3H), 2.38 - 2.18 (m, 5H), 2.03 (t, J = 10.6 Hz, 1H), 1.96 - 1.83 (m, 1H), 0.93 (s, 9H).

### Example 3 Preparation of Compound H3

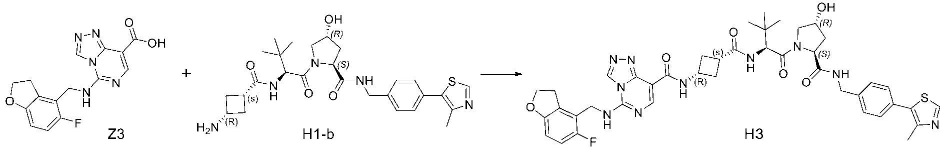

Step 1: H1-b (156 mg, 296 µmol) and Z3 (120 mg, 0.365 mmol) were dissolved in DMF (2 mL). TCFH (83 mg, 296 µmol) and N-methylimidazole (1 mL) were then added, and the mixture was stirred at room temperature for 1 hour. After the reaction was completed, the reaction solution was concentrated and purified by HPLC (waters-sunfire-10µm-19×150 mm column (mobile phase: 28%-38% (v/v) acetonitrile/aqueous formic acid)) to obtain H3 (13.3 mg), yield: 5.4%. MS m/z (ESI): 420.2 [M+2H]²⁺/2. ¹H NMR (400 MHz, DMSO-d₆) δ 9.32 (t, J=5.7 Hz, 1H), 8.96 (s, 1H), 8.79 (d, J=8.0 Hz, 1H), 8.68 (s, 1H), 8.51 (d, J=23.7 Hz, 2H), 7.90 (d, J=9.2 Hz, 1H), 7.45-7.29 (m, 4H), 6.87 (dd, J=10.2, 8.8 Hz, 1H), 6.63 (dd, J=8.7, 3.8 Hz, 1H), 5.11 (d, J=3.6 Hz, 1H), 4.75 (d, J=5.6 Hz, 2H), 4.57-4.31 (m, 7H), 4.20 (dd, J=15.9, 5.4 Hz, 1H), 3.64 (d, J=11.6 Hz, 2H), 3.27-3.19 (m, 3H), 3.01 (t, J=8.5 Hz, 1H), 2.42 (s, 3H), 2.40-2.38 (m, 1H), 2.06 (dq, J=28.2, 9.2, 8.5 Hz, 3H), 1.89 (ddd, J=13.0, 8.6, 4.7 Hz, 1H), 0.92 (s, 9H).

### Example 4 Preparation of Compound H4

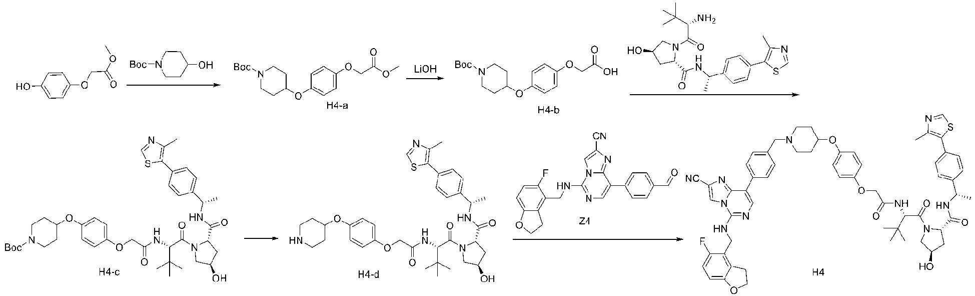

Step 1: tert-Butyl 4-hydroxypiperidine-1-carboxylate (3.39 g, 16.82 mmol) and methyl 2-(4-hydroxyphenoxy)acetate (3 g, 15.29 mmol) were dissolved in THF (60 mL). PPh₃ (6.02 g, 22.94 mmol) was then added. The temperature was cooled to 0°C, and a solution of DEAD (3.33 g, 19.11 mmol) in THF was added dropwise. After the drop addition was completed, the mixture was stirred at room temperature for 18 h. After the reaction was completed, the reaction solution was concentrated and purified by CombiFlash (80 g, 0-100% DCM/PE) to obtain H4-a (3.4 g, colorless oil), yield: 58.60%. MS m/z (ESI): 266.1 [M-100+H]⁺.

Step 2: H4-a (3.4 g, 8.96 mmol) was dissolved in a mixed solution of THF (20 mL)/water (10 mL). LiOH (643.76 mg, 26.88 mmol) was then added. The mixture was stirred at room temperature for 4 h. After the reaction was completed, the reaction solution was concentrated to remove the organic solvent. The aqueous phase was adjusted to pH=4 with dilute hydrochloric acid (0.7 mol/L), and then extracted with a mixed solution of DCM/MeOH (v/v = 10:1). The organic phases were combined and dried over anhydrous sodium sulfate and concentrated to obtain H4-b (2 g, white solid, crude), yield: 63.52%. MS m/z (ESI): 252.1 [M-100+H]⁺.

Step 3: H4-b (1 g, 2.85 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (1.46 g, 3.27 mmol) were dissolved in DMF (21.49 mL). DIPEA (1.10 g, 8.54 mmol, 1.49 mL) and HATU (1.61 g, 4.27 mmol) were then added, and the mixture was stirred at room temperature for 2 h. After the reaction was completed, the reaction solution was concentrated and purified by CombiFlash (40 g, 0-12% MeOH/DCM) to obtain H4-c (0.8 g, white solid), yield: 36.13%. MS m/z (ESI): 678.3 [M-100+H]⁺.

Step 4: H4-c (0.8 g, 1.03 mmol) was dissolved in DCM (10 mL). A solution of HCl in ethyl acetate (4 mol/L, 3 mL) was then added, and the mixture was stirred at room temperature for 1 h. After the reaction was completed, the reaction solution was concentrated to obtain H4-d (734 mg, light yellow solid), yield: 99.93%. MS m/z (ESI): 678.3 [M+H]⁺.

Step 5: Z4 (100 mg, 241.89 µmol) and H4-d (229.56 mg, 338.65 µmol) were dissolved in DMSO (3 mL) and EtOH (1 mL). NaBH₃CN (121.61 mg, 1.94 mmol) and CH₃COOH (72.63 mg, 1.21 mmol) were then added, and the mixture was stirred under microwave at 90°C for 1 h. After the reaction was completed, the reaction solution was cooled to room temperature, and purified by preparative HPLC (preparative column: 21.2×250 mm C18 column; system: 10 mM NH₄HCO₃ H₂O/acetonitrile; wavelength: 254/214 nm; gradient: 0%-60% acetonitrile change) to obtain H4 (73.88 mg), yield: 26.47%. MS m/z (ESI): 538.3 [M/2+H]⁺. ¹H NMR (400 MHz, DMSO-d₆) δ 8.96 (s, 1H), 8.91 (s, 1H), 8.46 (s, 1H), 8.41 (d, J=7.7 Hz, 1H), 8.07 (d, J=5.5 Hz, 1H), 7.89 (d, J=8.3 Hz, 2H), 7.60 (d, J=9.3 Hz, 1H), 7.45-7.29 (m, 6H), 6.97-6.81 (m, 5H), 6.68 (dd, J=8.6, 3.9 Hz, 1H), 5.11 (d, J=3.5 Hz, 1H), 4.93-4.85 (m, 1H), 4.71 (s, 2H), 4.58-4.40 (m, 6H), 4.27 (s, 2H), 3.63-3.46 (m, 4H), 3.28 (d, J=8.9 Hz, 2H), 2.67 (d, J=4.9 Hz, 2H), 2.43 (s, 3H), 2.22 (t, J=9.1 Hz, 2H), 2.06-1.97 (m, 1H), 1.88 (s, 2H), 1.76 (ddd, J=12.9, 8.8, 4.4 Hz, 1H), 1.60 (d, J=8.9 Hz, 2H), 1.36 (d, J=7.0 Hz, 3H), 0.89 (s, 9H).

### Example 5 Preparation of Compound H5

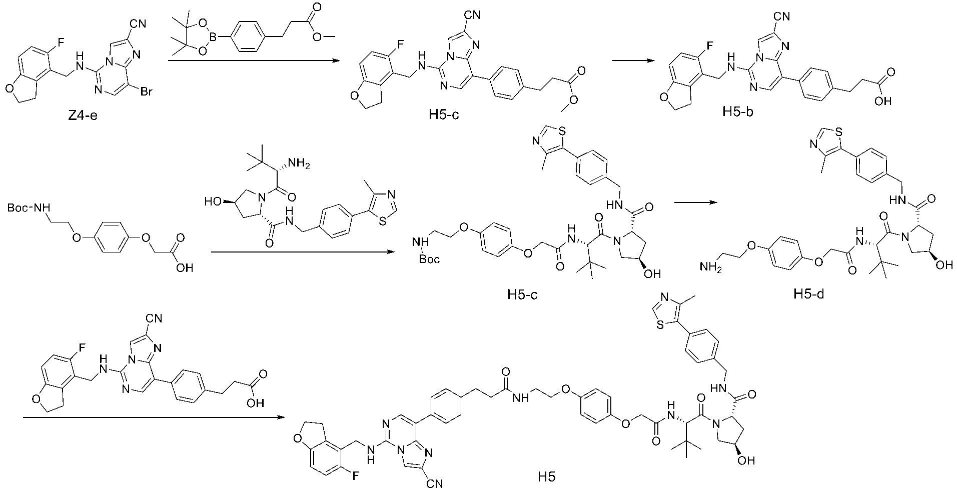

Step 1: Z4-e (300 mg, 772.81 µmol) and methyl 3-(4-(4,4,5,5-tetramethyl-1,3,2-dioxaborolan-2-yl)phenyl)propanoate (246.66 mg, 850.09 µmol) were dissolved in water (1.5 mL) and 1,4-dioxane (7.5 mL). Pd(dppf)Cl₂ (56.55 mg, 77.28 µmol) and NaHCO₃ (194.76 mg, 2.32 mmol) were added, and the mixture was stirred at 100°C for 1.5 hours. After the reaction was completed, the reaction solution was cooled to room temperature, filtered, and the filtrate was concentrated and purified by CombiFlash (12 g, 0-5% MeOH/DCM) to obtain H5-a (320 mg, light yellow solid), yield: 87.82%. MS m/z (ESI): 472.2 [M+H]⁺.

Step 2: H5-a (320 mg, 678.71 µmol) was dissolved in THF (9 mL) and water (5 mL). LiOH (211.30 mg, 8.82 mmol) was added, and the mixture was stirred at room temperature for 4 hours. After the reaction was completed, the mixture was neutralized to pH=3-4 with dilute hydrochloric acid (2 mol/L) and concentrated to obtain H5-b (300 mg, light yellow solid), yield: 96.62%. MS m/z (ESI): 458.1 [M+H]⁺.

Step 3: 2-(4-(2-((tert-Butoxycarbonyl)amino)ethoxy)phenoxy)acetic acid (560 mg, 1.8 mmol) was dissolved in DMF (3 mL). (2S,4R)-1-((S)-2-Amino-3,3-dimethylbutanoyl)-4-hydroxy-N-(4-(4-methylthiazol-5-yl)benzyl)pyrrolidine-2-carboxamide (774 mg, 1.8 mmol), HATU (683 mg, 1.8 mmol), and DIPEA (230 mg, 0.44 mmol) were added, and the mixture was stirred at room temperature for 18 hours. After the reaction was completed, the reaction solution was concentrated, then saturated aqueous ammonium chloride solution (20 mL) was added, and the mixture was extracted with dichloromethane (60 mL×3). The combined organic phases were washed with saturated brine (20 mL×2), dried over anhydrous sodium sulfate, concentrated, and purified by CombiFlash (12 g, 0-10% MeOH/DCM) to obtain H5-c (650 mg, light yellow solid), yield: 50%. MS m/z (ESI): 724 [M+H]⁺.

Step 4: H5-c (650 mg, 0.9 mmol) was dissolved in DCM (10 mL). A solution of hydrogen chloride in dioxane (4 mol/L) was then added dropwise, and the mixture was stirred at room temperature for 1 hour. After the reaction was completed, the mixture was concentrated to obtain H5-d (562 mg, light yellow solid), yield: 100% yield. MS m/z (ESI): 624 [M+H]⁺.

Step 5: H5-b (150 mg, 327.90 µmol) and H5-d (245.44 mg, 393.48 µmol) were dissolved in DMF (6 mL). DIPEA (127.14 mg, 983.70 µmol, 171.34 µL) and HATU (185.56 mg, 491.85 µmol) were added, and the mixture was stirred at room temperature for 18 hours. After the reaction was completed, the reaction solution was concentrated and preliminarily purified by CombiFlash (12 g, 0-15% MeOH/DCM), then further purified by preparative HPLC (preparative column: 21.2×250 mm C18 column; system: 10 mM NH₄HCO₃ H₂O/acetonitrile; wavelength: 254/214 nm; gradient: 0%-70% acetonitrile change) to obtain H5 (75.54 mg), yield: 20.96%. MS m/z (ESI): 532.4 [M/2+H]⁺. ¹H NMR (400 MHz, DMSO-d₆) δ 8.96 (s, 1H), 8.91 (s, 1H), 8.57 (t, J = 6.0 Hz, 1H), 8.44 (t, J = 4.9 Hz, 1H), 8.09 (t, J = 5.5 Hz, 1H), 8.01 (d, J = 6.9 Hz, 1H), 7.83 (d, J = 8.2 Hz, 2H), 7.69 (d, J = 9.4 Hz, 1H), 7.44-7.32 (m, 4H), 7.27 (d, J = 8.3 Hz, 2H), 6.97-6.89 (m, 1H), 6.85 (s, 4H), 6.68 (dd, J = 8.6, 3.9 Hz, 1H), 5.13 (d, J = 3.5 Hz, 1H), 4.71 (d, J = 4.8 Hz, 2H), 4.62-4.48 (m, 5H), 4.46-4.37 (m, 3H), 4.23 (dd, J = 15.7, 5.7 Hz, 1H), 3.88 (t, J = 5.6 Hz, 2H), 3.58-3.67 (m, 2H), 3.42-3.33 (m, 2H), 3.30-3.27 (m, 3H), 2.84 (t, J = 7.8 Hz, 2H), 2.45-2.40 (m, 4H), 2.10-1.99 (m, 1H), 1.92-1.85 (m, 1H), 0.90 (s, 9H).

### Example 6 Preparation of Compound H6

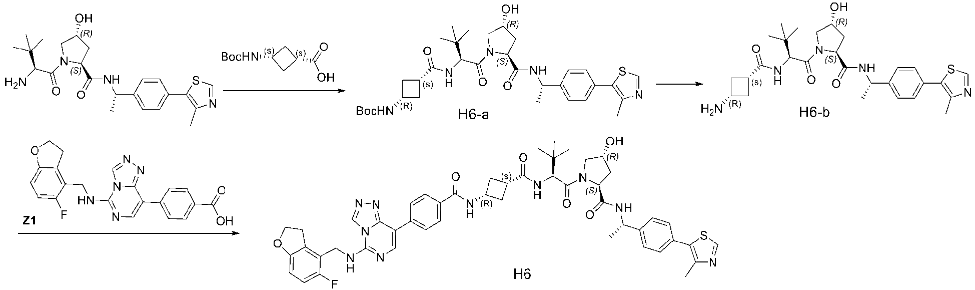

Step 1: (1s,3s)-3-((tert-Butoxycarbonyl)amino)cyclobutane-1-carboxylic acid (1.2 g, 5.57 mmol) and (2S,4R)-1-((S)-2-amino-3,3-dimethylbutanoyl)-4-hydroxy-N-((S)-1-(4-(4-methylthiazol-5-yl) phenyl)ethyl)pyrrolidine-2-carboxamide (2 g, 4.6 mmol) were added to DMF (2 mL). HATU (2.12 g, 5.57 mmol) and DIPEA (2 mL) were added, and the mixture was stirred at room temperature for 10 min. After the reaction was completed, the reaction solution was concentrated and purified by column chromatography (DCM/MeOH = 0-10%) to obtain H6-a (2.2 g, 3.4 mmol), yield: 75.4%. MS m/z (ESI): 642 [M+H]⁺.

Step 2: H6-a (2.2 g, 3.4 mmol) was dissolved in EA (20 mL). A solution of hydrogen chloride in ethyl acetate (4 mol/L, 6 mL) was then added dropwise, and the mixture was stirred at room temperature for 2 hours. After the reaction was completed, the reaction solution was concentrated to obtain H6-b (1.84 g, 3.5 mmol), yield: 100%. MS m/z (ESI): 542 [M+H]⁺.

Step 3: H6-b (156 mg, 288 µmol) and Z1 (100 mg, 246.7 µmol) were dissolved in DMF (2 mL). TCFH (83 mg, 296 µmol) and N-methylimidazole (1 mL) were added, and the mixture was stirred at room temperature. After the reaction was completed, the reaction solution was concentrated and purified by preparative HPLC (waters-sunfire-10µm-19×150 mm column (mobile phase: 28%-38% (v/v) acetonitrile/aqueous formic acid)) to obtain H6 (12 mg), yield: 5.23%. MS m/z (ESI): 465.2 [M+2H]²⁺/2. ¹H NMR (400 MHz, DMSO-d₆) δ 9.49 (s, 1H), 8.96 (s, 1H), 8.79 (t, J=5.2 Hz, 1H), 8.70 (d, J=7.6 Hz, 1H), 8.37 (d, J=7.9 Hz, 1H), 8.28-8.20 (m, 2H), 8.16 (s, 1H), 7.95 (d, J=8.6 Hz, 2H), 7.73 (d, J=9.2 Hz, 1H), 7.47-7.34 (m, 4H), 7.02-6.87 (m, 1H), 6.69 (dd, J=8.6, 3.9 Hz, 1H), 5.08 (d, J=3.6 Hz, 1H), 4.90 (q, J=7.3 Hz, 1H), 4.73 (d, J=5.0 Hz, 2H), 4.62-4.48 (m, 3H), 4.47-4.20 (m, 3H), 3.61 (d, J=5.3 Hz, 2H), 2.92 (t, J=8.6 Hz, 2H), 2.44 (s, 3H), 2.41 (s, 1H), 2.34-2.12 (m, 3H), 2.01 (dd, J=22.2, 11.0 Hz, 1H), 1.78 (ddd, J=12.9, 8.4, 4.7 Hz, 1H), 1.36 (d, J=7.0 Hz, 3H), 1.22 (s, 1H), 0.93 (s, 9H).

### Example 7 Preparation of Compound H7

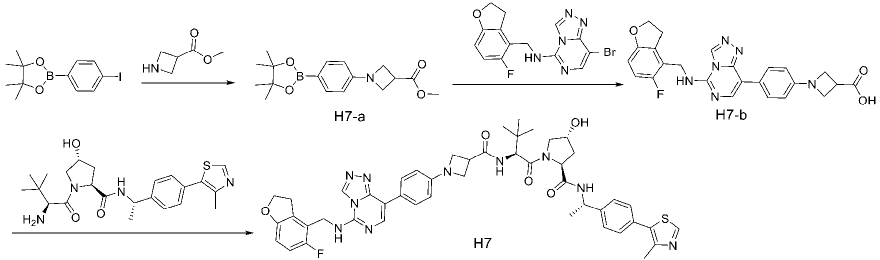

Step 1: 2-(4-Iodophenyl)-4,4,5,5-tetramethyl-1,3,2-dioxaborolane (1 g, 303 µmol) and methyl azetidine-3-carboxylate (500 mg, 4.35 mmol) were mixed and added to dioxane (5 mL). Cesium carbonate (2.8 g, 8.7 mmol), catalyst Pd₂(dba)₃ (27 mg, 30 µmol), and X-Phos (30 mg) were then added. Under a nitrogen atmosphere, the mixture was heated to 110°C and stirred for 12 hours. After the reaction was completed, the reaction solution was purified by column chromatography (PE/EA = 5-40%) to obtain H7-a (317 mg), yield: 23%. MS m/z (ESI): 318 [M+H]⁺.

Step 2: H7-a (317 mg, 1 mmol) and Z1-a (200 mg, 0.55 mmol) were added to a dioxane aqueous solution (5 mL, 20%). Cesium carbonate (350 mg, 1.1 mmol) and catalyst Pd(PPh₃)₄ (63 mg, 55 µmol) were then added. Under a nitrogen atmosphere, the mixture was heated to 110°C and stirred for 2 hours. After the reaction was completed, the reaction solution was purified by column chromatography (DCM/MeOH = 0-10%) to obtain H7-b (50 mg, 0.11 mmol), yield: 20%. MS m/z (ESI): 461 [M+H]⁺.

Step 3: Compound (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (49 mg, 0.11 mmol) and H7-b (50 mg, 0.11 mmol) were dissolved in DMF (2 mL). TCFH (31 mg, 110 µmol) and N-methylimidazole (1 ml) were added, and the mixture was stirred at room temperature for 1 hour. After the reaction was completed, the reaction solution was concentrated and purified by HPLC (waters-sunfire-10µm-19×150 mm column (mobile phase: 28%-38% (v/v) acetonitrile/aqueous formic acid)) to obtain H7 (4.4 mg), yield: 4.5%. MS m/z (ESI): 444.2 [M+2H]²⁺/2. ¹H NMR (400 MHz, DMSO-d₆) δ 9.43 (s, 1H), 8.96 (s, 1H), 8.54-8.24 (m, 2H), 7.88-7.78 (m, 3H), 7.70 (d, J=9.1 Hz, 1H), 7.49-7.29 (m, 4H), 6.98-6.88 (m, 1H), 6.68 (dd, J=8.7, 3.8 Hz, 3H), 6.13 (t, J=6.0 Hz, 1H), 5.84 (s, 1H), 5.51 (s, 1H), 5.11 (d, J=3.5 Hz, 1H), 4.89 (t, J=7.2 Hz, 1H), 4.69 (d, J=4.9 Hz, 2H), 4.61 (d, J=9.2 Hz, 1H), 4.52 (t, J=8.7 Hz, 2H), 4.44 (t, J=8.1 Hz, 1H), 4.29 (s, 1H), 3.95 (dd, J=10.4, 5.9 Hz, 2H), 3.62 (d, J=3.1 Hz, 2H), 3.27 (s, 1H), 2.44 (s, 3H), 2.04 (d, J=14.5 Hz, 1H), 1.78 (ddd, J=12.9, 8.7, 4.7 Hz, 1H), 1.35 (d, J=7.0 Hz, 3H), 1.22 (s, 1H), 0.95 (s, 9H).

### Example 8 Preparation of Compound H8

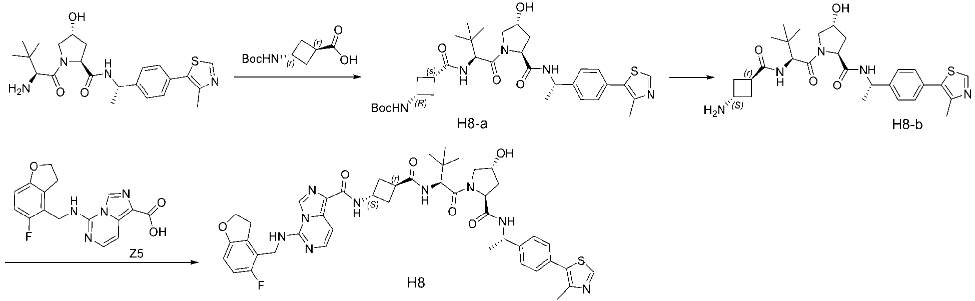

Step 1: (2S,4R)-1-((S)-2-Amino-3,3-dimethylbutanoyl)-4-hydroxy-N-((S)-1-(4-(4-methylthiazol-5-yl)phenyl)ethyl)pyrrolidine-2-carboxamide (380 mg, 0.38 mmol) and trans-3-(BOC-amino)cyclobutanecarboxylic acid (81 mg, 0.38 mmol) were dissolved in DMF (3 mL). HATU (172 mg, 0.45 mmol) and DIPEA (393 mg, 3.04 mmol) were added, and the mixture was stirred at 25°C for 1 h. After the reaction was completed, the reaction solution was poured into water (10 mL), and the aqueous phase was extracted with ethyl acetate (20 mL×3). The organic phases were combined and dried over anhydrous sodium sulfate, concentrated, and purified by silica gel column chromatography (DCM/EA = 70/30 to 50/50) to obtain H8-a (130 mg, colorless gum), yield: 53.3%. MS m/z (ESI): 642.4 [M+H]⁺.

Step 2: H8-a (70 mg, 0.11 mmol) was dissolved in 1,4-dioxane (1 mL). A solution of hydrogen chloride in ethyl acetate (2 mol/L, 1 mL) was then added, and the reaction solution was stirred at 25°C for 2 h. After the reaction was completed, the reaction solution was concentrated to obtain H8-b (55 mg, white solid), yield: 93.09%. MS m/z (ESI): 542.3 [M+H]⁺.

Step 3: Z5 (29.09 mg, 0.09 mmol) and H8-b (40 mg, 0.07 mmol) were dissolved in a mixed solution of acetonitrile (1 mL) and DMF (0.3 mL). N-Methylimidazole (0.06 mL, 0.74 mmol) and TCFH (41.44 mg, 0.15 mmol) were then added, and the reaction solution was stirred at 25°C for 18 h. After the reaction was completed, the reaction solution was concentrated and purified by preparative HPLC (Waters-Xbridge-C18-10µm-19×250 mm column (mobile phase: 10%-95% (v/v) acetonitrile and water containing (10 mmol ammonium bicarbonate))) to obtain H8 (3.62 mg), yield: 5.75%. MS m/z (ESI): 426.6 [1/2 M+H]⁺. ¹H NMR (400 MHz, DMSO-d₆) δ 8.98 (s, 1H), 8.64 (s, 1H), 8.47-8.21 (m, 3H), 7.76 (d, J = 9.2 Hz, 1H), 7.46-7.36 (m, 5H), 7.23 (d, J = 6.4 Hz, 1H), 6.98-6.90 (m, 1H), 6.70 (dd, J = 8.8, 4.0 Hz, 1H), 5.13 (d, J = 3.6 Hz, 1H), 4.97-4.87 (m, 1H), 4.74-4.48 (m, 6H), 4.43 (t, J = 8.0 Hz, 1H), 4.30 (s, 1H), 3.65-3.63 (m, 1H), 3.31-3.20 (m, 3H), 3.12-3.02 (m, 1H), 2.45 (s, 3H), 2.39 (d, J = 9.2 Hz, 1H), 2.35-2.23 (m, 3H), 2.02 (m, 1H), 1.85-1.70 (m, 1H), 1.37 (d, J = 7.2 Hz, 3H), 0.95 (s, 9H).

### Example 9 Preparation of Compound H9

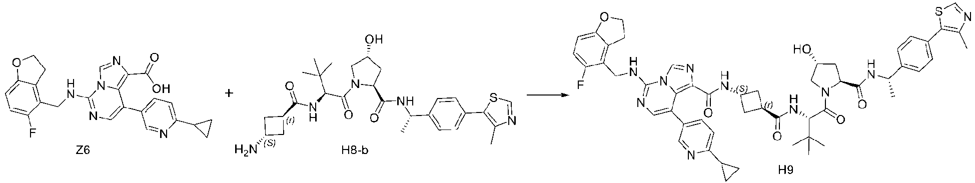

Step 1: Z6 (100 mg, 0.22 mmol) and H8-b (182.41 mg, 0.34 mmol) were dissolved in acetonitrile (4 mL). N-Methylimidazole (368.66 mg, 4.49 mmol) and TCFH (125.98 mg, 0.45 mmol) were then added, and the reaction solution was stirred at 25°C for 18 h. After the reaction was completed, the reaction solution was concentrated and purified by preparative HPLC (Waters-Xbridge-C18-10µm-19×250 mm column (mobile phase: 47%-61% (v/v) acetonitrile and water (containing 10 mmol ammonium bicarbonate))) to obtain H9 (5.51 mg), yield: 2.53%. MS m/z (ESI): 969.9 [M+H]⁺. ¹H NMR (400 MHz, DMSO-d₆) δ 8.98 (s, 1H), 8.77 (s, 1H), 8.50-8.46 (m, 1H), 8.37 (d, J=7.6 Hz, 1H), 8.27 (d, J=2.4 Hz, 1H), 8.20 (d, J=8.4 Hz, 1H), 7.71 (d, J=9.2 Hz, 1H), 7.46-7.41 (m, 3H), 7.38 (d, J=8.4 Hz, 2H), 7.30 (s, 1H), 7.21 (d, J=7.6 Hz, 1H), 6.97-6.91 (m, 1H), 6.71-6.68 (m, 1H), 5.11 (d, J=3.6 Hz, 1H), 4.91 (t, J=7.2 Hz, 1H), 4.73 (d, J=4.8 Hz, 2H), 4.57-4.50 (m, 3H), 4.45-4.26 (m, 3H), 3.64-3.60 (m, 2H), 3.29-3.26 (m, 3H), 2.45 (s, 3H), 2.21-1.98 (m, 7H), 1.75-1.81 (m, 1H), 1.37 (d, J=7.2 Hz, 3H), 0.94 (s, 12H).

### Example 10 Preparation of Compound H10

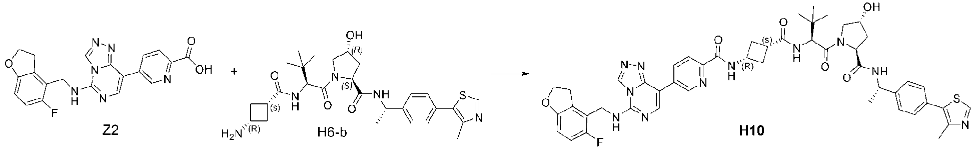

Step 1: H6-b (131 mg, 296 µmol) and Z2 (148 mg, 0.365 mmol) were dissolved in DMF (2 mL). TCFH (83 mg, 296 µmol) and N-methylimidazole (1 mL) were then added, and the mixture was stirred at room temperature for 1 hour. After the reaction was completed, the reaction solution was concentrated and purified by HPLC (waters-sunfire-10µm-19×150 mm column (mobile phase: 28%-38% (v/v) acetonitrile/aqueous formic acid)) to obtain H10 (2.3 mg), yield: 8.1%. MS m/z (ESI): 465.7 [M+2H]²⁺/2. ¹H NMR (400 MHz, DMSO-d₆) δ 9.52 (s, 1H), 9.38 (d, J=2.3 Hz, 1H), 8.96 (s, 1H), 8.90 (d, J=8.2 Hz, 1H), 8.72 (dd, J=8.2, 2.2 Hz, 1H), 8.49-8.33 (m, 2H), 8.29 (s, 1H), 8.06 (d, J=8.3 Hz, 1H), 7.73 (d, J=9.2 Hz, 1H), 7.46-7.32 (m, 4H), 7.01-6.89 (m, 1H), 6.69 (dd, J=8.7, 3.9 Hz, 1H), 5.10 (s, 1H), 4.90 (t, J=7.2 Hz, 1H), 4.74 (s, 3H), 4.63-4.42 (m, 4H), 4.46-4.22 (m, 3H), 3.60 (s, 2H), 2.96-2.87 (m, 1H), 2.43 (s, 3H), 2.34-2.19 (m, 3H), 2.00 (t, J=10.3 Hz, 1H), 1.79 (d, J=13.0 Hz, 1H), 1.36 (d, J=7.0 Hz, 3H), 1.21 (s, 1H), 0.93 (s, 9H).

### Example 11 and Example 12 Preparation of Compounds H11 and H12

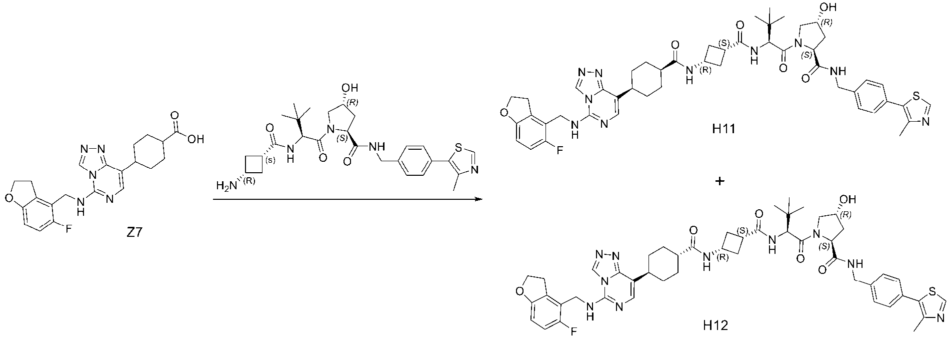

Step 1: Compound (2S,4R)-1-((S)-2-((1s,3R)-3-aminocyclobutane-1-carboxamido)-3,3-dimethylbutanoyl)-4-hydroxy-N-(4-(4-methylthiazol-5-yl)benzyl)pyrrolidine-2-carboxamide (156 mg, 296 µmol) and Intermediate Z7 (150 mg, 0.365 mmol) were dissolved in DMF (2 mL). TCFH (83 mg, 296 µmol) and N-methylimidazole (1 mL) were added, and the mixture was stirred at room temperature for 2 hours. After the reaction was completed, the reaction solution was concentrated and separated by preparative HPLC, and purified by high pressure liquid chromatography (waters-sunfire-10 µm-19×150 mm column (mobile phase: 28%-38% (v/v) acetonitrile and aqueous formic acid)) to obtain H11 (6 mg) and H12 (12 mg). Yield: 6.61%. MS m/z (ESI): 461.3 [M+2H]²⁺/2. H11: ¹H NMR (400 MHz, DMSO-d₆) δ 9.35 (s, 1H), 8.97 (s, 1H), 8.54 (t, J = 6.2 Hz, 1H), 8.32 (t, J = 5.1 Hz, 1H), 7.94 (d, J = 7.7 Hz, 1H), 7.75 (d, J = 9.3 Hz, 1H), 7.45-7.30 (m, 5H), 6.97-6.84 (m, 1H), 6.67 (dd, J = 8.7, 3.8 Hz, 1H), 5.10 (d, J = 3.6 Hz, 1H), 4.62 (d, J = 4.9 Hz, 2H), 4.55-4.44 (m, 3H), 4.46-4.06 (m, 5H), 3.71-3.57 (m, 2H), 3.03-2.79 (m, 2H), 2.43 (s, 3H), 2.29 (dd, J = 41.3, 23.5 Hz, 4H), 1.98 (ddd, J = 41.7, 20.4, 11.0 Hz, 8H), 1.76 (d, J = 11.2 Hz, 2H), 1.55 (s, 3H), 0.91 (s, 9H).

### Example 13 Preparation of Compound H13

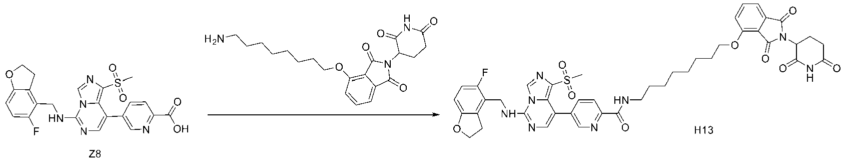

Step 1: 4-((8-aminooctyl)oxy)-2-(2,6-dioxopiperidin-3-yl)isoindoline-1,3-dione (32 mg, 0.07 mmol), HATU (35 mg, 0.09 mmol), and DIPEA (32 mg, 0.25 mmol) were added to a solution of Intermediate Z8 (30 mg, 0.06 mmol) in DMF (2 mL). The mixture was stirred at 25°C under a nitrogen atmosphere for 2 hours. After the reaction was completed, the reaction solution was poured into water (5 mL), filtered, and the filter cake was dried to obtain a crude product. The crude product was purified by preparative HPLC (Gilson_306_1741, column: Waters-SunFire-C18-10µm-19×250mm; mobile phase: water (containing 0.1% formic acid) and acetonitrile, gradient ratio: acetonitrile 47%-95%, flow rate: 25 mL/min) to obtain H13 (5 mg), yield: 9.30%. MS m/z (ESI): 867.4 [M+H]⁺. ¹H NMR (400 MHz, DMSO-*d₆*) δ 11.09 (s, 1H), 8.91 (s, 1H), 8.85-8.74 (m, 2H), 8.64-8.58 (m, 1H), 8.03-7.92 (m, 2H), 7.82-7.75 (m, 1H), 7.53-7.47 (m, 2H), 7.42 (d, J = 7.2 Hz, 1H), 6.99-6.90 (m, 1H), 6.75-6.67 (m, 1H), 5.12-5.04 (m, 1H), 4.75 (d, J = 4.8 Hz, 2H), 4.55 (t, J = 8.8 Hz, 2H), 4.20 (t, J = 6.4 Hz, 2H), 3.31-3.26 (m, 3H), 3.10 (s, 3H), 2.93-2.81 (m, 1H), 2.63-2.52 (m, 2H), 2.07-1.98 (m, 1H), 1.81-1.71 (m, 2H), 1.61-1.51 (m, 2H), 1.50-1.41 (m, 2H), 1.39-1.19 (m, 7H).

### Example 14 Preparation of Compound H14

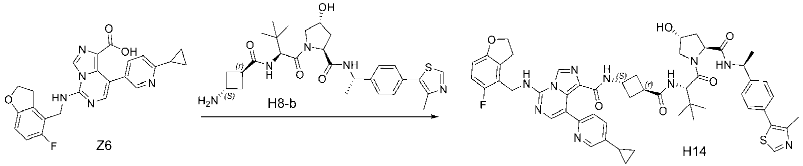

Step 1: Intermediate Z6 (40 mg, 0.09 mmol) and H8-b (49 mg, 0.09 mmol) were dissolved in DMF (3 mL). DIPEA (58 mg, 0.45 mmol) and HATU (68 mg, 0.18 mmol) were added, and the mixture was stirred at 50°C for 1 hour. H8-b (49 mg, 0.09 mmol) was added to the reaction solution, and the reaction solution was stirred at 50°C for another 2 hours. After the reaction was completed, the reaction solution was filtered, and the filtrate was concentrated and purified by preparative HPLC (Waters-Xbridge-C18-10µm-19×250 mm column (mobile phase: 47%-61% (v/v) acetonitrile and water (0.1% formic acid))) to obtain H14 (23 mg), yield: 26.43%. MS m/z (ESI): 969.3 [M+H]⁺. ¹H NMR (400 MHz, DMSO-d₆) δ 8.98 (s, 1H), 8.74 (s, 1H), 8.50 (t, J = 5.2 Hz, 1H), 8.37 (d, J = 7.6 Hz, 1H), 8.29 (d, J = 2.0 Hz, 1H), 8.14-8.12 (m, 1H), 7.72 (d, J = 8.8 Hz, 1H), 7.48-7.37 (m, 5H), 7.33 (dd, J = 8.2, 2.3 Hz, 1H), 7.19-7.17 (m, 1H), 6.98-6.91 (m, 1H), 6.73-6.67 (m, 1H), 5.15-5.09 (m, 1H), 4.96-4.87 (m, 1H), 4.75 (d, J = 4.8 Hz, 1H), 4.53-4.51 (m, 3H), 4.45-4.40 (m, 1H), 4.38-4.31 (m, 1H), 4.30-4.25 (m, 1H), 3.63 (s, 2H), 3.29-3.26 (m, 1H), 3.05-2.96 (m, 1H), 2.45 (s, 3H), 2.22-2.08 (m, 4H), 2.06-1.90 (m, 3H), 1.83-1.75 (m, 1H), 1.37 (d, J = 6.8 Hz, 3H), 1.03-0.98 (m, 2H), 0.94 (s, 9H), 0.77-0.72 (m, 2H).

### Example 15 and Example 16 Preparation of Compounds H15 and H16

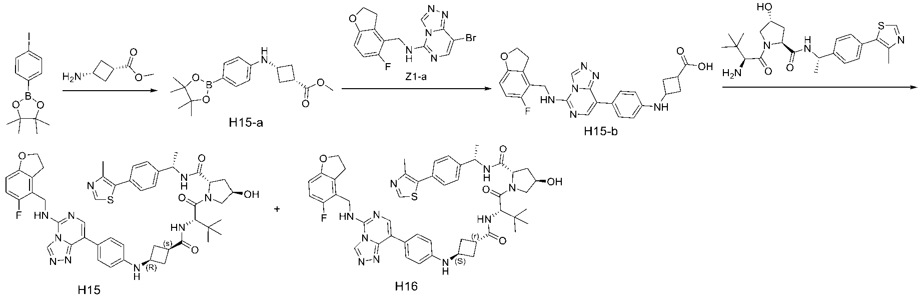

Step 1: 2-(4-Iodophenyl)-4,4,5,5-tetramethyl-1,3,2-dioxaborolane (1 g, 303 µmol) and methyl (1s,3s)-3-aminocyclobutanecarboxylate (561 mg, 4.35 mmol) were added to dioxane (5 mL). Cesium carbonate (2.8 g, 8.7 mmol), Pd₂(dba)₃ (27 mg, 30 µmol), and X-Phos (30 mg) were added. Under a nitrogen atmosphere, the mixture was heated to 110°C and stirred for 12 hours. After the reaction was completed, the reaction solution was purified by silica gel column chromatography (PE/EA 5-40%) to obtain H15-a (331 mg, 1 mmol), yield: 23%. MS m/z (ESI): 332 [M+H]⁺.

Step 2: H15-a (331 mg, 1 mmol) and Z1-a (200 mg, 0.55 mmol) were added to a dioxane aqueous solution (5 mL, v/v = 20%). Cesium carbonate (350 mg, 1.1 mmol) and Pd(PPh₃)₄ (63 mg, 55 µmol) were added. Under a nitrogen atmosphere, the mixture was heated to 110°C and stirred for 2 hours. After the reaction was completed, the reaction solution was purified by silica gel column chromatography (0-10% DCM/MeOH) to obtain H15-b (52 mg, 0.11 mmol), yield: 20%. MS m/z (ESI): 475 [M+H]⁺.

Step 3: H15-b (52 mg, 0.11 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (45 mg, 0.11 mmol) were dissolved in DMF (2 mL). TCFH (31 mg, 110 µmol) and N-methylimidazole (1 mL) were then added, and the mixture was stirred at room temperature for 1 hour. After the reaction was completed, the reaction solution was concentrated and purified by preparative HPLC (waters-sunfire-10 µm-19×150 mm column (mobile phase: 28%-38% (v/v) acetonitrile and aqueous formic acid)) to obtain H15 (2 mg) and H16 (2 mg), yield: 4.5%. MS m/z (ESI): 451.2 [M+2H]²⁺/2.

H15: ¹H NMR (400 MHz, DMSO-*d₆*) δ 8.96 (s, 1H), 8.54 (s, 1H), 8.51 (s, 1H), 8.36 (d, J = 7.8 Hz, 1H), 8.10 (s, 1H), 7.76 (d, J = 8.6 Hz, 3H), 7.45-7.32 (m, 4H), 6.98-6.81 (m, 1H), 6.68 - 6.55 (m, 3H), 6.08 (d, J = 7.2 Hz, 1H), 5.08 (d, J = 3.6 Hz, 1H), 4.90 (t, J = 7.2 Hz, 1H), 4.72 (d, J = 5.8 Hz, 2H), 4.54-4.44 (m, 3H), 4.40 (t, J = 8.0 Hz, 1H), 4.26 (s, 1H), 3.87-3.69 (m, 1H), 3.59 (s, 2H), 3.25 (d, J = 8.8 Hz, 3H), 2.94 (q, J = 8.7 Hz, 1H), 2.43 (s, 3H), 2.38-2.36 (m, 1H), 1.97 (dt, J = 18.9, 9.6 Hz, 3H), 1.77 (ddd, J = 13.0, 8.5, 4.6 Hz, 1H), 1.35 (d, J = 6.9 Hz, 3H), 0.91 (s, 9H).

H16: ¹H NMR (400 MHz, DMSO-d₆) δ 8.96 (s, 1H), 8.54 (s, 1H), 8.51 (s, 1H), 8.36 (d, J = 7.8 Hz, 1H), 8.10 (s, 1H), 7.75 (d, J = 8.6 Hz, 3H), 7.45-7.31 (m, 4H), 6.98-6.80 (m, 1H), 6.68-6.55 (m, 3H), 6.08 (d, J = 7.2 Hz, 1H), 5.08 (d, J = 3.6 Hz, 1H), 4.90 (t, J = 7.2 Hz, 1H), 4.72 (d, J = 5.8 Hz, 2H), 4.54-4.43 (m, 3H), 4.39 (t, J = 8.0 Hz, 1H), 4.26 (s, 1H), 3.87-3.68 (m, 1H), 3.60 (s, 2H), 3.25 (d, J = 8.8 Hz, 3H), 2.94 (q, J = 8.7 Hz, 1H), 2.43 (s, 3H), 2.38-2.36 (m, 1H), 1.97 (dt, J = 18.9, 9.6 Hz, 3H), 1.78 (ddd, J = 13.0, 8.5, 4.6 Hz, 1H), 1.35 (d, J = 6.9 Hz, 3H), 0.90 (s, 9H).

### Example 17 Preparation of Compound H17

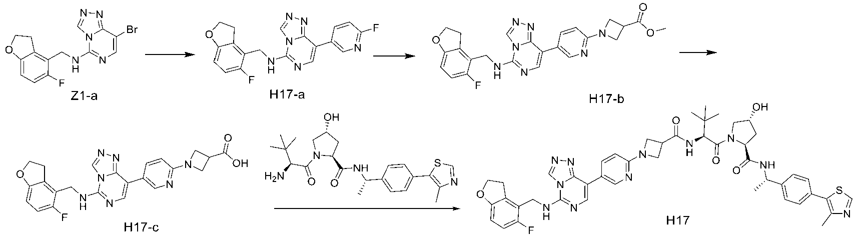

Step 1: Z1-a (300 mg, 823.8 µmol) and compound (6-fluoropyridin-3-yl)boronic acid (232 mg, 1.65 mmol) were mixed and added to a dioxane aqueous solution (5 mL, 20%). K₂CO₃ (342 mg, 2.47 mmol) and Pd(dppf)Cl₂ (73.1 mg, 0.1 mmol) were added. The mixture was purged with nitrogen three times, then heated to 100°C and stirred at 100°C for 12 hours. After the reaction was completed, the reaction solution was purified by silica gel column chromatography (PE/EA 15-100%) to obtain H17-a (175 mg), yield: 56.10%. MS m/z (ESI): 381 [M+H]⁺.

Step 2: H17-a (175 mg, 0.46 mmol) and methyl azetidine-3-carboxylate (190 mg, 1.65 mmol) were added to DMSO (5 mL). DIPEA (1 mL) was added, and the mixture was heated to 130°C and stirred at 130°C for 4 hours. After the reaction was completed, the reaction solution was purified by silica gel column chromatography (PE/EA 5-100%) to obtain H17-b (219 mg), yield: 56.10%. MS m/z (ESI): 476 [M+H]⁺.

Step 3: H17-b (219 mg, 0.46 mmol) was added to a tetrahydrofuran aqueous solution (2 mL, 50%). Lithium hydroxide (40 mg) was added, and the mixture was stirred at room temperature for 12 hours. After the reaction was completed, the pH was adjusted to neutrality with dilute hydrochloric acid (2 mol/L), and the reaction solution was purified by silica gel column chromatography (DCM/MeOH 0-10%) to obtain H17-c (184 mg), yield: 86.9%. MS m/z (ESI): 462 [M+H]⁺.

Step 4: H17-c (51 mg, 0.11 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (45 mg, 0.11 mmol) were dissolved in DMF (2 mL). TCFH (31 mg, 110 µmol) and N-methylimidazole (1 mL) were added, and the mixture was stirred at room temperature for 1 hour. After the reaction was completed, the reaction solution was concentrated and purified by preparative HPLC (waters-sunfire-10 µm-19×150 mm column (mobile phase: 28%-38% (v/v) acetonitrile and aqueous formic acid)) to obtain H17 (20 mg), yield: 46%. MS m/z (ESI): 444.7 [M+2H]²⁺/2. ¹H NMR (400 MHz, DMSO-d₆) δ 8.96 (s, 1H), 8.74 - 8.70 (m, 1H), 8.67 (s, 1H), 8.58 (s, 1H), 8.37 (d, J = 7.8 Hz, 1H), 8.24-8.05 (m, 3H), 7.42 (d, J = 8.3 Hz, 2H), 7.36 (d, J = 8.2 Hz, 2H), 6.91-6.80 (m, 1H), 6.62 (dd, J = 8.6, 3.8 Hz, 1H), 6.50 (s, 1H), 5.08 (s, 1H), 4.99-4.84 (m, 1H), 4.73 (d, J = 5.8 Hz, 2H), 4.55-4.41 (m, 4H), 4.26 (s, 1H), 4.18-3.84 (m, 5H), 3.70 (t, J = 7.6 Hz, 1H), 3.60 (s, 2H), 2.44 (s, 3H), 2.32 (d, J = 9.0 Hz, 1H), 2.06-1.95 (m, 1H), 1.83-1.69 (m, 1H), 1.36 (d, J = 7.1 Hz, 4H), 0.94 (s, 9H).

### Example 18 Preparation of Compound H18

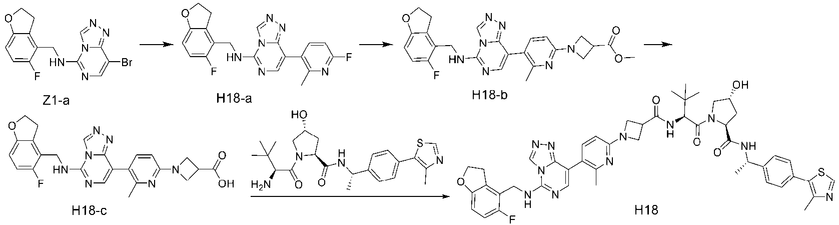

Step 1: Z1-a (300 mg, 823.8 µmol) and ((6-fluoro-2-methylpyridin-3-yl)boronic acid (256 mg, 1.65 mmol) were added to a dioxane aqueous solution (5 mL, 20%). K₂CO₃ (342 mg, 2.47 mmol) and Pd(dppf)Cl₂ (73.1 mg, 0.1 mmol) were added. The mixture was purged with nitrogen three times, then heated to 100°C and stirred at 100°C for 12 hours. After the reaction was completed, the reaction solution was purified by silica gel column chromatography (PE/EA 15-100%) to obtain H18-a (182 mg), yield: 56.10%. MS m/z (ESI): 395 [M+H]⁺.

Step 2: H18-a (182 mg, 0.46 mmol) and methyl azetidine-3-carboxylate (190 mg, 1.65 mmol) were added to DMSO (5 mL). DIPEA (1 mL) was added, and the mixture was heated to 130°C and stirred at 130°C for 4 hours. After the reaction was completed, the reaction solution was purified by silica gel column chromatography (PE/EA 5-100%) to obtain H18-b (225 mg), yield: 56.10%. MS m/z (ESI): 490 [M+H]⁺.

Step 3: H18-b (225 mg, 0.46 mmol) was added to a tetrahydrofuran aqueous solution (2 mL, 50%). Lithium hydroxide (40 mg) was added, and the mixture was stirred at room temperature for 12 hours. After the reaction was completed, the pH was adjusted to neutrality with dilute hydrochloric acid (2 mol/L), and then the reaction solution was purified by silica gel column chromatography (DCM/MeOH 0-10%) to obtain H18-c (190 mg), yield: 86.9%. MS m/z (ESI): 476 [M+H]⁺.

Step 4: H18-c (53 mg, 0.11 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (45 mg, 0.11 mmol) were dissolved in DMF (2 mL). TCFH (31 mg, 110 µmol) and N-methylimidazole (1 mL) were added, and the mixture was stirred at room temperature for 2 hours. After the reaction was completed, the reaction solution was concentrated and purified by preparative HPLC (waters-sunfire-10 µm-19×150 mm column (mobile phase: 28%-38% (v/v) acetonitrile and aqueous formic acid)) to obtain H18 (3.56 mg), yield: 8.1%. MS m/z (ESI): 451.7 [M+2H]²⁺/2. ¹H NMR (400 MHz, DMSO-d₆) δ 8.96 (s, 1H), 8.63 (t, J = 5.8 Hz, 1H), 8.49 (s, 1H), 8.37 (d, J = 7.8 Hz, 1H), 8.08 (d, J = 9.2 Hz, 1H), 7.80 (s, 1H), 7.49-7.29 (m, 5H), 6.87 (dd, J = 10.4, 8.7 Hz, 1H), 6.63 (dd, J = 8.6, 3.8 Hz, 1H), 6.29 (d, J = 8.4 Hz, 1H), 5.30 (t, J = 4.8 Hz, 1H), 5.09 (d, J = 3.6 Hz, 1H), 4.98-4.83 (m, 1H), 4.72 (d, J = 5.7 Hz, 2H), 4.59-4.35 (m, 4H), 4.26 (s, 1H), 3.99 (ddt, J = 51.8, 25.2, 7.6 Hz, 5H), 3.69 (q, J = 7.2 Hz, 1H), 3.60 (s, 2H), 2.44 (s, 3H), 2.20 (s, 3H), 1.77 (ddd, J = 13.2, 8.8, 4.7 Hz, 1H), 1.45 (s, 1H), 1.36 (d, J = 7.1 Hz, 3H), 0.94 (s, 9H).

### Example 19 Preparation of Compound H19

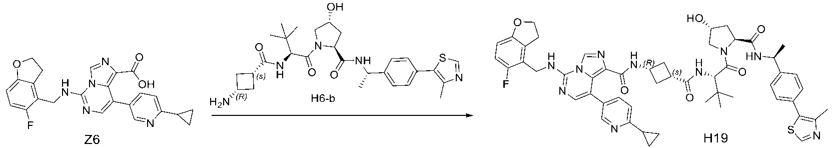

Step 1: Intermediate Z6 (50.00 mg, 0.11 mmol) and H6-b (60.80 mg, 0.11 mmol) were dissolved in DMF (3 mL). DIPEA (72.54 mg, 0.56 mmol) and HATU (42.68 mg, 0.11 mmol) were added, and the mixture was stirred at room temperature for 10 minutes. After the reaction was completed, the reaction solution was filtered, and the filtrate was concentrated and purified by preparative HPLC (Waters-Xbridge-C18-10µm-19×250 mm column (mobile phase: 47%-61% (v/v) acetonitrile and water (0.1% ammonium bicarbonate))) to obtain H19 (15 mg), yield: 13.79%. MS m/z (ESI): 485.1 [M/2+H]⁺. ¹H NMR (400 MHz, DMSO-d₆) δ 8.76 (s, 1H), 8.76 (s, 1H), 8.48 (t, J = 4.8 Hz, 1H), 8.38 (d, J = 7.8 Hz, 1H), 8.27 (d, J = 2.0 Hz, 1H), 8.12 (d, J = 8.3 Hz, 1H), 7.70 (d, J = 9.2 Hz, 1H), 7.45-7.42 (m, 2H), 7.38 (d, J = 8.3 Hz, 2H), 7.30 (s, 1H), 7.21 (d, J = 8.1 Hz, 1H), 6.98-6.89 (m, 1H), 6.70 (dd, J = 8.6, 3.8 Hz, 1H), 5.10 (d, J = 3.6 Hz, 1H), 4.96-4.87 (m, 1H), 4.73 (d, J = 4.7 Hz, 2H), 4.58-4.49 (m, 3H), 4.42 (t, J = 8.1 Hz, 1H), 4.28 (s, 1H), 4.03 (d, J = 7.9 Hz, 1H), 3.60 (d, J = 7.1 Hz, 2H), 3.29 (d, J = 8.6 Hz, 2H), 2.82 (s, 1H), 2.45 (s, 3H), 2.31-1.88 (m, 7H), 1.79 (s, 1H), 1.37 (d, J = 7.0 Hz, 3H), 0.94 (d, J = 12.9 Hz, 13H).

### Example 20 Preparation of Compound H20

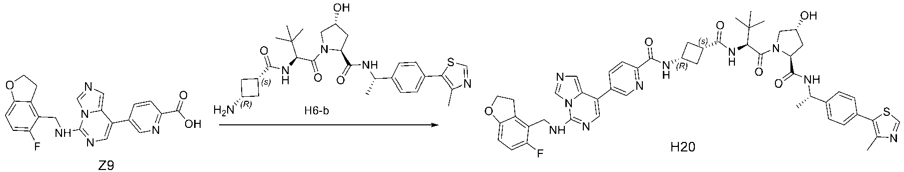

Step 1: H6-b (97.0 mg, 0.170 mmol) and Intermediate Z9 (68.0 mg, 0.170 mmol) were dissolved in DMF (2 mL). DIPEA (87.7 µL, 0.50 mmol) and HATU (76.5 mg, 0.20 mmol) were added, and the mixture was stirred at 25°C for 30 minutes. After the reaction was completed, the reaction solution was purified by reverse phase preparative HPLC (10 mM ammonium bicarbonate aqueous solution/acetonitrile 100/36-100/95) to obtain H20 (41.2 mg), yield: 26.44%. MS m/z (ESI): 928.9 [M+H]⁺. ¹H NMR (400 MHz, DMSO-d₆) δ 8.98 (s, 1H), 8.94 - 8.88 (m, 2H), 8.75 (s, 1H), 8.55 (t, J = 4.8 Hz, 1H), 8.39 (d, J = 7.8 Hz, 1H), 8.26 (dd, J = 8.2, 2.3 Hz, 1H), 8.07 (d, J = 8.9 Hz, 1H), 7.75 (d, J = 9.2 Hz, 1H), 7.57 (d, J = 21.7 Hz, 2H), 7.47-7.35 (m, 4H), 7.00-6.89 (m, 1H), 6.70 (dd, J = 8.6, 3.9 Hz, 1H), 5.11 (d, J = 3.6 Hz, 1H), 4.92 (t, J = 7.3 Hz, 1H), 4.76 (d, J = 4.8 Hz, 2H), 4.59-4.50 (m, 3H), 4.44 (t, J = 8.1 Hz, 1H), 4.40-4.31 (m, 1H), 4.29 (s, 1H), 3.62 (s, 2H), 3.29 (s, 1H), 2.94 (t, J = 8.3 Hz, 1H), 2.46-2.44 (m, 4H), 2.42-2.19 (m, 4H), 2.08-1.97 (m, 1H), 1.88-1.73 (m, 1H), 1.38 (d, J = 7.0 Hz, 3H), 0.95 (s, 9H).

### Example 21 Preparation of Compound H21

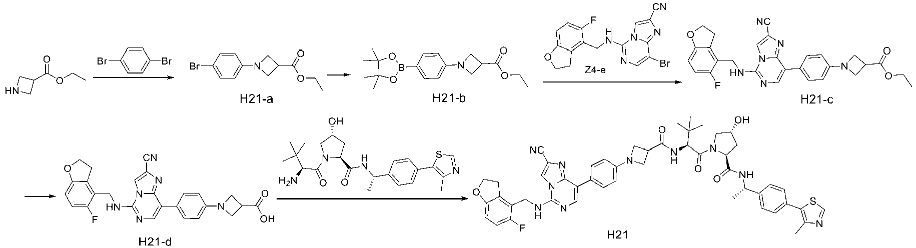

Step 1: 1,4-Dibromobenzene (11.67 g, 49.48 mmol) and ethyl azetidine-3-carboxylate (2.5 g, 16.49 mmol, HCl) were dissolved in toluene (100 mL). Cs₂CO₃ (16.12 g, 49.48 mmol) and Pd₂(dba)₃ (1.51 g, 1.65 mmol) were added, and the mixture was stirred at 110°C for 18 hours. After the reaction was completed, the reaction solution was cooled to room temperature, filtered, and the filtrate was concentrated and purified by CombiFlash (80 g, 0-20% EA/PE) to obtain H21-a (4.2 g, brown oil), yield: 94.28%. MS m/z (ESI): 284.0 [M+H]⁺.

Step 2: H21-a (4.2 g, 14.78 mmol) was dissolved in 1,4-dioxane (40 mL). (Pin)₂B₂ (6.38 g, 25.13 mmol), KOAc (4.35 g, 44.34 mmol), and Pd(dppf)Cl₂ (1.08 g, 1.48 mmol) were added, and the mixture was stirred at 120°C for 3 hours. After the reaction was completed, the reaction solution was cooled to room temperature, filtered, and the filtrate was concentrated and purified by CombiFlash (80 g, 0-30% EA/PE) to obtain H21-b (3 g, white solid), yield: 61.28%. MS m/z (ESI): 332.2 [M+H]⁺.

Step 3: Z4-e (300 mg, 772.81 µmol) and H21-b (281.56 mg, 850.09 µmol) were dissolved in water (1.5 mL) and 1,4-dioxane (7.5 mL). Pd(dppf)Cl₂ (56.55 mg, 77.28 µmol) and NaHCO₃ (194.76 mg, 2.32 mmol) were added, and the mixture was stirred at 100°C for 2 hours. After the reaction was completed, the reaction solution was cooled to room temperature, filtered, and the filtrate was concentrated and purified by CombiFlash (12 g, 0-5% MeOH/DCM) to obtain H21-c (320 mg, light yellow solid), yield: 80.79%. MS m/z (ESI): 513.2 [M+H]⁺.

Step 4: H21-c (320 mg, 624.35 µmol) was dissolved in THF (10 mL) and water (5 mL). LiOH (300 mg, 12.53 mmol) was added, and the mixture was stirred at room temperature for 4 hours. After the reaction was completed, the pH was adjusted to 3-4 with dilute hydrochloric acid (2 mol/L), and the reaction solution was concentrated to obtain H21-d (300 mg, light yellow solid), yield: 99.18%. MS m/z (ESI): 485.1 [M+H]⁺.

Step 5: H21-d (100 mg, 206.41 µmol) was dissolved in DMF (5 mL). DIPEA (106.71 mg, 825.63 µmol, 143.81 µL) and HATU (116.81 mg, 309.61 µmol) were added, and the mixture was reacted at room temperature for 30 minutes. Then (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (110.12 mg, 247.69 µmol) was added, and the mixture was stirred at room temperature for 1 hour. After the reaction was completed, the reaction solution was purified by basic preparative HPLC (preparative column: 21.2×250 mm C18 column; system: 10 mM NH₄HCO₃ H₂O/acetonitrile; wavelength: 254/214 nm; gradient: 0%-70% acetonitrile change) to obtain H21 (51.52 mg), yield: 27.40%. MS m/z (ESI): 456.3 [M/2+H]⁺. ¹H NMR (400 MHz, DMSO-*d₆*) δ 8.96 (s, 1H), 8.88 (s, 1H), 8.37 (d, J = 7.9 Hz, 1H), 8.32 (s, 1H), 8.08 (d, J = 9.2 Hz, 1H), 7.94 (s, 1H), 7.78 (d, J = 8.6 Hz, 2H), 7.42 (d, J = 8.3 Hz, 2H), 7.36 (d, J = 8.3 Hz, 2H), 6.96-6.88 (m, 1H), 6.68 (dd, J = 8.6, 3.8 Hz, 1H), 6.51 (d, J = 8.7 Hz, 2H), 5.08 (d, J = 3.5 Hz, 1H), 4.91 (dd, J = 13.8, 6.5 Hz, 1H), 4.69 (d, J = 4.4 Hz, 2H), 4.57-4.48 (m, 3H), 4.42 (t, J = 8.2 Hz, 1H), 4.26 (s, 1H), 4.02 (t, J = 7.5 Hz, 1H), 3.95 (t, J = 7.5 Hz, 1H), 3.84 (t, J = 6.7 Hz, 1H), 3.78 (t, J = 6.5 Hz, 1H), 3.71 (dd, J = 14.1, 6.7 Hz, 1H), 3.60 (s, 2H), 3.27 (d, J = 8.6 Hz, 2H), 2.44 (s, 3H), 2.04-1.95 (m, 1H), 1.80-1.73 (m, 1H), 1.36 (d, J = 6.9 Hz, 3H), 0.94 (s, 9H).

### Example 22 Preparation of Compound H22

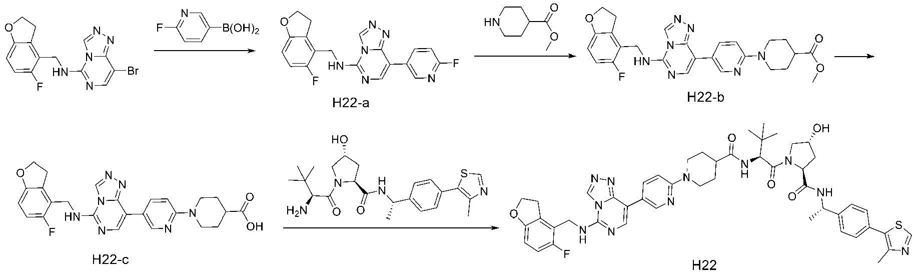

Step 1: 8-Bromo-N-((5-fluoro-2,3-dihydrobenzofuran-4-yl)methyl)-[1,2,4]triazolo[4,3-c] pyrimidin-5-amine (400 mg, 1.10 mmol) and (6-fluoropyridin-3-yl)boronic acid (464 mg, 3.30 mmol) were dissolved in 1,4-dioxane (10 mL) and water (2 mL). Pd(dppf)Cl₂ (81 mg, 0.11 mmol) and K₂CO₃ (304 mg, 2.20 mmol) were added, and the mixture was heated to 90°C under argon protection for 6 hours. After the reaction was completed, the reaction solution was poured into water, extracted with ethyl acetate (30 mL×2). The organic phases were combined and dried over anhydrous sodium sulfate, concentrated, and purified by silica gel column chromatography (PE:EA = 0-100%) to obtain H22-a (280 mg, yellow solid), yield: 67%. MS m/z (ESI): 381.1 [M+H]⁺.

Step 2: H22-a (160 mg, 0.42 mmol) and methyl isonipecotate (362 mg, 2.52 mmol) were dissolved in DMF (8 mL). K₂CO₃ (582 mg, 4.21 mmol) was added, and the mixture was stirred at 130°C for 4 hours. After the reaction was completed, the reaction solution was poured into water, extracted with ethyl acetate (30 mL×2). The organic phases were combined and dried over anhydrous sodium sulfate, concentrated, and purified by silica gel column chromatography (PE:EA = 0-100%) to obtain H22-b (140 mg, yellow solid), yield: 64.3%. MS m/z (ESI): 504.2 [M+H]⁺.

Step 3: H22-b (140 mg, 0.28 mmol) was dissolved in methanol (10 mL) and water (2 mL). Lithium hydroxide monohydrate (117 mg, 2.78 mmol) was added, and the mixture was stirred at room temperature overnight. After the reaction was completed, the pH was adjusted to 5 with dilute hydrochloric acid (1 mol/L), and the reaction solution was extracted with dichloromethane (30 mL×2). The organic phases were combined and dried over anhydrous sodium sulfate and concentrated to obtain H22-c (100 mg, yellow solid, crude), yield: 73.5%. MS m/z (ESI): 490.2 [M+H]⁺.

Step 4: H22-c (50 mg, 0.1 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (50 mg, 0.11 mmol) were dissolved in DMF (5 mL). DIPEA (40 mg, 0.31 mmol) and HATU (58 mg, 0.15 mmol) were added, and the mixture was stirred at room temperature for 4 hours. After the reaction was completed, the reaction solution was poured into water, extracted with ethyl acetate (30 mL×2). The organic phases were combined and dried over anhydrous sodium sulfate, concentrated, and purified by preparative HPLC (preparative column: 21.2×250 mm C18 column; system: 10 mM NH₄HCO₃ H₂O/acetonitrile; wavelength: 254/214 nm; gradient: 0%-60% acetonitrile change) to obtain H22 (12.42 mg), yield: 12.56%. MS m/z (ESI): 458.8 [M/2+H]⁺. ¹H NMR (400 MHz, DMSO-d₆) δ 8.96 (s, 1H), 8.76 (t, J = 4.3 Hz, 1H), 8.64 (d, J = 8.1 Hz, 1H), 8.58 (s, 1H), 8.36 (d, J = 7.8 Hz, 1H), 8.21 (s, 1H), 8.16 (dd, J = 9.0, 2.5 Hz, 1H), 7.82 (d, J = 9.1 Hz, 1H), 7.39 (dd, J = 22.2, 8.3 Hz, 4H), 6.96-6.83 (m, 3H), 6.62 (dd, J = 8.6, 3.9 Hz, 1H), 5.08 (d, J = 3.5 Hz, 1H), 4.95-4.86 (m, 1H), 4.73 (d, J = 4.5 Hz, 2H), 4.51-4.46 (m, 3H), 4.41 (t, J = 8.1 Hz, 1H), 4.35 (d, J = 10.2 Hz, 2H), 4.26 (s, 1H), 3.63-3.53 (m, 2H), 3.26 (t, J = 8.8 Hz, 2H), 2.84 (s, 1H), 2.66 (td, J = 11.7, 9.3 Hz, 1H), 2.43 (s, 3H), 2.00 (dd, J = 15.0, 4.9 Hz, 1H), 1.80-1.73 (m, 2H), 1.65 (d, J = 11.1 Hz, 1H), 1.56-1.49 (m, 2H), 1.36 (d, J = 7.0 Hz, 3H), 0.92 (d, J = 8.7 Hz, 9H).

### Example 23 Preparation of Compound H23

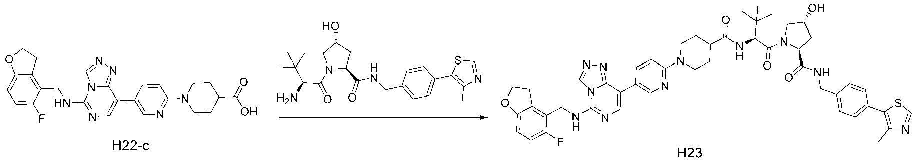

Step 1: H22-c (50 mg, 0.1 mmol) and (2S,4R)-1-((S)-2-amino-3,3-dimethylbutanoyl)-4-hydroxy-N-(4-(4-methylthiazol-5-yl)benzyl)pyrrolidine-2-carboxamide (48 mg, 0.11 mmol) were dissolved in DMF (5 mL). DIPEA (40 mg, 0.31 mmol) and HATU (58 mg, 0.15 mmol) were added, and the mixture was stirred at room temperature for 4 hours. After the reaction was completed, the reaction solution was poured into water, extracted with ethyl acetate (30 mL×2). The organic phases were combined and dried over anhydrous sodium sulfate, concentrated, and purified by preparative HPLC (preparative column: 21.2×250 mm C18 column; system: 10 mM NH₄HCO₃ H₂O/acetonitrile; wavelength: 254/214 nm; gradient: 0%-60% acetonitrile change) to obtain H23 (16.81 mg), yield: 18.24%. MS m/z (ESI): 451.7 [M/2+H]⁺. ¹H NMR (400 MHz, DMSO-*d₆*) δ 8.97 (s, 1H), 8.75 (d, J = 2.5 Hz, 1H), 8.65 (t, J = 5.8 Hz, 1H), 8.59-8.53 (m, 2H), 8.21-8.13 (m, 2H), 7.88 (d, J = 9.3 Hz, 1H), 7.39 (q, J = 8.4 Hz, 4H), 6.95-6.83 (m, 3H), 6.62 (dd, J = 8.6, 3.9 Hz, 1H), 5.11 (d, J = 3.5 Hz, 1H), 4.73 (d, J = 5.7 Hz, 2H), 4.53 - 4.46 (m, 3H), 4.45-4.38 (m, 2H), 4.35 (d, J = 13.2 Hz, 3H), 4.20 (dd, J = 15.8, 5.4 Hz, 1H), 3.63 (dt, J = 17.3, 7.1 Hz, 2H), 3.25 (d, J = 8.7 Hz, 2H), 2.83 (t, J = 13.1 Hz, 2H), 2.71-2.64 (m, 1H), 2.43 (s, 3H), 2.04-1.98 (m, 1H), 1.92-1.84 (m, 1H), 1.77 (d, J = 11.3 Hz, 1H), 1.65 (d, J = 11.2 Hz, 1H), 1.57-1.51 (m, 1H), 0.93 (s, 9H).

### Example 24 Preparation of Compound H24

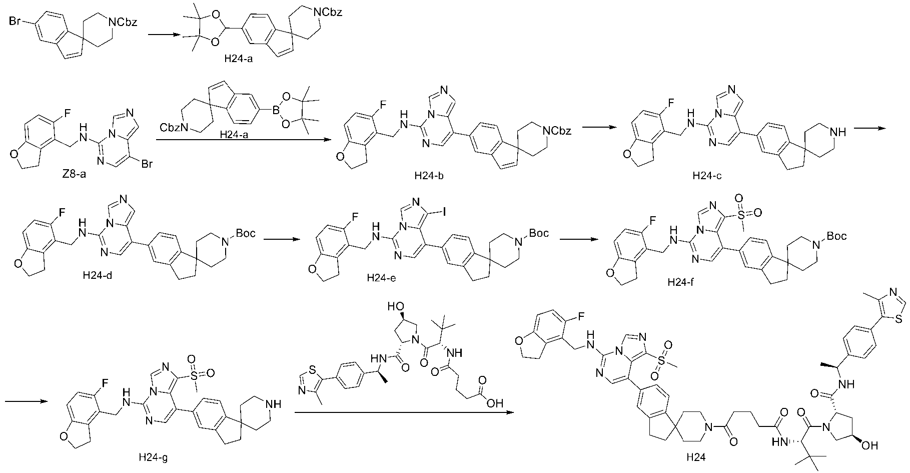

Step 1: 5-tetramethyl-1,3,2-dioxaborolan-2-yl)-1,3,2-dioxaborolane (1.28 g, 5.02 mmol), potassium acetate (739.19 mg, 7.53 mmol), and Pd(dppf)Cl₂ (183.71 mg, 0.25 mmol) were added to a solution of benzyl 5-bromospiro[indene-1,4'-piperidine]-1'-carboxylate (1 g, 2.51 mmol) in 1,4-dioxane (10 mL). The mixture was stirred at 90°C under a nitrogen atmosphere for 5 hours. After the reaction was completed, the reaction solution was filtered, and the filter cake was washed with ethyl acetate (20 mL×3). The filtrate was concentrated and purified by silica gel chromatography (EA:PE = 5%-10%) to obtain H24-a (1.25 g, yellow oil), yield: 99.9%. MS m/z (ESI): 446.4 [M+H]⁺.

Step 2: H24-a (386 mg, 0.87 mmol), K₂CO₃ (159 mg, 1.16 mmol), and Pd(PPh₃)₄ (133 mg, 0.12 mmol) were added to a solution of Z8-a (210 mg, 0.58 mmol) in 1,4-dioxane (6 mL) and water (1.5 mL). The mixture was stirred at 90°C under a nitrogen atmosphere for 2 hours. After the reaction was completed, the reaction solution was concentrated and purified by silica gel column chromatography (PE:EA = 5:1 to 1:8) to obtain H24-b (320 mg, red solid), yield: 91.2%. MS m/z (ESI): 602.2 [M+H]⁺.

Step 3: 10% palladium on carbon (7 mg, 0.08 mmol) and ammonia water (0.1 mL) were added to a solution of H24-b (270 mg, 0.45 mmol) in isopropanol (5 mL). The mixture was stirred at 60°C under a hydrogen atmosphere for 18 hours. After the reaction was completed, the reaction solution was filtered, and the filtrate was concentrated to obtain H24-c (197 mg, yellow solid), yield: 93.5%. MS m/z (ESI): 470.2 [M+H]⁺.

Step 4: At 0°C under a nitrogen atmosphere, triethylamine (127 mg, 1.26 mmol) and di-tert-butyl dicarbonate (137 mg, 0.63 mmol) were added to a solution of H24-c (197 mg, yellow solid) in DCM (5 mL). The mixture was stirred at 25°C under a nitrogen atmosphere for 2 hours. After the reaction was completed, the reaction solution was poured into water (20 mL) and extracted with ethyl acetate (30 mL×3). The organic phases were combined and washed with saturated brine (10 mL), dried over anhydrous sodium sulfate, concentrated, and purified by silica gel column chromatography (DCM:MeOH = 100:1 to 30:1) to obtain H24-d (199 mg, yellow solid), yield: 83.2%. MS m/z (ESI): 570.0 [M+H]⁺.

Step 5: At 0°C under a nitrogen atmosphere, NIS (81 mg, 0.47 mmol) was added to a solution of H24-d (179 mg, 0.31 mmol) in DMF (5 mL). The mixture was stirred at 0°C for 30 min, then stirred at 25°C under a nitrogen atmosphere for 30 min. After the reaction was completed, the reaction solution was poured into cold water (20 mL) and extracted with ethyl acetate (30 mL×3). The organic phases were combined and washed with saturated brine (10 mL), dried over anhydrous sodium sulfate, concentrated, and purified by silica gel chromatography (DCM:MeOH = 100:1 to 30:1) to obtain H24-e (146 mg, yellow solid), yield: 66.8%. MS m/z (ESI): 696.2 [M+H]⁺.

Step 6: Sodium methanesulfinate (52 mg, 0.52 mmol) and Cul (98 mg, 0.52 mmol) were added to a solution of H24-e (120 mg, 0.17 mmol) in DMSO (3 mL). The reaction solution was bubbled with nitrogen for 10 minutes, then reacted under microwave at 120°C under a nitrogen atmosphere for 20 min. The temperature was then cooled to 100°C, and microwave reaction was continued for 3 hours. After the reaction was completed, the reaction solution was poured into water (20 mL) and extracted with ethyl acetate (30 mL×3). The organic phases were combined and washed with saturated brine (10 mL), dried over anhydrous sodium sulfate, concentrated, and purified by silica gel column chromatography (DCM:MeOH = 100:1 to 30:1) to obtain H24-f (63 mg, yellow solid), yield: 56.4%. MS m/z (ESI): 648.4 [M+H]⁺.

Step 7: A solution of hydrogen chloride in ethyl acetate (2 ml, 4 mol/L) was added to a solution of H24-f (63 mg, 0.10 mmol) in EA (2 mL). The mixture was stirred at 25°C for 2 hours. After the reaction was completed, the reaction solution was concentrated to obtain H24-g (70 mg, yellow solid, crude), yield: 95.9%. MS m/z (ESI): 548.1 [M+H]⁺.

Step 8: 5-(((S)-1-((2S,4R)-4-hydroxy-2-(((S)-1-(4-(4-methylthiazol-5-yl)phenyl)ethyl)carbamoyl) pyrrolidin-1-yl)-3,3-dimethyl-1-oxobutan-2-yl)amino)-5-oxopentanoic acid (88 mg, 0.16 mmol), HATU (45 mg, 0.12 mmol), and DIPEA (61 mg, 0.48 mmol) were added to a solution of H24-g (58 mg, 0.08 mmol) in DMF (2 mL). The mixture was stirred at 25°C under a nitrogen atmosphere for 2 hours. After the reaction was completed, the reaction solution was filtered, and the filtrate was concentrated and purified by preparative HPLC (Gilson_306_1741, column: Waters-Xbridge-C18-10µm-19×250mm; mobile phase: water (containing 10 mM ammonium bicarbonate) and acetonitrile, gradient ratio: acetonitrile 39%-95%, flow rate: 25 mL/min) to obtain H24 (35 mg), yield: 40.1%. MS m/z (ESI): 1089.8 [M+H]⁺. ¹H NMR (400 MHz, DMSO-d₆) δ 8.98 (s, 1H), 8.84 (s, 1H), 8.59 (s, 1H), 8.36 (d, J = 7.6 Hz, 1H), 7.85 (d, J = 7.2 Hz, 1H), 7.45-7.40 (m, 2H), 7.40-7.35 (m, 3H), 7.25 (s, 1H), 7.21-7.15 (m, 2H), 6.99-6.90 (m, 1H), 6.74-6.67 (m, 1H), 5.09 (d, J = 3.6 Hz, 1H), 4.96-4.86 (m, 1H), 4.72 (s, 2H), 4.58-4.49 (m, 3H), 4.47-4.37 (m, 2H), 4.28 (s, 1H), 3.85 (d, J = 12.0 Hz, 1H), 3.62 (d, J = 2.4 Hz, 2H), 3.25-3.13 (m, 1H), 2.94 (s, 3H), 2.92-2.85 (m, 2H), 2.80-2.66 (m, 1H), 2.45 (s, 3H), 2.41-2.13 (m, 5H), 2.09 (t, J = 7.2 Hz, 2H), 2.05-1.97 (m, 1H), 1.84-1.44 (m, 8H), 1.37 (d, J = 6.8 Hz, 3H), 0.95 (s, 9H).

### Example 25 Preparation of Compound H25

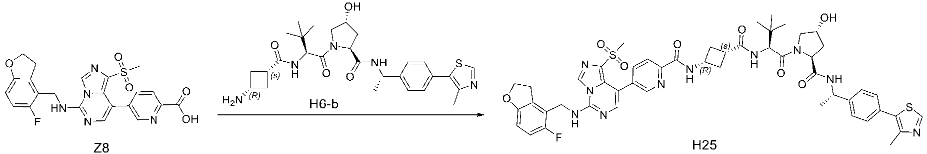

Step 1: H6-b (36 mg, 0.07 mmol), HATU (32 mg, 0.08 mmol), and DIPEA (36 mg, 0.28 mmol) were added to a solution of Intermediate Z8 (45 mg, 0.06 mmol) in DMF (1.5 mL). The mixture was stirred at 25°C under a nitrogen atmosphere for 2 hours. After the reaction was completed, the mixture was filtered, and the filtrate was purified by preparative HPLC (Gilson_306_1741, column: Waters-Xbridge-C18-10µm-19×250mm; mobile phase: water (containing 10 mM ammonium bicarbonate) and acetonitrile, gradient ratio: acetonitrile 35%-95%, flow rate: 25 mL/min) to obtain H25 (10 mg), yield: 17.8%. MS m/z (ESI): 1008.8 [M+H]⁺. ¹H NMR (400 MHz, DMSO-d₆) δ 8.98 (s, 1H), 8.95-8.88 (m, 2H), 8.81 (s, 1H), 8.63 (s, 1H), 8.39 (d, J = 8.0 Hz, 1H), 8.02-7.93 (m, 2H), 7.74 (d, J = 9.2 Hz, 1H), 7.50 (s, 1H), 7.46-7.34 (m, 4H), 6.99-6.89 (m, 1H), 6.74-6.68 (m, 1H), 5.11 (d, J = 3.6 Hz, 1H), 4.97-4.87 (m, 1H), 4.75 (s, 2H), 4.59-4.50 (m, 3H), 4.47-4.25 (m, 3H), 3.61 (s, 2H), 3.34 (s, 2H), 3.09 (s, 3H), 3.00-2.86 (m, 1H), 2.45 (s, 3H), 2.41-2.21 (m, 4H), 2.07-1.95 (m, 1H), 1.86-1.72 (m, 1H), 1.38 (d, J = 7.2 Hz, 3H), 0.95 (s, 9H).

### Example 26 Preparation of Compound H26

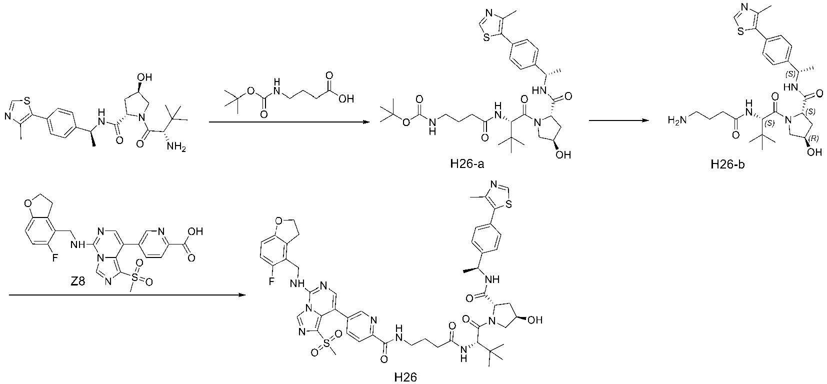

Step 1: (2S,4R)-1-((S)-2-Amino-3,3-dimethylbutanoyl)-4-hydroxy-N-((S)-1-(4-(4-methylthiazol-5-yl)phenyl)ethyl)pyrrolidine-2-carboxamide (670 mg, 0.45 mmol) and 4-((tert-butoxycarbonyl)amino)butanoic acid (92 mg, 0.45 mmol) were dissolved in DMF (5 mL). HATU (206 mg, 0.54 mmol) and DIPEA (0.75 mL, 4.52 mmol) were added, and the mixture was stirred at 25°C for 1 hour. After the reaction was completed, the reaction solution was poured into water (20 mL), and the aqueous phase was extracted with ethyl acetate (20 mL×3). The organic phases were combined and dried over anhydrous sodium sulfate, concentrated, and purified by silica gel column chromatography (DCM/EA = 80/20 to 50/50) to obtain H26-a (110 mg, light yellow gum), yield: 38.63%. MS m/z (ESI): 530.2 [M-100+H]⁺.

Step 2: A solution of hydrogen chloride in ethyl acetate (2 mL, 4 mol/L) was added to a solution of H26-a (110 mg, 0.17 mmol) in EA (2 mL). The mixture was stirred at 25°C for 2 hours. After the reaction was completed, the mixture was concentrated to obtain H26-b (110 mg, white solid), yield: 97.1%. MS m/z (ESI): 530.2 [M+H]⁺.

Step 3: H26-b (55 mg, 0.09 mmol), HATU (42 mg, 0.11 mmol), and DIPEA (38 mg, 0.30 mmol) were added to a solution of Intermediate Z8 (55 mg, 0.07 mmol) in DMF (1.5 mL). The mixture was stirred at 45°C for 1.5 hours. After the reaction was completed, the reaction solution was filtered, and the filtrate was purified by preparative HPLC (Gilson_306_1741, column: Waters-Xbridge-C18-10 µm-19×250 mm; mobile phase: water (containing 10 mM ammonium bicarbonate) and acetonitrile, gradient ratio: acetonitrile 35%-95%, flow rate: 25 mL/min) to obtain H26 (15 mg), yield: 20.4%. MS m/z (ESI): 994.8 [M+H]⁺. ¹H NMR (400 MHz, DMSO-d₆) δ 8.98 (s, 1H), 8.94-8.88 (m, 2H), 8.82 (s, 1H), 8.63 (d, J = 1.2 Hz, 1H), 8.39 (d, J = 7.6 Hz, 1H), 8.04-7.91 (m, 3H), 7.50 (s, 1H), 7.45-7.35 (m, 4H), 7.00-6.91 (m, 1H), 6.75-6.67 (m, 1H), 5.11 (d, J = 3.6 Hz, 1H), 4.95-4.88 (m, 1H), 4.75 (s, 2H), 4.59-4.49 (m, 3H), 4.43 (t, J = 8.0 Hz, 1H), 4.28 (s, 1H), 3.61 (s, 2H), 3.32-3.27 (m, 3H), 3.10 (s, 3H), 2.45 (s, 3H), 2.37-2.11 (m, 3H), 2.07-1.94 (m, 1H), 1.83-1.72 (m, 3H), 1.37 (d, J = 7.2 Hz, 3H), 0.95 (s, 9H).

### Example 27 Preparation of Compound H27

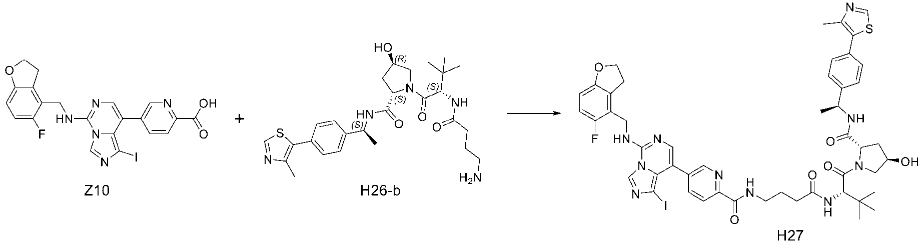

Step 1: Intermediate Z10 (48 mg, 0.09 mmol) and H26-b (96 mg, 0.18 mmol) were dissolved in DMF (5 mL). DIPEA (35 mg, 0.27 mmol) and HATU (51 mg, 0.14 mmol) were added, and the mixture was stirred at room temperature for 4 hours. After the reaction was completed, the reaction solution was poured into water, extracted with dichloromethane (30 mL×2). The organic phases were combined and dried over anhydrous sodium sulfate, concentrated, and purified by CombiFlash (MeOH/DCM = 0-15%), followed by preparative HPLC (preparative column: 21.2×250 mm C18 column; system: 10 mM NH₄HCO₃/H₂O/acetonitrile; wavelength: 254/214 nm; gradient: 0%-60% acetonitrile change) to obtain H27 (1.75 mg), yield: 1.84%. MS m/z (ESI): 522.2 [M/2+H]⁺. ¹H NMR (400 MHz, DMSO-d₆) δ 8.99-8.89 (m, 2H), 8.75 (s, 1H), 8.64 (d, J = 1.5 Hz, 1H), 8.50 (dd, J = 11.6, 5.1 Hz, 1H), 8.36 (d, J = 7.8 Hz, 1H), 8.08 (d, J = 8.5 Hz, 1H), 7.99 (dd, J = 8.0, 2.2 Hz, 1H), 7.92 (d, J = 9.2 Hz, 1H), 7.41 (d, J = 8.3 Hz, 2H), 7.38-7.30 (m, 2H), 7.20 (d, J = 5.8 Hz, 1H), 6.95-6.88 (m, 1H), 6.68 (dd, J = 8.7, 3.9 Hz, 1H), 5.08 (d, J = 3.6 Hz, 1H), 4.90 (dd, J = 14.0, 6.9 Hz, 2H), 4.70 (d, J = 4.3 Hz, 2H), 4.51 (dd, J = 14.7, 6.0 Hz, 3H), 4.41 (t, J = 8.1 Hz, 1H), 4.25 (s, 1H), 3.59 (s, 2H), 3.27 (d, J = 6.7 Hz, 3H), 2.42 (d, J = 10.1 Hz, 2H), 2.26 (dd, J = 19.1, 11.8 Hz, 2H), 2.16 (dt, J = 18.4, 9.1 Hz, 2H), 2.05-1.90 (m, 2H), 1.80-1.70 (m, 2H), 1.35 (d, J = 7.0 Hz, 2H), 0.93 (s, 9H).

### Example 28 Preparation of Compound H28

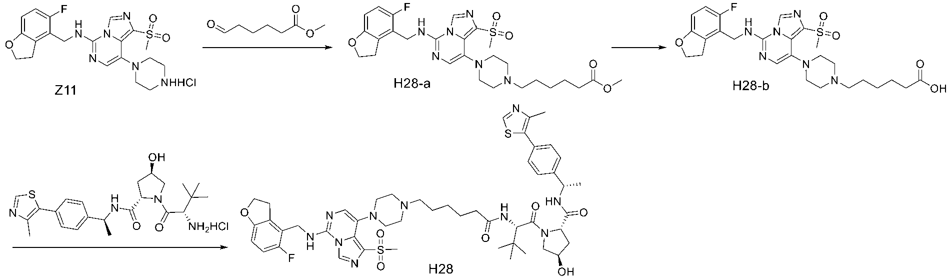

Step 1: Intermediate Z11 (18 mg, 0.04 mmol) was dissolved in DCE (5 mL). Triethylamine (4.08 mg, 0.04 mmol) was added, and the mixture was stirred at 25°C for 10 minutes. NaBH(OAc)₃ (5.81 mg, 0.04 mmol) was added, and the mixture was stirred at 25°C for 1 hour. After the reaction was completed, the reaction was quenched with saturated sodium bicarbonate solution, extracted twice with dichloromethane. The organic phases were combined and washed with saturated brine, dried over anhydrous sodium sulfate, and concentrated to obtain H28-a (20 mg, white solid), yield: 86.3%. MS m/z (ESI): 575.4 [M+H]⁺.

Step 2: H28-a (20 mg, 0.03 mmol) was dissolved in a mixed solution of THF (2 mL), water (1 mL), and methanol (2 mL). Lithium hydroxide monohydrate (4.38 mg, 0.10 mmol) was added, and the mixture was stirred at 20-30°C for 18 hours. After the reaction was completed, the reaction solution was concentrated to obtain H28-b (16 mg, white solid, crude). MS m/z (ESI): 559.6 [M-H]⁻.

Step 3: H28-b (16 mg, 0.03 mmol), DIPEA (14.75 mg, 0.11 mmol), and HATU (22.81 mg, 0.06 mmol) were dissolved in DMF (3 mL). (2S,4R)-1-((S)-2-Amino-3,3-dimethylbutanoyl)-4-hydroxy-N-((S)-1-(4-(4-methylthiazol-5-yl)phenyl)ethyl)pyrrolidine-2-carboxamide hydrochloride was added, and the mixture was stirred at 25°C for 18 hours. After the reaction was completed, the reaction solution was purified by HPLC (Waters-SunFire-C18-10µm-19×250mm (mobile phase: 5%-95% (v/v) CH₃CN and aqueous solution containing 0.1% FA)) to obtain a formate salt of H28 (1.5 mg), yield: 5.32%. MS m/z (ESI): 987.9 [M+H]⁺. ¹H NMR (400 MHz, DMSO-d₆) δ 8.98 (s, 1H), 8.71 (s, 1H), 8.37 (d, J = 7.8 Hz, 2H), 8.28 (s, FA, 2H), 7.80 (d, J = 9.4 Hz, 1H), 7.50 (s, 1H), 7.41 (dd, J = 22.7, 8.3 Hz, 4H), 6.95-6.85 (m, 1H), 6.69 (dd, J = 8.6, 3.8 Hz, 1H), 4.95-4.85 (m, 1H), 4.65 (d, J = 4.7 Hz, 2H), 4.53 (dd, J = 14.3, 5.5 Hz, 3H), 4.41 (d, J = 8.0 Hz, 1H), 4.28 (s, 1H), 3.60 (s, 2H), 3.43 (s, 3H), 3.28 (m, 6H), 2.45-2.44 (m, 4H), 2.34-2.24 (m, 5H), 2.13 (m, 3H), 1.81 (dd, J = 8.4, 4.6 Hz, 1H), 1.45 (m, 4H), 1.37 (d, J = 7.0 Hz, 3H), 1.31-1.21 (m, 3H), 0.94 (s, 9H).

### Example 29 Preparation of Compound H29

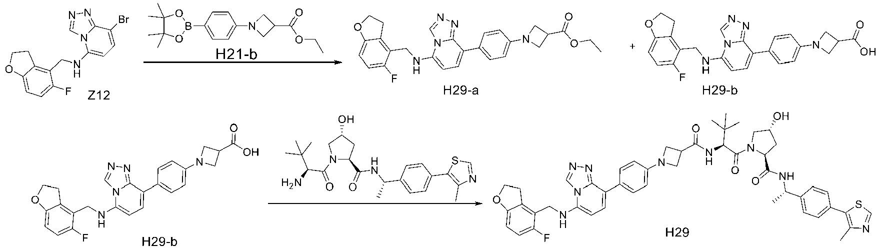

Step 1: Intermediate Z12 (360 mg, 0.99 mmol) and H21-b (493 mg, 1.49 mmol) were dissolved in 1,4-dioxane (25 mL) and water (4 mL). Pd(PPh₃)₄ (172 mg, 0.15 mmol) and K₂CO₃ (274 mg, 1.98 mmol) were added, and the mixture was stirred at 105°C for 8 hours. After the reaction was completed, the reaction solution was concentrated and purified by CombiFlash (0-30% MeOH/DCM) to obtain H29-a (230 mg, yellow solid), yield: 47.59%, MS m/z (ESI): 488.2 [M+H]⁺, and H29-b (160 mg), yield: 35.13%, MS m/z (ESI): 460.2 [M+H]⁺.

Step 2: H29-b (100 mg, 0.22 mmol) and DIPEA (85 mg, 0.65 mmol) were dissolved in DMF (8 mL). HATU (123 mg, 0.33 mmol) was added, followed by (2S,4R)-1-((S)-2-amino-3,3-dimethylbutanoyl)-4-hydroxy-N-((S)-1-(4-(4-methylthiazol-5-yl)phenyl)et hyl)pyrrolidine-2-carboxamide (126 mg, 0.28 mmol). The mixture was stirred at room temperature for 2 hours. After the reaction was completed, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to obtain H29 (21.65 mg), yield: 10.82%. MS m/z (ESI): 443.8 [M/2+H]⁺. ¹H NMR (400 MHz, DMSO-d₆) δ 9.42 (s, 1H), 8.98 (s, 1H), 8.39 (d, J = 7.7 Hz, 1H), 8.13-7.95 (m, 3H), 7.53 - 7.34 (m, 6H), 7.02-6.94 (m, 1H), 6.73 (dd, J = 8.7, 3.9 Hz, 1H), 6.53 (t, J = 9.5 Hz, 2H), 6.00 (d, J = 7.9 Hz, 1H), 5.10 (d, J = 3.5 Hz, 1H), 4.94 (dd, J = 13.5, 6.1 Hz, 1H), 4.60-4.48 (m, 5H), 4.44 (t, J = 8.2 Hz, 1H), 4.28 (s, 1H), 4.01 (dt, J = 25.2, 7.5 Hz, 2H), 3.90-3.78 (m, 2H), 3.73 (dd, J = 13.8, 7.2 Hz, 1H), 3.62 (s, 2H), 3.26 (t, J = 8.7 Hz, 2H), 2.46 (s, 3H), 2.02 (dd, J = 14.9, 5.4 Hz, 1H), 1.83-1.75 (m, 1H), 1.37 (t, J = 9.6 Hz, 3H), 0.95 (d, J = 10.4 Hz, 9H).

### Example 30 Preparation of Compound H30

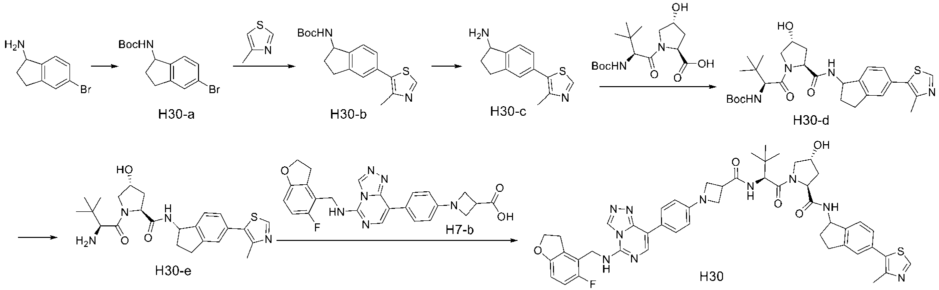

Step 1: 5-Bromo-2,3-dihydro-1H-inden-1-amine (1 g, 4.72 mmol) was dissolved in THF (30 mL). Triethylamine (716 mg, 7.07 mmol) was added, and di-tert-butyl dicarbonate (1.23 g, 5.66 mmol) was added dropwise at 0°C. The mixture was stirred at room temperature overnight. After the reaction was completed, the reaction solution was concentrated and purified by CombiFlash (0-40% EA/PE) to obtain H30-a (1.33 g, yellow solid), yield: 90.35%. MS m/z (ESI): 256.0 [M+H-56]⁺.

Step 2: H30-a (600 mg, 1.92 mmol) and 4-methylthiazole (381 mg, 3.84 mmol) were dissolved in DMAC (10 mL). Pd(OAc)₂ (43 mg, 0.19 mmol) and KOAc (377 mg, 3.84 mmol) were added, and the mixture was stirred at 110°C overnight under argon protection. After the reaction was completed, the reaction solution was poured into water, extracted with EA (30 mL×2). The organic phases were combined and dried over anhydrous sodium sulfate, concentrated, and purified by silica gel column chromatography (0-60% EA/PE) to obtain H30-b (430 mg, yellow solid), yield: 67.71%. MS m/z (ESI): 331.1 [M+H]⁺.

Step 3: H30-b (430 mg, 1.30 mmol) was dissolved in DCM (15 mL). A solution of hydrogen chloride in ethyl acetate (4 mol/L, 24.00 mmol, 6 mL) was added, and the mixture was stirred at room temperature for 4 hours. After the reaction was completed, the reaction solution was concentrated to obtain H30-c (299 mg), yield: 99.76%, which was used directly in the next step. MS m/z (ESI): 231.1 [M+H]⁺.

Step 4: (2S,4R)-1-((S)-2-((tert-Butoxycarbonyl)amino)-3,3-dimethylbutanoyl)-4-hydroxypyrrolidine-2-carboxylic acid (447 mg, 1.30 mmol) and DIPEA (503 mg, 3.89 mmol) were dissolved in DMF (10 mL). HATU (735 mg, 1.95 mmol) was added, followed by H30-c (299 mg, 1.30 mmol). The mixture was stirred at room temperature for 2 hours. After the reaction was completed, the reaction solution was concentrated and purified by Combiflash (0-10% MeOH/DCM) to obtain H30-d (700 mg, yellow solid), yield: 96.86%. MS m/z (ESI): 557.3 [M+H]⁺.

Step 5: H30-d (700 mg, 1.26 mmol) was dissolved in DCM (15 mL). A solution of hydrogen chloride in ethyl acetate (4 mol/L, 12.57 mmol, 3.14 mL) was added, and the mixture was stirred at room temperature for 2 hours. After the reaction was completed, the reaction solution was concentrated to obtain H30-e (570 mg, crude), yield: 99.28%, which was used directly in the next step. MS m/z (ESI): 457.3 [M+H]⁺.

Step 6: H7-b (40 mg, 0.087 mmol) and DIPEA (34 mg, 0.26 mmol) were dissolved in DMF (5 mL). HATU (49 mg, 0.13 mmol) was added, followed by H30-e (52 mg, 0.11 mmol). The mixture was stirred at room temperature for 2 hours. After the reaction was completed, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to obtain H30 (28.73 mg), yield: 36.10%. MS m/z (ESI): 450.3 [M/2+H]⁺. ¹H NMR (400 MHz, DMSO-*d₆*) δ 9.45 (s, 1H), 8.96 (d, J = 19.5 Hz, 1H), 8.49 (s, 1H), 8.38 (dd, J = 36.4, 8.2 Hz, 1H), 7.87-7.80 (m, 3H), 7.45-7.15 (m, 4H), 6.95 (t, J = 9.5 Hz, 1H), 6.70 (d, J = 8.6 Hz, 3H), 6.17 (dd, J = 11.5, 5.8 Hz, 1H), 5.85 (s, 1H), 5.54-5.49 (m, 1H), 5.34-5.23 (m, 1H), 5.18 (s, 1H), 4.74-4.62 (m, 3H), 4.54 (t, J = 8.7 Hz, 2H), 4.39 (dd, J = 11.5, 7.4 Hz, 2H), 4.04-3.89 (m, 2H), 3.78-3.63 (m, 2H), 3.30 (t, J = 8.9 Hz, 2H), 2.96 (dd, J = 23.2, 7.4 Hz, 1H), 2.85 (dd, J = 15.9, 8.0 Hz, 1H), 2.42 (d, J = 12.9 Hz, 4H), 2.09-2.01 (m, 1H), 2.00-1.90 (m, 1H), 1.90-1.78 (m, 1H), 0.98 (t, J = 17.8 Hz, 9H).

### Example 31 Preparation of Compound H31

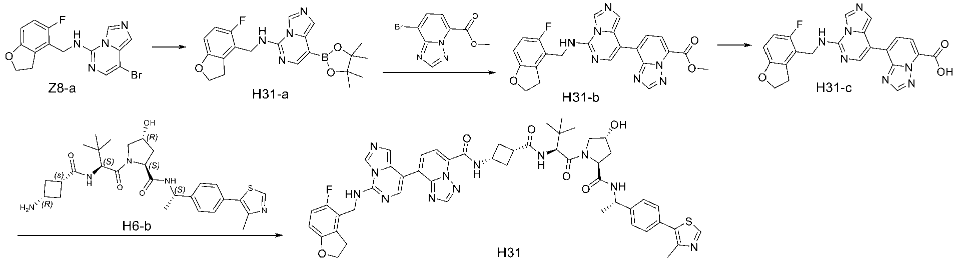

Step 1: At 25°C under a nitrogen atmosphere, Z8-a (1.50 g, 4.13 mmol), KOAc (1.22 g, 12.39 mmol), and bis(pinacolato)diboron (2.10 g, 8.26 mmol) were dissolved in 1,4-dioxane (50 mL). Pd(PPh₃)₄ (0.95 g, 0.83 mmol) was added, and the mixture was stirred at 100°C for 18 hours. After the reaction was completed, the reaction solution was concentrated and purified by silica gel column chromatography (DCM:MeOH = 1:0 to 10:1) to obtain H31-a (400.0 mg, yellow solid), yield: 23.61%. MS m/z (ESI): 411.2 [M+H]⁺.

Step 2: At 25°C under a nitrogen atmosphere, H31-a (370 mg, 0.90 mmol), methyl 8-bromo-[1,2,4]triazolo[1,5-a]pyridine-5-carboxylate (461.9 mg, 1.80 mmol), and K₂CO₃ (373.9 mg, 2.71 mmol) were dissolved in DME (20 mL) and water (2 mL). Pd(PPh₃)₄ (104.2 mg, 0.09 mmol) was added, and the mixture was stirred at 80°C for 2 hours. After the reaction was completed, the reaction solution was concentrated and purified by silica gel column chromatography (DCM:MeOH = 1:0 to 10:1) to obtain H31-b (16 mg, yellow solid), yield: 38.61%. MS m/z (ESI): 460.2 [M+H]⁺.

Step 3: At 25°C under a nitrogen atmosphere, H31-b (53 mg, 0.12 mmol) and lithium hydroxide monohydrate (24.2 mg, 0.58 mmol) were dissolved in MeOH (2 mL) and water (0.5 mL). The mixture was stirred at 25°C for 2 hours. After the reaction was completed, the reaction solution was concentrated to obtain H31-c (crude), which was used directly in the next step. MS m/z (ESI): 446.2 [M+H]⁺.

Step 4: H31-c (51.0 mg, 0.12 mmol), H6-b (74.4 mg, 0.14 mmol), and HATU (65.3 mg, 0.17 mmol) were dissolved in DMF (5 mL). DIPEA (74.0 mg, 0.57 mmol) was added, and the mixture was reacted at 25°C under a nitrogen atmosphere for 2 hours. After the reaction was completed, the reaction solution was concentrated and purified by preparative HPLC (Gilson_306_1741, column: Waters-SunFire-C18-10µm-19×250mm; mobile phase: water (containing 0.1 wt% formic acid) and acetonitrile, gradient ratio: acetonitrile 47%-95%, flow rate: 25 mL/min) to obtain H31 (14.5 mg), yield: 13.07%. MS m/z (ESI): 969.4 [M+H]⁺. ¹H NMR (500 MHz, DMSO-*d₆*) δ 9.99 (d, J = 7.6 Hz, 1H), 8.98 (s, 1H), 8.80 (s, 1H), 8.76 (s, 1H), 8.69 (t, J = 5.2 Hz, 1H), 8.40 (d, J = 8.0 Hz, 1H), 8.15 (d, J = 7.6 Hz, 1H), 7.93-7.89 (m, 2H), 7.57 (s, 1H), 7.44 (d, J = 8.0 Hz, 2H), 7.38 (d, J = 8.4 Hz, 2H), 6.95 (t, J = 9.2 Hz, 1H), 6.70 (dd, J = 8.8, 4.0 Hz, 1H), 5.12 (d, J = 3.6 Hz, 1H), 4.92 (t, J = 7.2 Hz, 1H), 4.79 (d, J = 5.2 Hz, 2H), 4.55 (t, J = 8.8 Hz, 3H), 4.48-4.41 (m, 2H), 4.29 (s, 1H), 3.62 (s, 2H), 3.35-3.33 (m, 4H), 3.08-3.03 (m, 1H), 2.45 (s, 3H), 2.40-2.34 (m, 1H), 2.26-2.13 (m, 2H), 2.04-2.00 (m, 1H), 1.79 (s, 1H), 1.38 (d, J = 7.2 Hz, 3H), 0.94 (s, 9H).

### Example 32 Preparation of Compound H32

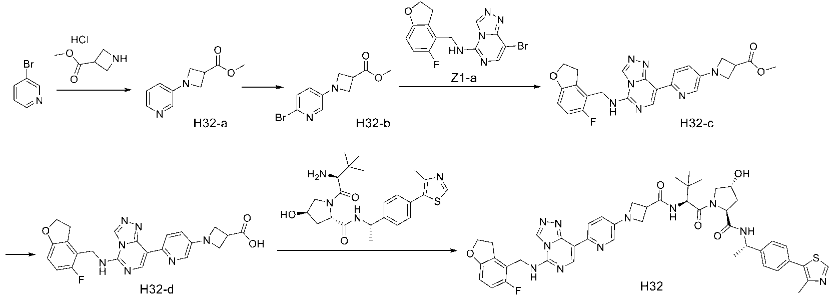

Step 1: Xantphos (0.76 g, 1.32 mmol), Pd₂(dba)₃ (0.60 g, 0.66 mmol), and Cs₂CO₃ (12.90 g, 39.58 mmol) were added to a solution of methyl azetidine-3-carboxylate hydrochloride (2.00 g, 13.19 mmol) and 3-bromopyridine (2.08 g, 13.19 mmol) in 1,4-dioxane (20 mL). The mixture was stirred at 100°C under a nitrogen atmosphere for 18 hours. After the reaction was completed, the reaction solution was filtered and the filter cake was washed with dichloromethane (20 mL×3). The filtrate was concentrated and purified by silica gel column chromatography (PE:EA = 10:1 to 1:1) to obtain H32-a (400 mg, yellow oil), yield: 15.77%. MS m/z (ESI): 193.2 [M+H]⁺.

Step 2: NBS (250.01 mg, 1.40 mmol) was added to a solution of H32-a (300 mg, 1.56 mmol) in acetonitrile (5 mL). The mixture was stirred at 25°C under a nitrogen atmosphere for 1 hour. After the reaction was completed, the reaction solution was concentrated and purified by silica gel column chromatography (PE:EA = 10:1 to 3:1) to obtain H32-b (300 mg, yellow oil), yield: 70.90%. MS m/z (ESI): 273.0 [M+H]⁺.

Step 3: Pd(OAc)₂ (29.96 mg, 0.13 mmol), n-butyldi(1-adamantyl)phosphine (95.70 mg, 0.27 mmol), B₂Pin₂ (508.33 mg, 2.00 mmol), and K₂CO₃ (461.07 mg, 3.34 mmol) were added to a solution of Z1-a (243 mg, 0.67 mmol) and H32-b (361.80 mg, 1.33 mmol) in water (0.5 mL) and DME (5 mL). The mixture was stirred at 70°C under a nitrogen atmosphere for 18 hours. After the reaction was completed, the reaction solution was filtered and the filter cake was washed with ethyl acetate (10 mL×). The filtrate was concentrated and purified by silica gel column chromatography (PE:EA = 10:1 to 1:1) to obtain H32-c (268 mg, yellow oil), yield: 84.47%. MS m/z (ESI): 476.2 [M+H]⁺.

Step 4: H32-c (218 mg, 0.46 mmol) and NaOH (36.68 mg, 0.92 mmol) were dissolved in water (2 mL) and methanol (2 mL). The mixture was stirred at 25°C under a nitrogen atmosphere for 18 hours. After the reaction was completed, the reaction solution was concentrated to obtain H32-d (160 mg, yellow oil, crude), yield: 75.63%. No further purification was needed. MS m/z (ESI): 462.2 [M+H]⁺.

Step 5: H32-d (140 mg, 0.30 mmol), (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (202.32 mg, 0.46 mmol), DIPEA (201.09 µL, 1.21 mmol), HOBT (61.49 mg, 0.46 mmol), and EDCI (87.24 mg, 0.46 mmol) were added to DMF (3 mL). The mixture was stirred at 25°C for 18 hours. After the reaction was completed, the reaction solution was extracted with ethyl acetate (10 mL×3). The organic phases were combined and washed with saturated brine (10 mL), dried over anhydrous sodium sulfate, concentrated, and purified by preparative HPLC (Phenomenex Gemini 150 mm × 25 mm × 10 µm column (eluent: 30% to 60% (v/v) acetonitrile and water containing 0.025% formic acid)) to obtain H32 (5.1 mg), yield: 1.9%. MS m/z (ESI): 888.1 [M+H]⁺. ¹H NMR (400 MHz, DMSO-*d₆*) δ 9.50 (s, 1H), 8.98 (s, 1H), 8.67-8.69 (m, 1H), 8.61-8.62 (m, 1H), 8.47 (s, 1H), 8.38-8.40 (m, 1H), 8.14 (d, J = 7.2 Hz, 1H), 7.92-7.93 (m, 1H), 7.42-7.44 (m, 2H), 7.37-6.39 (m, 2H), 6.93-6.99 (m, 2H), 6.69-6.71 (m, 1H), 5.10 (d, J = 2.8 Hz, 1H), 4.91-4.95 (m, 1H), 4.72 (d, J = 4.0 Hz, 1H), 4.52-4.57 (m, 3H), 4.42-4.45 (m, 1H), 4.28-4.29 (m, 1H), 4.09-4.13 (m, 1H), 4.03-4.07 (m, 1H), 3.92-3.96 (m, 1H), 3.87-3.90 (m, 1H), 3.75-3.79 (m, 1H), 3.61-3.62 (m, 1H), 3.29-3.30 (m, 2H), 2.45 (s, 3H), 1.99-2.03 (m, 3H), 1.76-1.81 (m, 1H), 1.37 (d, J = 5.6 Hz, 3H), 0.94 (s, 9H).

### Example 33 to Example 37

Compounds H33 to H37 were prepared referring to similar methods in the examples of the specification.

| | |
|---|---|
| 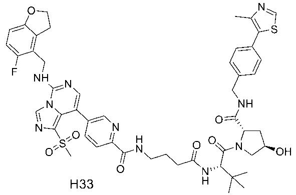 | MS m/z (ESI):485[M/2+H]⁺. |
| 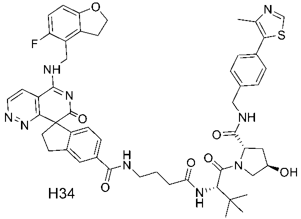 | MS m/z (ESI):485[M/2+H]⁺. |
| | ¹H NMR (400 MHz, DMSO-*d₆*) δ 11.03 (s, 1H), 8.97 (s, 1H), 8.34 (dd, J = 19.6, 6.4 Hz, 2H), 8.13 (d, J = 5.8 Hz, 1H), 7.87 (d, J = 8.7 Hz, 1H), 7.79 (d, J = 21.0 Hz, 1H), 7.54 (d, J = 7.9 Hz, 1H), 7.39 (dd, J = 23.2, 8.3 Hz, 4H), 6.89 (dd, J = 37.1, 8.5 Hz, 2H), 6.69 (s, 2H), 5.09 (d, J = 3.1 Hz, 1H), 4.95-4.33 (m, 10H), 3.59 (s, 2H), 2.99 (dd, J = 37.9, 7.0 Hz, 2H), 2.44 (s, 3H), 2.34-2.15 (m, 3H), 2.01-1.92 (m, 2H), 1.73 (d, J = 27.0 Hz, 3H), 1.38 (t, J = 17.6 Hz, 3H), 0.92 (s, 9H). |
| 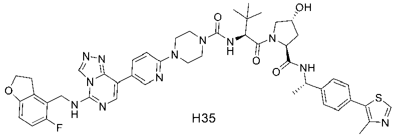 | MS m/z (ESI):459.3 [M/2+H]⁺. |
| | ¹H NMR (400 MHz, DMSO-*d₆*) δ 8.99 (s, 1H), 8.82 (d, *J =* 2.5 Hz, 1H), 8.71 (t, *J =* 5.8 Hz, 1H), 8.61 (s, 1H), 8.41 (d, *J* = 7.9 Hz, 1H), 8.26-8.21 (m, 2H), 7.41 (dd, *J =* 23.2, 8.3 Hz, 4H), 6.96 (d, *J =* 9.0 Hz, 1H), 6.92-6.86 (m, 1H), 6.65 (dd, *J =* 8.6, 3.9 Hz, 1H), 6.01 (d, *J =* 9.3 Hz, 1H), 5.11 (d, *J* = 3.6 Hz, 1H), 4.97-4.88 (m, 1H), 4.75 (d, *J* = 5.4 Hz, 2H), 4.54-4.41 (m, 4H), 4.30 (s, 1H), 3.70-3.61 (m, 2H), 3.55 (d, *J =* 5.6 Hz, 4H), 3.48 (d, *J =* 5.2 Hz, 4H), 3.29 (t, *J =* 8.7 Hz, 2H), 2.46 (s, 3H), 2.06-1.98 (m, 1H), 1.84-1.77 (m, 1H), 1.38 (d, *J* = 7.0 Hz, 3H), 0.97 (s, 9H). |
| 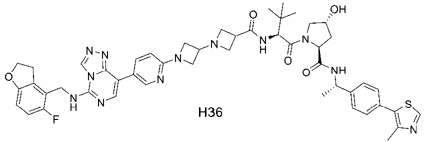 | MS m/z (ESI): 943.3[ [M+H]⁺ . |
| | ¹H NMR (400 MHz, DMSO-*d₆*) 9.46 (s, 1H), 8.98 (s, 1H), 8.77 (d, J = 2.5 Hz, 1H), 8.59 (s, 1H), 8.38 (d, J = 8.1 Hz, 1H), 8.21 (dd, J = 8.7, 2.4 Hz, 1H), 7.93 (d, J = 6.2 Hz, 2H), 7.43 (d, J = 8.3 Hz, 2H), 7.37 (d, J = 8.3 Hz, 2H), 7.01-6.90 (m, 1H), 6.70 (dd, J = 8.6, 3.8 Hz, 1H), 6.48 (d, J = 8.5 Hz, 1H), 5.11 (d, J = 3.5 Hz, 1H), 4.97-4.86 (m, 1H), 4.71 (s, 2H), 4.54 (t, J = 8.7 Hz, 3H), 4.42 (t, J = 8.2 Hz, 1H), 4.29 (s, 1H), 3.98 (t, J = 7.7 Hz, 2H), 3.76 (dd, J = 8.6, 4.3 Hz, 2H), 3.61 (s, 2H), 3.54 (s, 1H), 3.39 (dd, J = 19.5, 5.6 Hz, 3H), 3.31-3.23 (m, 4H), 3.19 (s, 1H), 2.45 (s, 3H), 2.00 (d, J = 10.0 Hz, 1H), 1.37 (d, J = 7.0 Hz, 3H), 0.92 (d, J = 7.1 Hz, 9H). |
| 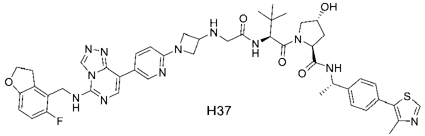 | MS m/z (ESI): 917.3[ [M+H]⁺. |
| | ¹H NMR (400 MHz, DMSO-*d₆*) δ 9.46 (s, 1H), 8.98 (s, 1H), 8.77 (d, J = 1.8 Hz, 1H), 8.60 (s, 1H), 8.43 (d, J = 7.9 Hz, 1H), 8.21 (dd, J = 8.7, 2.4 Hz, 1H), 7.96-7.86 (m, 2H), 7.44 (d, J = 8.3 Hz, 2H), 7.37 (d, J = 8.2 Hz, 2H), 6.94 (d, J = 9.8 Hz, 1H), 6.70 (dd, J = 8.6, 3.9 Hz, 1H), 6.50 (d, J = 8.6 Hz, 1H), 5.14 (d, J = 2.9 Hz, 1H), 4.91 (s, 1H), 4.71 (s, 2H), 4.54 (t, J = 8.7 Hz, 3H), 4.45 (s, 1H), 4.29 (s, 1H), 4.13 (d, J = 5.1 Hz, 2H), 3.69 (d, J = 5.3 Hz, 3H), 3.60 (d, J = 9.7 Hz, 2H), 3.29 (d, J = 9.2 Hz, 2H), 3.17 (s, 2H), 2.45 (d, J = 4.6 Hz, 4H), 2.05 (s, 1H), 1.78 (s, 1H), 1.37 (d, J = 6.9 Hz, 3H), 0.95 (s, 9H). |

### Example 38 Preparation of Compound H38

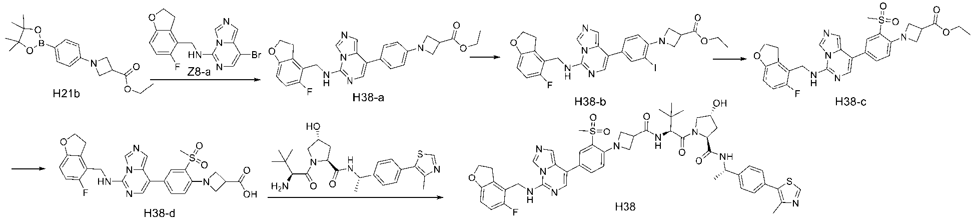

Step 1: Z8-a (1 g, 2.75 mmol) and H21-b (1.36 g, 4.12 mmol) were dissolved in a mixed solution of 1,4-dioxane (20 mL) and water (2 mL). Pd(dppf)Cl₂ (200.92 mg, 274.60 µmol) and NaHCO₃ (692.04 mg, 8.24 mmol) were added. After purging with nitrogen, the mixture was stirred at 100°C for 24 hours. After the reaction was completed, the reaction solution was concentrated and purified by combiflash (EA:PE = 0-80%) to obtain H38-a (360 mg, brown solid), yield: 26.84%.

Step 2: H38-a (700 mg, 1.44 mmol) was dissolved in THF (10 mL) and TFA (1 mL). The temperature was cooled to 0°C, NIS (226.13 mg, 1.01 mmol) was added, and the mixture was stirred at 0°C for 0.5 hour. Saturated aqueous sodium bicarbonate solution was added, and the mixture was extracted with ethyl acetate (70 mL×3). The organic phases were combined and dried over anhydrous sodium sulfate, concentrated, and purified by CombiFlash (12 g, 0-50% EA/PE) to obtain intermediate H38-b (88 mg, light yellow solid), yield: 9.99%. MS m/z (ESI): 614.2 [M+H]⁺.

Step 3: H38-b (260 mg, 423.85 µmol) was dissolved in DMSO (3 mL). Sodium methanesulfonate (150.16 mg, 1.27 mmol) and CuI (242.17 mg, 1.27 mmol) were then added. The reaction was stirred under microwave at 120°C for 30 minutes. After the reaction was completed, the reaction solution was cooled to room temperature, quenched with water, and extracted with dichloromethane (50 mL×3). The organic phases were combined and washed with saturated brine (50 mL), dried over anhydrous sodium sulfate, concentrated, and purified by CombiFlash (12 g, 0-100% EA/PE) to obtain H38-c (180 mg, light yellow solid). MS m/z (ESI): 566.2 [M+H]⁺.

Step 4: H38-c (180 mg, 318.24 µmol) was dissolved in THF (10 mL) and water (2.5 mL). LiOH (152.43 mg, 6.36 mmol) was then added, and the mixture was stirred at room temperature for 5 hours. After the reaction was completed, the reaction solution was concentrated, neutralized to neutrality with dilute hydrochloric acid, and lyophilized to obtain H38-d (171 mg, light yellow solid). MS m/z (ESI): 538.2 [M+H]⁺.

Step 5: H38-d (170 mg, 316.24 µmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (281.20 mg, 632.49 µmol) were dissolved in DMF (6 mL). DIPEA (817.44 mg, 6.32 mmol, 1.10 mL) and HATU (238.62 mg, 632.49 µmol) were then added, and the mixture was stirred at room temperature for 1 hour. After the reaction was completed, the reaction solution was concentrated and preliminarily purified by CombiFlash (24 g, 0-12% MeOH/DCM), then further purified by preparative HPLC (preparative column: 21.2×250 mm C18 column; system: 10 mM NH₄HCO₃ H₂O/acetonitrile; wavelength: 254/214 nm; gradient: 0%-70% acetonitrile change) to obtain H38 (47.79 mg), yield: 15.02%. MS m/z (ESI): 482.8 [M/2+H]⁺. ¹H NMR (400 MHz, DMSO-*d₆*) δ 8.97 (s, 1H), 8.81 (s, 1H), 8.56 (s, 1H), 8.39 (d, J = 7.4 Hz, 1H), 8.09 (d, J = 8.9 Hz, 1H), 7.42 (d, J = 8.2 Hz, 2H), 7.39-7.33 (m, 2H), 7.28 (s, 1H), 7.18 (d, J = 8.3 Hz, 1H), 7.11 (d, J = 8.5 Hz, 1H), 6.96-6.88 (m, 1H), 6.68 (dd, J = 8.7, 3.9 Hz, 1H), 6.59 (d, J = 8.4 Hz, 1H), 6.40 (d, J = 8.3 Hz, 1H), 5.10 (s, 1H), 4.95-4.85 (m, 1H), 4.70 (d, J = 4.0 Hz, 2H), 4.53 (dd, J = 14.9, 6.2 Hz, 3H), 4.42 (t, J = 8.1 Hz, 1H), 4.26 (s, 1H), 3.99 (t, J = 7.4 Hz, 1H), 3.92 (d, J = 6.9 Hz, 1H), 3.81 (t, J = 6.3 Hz, 1H), 3.79-3.73 (m, 1H), 3.71 (d, J = 6.5 Hz, 1H), 3.61 (s, 2H), 3.28 (d, J = 8.9 Hz, 2H), 2.95-2.86 (m, 3H), 2.42 (d, J = 10.6 Hz, 3H), 2.00 (s, 1H), 1.77 (s, 1H), 1.42-1.27 (m, 3H), 0.93 (d, J = 8.5 Hz, 9H).

### Example 39 to Example 42

Compounds H39 to H41 were prepared using H7-b as starting material and referring to the method of Example 30. Compound H42 was prepared using H86-b as starting material and referring to the method of Example 30.

| | |
|---|---|
| 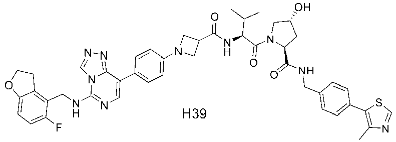 | MS m/z (ESI):430.3[M/2+H]⁺. |
| | ¹H NMR (400 MHz, DMSO-*d₆*) δ 9.44 (s, 1H), 8.96 (s, 1H), 8.51-8.42 (m, 2H), 8.20 (d, J = 8.7 Hz, 1H), 7.97-7.84 (m, 3H), 7.38 (q, J = 8.3 Hz, 4H), 7.00-6.88 (m, 1H), 6.68 (dd, J = 8.6, 3.9 Hz, 1H), 6.51 (d, J = 8.6 Hz, 2H), 5.08 (d, J = 3.7 Hz, 1H), 4.69 (d, J = 5.0 Hz, 2H), 4.52 (t, J = 8.7 Hz, 2H), 4.44-4.23 (m, 5H), 4.00 (dt, J = 15.4, 7.7 Hz, 2H), 3.83 (dt, J = 12.2, 6.6 Hz, 2H), 3.63 (d, J = 13.7 Hz, 3H), 2.43 (s, 3H), 2.10-1.80 (m, 4H), 1.22 (s, 1H), 0.86 (dd, J = 16.8, 6.7 Hz, 6H). |
| 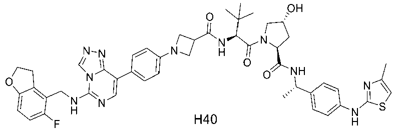 | MS m/z (ESI):451.8 [M/2+H]⁺. |
| | ¹H NMR (400 MHz, DMSO-*d₆*) δ 10.02 (s, 1H), 9.45 (s, 1H), 8.49 (s, 1H), 8.28 (d, *J* = 7.8 Hz, 1H), 7.85 (d, *J =* 8.4 Hz, 3H), 7.74 (d, *J* = 9.1 Hz, 1H), 7.51 (d, *J* = 8.5 Hz, 2H), 7.19 (d, *J* = 8.5 Hz, 2H), 6.99-6.90 (m, 1H), 6.74-6.66 (m, 3H), 6.41 (s, 1H), 6.18 (d, *J* = 5.5 Hz, 1H), 5.86 (s, 1H), 5.52 (s, 1H), 5.14 (d, *J* = 3.3 Hz, 1H), 4.81 (dd, *J* = 14.0, 7.0 Hz, 1H), 4.70 (d, *J* = 4.5 Hz, 2H), 4.62 (d, *J* = 9.2 Hz, 1H), 4.54 (t, *J =* 8.7 Hz, 2H), 4.43 (t, *J =* 8.1 Hz, 1H), 4.31 (dd, *J =* 6.7, 2.6 Hz, 1H), 4.02 - 3.90 (m, 2H), 3.63 (s, 2H), 3.29 (d, *J =* 8.5 Hz, 2H), 2.21 (s, 3H), 1.99 (dd, *J* = 16.4, 7.6 Hz, 1H), 1.78 (d, *J* = 20.9 Hz, 1H), 1.32 (d, *J* = 6.9 Hz, 3H), 0.96 (d, *J* = 11.1 Hz, 9H). |
| 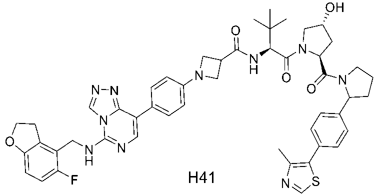 | MS m/z (ESI):457.3[M/2+H]⁺. |
| | ¹H NMR (400 MHz, DMSO-*d₆*) δ 9.44 (d, J = 1.7 Hz, 1H), 8.95 (d, J = 1.3 Hz, 1H), 8.49 (s, 1H), 8.21 (d, J = 9.3 Hz, 1H), 7.89 (d, J = 14.6 Hz, 3H), 7.51-7.18 (m, 4H), 6.93 (s, 1H), 6.69 (d, J = 3.9 Hz, 1H), 6.52 (d, J = 8.6 Hz, 2H), 5.18-4.98 (m, 1H), 4.83-4.64 (m, 3H), 4.62-4.48 (m, 3H), 4.38 (s, 1H), 4.12-3.51 (m, 8H), 2.67 (s, 2H), 2.07 (s, 2H), 1.87 (s, 6H), 0.90 (d, J = 5.3 Hz, 12H). |
| 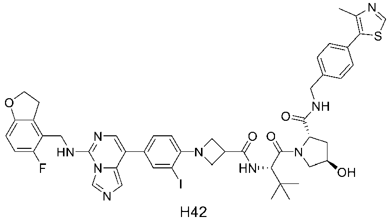 | MS m/z (ESI): 499.7[M/2+H]⁺_{∘} |
| | ¹H NMR (400 MHz, DMSO-*d₆*) δ 8.97 (s, 1H), 8.67 (s, 1H), 8.58 (d, J = 5.7 Hz, 1H), 8.19 (dd, J = 12.7, 7.4 Hz, 2H), 7.39 (q, J = 8.3 Hz, 4H), 7.14 (d, J = 8.3 Hz, 2H), 6.98 (s, 1H), 6.95-6.87 (m, 1H), 6.67 (dd, J = 8.5, 3.8 Hz, 1H), 6.46 (d, J = 8.4 Hz, 2H), 5.13 (d, J = 3.5 Hz, 1H), 4.66 (d, J = 4.5 Hz, 2H), 4.57 (d, J = 9.3 Hz, 1H), 4.51 (t, J = 8.7 Hz, 2H), 4.43 (dd, J = 15.7, 7.3 Hz, 2H), 4.33 (s, 1H), 4.20 (dd, J = 15.9, 5.4 Hz, 1H), 4.00 (t, J = 7.2 Hz, 1H), 3.94 (t, J = 7.3 Hz, 1H), 3.87-3.80 (m, 1H), 3.74 (td, J = 13.1, 6.5 Hz, 2H), 3.69-3.58 (m, 2H), 3.27 (t, J = 8.6 Hz, 2H), 2.43 (s, 3H), 2.01 (d, J = 7.8 Hz, 1H), 1.89 (dd, J = 14.9, 6.4 Hz, 1H), 0.94 (s, 9H). |

### Example 43 Preparation of Compound H43

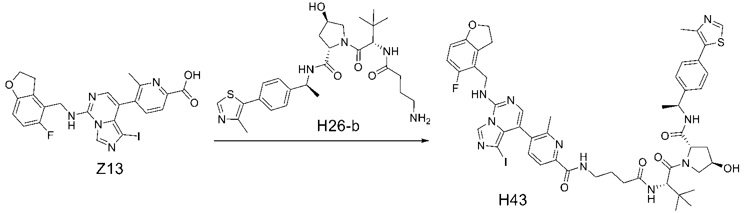

Step 1: H26-b (16 mg, 0.02 mmol), HATU (14 mg, 0.04 mmol), and DIPEA (12 mg, 0.10 mmol) were added to a solution of Intermediate Z13 (22 mg, 0.02 mmol) in DMF (2 mL). The mixture was stirred at 25°C for 2 hours. After filtration, the mixture was purified by preparative HPLC (Gilson_306_1741, column: Welch-Xtimate-C18-5µm-21.2×150mm; mobile phase: water (containing 10 mM ammonium bicarbonate) and acetonitrile, gradient ratio: acetonitrile 46%-95%, flow rate: 25 mL/min) to obtain H43 (3 mg), yield: 11.7%. MS m/z (ESI): 1057.2 [M+H]⁺. ¹H NMR (400 MHz, DMSO-*d₆*) δ 8.98 (s, 1H), 8.81-8.76 (m, 1H), 8.74 (s, 1H), 8.46-8.40 (m, 1H), 8.37 (d, J = 7.6 Hz, 1H), 7.93 (d, J = 7.6 Hz, 2H), 7.75 (d, J = 7.6 Hz, 1H), 7.45-7.36 (m, 4H), 7.12 (d, J = 1.2 Hz, 1H), 6.99-6.91 (m, 1H), 6.75-6.68 (m, 1H), 5.37-5.27 (m, 1H), 5.10 (d, J = 3.2 Hz, 1H), 4.92 (t, J = 7.2 Hz, 1H), 4.76-4.62 (m, 2H), 4.58-4.49 (m, 3H), 4.43 (t, J = 8.0 Hz, 1H), 4.28 (s, 1H), 3.61 (s, 2H), 2.45 (s, 3H), 2.34 (s, 3H), 2.07-1.91 (m, 4H), 1.84-1.75 (m, 3H), 1.54-1.41 (m, 2H), 1.37 (d, J = 7.2 Hz, 3H), 0.95 (s, 9H).

### Example 44 Preparation of Compound H44

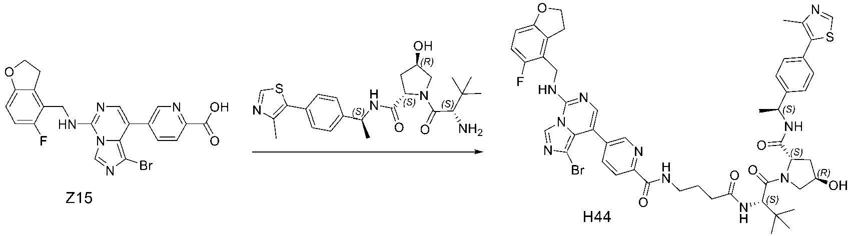

Step 1: Intermediate Z15 (43.73 mg, 90.30 µmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxami de (95.66 mg, 180.60 µmol) were dissolved in DMF (5 mL). HATU (68.13 mg, 180.60 µmol) and DIPEA (58.35 mg, 451.50 µmol, 78.64 µL) were added with stirring at room temperature, and the mixture was stirred at room temperature for 2 hours. After the reaction was completed, the reaction solution was concentrated and purified by silica gel column chromatography (MeOH:DCM = 0-10%), followed by preparative HPLC purification, to obtain H44 (7.41 mg, purity 100%), yield: 8.24%. MS m/z (ESI): 995 [M+H]⁺. ¹H NMR (400 MHz, DMSO-*d₆*) δ 9.02-8.89 (m, 2H), 8.74 (s, 1H), 8.67 (s, 1H), 8.59 (s, 1H), 8.39 (d, J = 8.0 Hz, 1H), 8.07 (d, J = 8.0 Hz, 1H), 8.04-7.98 (m, 1H), 7.95 (d, J = 9.3 Hz, 1H), 7.42 (d, J = 8.2 Hz, 2H), 7.35 (d, J = 8.2 Hz, 2H), 7.27 (d, J = 6.0 Hz, 1H), 6.98-6.88 (m, 1H), 6.68 (dd, J = 8.7, 3.8 Hz, 1H), 5.11 (d, J = 3.4 Hz, 1H), 4.95-4.83 (m, 1H), 4.71 (d, J = 4.8 Hz, 2H), 4.58-4.44 (m, 3H), 4.40 (t, J = 8.0 Hz, 1H), 4.26 (s, 1H), 3.59 (s, 2H), 3.29 (t, J = 8.7 Hz, 3H), 2.42 (d, J = 11.5 Hz, 3H), 2.32-2.24 (m, 1H), 2.22-2.09 (m, 1H), 1.98 (d, J = 8.8 Hz, 1H), 1.75 (d, J = 4.1 Hz, 3H), 1.35 (d, J = 6.9 Hz, 3H), 1.14 (t, J = 7.2 Hz, 1H), 0.92 (s, 9H).

### Example 45 Preparation of Compound H45

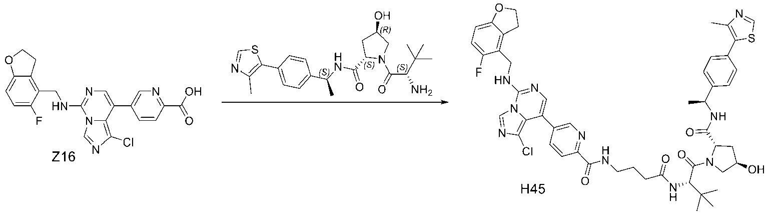

Step 1: Intermediate Z16 (80 mg, 181.89 µmol) was dissolved in DMF (3 mL). (2S,4R)-1-((S)-2-Amino-3,3-dimethylbutanoyl)-4-hydroxy-N-((S)-1-(4-(4-methylthiazol-5-yl)phenyl) ethyl)pyrrolidine-2-carboxamide (96.35 mg, 181.89 µmol), HATU (102.93 mg, 272.83 µmol), and DIPEA (117.54 mg, 909.45 µmol) were added, and the mixture was reacted at room temperature for 1 h. After the reaction was completed, the reaction solution was filtered, and the filtrate was concentrated and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to obtain H45 (10.02 mg, purity 99%), yield: 5.73%. MS m/z (ESI): 476.3 [M/2+H]⁺. ¹H NMR (400 MHz, DMSO-*d₆*) δ 8.97 (s, 1H), 8.92 (t, J = 8 Hz, 1H), 8.75-8.64 (m, 2H), 8.61 (s, 1H), 8.39 (d, J = 8 Hz, 1H), 8.09-7.99 (m, 2H), 7.94 (d, J = 8 Hz, 1H), 7.42 (d, J = 8 Hz, 2H), 7.36 (d, J = 8 Hz, 2H), 7.31-7.28 (m, 1H), 6.97-6.89 (m, 1H), 6.68 (dd, J = 4, 4 Hz, 1H), 5.10 (d, J = 4 Hz, 1H), 4.95-4.85 (m, 1H), 4.71 (s, 2H), 4.58-4.46 (m, 3H), 4.40 (t, J = 8.0 Hz, 1H), 4.26 (s, 1H), 3.59 (s, 2H), 3.29 (t, J = 8 Hz, 4H), 2.43 (s, 3H), 2.31-2.20 (m, 1H), 2.19-2.12 (m, 1H), 2.04-1.94 (m, 1H), 1.82-1.68 (m, 3H), 1.35 (d, J = 8 Hz, 3H), 0.92 (s, 9H).

### Example 46 Preparation of Compound H46

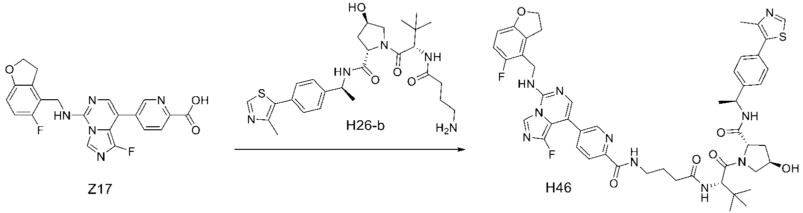

Step 1: Intermediate Z17 (33 mg, 0.078 mmol) was dissolved in DMF (5 mL). DIPEA (60 mg, 0.47 mmol) and HATU (88 mg, 0.23 mmol) were added. After stirring for 10 minutes, H26-b (66 mg, 0.12 mmol) was added, and the mixture was stirred at room temperature for 20 h. After the reaction was completed, the reaction solution was concentrated and preliminarily purified by CombiFlash (0-10% MeOH/DCM), then further purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to obtain H46 (1.75 mg), yield: 2.40%. MS m/z (ESI): 468.3 [M/2+H]⁺. ¹H NMR (400 MHz, DMSO-*d₆*) δ 9.01-8.90 (m, 2H), 8.77 (d, J = 10.0 Hz, 1H), 8.62-8.57 (m, 1H), 8.46-8.39 (m, 2H), 8.15-8.06 (m, 2H), 7.96 (d, J = 9.5 Hz, 1H), 7.47-7.35 (m, 5H), 6.98-6.92 (m, 1H), 6.71 (dd, J = 8.7, 3.8 Hz, 1H), 5.13 (d, J = 3.6 Hz, 1H), 4.94-4.88 (m, 1H), 4.73 (d, J = 4.4 Hz, 2H), 4.54 (dd, J = 16.6, 8.2 Hz, 3H), 4.42 (t, J = 8.0 Hz, 1H), 4.30-4.24 (m, 1H), 3.61 (s, 2H), 3.30 (dt, J = 12.3, 7.7 Hz, 4H), 2.45 (s, 3H), 2.33-2.27 (m, 1H), 2.21-2.14 (m, 1H), 2.00 (d, J = 7.6 Hz, 1H), 1.82-1.72 (m, 3H), 1.37 (d, J = 7.0 Hz, 3H), 0.94 (s, 9H).

### Example 47 Preparation of Compound H47

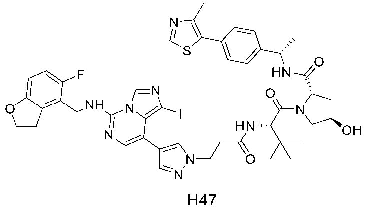

Compound H47 can be prepared referring to the method of the above examples. MS m/z (ESI): 488.3 [M/2+H]⁺. ¹H NMR (400 MHz, DMSO-*d₆*) δ 8.97 (s, 1H), 8.67 (s, 1H), 8.38 (d, J = 7.8 Hz, 1H), 8.25 (d, J = 5.2 Hz, 1H), 8.04 (d, J = 9.2 Hz, 1H), 7.80 (s, 1H), 7.47 (s, 1H), 7.44-7.33 (m, 4H), 7.03 (s, 1H), 6.95-6.86 (m, 1H), 6.67 (dd, J = 8.6, 3.9 Hz, 1H), 5.11 (d, J = 3.5 Hz, 1H), 4.89 (d, J = 7.3 Hz, 1H), 4.66 (d, J = 5.0 Hz, 2H), 4.51 (t, J = 8.9 Hz, 3H), 4.44-4.23 (m, 4H), 3.59 (d, J = 6.5 Hz, 2H), 3.25 (t, J = 8.7 Hz, 2H), 2.94-2.83 (m, 1H), 2.77-2.63 (m, 1H), 2.43 (s, 3H), 1.98 (d, J = 8.9 Hz, 1H), 1.83-1.72 (m, 1H), 1.35 (d, J = 7.0 Hz, 3H), 0.89 (s, 9H).

### Example 48 Preparation of Compound H48

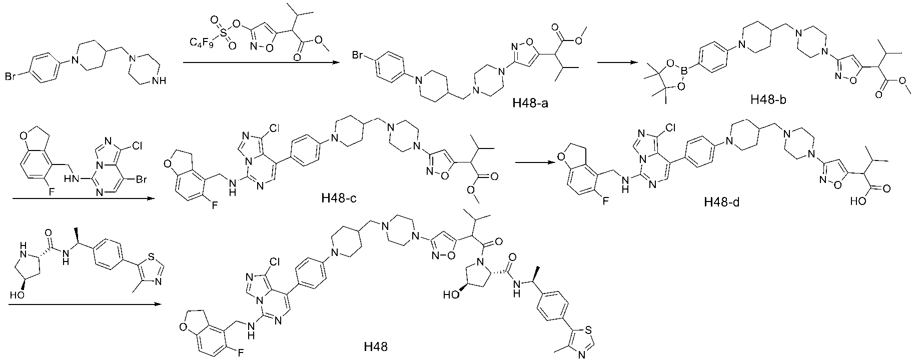

Step 1: 1-((1-(4-Bromophenyl)piperidin-4-yl)methyl)piperazine (4 g, 8.84 mmol), methyl 3-methyl-2-(3-(((perfluorobutyl)sulfonyl)oxy)isoxazol-5-yl)butanoate (6.38 g, 13.27 mmol), DIPEA (3.43 g, 26.53 mmol, 4.62 mL), and DMSO (50 mL) were mixed and heated to 110°C for reaction for 16 hours. After the reaction was completed, the reaction solution was poured into ice water, extracted with ethyl acetate (30 mL×2). The organic layers were combined and washed three times with water, once with saturated brine, dried over anhydrous sodium sulfate, and concentrated to obtain a crude product. The crude product was purified by silica gel column chromatography (dichloromethane:ammonia/methanol = 95%:5%) to obtain H48-a (4.3 g, brown liquid), yield: 93.60%. MS m/z (ESI): 519.2 [M+H]⁺.

Step 2: H48-a (1 g, 1.93 mmol) and bis(pinacolato)diboron (1.47 g, 5.78 mmol) were dissolved in 1,4-dioxane (20 mL). Under argon protection, Pd(dba)₂ (218.28 mg, 385.01 µmol) and PCy₃ (259.12 mg, 924.01 µmol) were added, and the mixture was heated to 120°C and stirred for 24 hours. After the reaction was completed, the reaction solution was filtered, and the filtrate was concentrated and purified by silica gel column chromatography (petroleum ether/triethylamine:dichloromethane = 85%:15%) to obtain H48-b (700 mg, yellow solid), yield: 64.18%. MS m/z (ESI): 567.4 [M+H]⁺.

Step 3: Under argon protection, 8-bromo-1-chloro-N-((5-fluoro-2,3-dihydrobenzofuran-4-yl)methyl)imidazo[1,5-c]pyrimidin-5-amine (130 mg, 326.94 µmol), H48-b (203.74 mg, 359.63 µmol), Pd(dppf)Cl₂ (23.92 mg, 32.69 µmol), K₂CO₃ (135.56 mg, 980.81 µmol), 1,4-dioxane (10 mL), and DMSO (1 mL) were mixed and heated to 100°C for reaction for 2 hours. After the reaction was completed, the reaction solution was filtered, ethyl acetate and saturated brine were added to the filtrate for extraction, the organic layer was separated, dried over anhydrous sodium sulfate, and concentrated to obtain a crude product. The crude product was purified by silica gel column chromatography (petroleum ether/triethylamine:ethyl acetate = 30%:70%) to obtain H48-c (166 mg, yellow solid), yield: 67.05%. MS m/z (ESI): 379.2 [M/2+H]⁺.

Step 4: At room temperature, LiOH (15.81 mg, 660.25 µmol) was added to a solution of H48-c (50 mg, 66.02 µmol) in THF (1 mL)/MeOH (1 mL)/water (0.5 mL). The mixture was reacted at room temperature for 1 hour. After the reaction was completed, the reaction solution was concentrated, the pH was adjusted to 5-6 with dilute hydrochloric acid (1 mol/L) at 0°C, then extracted with dichloromethane (10 mL×2). The organic layers were combined and washed with saturated brine, dried over anhydrous sodium sulfate, and concentrated to obtain H48-d (49 mg, yellow solid), yield: 99.85%. MS m/z (ESI): 743.3 [M+H]⁺.

Step 5: At room temperature, H48-d (44 mg, 59.20 µmol), (2S,4R)-4-hydroxy-N-((S)-1-(4-(4-methylthiazol-5-yl)phenyl)ethyl)pyrrolidine-2-carboxamide (21.58 mg, 65.12 µmol), and DIPEA (22.95 mg, 177.59 µmol, 30.93 µL) were added to DMF (2 mL). HATU (24.57 mg, 65.12 µmol) was then added, and the mixture was reacted at room temperature for 1 hour. After the reaction was completed, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to obtain H48 (33.08 mg), yield: 52.45%. MS m/z (ESI): 528.8 [M/2+H]⁺. ¹H NMR (400 MHz, DMSO-*d₆*) δ 8.97 (s, 1H), 8.62 (s, 1H), 8.39 & 8.20 (d, J = 7.8 Hz, 1H), 8.27 (t, J = 4.8 Hz, 1H), 7.44 (dd, J = 15.6, 6.4 Hz, 1H), 7.40 (d, J = 9.6 Hz, 1H), 7.35 (d, J = 8.2 Hz, 1H), 7.30 (d, J = 8.2 Hz, 1H), 7.21 (d, J = 8.6 Hz, 2H), 7.06 (s, 1H), 6.97-6.90 (m, 3H), 6.68 (dd, J = 8.8, 3.8 Hz, 1H), 6.15 (s, 1H), 5.09 (d, J = 3.6 Hz, 1H), 5.02-4.93 (m, 1H), 4.91-4.84 (m, 1H), 4.68 (d, J = 4.8 Hz, 2H), 4.52 (t, J = 8.8 Hz, 2H), 4.42-4.33 (m, 1H), 4.29-4.23 (m, 1H), 3.76-3.66 (m, 3H), 3.55-3.52 (m, 1H), 3.44-3.37 (m, 1H), 3.28-3.26 (m, 2H), 3.18-3.10 (m, 4H), 2.67 (t, J = 12.0 Hz, 2H), 2.44-2.40 (m, 5H), 2.38-2.35 (m, 1H), 2.19 (d, J = 7.2 Hz, 1H), 2.15 (d, J = 6.8 Hz, 1H), 2.05-1.97 (m, 1H), 1.83-1.74 (m, 3H), 1.71-1.63 (m, 1H), 1.44 (d, J = 6.8 Hz, 1H), 1.35 (dd, J = 9.6, 7.2 Hz, 2H), 1.25-1.13 (m, 3H), 0.96-0.93 (m, 3H), 0.83-0.77 (m, 3H).

### Example 49 Preparation of Compound H49

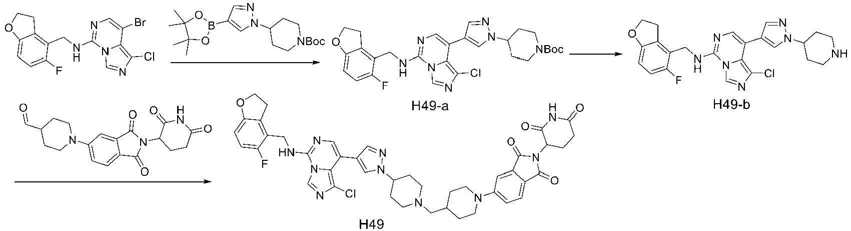

Step 1: Compound tert-butyl 4-(4-(4,4,5,5-tetramethyl-1,3,2-dioxaborolan-2-yl)-1H-pyrazol-1-yl)piperidine-1-carboxylate (755 mg, 2 mmol), 8-bromo-1-chloro-N-((5-fluoro-2,3-dihydrobenzofuran-4-yl)methyl)imidazo[1,5-c]pyrimidin-5-amine (792 mg, 2 mmol), K₂CO₃ (414 mg, 3 mmol), and Pd(dppf)Cl₂ (146 mg, 0.2 mmol) were added to 1,4-dioxane (5 mL) for dissolution, then water (1 mL) was added. The mixture was purged with nitrogen three times, then heated to 100°C and stirred for 5 hours. After the reaction was completed, the reaction solution was concentrated and purified by silica gel column chromatography (30-100% PE/EA) to obtain H49-a (850 mg), yield: 75%. MS m/z (ESI): 568 [M+H]⁺.

Step 2: H49-a (850 mg, 1.5 mmol) was added to a solution of trifluoroacetic acid in dichloromethane (10 mL, v/v = 30%) and stirred at room temperature for 1 hour. After the reaction was completed, the reaction solution was concentrated to obtain H49-b (700 mg), yield: 100%. MS m/z (ESI): 468 [M+H]⁺.

Step 3: H49-b (70 mg, 0.15 mmol) and 1-(2-(2,6-dioxopiperidin-3-yl)-1,3-dioxoisoindolin-5-yl)piperidine-4-carbaldehyde (56 mg, 0.15 mmol) were added to DMSO. Two drops of acetic acid were added, followed by NaBH(OAc)₃ (100 mg). The mixture was heated under microwave at 60°C for 45 minutes. After the reaction was completed, the reaction solution was filtered, and the filtrate was purified by preparative HPLC (waters-sunfire-10 µm-19×150 mm column (mobile phase: 28%-38% (v/v) acetonitrile and formic acid/water)) to obtain H49 (20 mg), yield: 16%. MS m/z (ESI): 821 [M+H]⁺. ¹H NMR (400 MHz, DMSO-*d₆*) δ 11.05 (s, 1H), 8.61 (s, 1H), 8.29 (s, 1H), 7.94 (s, 1H), 7.57 (s, 1H), 7.15 (s, 1H), 6.92 (t, J = 8.8 Hz, 2H), 6.81 (s, 1H), 6.75-6.55 (m, 2H), 4.67 (d, J = 4.9 Hz, 2H), 4.52 (t, J = 8.7 Hz, 2H), 4.17 (s, 1H), 2.91 (d, J = 25.2 Hz, 3H), 2.62 (q, J = 13.7, 11.8 Hz, 5H), 2.20 (s, 3H), 1.99 (d, J = 14.0 Hz, 7H), 1.80 (d, J = 12.5 Hz, 3H), 1.64 (s, 1H), 1.24 (d, J = 13.8 Hz, 4H).

### Example 50 Preparation of Compound H50

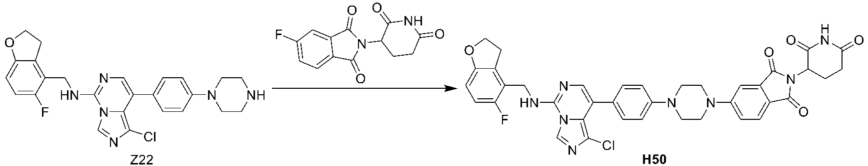

Compound Z22 (50 mg, 104.40 µmol) and 2-(2,6-dioxopiperidin-3-yl)-5-fluoroisoindoline-1,3-dione (86.51 mg, 313.19 µmol) were dissolved in DMSO (2 mL). DIEA (67.46 mg, 521.98 µmol, 90.92 µL) was added, and the mixture was heated to 120°C and stirred for 4 hours. The reaction solution was concentrated under reduced pressure to remove the solvent, and obtain a crude product. The crude product was purified by preparative liquid chromatography (preparative column: 21.2×250 mm C18 column; system: 10 mM NH₄HCO₃ H₂O/acetonitrile; wavelength: 254/214 nm; gradient: 5%-95% acetonitrile change) to obtain compound H50 (32.03 mg, 42.27 µmol, 40.49% yield, 97.01% purity) as a yellow solid. MS m/z (ESI): 735.3 [M+H]⁺. ¹H NMR (400 MHz, DMSO-*d₆*) δ 11.08 (s, 1H), 8.63 (s, 1H), 8.30 (t, J = 5.2 Hz, 1H), 7.70 (d, J = 8.4 Hz, 1H), 7.41 (d, J = 2.0 Hz, 1H), 7.32 (dd, J = 8.8, 2.4 Hz, 1H), 7.28 (d, J = 8.4 Hz, 2H), 7.08 (s, 1H), 7.03 (d, J = 8.4 Hz, 2H), 6.93 (t, J = 9.6 Hz, 1H), 6.68 (dd, J = 8.8, 4.0 Hz, 1H), 5.07 (dd, J = 12.8, 5.2 Hz, 1H), 4.68 (d, J = 4.8 Hz, 2H), 4.52 (t, J = 8.8 Hz, 2H), 3.69-3.57 (m, 4H), 3.40-3.35 (m, 4H), 3.29-3.25 (m, 2H), 2.93-2.81 (m, 1H), 2.65-2.51 (m, 2H), 2.06-1.94 (m, 1H).

### Example 51 Preparation of Compound H51

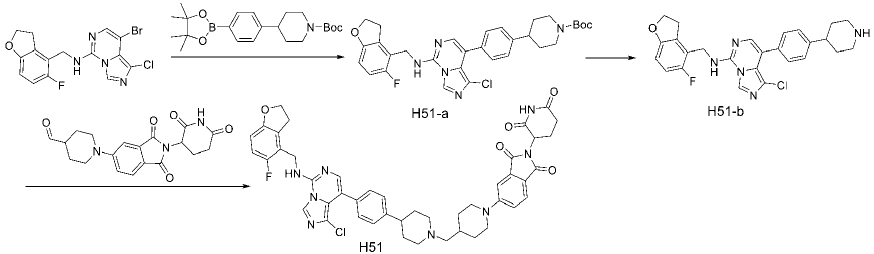

Step 1: 8-Bromo-1-chloro-N-((5-fluoro-2,3-dihydrobenzofuran-4-yl)methyl)imidazo[1,5-c] pyrimidin-5-amine (200 mg, 502.98 µmol) and tert-butyl 4-(4-(4,4,5,5-tetramethyl-1,3,2-dioxaborolan-2-yl)phenyl)piperidine-1-carboxylate (233.78 mg, 603.58 µmol) were dissolved in a mixed solution of DMSO/water/1,4-dioxane (1 mL/l mL/10 mL). K₂CO₃ (208.54 mg, 1.51 mmol) and Pd(dppf)Cl₂ (55.21 mg, 75.45 µmol) were then added, and the mixture was stirred at 90°C for 3 hours. After the reaction was completed, the reaction solution was cooled to room temperature, quenched with water, and extracted with ethyl acetate (80 mL×3). The organic phases were combined and dried over anhydrous sodium sulfate, concentrated, and purified by CombiFlash (4 g, 0-50% EA/PE) to obtain H51-a (250 mg, white solid), yield: 85.98%. MS m/z (ESI): 578.3 [M+H]⁺.

Step 2: H51-a (250 mg, 432.47 µmol) was dissolved in DCM (15 mL). TFA (2 g, 17.54 mmol) was added, and the mixture was stirred at room temperature for 2 hours. After the reaction was completed, the mixture was quenched with saturated aqueous sodium bicarbonate solution, extracted with dichloromethane (80 mL×3). The organic phases were combined and dried over anhydrous sodium sulfate and concentrated to obtain H51-b (200 mg, white solid), yield: 96.76%. MS m/z (ESI): 478.2 [M+H]⁺.

Step 3: H51-b (100 mg, 209.22 µmol) and 1-(2-(2,6-dioxopiperidin-3-yl)-1,3-dioxoisoindolin-5-yl)piperidine-4-carbaldehyde (120 mg, 324.88 µmol) were dissolved in DCM (10 mL). NaBH(OAc)₃ (133.03 mg, 627.67 µmol) was added, and the mixture was stirred at room temperature for 2 hours. After the reaction was completed, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-70% acetonitrile change) to obtain H51 (48.21 mg), yield: 27.51%. MS m/z (ESI): 416.3 [M/2+H]⁺. ¹H NMR (400 MHz, DMSO-*d₆*) δ 11.07 (s, 1H), 8.65 (s, 1H), 8.35 (t, J = 4.9 Hz, 1H), 7.63 (d, J = 8.6 Hz, 1H), 7.30 (dd, J = 17.6, 8.3 Hz, 4H), 7.21 (d, J = 8.7 Hz, 1H), 7.12 (s, 1H), 6.96-6.88 (m, 1H), 6.68 (dd, J = 8.6, 3.9 Hz, 1H), 5.05 (dd, J = 12.9, 5.3 Hz, 1H), 4.68 (d, J = 4.8 Hz, 2H), 4.52 (t, J = 8.7 Hz, 2H), 4.03 (d, J = 12.9 Hz, 2H), 3.29 (t, J = 8.7 Hz, 3H), 2.89 (dt, J = 19.8, 8.6 Hz, 5H), 2.55 (dd, J = 16.4, 9.9 Hz, 3H), 2.17 (s, 2H), 2.08-1.90 (m, 3H), 1.81 (dd, J = 32.0, 20.7 Hz, 5H), 1.72-1.60 (m, 2H), 1.14 (d, J = 11.0 Hz, 2H).

### Example 52 Preparation of Compound H52

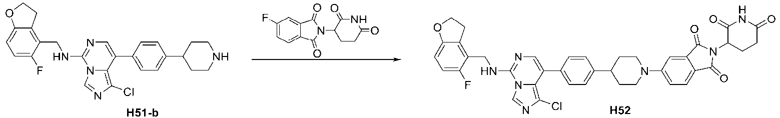

Compound H52 was prepared referring to the method of Example 50. MS m/z (ESI): 734.3 [M+H]⁺. ¹H NMR (400 MHz, DMSO-d₆) δ 11.07 (s, 1H), 8.65 (s, 1H), 8.36 (t, J = 5.0 Hz, 1H), 7.67 (d, J = 8.4 Hz, 1H), 7.39 - 7.25 (m, 6H), 7.13 (s, 1H), 6.92 (t, J = 9.6 Hz, 1H), 6.68 (dd, J = 8.8, 4.0 Hz, 1H), 5.06 (dd, J = 12.8, 5.6 Hz, 1H), 4.69 (d, J = 4.8 Hz, 2H), 4.52 (t, J = 8.8 Hz, 2H), 4.20 (d, J = 12.4 Hz, 2H), 3.27 (d, J = 8.8 Hz, 2H), 3.08 (t, J = 12.4 Hz, 2H), 2.93 - 2.82 (m, 2H), 2.60-2.51 (m, 2H), 2.04 - 1.97 (m, 1H), 1.93 (s, 1H), 1.90 (s, 1H), 1.76-1.67 (m, 2H).

### Example 53 Preparation of Compound H53

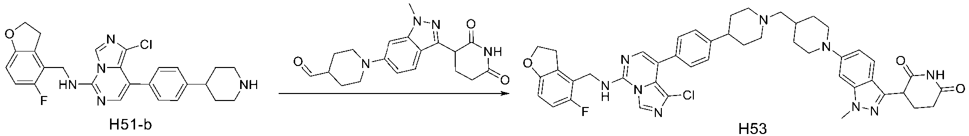

H51-b (50 mg, 104.61 µmol) and 1-(3-(2,6-dioxopiperidin-3-yl)-1-methyl-1H-indazol-6-yl)piperidine-4-carbaldehyde (44.61 mg, 125.53 µmol) were dissolved in a mixed solution of EtOH/DMSO (0.5 mL/1.5 mL). The mixture was heated under microwave to 85°C and stirred for 30 min. NaBH(OAc)₃ (72.81 mg, 313.83 µmol) was then added, and the mixture was heated under microwave to 85°C and stirred for 30 min. After the reaction was complete, the reaction solution was concentrated and preliminarily purified by CombiFlash (4 g, 0-12% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H53 (23.32 mg, purity 98.9%), yield: 27.01%. MS m/z (ESI): 408.7 [M/2+H]⁺. ¹H NMR (400 MHz, DMSO-d₆) δ 10.84 (s, 1H), 8.65 (s, 1H), 8.41 - 8.29 (m, 1H), 7.46 (d, J = 8.8 Hz, 1H), 7.31 (q, J = 8.1 Hz, 4H), 7.13 (d, J = 1.3 Hz, 1H), 6.98 - 6.86 (m, 2H), 6.81 (s, 1H), 6.68 (dd, J = 8.7, 3.8 Hz, 1H), 4.69 (d, J = 4.8 Hz, 2H), 4.53 (t, J = 8.7 Hz, 2H), 4.23 (dd, J = 9.2, 5.1 Hz, 1H), 3.87 (s, 3H), 3.78 (d, J = 12.1 Hz, 2H), 3.28 (d, J = 8.8 Hz, 2H), 2.97 (d, J = 10.4 Hz, 2H), 2.72 (t, J = 11.9 Hz, 2H), 2.60 (q, J = 6.8, 5.8 Hz, 2H), 2.52 (s, 1H), 2.36 - 2.09 (m, 4H), 1.99 (s, 2H), 1.75 (dt, J = 42.7, 12.6 Hz, 7H), 1.32 - 1.17 (m, 2H).

### Example 54 Preparation of Compound H54

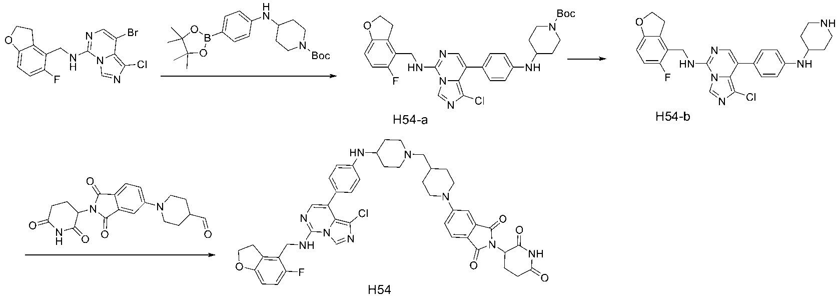

Step 1: 8-Bromo-1-chloro-N-((5-fluoro-2,3-dihydrobenzofuran-4-yl)methyl)imidazo[1,5-c] pyrimidin-5-amine (200 mg, 502.98 µmol) and tert-butyl 4-((4-(4,4,5,5-tetramethyl-1,3,2-dioxaborolan-2-yl)phenyl)amino)piperidine-1-carboxylate (212.48 mg, 528.13 µmol) were dissolved in a mixed solution of 1,4-dioxane (10 mL), DMSO (1 mL), and water (1 mL). Pd(dppf)Cl₂ (36.80 mg, 50.30 µmol) and K₂CO₃ (208.54 mg, 1.51 mmol) were added. After purging with nitrogen, the mixture was stirred at 90°C for 3 hours. After the reaction was complete, water (20 mL) was added to the reaction solution, extraction was performed with ethyl acetate (30 mL×3), the combined organic phases were dried over anhydrous sodium sulfate, concentrated, and purified by combiflash (EA:PE = 0-40%) to give H54-a (87 mg, gray oil), yield: 29.16%. MS m/z (ESI): 593.3 [M+H]⁺.

Step 2: H54-a (87 mg, 146.69 µmol) was dissolved in DCM (2 mL). TFA (16.73 mg, 146.69 µmol, 1 mL) was added, and the mixed solution was stirred at room temperature (25°C) for 1 hour. After the reaction was complete, the reaction solution was concentrated to give H54-b (72 mg, crude, brown liquid), MS m/z (ESI): 493.2 [M+H]⁺. It was used directly in the next step without further purification.

Step 3: H54-b (72 mg, 146.05 µmol) and 1-(2-(2,6-dioxopiperidin-3-yl)-1,3-dioxoisoindolin-5-yl)piperidine-4-carbaldehyde (107.89 mg, 292.10 µmol) were dissolved in DMSO (3 mL) and anhydrous ethanol (1 mL). NaBH(OAc)₃ (92.86 mg, 438.16 µmol) was added, and the mixed solution was stirred at room temperature (25°C) for 16 hours. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 62%-67% acetonitrile change) to give H54 (17.55 mg), yield: 13.97%. MS m/z (ESI): 423.8 [M/2+H]⁺. ¹H NMR (400 MHz, DMSO-d₆) δ 11.07 (s, 1H), 8.60 (s, 1H), 8.21 (s, 1H), 7.63 (d, J = 8.5 Hz, 1H), 7.29 (s, 1H), 7.21 (d, J = 8.7 Hz, 1H), 7.08 (d, J = 8.1 Hz, 2H), 7.01 (d, J = 1.2 Hz, 1H), 6.92 (t, J = 9.5 Hz, 1H), 6.67 (dd, J = 8.7, 3.8 Hz, 1H), 6.59 (d, J = 8.2 Hz, 2H), 5.58 (d, J = 7.8 Hz, 1H), 5.04 (dd, J = 12.9, 5.4 Hz, 1H), 4.67 (d, J = 4.9 Hz, 2H), 4.52 (t, J = 8.7 Hz, 2H), 4.02 (d, J = 12.9 Hz, 2H), 3.42 - 3.37 (m, 1H), 3.27 (t, J = 8.7 Hz, 2H), 3.04 - 2.72 (m, 5H), 2.68 - 2.49 (m, 2H), 2.13 (d, J = 6.6 Hz, 2H), 2.01 (s, 3H), 1.90 (d, J = 11.7 Hz, 2H), 1.78 (d, J = 12.7 Hz, 3H), 1.38 (d, J = 11.4 Hz, 2H), 1.12 (d, J = 12.1 Hz, 2H).

### Example 55 Preparation of Compound H55

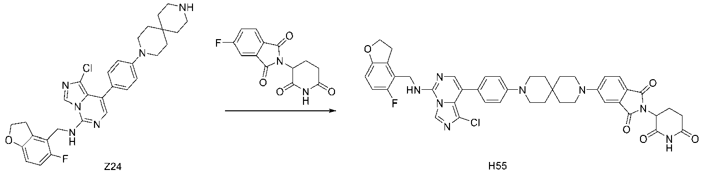

Compound H55 was prepared referring to the method of Example 50. MS m/z (ESI): 804.3 [M+H]⁺. ¹H NMR (400 MHz, DMSO-d₆) δ 11.06 (s, 1H), 8.63 (s, 1H), 8.28 (t, J = 5.2 Hz, 1H), 7.64 (d, J = 8.5 Hz, 1H), 7.31 (d, J = 2.2 Hz, 1H), 7.23 (dd, J = 8.6, 1.9 Hz, 3H), 7.06 (s, 1H), 6.94 (dd, J = 24.6, 9.0 Hz, 3H), 6.68 (dd, J = 8.7, 3.9 Hz, 1H), 5.05 (dd, J = 12.9, 5.4 Hz, 1H), 4.68 (d, J = 4.9 Hz, 2H), 4.52 (t, J = 8.7 Hz, 2H), 3.50 (t, J = 5.5 Hz, 4H), 3.28 (s, 2H), 3.22 (t, J = 5.8 Hz, 4H), 2.85 (d, J = 12.7 Hz, 1H), 2.69 (d, J = 6.6 Hz, 1H), 2.59 - 2.53 (m, 1H), 2.05 - 1.93 (m, 1H), 1.60 (dt, J = 16.3, 5.4 Hz, 8H).

### Example 56 Preparation of Compound H56

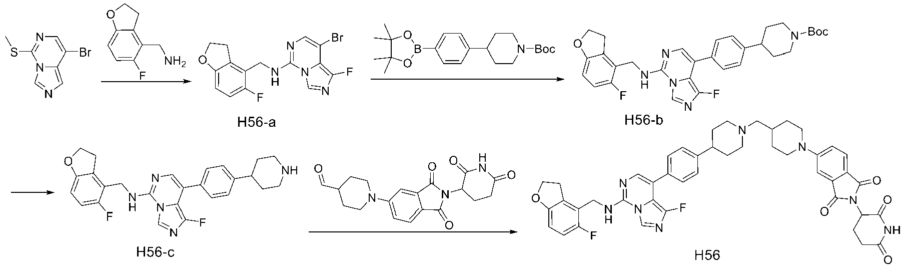

Step 1: 8-Bromo-5-(methylthio)imidazo[1,5-c]pyrimidine (1200 mg, 4.92 mmol) was dissolved in acetonitrile (30 mL). Selectfluor (2.09 g, 5.90 mmol) was added, and the mixture was stirred at room temperature for 18 hours. Then Et₃N (2.49 g, 24.58 mmol) and (5-fluoro-2,3-dihydrobenzofuran-4-yl)methanamine (986.18 mg, 5.90 mmol) were added, and the mixture was stirred at room temperature for another 5 hours. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-70% acetonitrile change) to give H56-a (130 mg, light yellow solid), yield: 6.94%. MS m/z (ESI): 381.0 [M+H]⁺. ¹H NMR (400 MHz, DMSO-d₆) δ 8.41 (s, 1H), 8.34 (s, 1H), 7.29 (s, 1H), 6.96 - 6.87 (m, 1H), 6.68 (dd, J = 8.6, 3.9 Hz, 1H), 4.62 (s, 2H), 4.51 (t, J = 8.8 Hz, 2H), 3.24 (t, J = 8.7 Hz, 2H).

Step 2: H56-a (130 mg, 341.05 µmol) and tert-butyl 4-(4-(4,4,5,5-tetramethyl-1,3,2-dioxaborolan-2-yl)phenyl)piperidine-1-carboxylate (158.52 mg, 409.26 µmol) were dissolved in DMSO (1 mL), water (1 mL), and 1,4-dioxane (10 mL). Then K₂CO₃ (141.41 mg, 1.02 mmol) and Pd(dppf)Cl₂ (49.91 mg, 68.21 µmol) were added, and the mixture was stirred at 90°C for 3 hours. After the reaction was complete, the reaction solution was cooled to room temperature, quenched with water, and extracted with ethyl acetate (80 mL×3). The combined organic phases were dried over anhydrous sodium sulfate, concentrated, and purified by CombiFlash (4 g, 0-50% EA/PE) to give H56-b (120 mg, light yellow solid), yield: 62.65%. MS m/z (ESI): 562.3 [M+H]⁺.

Step 3: H56-b (120 mg, 213.67 µmol) was dissolved in DCM (8 mL). TFA (1 g, 8.77 mmol) was then added, and the mixture was stirred at room temperature for 2 hours. After the reaction was complete, the reaction solution was quenched with aqueous sodium bicarbonate solution, extracted with dichloromethane (80 mL×3). The combined organic phases were dried over anhydrous sodium sulfate and concentrated to give H56-c (98 mg, light yellow solid), yield: 99.38%. MS m/z (ESI): 462.2 [M+H]⁺.

Step 4: H56-c (98 mg, 212.35 µmol) and 1-(2-(2,6-dioxopiperidin-3-yl)-1,3-dioxoisoindolin-5-yl)piperidine-4-carbaldehyde (109.81 mg, 297.29 µmol) were dissolved in DCM (10 mL). NaBH(OAc)₃ (112.51 mg, 530.87 µmol) was then added, and the mixture was stirred at room temperature for 2 hours. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-70% acetonitrile change) to give H56 (59.83 mg), yield: 34.19%. MS m/z (ESI): 408.2 [M/2+H]⁺. ¹H NMR (400 MHz, DMSO-d₆) δ 11.07 (s, 1H), 8.38 (s, 1H), 8.30 (t, J = 4.9 Hz, 1H), 7.63 (d, J = 8.5 Hz, 1H), 7.41 (d, J = 6.2 Hz, 2H), 7.30 (d, J = 6.8 Hz, 3H), 7.21 (d, J = 8.6 Hz, 1H), 7.16 (s, 1H), 6.98 - 6.87 (m, 1H), 6.68 (dd, J = 8.6, 3.8 Hz, 1H), 5.05 (dd, J = 12.8, 5.3 Hz, 1H), 4.68 (d, J = 4.5 Hz, 2H), 4.52 (t, J = 8.7 Hz, 2H), 4.02 (d, J = 12.5 Hz, 2H), 3.28 (d, J = 8.8 Hz, 2H), 3.02 - 2.80 (m, 5H), 2.56 (d, J = 21.6 Hz, 3H), 2.16 (s, 2H), 2.08 - 1.90 (m, 3H), 1.90 - 1.71 (m, 5H), 1.71 - 1.56 (m, 2H), 1.24 - 1.06 (m, 2H).

### Example 57 Preparation of Compound H57

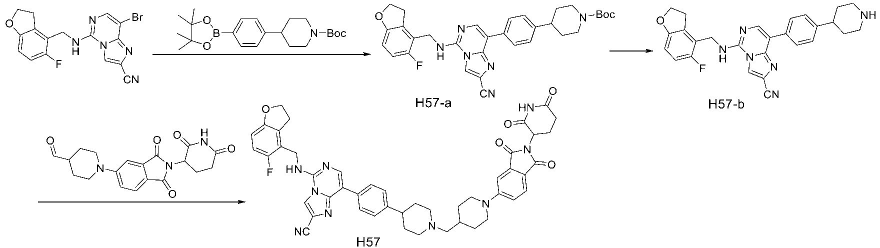

Step 1: 8-Bromo-5-((5-fluoro-2,3-dihydrobenzofuran-4-yl)methylamino)imidazo[1,2-c] pyrimidine-2-carbonitrile (150 mg, 386.41 µmol) and tert-butyl 4-[4-(4,4,5,5-tetramethyl-1,3,2-dioxaborolan-2-yl)phenyl]piperidine-1-carboxylate (194.56 mg, 502.33 µmol) were dissolved in a mixed solution of water (1.5 mL) and 1,4-dioxane (7.5 mL). Then Pd(dppf)Cl₂ (28.27 mg, 38.64 µmol) and NaHCO₃ (97.38 mg, 1.16 mmol) were added, and the mixture was stirred at 100°C for 1.5 hours. After the reaction was complete, water was added, extraction was performed with ethyl acetate (80 mL×3), the combined organic phases were dried over anhydrous sodium sulfate, and concentrated to give product H57-a (219 mg, black solid), yield: 99.67%. MS m/z (ESI): 469.2 [M-100+H]⁺.

Step 2: H57-a (219 mg, 385.13 µmol) was dissolved in DCM (5 mL). TFA (28.18 mg, 247.14 µmol, 2 mL) was then added, and the mixture was stirred at room temperature for 3 hours. After the reaction was complete, the reaction solution was neutralized with saturated aqueous sodium bicarbonate solution, extracted with dichloromethane (80 mL×3), the combined organic phases were dried over anhydrous sodium sulfate, and concentrated to give H57-b (160 mg, light yellow solid), yield: 88.67%. MS m/z (ESI): 469.2 [M+H]⁺.

Step 3: H57-b (160 mg, 341.50 µmol) and 1-[2-(2,6-dioxopiperidin-3-yl)-1,3-dioxoisoindolin-5-yl]piperidine-4-carbaldehyde (176.60 mg, 478.10 µmol) were dissolved in DCM (10 mL) and stirred at room temperature for 1 hour. Then NaBH(OAc)₃ (217.13 mg, 1.02 mmol) was added, and the mixture was stirred at room temperature for 1 hour. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-70% acetonitrile change) to give H57 (52.86 mg), yield: 18.53%. MS m/z (ESI): 822.4 [M+H]⁺. ¹H NMR (400 MHz, DMSO-d₆) δ 11.07 (s, 1H), 8.82 (s, 1H), 8.69 (s, 1H), 7.64 (d, J = 8.7 Hz, 1H), 7.53 (s, 1H), 7.43 (d, J = 8.0 Hz, 2H), 7.37 - 7.26 (m, 3H), 7.22 (d, J = 8.7 Hz, 1H), 6.93 (t, J = 9.5 Hz, 1H), 6.72-6.65 (m, 1H), 5.05 (dd, J = 13.0, 5.1 Hz, 1H), 4.71 (d, J = 4.1 Hz, 2H), 4.53 (t, J = 8.6 Hz, 2H), 4.04 (d, J = 12.5 Hz, 2H), 3.28 (s, 1H), 2.90-3.02 (m, 4H), 2.87-2.81 (m, 1H), 2.58-2.48 (m, 3H), 2.16 (s, 2H), 2.04-1.93 (m, 3H), 1.92-1.72 (m, 5H), 1.73-1.58 (m, 2H), 1.22-1.10 (m, 3H).

### Example 58 Preparation of Compound H58

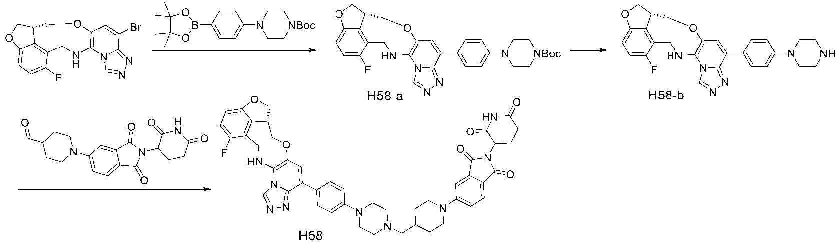

Step 1: (S)-4-Bromo-12-fluoro-7a,8,13,14-tetrahydro-7H-[1,2,4]triazolo[4',3':1,6]pyrido[3,2-b] benzofuro[4,3-fg][1,4]oxazocine (23 mg, 0.059 mmol) and tert-butyl 4-(4-(4,4,5,5-tetramethyl-1,3,2-dioxaborolan-2-yl)phenyl)piperazine-1-carboxylate (46 mg, 0.12 mmol) were dissolved in 1,4-dioxane (10 mL) and water (1 mL). Pd(PPh₃)₄ (10 mg, 0.088 mmol) and K₂CO₃ (16 mg, 0.12 mmol) were added, and the mixture was stirred at 100°C for 4 hours. After the reaction was complete, the reaction solution was concentrated and purified by CombiFlash (0-10% MeOH/DCM) to give H58-a (27 mg, yellow solid), yield: 80.20%. MS m/z (ESI): 573.3 [M+H]⁺.

Step 2: H58-a (27 mg, 0.047 mmol) was dissolved in DCM (8 mL). A solution of hydrogen chloride in ethyl acetate (4 mol/L, 1 mL) was added, and the mixture was stirred at room temperature for 2 hours. After the reaction was complete, the reaction solution was concentrated to give H58-b (22 mg, crude), yield: 98.75%. MS m/z (ESI): 473.3 [M+H]⁺.

Step 3: H58-b (20 mg, 0.042 mmol) and 1-[2-(2,6-dioxopiperidin-3-yl)-1,3-dioxoisoindolin-5-yl]piperidine-4-carbaldehyde (31 mg, 0.084 mmol) were dissolved in DCM (15 mL). Acetic acid (5 mg, 0.084 mmol) was added, and the mixture was stirred at room temperature for 0.5 hour. Then NaBH(OAc)₃ (36 mg, 0.17 mmol) was added, and stirring was continued overnight. After the reaction was complete, the reaction solution was concentrated and purified by CombiFlash (0-10% MeOH/DCM), then further purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H58 (12.11 mg), yield: 34.64%. MS m/z (ESI): 413.8 [M/2+H]⁺. ¹H NMR (400 MHz, DMSO-d₆) δ 11.06 (s, 1H), 9.40 (s, 1H), 8.07 (d, J = 8.7 Hz, 2H), 7.65 (d, J = 8.5 Hz, 1H), 7.51 (s, 1H), 7.33 (dd, J = 13.8, 3.6 Hz, 2H), 7.24 (d, J = 8.9 Hz, 1H), 7.00 (d, J = 8.9 Hz, 2H), 6.97 - 6.89 (m, 1H), 6.67 (dd, J = 8.7, 3.8 Hz, 1H), 5.07 (dd, J = 12.9, 5.3 Hz, 1H), 4.88 (dd, J = 15.0, 6.0 Hz, 1H), 4.75 (dd, J = 14.8, 6.5 Hz, 1H), 4.53 (t, J = 9.0 Hz, 2H), 4.21 (d, J = 6.8 Hz, 1H), 4.06 (d, J = 13.4 Hz, 3H), 3.92 (t, J = 11.2 Hz, 1H), 3.27-3.13 (m, 4H), 2.98 (t, J = 12.0 Hz, 2H), 2.91 - 2.82 (m, 1H), 2.69 - 2.51 (m, 5H), 2.21 (d, J = 6.7 Hz, 2H), 2.02 (dd, J = 12.8, 6.9 Hz, 1H), 1.94 - 1.78 (m, 3H), 1.27 - 1.10 (m, 3H).

### Example 59 Preparation of Compound H59

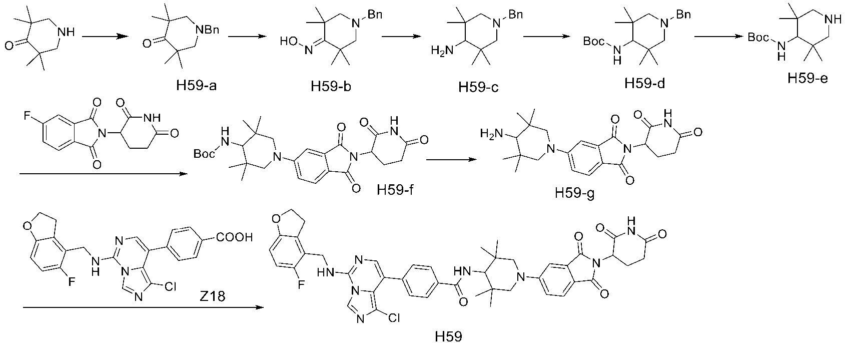

Step 1: 3,3,5,5-Tetramethylpiperidin-4-one (3.7 g, 19.30 mmol, HCl) and K₂CO₃ (8.00 g, 57.90 mmol) were dissolved in DMF (40.00 mL). Benzyl bromide (4.95 g, 28.95 mmol, 3.44 mL) was added, and the temperature was raised to 60°C and stirred overnight. After the reaction was complete, the reaction solution was cooled to room temperature, water and ethyl acetate were added, extraction was performed three times with ethyl acetate, the combined organic phases were washed with saturated brine, dried over anhydrous sodium sulfate, concentrated, and purified by CombiFlash (40 g, 0-5% DCM/PE) to give H59-a (4.7 g, colorless oil), yield: 99.25%. MS m/z (ESI): 246.2 [M+H]⁺.

Step 2: H59-a (4.9 g, 19.97 mmol) and hydroxylamine hydrochloride (6.94 g, 99.85 mmol, HCl) were dissolved in MeOH (120 mL). Sodium acetate (12.29 g, 149.78 mmol) was added, and the temperature was raised to 70°C and stirred for 72 hours. After the reaction was complete, the reaction solution was concentrated, water and dichloromethane were added, extraction was performed three times with dichloromethane, the combined organic phases were washed with saturated brine, dried over anhydrous sodium sulfate, and concentrated to give H59-b (4.9 g, white solid, crude), yield: 94.23%, which was used directly in the next step without purification.

Step 3: H59-b (5.49 g, 21.09 mmol) was dissolved in THF (100 mL). LiAlH₄ (2.40 g, 63.27 mmol) was added with stirring at room temperature, and the temperature was raised to 65°C and stirred for 3 hours. After the reaction was complete, the reaction solution was cooled to room temperature, 10 g of sodium sulfate decahydrate was added portionwise, and the mixture was stirred at room temperature for 16 hours. The reaction solution (containing H59-c) was used directly in the next step without any workup. MS m/z (ESI): 247 [M+H]⁺.

Step 4: To the reaction solution obtained in Step 3, (Boc)₂O (7.09 g, 32.47 mmol) and triethylamine (8.20 g, 81.17 mmol, 11.29 mL) were added with stirring at room temperature, and the mixture was stirred at room temperature for 3 hours. After the reaction was complete, the reaction solution was filtered, concentrated, and purified by silica gel column chromatography (EA:PE = 0-100%) to give H59-d (4.9 g, yellow oil), yield: 87.11%. MS m/z (ESI): 347 [M+H]⁺.

Step 5: H59-d (4.9 g, 14.14 mmol) was dissolved in THF (50 mL). Palladium on carbon (15.04 g, 14.14 mmol, 10% purity) was added, and the temperature was raised to 45°C under hydrogen protection and stirred overnight. After the reaction was complete, the reaction solution was filtered and concentrated to give H59-e (3.5 g, off-white solid), yield: 96.54%. MS m/z (ESI): 257 [M+H]⁺.

Step 6: 2-(2,6-Dioxopiperidin-3-yl)-5-fluoroisoindoline-1,3-dione (0.5 g, 1.81 mmol) and H59-e (1.39 g, 5.43 mmol) were dissolved in NMP (15.01 mL), and the temperature was raised to 120°C and stirred for 2 minutes. After the reaction was complete, the reaction solution was concentrated and purified by silica gel column chromatography (MeOH:DCM = 0-10%) to give H59-f (600 mg, yellow solid), yield: 64.66%. MS m/z (ESI): 513 [M+H]⁺.

Step 7: H59-f (160 mg, 312.14 µmol) was dissolved in DCM (10 mL). A solution of hydrogen chloride in ethyl acetate (4.0 mol/L, 5 mL) was added with stirring at room temperature, and the mixture was stirred at room temperature for 2 hours. After the reaction was complete, the reaction solution was concentrated to give H59-g (128.75 mg, yellow solid), yield: 100.00%. MS m/z (ESI): 413 [M+H]⁺.

Step 8: Z18 (80 mg, 182.30 µmol) was dissolved in DCM (10 mL). Oxalyl chloride (115.69 mg, 911.50 µmol) and DMF (1.33 mg, 18.23 µmol, 1.41 µL) were added with stirring at room temperature under argon protection, and the mixture was stirred at room temperature for 2 hours. After the reaction was complete, the reaction solution was concentrated. H59-g (75.20 mg, 182.30 µmol) and DIPEA (235.61 mg, 1.82 mmol, 317.53 µL) were dissolved in DCM (10 mL) and stirred at room temperature for 2 hours. After the reaction was complete, the reaction solution was purified by preparative HPLC to give H59 (4.18 mg, purity 100%), yield: 2.75%. MS m/z (ESI): 833 [M+H]⁺. ¹H NMR (400 MHz, DMSO-d₆) δ 11.07 (s, 1H), 8.70 (s, 1H), 8.51 (s, 1H), 7.90 (d, J = 8.2 Hz, 2H), 7.75 (d, J = 10.3 Hz, 1H), 7.60 (d, J = 8.6 Hz, 1H), 7.50 (d, J = 8.2 Hz, 2H), 7.35 (s, 1H), 7.27 (d, J = 8.9 Hz, 1H), 7.19 (s, 1H), 6.99 - 6.88 (m, 1H), 6.69 (dd, J = 8.7, 3.7 Hz, 1H), 5.05 (dd, J = 13.1, 5.3 Hz, 1H), 4.71 (d, J = 4.4 Hz, 2H), 4.53 (t, J = 8.8 Hz, 2H), 4.03 (d, J = 11.0 Hz, 3H), 3.28 (d, J = 8.9 Hz, 3H), 2.99 - 2.81 (m, 3H), 2.56 (d, J = 22.1 Hz, 1H), 2.04 - 1.94 (m, 1H), 1.03 (s, 6H), 0.90 (s, 6H).

### Example 60 Preparation of Compound H60

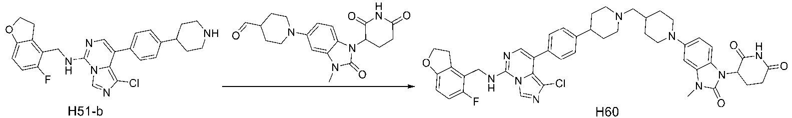

Step 1: H51-b (35 mg, 73.23 µmol) and 1-[1-(2,6-dioxopiperidin-3-yl)-3-methyl-2-oxobenzimidazol-5-yl]piperidine-4-carbaldehyde (54.25 mg, 146.46 µmol) were dissolved in DCM (5 mL). NaBH(OAc)₃ (46.56 mg, 219.68 µmol) was added with stirring at room temperature, and the mixture was stirred at room temperature overnight. After the reaction was complete, the reaction solution was concentrated and purified by preparative HPLC to give H60 (22.95 mg, purity 97.39%), yield: 36.67%. MS m/z (ESI): 417 [M/2+H]⁺. ¹H NMR (400 MHz, DMSO-d₆) δ 11.05 (s, 1H), 8.65 (s, 1H), 8.36 (s, 1H), 8.18 (s, 1H), 7.33-7.26 (m, 3H), 7.13 (s, 1H), 6.95-6.87 (m, 2H), 6.80 (s, 1H), 6.68 (dd, J = 8.6, 3.8 Hz, 1H), 6.61 (d, J = 8.5 Hz, 1H), 5.31 - 5.21 (m, 1H), 4.68 (d, J = 4.7 Hz, 2H), 4.52 (t, J = 8.7 Hz, 2H), 3.57 (d, J = 12.2 Hz, 2H), 3.40 - 3.28 (m, 5H), 2.98-2.83 (m, 4H), 2.67-2.58 (m, 4H), 2.21 (d, J = 6.8 Hz, 2H), 2.05-1.90 (m, 3H), 1.85-1.58 (m, 7H), 1.25 (d, J = 9.8 Hz, 2H).

### Example 61 Preparation of Compound H61

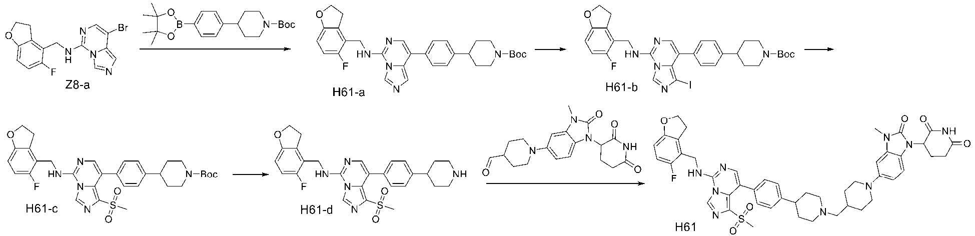

Step 1: Z8-a (200 mg, 550.69 µmol) and tert-butyl 4-[4-(4,4,5,5-tetramethyl-1,3,2-dioxaborolan-2-yl)phenyl]piperidine-1-carboxylate (213.29 mg, 550.69 µmol) were dissolved in 1,4-dioxane (10 mL) and water (1 mL). Under argon protection, Pd(dppf)Cl₂ (402.94 mg, 550.69 µmol) and K₂CO₃ (76.11 mg, 550.69 µmol) were added, and the temperature was raised to 95°C and stirred for 5 hours. After the reaction was complete, the reaction solution was concentrated and purified by silica gel column chromatography (EA:PE = 0-50%) to give H61-a (230 mg, yellow solid), yield: 76.83%. MS m/z (ESI): 544 [M+H]⁺.

Step 2: H61-a (100 mg, 183.95 µmol) was dissolved in DIPEA (2 mL). NIS (41.39 mg, 183.95 µmol) was added under ice bath conditions, and the mixture was stirred at room temperature for 15 minutes. After the reaction was complete, the reaction solution was concentrated and purified by silica gel column chromatography (EA:PE = 0-100%) to give H61-b (100 mg, yellow solid), yield: 81.20%. MS m/z (ESI): 670 [M+H]⁺.

Step 3: H61-b (200 mg, 298.72 µmol) was dissolved in DMSO (4 mL). Under argon protection, sodium methanesulfinate (91.49 mg, 896.16 µmol) and CuI (170.67 mg, 896.16 µmol) were added, and the temperature was raised to 100°C and stirred for 1 hour. After the reaction was complete, the reaction solution was concentrated and purified by silica gel column chromatography (EA:PE = 0-100%) to give H61-c (120 mg, yellow solid), yield: 64.61%. MS m/z (ESI): 622 [M+H]⁺.

Step 4: H61-c (120 mg, 193.01 µmol) was dissolved in DCM (5 mL). A solution of hydrogen chloride in ethyl acetate (4.0 mol/L, 3 mL) was added with stirring at room temperature, and the mixture was stirred at room temperature for 2 hours. After the reaction was complete, the reaction solution was concentrated to give H61-d (100.68 mg, yellow solid), yield: 100.00%. MS m/z (ESI): 522 [M+H]⁺.

Step 5: H61-d (80 mg, 153.37 µmol) and 1-(1-(2,6-dioxopiperidin-3-yl)-3-methyl-2-oxo-2,3-dihydro-1H-benzo[d]imidazol-5-yl)piperidine-4-carbaldehyde(113.62 mg, 306.75 µmol) were dissolved in DCM (10 mL). NaBH(OAc)₃ (97.52 mg, 460.12 µmol) was added with stirring at room temperature, and the mixture was stirred at room temperature overnight. After the reaction was complete, the reaction solution was concentrated and purified by preparative HPLC to give H61 (37.10 mg, purity 99.25%), yield: 27.41%. MS m/z (ESI): 876.4 [M+H]⁺. ¹H NMR (400 MHz, DMSO-d₆) δ 11.05 (s, 1H), 8.84 (s, 1H), 8.61 (s, 1H), 7.35 (s, 1H), 7.31 (d, J = 8.1 Hz, 2H), 7.22 (d, J = 8.1 Hz, 2H), 6.92 (dd, J = 12.4, 9.3 Hz, 2H), 6.81 (s, 1H), 6.69 (dd, J = 8.7, 3.8 Hz, 1H), 6.62 (d, J = 8.7 Hz, 1H), 5.27 (dd, J = 12.8, 5.2 Hz, 1H), 4.71 (d, J = 4.6 Hz, 2H), 4.53 (t, J = 8.7 Hz, 2H), 3.57 (d, J = 11.8 Hz, 2H), 3.29 (s, 6H), 3.00-2.79 (m, 6H), 2.71-2.50 (m, 5H), 2.21 (d, J = 7.2 Hz, 2H), 2.07 - 1.91 (m, 3H), 1.83 - 1.60 (m, 6H), 1.31-1.19 (m, 2H).

### Example 62 Preparation of Compound H62

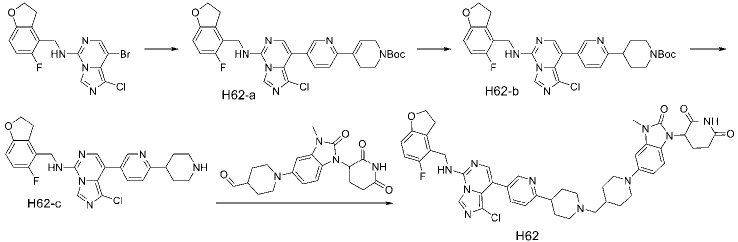

Step 1: Compound tert-butyl 5-(4,4,5,5-tetramethyl-1,3,2-dioxaborolan-2-yl)-3',6'-dihydro-[2,4'-bipyridine]-1'(2'H)-carboxylate (772 mg, 2 mmol), 8-bromo-1-chloro-N-((5-fluoro-2,3-dihydrobenzofuran-4-yl)methyl)imidazo[1,5-c]pyrimidin-5-amine (792 mg, 2 mmol), K₂CO₃ (414 mg, 3 mmol), and Pd(dppf)Cl₂ (146 mg, 0.2 mmol) were added to 1,4-dioxane (5 mL) for dissolution, then water (1 mL) was added. Under a nitrogen atmosphere, the mixture was heated to 100°C and stirred for 5 hours. After the reaction was complete, the reaction solution was purified by silica gel column chromatography (30-100% PE/EA) to give H62-a (864 mg). Yield: 75%. MS m/z (ESI): 577 [M+H]⁺.

Step 2: H62-a (864 mg, 1.5 mmol) was added to MeOH (10 mL). Palladium on carbon (150 mg, 10 wt%) was then added. After purging with hydrogen three times, the mixture was stirred at room temperature under a hydrogen atmosphere for 1 hour. After the reaction was complete, the reaction solution was filtered, and the filtrate was concentrated to give H62-b (864 mg), yield: 100%. MS m/z (ESI): 579 [M+H]⁺.

Step 3: H62-b (864 mg, 1.5 mmol) was added to a solution of trifluoroacetic acid in dichloromethane (10 mL, 30 v/v%) and stirred at room temperature for 1 hour. After the reaction was complete, the reaction solution was concentrated to give H62-c (718 mg), yield: 100%. MS m/z (ESI): 479 [M+H]⁺.

Step 4: H62-c (72 mg, 0.15 mmol) and 1-(2-(2,6-dioxopiperidin-3-yl)-1,3-dioxoisoindolin-5-yl)piperidine-4-carbaldehyde (56 mg, 0.15 mmol) were added to DMSO. Two drops of acetic acid were added, followed by NaBH(OAc)₃ (100 mg). The mixture was heated under microwave to 60°C and reacted for 45 minutes. After the reaction was complete, the reaction solution was purified by preparative HPLC (waters-sunfire-10 µm-19×150 mm column (mobile phase: 28% - 38% (v/v) acetonitrile and formic acid/water)) to give H62 (9 mg), yield: 7.2%. MS m/z (ESI): 833 [M+H]⁺. ¹H NMR (400 MHz, DMSO-d₆) δ 11.04 (s, 1H), 8.67 (s, 1H), 8.58 - 8.40 (m, 2H), 7.74 (dd, J = 8.0, 2.4 Hz, 1H), 7.34 (d, J = 8.0 Hz, 1H), 7.21 (s, 1H), 7.03 - 6.86 (m, 2H), 6.81 (d, J = 2.2 Hz, 1H), 6.65 (ddd, J = 24.0, 8.7, 3.0 Hz, 2H), 5.26 (dd, J = 12.9, 5.4 Hz, 1H), 4.70 (d, J = 4.9 Hz, 2H), 4.52 (t, J = 8.7 Hz, 2H), 3.57 (d, J = 11.7 Hz, 3H), 2.91 (dd, J = 45.5, 13.0 Hz, 3H), 2.79 - 2.54 (m, 6H), 2.20 (d, J = 6.9 Hz, 2H), 1.98 (dd, J = 13.6, 8.4 Hz, 4H), 1.93 - 1.71 (m, 6H), 1.24 (d, J = 17.7 Hz, 5H).

### Example 63 Preparation of Compound H63

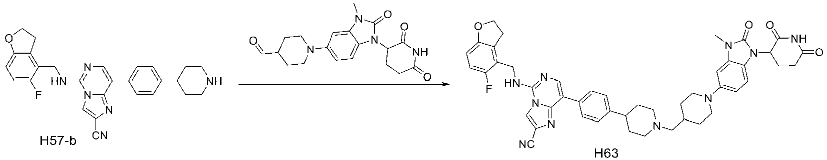

H57-b (130 mg, 277.47 µmol) and 1-[1-(2,6-dioxopiperidin-3-yl)-3-methyl-2-oxobenzimidazol-5-yl]piperidine-4-carbaldehyde (154.16 mg, 416.20 µmol) were dissolved in DCM (15 mL). NaBH(OAc)₃ (235.23 mg, 1.11 mmol) was then added, and the mixture was stirred at room temperature for 2 hours. After the reaction was complete, the reaction solution was concentrated and purified by preparative HPLC (preparative column: 21.2×250 mm C18 column; system: water + 0.04% FA/acetonitrile; wavelength: 254/214 nm; gradient: 40%-70% acetonitrile change) to give H63 (63.39 mg, purity 99.1%), yield: 27.51%. MS m/z (ESI): 823.4 [M+H]⁺. ¹H NMR (400 MHz, DMSO-d₆) δ 11.05 (s, 1H), 8.82 (s, 1H), 8.70 (s, 1H), 7.52 (s, 1H), 7.43 (d, J = 8.0 Hz, 2H), 7.33 (d, J = 8.0 Hz, 2H), 6.92 (dd, J = 12.4, 9.2 Hz, 2H), 6.80 (s, 1H), 6.69 (dd, J = 8.5, 3.7 Hz, 1H), 6.62 (d, J = 8.4 Hz, 1H), 5.27 (dd, J = 12.7, 5.0 Hz, 1H), 4.71 (s, 2H), 4.53 (t, J = 8.7 Hz, 2H), 3.57 (d, J = 11.5 Hz, 2H), 3.29 (s, 3H), 3.09 - 2.76 (m, 4H), 2.74 - 2.51 (m, 5H), 2.20 (d, J = 6.3 Hz, 2H), 2.00 (t, J = 18.4 Hz, 3H), 1.87 - 1.73 (m, 4H), 1.73 - 1.59 (m, 3H), 1.25 (d, J = 11.6 Hz, 3H).

### Example 64 Preparation of Compound H64

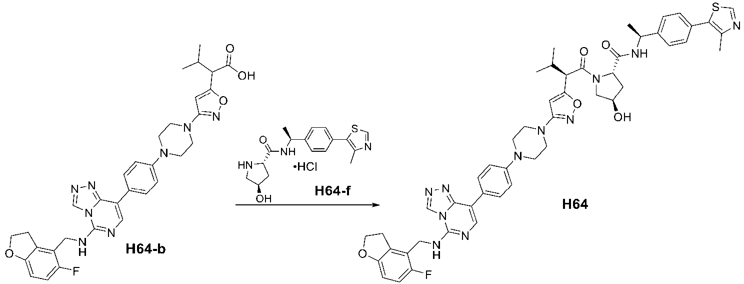

At room temperature, H64-b (40 mg, 65.29 µmol), H64-f (25.22 mg, 68.55 µmol, HCl), DIPEA (25.31 mg, 195.87 µmol, 34.12 µL), and DMF (2 mL) were mixed, then HATU (27.10 mg, 71.82 µmol) was added. After addition, the mixture was reacted at room temperature for 1 hour. Water was added to the reaction solution, followed by extraction with ethyl acetate (10 mL×2). The combined organic layers were dried over anhydrous sodium sulfate and concentrated to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give H64 (1.51 mg, 1.63 µmol, yield 2.50%) as a white solid. MS m/z (ESI): 463.8 [M/2+H]⁺. ¹H NMR (400 MHz, DMSO-d₆) δ 9.45 (s, 1H), 8.97 (s, 1H), 8.57 (s, 1H), 8.41 (d, J = 7.6 Hz, 1H), 8.00 (d, J = 8.8 Hz, 2H), 7.94 (s, 1H), 7.42 (d, J = 8.4 Hz, 2H), 7.35 (d, J = 8.2 Hz, 2H), 7.08 (d, J = 8.8 Hz, 2H), 6.96 - 6.91 (m, 1H), 6.69 (d, J = 3.6 Hz, 1H), 6.23 (s, 1H), 5.11 (d, J = 3.4 Hz, 1H), 5.03 - 4.95 (m, 1H), 4.93-4.85 (m, 2H), 4.69 (d, J = 2.4 Hz, 3H), 4.53 (t, J = 8.4 Hz, 3H), 4.35 (t, J = 8.0 Hz, 1H), 4.27 (s, 1H), 3.71 (dd, J = 11.2, 4.0 Hz, 1H), 3.59 (d, J = 10.0 Hz, 1H), 3.44-3.41 (m, 1H), 3.30-3.27 (m, 3H), 2.44 (s, 2H), 2.29 - 2.17 (m, 2H), 2.05 - 1.95 (m, 2H), 1.83 - 1.71 (m, 2H), 1.36 (d, J = 7.2 Hz, 3H), 1.21 (s, 1H), 0.94 (d, J = 6.4 Hz, 3H), 0.79 (d, J = 6.4 Hz, 3H).

### Example 65 Preparation of Compound H65

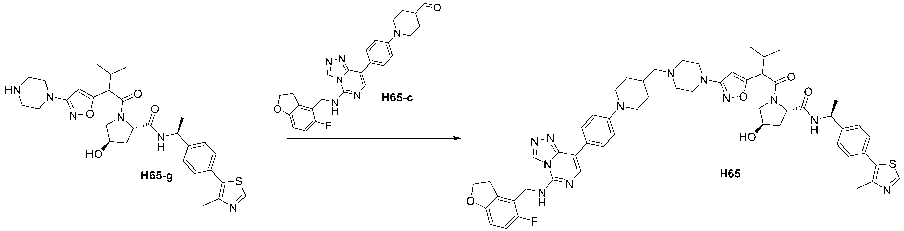

H65-c (36 mg, 76.19 µmol), H65-g (30 mg, 52.94 µmol), DMSO (1.5 mL), ethanol (0.5 mL), and AcOH (0.05 mL) were mixed and reacted under microwave at 110°C for 45 minutes. NaBH(OAc)₃ (16.63 mg, 78.48 µmol) was then added, and the mixture was reacted under microwave at 85°C for 10 minutes. Water was added dropwise to the reaction solution, and the mixture was concentrated to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give H65 (1.25 mg, 1.13 µmol, yield 2.13%) as a white solid. MS m/z (ESI): 512.3 [M/2+H]⁺. ¹H NMR (400 MHz, DMSO-d₆) δ 9.44 (s, 1H), 8.97 (s, 1H), 8.52 (s, 1H), 8.39 (d, J = 8.4 Hz, 1H), 7.94 (d, J = 8.8 Hz, 2H), 7.90 (s, 1H), 7.48 - 7.43 (m, 1H), 7.42 - 7.39 (m, 1H), 7.35 (d, J = 8.2 Hz, 1H), 7.30 (d, J = 8.2 Hz, 1H), 7.00 (d, J = 8.8 Hz, 2H), 6.96 - 6.90 (m, 1H), 6.69 (dd, J = 8.4, 3.6 Hz, 1H), 6.14 (d, J = 6.8 Hz, 1H), 5.09 (s, 1H), 5.02 - 4.93 (m, 1H), 4.92 - 4.85 (m, 1H), 4.69 (d, J = 4.4 Hz, 2H), 4.53 (t, J = 8.4 Hz, 2H), 4.38 (dt, J = 20.6, 7.8 Hz, 1H), 4.27 (s, 1H), 3.76 (d, J = 12.6 Hz, 2H), 3.72 - 3.66 (m, 1H), 3.62 - 3.51 (m, 2H), 3.46 - 3.38 (m, 1H), 3.18 - 3.09 (m, 3H), 2.70 (t, J = 11.6 Hz, 2H), 2.44 (t, J = 4.0 Hz, 3H), 2.39 - 2.35 (m, 1H), 2.19 (d, J = 6.8 Hz, 1H), 2.15 (d, J = 7.2 Hz, 1H), 2.04 - 1.97 (m, 1H), 1.93 - 1.86 (m, 3H), 1.83 - 1.73 (m, 3H), 1.72 - 1.63 (m, 3H), 1.37 - 1.33 (m, 3H), 1.22 (s, 2H), 0.95 - 0.86 (m, 3H), 0.82 - 0.73 (m, 3H).

### Example 67 Preparation of Compound H67

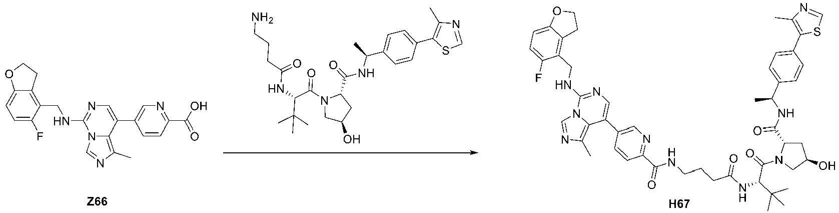

Z66 (87 mg, 207.44 µmol) and (2S,4R)-1-((S)-2-(4-aminobutanamido)-3,3-dimethylbutanoyl)-4-hydroxy-N-((S)-1-(4-(4-methylthiazol-5-yl)phenyl)ethyl)pyrrolidine-2-carboxamide (131.85 mg, 248.92 µmol) were dissolved in DMF (5 mL). DIPEA (742.00 mg, 5.74 mmol, 1 mL) and HATU (117.39 mg, 311.15 µmol) were then added, and the reaction was stirred at room temperature for 1 hour. After the reaction was complete, the solvent was concentrated under reduced pressure, and the product was purified by acidic preparation (preparative column: 21.2×250 mm C18 column; system: water + 0.04% FA/acetonitrile; wavelength: 254/214 nm; gradient: 40%-70% acetonitrile change) to successfully give product H67 (51.57 mg, 55.05 µmol, 26.54% yield) as a light yellow solid. MS m/z (ESI): 466.3 [M/2+H]⁺. ¹H NMR (400 MHz, DMSO-d₆) δ 8.97 (s, 1H), 8.92 (t, J = 6.1 Hz, 1H), 8.67 (d, J = 1.4 Hz, 1H), 8.62 (s, 1H), 8.40 - 8.32 (m, 2H), 8.08 (d, J = 8.3 Hz, 1H), 8.01 (dd, J = 8.0, 2.1 Hz, 1H), 7.94 (d, J = 9.3 Hz, 1H), 7.42 (d, J = 8.3 Hz, 2H), 7.36 (d, J = 8.3 Hz, 2H), 7.10 (s, 1H), 6.95 - 6.88 (m, 1H), 6.68 (dd, J = 8.6, 3.9 Hz, 1H), 5.10 (d, J = 3.5 Hz, 1H), 4.93 - 4.86 (m, 1H), 4.70 (d, J = 4.9 Hz, 2H), 4.52 (t, J = 8.8 Hz, 3H), 4.41 (t, J = 7.9 Hz, 1H), 4.26 (s, 1H), 3.59 (s, 2H), 3.32 - 3.22 (m, 4H), 2.44 (s, 3H), 2.28 (dd, J = 14.8, 7.5 Hz, 1H), 2.20 - 2.13 (m, 1H), 2.00 (d, J = 7.3 Hz, 4H), 1.77 (s, 3H), 1.35 (d, J = 7.0 Hz, 3H), 0.92 (s, 9H).

### Example 68 to Example 69

Compounds H68 and H69 were prepared using Z67 and Z68 as starting materials, referring to the method of Example 67.

| | |
|---|---|
| 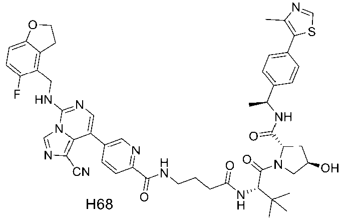 | MS m/z (ESI):471.8[M/2+H]⁺. |
| | ¹H NMR (400 MHz, DMSO-d₆) δ 8.97 (s, 1H), 8.94 (t, J = 6.1 Hz, 1H), 8.86 (s, 1H), 8.78 (d, J = 1.2 Hz, 1H), 8.37 (d, J = 7.8 Hz, 1H), 8.13 (dt, J = 15.4, 4.8 Hz, 2H), 7.93 (d, J = 9.1 Hz, 1H), 7.68 (d, J = 6.6 Hz, 1H), 7.39 (dd, J = 23.3, 8.3 Hz, 4H), 6.97 - 6.89 (m, 1H), 6.69 (dd, J = 8.6, 3.9 Hz, 1H), 5.09 (d, J = 3.6 Hz, 1H), 5.00 - 4.79 (m, 2H), 4.73 (s, 2H), 4.52 (dd, J = 16.4, 7.8 Hz, 3H), 4.41 (t, J = 8.1 Hz, 1H), 4.26 (s, 1H), 3.59 (s, 2H), 3.29 (s, 3H), 2.43 (s, 3H), 2.28 (dd, J = 14.2, 7.4 Hz, 1H), 2.16 (dd, J = 14.3, 7.8 Hz, 1H), 2.03 - 1.95 (m, 1H), 1.90 - 1.59 (m, 4H), 1.35 (d, J = 7.0 Hz, 3H), 0.93 (s, 9H). |
| 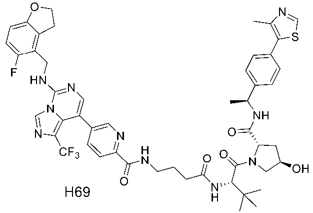 | MS m/z (ESI):493.3 [M/2+H]⁺ |
| | ¹H NMR (400 MHz, DMSO-d₆) δ 9.01 - 8.92 (m, 2H), 8.89 (s, 1H), 8.86 - 8.76 (m, 1H), 8.64 (s, 1H), 8.40 (d, *J =* 7.8 Hz, 1H), 8.09 (d, *J =* 8.0 Hz, 1H), 8.03 - 7.93 (m, 2H), 7.47 - 7.41 (m, 3H), 7.41 - 7.32 (m, 2H), 6.99 - 6.92 (m, 1H), 6.71 (dd, *J* = 8.6, 3.8 Hz, 1H), 5.12 (d, *J =* 3.5 Hz, 1H), 4.94 - 4.87 (m, 1H), 4.75 (s, 2H), 4.59 - 4.49 (m, 3H), 4.43 (t, *J* = 8.1 Hz, 1H), 4.27 (s, 1H), 3.61 (s, 2H), 3.34 - 3.24 (m, 4H), 2.44 (d, *J =* 9.8 Hz, 3H), 2.35 - 2.27 (m, 1H), 2.19 (dt, *J =* 13.8, 7.0 Hz, 1H), 2.02 (dd, *J =* 12.3, 8.3 Hz, 1H), 1.83 - 1.72 (m, 3H), 1.37 (d, *J* = 7.0 Hz, 3H), 0.95 (s, 9H). |

### Example 71 Preparation of Compound H71

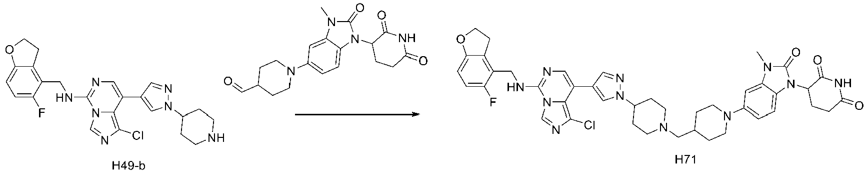

Compound H49-b (60 mg, 128.23 µmol) and 1-(1-(2,6-dioxopiperidin-3-yl)-3-methyl-2-oxo-2,3-dihydro-1H-benzo[d]imidazol-5-yl)piperidine-4-carbaldehyde (71.24 mg, 192.34 µmol) were dissolved in dichloromethane (5 mL). Sodium triacetoxyborohydride (81.53 mg, 384.68 µmol) was added with stirring at room temperature, and the mixture was stirred at room temperature overnight. The reaction solution was concentrated to dryness under reduced pressure, and the residue was purified by HPLC to give H71 (24.30 mg, 28.51 µmol, 22.23% yield, 96.47% purity) as a white solid. MS m/z (ESI): 822 [M+H]⁺. ¹H NMR (400 MHz, DMSO-d₆) δ 11.04 (s, 1H), 8.61 (s, 1H), 8.29 (t, J = 5.1 Hz, 1H), 7.94 (s, 1H), 7.57 (s, 1H), 7.15 (s, 1H), 6.92 (t, J = 8.7 Hz, 2H), 6.81 (d, J = 1.9 Hz, 1H), 6.64 (ddd, J = 10.6, 8.7, 2.9 Hz, 2H), 5.27 (dd, J = 12.8, 5.4 Hz, 1H), 4.67 (d, J = 4.8 Hz, 2H), 4.52 (t, J = 8.7 Hz, 2H), 4.16 (s, 1H), 3.57 (d, J = 12.0 Hz, 2H), 3.28 (dd, J = 17.4, 8.8 Hz, 8H), 2.89 (dd, J = 33.2, 13.5 Hz, 3H), 2.69 - 2.54 (m, 3H), 2.20 (d, J = 6.9 Hz, 2H), 2.01 (dt, J = 20.7, 9.6 Hz, 5H), 1.80 (d, J = 12.4 Hz, 2H), 1.63 (s, 1H), 1.24 (d, J = 11.4 Hz, 2H).

### Example 72 Preparation of Compound H72

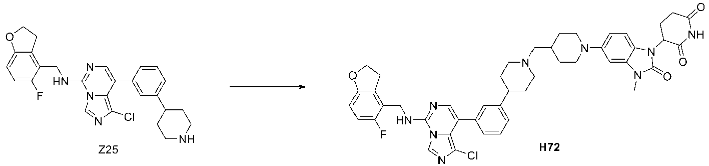

Compound H72 was prepared referring to the method of Example 71. MS m/z (ESI): 833 [M+H]⁺. ¹H NMR (400 MHz, DMSO-d₆) δ 8.66 (s, 1H), 8.38 (s, 1H), 7.33 (t, J = 7.5 Hz, 1H), 7.28 - 7.17 (m, 3H), 7.15 (s, 1H), 6.96 - 6.86 (m, 2H), 6.80 (d, J = 1.9 Hz, 1H), 6.68 (dd, J = 8.6, 3.8 Hz, 1H), 6.64 - 6.56 (m, 1H), 5.26 (dd, J = 12.7, 5.4 Hz, 1H), 4.69 (s, 2H), 4.52 (t, J = 8.7 Hz, 2H), 3.56 (d, J = 12.2 Hz, 2H), 3.38 (s, 2H), 3.29 (d, J = 3.6 Hz, 4H), 2.89 (dd, J = 34.1, 14.3 Hz, 3H), 2.72 - 2.53 (m, 4H), 2.17 (d, J = 7.1 Hz, 2H), 1.96 (t, J = 10.1 Hz, 3H), 1.82 - 1.59 (m, 6H), 1.35 - 1.15 (m, 3H).

### Example 73 Preparation of Compound H73

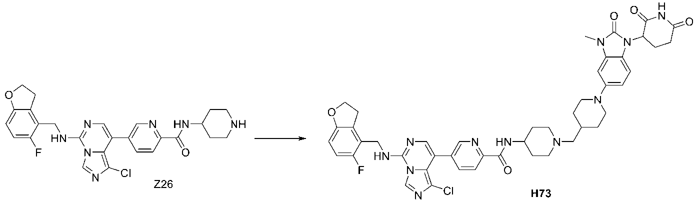

Compound Z26 (78 mg, 0.15 mmol) and 1-(1-(2,6-dioxopiperidin-3-yl)-3-methyl-2-oxo-2,3-dihydro-1H-benzo[d]imidazol-5-yl)piperidine-4-carbaldehyde (56 mg, 0.15 mmol) were added to DMSO. Two drops of acetic acid were added, followed by 25 mg of sodium triacetoxyborohydride. The resulting mixture was heated under microwave at 60°C for 45 minutes. The resulting mixture solution was then sent for purification, and the filtrate was purified by high pressure liquid chromatography (waters-sunfire-10 µm-19×150 mm column (eluent: 28% to 38% (v/v) acetonitrile and aqueous formic acid)) to give compound H73 (10 mg, 11.4 µmol). Yield: 7.6%. MS m/z (ESI): 876 [M+H]⁺. ¹H NMR (400 MHz, DMSO-d₆) δ 11.04 (s, 1H), 8.75 - 8.51 (m, 4H), 8.17 - 7.99 (m, 2H), 7.30 (s, 1H), 6.98 - 6.85 (m, 2H), 6.80 (d, J = 2.2 Hz, 1H), 6.75 - 6.53 (m, 2H), 4.72 (d, J = 4.9 Hz, 2H), 4.53 (t, J = 8.7 Hz, 3H), 3.82 (s, 1H), 3.57 (d, J = 11.7 Hz, 3H), 2.90-2.86 (m, 4H), 2.61 (t, J = 12.9 Hz, 7H), 2.27 (d, J = 35.0 Hz, 3H), 2.17 - 1.88 (m, 3H), 1.90 - 1.52 (m, 4H), 1.24 (d, J = 13.7 Hz, 4H).

### Example 74 Preparation of Compound H74

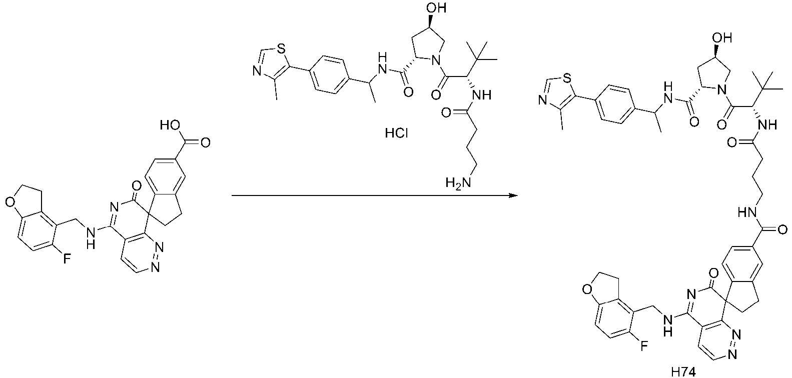

Compound 5'-[(5-fluoro-2,3-dihydrobenzofuran-4-yl)methylamino]-7'-oxo-spiro[indene-1,8'-pyrido[4,3-c]pyridazine]-5-carboxylic acid (50 mg, 109.07 µmol) and rac-(2S,4R)-4-hydroxy-N-[1-[4-(4-methylthiazol-5-yl)phenyl]ethyl]-1-[rac-(2S)-2-(4-aminobutanamid o)-3,3-dimethylbutanoyl]pyrrolidine-2-carboxamide (86.66 mg, 163.60 µmol) were dissolved in tetrahydrofuran (5 mL) and N-methylpyrrolidone (1 mL) and stirred at room temperature for 2 hours. The reaction solution was poured into methanol, concentrated to dryness under reduced pressure, and purified by silica gel column chromatography (MeOH:DCM = 0-10%) to give a crude product. This was further purified by HPLC separation: basic method: (preparative column: 21.2×250 mm C18 column; system: 10 mM NH₄HCO₃/H₂O/acetonitrile; wavelength: 254/214 nm; gradient: 0%-70% acetonitrile change) to give H74 (1.77 mg, 1.67 µmol, 1.53% yield, 91.28% purity) as a white solid. MS m/z (ESI): 485 [M/2+H]⁺. ¹H NMR (400 MHz, DMSO-d₆) δ 11.03 (s, 1H), 8.97 (s, 1H), 8.34 (dd, J = 19.6, 6.4 Hz, 2H), 8.13 (d, J = 5.8 Hz, 1H), 7.87 (d, J = 8.7 Hz, 1H), 7.79 (d, J = 21.0 Hz, 1H), 7.54 (d, J = 7.9 Hz, 1H), 7.39 (dd, J = 23.2, 8.3 Hz, 4H), 6.89 (dd, J = 37.1, 8.5 Hz, 2H), 6.69 (s, 2H), 5.09 (d, J = 3.1 Hz, 1H), 4.95 - 4.33 (m, 10H), 3.59 (s, 2H), 2.99 (dd, J = 37.9, 7.0 Hz, 2H), 2.44 (s, 3H), 2.34 - 2.15 (m, 3H), 2.01 - 1.92 (m, 2H), 1.73 (d, J = 27.0 Hz, 3H), 1.38 (t, J = 17.6 Hz, 4H), 0.92 (s, 9H).

### Example 80 Preparation of Compound H80

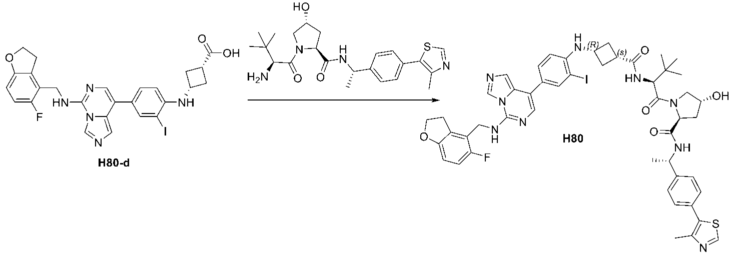

At room temperature, H80-d (43 mg, 71.74 µmol), (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxami de (29.77 mg, 78.91 µmol), DIPEA (18.54 mg, 143.48 µmol, 24.99 µL), and DMF (2 mL) were stirred for 5 minutes. HATU (35.08 mg, 78.91 µmol) was then added, and after addition, the mixture was reacted at room temperature for 30 minutes. The reaction solution was poured into ice water, extracted with ethyl acetate (5 mL×2). The combined organic layers were dried over anhydrous sodium sulfate and concentrated to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give compound H80 (20.97 mg, 18.91 µmol, yield 26.36%) as a white solid. MS m/z (ESI): 513.7 [M/2+H]⁺. ¹H NMR (400 MHz, DMSO-d₆) δ 8.97 (s, 1H), 8.66 (s, 1H), 8.40 (d, J = 7.6 Hz, 1H), 8.18 (t, J = 5.0 Hz, 1H), 7.79 (d, J = 9.2 Hz, 1H), 7.42 (d, J = 8.0 Hz, 2H), 7.36 (d, J = 8.0 Hz, 2H), 7.03 (d, J = 8.4 Hz, 2H), 6.96 (s, 1H), 6.94 - 6.89 (m, 1H), 6.67 (dd, J = 8.6, 3.8 Hz, 1H), 6.53 (d, J = 8.4 Hz, 2H), 6.04 (d, J = 7.0 Hz, 1H), 5.12 (d, J = 3.4 Hz, 1H), 4.93 - 4.86 (m, 1H), 4.66 (d, J = 4.8 Hz, 2H), 4.54 - 4.48 (m, 3H), 4.40 (t, J = 8.0 Hz, 1H), 4.28-4.24 (m, 1H), 3.79-3.69 (m, 1H), 3.63 - 3.55 (m, 2H), 3.26 (t, J = 8.6 Hz, 2H), 2.99-2.91 (m, 1H), 2.55-2.51 (m, 1H), 2.45-2.37 (m, 4H), 2.05 - 1.90 (m, 3H), 1.79 - 1.73 (m, 1H), 1.35 (d, J = 7.0 Hz, 3H), 0.91 (s, 9H).

### Examples 75-76, 78-79

Compounds H75, H76, H78, H79 were prepared referring to the above methods.

| | |
|---|---|
| 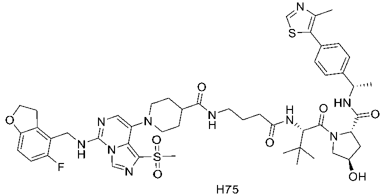 | MS m/z (ESI): 1001.4 [M+H]⁺ |
| 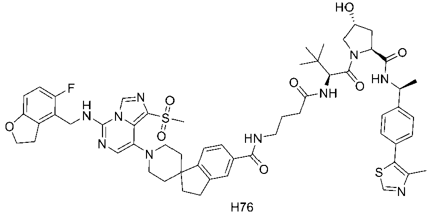 | MS m/z (ESI): 1103.1 [M+H]⁺ |
| 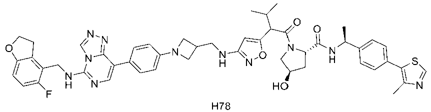 | MS m/z (ESI): 926.4[M+Hl⁺ |
| 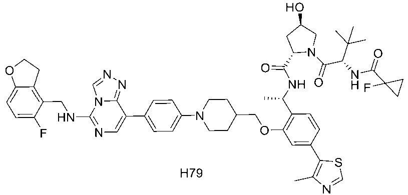 | MS m/z (ESI): 1003.4 [M+H]⁺ |

### Example 86 Preparation of Compounds H86 and H66

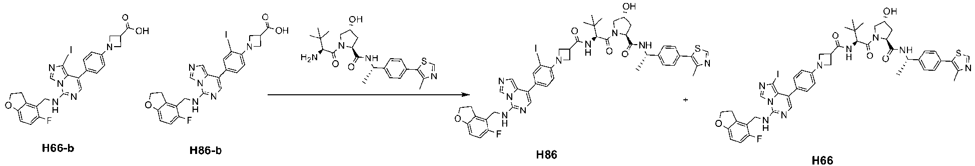

The mixture of H86-b and H66-b and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (50.13 mg, 112.75 µmol) were dissolved in DMF (6 mL). DIPEA (194.29 mg, 1.50 mmol, 261.85 µL) and HATU (113.43 mg, 300.67 µmol) were then added, and the reaction was stirred at room temperature for 1 hour. The reaction solution was then directly subjected to purification (preparative column: 21.2×250 mm C18 column; system: 10 mM NH₄HCO₃/H₂O/acetonitrile; wavelength: 254/214 nm; gradient: 0%-70% acetonitrile change) to successfully give white solid product H86 (5.98 mg, 5.84 µmol, 7.76% yield); MS m/z (ESI): 506.7 [M/2+H]⁺. ¹H NMR (400 MHz, DMSO-d₆) δ 8.97 (s, 1H), 8.68 (s, 1H), 8.42 (d, J = 7.8 Hz, 1H), 8.23 (s, 1H), 8.14 (d, J = 9.2 Hz, 1H), 7.42 (d, J = 8.2 Hz, 2H), 7.36 (d, J = 8.2 Hz, 2H), 7.14 (d, J = 8.3 Hz, 2H), 6.98 (s, 1H), 6.95 - 6.88 (m, 1H), 6.67 (dd, J = 8.6, 3.8 Hz, 1H), 6.46 (d, J = 8.3 Hz, 2H), 5.12 (d, J = 3.2 Hz, 1H), 4.94 - 4.85 (m, 1H), 4.66 (d, J = 4.5 Hz, 2H), 4.52 (dd, J = 15.1, 6.5 Hz, 3H), 4.41 (t, J = 8.1 Hz, 1H), 4.26 (s, 1H), 4.00 (t, J = 7.4 Hz, 1H), 3.94 (t, J = 7.2 Hz, 1H), 3.82 (t, J = 6.5 Hz, 1H), 3.73 (dt, J = 15.7, 7.5 Hz, 2H), 3.59 (s, 2H), 3.27 (t, J = 8.6 Hz, 2H), 2.43 (s, 3H), 1.99 (dd, J = 16.0, 7.0 Hz, 1H), 1.76 (dd, J = 13.8, 9.8 Hz, 1H), 1.35 (d, J = 6.9 Hz, 3H), 0.93 (s, 9H).

And white solid product H66 (2.37 mg, 1.83 µmol, 2.44% yield). MS m/z (ESI): 506.7 [M/2+H]⁺. ¹H NMR (400 MHz, DMSO-d₆) δ 8.97 (s, 1H), 8.67 (s, 1H), 8.44 (d, J = 7.3 Hz, 1H), 8.20 (s, 1H), 7.74 (d, J = 8.8 Hz, 1H), 7.38 (dd, J = 26.9, 8.3 Hz, 4H), 7.06 (d, J = 8.4 Hz, 2H), 6.98 (s, 1H), 6.95 - 6.88 (m, 1H), 6.67 (dd, J = 8.6, 3.8 Hz, 1H), 6.63 (d, J = 8.3 Hz, 1H), 6.10 (s, 1H), 5.85 (s, 1H), 5.52 (s, 1H), 5.15 (d, J = 3.4 Hz, 1H), 4.93 - 4.85 (m, 1H), 4.66 (d, J = 4.5 Hz, 2H), 4.60 (d, J = 9.0 Hz, 1H), 4.51 (t, J = 8.7 Hz, 2H), 4.42 (t, J = 8.2 Hz, 1H), 4.28 (s, 1H), 3.94 (s, 2H), 3.61 (s, 2H), 3.29 - 3.23 (m, 2H), 2.43 (s, 3H), 2.01 (s, 2H), 1.74 (d, J = 8.5 Hz, 1H), 1.34 (d, J = 6.9 Hz, 3H), 0.94 (s, 9H).

### Example 87 Preparation of Compounds H87 and H70

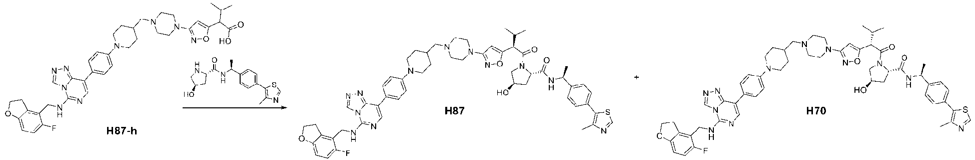

At room temperature, HATU (222.17 mg, 588.89 µmol) was added to a solution of H87-h (380 mg, 535.35 µmol), (2S,4R)-4-hydroxy-N-((S)-1-(4-(4-methylthiazol-5-yl)phenyl)ethyl)pyrrolidine-2-carboxamide (206.80 mg, 562.12 µmol), DIPEA (207.57 mg, 1.61 mmol, 279.75 µL), and DMF (10 mL). After addition, the mixture was reacted at room temperature for 1 hour. The reaction was quenched with water, and the reaction solution was concentrated to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give 210 mg of a white solid. 96 mg of this was submitted for SFC to separately give: H87 (35.16 mg, 34.36 µmol, yield 36.63%) as a white solid. MS m/z (ESI): 512.3 [M+H]⁺. ¹H NMR (400 MHz, DMSO-d₆) δ 9.45 (s, 1H), 8.97 (s, 1H), 8.52 (t, J = 5.2 Hz, 1H), 8.19 (d, J = 7.8 Hz, 1H), 7.94 (d, J = 8.8 Hz, 2H), 7.90 (s, 1H), 7.46 (d, J = 8.4 Hz, 1H), 7.42-7.38 (m, 2H), 7.30 (d, J = 8.2 Hz, 1H), 7.00 (d, J = 9.0 Hz, 2H), 6.96-6.90 (m, 1H), 6.69 (dd, J = 8.6, 4.0 Hz, 1H), 6.15 (s, 1H), 5.09 (d, J = 3.6 Hz, 1H), 4.90-4.83 (m, 1H), 4.69 (d, J = 4.8 Hz, 2H), 4.52 (t, J = 8.8 Hz, 2H), 4.40 (t, J = 7.6 Hz, 1H), 4.29-4.22 (m, 1H), 3.75 (d, J = 12.4 Hz, 2H), 3.68 (d, J = 8.4 Hz, 1H), 3.53 (d, J = 3.2 Hz, 1H), 3.44-3.39 (m, 1H), 3.29-3.27 (m, 4H), 3.19-3.16 (m, 1H), 3.14-3.08 (m, 2H), 2.69 (t, J = 12.0 Hz, 2H), 2.43 (s, 3H), 2.39-2.34 (m, 2H), 2.28-2.18 (m, 2H), 2.15 (d, J = 7.2 Hz, 1H), 2.05-1.99 (m, 1H), 1.83-1.74 (m, 3H), 1.68 (s, 1H), 1.34 (d, J = 7.0 Hz, 3H), 1.27-1.16 (m, 3H), 0.94 (d, J = 6.6 Hz, 3H), 0.83-0.78 (d, J = 6.8 Hz, 3H).

And H70 (29.48 mg, 28.81 µmol, yield 30.71%) as a white solid. MS m/z (ESI): 512.4 [M+H]⁺. ¹H NMR (400 MHz, DMSO-d₆) δ 9.45 (s, 1H), 8.97 (s, 1H), 8.54 (t, J = 5.0 Hz, 1H), 8.39 (d, J = 7.6 Hz, 1H), 7.94 (d, J = 8.8 Hz, 2H), 7.90 (s, 1H), 7.42 (d, J = 8.0 Hz, 2H), 7.35 (d, J = 8.4 Hz, 2H), 7.00 (d, J = 9.0 Hz, 2H), 6.96 - 6.90 (m, 1H), 6.69 (dd, J = 8.8, 3.8 Hz, 1H), 6.13 (s, 1H), 5.10 (d, J = 3.6 Hz, 1H), 4.93-4.86 (m, 1H), 4.69 (d, J = 4.2 Hz, 2H), 4.52 (t, J = 8.6 Hz, 2H), 4.35 (t, J = 7.8 Hz, 1H), 4.29 - 4.23 (m, 1H), 3.76 (d, J = 12.4 Hz, 2H), 3.70 (dd, J = 10.8, 4.4 Hz, 1H), 3.56 (d, J = 10.0 Hz, 1H), 3.41 (d, J = 9.6 Hz, 1H), 3.28 (d, J = 8.8 Hz, 2H), 3.20 - 3.11 (m, 4H), 2.70 (t, J = 11.6 Hz, 2H), 2.42 (s, 6H), 2.24 - 2.15 (m, 3H), 2.03 - 1.97 (m, 1H), 1.82 - 1.67 (m, 4H), 1.36 (d, J = 7.0 Hz, 3H), 1.27 - 1.17 (m, 3H), 0.93 (d, J = 6.4 Hz, 3H), 0.78 (d, J = 6.8 Hz, 3H).

### Example 88 Preparation of Compound H88

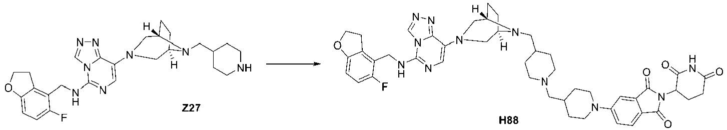

Compound H88 was prepared referring to the method of Example 73. MS m/z (ESI): 846 [M+H]⁺. ¹H NMR (400 MHz, DMSO-d₆) δ 11.06 (s, 1H), 9.33 (s, 1H), 7.93 (s, 1H), 7.63 (d, J = 8.5 Hz, 1H), 7.35 - 7.12 (m, 2H), 6.98 - 6.84 (m, 2H), 6.66 (dd, J = 8.7, 3.8 Hz, 1H), 5.04 (dd, J = 13.0, 5.4 Hz, 1H), 4.64 - 4.45 (m, 4H), 4.01 (d, J = 12.7 Hz, 2H), 3.66 (d, J = 10.2 Hz, 2H), 3.26 - 3.14 (m, 4H), 2.97 - 2.74 (m, 7H), 2.62 (d, J = 27.5 Hz, 2H), 2.13 (dd, J = 29.1, 6.7 Hz, 4H), 2.06 - 1.94 (m, 2H), 1.80 (dq, J = 26.2, 12.6 Hz, 12H), 1.37 (s, 1H), 1.11 (d, J = 12.1 Hz, 2H).

### Example 89 Preparation of Compound H89

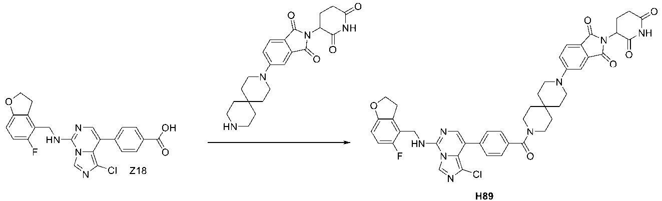

Compound Z18 (100 mg, 227.87 µmol) and 2-(2,6-dioxopiperidin-3-yl)-5-(3,9-diazaspiro[5.5]undecan-3-yl)isoindoline-1,3-dione (102.89 mg, 250.66 µmol) were dissolved in DMF (5 mL). HATU (171.94 mg, 455.75 µmol) and DIPEA (117.80 mg, 911.50 µmol, 158.77 µL) were added, and the mixed solution was stirred at room temperature (25°C) for 2 hours, and the starting material was completely reacted. The reaction solution was concentrated to dryness under reduced pressure to give a crude product. The crude product was purified by basic HPLC (preparative column: 21.2×250 mm C18 column; system: 10 mM NH₄HCO₃/H₂O/acetonitrile; wavelength: 254/214 nm; gradient: 60%-65% acetonitrile change) to give white solid product H89 (51.80 mg, yield: 27.27%, purity: 99.72%). MS m/z (ESI): 831.3 [M+H]⁺. ¹H NMR (400 MHz, DMSO-d₆) δ 11.06 (s, 1H), 8.67 (s, 1H), 8.51 - 8.39 (m, 1H), 7.63 (d, J = 8.5 Hz, 1H), 7.44 (q, J = 8.2 Hz, 4H), 7.33 - 7.27 (m, 1H), 7.25 - 7.16 (m, 2H), 6.92 (t, J = 9.4 Hz, 1H), 6.68 (dd, J = 8.7, 3.8 Hz, 1H), 5.05 (dd, J = 12.9, 5.4 Hz, 1H), 4.69 (d, J = 4.9 Hz, 2H), 4.53 (t, J = 8.7 Hz, 2H), 3.55 (dd, J = 62.4, 11.2 Hz, 7H), 3.28 (d, J = 8.8 Hz, 3H), 2.94 - 2.80 (m, 1H), 2.68 - 2.50 (m, 2H), 2.00 (d, J = 6.1 Hz, 1H), 1.56 (d, J = 25.3 Hz, 8H).

### Example 90 Preparation of Compound H90

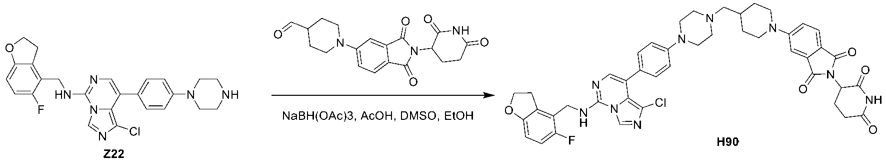

Compound H90 was prepared referring to the method of Example 73. MS m/z (ESI): 416.8 [M/2+H]⁺. ¹H NMR (400 MHz, DMSO-d₆) δ 11.07 (s, 1H), 8.63 (s, 1H), 8.29 (t, J = 5.2 Hz, 1H), 7.64 (d, J = 8.4 Hz, 1H), 7.30 (s, 1H), 7.28 - 7.19 (m, 3H), 7.07 (d, J = 1.2 Hz, 1H), 6.93 (dd, J = 18.8, 9.2 Hz, 3H), 6.68 (dd, J = 8.8, 4.0 Hz, 1H), 5.05 (dd, J = 12.8, 5.6 Hz, 1H), 4.68 (d, J = 4.8 Hz, 2H), 4.52 (t, J = 8.8 Hz, 2H), 4.04 (d, J = 12.8 Hz, 2H), 3.28 (t, J = 8.4 Hz, 3H), 3.17 (d, J = 6.0 Hz, 4H), 2.96 (t, J = 12.4 Hz, 2H), 2.85 (d, J = 12.4 Hz, 1H), 2.62 - 2.50 (m, 4H), 2.19 (d, J = 6.8 Hz, 2H), 2.05 - 1.95 (m, 1H), 1.83 (t, J = 15.2 Hz, 3H), 1.24 - 1.07 (m, 2H).

### Example 91 Preparation of Compound H91

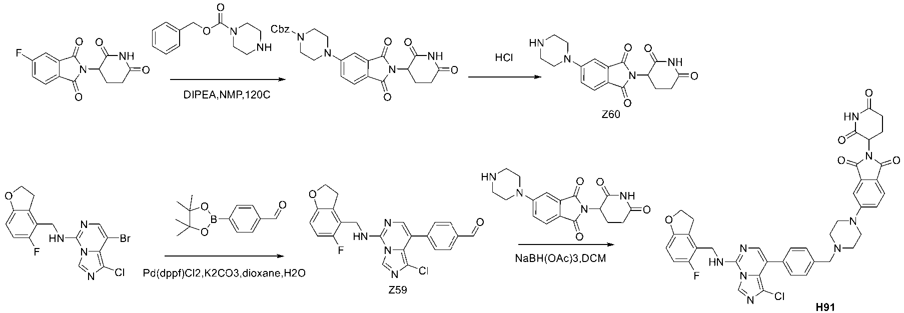

Compound H91 was prepared according to the above synthetic method. MS m/z (ESI): 749 [M+H]⁺. ¹H NMR (400 MHz, DMSO-d₆) δ 11.06 (s, 1H), 8.66 (s, 1H), 8.39 (t, J = 4.9 Hz, 1H), 8.25 (s, 1H), 7.66 (d, J = 8.5 Hz, 1H), 7.37 (s, 3H), 7.32 (s, 1H), 7.24 (d, J = 8.7 Hz, 1H), 7.16 (s, 1H), 6.98 - 6.87 (m, 1H), 6.68 (dd, J = 8.6, 3.8 Hz, 1H), 5.05 (dd, J = 12.9, 5.4 Hz, 1H), 4.69 (d, J = 4.8 Hz, 2H), 4.52 (t, J = 8.7 Hz, 2H), 3.57 (s, 2H), 3.49-3.42 (m, 4H), 3.29 (t, J = 8.7 Hz, 3H), 2.92 - 2.80 (m, 1H), 2.56 (d, J = 23.9 Hz, 5H), 2.05 - 1.95 (m, 1H).

### Example 92 Preparation of Compound H92

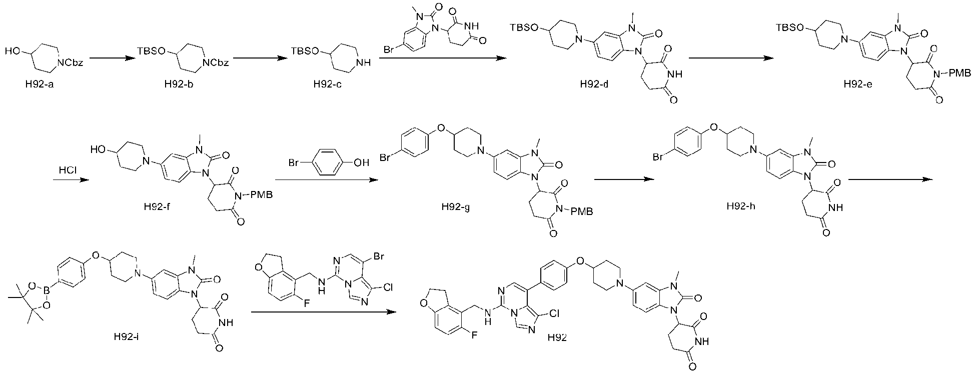

Step 1: H92-a (10 g, 42.50 mmol) and imidazole (8.68 g, 127.51 mmol) were dissolved in DCM (100 mL). TBSCl (7.05 g, 46.75 mmol) was added, and the mixture was stirred at room temperature overnight. Water was added, and then the mixture was extracted three times with DCM. The combined organic phases were washed with brine, dried over anhydrous sodium sulfate, and concentrated under reduced pressure to give a crude product. The residue was separated by CombiFlash column chromatography (120 g, 0-20% DCM/PE) to give H92-b (13 g, 37.19 mmol, 87.50% yield) as a colorless oil. MS m/z (ESI): 350.2 [M+H]⁺.

Step 2: H92-b (5 g, 14.30 mmol) was dissolved in THF (100 mL). Palladium on carbon (1.52 g, 1.43 mmol, 10% purity) was added, and the mixture was purged with a hydrogen balloon three times and stirred at room temperature overnight. The catalyst was filtered off, and the mother liquor was concentrated under reduced pressure to give crude product H92-c (3 g, 13.93 mmol, 97.36% yield) as a light brown oil. It was used directly in the next step without purification. MS m/z (ESI): 216.2 [M+H]⁺.

Step 3: 3-(5-Bromo-3-methyl-2-oxo-2,3-dihydro-1H-benzo[d]imidazol-1-yl)piperidine-2,6-dione (2 g, 5.91 mmol) and H92-c (2.55 g, 11.83 mmol) were dissolved in toluene (40 mL). Under argon protection, Ruphos-Pd-G₃ (495.26 mg, 591.44 µmol) and Ruphos (551.97 mg, 1.18 mmol) were added. The mixture was purged with argon three times, then LiHMDS (1 M, 29.57 mL) was added. The temperature was raised to 80°C and stirred for 2 hours. After cooling to room temperature, water and ethyl acetate were added, extraction was performed three times with ethyl acetate, the combined organic phases were washed with brine, dried over anhydrous sodium sulfate, and concentrated under reduced pressure to give a crude product. The residue was separated by CombiFlash column chromatography (20 g, 0-100% EA/PE) to give H92-d (2 g, 4.23 mmol, 71.54% yield) as a light brown solid. MS m/z (ESI): 473.3 [M+H]⁺.

Step 4: H92-d (1.4 g, 2.96 mmol) and K₂CO₃ (1.23 g, 8.89 mmol) were dissolved in DMF (15 mL). 4-Methoxybenzyl chloride (927.76 mg, 5.92 mmol, 803.25 µL) was added, and the mixture was stirred at room temperature overnight. Water and ethyl acetate were added, extraction was performed three times with ethyl acetate, the combined organic phases were washed with brine, dried over anhydrous sodium sulfate, and concentrated under reduced pressure to give a crude product. The residue was separated by CombiFlash column chromatography (12 g, 0-60% EA/PE) to give H92-e (1.2 g, 2.02 mmol, 68.34% yield) as a light yellow oil. MS m/z (ESI): 593.3 [M+H]⁺.

Step 5: H92-e (1.2 g, 2.02 mmol) was dissolved in HCl/dioxane (4 M) (15 mL) and stirred at room temperature for 1 hour. Water and ethyl acetate were added, extraction was performed three times with ethyl acetate, the combined organic phases were washed with brine, dried over anhydrous sodium sulfate, and concentrated under reduced pressure to give a crude product. The residue was separated by CombiFlash column chromatography (12 g, 0-10% MeOH/DCM) to give H92-f (0.6 g, 1.25 mmol, 61.94% yield) as a light yellow oil. MS m/z (ESI): 479.3 [M+H]⁺.

Step 6: H92-f (0.5 g, 1.04 mmol), 4-bromophenol (216.92 mg, 1.25 mmol), and triphenylphosphine (548.10 mg, 2.09 mmol) were dissolved in DCM (10 mL). Under argon protection, the temperature was lowered to 0°C. DIAD (633.18 mg, 3.13 mmol) was added dropwise. The mixture was stirred at room temperature overnight. The solvent was concentrated under reduced pressure, water and ethyl acetate were added, extraction was performed three times with ethyl acetate, the combined organic phases were washed with brine, dried over anhydrous sodium sulfate, and concentrated under reduced pressure to give a crude product. The residue was separated by CombiFlash column chromatography (12 g, 0-60% EA/PE) to give H92-g (0.4 g, 631.38 µmol, 60.43% yield) as a light yellow solid. MS m/z (ESI): 633.2 [M+H]⁺.

Step 7: H92-g (0.4 g, 631.38 µmol) and methanesulfonic acid (606.79 mg, 6.31 mmol) were dissolved in toluene (10 mL), and the temperature was raised to 110°C and stirred for 16 hours. The solvent was concentrated under reduced pressure, the pH was adjusted to 7 with saturated aqueous sodium bicarbonate solution in an ice bath, extraction was performed three times with ethyl acetate, the combined organic phases were washed with brine, dried over anhydrous sodium sulfate, and concentrated under reduced pressure to give a crude product. The residue was separated by CombiFlash column chromatography (4 g, 0-8% MeOH/DCM) to give H92-h (170 mg, 331.14 µmol, 52.45% yield) as a light brown solid. MS m/z (ESI): 513.1 [M+H]⁺.

Step 8: H92-h (170 mg, 331.14 µmol) and bis(pinacolato)diboron (168.18 mg, 662.27 µmol) were dissolved in dioxane (5 mL). Under argon protection, potassium acetate (105.30 mg, 993.41 µmol) and Pd(dppf)Cl₂ (24.23 mg, 33.11 µmol) were added. The temperature was raised to 90°C and stirred overnight. The solvent was concentrated under reduced pressure to give a crude product. The residue was separated by CombiFlash column chromatography (4 g, 0-8% MeOH/DCM) to give H92-i (180 mg, 321.17 µmol, 96.99% yield) as a light brown solid. MS m/z (ESI): 561.3 [M+H]⁺.

Step 9: H92-i (70 mg, 176.04 µmol) and 8-bromo-1-chloro-N-((5-fluoro-2,3-dihydrobenzofuran-4-yl)methyl)imidazo[1,5-c]pyrimidin-5-amine (118.40 mg, 211.25 µmol) were dissolved in dioxane (5 mL) and water (0.5 mL). Under argon protection, Pd(aMphos)Cl₂ (25.00 mg, 35.21 µmol) and cesium fluoride (80.22 mg, 528.13 µmol) were added. The temperature was raised to 80°C and stirred for 2 hours. Water and ethyl acetate were added, extraction was performed three times with ethyl acetate, the combined organic phases were washed with brine, dried over anhydrous sodium sulfate, and concentrated under reduced pressure to give a crude product. The residue was separated by CombiFlash column chromatography (4 g, 0-10% MeOH/DCM) to give a crude product, which was then purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give H92 (6.52 mg, 8.16 µmol, 4.63% yield, 93.99% purity) as a white solid. MS m/z (ESI): 751.3 [M+H]⁺. ¹H NMR (400 MHz, DMSO-d₆) δ 11.05 (s, 1H), 8.64 (s, 1H), 8.32 (t, J = 5.2 Hz, 1H), 7.31 (d, J = 8.4 Hz, 2H), 7.10 (s, 1H), 7.03 (d, J = 8.4 Hz, 2H), 6.92 (t, J = 9.2 Hz, 2H), 6.87 (d, J = 2.0 Hz, 1H), 6.72 - 6.64 (m, 2H), 5.28 (dd, J = 12.8, 5.2 Hz, 1H), 4.68 (d, J = 4.8 Hz, 2H), 4.58 (s, 1H), 4.52 (t, J = 8.8 Hz, 2H), 3.51 - 3.41 (m, 2H), 3.29 (d, J = 4.4 Hz, 5H), 3.00 (t, J = 10.0 Hz, 2H), 2.87 (t, J = 14.8 Hz, 1H), 2.73 - 2.58 (m, 2H), 2.07 (d, J = 11.6 Hz, 2H), 2.03 - 1.92 (m, 1H), 1.85-1.72 (m, 2H).

### Example 93 Preparation of Compound H93

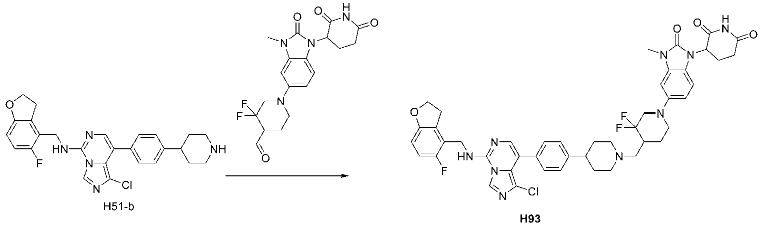

Compound H93 was prepared referring to the method of Example 71. MS m/z (ESI): 868.3 [M+H]⁺. ¹H NMR (400 MHz, DMSO-d₆) δ 11.04 (s, 1H), 8.65 (d, J = 1.4 Hz, 1H), 8.34 (t, J = 5.2 Hz, 1H), 7.31 (q, J = 8.0 Hz, 4H), 7.13 (d, J = 1.4 Hz, 1H), 6.98 - 6.86 (m, 3H), 6.68 (dt, J = 9.2, 4.3 Hz, 2H), 5.28 (dd, J = 12.9, 5.3 Hz, 1H), 4.69 (d, J = 4.9 Hz, 2H), 4.53 (t, J = 8.7 Hz, 2H), 3.76 (s, 1H), 3.59 (d, J = 12.1 Hz, 1H), 3.27 (s, 1H), 3.11 - 2.98 (m, 2H), 2.97 - 2.70 (m, 4H), 2.63 (dd, J = 25.0, 14.8 Hz, 4H), 2.44 - 2.15 (m, 3H), 2.15 - 1.86 (m, 4H), 1.79 (s, 2H), 1.75 - 1.49 (m, 3H), 1.22 (s, 2H).

### Example 94 Preparation of Compound H94

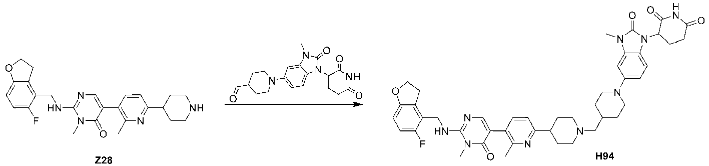

Compound H94 was prepared referring to the method of Example 71. MS m/z (ESI): 804.4 [M+H]⁺. ¹H NMR (400 MHz, DMSO-d₆) δ 11.05 (s, 1H), 7.64 (d, J = 2.4 Hz, 2H), 7.39 (d, J = 7.6 Hz, 1H), 7.06 (d, J = 8.0 Hz, 1H), 6.94 - 6.86 (m, 2H), 6.81 (d, J = 2.0 Hz, 1H), 6.68 - 6.59 (m, 2H), 5.27 (dd, J = 12.8, 5.4 Hz, 1H), 4.59 - 4.48 (m, 4H), 3.57 (d, J = 11.6 Hz, 2H), 3.34 (s, 3H), 3.28 (d, J = 7.6 Hz, 6H), 2.99 - 2.81 (m, 3H), 2.72 - 2.54 (m, 5H), 2.28 (s, 3H), 2.20 (d, J = 7.2 Hz, 2H), 1.97 (d, J = 10.0 Hz, 3H), 1.83 - 1.62 (m, 7H), 1.25 (q, J = 11.2, 10.4 Hz, 2H).

### Example 95 Preparation of Compound H95

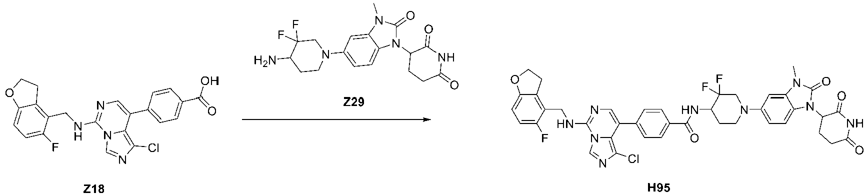

Z18 (100 mg, 227.87 µmol) and Z29 (116.54 mg, 296.24 µmol) were dissolved in DMF (5 mL). DIPEA (147.26 mg, 1.14 mmol, 198.46 µL) and HATU (171.94 mg, 455.75 µmol) were then added, and the reaction was stirred at room temperature for 1 hour. After the reaction was complete, the solvent was concentrated under reduced pressure, and the product was purified by preparation (preparative column: 21.2×250 mm C18 column; system: 10 mM NH₄HCO₃/H₂O/acetonitrile; wavelength: 254/214 nm; gradient: 0%-60% acetonitrile change) to successfully give white solid product H95 (53.90 mg, 62.93 µmol, 27.62% yield). MS m/z (ESI): 814.2 [M+H]⁺. ¹H NMR (400 MHz, DMSO-d₆) δ 11.06 (s, 1H), 8.69 (s, 1H), 8.61 (d, J = 8.8 Hz, 1H), 8.48 (t, J = 4.9 Hz, 1H), 7.96 (d, J = 8.3 Hz, 2H), 7.52 (d, J = 8.3 Hz, 2H), 7.21 (s, 1H), 6.94 (dd, J = 16.9, 9.3 Hz, 3H), 6.74 - 6.60 (m, 2H), 5.29 (dd, J = 12.6, 5.4 Hz, 1H), 4.71 (d, J = 4.8 Hz, 2H), 4.67 - 4.57 (m, 1H), 4.53 (t, J = 8.7 Hz, 2H), 3.98 - 3.86 (m, 1H), 3.69 (d, J = 11.2 Hz, 1H), 3.31 - 3.24 (m, 5H), 3.24 - 3.13 (m, 1H), 3.03 - 2.80 (m, 2H), 2.66 (dt, J = 29.6, 13.4 Hz, 2H), 2.08 (dd, J = 22.1, 12.5 Hz, 1H), 2.02 - 1.85 (m, 2H).

### Example 96 Preparation of Compound H96

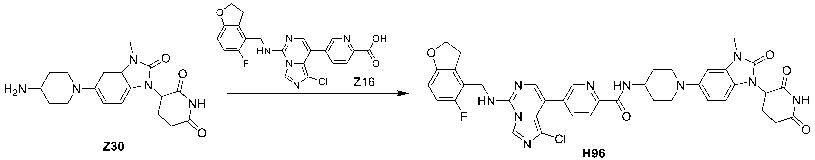

Compound H96 was prepared referring to the method of Example 95. MS m/z (ESI): 779.2 [M+H]⁺. ¹H NMR (400 MHz, DMSO-d₆) δ 11.05 (s, 1H), 8.76 - 8.66 (m, 3H), 8.59 (t, J = 5.2 Hz, 1H), 8.09 (dd, J = 8.0, 0.8 Hz, 1H), 8.03 (dd, J = 8.0, 2.4 Hz, 1H), 7.30 (s, 1H), 6.93 (dt, J = 9.6, 5.2 Hz, 2H), 6.87 (d, J = 2.4 Hz, 1H), 6.71-6.65 (m, 2H), 5.28 (dd, J = 12.8, 5.6 Hz, 1H), 4.72 (d, J = 4.8 Hz, 2H), 4.53 (t, J = 8.8 Hz, 2H), 3.96 (d, J = 8.4 Hz, 1H), 3.61 (d, J = 11.6 Hz, 2H), 3.31 (s, 2H), 3.27 (s, 3H), 2.95 - 2.82 (m, 1H), 2.81 - 2.71 (m, 2H), 2.70 - 2.56 (m, 2H), 2.04 - 1.94 (m, 1H), 1.93-1.79 (m, 4H).

### Example 97 Preparation of Compound H97

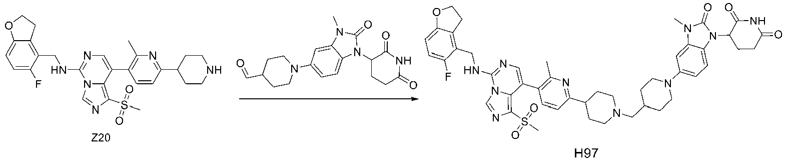

Compound Z20 (40 mg, 74.54 µmol) and 1-(1-(2,6-dioxopiperidin-3-yl)-3-methyl-2-oxo-2,3-dihydro-1H-benzo[d]imidazol-5-yl)piperidine-4-carbaldehyde (33.13 mg, 89.45 µmol) were dissolved in DCM (3 mL) and stirred at room temperature for 2 h. NaBH(OAc)₃ (47.39 mg, 223.62 µmol) was then added, and the mixture was stirred at room temperature overnight. After the reaction was complete, the reaction solution was concentrated and preliminarily purified by CombiFlash (4 g, 0-12% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H97 (8.54 mg, purity 96.9%), yield: 12.46%. MS m/z (ESI): 891.3 [M+H]⁺. ¹H NMR (400 MHz, DMSO-d₆) δ 11.04 (s, 1H), 8.86 (s, 1H), 8.65 (t, J = 4.9 Hz, 1H), 7.44 (d, J = 7.8 Hz, 1H), 7.34 (s, 1H), 7.06 (d, J = 7.8 Hz, 1H), 7.02 - 6.84 (m, 2H), 6.81 (d, J = 2.2 Hz, 1H), 6.70 (dd, J = 8.7, 3.8 Hz, 1H), 6.62 (dd, J = 8.7, 2.2 Hz, 1H), 5.27 (dd, J = 12.9, 5.3 Hz, 1H), 4.71 (dd, J = 16.5, 4.7 Hz, 2H), 4.54 (t, J = 8.7 Hz, 2H), 3.58 (d, J = 11.7 Hz, 3H), 3.46-3.39 (m, 2H), 3.31-3.27 (m, 4H), 3.02-2.96 (m, 2H), 2.92 (s, 3H), 2.89-2.83 (m, 1H), 2.63 (dt, J = 19.0, 6.8 Hz, 6H), 2.45 - 2.30 (m, 1H), 2.28-2.18 (m, 4H), 1.97-1.95 (m, 3H), 1.89-1.79 (m, 5H), 1.30 - 1.21 (m, 2H).

### Example 98 Preparation of Compound H98

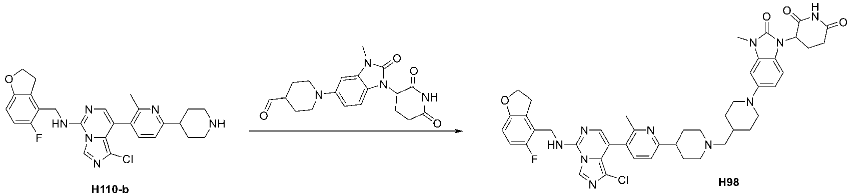

Compound H98 was prepared referring to the method of Example 71. MS m/z (ESI): 847.4 [M+H]⁺. ¹H-NMR (400 MHz, DMSO-d₆) δ 11.04 (s, 1H), 8.63 (s, 1H), 8.39 (t, J = 4.8 Hz, 1H), 7.48 (d, J = 7.8 Hz, 1H), 7.14 (d, J = 7.8 Hz, 1H), 7.09 (s, 1H), 6.96 - 6.89 (m, 2H), 6.81 (d, J = 1.6 Hz, 1H), 6.69 (dd, J = 8.6, 3.8 Hz, 1H), 6.62 (dd, J = 8.6, 1.8 Hz, 1H), 5.26 (dd, J = 12.8, 5.2 Hz, 1H), 4.75-4.62 (m, 2H), 4.53 (t, J = 8.8 Hz, 2H), 3.57 (d, J = 11.6 Hz, 2H), 3.30-3.29 (m, 4H), 2.96 (d, J = 10.0 Hz, 2H), 2.90 - 2.83 (m, 1H), 2.68-2.58 (m, 4H), 2.52 (s, 2H), 2.25 (s, 3H), 2.20 (d, J = 6.8 Hz, 2H), 2.02-1.96 (m, 3H), 1.86-1.75 (m, 5H), 1.71-1.59 (m, 2H), 1.30-1.20 (m, 2H).

### Example 99 and Example 100 Preparation of Compounds H99 and H100

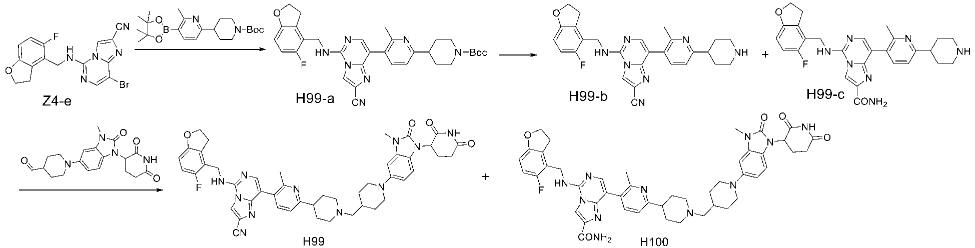

Step 1: Z4-e (170 mg, 437.93 µmol) and tert-butyl 4-(6-methyl-5-(4,4,5,5-tetramethyl-1,3,2-dioxaborolan-2-yl)pyridin-2-yl)piperidine-1-carboxylate (202.62 mg, 503.61 µmol) were dissolved in 1,4-dioxane (10 mL) and water (2 mL). Pd(dppf)Cl₂ (64.09 mg, 87.59 µmol) and K₂CO₃ (181.57 mg, 1.31 mmol) were then added, and the mixture was stirred at 100°C for 3 hours. After the reaction was complete, the reaction solution was concentrated and purified by CombiFlash (12 g, 0-100% EA/PE) to give H99-a (170 mg, light yellow solid), yield: 66.51%. MS m/z (ESI): 584.3 [M+H]⁺.

Step 2: H99-a (170 mg, 291.27 µmol) was dissolved in DCM (10 mL). HCl (4 mol/L solution in 1,4-dioxane, 3 mL) was then added, and the mixture was stirred at room temperature for 1 hour. After the reaction was complete, the mixture was neutralized with saturated aqueous sodium bicarbonate solution, extracted with dichloromethane (50 mL×3). The combined organic phases were dried over anhydrous sodium sulfate and concentrated to give a mixture of H99-b and H99-c (280 mg, light yellow solid), yield: 98.0%. H99-b MS m/z (ESI): 484.2 [M+H]⁺. H99-c MS m/z (ESI): 502.2 [M+H]⁺.

Step 3: The mixture of H99-b and H99-c obtained in the previous step (140 mg, 280 µmol) and 1-(1-(2,6-dioxopiperidin-3-yl)-3-methyl-2-oxo-2,3-dihydro-1H-benzo[d]imidazol-5-yl)piperidine-4-ca rbaldehyde (160.86 mg, 434.30 µmol) were dissolved in DCM (12 mL). NaBH(OAc)₃ (214.77 mg, 1.01 mmol) was then added, and the mixture was stirred at room temperature for 1 hour. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H99 (54.50 mg). MS m/z (ESI): 838.4 [M+H]⁺. ¹H NMR (400 MHz, DMSO-d₆) δ 11.05 (s, 1H), 8.91 (s, 1H), 8.49 (s, 1H), 7.75 (s, 1H), 7.60 (d, J = 7.9 Hz, 1H), 7.18 (d, J = 7.7 Hz, 1H), 6.93 (dd, J = 16.5, 8.9 Hz, 2H), 6.81 (s, 1H), 6.70 (dd, J = 8.5, 3.7 Hz, 1H), 6.62 (d, J = 8.9 Hz, 1H), 5.27 (dd, J = 12.6, 5.3 Hz, 1H), 4.71 (d, J = 4.4 Hz, 2H), 4.54 (t, J = 8.6 Hz, 2H), 3.58 (d, J = 11.8 Hz, 2H), 3.33 (s, 2H), 3.30-3.25 (m, 4H), 2.97 (d, J = 10.1 Hz, 2H), 2.92-2.81 (m, 1H), 2.68-2.56 (m, 5H), 2.32 (s, 3H), 2.20 (s, 2H), 2.06-1.95 (m, 3H), 1.88-1.77 (m, 5H), 1.67 (bs, 1H), 1.29-1.21 (m, 2H).

And H100 (32.66 mg). MS m/z (ESI): 856.4 [M+H]⁺. ¹H NMR (400 MHz, DMSO-d₆) δ 11.05 (s, 1H), 8.63 (s, 1H), 8.37 (d, J = 4.8 Hz, 1H), 7.63 (d, J = 6.6 Hz, 2H), 7.47 (s, 1H), 7.38 (s, 1H), 7.16 (d, J = 7.9 Hz, 1H), 6.97-6.87 (m, 2H), 6.81 (s, 1H), 6.68 (dd, J = 8.7, 3.8 Hz, 1H), 6.62 (d, J = 8.9 Hz, 1H), 5.27 (dd, J = 12.8, 5.4 Hz, 1H), 4.70 (d, J = 4.7 Hz, 2H), 4.53 (t, J = 8.7 Hz, 2H), 3.58 (d, J = 11.8 Hz, 2H), 3.31 (s, 2H), 3.29-3.25 (m, 4H), 2.97 (d, J = 10.2 Hz, 2H), 2.92-2.80 (m, 1H), 2.74 - 2.52 (m, 5H), 2.36 (s, 3H), 2.20 (d, J = 6.6 Hz, 2H), 2.08-1.90 (m, 3H), 1.90-1.72 (m, 5H), 1.71-1.58 (m, 1H), 1.29-1.21 (m, 2H).

### Example 101 to Example 102

Compounds H101 and H102 were prepared referring to the above methods.

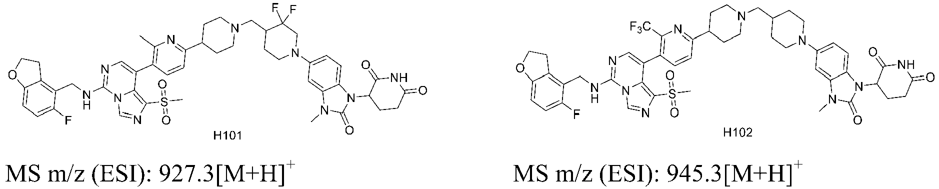

### Example 103 Preparation of Compound H103

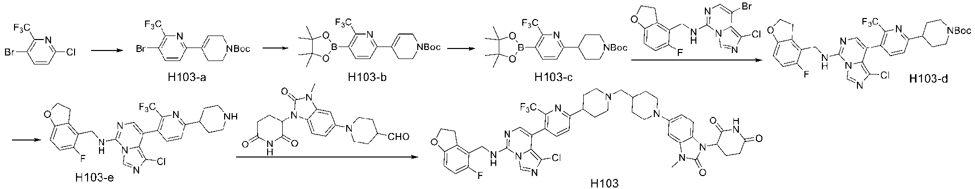

Step 1: 3-Bromo-6-chloro-2-(trifluoromethyl)pyridine (515 mg, 2 mmol), tert-butyl 4-(4,4,5,5-tetramethyl-1,3,2-dioxaborolan-2-yl)-3,6-dihydropyridine-1(2H)-carboxylate (618 mg, 2 mmol), K₂CO₃ (414 mg, 3 mmol), and Pd(dppf)Cl₂ (146 mg, 0.2 mmol) were added to a mixed solution of 1,4-dioxane (5 mL) and water (1 mL). After purging with nitrogen three times, the mixture was heated to 65°C and stirred for 10 hours. After the reaction was complete, the reaction solution was purified by column chromatography (30-100% PE/EA) to give H103-a (203 mg), yield: 50%. MS m/z (ESI): 407 [M+H]⁺.

Step 2: H103-a (203 mg, 1 mmol), bis(pinacolato)diboron (254 mg, 1 mmol), K₂CO₃ (414 mg, 3 mmol), and Pd(dppf)Cl₂ (146 mg, 0.2 mmol) were added to a mixed solvent of 1,4-dioxane (5 mL) and water (1 mL). After purging with nitrogen three times, the mixture was heated to 110°C and stirred for 20 hours. After the reaction was complete, the reaction solution was purified by column chromatography (30-100% PE/EA) to give H103-b (227 mg), yield: 50%. MS m/z (ESI): 455 [M+H]⁺.

Step 3: H103-b (227 mg, 0.5 mmol) and Pd/C (25 mg, 10% purity) were added to methanol (5 mL). After purging with hydrogen, the mixture was stirred at room temperature for 2 hours. After the reaction was complete, the reaction solution was filtered, and the filtrate was concentrated to give H103-c (331 mg), yield: 100%. MS m/z (ESI): 457 [M+H]⁺.

Steps 4-6: Referring to the preparation method of Example 49, following the above synthetic route, H103 was prepared. The purification conditions for preparative HPLC were (waters-sunfire-10 µm-19×150 mm column (mobile phase: 28% - 38% (v/v) acetonitrile and aqueous formic acid)). MS m/z (ESI): 901 [M+H]⁺. ¹H NMR (400 MHz, DMSO-d₆) δ 11.05 (s, 1H), 8.64 (s, 1H), 8.50 (t, J = 5.0 Hz, 1H), 7.88 (d, J = 8.0 Hz, 1H), 7.67 (d, J = 8.0 Hz, 1H), 7.12 (s, 1H), 7.01 - 6.86 (m, 2H), 6.81 (d, J = 2.2 Hz, 1H), 6.70 (dd, J = 8.7, 3.8 Hz, 1H), 6.62 (dd, J = 8.6, 2.2 Hz, 1H), 5.27 (dd, J = 12.9, 5.4 Hz, 1H), 4.81 - 4.61 (m, 2H), 4.53 (t, J = 8.7 Hz, 2H), 3.58 (d, J = 11.7 Hz, 2H), 2.97 (s, 2H), 2.91 - 2.75 (m, 2H), 2.73 - 2.50 (m, 6H), 2.21 (s, 2H), 2.09 - 1.48 (m, 12H), 1.26 (dd, J = 22.7, 10.6 Hz, 3H).

### Example 104 Preparation of Compound H104

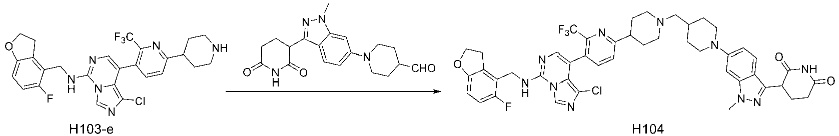

Referring to the preparation method of Example 49, following the above synthetic route, H104 was prepared. The purification conditions for preparative HPLC were waters-sunfire-10 µm-19×150 mm column (mobile phase: 28%-38% (v/v) acetonitrile and aqueous formic acid). MS m/z (ESI): 885 [M+H]⁺. ¹H NMR (400 MHz, DMSO-d₆) δ 10.83 (s, 1H), 8.64 (s, 1H), 8.51 (t, J = 5.0 Hz, 1H), 8.25 (s, 1H), 7.87 (d, J = 8.0 Hz, 1H), 7.67 (d, J = 8.0 Hz, 1H), 7.46 (d, J = 8.9 Hz, 1H), 7.12 (s, 1H), 7.03 - 6.84 (m, 2H), 6.81 (d, J = 1.9 Hz, 1H), 6.70 (dd, J = 8.7, 3.8 Hz, 1H), 4.79 - 4.61 (m, 2H), 4.53 (t, J = 8.7 Hz, 2H), 4.23 (dd, J = 9.1, 5.1 Hz, 1H), 3.87 (s, 3H), 3.78 (d, J = 12.0 Hz, 2H), 2.98 (d, J = 10.7 Hz, 3H), 2.80 - 2.64 (m, 3H), 2.33 - 2.10 (m, 5H), 2.02 (t, J = 11.2 Hz, 2H), 1.86 (dd, J = 27.2, 12.4 Hz, 7H), 1.39 - 1.12 (m, 3H).

### Example 105 Preparation of Compound H105

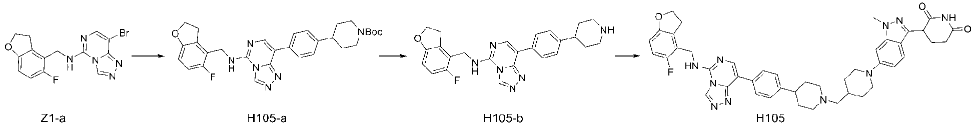

Step 1: Z1-a (726 mg, 2 mmol), tert-butyl 4-(4-(4,4,5,5-tetramethyl-1,3,2-dioxaborolan-2-yl)phenyl)piperidine-1-carboxylate (774 mg, 2 mmol), K₂CO₃ (414 mg, 3 mmol), and Pd(dppf)Cl₂ (146 mg, 0.2 mmol) were added to a mixed solvent of 1,4-dioxane (5 mL) and water (1 mL). After purging with nitrogen three times, the mixture was heated to 100°C and stirred for 5 hours. After the reaction was complete, silica gel was directly added to the mixture for sample loading, and the mixture was purified by column chromatography (30-100% PE/EA) to give H105-a (273 mg), yield: 50%. MS m/z (ESI): 545 [M+H]⁺.

Step 2: H105-a (273 mg, 1 mmol) was added to a mixed solvent of dichloromethane (5 mL) and trifluoroacetic acid (1 mL) and stirred at room temperature for 1 hour. After the reaction was complete, the reaction solution was concentrated to give H105-b (444 mg), yield: 100%. MS m/z (ESI): 445 [M+H]⁺.

Step 3: H105-b (63 mg, 0.15 mmol) and 1-(3-(2,6-dioxopiperidin-3-yl)-1-methyl-1H-indazol-6-yl)piperidine-4-carbaldehyde (53 mg, 0.15 mmol) were added to dichloromethane (2 mL). Two drops of DMSO were added, followed by sodium triacetoxyborohydride (20 mg). The resulting mixture was heated under microwave at 35°C for 45 minutes. After the reaction was complete, the reaction solution was purified by preparative HPLC (waters-sunfire-10 µm-19×150 mm column, mobile phase: 28%-38% (v/v) acetonitrile and aqueous formic acid) to give H105 (25 mg), yield: 20.7%. MS m/z (ESI): 783 [M+H]⁺. ¹H NMR (400 MHz, DMSO-d₆) δ 10.83 (s, 1H), 9.47 (s, 1H), 8.64 (t, J = 5.1 Hz, 1H), 8.00 (d, J = 8.4 Hz, 3H), 7.46 (d, J = 8.9 Hz, 1H), 7.34 (d, J = 8.0 Hz, 2H), 7.00 - 6.85 (m, 2H), 6.81 (s, 1H), 6.70 (dd, J = 8.6, 3.9 Hz, 1H), 4.71 (d, J = 4.9 Hz, 2H), 4.53 (t, J = 8.7 Hz, 2H), 4.23 (dd, J = 9.1, 5.1 Hz, 1H), 3.87 (s, 3H), 3.78 (d, J = 12.0 Hz, 2H), 2.97 (d, J = 10.6 Hz, 2H), 2.81 - 2.51 (m, 6H), 2.33 - 2.07 (m, 4H), 2.00 (s, 2H), 1.88 - 1.57 (m, 7H), 1.36 - 1.11 (m, 3H).

### Example 106 Preparation of Compound H106

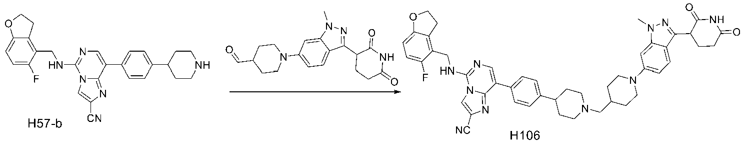

Referring to the preparation method of Example 109, following the above synthetic route, H106 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 404.2 [M/2+H]⁺. ¹H NMR (400 MHz, DMSO-d₆) δ 10.86 (s, 1H), 8.93 (s, 1H), 8.48 (s, 1H), 8.06 (s, 1H), 7.87 (d, J = 8.2 Hz, 2H), 7.48 (d, J = 8.9 Hz, 1H), 7.34 (d, J = 8.3 Hz, 2H), 6.94 (dd, J = 20.5, 10.3 Hz, 2H), 6.83 (s, 1H), 6.71 (dd, J = 8.6, 3.9 Hz, 1H), 4.73 (s, 2H), 4.55 (t, J = 8.7 Hz, 2H), 4.25 (dd, J = 9.1, 5.1 Hz, 1H), 3.89 (s, 3H), 3.79 (d, J = 11.8 Hz, 2H), 3.30 (d, J = 8.7 Hz, 2H), 2.98 (d, J = 10.6 Hz, 2H), 2.74 (t, J = 11.4 Hz, 2H), 2.68 - 2.59 (m, 2H), 2.55 (d, J = 14.6 Hz, 1H), 2.30 (dt, J = 13.7, 8.6 Hz, 1H), 2.24 - 2.11 (m, 3H), 2.00 (t, J = 10.6 Hz, 2H), 1.89 - 1.61 (m, 7H), 1.27 (dd, J = 20.8, 10.8 Hz, 2H).

### Example 107 Preparation of Compound H107

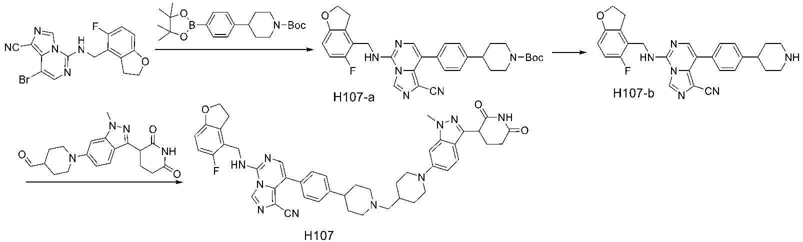

Referring to the preparation method of Example 51, following the above synthetic route, H107 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 807.4 [M+H]⁺. ¹H NMR (400 MHz, DMSO-d₆) δ 10.84 (s, 1H), 8.82 (s, 1H), 8.69 (t, J = 4.9 Hz, 1H), 7.53 (s, 1H), 7.45 (dd, J = 12.7, 8.6 Hz, 3H), 7.33 (d, J = 8.3 Hz, 2H), 6.92 (dd, J = 20.0, 10.5 Hz, 2H), 6.81 (s, 1H), 6.69 (dd, J = 8.7, 3.8 Hz, 1H), 4.71 (d, J = 4.6 Hz, 2H), 4.53 (t, J = 8.7 Hz, 2H), 4.23 (dd, J = 9.1, 5.1 Hz, 1H), 3.87 (s, 3H), 3.77 (d, J = 11.9 Hz, 2H), 3.29 (d, J = 8.9 Hz, 2H), 2.97 (d, J = 10.0 Hz, 2H), 2.72 (t, J = 11.3 Hz, 2H), 2.65 - 2.51 (m, 3H), 2.33 - 2.09 (m, 4H), 1.99 (t, J = 10.3 Hz, 2H), 1.89 - 1.58 (m, 7H), 1.25 (dd, J = 22.3, 12.6 Hz, 2H).

### Example 108 Preparation of Compound H108

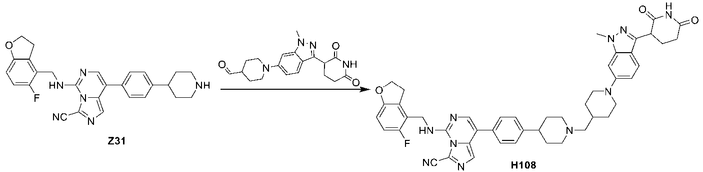

Compound H108 was prepared referring to the method of Example 71. MS m/z (ESI): 807.4 [M+H]⁺. ¹H NMR (400 MHz, DMSO-d₆) δ 10.83 (s, 1H), 7.70 (d, J = 10.0 Hz, 2H), 7.62 - 7.49 (m, 3H), 7.46 (d, J = 8.8 Hz, 1H), 7.38 (d, J = 8.0 Hz, 2H), 6.98 - 6.88 (m, 2H), 6.81 (d, J = 2.0 Hz, 1H), 6.69 (dd, J = 8.8, 4.0 Hz, 1H), 4.68 (s, 2H), 4.55 (t, J = 8.8 Hz, 2H), 4.23 (dd, J = 9.2, 5.2 Hz, 1H), 3.87 (s, 3H), 3.78 (d, J = 12.0 Hz, 2H), 3.41 (s, 2H), 2.98 (s, 2H), 2.73 (t, J = 12.0 Hz, 2H), 2.60 (q, J = 6.8, 5.6 Hz, 3H), 2.35 - 2.10 (m, 4H), 1.98 (d, J = 10.0 Hz, 2H), 1.89 - 1.64 (m, 7H), 1.32-1.24 (m, 2H).

### Example 109 Preparation of Compound H109

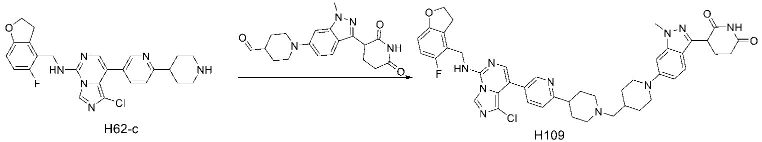

Under argon protection, H62-c (65 mg, 135.71 µmol) and 1-(3-(2,6-dioxopiperidin-3-yl)-1-methyl-1H-indazol-6-yl)piperidine-4-carbaldehyde (144.29 mg, 407.14 µmol) were added to a mixed solution of DMSO (1 mL), EtOH (0.3 mL), and AcOH (0.01 mL). The mixture was heated under microwave at 90°C for 0.5 hour. Then NaBH(OAc)₃ (143.82 mg, 678.57 µmol) was added, and the mixture was heated under microwave at 90°C for another 0.5 hour. After the reaction was complete, water was added dropwise to quench the reaction, and the reaction solution was concentrated and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H109 (33.90 mg, purity 99.12%), yield: 30.29%. MS m/z (ESI): 817.3 [M+H]⁺. ¹H-NMR (400 MHz, DMSO-d₆) δ 10.83 (s, 1H), 8.68 (s, 1H), 8.53 (d, J = 2.2 Hz, 1H), 8.47 (s, 1H), 7.74 (dd, J = 8.0, 2.2 Hz, 1H), 7.46 (d, J = 9.0 Hz, 1H), 7.33 (d, J = 8.0 Hz, 1H), 7.21 (s, 1H), 6.95 - 6.87 (m, 2H), 6.81 (d, J = 0.8 Hz, 1H), 6.68 (dd, J = 8.6, 3.8 Hz, 1H), 4.70 (s, 2H), 4.52 (t, J = 8.8 Hz, 2H), 4.23 (dd, J = 9.2, 5.2 Hz, 1H), 3.87 (s, 3H), 3.77 (d, J = 12.0 Hz, 2H), 3.28 (d, J = 8.8 Hz, 2H), 2.96 (d, J = 10.8 Hz, 2H), 2.76 - 2.67 (m, 3H), 2.64 - 2.57 (m, 2H), 2.32 - 2.25 (m, 1H), 2.19 (d, J = 6.8 Hz, 2H), 2.16-2.11 (m, 1H), 1.99 (t, J = 10.6 Hz, 2H), 1.86 - 1.72 (m, 6H), 1.29-1.20 (m, 3H).

### Example 110 Preparation of Compound H110

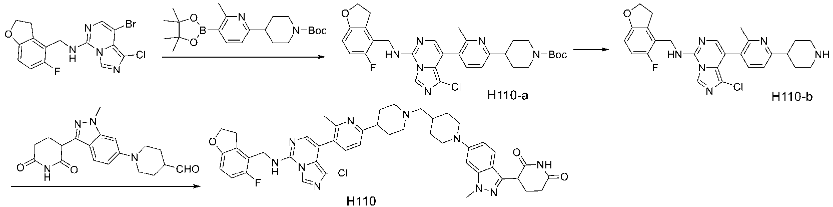

Referring to the preparation method of Example 49, following the above synthetic route, H110 was prepared. MS m/z (ESI): 831.4 [M+H]⁺. ¹H NMR (400 MHz, DMSO-d₆) δ 10.83 (s, 1H), 8.64 (s, 1H), 8.40 (t, J = 4.9 Hz, 1H), 7.49 (dd, J = 16.0, 8.3 Hz, 2H), 7.16 (d, J = 7.8 Hz, 1H), 7.09 (s, 1H), 6.92 (dd, J = 18.2, 9.4 Hz, 2H), 6.82 (s, 1H), 6.69 (dd, J = 8.6, 3.8 Hz, 1H), 4.80 - 4.61 (m, 2H), 4.54 (t, J = 8.8 Hz, 2H), 4.24 (dd, J = 9.3, 5.1 Hz, 1H), 3.87 (s, 3H), 3.79 (d, J = 11.8 Hz, 2H), 3.29 (dd, J = 16.9, 8.4 Hz, 6H), 3.16 (s, 2H), 2.74 (t, J = 11.5 Hz, 3H), 2.66 - 2.54 (m, 2H), 2.39 - 2.21 (m, 5H), 2.14 (dd, J = 13.2, 5.6 Hz, 1H), 1.99 - 1.76 (m, 6H), 1.29 (d, J = 11.3 Hz, 2H).

### Example 111 Preparation of Compound H111

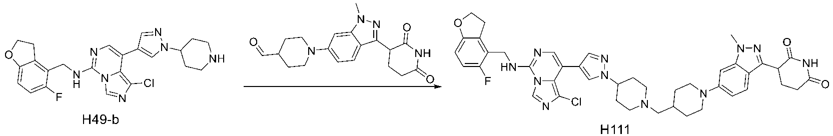

Referring to the preparation method of Example 109, following the above synthetic route, H111 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 806.3 [M+H]⁺. ¹H NMR (400 MHz, DMSO-d₆) δ 10.83 (s, 1H), 8.61 (s, 1H), 8.29 (t, J = 5.2 Hz, 1H), 7.94 (s, 1H), 7.57 (s, 1H), 7.46 (d, J = 9.0 Hz, 1H), 7.15 (s, 1H), 6.94-6.88 (m, 2H), 6.81 (d, J = 1.2 Hz, 1H), 6.67 (dd, J = 8.6, 4.0 Hz, 1H), 4.67 (d, J = 4.8 Hz, 2H), 4.52 (t, J = 8.8 Hz, 2H), 4.23 (dd, J = 9.2, 5.2 Hz, 1H), 4.19 - 4.12 (m, 1H), 3.87 (s, 3H), 3.77 (d, J = 12.4 Hz, 2H), 3.27 (t, J = 9.2 Hz, 2H), 2.94 (d, J = 10.4 Hz, 2H), 2.72 (t, J = 11.2 Hz, 2H), 2.63 - 2.57 (m, 2H), 2.32 - 2.25 (m, 1H), 2.22 - 2.17 (m, 2H), 2.17 - 2.11 (m, 1H), 2.05 - 1.94 (m, 5H), 1.81 (d, J = 10.8 Hz, 2H), 1.74 - 1.66 (m, 1H), 1.29 - 1.19 (m, 3H).

### Example 112 Preparation of Compound H112

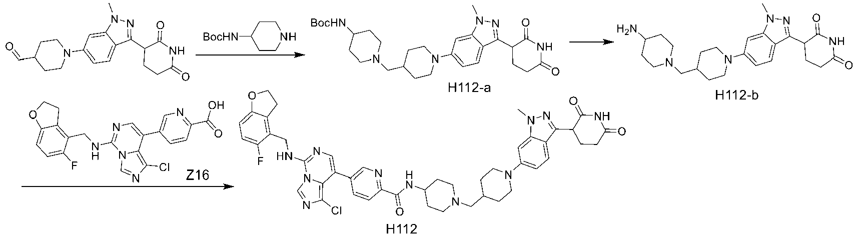

Step 1: 1-(3-(2,6-Dioxopiperidin-3-yl)-1-methyl-1H-indazol-6-yl)piperidine-4-carbaldehyde (0.5 g, 1.41 mmol) and tert-butyl piperidin-4-ylcarbamate (282.56 mg, 1.41 mmol) were dissolved in DCM (10 mL). NaBH(OAc)₃ (897.03 mg, 4.23 mmol) was added, and the mixture was stirred at room temperature for 3 hours. After the reaction was complete, saturated aqueous sodium bicarbonate solution was added to the reaction solution, extraction was performed three times with dichloromethane, the combined organic phases were dried over anhydrous sodium sulfate, concentrated, and purified by CombiFlash (12 g, 0-10% MeOH/DCM) to give H112-a (0.4 g, light yellow solid), yield: 52.63%. MS m/z (ESI): 539.4 [M+H]⁺.

Step 2: H112-a (0.4 g, 742.56 µmol) was dissolved in DCM (2 mL). HCl/1,4-dioxane (4 mol/L, 5 mL) was added, and the mixture was stirred at room temperature for 1 hour. After the reaction was complete, the reaction solution was concentrated to give H112-b (0.3 g, light yellow solid), yield: 92.12%. MS m/z (ESI): 439.2 [M+H]⁺.

Step 3: Intermediate Z16 (50 mg, 113.68 µmol) and H112-b (29.91 mg, 68.21 µmol) were dissolved in DMF (3 mL). HATU (64.33 mg, 170.52 µmol) and DIEA (44.08 mg, 341.04 µmol, 59.40 µL) were added, and the mixture was stirred at room temperature for 1 hour. After the reaction was complete, water and ethyl acetate were added to the reaction solution, extraction was performed three times with ethyl acetate, the combined organic phases were dried over anhydrous sodium sulfate, concentrated, and purified by CombiFlash (4 g, 0-15% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H112 (9.85 mg, purity 99.161%), yield: 9.99%. MS m/z (ESI): 860.3 [M+H]⁺. ¹H NMR (400 MHz, DMSO-d₆) δ 10.83 (s, 1H), 8.71 (s, 1H), 8.68 (dd, J = 2.4, 1.2 Hz, 1H), 8.59 (t, J = 5.2 Hz, 2H), 8.07 (d, J = 8.0 Hz, 1H), 8.02 (dd, J = 8.0, 2.0 Hz, 1H), 7.46 (d, J = 8.8 Hz, 1H), 7.30 (s, 1H), 6.98 - 6.86 (m, 2H), 6.85 - 6.78 (m, 1H), 6.69 (dd, J = 8.8, 4.0 Hz, 1H), 4.72 (d, J = 4.8 Hz, 2H), 4.53 (t, J = 8.8 Hz, 2H), 4.23 (dd, J = 9.2, 5.2 Hz, 1H), 3.87 (s, 3H), 3.77 (d, J = 12.4 Hz, 3H), 3.28 (d, J = 9.2 Hz, 2H), 2.84 (s, 2H), 2.72 (t, J = 12.0 Hz, 2H), 2.60 (q, J = 6.4, 5.6 Hz, 2H), 2.36 - 2.22 (m, 1H), 2.14 (d, J = 13.2 Hz, 3H), 1.99 (s, 2H), 1.83 - 1.61 (m, 7H), 1.23 (d, J = 12.4 Hz, 2H).

### Example 113 Preparation of Compound H113

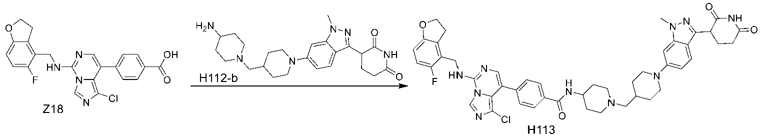

Referring to the preparation method of Example 112, following the above synthetic route, H113 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 430.3 [M/2+H]⁺. ¹H NMR (400 MHz, DMSO-d₆) δ 10.83 (s, 1H), 8.68 (s, 1H), 8.47 (t, J = 5.2 Hz, 1H), 8.28 (d, J = 7.6 Hz, 1H), 7.92 - 7.85 (m, 2H), 7.47 (t, J = 8.4 Hz, 3H), 7.20 (s, 1H), 6.97 - 6.87 (m, 2H), 6.81 (d, J = 2.0 Hz, 1H), 6.68 (dd, J = 8.8, 4.0 Hz, 1H), 4.70 (d, J = 5.2 Hz, 2H), 4.53 (t, J = 8.8 Hz, 2H), 4.23 (dd, J = 9.2, 5.2 Hz, 1H), 3.87 (s, 3H), 3.77 (d, J = 12.0 Hz, 3H), 3.28 (d, J = 8.8 Hz, 2H), 2.86 (d, J = 10.4 Hz, 2H), 2.78 - 2.68 (m, 2H), 2.60 (q, J = 6.4, 5.6 Hz, 2H), 2.36 - 2.23 (m, 1H), 2.22 - 2.10 (m, 3H), 1.96 (t, J = 11.2 Hz, 2H), 1.83-1.74 (m, 4H), 1.72-1.52 (m, 3H), 1.30-1.16 (m, 2H).

### Example 114 Preparation of Compound H114

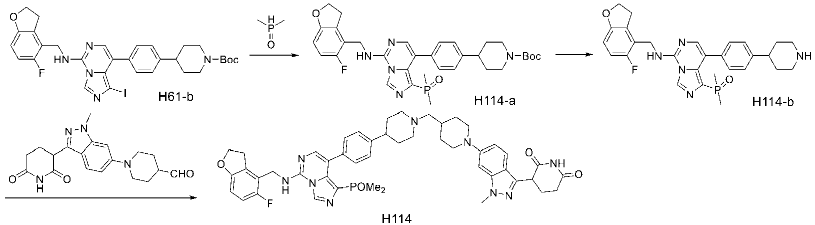

Step 1: H61-b (100 mg, 149.36 µmol) and methylphosphonylmethane (34.97 mg, 448.08 µmol) were dissolved in 1,4-dioxane (4 mL). Under argon protection, triethylamine (75.43 mg, 746.80 µmol, 0.4 mL), Pd₂(dba)₃ (27.35 mg, 29.87 µmol), and Xantphos (34.57 mg, 59.74 µmol) were added sequentially. The temperature was raised to 100°C and stirred overnight. After the reaction was complete, the reaction solution was concentrated to dryness under reduced pressure, and purified by silica gel column chromatography (MeOH:DCM = 0-20%) to give H114-a (50 mg, yellow solid), yield: 54.02%. MS m/z (ESI): 620 [M+H]⁺.

Step 2: H114-a (50 mg, 80.69 µmol) was dissolved in dichloromethane (10 mL). A solution of hydrogen chloride in dioxane (4.0 mol/L, 5 mL) was added with stirring at room temperature, and the mixture was stirred at room temperature for 3 hours. After the reaction was complete, the reaction solution was concentrated to give H114-b (41.92 mg, yellow solid), yield: 100.00%. MS m/z (ESI): 520 [M+H]⁺.

Step 3: H114-b (30 mg, 57.74 µmol) and 1-[3-(2,6-dioxopiperidin-3-yl)-1-methylindazol-6-yl]piperidine-4-carbaldehyde (30.70 mg, 86.61 µmol) were dissolved in dichloromethane (5 mL). NaBH(OAc)₃ (12.2 mg, 57.74 µmol) was added with stirring at room temperature, and the mixture was stirred at room temperature overnight. After the reaction was complete, the reaction solution was concentrated and purified by preparative HPLC to give H114 (7.15 mg, purity 100%), yield: 14.43%. MS m/z (ESI): 858 [M+H]⁺. ¹H NMR (400 MHz, DMSO-d₆) δ 10.84 (s, 1H), 8.81 (s, 1H), 8.46 (s, 1H), 7.48 (dd, J = 11.3, 8.5 Hz, 3H), 7.28 (d, J = 10.2 Hz, 3H), 6.96 - 6.86 (m, 2H), 6.82 (s, 1H), 6.68 (dd, J = 8.5, 4.0 Hz, 1H), 4.71 (d, J = 4.7 Hz, 2H), 4.53 (t, J = 8.8 Hz, 2H), 4.23 (dd, J = 9.2, 5.2 Hz, 1H), 3.87 (s, 3H), 3.78 (d, J = 12.2 Hz, 2H), 3.28 (d, J = 9.1 Hz, 2H), 2.98 (s, 2H), 2.80 - 2.52 (m, 6H), 2.34 - 1.96 (m, 6H), 1.83 (d, J = 12.3 Hz, 6H), 1.25 (d, J = 25.5 Hz, 2H), 1.15 (d, J = 13.5 Hz, 6H).

### Example 115 Preparation of Compound H115

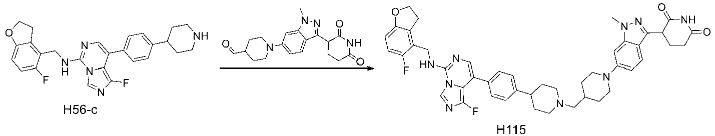

Referring to the preparation method of Example 109, following the above synthetic route, H115 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 800.4 [M+H]⁺. ¹H NMR (400 MHz, DMSO-d₆) δ 10.84 (s, 1H), 8.37 (s, 1H), 8.29 (t, J = 5.1 Hz, 1H), 7.46 (d, J = 9.0 Hz, 1H), 7.44 - 7.38 (m, 2H), 7.31 (d, J = 8.2 Hz, 2H), 7.17 (s, 1H), 6.91 (dd, J = 18.5, 9.2 Hz, 2H), 6.81 (s, 1H), 6.68 (dd, J = 8.7, 3.9 Hz, 1H), 4.69 (d, J = 4.9 Hz, 2H), 4.53 (t, J = 8.7 Hz, 2H), 4.23 (dd, J = 9.1, 5.1 Hz, 1H), 3.87 (s, 3H), 3.77 (d, J = 11.5 Hz, 2H), 3.28 (d, J = 8.5 Hz, 3H), 2.97 (d, J = 10.1 Hz, 2H), 2.72 (t, J = 11.7 Hz, 2H), 2.64 - 2.52 (m, 3H), 2.33 - 2.25 (m, 1H), 2.24 - 2.09 (m, 3H), 1.99 (t, J = 10.4 Hz, 2H), 1.84 - 1.66 (m, 6H), 1.25 (dd, J = 24.3, 13.4 Hz, 2H).

### Example 116 Preparation of Compound H116

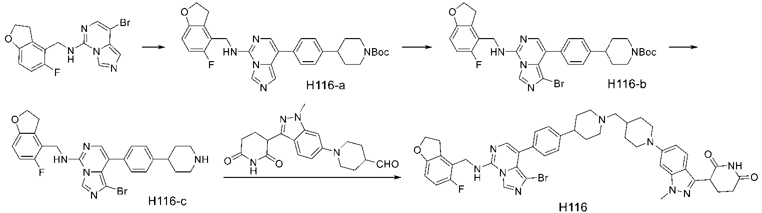

Step 1: Compound 8-bromo-N-((5-fluoro-2,3-dihydrobenzofuran-4-yl)methyl)imidazo[1,5-c] pyrimidin-5-amine (724 mg, 2 mmol), tert-butyl 4-(4-(4,4,5,5-tetramethyl-1,3,2-dioxaborolan-2-yl)phenyl)piperidine-1-carboxylate (774 mg, 2 mmol), K₂CO₃ (414 mg, 3 mmol), and Pd(dppf)Cl₂ (146 mg, 0.2 mmol) were added to a mixed solvent of 1,4-dioxane (10 mL) and water (1 mL). After purging with nitrogen three times, the mixture was heated to 100°C and stirred for 5 hours. After the reaction was complete, the reaction solution was purified by silica gel column chromatography (30-100% PE/EA) to give H116-a (544 mg), yield: 50%. MS m/z (ESI): 544 [M+H]⁺.

Step 2: H116-a (544 mg, 1 mmol) was dissolved in DMF (5 mL). NBS (178 mg, 1 mmol) was then added, and the mixture was stirred at room temperature overnight. After the reaction was complete, the reaction solution was purified by silica gel column chromatography (30-100% PE/EA) to give H116-b (311 mg), yield: 50%. MS m/z (ESI): 622 [M+H]⁺.

Step 3: H116-b (311 mg, 0.5 mmol) was added to a mixed solution of dichloromethane (5 mL) and trifluoroacetic acid (1 mL) and stirred at room temperature for 1 hour. After the reaction was complete, the reaction solution was concentrated to give H116-c (261 mg), yield: 100%. MS m/z (ESI): 522 [M+H]⁺.

Step 4: H116-c (78 mg, 0.15 mmol) and 1-(3-(2,6-dioxopiperidin-3-yl)-1-methyl-1H-indazol-6-yl)piperidine-4-carbaldehyde (53 mg, 0.15 mmol) were added to dichloromethane (2 mL). Two drops of DMSO were added, followed by NaBH(OAc)₃ (20 mg). The resulting mixture was heated under microwave at 35°C for 45 minutes. After the reaction was complete, the reaction solution was purified by preparative HPLC (waters-sunfire-10 µm-19×150 mm column (mobile phase: 28%-38% (v/v) acetonitrile and aqueous formic acid)) to give H116 (10 mg), yield: 7.7%. MS m/z (ESI): 860 [M+H]⁺. ¹H NMR (400 MHz, DMSO-d₆) δ 10.83 (s, 1H), 8.68 (s, 1H), 8.34 (t, J = 5.1 Hz, 1H), 7.46 (d, J = 8.9 Hz, 1H), 7.38 - 7.24 (m, 4H), 7.11 (s, 1H), 6.98 - 6.86 (m, 2H), 6.81 (d, J = 1.9 Hz, 1H), 6.76 - 6.64 (m, 1H), 4.68 (d, J = 4.9 Hz, 2H), 4.52 (t, J = 8.7 Hz, 2H), 4.23 (dd, J = 9.1, 5.1 Hz, 1H), 3.87 (s, 3H), 3.76 (s, 2H), 2.98 (s, 2H), 2.74 - 2.60 (m, 3H), 2.33 - 2.09 (m, 4H), 2.08 - 1.90 (m, 3H), 1.75 (dt, J = 42.2, 12.6 Hz, 7H), 1.35 - 1.10 (m, 5H).

### Example 117 Preparation of Compound H117

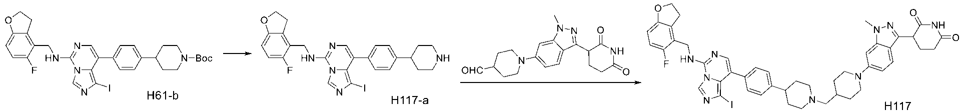

Referring to the preparation method of Example 49, following the above synthetic route, H117 was prepared. MS m/z (ESI): 908 [M+H]⁺. ¹H NMR (400 MHz, DMSO-d₆) δ 8.54 (s, 2H), 7.46 (d, J = 9.0 Hz, 1H), 7.26 (s, 4H), 7.02 (s, 1H), 6.89 (d, J = 8.1 Hz, 2H), 6.81 (s, 1H), 6.64 (s, 1H), 4.65 (s, 2H), 4.50 (t, J = 8.2 Hz, 2H), 4.21 (s, 1H), 3.87 (s, 2H), 3.77 (d, J = 12.3 Hz, 2H), 3.25 (s, 3H), 2.97 (d, J = 11.0 Hz, 2H), 2.79 - 2.57 (m, 4H), 2.32 - 2.12 (m, 4H), 1.98 (d, J = 11.0 Hz, 2H), 1.85 - 1.59 (m, 7H), 1.24 (d, J = 17.4 Hz, 4H).

### Example 118 Preparation of Compound H118

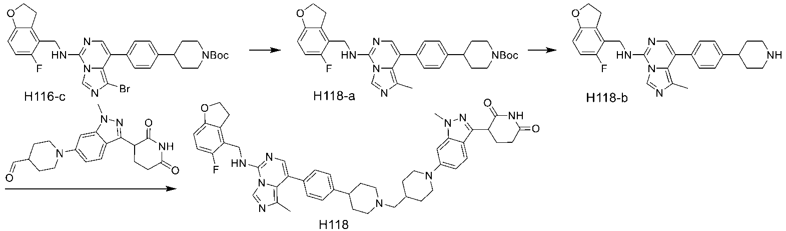

Step 1: H116-c (0.15 g, 240.95 µmol) and trimethylcyclotriboroxane (3.5 mol/L, 206.53 µL) were dissolved in 1,4-dioxane (5 mL) and water (1 mL). Under argon protection, Pd(dppf)Cl₂ (35.26 mg, 48.19 µmol) and K₂CO₃ (99.90 mg, 722.86 µmol) were added, and the temperature was raised to 100°C and stirred for 3 hours. After the reaction was complete, the reaction solution was concentrated, water and ethyl acetate were added, extraction was performed three times with ethyl acetate, the combined organic phases were dried over anhydrous sodium sulfate, concentrated, and purified by CombiFlash (4 g, 0-100% EA/PE) to give H118-a (80 mg, light yellow solid), yield: 59.54%. MS m/z (ESI): 558.3 [M+H]⁺.

Step 2: H118-a (80 mg, 143.46 µmol) was dissolved in DCM (3 mL). TFA (1 mL) was added, and the mixture was stirred at room temperature for 1 hour. After the reaction was complete, the reaction solution was concentrated, water and dichloromethane were added, the pH was adjusted to greater than 8 with saturated aqueous sodium bicarbonate solution, extraction was performed three times with organic solvent (dichloromethane:methanol = 10:1), the combined organic phases were dried over anhydrous sodium sulfate, and concentrated to give H118-b (60 mg, light yellow solid, crude), yield: 91.41%. MS m/z (ESI): 458.2 [M+H]⁺.

Step 3: H118-b (60.00 mg, 131.14 µmol) and 1-(3-(2,6-dioxopiperidin-3-yl)-1-methyl-1H-indazol-6-yl)piperidine-4-carbaldehyde (92.95 mg, 262.27 µmol) were dissolved in DCM (5 mL). NaBH(OAc)₃ (83.38 mg, 393.41 µmol) was added, and the mixture was stirred at room temperature for 1 hour. After the reaction was complete, saturated aqueous sodium bicarbonate solution was added to the reaction solution, extraction was performed three times with dichloromethane, the combined organic phases were dried over anhydrous sodium sulfate, concentrated, and purified by CombiFlash (4 g, 0-15% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H118 (9.35 mg, purity: 94.04%), yield: 8.42%. MS m/z (ESI): 797.4 [M+H]⁺. ¹H NMR (400 MHz, DMSO-d₆) δ 10.83 (s, 1H), 8.55 (s, 1H), 8.09 (t, J = 5.2 Hz, 1H), 7.46 (d, J = 9.2 Hz, 1H), 7.30 (s, 4H), 6.95 - 6.87 (m, 3H), 6.81 (d, J = 2.0 Hz, 1H), 6.67 (dd, J = 8.8, 4.0 Hz, 1H), 4.68 (d, J = 5.2 Hz, 2H), 4.52 (t, J = 8.8 Hz, 2H), 4.23 (dd, J = 9.2, 5.2 Hz, 1H), 3.87 (s, 3H), 3.78 (d, J = 12.0 Hz, 2H), 3.27 (d, J = 9.2 Hz, 2H), 2.97 (d, J = 10.8 Hz, 2H), 2.72 (t, J = 12.0 Hz, 2H), 2.64-2.54 (m, 3H), 2.35 - 2.24 (m, 1H), 2.24 - 2.09 (m, 3H), 1.97 (s, 5H), 1.85-1.65 (m, 7H), 1.32 - 1.20 (m, 2H).

### Example 119 Preparation of Compound H119

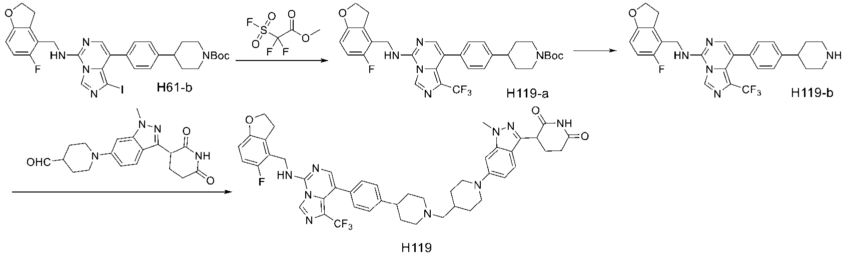

Step 1: H61-b (102 mg, 152.21 µmol) was dissolved in DMF (8 mL). CuI (289.88 mg, 1.52 mmol), Pd(dppf)Cl₂ (11.14 mg, 15.22 µmol), and methyl 2,2-difluoro-2-(fluorosulfonyl)acetate (292.41 mg, 1.52 mmol) were added. The reaction solution was stirred at 90°C overnight under argon protection. After the reaction was complete, the reaction solution was purified by silica gel column chromatography (30-100% PE/EA) to give H119-a (28 mg), yield: 30%. MS m/z (ESI): 612 [M+H]⁺.

Step 2: H119-a (28 mg, 45.66 µmol) was added to a mixed solvent of dichloromethane (5 mL) and trifluoroacetic acid (1 mL) and stirred at room temperature for 1 hour. After the reaction was complete, the reaction solution was concentrated to give H119-b (24 mg), yield: 100%. MS m/z (ESI): 512 [M+H]⁺.

Step 3: H119-b (24 mg, 45.66 µmol) and 1-(3-(2,6-dioxopiperidin-3-yl)-1-methyl-1H-indazol-6-yl)piperidine-4-carbaldehyde (16 mg, 45.66 µmol) were added to dichloromethane (2 mL). Two drops of DMSO were added, followed by NaBH(OAc)₃ (10 mg). The resulting mixture was heated under microwave at 30°C for 45 minutes. After the reaction was complete, the reaction solution was purified by preparative HPLC (waters-sunfire-10 µm-19×150 mm column, mobile phase: 28%-38% (v/v) acetonitrile and aqueous formic acid) to give H119 (10 mg), yield: 25.8%. MS m/z (ESI): 850 [M+H]⁺. ¹H NMR (400 MHz, DMSO-d₆) δ 10.83 (s, 1H), 8.82 (s, 1H), 8.55 (t, J = 5.2 Hz, 1H), 7.46 (d, J = 8.9 Hz, 1H), 7.28 (d, J = 8.4 Hz, 5H), 6.98 - 6.85 (m, 2H), 6.81 (d, J = 1.9 Hz, 1H), 6.69 (dd, J = 8.7, 3.8 Hz, 1H), 4.71 (d, J = 4.9 Hz, 2H), 4.53 (t, J = 8.7 Hz, 2H), 4.23 (dd, J = 9.1, 5.1 Hz, 1H), 3.87 (s, 3H), 3.77 (d, J = 12.2 Hz, 2H), 2.97 (d, J = 10.4 Hz, 2H), 2.88 - 2.53 (m, 5H), 2.34 - 2.07 (m, 4H), 2.08 - 1.91 (m, 3H), 1.75 (dt, J = 41.5, 13.4 Hz, 7H), 1.24 (d, J = 19.6 Hz, 3H).

### Example 120 Preparation of Compound H120

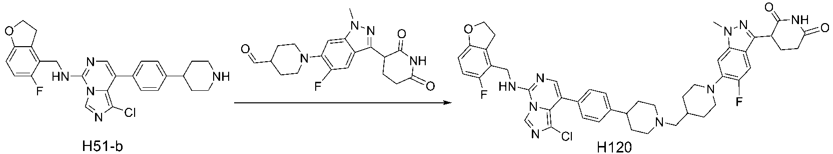

Referring to the preparation method of Example 109, following the above synthetic route, H120 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 834.3 [M+H]⁺. ¹H NMR (400 MHz, DMSO-d₆) δ 10.84 (s, 1H), 8.65 (s, 1H), 8.35 (t, J = 5.1 Hz, 1H), 7.42 (d, J = 12.5 Hz, 1H), 7.31 (q, J = 8.2 Hz, 3H), 7.13 (s, 1H), 7.08 (d, J = 7.0 Hz, 1H), 6.97 - 6.88 (m, 1H), 6.68 (dd, J = 8.7, 3.9 Hz, 1H), 4.69 (d, J = 4.8 Hz, 2H), 4.52 (t, J = 8.7 Hz, 2H), 4.25 (dd, J = 9.6, 5.1 Hz, 1H), 3.93 (s, 3H), 3.42 (d, J = 10.3 Hz, 2H), 3.28 (d, J = 8.6 Hz, 3H), 2.96 (s, 2H), 2.68 (t, J = 10.6 Hz, 2H), 2.64 - 2.50 (m, 3H), 2.38 - 2.28 (m, 1H), 2.23 (d, J = 3.7 Hz, 2H), 2.17 - 2.07 (m, 1H), 2.06 - 1.93 (m, 2H), 1.82 (dd, J = 21.9, 11.2 Hz, 4H), 1.69 (dd, J = 22.7, 13.3 Hz, 3H), 1.32 (dd, J = 24.1, 13.1 Hz, 2H).

### Example 121 Preparation of Compound H121

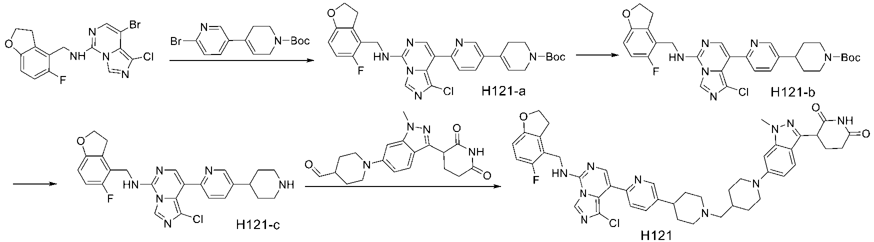

Step 1: Under argon protection, 8-bromo-1-chloro-N-((5-fluoro-2,3-dihydrobenzofuran-4-yl)methyl)imidazo[1,5-c]pyrimidin-5-amine (500 mg, 1.26 mmol), tert-butyl 6-bromo-3',6'-dihydro-[3,4'-bipyridine]-1'(2'H)-carboxylate (639.84 mg, 1.89 mmol), Pd(OAc)₂ (28.23 mg, 125.75 µmol), CataCXium A (90.17 mg, 251.49 µmol), K₂CO₃ (695.17 mg, 5.03 mmol), and (Pin)₂B₂ (798.29 mg, 3.14 mmol) were added to a mixed solution of DME (20 mL), DMSO (1 mL), and water (4 mL). The mixture was heated to 70°C and reacted for 16 hours. After the reaction was complete, the reaction solution was filtered, and the filtrate was separated to obtain the organic phase. The obtained organic phase was washed with saturated brine, concentrated, and purified by silica gel column chromatography (PE:EA = 25%:75%) to give H121-a (230 mg, yellow solid), yield: 31.70%. MS m/z (ESI): 577.2 [M+H]⁺.

Steps 2-4: Referring to the preparation method of Example 62, following the above synthetic route, H121 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 817.3 [M+H]⁺. ¹H NMR (400 MHz, DMSO-d₆) δ 10.84 (s, 1H), 8.67 (s, 1H), 8.53 (d, J = 1.8 Hz, 1H), 8.48 (t, J = 4.8 Hz, 1H), 7.73 (dd, J = 8.2, 2.2 Hz, 1H), 7.45 (t, J = 8.8 Hz, 2H), 7.34 (s, 1H), 6.95 - 6.88 (m, 2H), 6.81 (d, J = 1.2 Hz, 1H), 6.68 (dd, J = 8.6, 4.0 Hz, 1H), 4.71 (d, J = 4.4 Hz, 2H), 4.52 (t, J = 8.8 Hz, 2H), 4.23 (dd, J = 9.2, 5.2 Hz, 1H), 3.87 (s, 3H), 3.77 (d, J = 12.0 Hz, 2H), 3.31-3.26 (m, 2H), 2.98 (d, J = 9.8 Hz, 2H), 2.72 (t, J = 11.4 Hz, 2H), 2.62-2.57 (m, 2H), 2.32-2.25 (m, 1H), 2.23-2.18 (m, 2H), 2.16-2.10 (m, 1H), 2.04 -1.95 (m, 2H), 1.84-1.67 (m, 7H), 1.31-1.18 (m, 3H).

### Example 122 Preparation of Compound H122

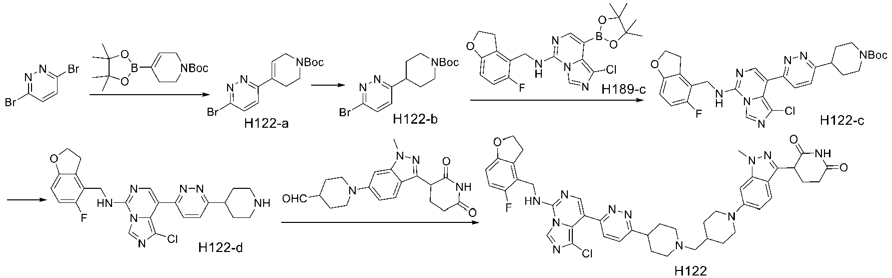

Step 1: Compound 3,6-dibromopyridazine (1180 mg, 5 mmol), tert-butyl 4-(4,4,5,5-tetramethyl-1,3,2-dioxaborolan-2-yl)-3,6-dihydropyridine-1(2H)-carboxylate (1545 mg, 5 mmol), K₂CO₃ (2070 mg, 15 mmol), and Pd(dppf)Cl₂ (365 mg, 0.5 mmol) were added to a mixed solvent of 1,4-dioxane (15 mL) and water (3 mL). After purging with nitrogen three times, the mixture was heated to 100°C and stirred for 5 hours. After the reaction was complete, the reaction solution was purified by silica gel column chromatography (30-100% PE/EA) to give H122-a (1190 mg), yield: 75%. MS m/z (ESI): 340 [M+H]⁺.

Step 2: H122-a (1190 mg, 3.5 mmol) and RhCl(PPh₃)₃ (150 mg) were added to methanol (5 mL), purged with hydrogen, and stirred overnight. After the reaction was complete, the reaction solution was filtered, the combined organic phases were concentrated to give H122-b (1193 mg), yield: 100%. MS m/z (ESI): 342 [M+H]⁺.

Steps 3-5: Referring to the preparation method of Example 189, following the above synthetic route, H122 was prepared. The purification conditions for preparative HPLC were: preparative column: waters-sunfire-10 µm-19×150 mm column, mobile phase: 28% - 38% (v/v) acetonitrile and aqueous formic acid. MS m/z (ESI): 818 [M+H]⁺. ¹H NMR (400 MHz, DMSO-d₆) δ 10.83 (s, 1H), 8.71 (s, 1H), 8.66 - 8.57 (m, 1H), 7.80 - 7.64 (m, 2H), 7.46 (d, J = 10.1 Hz, 2H), 6.92 (q, J = 9.1 Hz, 2H), 6.82 (d, J = 1.8 Hz, 1H), 6.69 (dd, J = 8.7, 3.9 Hz, 1H), 4.74 (d, J = 4.9 Hz, 2H), 4.53 (t, J = 8.7 Hz, 3H), 4.23 (dd, J = 9.1, 5.1 Hz, 1H), 3.87 (s, 3H), 3.78 (d, J = 12.1 Hz, 3H), 2.97 (d, J = 28.4 Hz, 2H), 2.73 (t, J = 12.0 Hz, 2H), 2.59 (t, J = 5.8 Hz, 2H), 2.36 - 2.10 (m, 4H), 1.92 - 1.78 (m, 5H), 1.22 (m, 7H).

### Example 123 Preparation of Compound H123

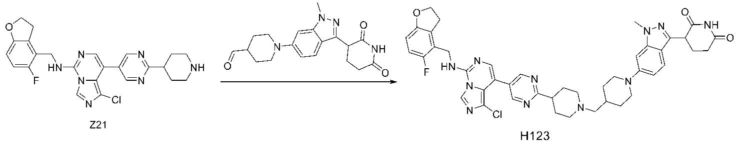

Compound Z21 (80 mg, 166.69 µmol) and 1-(3-(2,6-dioxopiperidin-3-yl)-1-methyl-1H-indazol-6-yl)piperidine-4-carbaldehyde (112.91 mg, 250.03 µmol) were dissolved in DCM (8 mL) and stirred at room temperature for 2 h. Then NaBH(OAc)₃ (141.31 mg, 666.76 µmol) was added, and the mixture was stirred at room temperature overnight. After the reaction was complete, the reaction solution was concentrated and preliminarily purified by CombiFlash (4 g, 0-12% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H123 (60 mg, purity 99.4%), yield: 43.72%. MS m/z (ESI): 818.3 [M+H]⁺. ¹H NMR (400 MHz, DMSO-d₆) δ 10.83 (s, 1H), 8.81 (s, 2H), 8.69 (s, 1H), 8.57 (s, 1H), 7.46 (d, J = 8.9 Hz, 1H), 7.30 (s, 1H), 6.98 - 6.86 (m, 2H), 6.81 (d, J = 1.9 Hz, 1H), 6.68 (dd, J = 8.7, 3.8 Hz, 1H), 4.71 (d, J = 4.2 Hz, 2H), 4.53 (t, J = 8.7 Hz, 2H), 4.23 (dd, J = 9.1, 5.1 Hz, 1H), 3.87 (s, 3H), 3.77 (d, J = 12.1 Hz, 2H), 3.28 (d, J = 8.7 Hz, 2H), 2.94 (d, J = 10.4 Hz, 2H), 2.89 - 2.78 (m, 1H), 2.72 (t, J = 12.0 Hz, 2H), 2.60 (q, J = 6.9, 5.5 Hz, 2H), 2.34 - 2.23 (m, 1H), 2.22 - 2.10 (m, 3H), 2.07 - 1.92 (m, 4H), 1.90 - 1.72 (m, 5H), 1.30 - 1.20 (m, 2H).

### Example 124 Preparation of Compound H124

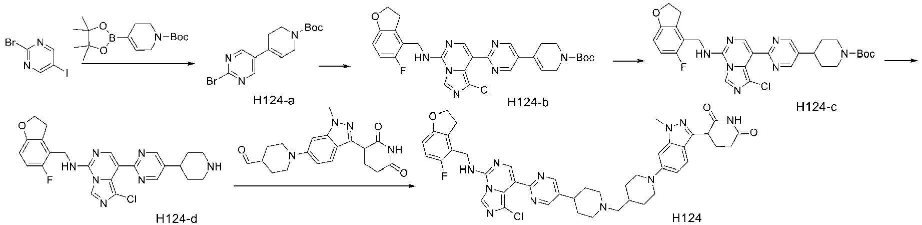

Step 1: 2-Bromo-5-iodopyrimidine (200 mg, 702.05 µmol) and tert-butyl 4-(4,4,5,5-tetramethyl-1,3,2-dioxaborolan-2-yl)-3,6-dihydro-2H-pyridine-1-carboxylate (238.79 mg, 772.25 µmol) were dissolved in water (2 mL) and 1,4-dioxane (10 mL). Under argon protection, Pd(dppf)Cl₂ (76.98 mg, 105.31 µmol) and K₂CO₃ (194.06 mg, 1.40 mmol) were added, and the temperature was raised to 80°C and stirred for 16 hours. After the reaction was complete, the reaction solution was filtered, and the filtrate was concentrated and purified by combi-flash (PE:EA = 2:1) to give H124-a (180 mg, yellow solid), yield: 75.36%. MS m/z (ESI): 342.0 [M+H]⁺.

Step 2: H124-a (200 mg, 502.98 µmol) was dissolved in water (1 mL) and DCE (10 mL). Palladium acetate (22.58 mg, 100.60 µmol), CataCXium A (36.11 mg, 100.60 µmol), K₂CO₃ (278.07 mg, 2.01 mmol), and (Pin)₂B₂ (267.23 mg, 1.06 mmol) were added, and the mixture was reacted at 75°C for 16 h. After the reaction was complete, the reaction solution was filtered, and the filtrate was concentrated and purified by combi-flash (PE:EA = 1:2) to give H124-b (50 mg, yellow oil), yield: 17.20%. MS m/z (ESI): 578.2 [M+H]⁺.

Step 3: H124-b (150 mg, 259.50 µmol) was dissolved in methanol (3 mL). Pd/C (15 mg, 25.95 µmol) was added, and the mixture was stirred at room temperature for 16 hours under hydrogen protection. After the reaction was complete, the reaction solution was filtered, and the filtrate was concentrated to give H124-c (crude). MS m/z (ESI): 580.2 [M+H]⁺.

Step 4: H124-c (150 mg, 258.60 µmol) was dissolved in DCM (3 mL), and TFA (1 mL) was added. The mixture was reacted at room temperature for 1 h. After the reaction was complete, the reaction solution was concentrated, the pH was adjusted to 8 with sodium bicarbonate, the aqueous phase was extracted with DCM, and the organic phase was concentrated to give H124-d (crude). MS m/z (ESI): 480.2 [M+H]⁺.

Step 5: H124-d (20 mg, 41.67 µmol) was dissolved in DCM (2 mL). 1-(3-(2,6-Dioxopiperidin-3-yl)-1-methyl-1H-indazol-6-yl)piperidine-4-carbaldehyde (28.23 mg, 62.51 µmol) was added, and the mixture was stirred at room temperature for 2 h. NaBH(OAc)₃ (35.33 mg, 166.69 µmol) was added, and the mixture was stirred at room temperature overnight. After the reaction was complete, the reaction solution was concentrated and preliminarily purified by CombiFlash (4 g, 0-12% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H124 (1.05 mg, purity 92.7%), yield: 2.85%. MS m/z (ESI): 818.3 [M+H]⁺. ¹H NMR (400 MHz, DMSO-d₆) δ 10.83 (s, 1H), 8.79 (s, 2H), 8.68 (s, 1H), 8.63 (s, 1H), 7.68 (s, 1H), 7.46 (d, J = 9.0 Hz, 1H), 6.92 (d, J = 9.6 Hz, 2H), 6.81 (s, 1H), 6.71 - 6.66 (m, 1H), 4.74 (d, J = 4.8 Hz, 2H), 4.52 (t, J = 8.7 Hz, 2H), 4.26 - 4.21 (m, 1H), 3.87 (s, 3H), 3.77 (d, J = 11.9 Hz, 2H), 2.98 (d, J = 10.7 Hz, 2H), 2.72 (s, 3H), 2.67 - 2.64 (m, 2H), 2.60 (d, J = 7.4 Hz, 4H), 2.33 - 2.29 (m, 2H), 2.21 (d, J = 6.8 Hz, 2H), 1.98 (d, J = 8.2 Hz, 4H), 1.81 (s, 3H), 1.74 (d, J = 11.2 Hz, 2H).

### Example 125 to Example 127

Compounds H125, H126 and H127 were prepared using Z32, Z33 and Z34 as starting materials, respectively, with the corresponding aldehydes, referring to the method of Example 71.

| | |
|---|---|
| 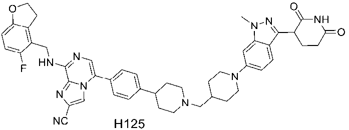 | MS m/z (ESI):808.4[M+H]⁺. |
| | ¹H NMR (400 MHz, DMSO-d₆) δ 10.83 (s, 1H), 8.69 (s, 1H), 8.40 (t, *J =* 6.0 Hz, 1H), 7.56 (d, *J =* 8.0 Hz, 2H), 7.46 (d, *J =* 9.2 Hz, 1H), 7.44 - 7.38 (m, 3H), 6.92 - 6.80 (m, 3H), 6.61 (dd, *J =* 8.4, 4.0 Hz, 1H), 4.67 (d, *J =* 6.0 Hz, 2H), 4.49 (t, *J =* 8.8 Hz, 2H), 4.23 (dd, *J* = 9.2, 5.2 Hz, 1H), 3.87 (s, 3H), 3.78 (d, *J =* 12.0 Hz, 2H), 3.28 (d, *J* = 9.2 Hz, 2H), 2.98 (d, *J* = 10.4 Hz, 2H), 2.73 (t, *J* = 12.0 Hz, 2H), 2.59 (tt, *J* = 11.6, 5.9 Hz, 3H), 2.35 - 2.09 (m, 4H), 2.00 (s, 2H), 1.88 - 1.63 (m, 7H), 1.26 (d, *J* = 11.2 Hz, 2H). |
| 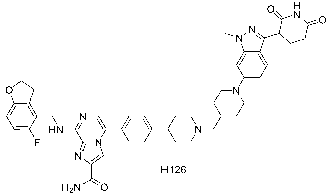 | MS m/z (ESI):826.4[M+H]⁺ |
| | ¹H NMR (400 MHz, DMSO-d₆) δ 10.82 (s, 1H), 8.12 (s, 1H), 7.74 (t, *J =* 6.0 Hz, 1H), 7.63 - 7.54 (m, 3H), 7.49 - 7.41 (m, 4H), 7.37 (s, 1H), 6.93 - 6.85 (m, 2H), 6.81 (d, *J =* 2.0 Hz, 1H), 6.63 (dd, *J =* 8.8, 4.0 Hz, 1H), 4.69 (d, *J =* 5.6 Hz, 2H), 4.51 (t, *J =* 8.8 Hz, 2H), 4.23 (dd, *J* = 9.2, 5.2 Hz, 1H), 3.87 (s, 3H), 3.78 (d, *J* = 12.0 Hz, 2H), 3.32 (d, *J* = 8.8 Hz, 2H), 2.98 (d, *J* = 10.4 Hz, 2H), 2.73 (t, *J* = 12.0 Hz, 2H), 2.60 (dd, *J =* 8.4, 4.0 Hz, 3H), 2.34 - 2.09 (m, 4H), 2.00 (t, *J* = 11.2 Hz, 2H), 1.88 - 1.68 (m, 7H), 1.31 - 1.19 (m, 2H). |
| 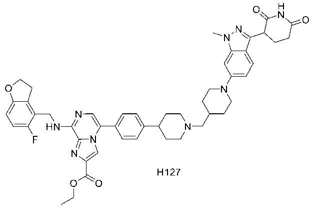 | MS m/z (ESI):854.4[M+H]⁺ |
| | ¹H NMR (400 MHz, DMSO-d₆) δ 10.82 (s, 1H), 8.33 (t, *J =* 6.0 Hz, 1H), 8.12 (s, 1H), 7.60 - 7.54 (m, 2H), 7.45 (dd, *J =* 11.6, 8.4 Hz, 3H), 7.37 (s, 1H), 6.95 - 6.76 (m, 3H), 6.61 (dd, *J =* 8.4, 4.0 Hz, 1H), 4.67 (d, *J =* 6.0 Hz, 2H), 4.49 (t, *J =* 8.8 Hz, 2H), 4.29 (q, *J* = 7.2 Hz, 2H), 4.23 (dd, *J* = 9.2, 5.2 Hz, 1H), 3.87 (s, 3H), 3.78 (d, *J =* 12.0 Hz, 2H), 3.32 (s, 1H), 3.27 (s, 1H), 2.98 (d, *J* = 10.4 Hz, 2H), 2.73 (t, *J =* 12.0 Hz, 2H), 2.67 - 2.54 (m, 3H), 2.34 - 2.10 (m, 4H), 2.00 (t, *J* = 11.2 Hz, 2H), 1.85-1.67 (m, 7H), 1.30 - 1.22 (m, 5H). |

### Example 128 Preparation of Compound H128

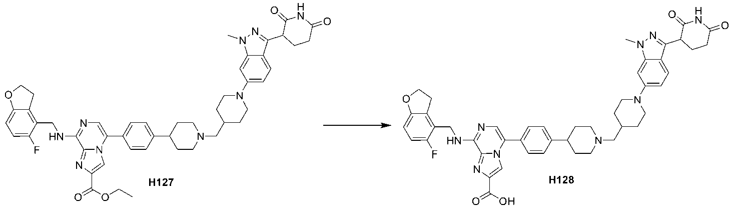

H127 (50 mg, 58.55 µmol) was dissolved in THF (5 mL). Potassium trimethylsilanolate (37.56 mg, 292.75 µmol) was added, and the mixture was stirred at room temperature for 1 hour. The pH was adjusted to weakly acidic with dilute hydrochloric acid, and the mixture was concentrated under reduced pressure to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give H128 (9.55 mg, 11.56 µmol, 19.75% yield, 100% purity) as a white solid. MS m/z (ESI): 827.4 [M+H]⁺. ¹H NMR (400 MHz, DMSO-d₆) δ 10.83 (s, 1H), 8.22 (s, 1H), 8.07 (s, 1H), 7.56 (d, J = 8.0 Hz, 2H), 7.46 (d, J = 8.8 Hz, 1H), 7.42 (d, J = 8.0 Hz, 2H), 7.36 (s, 1H), 6.94 - 6.79 (m, 3H), 6.61 (dd, J = 8.8, 3.6 Hz, 1H), 4.68 (d, J = 5.6 Hz, 2H), 4.49 (t, J = 8.8 Hz, 2H), 4.23 (dd, J = 9.2, 5.2 Hz, 1H), 3.87 (s, 3H), 3.78 (d, J = 12.0 Hz, 2H), 3.28 (s, 3H), 3.00 (d, J = 10.4 Hz, 2H), 2.73 (t, J = 12.0 Hz, 2H), 2.60 (dd, J = 8.8, 4.0 Hz, 3H), 2.33 - 2.21 (m, 3H), 2.14 (dd, J = 13.2, 6.0 Hz, 1H), 2.04 (t, J = 11.2 Hz, 2H), 1.90 - 1.61 (m, 8H), 1.32 - 1.19 (m, 2H).

### Example 129 Preparation of Compound H129

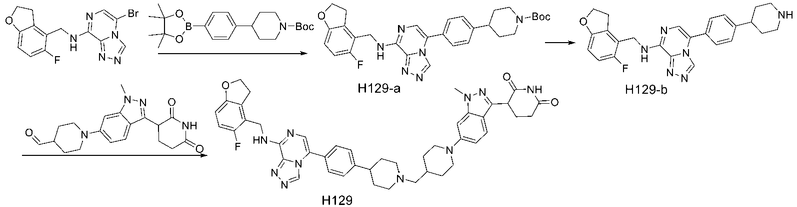

Referring to the preparation method of Example 49, following the above synthetic route, H129 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 783.4 [M+H]⁺. ¹H NMR (400 MHz, DMSO-d₆) δ 10.83 (s, 1H), 9.25 (s, 1H), 8.59 (t, J = 5.6 Hz, 1H), 7.59 (d, J = 8.2 Hz, 2H), 7.46 (d, J = 9.0 Hz, 1H), 7.41 (d, J = 8.4 Hz, 2H), 7.34 (s, 1H), 6.91-6.84 (m, 2H), 6.81 (d, J = 1.2 Hz, 1H), 6.62 (dd, J = 8.6, 3.8 Hz, 1H), 4.70 (d, J = 5.6 Hz, 2H), 4.49 (t, J = 8.8 Hz, 2H), 4.23 (dd, J = 9.2, 5.2 Hz, 1H), 3.87 (s, 3H), 3.77 (d, J = 12.4 Hz, 2H), 3.32-3.27 (m, 2H), 2.97 (d, J = 11.2 Hz, 2H), 2.72 (t, J = 12.0 Hz, 2H), 2.62-2.56 (m, 2H), 2.32 - 2.25 (m, 1H), 2.20 (d, J = 7.0 Hz, 2H), 2.17 - 2.11 (m, 1H), 1.99 (t, J = 11.2 Hz, 2H), 1.85 - 1.66 (m, 7H), 1.29-1.20 (m, 3H).

### Example 130 Preparation of Compound H130

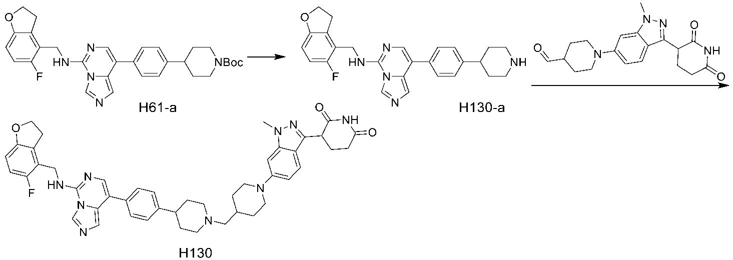

Referring to the preparation method of Example 49, following the above synthetic route, H130 was prepared. The purification conditions for preparative HPLC were preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 783.4 [M+H]⁺. ¹H NMR (400 MHz, DMSO-d₆) δ 10.83 (s, 1H), 8.67 (s, 1H), 8.27 (t, J = 5.2 Hz, 1H), 7.59 - 7.54 (m, 2H), 7.46 (d, J = 9.2 Hz, 1H), 7.41 (s, 1H), 7.34 (t, J = 4.0 Hz, 3H), 6.96 - 6.87 (m, 2H), 6.84 - 6.79 (m, 1H), 6.68 (dd, J = 8.8, 4.0 Hz, 1H), 4.71 (d, J = 5.2 Hz, 2H), 4.53 (t, J = 8.8 Hz, 2H), 4.23 (dd, J = 9.2, 5.2 Hz, 1H), 3.87 (s, 3H), 3.78 (d, J = 12.0 Hz, 2H), 3.28 (d, J = 8.8 Hz, 2H), 2.97 (d, J = 10.4 Hz, 2H), 2.72 (t, J = 12.0 Hz, 2H), 2.66-2.54 (m, 3H), 2.36 - 2.24 (m, 1H), 2.22-2.10 (m, 3H), 1.99 (t, J = 11.2 Hz, 2H), 1.86-1.65 (m, 7H), 1.31-1.18 (m, 2H).

### Example 131 Preparation of Compound H131

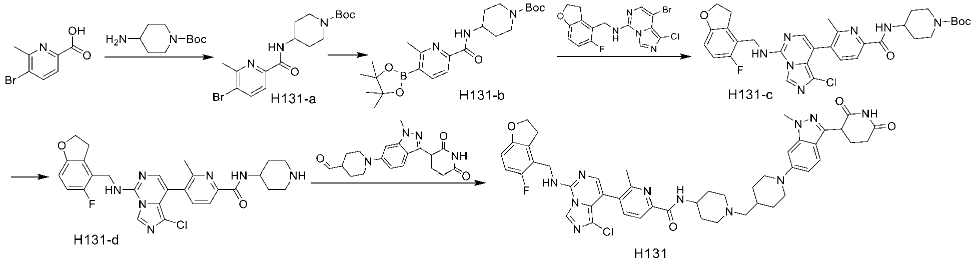

Step 1: 5-Bromo-6-methylpicolinic acid (0.9 g, 4.17 mmol) was dissolved in DMF (20 mL). tert-Butyl 4-aminopiperidine-1-carboxylate (1.25 g, 6.25 mmol), TEA (1.26 g, 12.50 mmol, 1.74 mL), and HATU (2.51 g, 6.67 mmol) were added, and the mixture was stirred at room temperature for 1 hour. After the reaction was complete, water and ethyl acetate were added to the reaction solution, extraction was performed three times with ethyl acetate, the combined organic phases were washed with saturated brine, dried over anhydrous sodium sulfate, concentrated, and purified by CombiFlash (12 g, 0-20% EA/PE) to give H131-a (1.4 g, light yellow solid), yield: 84.37%. MS m/z (ESI): 342.0 [M-56+H]⁺.

Step 2: H131-a (0.5 g, 1.26 mmol) and B₂(Pin)₂ (478.17 mg, 1.88 mmol) were dissolved in 1,4-dioxane (10 mL). Under argon protection, KOAc (226.16 mg, 3.77 mmol) and Pd(dppf)Cl₂ (91.85 mg, 125.54 µmol) were added, and the temperature was raised to 95°C and stirred overnight. After the reaction was complete, the reaction solution was filtered, and the filtrate was concentrated and purified by CombiFlash (12 g, 0-40% EA/PE) to give H131-b (0.4 g, colorless oil), yield: 71.55%. MS m/z (ESI): 446.3 [M+H]⁺.

Step 3: H131-b (0.3 g, 754.47 µmol) and 8-bromo-1-chloro-N-((5-fluoro-2,3-dihydrobenzofuran-4-yl)methyl)imidazo[1,5-c]pyrimidin-5-amine (403.21 mg, 905.37 µmol) were dissolved in 1,4-dioxane (10 mL) and water (1 mL). Under argon protection, Pd(dppf)Cl₂ (55.20 mg, 75.45 µmol) and K₂CO₃ (312.82 mg, 2.26 mmol) were added, and the temperature was raised to 100°C and stirred for 2 hours. After the reaction was complete, the reaction solution was concentrated, water and ethyl acetate were added, extraction was performed three times with ethyl acetate, the combined organic phases were dried over anhydrous sodium sulfate, concentrated, and purified by CombiFlash (12 g, 0-50% EA/PE) to give H131-c (0.3 g, light yellow solid), yield: 62.51%. MS m/z (ESI): 636.3 [M+H]⁺.

Step 4: H131-c (0.3 g, 471.61 µmol) was dissolved in DCM (6 mL). TFA (2 mL) was added, and the mixture was stirred at room temperature for 2 hours. After the reaction was complete, the reaction solution was concentrated, water and ethyl acetate were added, extraction was performed three times with ethyl acetate, the combined organic phases were dried over anhydrous sodium sulfate, concentrated, and purified by CombiFlash (12 g, 0-50% EA/PE) to give H131-d (0.22 g, light yellow solid), yield: 87.03%. MS m/z (ESI): 536.2 [M+H]⁺.

Step 5: H131-d (0.1 g, 186.57 µmol) and 1-(3-(2,6-dioxopiperidin-3-yl)-1-methyl-1H-indazol-6-yl)piperidine-4-carbaldehyde (99.18 mg, 279.85 µmol) were dissolved in DCM (5 mL). NaBH(OAc)₃ (197.71 mg, 932.84 µmol) was added, and the mixture was stirred at room temperature for 1 hour. After the reaction was complete, saturated aqueous sodium bicarbonate solution was added to the reaction solution, extraction was performed three times with dichloromethane, the combined organic phases were dried over anhydrous sodium sulfate, concentrated, and purified by CombiFlash (4 g, 0-15% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H131 (70.0 mg, purity: 100%), yield: 42.91%. MS m/z (ESI): 874.4 [M+H]⁺. ¹H NMR (400 MHz, DMSO-d₆) δ 10.83 (s, 1H), 8.67 (s, 1H), 8.50 (t, J = 5.2 Hz, 1H), 8.44 (d, J = 8.4 Hz, 1H), 7.89 (d, J = 7.6 Hz, 1H), 7.77 (d, J = 7.6 Hz, 1H), 7.46 (d, J = 8.8 Hz, 1H), 7.14 (s, 1H), 6.99 - 6.87 (m, 2H), 6.81 (d, J = 2.0 Hz, 1H), 6.69 (dd, J = 8.4, 4.0 Hz, 1H), 4.77-4.63 (m, 2H), 4.54 (t, J = 8.8 Hz, 2H), 4.23 (dd, J = 9.2, 5.2 Hz, 1H), 3.87 (s, 3H), 3.77 (d, J = 12.0 Hz, 3H), 3.33 (s, 1H), 3.29 (s, 1H), 2.84 (s, 2H), 2.72 (t, J = 12.0 Hz, 2H), 2.65-2.54 (m, 2H), 2.37 (s, 3H), 2.34-2.24 (m, 1H), 2.23 - 2.09 (m, 3H), 2.07-1.94 (m, 2H), 1.87 - 1.62 (m, 7H), 1.32-1.18 (m, 2H).

### Example 132 Preparation of Compound H132

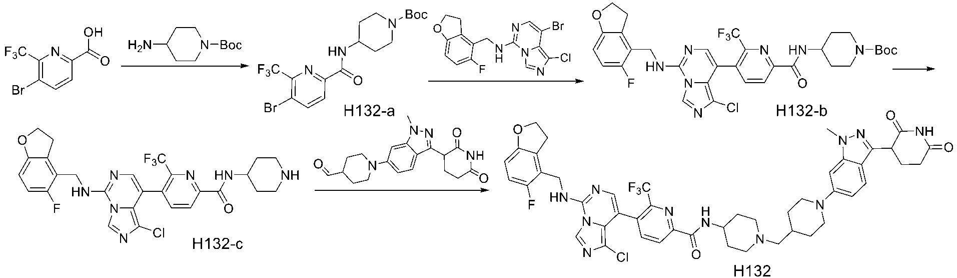

Step 1: At room temperature, 5-bromo-6-(trifluoromethyl)picolinic acid (2 g, 7.41 mmol), tert-butyl 4-aminopiperidine-1-carboxylate (1.56 g, 7.78 mmol), HATU (3.07 g, 8.15 mmol), TEA (1.50 g, 14.81 mmol, 2.07 mL), and DMF (20 mL) were mixed and reacted at room temperature for 1 hour. After the reaction was complete, the reaction solution was poured into ice water, extracted with ethyl acetate (30 mL×2), the combined organic layers were washed with water (15 mL×3) and saturated brine, concentrated, and purified by silica gel column chromatography (PE:EA = 85%:15%) to give H132-a (2.35 g, colorless oil), yield: 70.15%. MS m/z (ESI): 396.0 [M+H-56]⁺.

Step 2: Under argon protection, 8-bromo-1-chloro-N-((5-fluoro-2,3-dihydrobenzofuran-4-yl)methyl)imidazo[1,5-c]pyrimidin-5-amine (200 mg, 502.98 µmol), H132-a (272.98 mg, 603.58 µmol), Pd(OAc)₂ (11.29 mg, 50.30 µmol), CataCXium A (36.07 mg, 100.60 µmol), K₂CO₃ (208.54 mg, 1.51 mmol), (Pin)₂B₂ (319.31 mg, 1.26 mmol), DME (20 mL), DMSO (1 mL), and water (2 mL) were mixed and heated to 80°C for reaction for 16 hours. After the reaction was complete, the reaction solution was separated, the organic phase was concentrated, and purified by silica gel column chromatography (PE:EA = 50%:50%) to give H132-b (50 mg, brown liquid), yield: 14.41%. MS m/z (ESI): 634.2 [M+H-56]⁺.

Step 3: At room temperature, H132-b (45 mg, 65.21 µmol), DCM (1 mL), and TFA (0.5 mL) were mixed and stirred for 1 hour. After the reaction was complete, the reaction solution was concentrated, a small amount of dichloromethane was added, the pH was adjusted to 8-9 with saturated aqueous sodium bicarbonate solution, extraction was performed with organic solvent (DCM:MeOH = 10:1) (10 mL×2), the combined organic phases were dried over anhydrous sodium sulfate, and concentrated to give H132-c (36 mg, yellow oil), yield: 93.58%. MS m/z (ESI): 590.2 [M+H]⁺.

Step 4: At room temperature, H132-c (50 mg, 84.75 µmol), 1-(3-(2,6-dioxopiperidin-3-yl)-1-methyl-1H-indazol-6-yl)piperidine-4-carbaldehyde (39.05 mg, 110.17 µmol), NaBH(OAc)₃ (53.89 mg, 254.25 µmol), and DCM (3 mL) were mixed and stirred for 1 hour. After the reaction was complete, water was added to quench the reaction, the mixture was concentrated, and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H132 (19.25 mg), yield: 24.25%. MS m/z (ESI): 464.8 [M/2+H]⁺. ¹H NMR (400 MHz, DMSO-d₆) δ 10.82 (s, 1H), 8.67 (s, 1H), 8.59 (t, J = 4.8 Hz, 1H), 8.44 (d, J = 8.2 Hz, 1H), 8.30 (d, J = 8.0 Hz, 1H), 8.17 (d, J = 8.0 Hz, 1H), 7.46 (d, J = 9.0 Hz, 1H), 7.16 (s, 1H), 6.97 - 6.87 (m, 2H), 6.81 (s, 1H), 6.70 (dd, J = 8.6, 4.0 Hz, 1H), 4.71 (ddd, J = 19.2, 14.4, 4.8 Hz, 2H), 4.54 (t, J = 8.8 Hz, 2H), 4.23 (dd, J = 8.8, 4.8 Hz, 1H), 3.87 (s, 3H), 3.77 (d, J = 12.4 Hz, 2H), 3.31 - 3.25 (m, 2H), 2.84 (d, J = 10.2 Hz, 2H), 2.72 (t, J = 12.0 Hz, 2H), 2.63 - 2.56 (m, 2H), 2.34 - 2.22 (m, 2H), 2.21 - 2.11 (m, 3H), 2.02 (t, J = 11.2 Hz, 2H), 1.83 - 1.67 (m, 6H), 1.31 - 1.17 (m, 3H).

### Example 133 Preparation of Compound H133

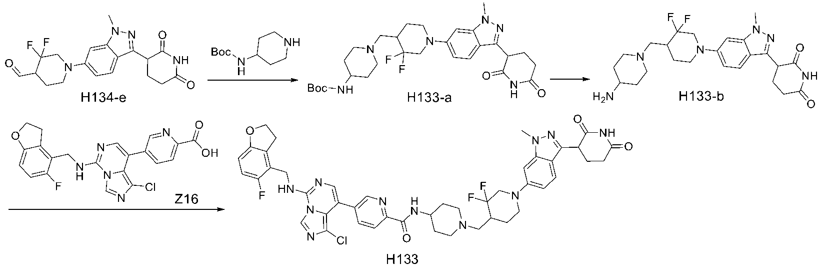

Step 1: H134-e (50 mg, 128.08 µmol) and tert-butyl N-(piperidin-4-yl)carbamate (76.95 mg, 384.24 µmol) were dissolved in dichloromethane (10 mL). NaBH(OAc)₃ (81.44 mg, 384.24 µmol) was added with stirring at room temperature, and the mixture was stirred at room temperature overnight. After the reaction was complete, the reaction solution was concentrated and purified by silica gel column chromatography (MeOH:DCM = 0-10%) to give H133-a (30 mg, yellow solid), yield: 40.76%. MS m/z (ESI): 575 [M+H]⁺.

Step 2: H133-a (25 mg, 43.50 µmol) was dissolved in dichloromethane (9.98 mL) and methanol (2 mL). A solution of hydrogen chloride in dioxane (4.0 mol/L, 3 mL) was added with stirring at room temperature, and the mixture was stirred at room temperature for 2 hours. After the reaction was complete, the reaction solution was concentrated to give H133-b (20.64 mg, yellow solid), yield: 100.00%. MS m/z (ESI): 475 [M+H]⁺.

Step 3: Z16 (46.34 mg, 105.36 µmol) and H133-b (25 mg, 52.68 µmol) were dissolved in DMF (6 mL). HATU (39.75 mg, 105.36 µmol) and DIPEA (68.09 mg, 526.82 µmol, 91.76 µL) were added with stirring at room temperature, and the mixture was stirred at room temperature overnight. After the reaction was complete, the reaction solution was purified by HPLC to give H133 (7.74 mg, purity 100%), yield: 16.39%. MS m/z (ESI): 897 [M+H]⁺. ¹H NMR (400 MHz, DMSO-d₆) δ 10.84 (s, 1H), 9.85 (s, 1H), 8.98 (s, 1H), 8.72 (d, J = 23.4 Hz, 3H), 8.06 (dd, J = 19.6, 7.9 Hz, 2H), 7.51 (d, J = 9.2 Hz, 1H), 7.30 (s, 1H), 7.00 - 6.86 (m, 2H), 6.68 (dd, J = 8.6, 3.8 Hz, 1H), 4.72 (d, J = 4.8 Hz, 2H), 4.53 (t, J = 8.7 Hz, 2H), 4.26 (dd, J = 9.3, 5.1 Hz, 1H), 4.14 - 3.37 (m, 8H), 3.20 (d, J = 58.9 Hz, 3H), 2.96 - 2.53 (m, 5H), 2.36 - 1.81 (m, 6H), 1.76 (s, 2H), 1.56 - 1.11 (m, 3H).

### Example 134 Preparation of Compound H134

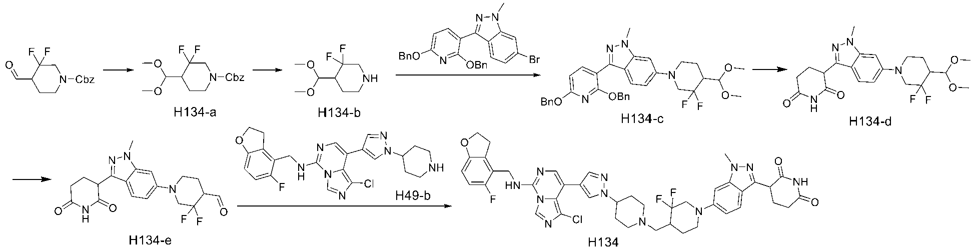

Step 1: Benzyl 3,3-difluoro-4-formylpiperidine-1-carboxylate (2.7 g, 9.53 mmol) was dissolved in methanol (50 mL). Trimethoxymethane (5.06 g, 47.66 mmol) and 4-methylbenzenesulfonic acid (82.07 mg, 476.58 µmol) were added with stirring at room temperature, and the mixture was stirred at room temperature overnight. After the reaction was complete, the reaction solution was concentrated and purified by silica gel column chromatography (EA:PE = 0-50%) to give H134-a (2.7 g, yellow oil), yield: 86.01%. MS m/z (ESI): 330 [M+H]⁺.

Step 2: H134-a (830 mg, 2.52 mmol) was dissolved in methanol (20 mL). Palladium on carbon (576.39 mg, 541.62 µmol, 10% purity) was added, and the mixture was stirred at room temperature for 2 hours under hydrogen protection. After the reaction was complete, the reaction solution was filtered, and the filtrate was concentrated to give H134-b (400 mg, colorless oil), yield: 81.31%. MS m/z (ESI): 196 [M+H]⁺.

Step 3: 6-Bromo-3-(2,6-dibenzyloxypyridin-3-yl)-1-methylindazole (1.0 g, 2.00 mmol) and H134-b (507.15 mg, 2.60 mmol) were dissolved in dioxane (5 mL). Under argon protection, Pd₂(dba)₃ (183.00 mg, 199.85 µmol), Xphos (190.54 mg, 399.69 µmol), and Cs₂CO₃ (1.30 g, 4.00 mmol) were added, and the temperature was raised to 100°C and stirred overnight. After the reaction was complete, the reaction solution was concentrated, water and ethyl acetate were added, extraction was performed three times with ethyl acetate, the combined organic phases were washed with brine, dried over anhydrous sodium sulfate, concentrated, and purified by silica gel column chromatography (80 g, 0-40% EA/PE) to give H134-c (1.1 g), yield: 89.55%.

Step 4: H134-c (1.1 g, 1.79 mmol) was dissolved in ethanol (20 mL) and tetrahydrofuran (2 mL). Palladium on carbon (190.44 mg, 178.95 µmol, 10% purity) was added, the mixture was purged with hydrogen three times, and stirred at room temperature overnight under a hydrogen atmosphere. After the reaction was complete, the reaction solution was filtered, and the filtrate was concentrated to give H134-d (700 mg, crude), yield: 89.62%.

Step 5: H134-d (200 mg, 458.24 µmol) was dissolved in formic acid (5 mL), heated to 40°C, and stirred for 2 hours. After the reaction was complete, the reaction solution was concentrated to give H134-e (178.89 mg, yellow solid), yield: 100.00%. MS m/z (ESI): 391 [M+H]⁺.

Step 6: H49-b (50 mg, 106.85 µmol) and H134-e (83.43 mg, 213.71 µmol) were dissolved in dichloromethane (5 mL) and methanol (5 mL). NaBH(OAc)₃ (67.94 mg, 320.56 µmol) was added with stirring at room temperature, and the mixture was stirred at room temperature overnight. After the reaction was complete, the reaction solution was concentrated and purified by preparative HPLC to give H134 (2.66 mg, purity 97.63%), yield: 2.89%. MS m/z (ESI): 842 [M+H]⁺. ¹H NMR (400 MHz, DMSO-d₆) δ 10.84 (s, 1H), 8.62 (s, 1H), 8.30 (d, J = 5.1 Hz, 1H), 7.95 (s, 1H), 7.57 (s, 1H), 7.50 (d, J = 9.5 Hz, 1H), 7.16 (s, 1H), 6.92 (t, J = 9.4 Hz, 3H), 6.68 (dd, J = 8.6, 3.9 Hz, 1H), 4.67 (d, J = 4.8 Hz, 2H), 4.52 (t, J = 8.7 Hz, 2H), 4.29 - 4.14 (m, 2H), 3.96 (s, 1H), 3.86 (d, J = 28.4 Hz, 3H), 3.81 (d, J = 11.5 Hz, 1H), 3.27 - 3.00 (m, 4H), 2.90 (s, 2H), 2.60 (dd, J = 21.7, 15.8 Hz, 3H), 2.43 - 2.21 (m, 4H), 2.21 - 1.86 (m, 7H), 1.57 (d, J = 12.5 Hz, 1H).

### Example 135 Preparation of Compound H135

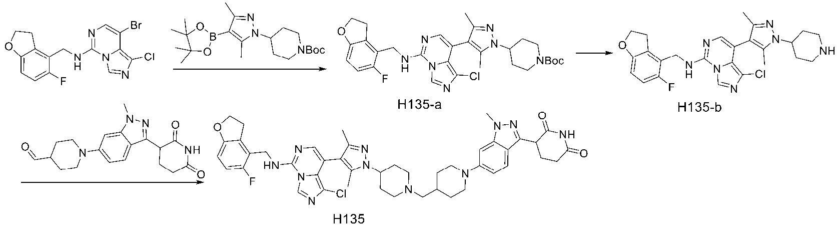

Referring to the synthetic method of Example 49, following the above synthetic route, H135 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 835.4 [M+H]⁺. ¹H NMR (400 MHz, DMSO-d₆) δ 10.84 (s, 1H), 8.59 (s, 1H), 8.27 (t, J = 5.0 Hz, 1H), 7.48 (d, J = 9.0 Hz, 1H), 7.00 (s, 1H), 6.94 (dd, J = 18.5, 8.9 Hz, 2H), 6.83 (s, 1H), 6.71 (dd, J = 8.7, 3.8 Hz, 1H), 4.68 (d, J = 4.9 Hz, 2H), 4.55 (t, J = 8.7 Hz, 2H), 4.25 (dd, J = 9.0, 5.1 Hz, 1H), 4.10 - 4.00 (m, 1H), 3.89 (s, 3H), 3.79 (d, J = 12.5 Hz, 2H), 3.34 (s, 1H), 3.30 - 3.28 (m, 1H), 3.00 - 2.92 (m, 2H), 2.75 (t, J = 11.5 Hz, 2H), 2.69 - 2.58 (m, 2H), 2.30 (dd, J = 13.8, 5.3 Hz, 1H), 2.24 - 2.12 (m, 3H), 2.07 (s, 6H), 1.98 (s, 3H), 1.78 (ddd, J = 28.7, 19.1, 4.1 Hz, 5H), 1.34 - 1.21 (m, 3H).

### Example 136 Preparation of Compound H136

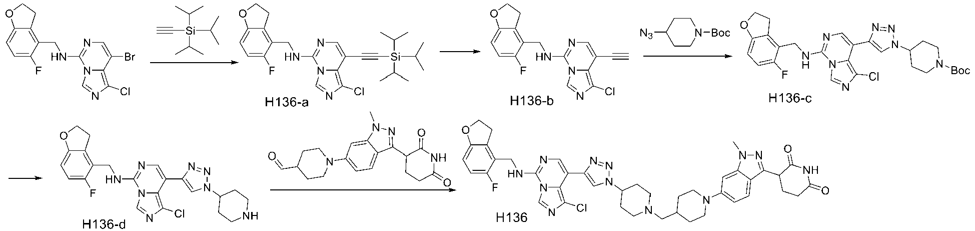

Step 1: 8-Bromo-1-chloro-N-((5-fluoro-2,3-dihydrobenzofuran-4-yl)methyl)imidazo[1,5-c] pyrimidin-5-amine (400 mg, 1.01 mmol) and triisopropylsilylacetylene (550.39 mg, 3.02 mmol) were dissolved in THF (15.22 mL). PdCl₂(dppf) (70.61 mg, 100.60 µmol), CuI (38.32 mg, 201.19 µmol), and DIPEA (650.05 mg, 5.03 mmol, 876.08 µL) were added. After purging with argon, the mixture was stirred under reflux at 85°C for 48 hours. After the reaction was complete, the reaction solution was concentrated and purified by combiflash (EA:PE = 0-40%) to give H136-a (395 mg, yellow solid), yield: 78.67%. MS m/z (ESI): 499.2 [M+H]⁺.

Step 2: H136-a (385 mg, 771.40 µmol) was dissolved in THF (15 mL). TBAF (1 mol/L, 1.16 mL) was added, and the mixture was stirred at room temperature (25°C) for 15 hours. After the reaction was complete, ethyl acetate (30 mL) was added to the reaction solution, washed with water (20 mL×1) and saturated brine (20 mL×1), dried over anhydrous sodium sulfate, and concentrated to give H136-b (264 mg, yellow solid, crude). MS m/z (ESI): 343.1 [M+H]⁺.

Step 3: H136-b (254 mg, 741.06 µmol) and tert-butyl 4-azidopiperidine-1-carboxylate (335.36 mg, 1.48 mmol) were dissolved in tert-butanol (10 mL) and water (5 mL). L-Ascorbic acid sodium salt (44.04 mg, 222.32 µmol) and copper(II) sulfate pentahydrate (92.52 mg, 370.53 µmol) were added. After purging with argon, the mixture was stirred at 70°C for 8 hours. After the reaction was complete, the reaction solution was concentrated and purified by combiflash (EA:PE = 0-80%) to give H136-c (101 mg, yellow solid), yield: 23.95%. MS m/z (ESI): 569.2 [M+H]⁺.

Step 4: H136-c (101 mg, 177.50 µmol) was dissolved in DCM (5 mL). TFA (20.24 mg, 177.50 µmol, 1 mL) was added at room temperature (25°C), and the mixture was stirred at room temperature (25°C) for 1 hour. After the reaction was complete, the reaction solution was concentrated, triethylamine (1 mL) was added to basify the product, and the mixture was concentrated to give H136-d (83 mg, brown solid, crude). MS m/z (ESI): 469.2 [M+H]⁺.

Step 5: H136-d (40 mg, 85.30 µmol) and 1-[3-(2,6-dioxopiperidin-3-yl)-1-methylindazol-6-yl]piperidine-4-carbaldehyde (45.35 mg, 127.96 µmol) were dissolved in DCM (5 mL). After stirring at room temperature (25°C) for 0.5 hour, NaBH(OAc)₃ (54.24 mg, 255.91 µmol) was added, and stirring was continued at room temperature (25°C) for 1 hour. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 60%-67% acetonitrile change) to give H136 (16.58 mg, purity: 100%), yield: 24.08%. MS m/z (ESI): 807.3 [M+H]⁺. ¹H NMR (400 MHz, DMSO-d₆) δ 10.81 (s, 1H), 8.65 (s, 1H), 8.46 (t, J = 5.1 Hz, 1H), 8.35 (s, 1H), 7.46 (d, J = 8.9 Hz, 1H), 7.36 (s, 1H), 6.97 - 6.86 (m, 2H), 6.81 (d, J = 1.9 Hz, 1H), 6.68 (dd, J = 8.6, 3.9 Hz, 1H), 4.71 (d, J = 5.0 Hz, 2H), 4.52 (t, J = 8.7 Hz, 3H), 4.23 (dd, J = 9.1, 5.1 Hz, 1H), 3.87 (s, 3H), 3.77 (d, J = 12.3 Hz, 2H), 3.27 (d, J = 8.8 Hz, 2H), 2.96 (d, J = 10.1 Hz, 2H), 2.73 (t, J = 12.0 Hz, 2H), 2.65 - 2.55 (m, 2H), 2.35 - 2.20 (m, 3H), 2.10 (q, J = 14.9, 13.7 Hz, 7H), 1.82 (d, J = 12.7 Hz, 2H), 1.71 (s, 1H), 1.32 - 1.18 (m, 2H).

### Example 137 Preparation of Compound H137

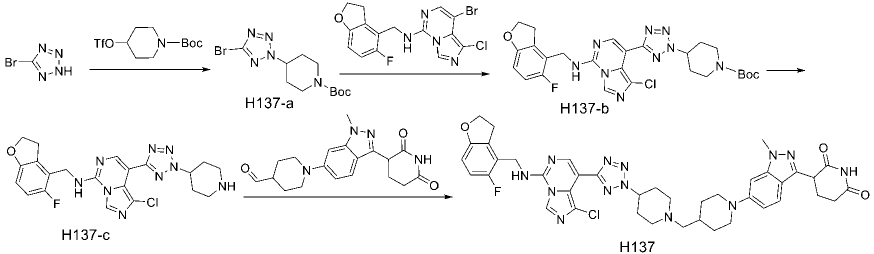

Step 1: 5-Bromo-2H-tetrazole (3.5 g, 23.50 mmol) and tert-butyl 4-(methylsulfonyloxy)piperidine-1-carboxylate (7.88 g, 28.20 mmol) were dissolved in DMF (50 mL). Cs₂CO₃ (15.31 g, 47.00 mmol) was added. After purging with argon, the mixture was stirred at 100°C for 16 hours. After the reaction was complete, the reaction solution was concentrated, H₂O (200 mL) was added, extraction was performed three times with EA (100 mL×3), the combined organic phases were dried over anhydrous sodium sulfate, concentrated, and purified by CombiFlash (40 g, 0-50% PE/THF) to give H137-a (4 g, colorless oil), yield: 51.24%. MS m/z (ESI): 332.1 [M+H]⁺. ¹H NMR (400 MHz, CDCl₃) δ 4.91 - 4.78 (m, 1H), 4.16 (d, J = 10.1 Hz, 2H), 3.02 (t, J = 11.7 Hz, 2H), 2.30 - 2.03 (m, 4H), 1.46 (s, 9H).

Step 2: 8-Bromo-1-chloro-N-((5-fluoro-2,3-dihydrobenzofuran-4-yl)methyl)imidazo[1,5-c] pyrimidin-5-amine (200 mg, 502.98 µmol) and H137-a (501.27 mg, 1.51 mmol) were dissolved in DME (10 mL) and water (1 mL). Pd(OAc)₂ (56.46 mg, 251.49 µmol), CataCXium A (108.20 mg, 301.79 µmol), K₂CO₃ (417.09 mg, 3.02 mmol), and (Pin)₂B₂ (510.90 mg, 2.01 mmol) were added, and the mixture was stirred at 70°C for 10 hours. After the reaction was complete, the reaction solution was concentrated and purified by CombiFlash (12 g, 0-100% EA/DCM) to give product H137-b (220 mg, light yellow solid), yield: 76.73%. MS m/z (ESI): 570.2 [M+H]⁺.

Step 3: H137-b (220 mg, 385.95 µmol) was dissolved in DCM (10 mL). HOAc (3 mL) was then added, and the mixture was stirred at room temperature for 2 hours. After the reaction was complete, the reaction solution was concentrated, neutralized with saturated aqueous sodium bicarbonate solution, extracted with dichloromethane (40 mL×3). The combined organic phases were dried over anhydrous sodium sulfate and concentrated to give H137-c (150 mg, light yellow solid), yield: 82.71%. MS m/z (ESI): 470.2 [M+H]⁺.

Step 4: H137-c (150 mg, 319.22 µmol) and 1-[3-(2,6-dioxopiperidin-3-yl)-1-methylindazol-6-yl]piperidine-4-carbaldehyde (147.07 mg, 414.98 µmol) were dissolved in DCM (15 mL). NaBH(OAc)₃ (202.96 mg, 957.65 µmol) was then added, and the mixture was stirred at room temperature for 2 hours. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H137 (74.10 mg), yield: 27.10%. MS m/z (ESI): 808.3 [M+H]⁺. ¹H NMR (400 MHz, DMSO-d₆) δ 10.82 (s, 1H), 8.78 - 8.67 (m, 2H), 7.62 (s, 1H), 7.46 (d, J = 9.0 Hz, 1H), 6.98 - 6.84 (m, 2H), 6.81 (s, 1H), 6.69 (dd, J = 8.6, 3.8 Hz, 1H), 4.96 - 4.85 (m, 1H), 4.74 (d, J = 4.8 Hz, 2H), 4.53 (t, J = 8.7 Hz, 2H), 4.23 (dd, J = 9.1, 5.1 Hz, 1H), 3.87 (s, 3H), 3.77 (d, J = 12.1 Hz, 2H), 3.27 (d, J = 8.8 Hz, 2H), 2.92 (d, J = 10.2 Hz, 2H), 2.72 (t, J = 11.6 Hz, 2H), 2.67 - 2.53 (m, 2H), 2.38 - 1.96 (m, 10H), 1.81 (d, J = 12.2 Hz, 2H), 1.71 (s, 1H), 1.25 (dd, J = 22.0, 11.6 Hz, 2H).

### Example 138 to Example 139 Preparation of Compounds H138 and H139

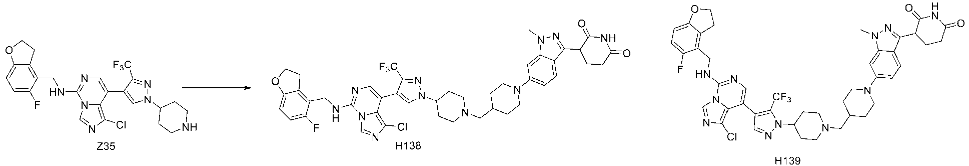

Z35 (80 mg, 0.15 mmol) was added to 2 ml of dichloromethane. Two drops of DMSO were added, followed by 20 mg of sodium triacetoxyborohydride. The resulting mixture was heated under microwave at 35°C for 45 minutes. The resulting mixture solution was then sent for purification, and the filtrate was purified by high pressure liquid chromatography (waters-sunfire-10 µm-19×150 mm column (eluent: 28% to 38% (v/v) acetonitrile and aqueous formic acid)) to give H138 (29 mg, 33.2 µmol). MS m/z (ESI): 874 [M+H]⁺. ¹H NMR (400 MHz, DMSO-d₆) δ 10.83 (s, 1H), 8.62 (s, 1H), 8.44 (t, J = 5.0 Hz, 1H), 8.13 (d, J = 1.3 Hz, 1H), 7.46 (d, J = 8.9 Hz, 1H), 7.11 (s, 1H), 6.99 - 6.85 (m, 2H), 6.81 (d, J = 1.9 Hz, 1H), 6.69 (dd, J = 8.7, 3.8 Hz, 1H), 4.68 (d, J = 4.9 Hz, 2H), 4.53 (t, J = 8.7 Hz, 2H), 4.36 - 4.15 (m, 2H), 3.87 (s, 3H), 3.77 (d, J = 12.1 Hz, 2H), 2.94 (d, J = 10.4 Hz, 2H), 2.72 (t, J = 11.9 Hz, 2H), 2.66 - 2.56 (m, 2H), 2.35 - 1.89 (m, 11H), 1.81 (d, J = 12.7 Hz, 2H), 1.70 (s, 1H), 1.24 (d, J = 15.5 Hz, 2H).
and H139 (2.2 mg, 2.5 µmol). Yield: 22.1%. MS m/z (ESI): 874 [M+H]⁺. ¹H NMR (400 MHz, DMSO-d₆) δ 10.84 (s, 1H), 8.62 (s, 1H), 8.47 (t, J = 5.1 Hz, 1H), 7.70 (s, 1H), 7.46 (d, J = 8.9 Hz, 1H), 7.14 (s, 1H), 7.04 - 6.85 (m, 2H), 6.82 (s, 1H), 6.69 (dd, J = 8.7, 3.8 Hz, 1H), 4.68 (s, 2H), 4.53 (t, J = 8.7 Hz, 2H), 4.23 (dd, J = 9.2, 5.0 Hz, 2H), 3.87 (s, 3H), 3.78 (d, J = 12.1 Hz, 2H), 3.00 (d, J = 10.6 Hz, 2H), 2.74 (d, J = 12.1 Hz, 2H), 2.67 - 2.56 (m, 2H), 2.36 - 2.00 (m, 11H), 1.90 (d, J = 11.3 Hz, 2H), 1.82 (d, J = 12.3 Hz, 2H), 1.24 (d, J = 15.5 Hz, 2H).

### Example 140 Preparation of Compound H140

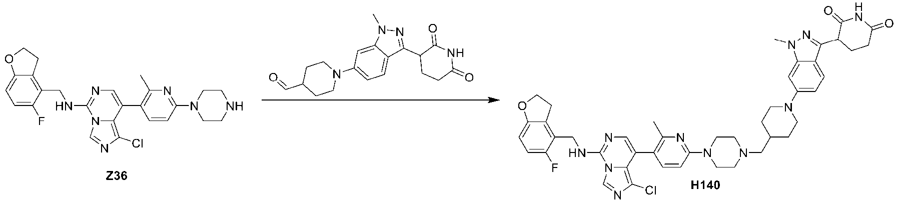

Compound H140 was prepared referring to the method of Example 71. MS m/z (ESI): 832.4 [M+H]⁺. ¹H NMR (400 MHz, DMSO-d₆) δ 10.83 (s, 1H), 8.61 (s, 1H), 8.32 (d, J = 5.2 Hz, 1H), 7.47 (d, J = 9.2 Hz, 1H), 7.30 (d, J = 8.4 Hz, 1H), 7.01 (s, 1H), 6.97 - 6.87 (m, 2H), 6.82 (d, J = 2.0 Hz, 1H), 6.73 - 6.63 (m, 2H), 4.68 (qd, J = 14.4, 4.8 Hz, 2H), 4.53 (t, J = 8.8 Hz, 2H), 4.23 (dd, J = 9.2, 5.2 Hz, 1H), 3.87 (s, 3H), 3.82-3.73 (m, 2H), 3.58 - 3.44 (m, 4H), 3.31 (d, J = 3.6 Hz, 1H), 3.28 (s, 1H), 2.74 (t, J = 12.0 Hz, 2H), 2.65 - 2.57 (m, 2H), 2.47-2.41 (m, 4H), 2.32-2.20 (m, 3H), 2.118-2.10 (m, 4H), 1.88-1.70 (m, 3H), 1.27 (d, J = 11.6 Hz, 2H).

### Example 141 Preparation of Compound H141

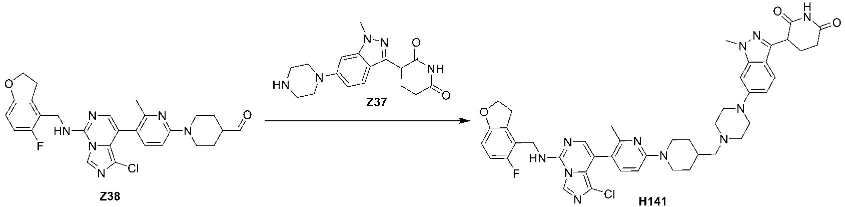

Z38 (0.1 g, 191.94 µmol) and Z37 (69.12 mg, 211.14 µmol) were dissolved in DCM (8 mL) and stirred at room temperature for 10 min. NaBH(OAc)₃ (203.40 mg, 959.72 µmol) was added, and the mixture was stirred at room temperature for 1 hour. Saturated sodium bicarbonate was added, extraction was performed three times with dichloromethane, the combined organic phases were dried over anhydrous sodium sulfate, and concentrated under reduced pressure to give a crude product. The residue was separated by CombiFlash column chromatography (4 g, 0-11% MeOH/DCM) to give a crude product. The crude product was purified by preparative liquid chromatography (preparative column: 21.2×250 mm C18 column; system: 10 mM NH₄HCO₃/H₂O/acetonitrile; wavelength: 254/214 nm; gradient: 5%-95% acetonitrile change) to give H141 (84.96 mg, 101.84 µmol, 53.05% yield, 99.77% purity) as a white solid. MS m/z (ESI): 832.3 [M+H]⁺. ¹H NMR (400 MHz, DMSO-d₆) δ 10.83 (s, 1H), 8.61 (s, 1H), 8.30 (t, J = 5.2 Hz, 1H), 7.49 (d, J = 9.2 Hz, 1H), 7.27 (d, J = 8.4 Hz, 1H), 7.00 (s, 1H), 6.97 - 6.90 (m, 2H), 6.83 (d, J = 2.0 Hz, 1H), 6.74 - 6.62 (m, 2H), 4.68 (qd, J = 14.4, 5.2 Hz, 2H), 4.53 (t, J = 8.8 Hz, 2H), 4.32 (t, J = 11.6 Hz, 2H), 4.24 (dd, J = 9.2, 5.2 Hz, 1H), 3.88 (s, 3H), 3.27 (s, 2H), 3.21 (d, J = 6.0 Hz, 4H), 2.79 (q, J = 11.2 Hz, 2H), 2.67 - 2.57 (m, 2H), 2.54 (d, J = 5.2 Hz, 4H), 2.35 - 2.13 (m, 4H), 2.12 (s, 3H), 1.81 (d, J = 11.6 Hz, 3H), 1.12 (d, J = 12.4 Hz, 2H).

### Example 142 Preparation of Compound H142

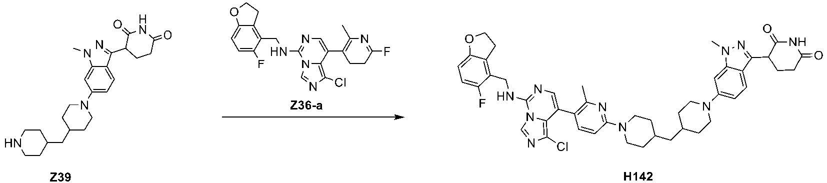

Z36-a (55 mg, 128.55 µmol) and Z39 (108.90 mg, 257.11 µmol) were dissolved in NMP (2 mL). DIPEA (222.60 mg, 1.72 mmol, 0.3 mL) was then added. The reaction was stirred under microwave at 200°C for 1 hour. After the reaction was complete, the reaction solution was cooled to room temperature and directly subjected to basic preparation (preparative column: 21.2×250 mm C18 column; system: 10 mM NH₄HCO₃/H₂O/acetonitrile; wavelength: 254/214 nm; gradient: 0%-60% acetonitrile change) to successfully give white solid product H142 (33.29 mg, 30.83% yield). MS m/z (ESI): 831.3 [M+H]⁺. ¹H NMR (400 MHz, DMSO-d₆) δ 10.82 (s, 1H), 8.61 (s, 1H), 8.29 (t, J = 5.0 Hz, 1H), 7.46 (d, J = 9.0 Hz, 1H), 7.27 (d, J = 8.5 Hz, 1H), 7.00 (s, 1H), 6.92 (dd, J = 19.7, 9.8 Hz, 2H), 6.81 (s, 1H), 6.73 - 6.60 (m, 2H), 4.76 - 4.60 (m, 2H), 4.53 (t, J = 8.8 Hz, 2H), 4.33 (t, J = 9.9 Hz, 2H), 4.23 (dd, J = 9.1, 5.2 Hz, 1H), 3.87 (s, 3H), 3.77 (d, J = 11.9 Hz, 2H), 3.32 (s, 1H), 3.27 (s, 1H), 2.85 - 2.64 (m, 4H), 2.60 (dd, J = 12.0, 6.0 Hz, 2H), 2.28 (dt, J = 23.0, 7.1 Hz, 1H), 2.16 (dd, J = 12.2, 6.7 Hz, 1H), 2.12 (s, 3H), 1.75 (t, J = 11.8 Hz, 4H), 1.60 (dd, J = 39.0, 15.8 Hz, 2H), 1.18 (tdd, J = 38.3, 24.5, 13.4 Hz, 6H).

### Example 143 Preparation of Compound H143

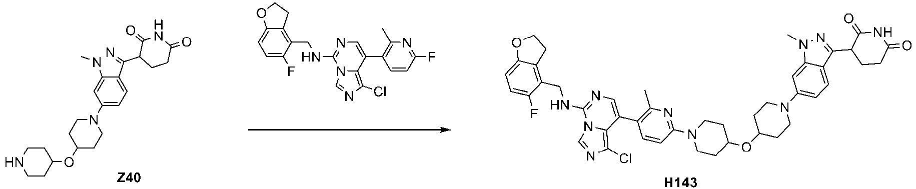

Compound H143 was prepared referring to the method of Example 142. MS m/z (ESI): 833.3 [M+H]⁺. ¹H NMR (400 MHz, DMSO-d₆) δ 10.83 (s, 1H), 8.61 (s, 1H), 8.30 (t, J = 5.1 Hz, 1H), 7.47 (d, J = 9.0 Hz, 1H), 7.28 (d, J = 8.5 Hz, 1H), 7.00 (s, 1H), 6.92 (dd, J = 16.6, 9.1 Hz, 2H), 6.84 (s, 1H), 6.73 - 6.62 (m, 2H), 4.68 (ddd, J = 27.7, 14.5, 4.7 Hz, 2H), 4.53 (t, J = 8.7 Hz, 2H), 4.24 (dd, J = 9.0, 5.1 Hz, 1H), 4.02 (t, J = 12.8 Hz, 2H), 3.87 (s, 3H), 3.71 (ddd, J = 13.1, 8.9, 5.0 Hz, 2H), 3.59 (dd, J = 7.1, 5.2 Hz, 2H), 3.27 (d, J = 6.1 Hz, 2H), 3.14 (dd, J = 22.5, 10.4 Hz, 2H), 2.98 (t, J = 9.9 Hz, 2H), 2.69 - 2.52 (m, 2H), 2.28 (dt, J = 16.2, 7.1 Hz, 1H), 2.21 - 2.14 (m, 1H), 2.12 (s, 3H), 1.90 (d, J = 15.2 Hz, 4H), 1.58 (dd, J = 16.7, 7.4 Hz, 2H), 1.44 (dd, J = 21.1, 12.0 Hz, 2H).

### Example 144 Preparation of Compound H144

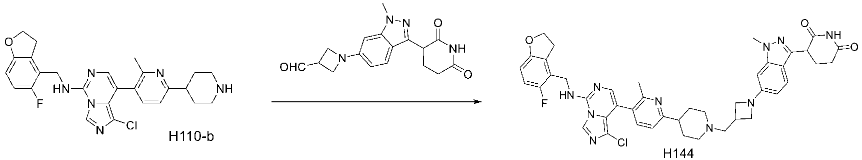

Referring to the preparation method of Example 49, following the above synthetic route, H144 was prepared. MS m/z (ESI): 803.3 [M+H]⁺. ¹H NMR (400 MHz, DMSO-d₆) δ 10.82 (s, 1H), 8.64 (s, 1H), 8.39 (t, J = 5.0 Hz, 1H), 7.47 (dd, J = 12.8, 8.2 Hz, 2H), 7.14 (d, J = 7.9 Hz, 1H), 7.09 (s, 1H), 6.99 - 6.88 (m, 1H), 6.69 (dd, J = 8.6, 3.8 Hz, 1H), 6.35 (dd, J = 13.0, 4.3 Hz, 2H), 4.69 (qd, J = 14.3, 4.8 Hz, 2H), 4.54 (t, J = 8.7 Hz, 2H), 4.22 (dd, J = 9.0, 5.1 Hz, 1H), 4.01 (t, J = 7.4 Hz, 2H), 3.83 (s, 3H), 3.53 (t, J = 6.5 Hz, 2H), 3.28 (s, 2H), 2.96 (d, J = 10.1 Hz, 3H), 2.73-2.56 (m, 5H), 2.27 (d, J = 11.6 Hz, 4H), 2.17 - 2.00 (m, 3H), 1.90 - 1.63 (m, 4H).

### Example 145 Preparation of Compound H145

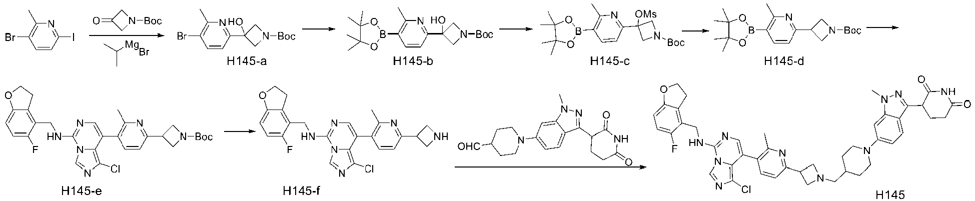

Step 1: 3-Bromo-6-iodo-2-methylpyridine (0.5 g, 1.68 mmol) was dissolved in THF (5 mL). Under argon protection, the temperature was lowered to -20°C, isopropylmagnesium chloride (2.0 mol/L, 923.07 µL) was added dropwise, and the mixture was stirred at -20°C for 10 minutes. A solution of tert-butyl 3-oxoazetidine-1-carboxylate (344.78 mg, 2.01 mmol) in tetrahydrofuran (1.0 mL) was added, and the mixture was stirred at -20°C, allowing it to naturally warm to room temperature, and stirred for 16 hours. After the reaction was complete, the reaction solution was poured into saturated aqueous ammonium chloride solution, extracted with ethyl acetate (30 mL×2). The combined organic phases were dried over anhydrous sodium sulfate, concentrated, and purified by silica gel column chromatography (EA:PE = 0-50%) to give H145-a (300 mg, yellow solid), yield: 52.08%. MS m/z (ESI): 343 [M+H]⁺.

Step 2: H145-a (1.0 g, 2.91 mmol) and 4,4,5,5-tetramethyl-2-(4,4,5,5-tetramethyl-1,3,2-dioxaborolan- 2-yl)-1,3,2-dioxaborolane (1.48 g, 5.83 mmol) were dissolved in 1,4-dioxane (20 mL). At room temperature under argon protection, potassium acetate (571.90 mg, 5.83 mmol) and Pd(dppf)Cl₂ (213.19 mg, 291.36 µmol) were added sequentially, and the temperature was raised to 95°C and stirred for 5 hours. After the reaction was complete, the reaction solution was concentrated and purified by silica gel column chromatography (EA:PE = 0-100%) to give H145-b (0.7 g, yellow solid), yield: 61.56%. MS m/z (ESI): 391 [M+H]⁺.

Step 3: H145-b (170 mg, 435.58 µmol) and triethylamine (66.12 mg, 653.38 µmol, 91.13 µL) were dissolved in dichloromethane (5 mL). Under argon protection under ice bath conditions, methanesulfonyl chloride (59.88 mg, 522.70 µmol) was added, and the mixture was stirred under ice bath for 0.5 hour. After the reaction was complete, the reaction solution was poured into saturated aqueous ammonium chloride solution, extracted with ethyl acetate, the organic phase was dried over anhydrous sodium sulfate, and concentrated to give H145-c (200 mg, yellow oil), yield: 98.03%.

Step 4: H145-c (200 mg, 427.01 µmol) was dissolved in methanol (10 mL). Palladium on carbon (45.44 mg, 42.70 µmol, 10% purity) was added, and the mixture was stirred at room temperature overnight under hydrogen protection. After the reaction was complete, the reaction solution was filtered, and the filtrate was concentrated and purified by silica gel column chromatography (EA:PE = 0-50%) to give H145-d (130 mg, yellow solid), yield: 81.34%. MS m/z (ESI): 375 [M+H]⁺.

Step 5: 8-Bromo-1-chloro-N-[(5-fluoro-2,3-dihydrobenzofuran-4-yl)methyl]imidazo[1,5-c] pyrimidin-5-amine (150 mg, 377.24 µmol) and H145-d (123.44 mg, 377.24 µmol) were dissolved in 1,4-dioxane (5 mL) and water (0.5 mL). Under argon protection, Pd(dppf)Cl₂ (55.21 mg, 75.45 µmol) and K₂CO₃ (156.41 mg, 1.13 mmol) were added sequentially, and the temperature was raised to 95°C and stirred overnight. After the reaction was complete, the reaction solution was concentrated and purified by silica gel column chromatography (MeOH:DCM = 0-10%) to give H145-e (60 mg, yellow solid), yield: 28.15%. MS m/z (ESI): 566 [M+H]⁺.

Step 6: H145-e (60 mg, 106.19 µmol) was dissolved in dichloromethane (5 mL). TFA (1 g, 8.77 mmol, 1 mL) was added with stirring at room temperature, and the mixture was stirred at room temperature for 2 hours. After the reaction was complete, the reaction solution was concentrated, saturated aqueous sodium carbonate solution was added, extraction was performed with ethyl acetate (30 mL×2), the combined organic phases were dried over anhydrous sodium sulfate, and concentrated to give H145-f (49.37 mg, yellow solid), yield: 100.00%. MS m/z (ESI): 465 [M+H]⁺.

Step 7: H145-f (50 mg, 107.55 µmol) and 1-[3-(2,6-dioxopiperidin-3-yl)-1-methylindazol-6-yl]piperidine-4-carbaldehyde (76.23 mg, 215.09 µmol) were dissolved in dichloromethane (5 mL). NaBH(OAc)₃ (68.40 mg, 322.64 µmol) was added with stirring at room temperature, and the mixture was stirred at room temperature overnight. After the reaction was complete, the reaction solution was concentrated and purified by preparative HPLC to give H145 (1.59 mg, purity 99.65%), yield: 1.83%. MS m/z (ESI): 804 [M+H]⁺. ¹H NMR (400 MHz, DMSO-d₆) δ 10.83 (s, 1H), 8.64 (s, 1H), 8.41 (t, J = 4.9 Hz, 1H), 7.53 (d, J = 7.8 Hz, 1H), 7.46 (d, J = 9.0 Hz, 1H), 7.26 (d, J = 7.9 Hz, 1H), 7.09 (s, 1H), 6.98 - 6.85 (m, 2H), 6.81 (s, 1H), 6.69 (dd, J = 8.6, 3.8 Hz, 1H), 4.69 (dt, J = 14.7, 9.8 Hz, 2H), 4.54 (t, J = 8.7 Hz, 2H), 4.23 (dd, J = 9.2, 5.1 Hz, 1H), 3.87 (s, 3H), 3.82 - 3.62 (m, 4H), 3.28 (s, 2H), 2.63 (ddd, J = 22.3, 20.1, 11.8 Hz, 4H), 2.43 - 2.02 (m, 7H), 1.80 (d, J = 11.8 Hz, 2H), 1.50 (s, 1H), 1.27 (dd, J = 23.7, 14.5 Hz, 3H).

### Example 146 Preparation of Compound H146

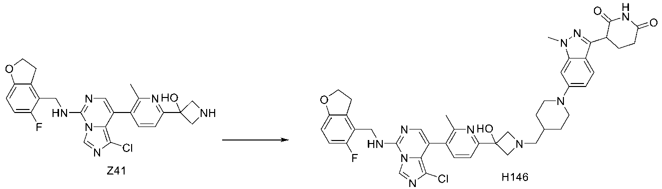

Compound H146 was prepared referring to the method of Example 71. MS m/z (ESI): 832 [M+H]⁺. ¹H NMR (400 MHz, DMSO-d₆) δ 10.83 (s, 1H), 8.66 (s, 1H), 8.43 (t, J = 4.9 Hz, 1H), 7.61 (dd, J = 16.0, 8.0 Hz, 2H), 7.46 (d, J = 9.0 Hz, 1H), 7.11 (s, 1H), 6.98 - 6.86 (m, 2H), 6.81 (s, 1H), 6.70 (dd, J = 8.6, 3.8 Hz, 1H), 6.00 (s, 1H), 4.70 (ddd, J = 28.6, 14.4, 4.8 Hz, 2H), 4.54 (t, J = 8.7 Hz, 2H), 4.23 (dd, J = 9.1, 5.1 Hz, 1H), 3.86 (s, 3H), 3.75 (t, J = 10.5 Hz, 3H), 3.66 (d, J = 6.9 Hz, 1H), 3.30 - 3.23 (m, 2H), 2.69 (t, J = 11.7 Hz, 2H), 2.60 (dd, J = 11.7, 6.9 Hz, 2H), 2.45 (s, 3H), 2.30 (d, J = 13.0 Hz, 3H), 2.27 (dd, J = 8.8, 4.8 Hz, 1H), 2.13 (dd, J = 13.3, 5.8 Hz, 1H), 1.81 (d, J = 11.4 Hz, 2H), 1.50 (s, 1H), 1.28 (dd, J = 26.4, 16.7 Hz, 3H).

### Example 147 Preparation of Compound H147

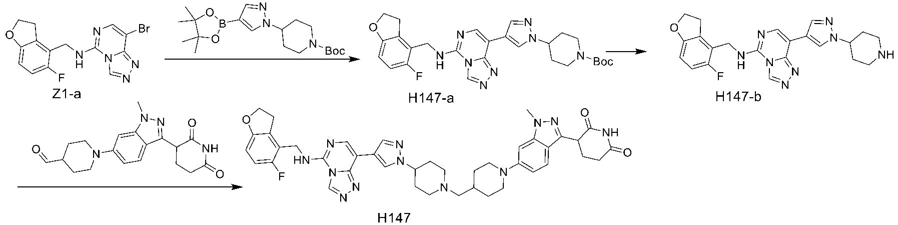

Referring to the preparation method of Example 49, following the above synthetic route, H147 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 773.4 [M+H]⁺. ¹H NMR (400 MHz, DMSO-d₆) δ 10.82 (s, 1H), 9.44 (s, 1H), 8.51 (t, J = 5.2 Hz, 1H), 8.48 (s, 1H), 8.15 (s, 1H), 8.02 (s, 1H), 7.46 (d, J = 8.8 Hz, 1H), 6.97 - 6.87 (m, 2H), 6.82 (d, J = 2.0 Hz, 1H), 6.69 (dd, J = 8.4, 4.0 Hz, 1H), 4.68 (d, J = 4.8 Hz, 2H), 4.53 (t, J = 8.8 Hz, 2H), 4.23 (dd, J = 9.2, 5.2 Hz, 2H), 3.87 (s, 3H), 3.78 (d, J = 12.0 Hz, 2H), 3.27 (d, J = 9.6 Hz, 2H), 2.95 (d, J = 10.4 Hz, 2H), 2.73 (t, J = 12.0 Hz, 2H), 2.65 - 2.57 (m, 2H), 2.35 - 2.19 (m, 3H), 2.16 - 1.93 (m, 7H), 1.82 (d, J = 12.8 Hz, 2H), 1.71 (s, 1H), 1.26 (q, J = 11.2 Hz, 2H).

### Example 148 Preparation of Compound H148

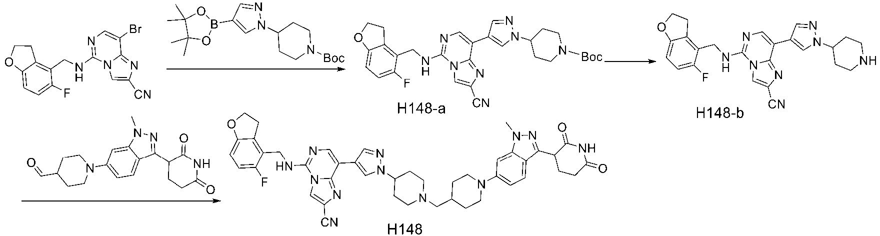

Referring to the preparation method of Example 49, following the above synthetic route, H148 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 797.4 [M+H]⁺. ¹H NMR (400 MHz, DMSO-d₆) δ 10.83 (s, 1H), 8.89 (s, 1H), 8.43 (s, 1H), 8.34 (t, J = 5.0 Hz, 1H), 8.15 (s, 1H), 8.11 (s, 1H), 7.46 (d, J = 9.0 Hz, 1H), 6.91 (dd, J = 17.9, 8.7 Hz, 2H), 6.81 (s, 1H), 6.68 (dd, J = 8.6, 3.9 Hz, 1H), 4.68 (d, J = 4.9 Hz, 2H), 4.52 (t, J = 8.7 Hz, 2H), 4.23 (dd, J = 9.2, 5.1 Hz, 2H), 3.87 (s, 3H), 3.78 (d, J = 12.4 Hz, 2H), 3.28 (t, J = 9.1 Hz, 2H), 2.95 (d, J = 10.5 Hz, 2H), 2.72 (t, J = 11.3 Hz, 2H), 2.60 (dd, J = 11.7, 6.8 Hz, 2H), 2.37 - 2.25 (m, 1H), 2.25 - 2.17 (m, 2H), 2.14 (dd, J = 13.3, 5.7 Hz, 1H), 2.11 - 1.88 (m, 6H), 1.82 (d, J = 12.1 Hz, 2H), 1.71 (s, 1H), 1.25 (dd, J = 21.3, 11.4 Hz, 2H).

### Example 149 Preparation of Compound H149

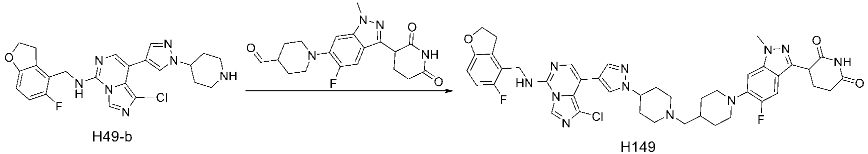

Referring to the preparation method of Example 109, following the above synthetic route, H149 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 824.3 [M+H]⁺. ¹H NMR (400 MHz, DMSO-d₆) δ 10.85 (s, 1H), 8.62 (s, 1H), 8.29 (t, J = 5.0 Hz, 1H), 7.94 (s, 1H), 7.57 (s, 1H), 7.42 (d, J = 12.5 Hz, 1H), 7.16 (s, 1H), 7.07 (d, J = 7.1 Hz, 1H), 6.98 - 6.85 (m, 1H), 6.67 (dd, J = 8.6, 3.9 Hz, 1H), 4.67 (d, J = 4.9 Hz, 2H), 4.52 (t, J = 8.7 Hz, 2H), 4.25 (dd, J = 9.6, 5.0 Hz, 1H), 4.21 - 4.10 (m, 1H), 3.93 (s, 3H), 3.41 (d, J = 10.7 Hz, 2H), 3.27 (t, J = 8.7 Hz, 2H), 2.95 (d, J = 10.0 Hz, 2H), 2.67 (t, J = 11.0 Hz, 2H), 2.58 (ddd, J = 17.1, 10.2, 5.3 Hz, 2H), 2.39 - 2.28 (m, 1H), 2.25 (t, J = 10.6 Hz, 2H), 2.18 - 1.90 (m, 7H), 1.83 (d, J = 11.7 Hz, 2H), 1.68 (s, 1H), 1.32 (dd, J = 21.3, 11.0 Hz, 2H).

### Example 150 Preparation of Compound H150

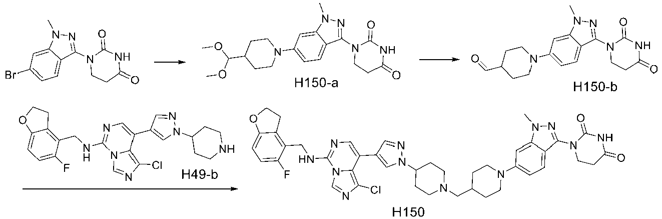

Step 1: 1-(6-Bromo-1-methyl-1H-indazol-3-yl)dihydropyrimidine-2,4(1H,3H)-dione (0.5 g, 1.55 mmol) and 4-(dimethoxymethyl)piperidine (739.11 mg, 4.64 mmol) were dissolved in 1,4-dioxane (15 mL). Under argon protection, Ruphos-Pd-G₃ (388.70 mg, 464.19 µmol) and Cs₂CO₃ (1.51 g, 4.64 mmol) were added, and the temperature was raised to 120°C and stirred for 16 h. After the reaction was complete, the reaction solution was concentrated and purified by CombiFlash (PE:EA = 30%:70%) to give H150-a (300 mg, yellow oil), yield: 48.30%. MS m/z (ESI): 402.2 [M+H]⁺.

Step 2: At room temperature, H150-a (270 mg, 672.55 µmol), DCM (2 mL), and TFA (1 mL) were mixed and stirred for 1 hour. After the reaction was complete, the reaction solution was concentrated, saturated aqueous sodium bicarbonate solution was added to adjust the pH to 8-9, then extraction was performed with organic solvent (DCM:MeOH = 10:1) (10 mL×3), the combined organic phases were dried over anhydrous sodium sulfate, and concentrated to give H150-b (180 mg, yellow oil, crude), yield: 75.31%. MS m/z (ESI): 356.1 [M+H]⁺.

Step 3: At room temperature, H49-b (40 mg, 85.48 µmol), H150-b (45.57 mg, 128.23 µmol), NaBH(OAc)₃ (54.35 mg, 256.45 µmol),DCM (3 mL), DMSO (0.5 mL), and AcOH (0.01 mL) were mixed and stirred for 1 hour. After the reaction was complete, water was added dropwise to quench the reaction, the mixture was concentrated, and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H150 (7.92 mg), yield: 11.48%. MS m/z (ESI): 807.3 [M+H]⁺. ¹H NMR (400 MHz, DMSO-d₆) δ 10.47 (s, 1H), 8.61 (s, 1H), 8.28 (t, J = 5.2 Hz, 1H), 7.94 (s, 1H), 7.57 (s, 1H), 7.41 (d, J = 9.0 Hz, 1H), 7.16 (s, 1H), 6.94 - 6.88 (m, 2H), 6.79 (d, J = 1.4 Hz, 1H), 6.67 (dd, J = 8.4, 4.0 Hz, 1H), 4.67 (d, J = 5.2 Hz, 2H), 4.52 (t, J = 8.8 Hz, 2H), 4.19 - 4.13 (m, 1H), 3.90 - 3.84 (m, 4H), 3.79 (d, J = 12.8 Hz, 2H), 3.26 (d, J = 8.8 Hz, 2H), 2.94 (d, J = 10.4 Hz, 2H), 2.76-2.70 (m, 3H), 2.21 (d, J = 6.4 Hz, 2H), 2.09 - 1.94 (m, 6H), 1.81 (d, J = 12.4 Hz, 2H), 1.76 - 1.66 (m, 2H), 1.30 - 1.19 (m, 3H).

### Example 151 Preparation of Compound H151

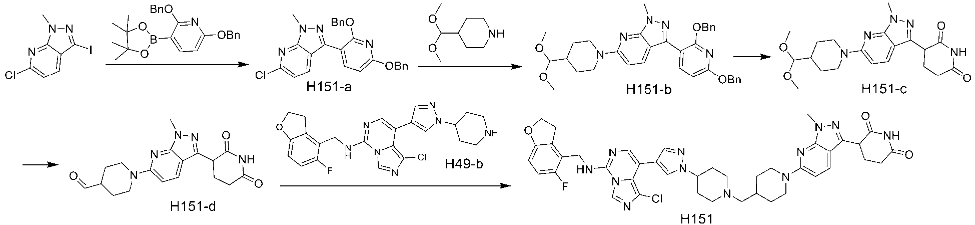

Step 1: 6-Chloro-3-iodo-1-methyl-1H-pyrazolo[3,4-b]pyridine (2 g, 6.81 mmol) and 2,6-bis(benzyloxy)-3-(4,4,5,5-tetramethyl-1,3,2-dioxaborolan-2-yl)pyridine (2.84 g, 6.81 mmol) were dissolved in 1,4-dioxane (30 mL) and water (6 mL). Under argon protection, Pd(dppf)Cl₂ (498.62 mg, 681.45 µmol) and Na₂CO₃ (2.17 g, 20.44 mmol) were added, and the temperature was raised to 100°C and stirred overnight. After the reaction was complete, the reaction solution was concentrated, water and ethyl acetate were added, extraction was performed three times with ethyl acetate, the combined organic phases were dried over anhydrous sodium sulfate, concentrated, and purified by CombiFlash (20 g, 0-30% DCM/PE) to give H151-a (0.8 g, white solid), yield: 25.69%. MS m/z (ESI): 457.1 [M+H]⁺.

Step 2: H151-a (0.8 g, 1.75 mmol) and 4-(dimethoxymethyl)piperidine (557.56 mg, 3.50 mmol) were dissolved in 1,4-dioxane (8 mL). Under argon protection, Ruphos-Pd-G₃ (146.61 mg, 175.08 µmol) and Cs₂CO₃ (1.71 g, 5.25 mmol) were added, and the temperature was raised to 100°C and stirred overnight. After the reaction was complete, the reaction solution was filtered, and the filtrate was concentrated and purified by CombiFlash (12 g, 0-20% EA/PE) to give H151-b (0.6 g, light yellow solid), yield: 59.12%. MS m/z (ESI): 580.3 [M+H]⁺.

Step 3: H151-b (0.7 g, 1.21 mmol) was dissolved in THF (10 mL) and EtOH (10 mL). Pd/C (128.00 mg, 120.75 µmol, 10% purity) was added, the mixture was purged with a hydrogen balloon three times, and the temperature was raised to 50°C and stirred for 6 hours. After the reaction was complete, the reaction solution was filtered, and the filtrate was concentrated to give H151-c (0.4 g, light yellow solid, crude), yield: 82.51%. MS m/z (ESI): 402.2 [M+H]⁺.

Step 4: H151-c (0.4 g, 996.37 µmol) was dissolved in DCM (6 mL). TFA (2 mL) was added, and the mixture was stirred at room temperature for 1 hour. After the reaction was complete, the reaction solution was concentrated, dichloromethane and saturated aqueous sodium bicarbonate solution were added, extraction was performed three times with dichloromethane, the combined organic phases were dried over anhydrous sodium sulfate, and concentrated to give H151-d (0.26 g, light yellow solid, crude), yield: 73.43%. MS m/z (ESI): 356.2 [M+H]⁺.

Step 5: H49-b (60 mg, 128.23 µmol) and H151-d (59.24 mg, 166.69 µmol) were dissolved in DCM (6 mL) and stirred at room temperature for 10 min. NaBH(OAc)₃ (135.88 mg, 641.13 µmol) was added, and stirring was continued at room temperature for 1 hour. After the reaction was complete, the reaction solution was concentrated, ethyl acetate and saturated aqueous sodium bicarbonate solution were added, extraction was performed three times with ethyl acetate, the combined organic phases were dried over anhydrous sodium sulfate, concentrated, and purified by CombiFlash (4 g, 0-11% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H151 (46.01 mg, purity 100%), yield: 44.45%. MS m/z (ESI): 807.3 [M+H]⁺. ¹H NMR (400 MHz, DMSO-d₆) δ 10.83 (s, 1H), 8.61 (s, 1H), 8.28 (t, J = 5.2 Hz, 1H), 7.94 (s, 1H), 7.79 (d, J = 9.2 Hz, 1H), 7.57 (s, 1H), 7.16 (s, 1H), 6.97 - 6.88 (m, 1H), 6.75 (d, J = 9.2 Hz, 1H), 6.68 (dd, J = 8.4, 4.0 Hz, 1H), 4.68 (d, J = 5.2 Hz, 2H), 4.52 (t, J = 8.8 Hz, 2H), 4.44 (d, J = 13.2 Hz, 2H), 4.17 (dd, J = 9.6, 5.2 Hz, 2H), 3.79 (s, 3H), 3.26 (d, J = 8.8 Hz, 2H), 2.91 (t, J = 12.8 Hz, 4H), 2.68 - 2.56 (m, 2H), 2.37 - 2.24 (m, 1H), 2.20-2.11 (m, 3H), 2.10 - 1.92 (m, 6H), 1.79 (d, J = 12.8 Hz, 3H), 1.10 (d, J = 12.0 Hz, 2H).

### Example 152 Preparation of Compound H152

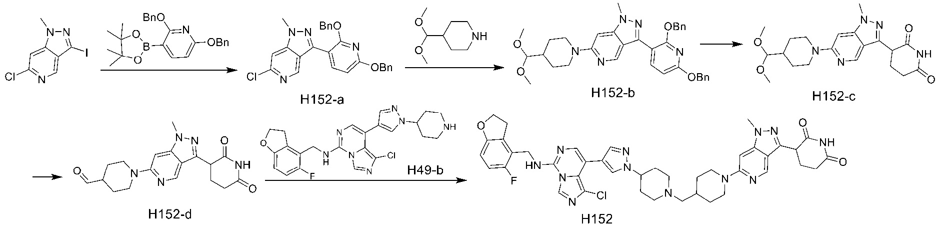

Referring to the preparation method of Example 151, following the above synthetic route, H152 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 807.3 [M+H]⁺. ¹H NMR (400 MHz, DMSO-d₆) δ 10.89 (s, 1H), 8.62 (s, 1H), 8.60 (d, J = 1.2 Hz, 1H), 8.29 (t, J = 5.2 Hz, 1H), 7.94 (s, 1H), 7.57 (s, 1H), 7.16 (s, 1H), 6.99 - 6.87 (m, 1H), 6.68 (dd, J = 8.4, 4.0 Hz, 1H), 6.64 (s, 1H), 4.68 (d, J = 5.2 Hz, 2H), 4.52 (t, J = 8.8 Hz, 2H), 4.39 - 4.27 (m, 3H), 4.16 (s, 1H), 3.84 (s, 3H), 3.26 (d, J = 8.8 Hz, 2H), 2.94 (d, J = 10.4 Hz, 2H), 2.80 (t, J = 12.4 Hz, 2H), 2.71 - 2.56 (m, 2H), 2.34 (dd, J = 14.0, 9.2 Hz, 1H), 2.18 (d, J = 6.0 Hz, 3H), 2.10-1.93 (m, 6H), 1.78 (d, J = 12.0 Hz, 3H), 1.13 (d, J = 12.4 Hz, 2H).

### Example 153 Preparation of Compound H153

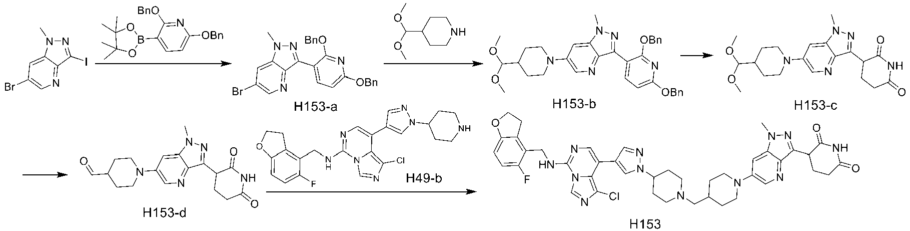

Referring to the preparation method of Example 151, following the above synthetic route, H153 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 807.3 [M+H]⁺. ¹H NMR (400 MHz, DMSO-d₆) δ 10.84 (s, 1H), 8.62 (s, 1H), 8.36 (d, J = 2.4 Hz, 1H), 8.28 (t, J = 5.2 Hz, 1H), 7.94 (s, 1H), 7.57 (s, 1H), 7.29 (d, J = 2.4 Hz, 1H), 7.16 (s, 1H), 6.92 (dd, J = 10.4, 8.8 Hz, 1H), 6.67 (dd, J = 8.8, 4.0 Hz, 1H), 4.68 (d, J = 5.2 Hz, 2H), 4.52 (t, J = 8.8 Hz, 2H), 4.25 (dd, J = 9.6, 5.2 Hz, 1H), 4.17 (s, 1H), 3.91 (s, 3H), 3.81 (d, J = 12.0 Hz, 2H), 3.26 (d, J = 8.8 Hz, 2H), 2.94 (d, J = 10.4 Hz, 2H), 2.78 (t, J = 12.0 Hz, 2H), 2.71-2.61 (m, 1H), 2.59-2.51 (m, 1H), 2.45-2.35 (m, 1H), 2.21 (d, J = 6.8 Hz, 2H), 2.15 - 1.91 (m, 7H), 1.87 - 1.78 (m, 2H), 1.78-1.66 (m, 1H), 1.34-1.21 (m, 2H).

### Examples 154 to 156

Compounds H154 to H156 were prepared using Z42, Z43, and Z44 as starting materials, respectively, referring to the method of Example 71.

| | |
|---|---|
| 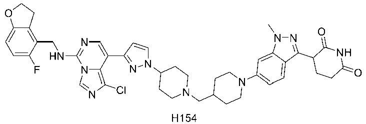 | MS m/z (ESI): 806.3 [M+H]⁺ |
| | ¹H NMR (400 MHz, DMSO-d₆) δ 10.82 (s, 1H), 8.65 (s, 1H), 8.55 (s, 1H), 7.53 (d, *J =* 1.8 Hz, 1H), 7.45 (d, *J =* 9.0 Hz, 1H), 7.19 (s, 1H), 6.98 - 6.85 (m, 2H), 6.80 (d, *J =* 1.9 Hz, 1H), 6.70 (dd, *J =* 8.6, 3.9 Hz, 1H), 6.27 (d, *J =* 1.8 Hz, 1H), 4.72 (s, 2H), 4.54 (t, *J =* 8.7 Hz, 2H), 4.23 (dd, *J* = 9.1, 5.1 Hz, 1H), 3.86 (s, 3H), 3.83 (s, 1H), 3.75 (d, *J =* 11.9 Hz, 2H), 2.87 (s, 2H), 2.76-2.54 (m, 5H), 2.33-2.07 (m, 5H), 2.04-1.50 (m, 9H), 1.21 (m, 2H). |
| 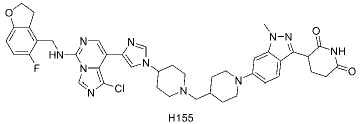 | MS m/z (ESI): 806.3 [M+H]⁺ |
| | ¹H NMR (400 MHz, DMSO-d₆) δ 10.82 (s, 1H), 8.63 (s, 1H), 8.52 (t, *J =* 5.0 Hz, 1H), 7.94 (s, 1H), 7.45 (d, *J =* 8.9 Hz, 1H), 7.18 (s, 1H), 6.98 - 6.84 (m, 3H), 6.79 (d, *J=* 1.9 Hz, 1H), 6.69 (dd, *J =* 8.6, 3.9 Hz, 1H), 4.70 (s, 2H), 4.53 (t, *J =* 8.7 Hz, 2H), 4.22 (dd, *J =* 9.2, 5.1 Hz, 1H), 3.86 (s, 3H), 3.74 (d, *J =* 12.0 Hz, 2H), 3.57 (s, 2H), 3.27 (d, *J =* 8.7 Hz, 2H), 2.84 (s, 2H), 2.68 (t, *J =* 11.9 Hz, 2H), 2.59 (q, *J=* 6.9, 5.6 Hz, 2H), 2.27 (d, *J =* 8.4 Hz, 1H), 2.12 (dd, *J =* 20.1, 6.7 Hz, 3H), 1.76 (d, *J =* 14.6 Hz, 6H), 1.62 (s, 1H), 1.20 (d, *J =* 14.3 Hz, 3H). |
| 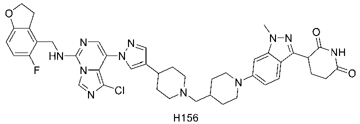 | MS m/z (ESI): 806.3 [M+H]⁺. |
| | ¹H NMR (400 MHz, DMSO-*d*₆) δ 10.84 (s, 1H), 8.85 (s, 1H), 8.54 (s, 1H), 8.31 (s, 1H), 7.68 (s, 1H), 7.47 (d, *J* = 9.0 Hz, 1H), 7.26 - 7.17 (m, 1H), 7.00 - 6.87 (m, 2H), 6.82 (s, 1H), 6.70 (dd, *J* = 8.6, 3.7 Hz, 1H), 4.82 (d, *J =* 3.5 Hz, 1H), 4.56 - 4.49 (m, 1H), 4.24 (dd, *J* = 9.1, 5.1 Hz, 1H), 3.92 - 3.71 (m, 4H), 3.33 - 3.18 (m, 3H), 2.92 (d, *J =* 11.4 Hz, 2H), 2.72 (dd, *J =* 24.2, 13.0 Hz, 2H), 2.62 (dd, *J =* 12.0, 6.8 Hz, 1H), 2.35 - 2.11 (m, 4H), 1.91 (ddd, *J* = 39.9, 21.2, 9.5 Hz, 5H), 1.78 - 1.52 (m, 4H), 1.43 - 1.11 (m, 6H).(d, *J =* 11.4 Hz, 2H), 2.72 (dd, *J =* 24.2, 13.0 Hz, 2H), 2.62 (dd, *J =* 12.0, 6.8 Hz, 1H), 2.35 - 2.11 (m, 4H), 1.91 (ddd, *J* = 39.9, 21.2, 9.5 Hz, 5H), 1.78 - 1.52 (m, 4H), 1.43 - 1.11 (m, 6H). |

### Example 157 Preparation of Compound H157

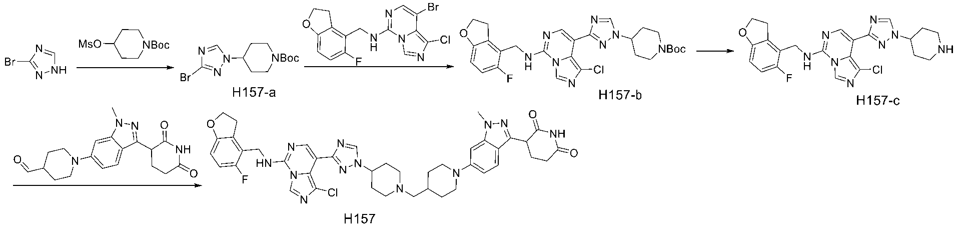

Step 1: 3-Bromo-1H-1,2,4-triazole (10 g, 67.59 mmol) and tert-butyl 4-(methylsulfonyloxy)piperidine-1-carboxylate (19.82 g, 70.96 mmol) were dissolved in DMF (50 mL). Cs₂CO₃ (44.04 g, 135.17 mmol) was added, and the mixture was stirred at 95°C for 4 hours. After the reaction was complete, the reaction solution was poured into water, extracted with ethyl acetate, the organic phase was dried over anhydrous sodium sulfate, concentrated, and purified by CombiFlash (0-60% EA/PE) to give H157-a (12.2 g, white solid), yield: 54.50%. MS m/z (ESI): 331.1 [M+H]⁺.

Step 2: 8-Bromo-1-chloro-N-[(5-fluoro-2,3-dihydrobenzofuran-4-yl)methyl]imidazo[1,5-c] pyrimidin-5-amine (1 g, 2.51 mmol) and H157-a (2.50 g, 7.54 mmol) were dissolved in DME (30 mL) and water (3 mL). Pd(OAc)₂ (112.92 mg, 502.98 µmol),CataCXium A (360.68 mg, 1.01 mmol), K₂CO₃ (1.04 g, 7.54 mmol), and (Pin)₂B₂ (1.92 g, 7.54 mmol) were added. The reaction solution was reacted at 70°C under argon protection for 8 hours. After the reaction was complete, the reaction solution was concentrated and purified by CombiFlash (0-70% EA/PE) to give H157-b (300 mg, yellow solid), yield: 20.96%. MS m/z (ESI): 569.2 [M+H]⁺.

Step 3: H157-b (930 mg, 1.63 mmol) was dissolved in DCM (30 mL). TFA (6 mL) was added, and the mixture was stirred at room temperature for 2 hours. After the reaction was complete, the reaction solution was concentrated, neutralized to pH = 8 with saturated aqueous sodium bicarbonate solution, extracted with dichloromethane, the combined organic phases were dried over anhydrous sodium sulfate, and concentrated to give H157-c (320 mg, crude), yield: 41.76%. MS m/z (ESI): 469.2 [M+H]⁺.

Step 4: H157-c (35 mg, 74.64 µmol) and 1-[3-(2,6-dioxopiperidin-3-yl)-1-methylindazol-6-yl]piperidine-4-carbaldehyde (29.10 mg, 82.10 µmol) were dissolved in DCM (10 mL). NaBH(OAc)₃ (25.31 mg, 119.42 µmol) was added, and the reaction solution was stirred at room temperature for 4 hours. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H157 (7.25 mg), yield: 12.03%. MS m/z (ESI): 807.3 [M+H]⁺. ¹H NMR (400 MHz, DMSO-d₆) δ 10.84 (s, 1H), 8.67 (s, 1H), 8.63 (s, 1H), 8.58 - 8.50 (m, 1H), 7.51 (s, 1H), 7.47 (d, J = 9.0 Hz, 1H), 6.98 - 6.86 (m, 2H), 6.86 - 6.79 (m, 1H), 6.70 (dd, J = 8.7, 3.8 Hz, 1H), 4.74 (s, 2H), 4.53 (t, J = 8.7 Hz, 2H), 4.37 - 4.21 (m, 2H), 3.88 (s, 3H), 3.83 - 3.73 (m, 2H), 3.28 (t, J = 8.9 Hz, 2H), 2.95 (d, J = 8.9 Hz, 2H), 2.79 - 2.68 (m, 2H), 2.68 - 2.58 (m, 2H), 2.35 - 1.95 (m, 10H), 1.90 - 1.79 (m, 2H), 1.76 - 1.68 (m, 1H), 1.32 - 1.20 (m, 2H).

### Example 158 Preparation of Compound H158

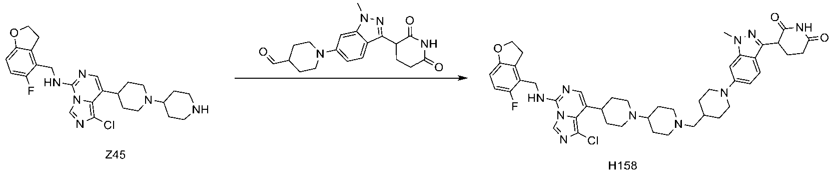

Compound H158 was prepared referring to the method of Example 71. MS m/z (ESI): 823.4 [M+H]⁺. ¹H NMR (400 MHz, DMSO-d₆) δ 10.82 (s, 1H), 8.53 (s, 1H), 8.07 (t, J = 4.8 Hz, 1H), 7.46 (d, J = 8.9 Hz, 1H), 7.06 (s, 1H), 6.97 - 6.84 (m, 2H), 6.80 (s, 1H), 6.66 (dd, J = 8.6, 3.7 Hz, 1H), 4.61 (d, J = 4.6 Hz, 2H), 4.51 (t, J = 8.7 Hz, 2H), 4.23 (dd, J = 8.9, 5.2 Hz, 1H), 3.87 (s, 3H), 3.75 (d, J = 11.9 Hz, 2H), 3.25 (t, J = 8.8 Hz, 2H), 2.97 (d, J = 10.1 Hz, 3H), 2.87 (d, J = 9.9 Hz, 2H), 2.70 (t, J = 11.7 Hz, 2H), 2.60 (dd, J = 11.6, 6.0 Hz, 2H), 2.35 - 2.04 (m, 7H), 1.96 - 1.50 (m, 11H), 1.44 (dd, J = 22.7, 12.1 Hz, 2H), 1.21 (dd, J = 24.0, 13.6 Hz, 2H).

### Example 159 Preparation of Compound H159

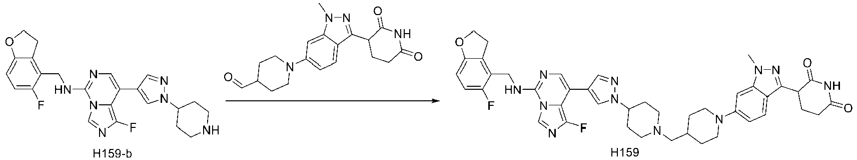

Compound H159-b (68 mg, 0.15 mmol) and 1-(3-(2,6-dioxopiperidin-3-yl)-1-methyl-1H-indazol-6-yl)piperidine-4-carbaldehyde (53 mg, 0.15 mmol) were added to 2 ml of dichloromethane. Two drops of DMSO were added, followed by 20 mg of sodium triacetoxyborohydride. The resulting mixture was heated under microwave at 35°C for 45 minutes. The resulting mixture solution was then sent for preparation to give H159. The purification conditions for preparative HPLC were: waters-sunfire-10 µm-19×150 mm column, mobile phase: 28%-38% (v/v) acetonitrile and aqueous formic acid. MS m/z (ESI): 790 [M+H]⁺. ¹H NMR (400 MHz, DMSO-d₆) δ 10.82 (s, 1H), 8.33 (s, 1H), 8.26-8.10 (m, 1H), 8.03 (s, 1H), 7.72 (s, 1H), 7.46 (d, J = 8.9 Hz, 1H), 7.27 (s, 1H), 6.97-6.86 (m, 2H), 6.86-6.77 (m, 1H), 6.67 (dd, J = 8.7, 3.8 Hz, 1H), 4.66 (d, J = 5.0 Hz, 2H), 4.52 (t, J = 8.7 Hz, 2H), 4.23 (dd, J = 9.1, 5.1 Hz, 2H), 3.87 (s, 3H), 3.78 (d, J = 11.7 Hz, 3H), 2.73 (t, J = 12.1 Hz, 2H), 2.66-2.55 (m, 2H), 2.34-1.91 (m, 10H), 1.82 (d, J = 12.7 Hz, 3H), 1.36-1.16 (m, 5H).

### Example 160 Preparation of Compound H160

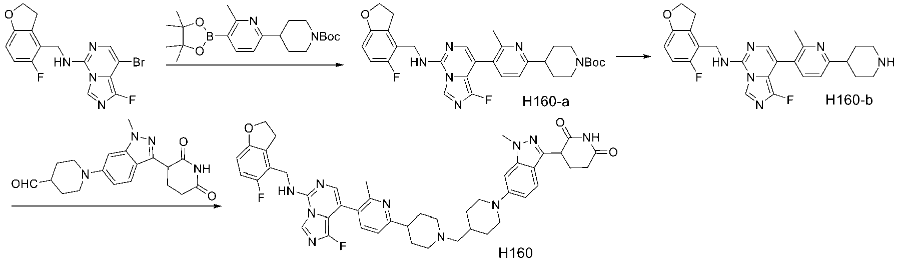

Compound H160 was prepared referring to the preparation method of Example 49, following the above synthetic route. The purification conditions for preparative HPLC were: waters-sunfire-10 µm-19×150 mm column (mobile phase: 28%-38% (v/v) acetonitrile and aqueous formic acid). MS m/z (ESI): 815 [M+H]⁺. ¹H NMR (400 MHz, DMSO-d₆) δ 10.83 (s, 1H), 8.35 (d, J = 10.8 Hz, 2H), 7.55 (d, J = 7.9 Hz, 1H), 7.46 (d, J = 9.0 Hz, 1H), 7.16 (d, J = 7.8 Hz, 1H), 7.05 (s, 1H), 6.98-6.87 (m, 2H), 6.81 (d, J = 1.9 Hz, 1H), 6.69 (dd, J = 8.7, 3.8 Hz, 1H), 4.69 (d, J = 4.8 Hz, 2H), 4.54 (t, J = 8.7 Hz, 2H), 4.23 (dd, J = 9.2, 5.1 Hz, 1H), 3.87 (s, 3H), 3.78 (d, J = 12.1 Hz, 2H), 2.97 (d, J = 10.5 Hz, 3H), 2.73 (t, J = 11.9 Hz, 2H), 2.69-2.55 (m, 4H), 2.33 (s, 3H), 2.25-2.08 (m, 4H), 1.98 (d, J = 10.8 Hz, 2H), 1.82 (d, J = 9.7 Hz, 5H), 1.32-1.19 (m, 4H).

### Example 162 Preparation of Compound H162

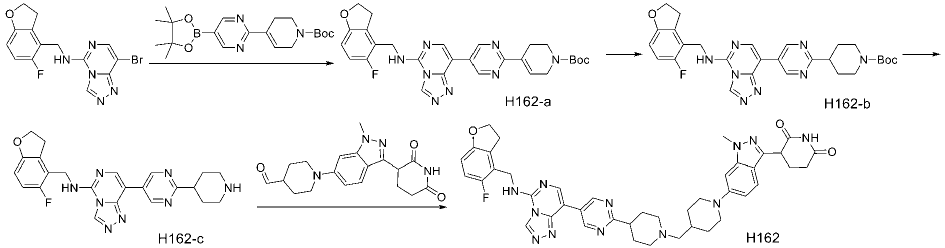

Referring to the preparation method of Example 62, following the above synthetic route, H162 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 785.4 [M+H]⁺. ¹H NMR (400 MHz, DMSO-d₆) δ 10.82 (s, 1H), 9.50 (s, 1H), 9.40 (s, 2H), 8.89 (s, 1H), 8.22 (s, 1H), 7.46 (d, J = 9.0 Hz, 1H), 7.05 - 6.87 (m, 2H), 6.81 (s, 1H), 6.70 (dd, J = 8.7, 3.8 Hz, 1H), 4.73 (s, 2H), 4.53 (t, J = 8.7 Hz, 2H), 4.23 (dd, J = 9.1, 5.1 Hz, 1H), 3.87 (s, 3H), 3.78 (d, J = 12.1 Hz, 2H), 2.96 (d, J = 10.5 Hz, 2H), 2.84 (s, 1H), 2.73 (t, J = 12.0 Hz, 2H), 2.64 - 2.56 (m, 2H), 2.19 (dd, J = 21.9, 6.3 Hz, 5H), 2.08 - 1.89 (m, 5H), 1.84 (t, J = 12.1 Hz, 5H), 1.25 (d, J = 12.4 Hz, 2H).

### Example 163 Preparation of Compound H163

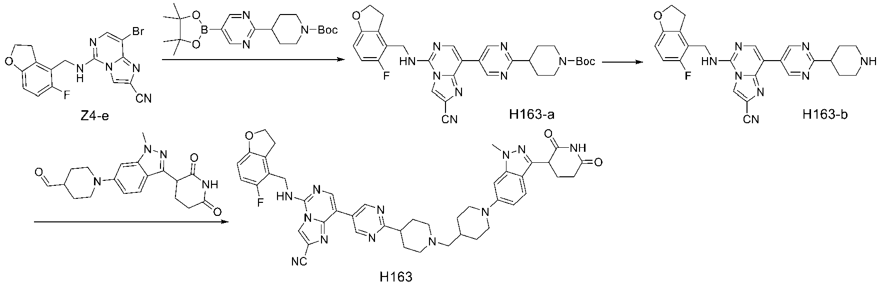

Referring to the preparation method of Example 49, following the above synthetic route, H163 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 809.4 [M+H]⁺. ¹H NMR (400 MHz, DMSO-d₆) δ 10.82 (s, 1H), 9.28 (s, 2H), 8.94 (s, 1H), 8.68 (s, 1H), 8.26 (s, 1H), 7.46 (d, J = 8.9 Hz, 1H), 6.98 - 6.86 (m, 2H), 6.81 (d, J = 1.9 Hz, 1H), 6.69 (dd, J = 8.6, 3.9 Hz, 1H), 4.73 (s, 2H), 4.53 (t, J = 8.7 Hz, 2H), 4.23 (dd, J = 9.1, 5.1 Hz, 1H), 3.87 (s, 3H), 3.77 (d, J = 12.1 Hz, 2H), 3.28 (d, J = 2.0 Hz, 1H), 2.95 (d, J = 10.4 Hz, 2H), 2.84 (s, 1H), 2.73 (t, J = 12.0 Hz, 2H), 2.60 (q, J = 6.5, 5.4 Hz, 2H), 2.34 - 2.09 (m, 4H), 2.02 (d, J = 10.9 Hz, 2H), 1.99 - 1.68 (m, 8H), 1.25 (d, J = 11.9 Hz, 2H).

### Example 164 Preparation of Compound H164

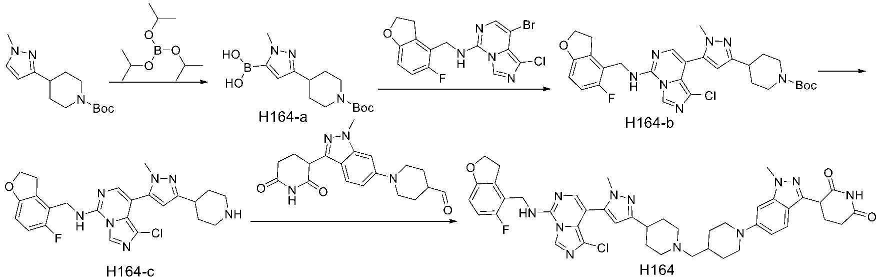

Step 1: Under argon protection at -78°C, n-BuLi (2.5 mol/L, 1.81 mL) was added dropwise to a solution of tert-butyl 4-(1-methyl-1H-pyrazol-3-yl)piperidine-1-carboxylate (600 mg, 2.26 mmol) in THF (20 mL). After reaction at -78°C for 1 hour, triisopropyl borate (1.28 g, 6.78 mmol) was added dropwise, and the mixture was reacted at room temperature for 2 hours after addition. After the reaction was complete, dilute hydrochloric acid (1 mol/L) was added to the reaction solution to adjust the pH to 5-6, then the mixture was stirred at room temperature for 0.5 hour, separated, the organic phase was dried over anhydrous sodium sulfate, and concentrated to give H164-a (150 mg, white solid, crude), yield: 21.46%. MS m/z (ESI): 310.2 [M+H]⁺.

Step 2: Under argon protection, 8-bromo-1-chloro-N-((5-fluoro-2,3-dihydrobenzofuran-4-yl)methyl)imidazo[1,5-c]pyrimidin-5-amine (120 mg, 301.79 µmol), H164-a (225.73 mg, 452.68 µmol), Pd(dppf)Cl₂ (17.67 mg, 24.14 µmol), K₂CO₃ (125.13 mg, 905.37 µmol), 1,4-dioxane (8 mL), and water (2 mL) were mixed and heated to 100°C for reaction for 5 hours. After the reaction was complete, the reaction solution was filtered, the filtrate was separated, the organic phase was dried over anhydrous sodium sulfate, concentrated, and purified by silica gel column chromatography (PE:EA = 70%:30%) to give H164-b (60 mg, yellow oil), yield: 34.16%. MS m/z (ESI): 582.3 [M+H]⁺.

Step 3: At room temperature, H164-b (55 mg, 94.49 µmol), TFA (0.5 mL), and DCM (0.5 mL) were mixed and reacted for 1 hour. After the reaction was complete, the reaction solution was concentrated, organic solvent (DCM:MeOH = 10:1) was added to dissolve, then saturated aqueous sodium bicarbonate solution was added to adjust the pH to 8-9, the layers were separated, the organic phase was dried over anhydrous sodium sulfate, and concentrated to give H164-c (45 mg, yellow oil, crude), yield: 98.81%. MS m/z (ESI): 482.2 [M+H]⁺.

Step 4: At room temperature, H164-c (40 mg, 83.00 µmol), 1-(3-(2,6-dioxopiperidin-3-yl)-1-methyl-1H-indazol-6-yl)piperidine-4-carbaldehyde (35.30 mg, 99.59 µmol), NaBH(OAc)₃ (52.77 mg, 248.99 µmol), DMSO (0.1 mL), and DCM (0.5 mL) were mixed and reacted for 1 hour. After the reaction was complete, water was added to quench the reaction, the mixture was concentrated, and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H164 (19.33 mg), yield: 27.40%. MS m/z (ESI): 820.4 [M+H]⁺. ¹H NMR (400 MHz, DMSO-d₆) δ 10.82 (s, 1H), 8.64 (s, 1H), 8.54 (t, J = 4.4 Hz, 1H), 7.46 (d, J = 9.0 Hz, 1H), 7.20 (s, 1H), 6.95 - 6.87 (m, 2H), 6.80 (s, 1H), 6.69 (dd, J = 8.6, 3.8 Hz, 1H), 6.13 (s, 1H), 4.69 (d, J = 4.4 Hz, 2H), 4.53 (t, J = 8.8 Hz, 2H), 4.23 (dd, J = 9.2, 5.2 Hz, 1H), 3.86 (s, 3H), 3.76 (d, J = 12.0 Hz, 2H), 3.53 (s, 3H), 3.29 (d, J = 8.8 Hz, 2H), 2.88 (d, J = 9.2 Hz, 2H), 2.71 (t, J = 11.4 Hz, 2H), 2.60 (dd, J = 11.6, 7.0 Hz, 2H), 2.33 - 2.22 (m, 2H), 2.19 - 2.14 (m, 2H), 2.01 - 1.93 (m, 2H), 1.88 - 1.78 (m, 4H), 1.67 - 1.57 (m, 3H), 1.28 - 1.28 (m, 3H).

### Example 165 Preparation of Compound H165

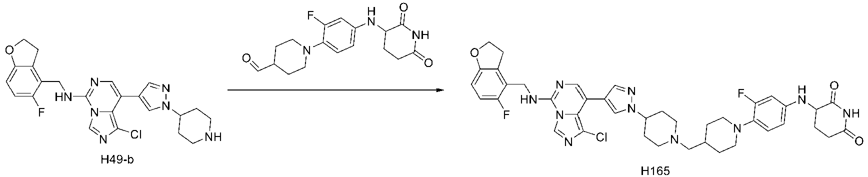

Compound H165 was prepared referring to the method of Example 71. MS m/z (ESI): 785 [M+H]⁺. ¹H NMR (400 MHz, DMSO-d₆) δ 10.75 (s, 1H), 8.61 (s, 1H), 8.28 (t, J = 5.1 Hz, 1H), 7.93 (s, 1H), 7.56 (s, 1H), 7.15 (s, 1H), 6.97 - 6.87 (m, 1H), 6.81 (t, J = 9.3 Hz, 1H), 6.67 (dd, J = 8.6, 3.9 Hz, 1H), 6.53 - 6.44 (m, 1H), 6.43 - 6.35 (m, 1H), 5.75 (d, J = 7.6 Hz, 1H), 4.67 (d, J = 5.0 Hz, 2H), 4.52 (t, J = 8.7 Hz, 2H), 4.29 - 4.01 (m, 3H), 3.26 (t, J = 8.7 Hz, 1H), 3.10 (d, J = 11.1 Hz, 1H), 2.93 (d, J = 10.5 Hz, 1H), 2.71 (ddd, J = 17.4, 12.0, 5.3 Hz, 1H), 2.60 - 2.50 (m, 3H), 2.20 (s, 2H), 2.11 - 1.90 (m, 7H), 1.86 - 1.70 (m, 3H), 1.57 (s, 1H), 1.23 (d, J = 9.2 Hz, 4H).

### Example 166 Preparation of Compound H166

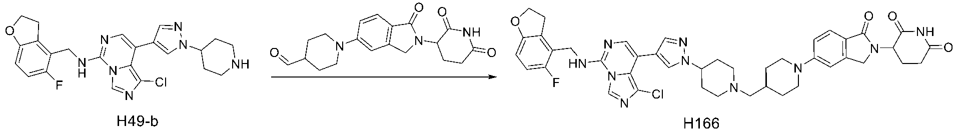

Referring to the preparation method of Example 109, following the above synthetic route, H166 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 807.3 [M+H]⁺. ¹H NMR (400 MHz, DMSO-d₆) δ 10.92 (s, 1H), 8.61 (s, 1H), 8.29 (t, J = 4.8 Hz, 1H), 7.93 (s, 1H), 7.57 (s, 1H), 7.48 (d, J = 9.2 Hz, 1H), 7.15 (s, 1H), 7.04-7.02 (m, 2H), 6.94 - 6.89 (m, 1H), 6.67 (dd, J = 8.8, 3.8 Hz, 1H), 5.02 (dd, J = 13.2, 5.2 Hz, 1H), 4.67 (d, J = 4.8 Hz, 2H), 4.52 (t, J = 8.8 Hz, 2H), 4.32-4.12 (m, 3H), 3.86 (d, J = 12.0 Hz, 2H), 3.27 (t, J = 8.8 Hz, 2H), 2.96 - 2.86 (m, 3H), 2.85 - 2.78 (m, 2H), 2.61 - 2.53 (m, 2H), 2.34 (dd, J = 13.0, 4.4 Hz, 1H), 2.18 (d, J = 6.4 Hz, 2H), 2.08 - 1.93 (m, 6H), 1.78 (d, J = 11.6 Hz, 2H), 1.22-1.13 (m, 3H).

### Example 167 Preparation of Compound H167

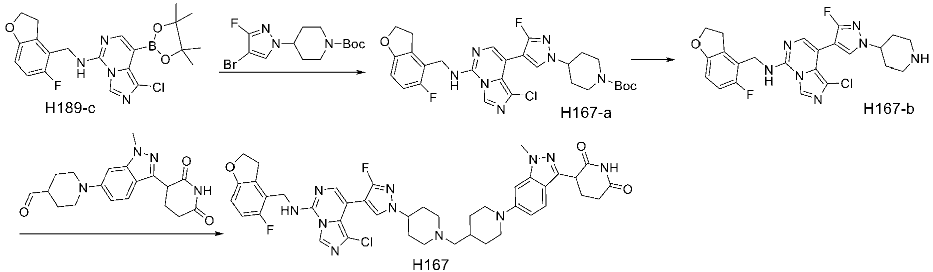

Referring to the preparation method of Example 189, following the above synthetic route, H167 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 824.3 [M+H]⁺. ¹H NMR (400 MHz, DMSO-d₆) δ 10.81 (s, 1H), 8.60 (s, 1H), 8.39 (t, J = 5.2 Hz, 1H), 7.86 (d, J = 2.3 Hz, 1H), 7.45 (d, J = 9.0 Hz, 1H), 7.11 (s, 1H), 6.98 - 6.85 (m, 2H), 6.79 (d, J = 1.9 Hz, 1H), 6.67 (dd, J = 8.7, 3.9 Hz, 1H), 4.66 (d, J = 5.0 Hz, 2H), 4.51 (t, J = 8.7 Hz, 2H), 4.22 (dd, J = 9.1, 5.1 Hz, 1H), 4.1-3.97 (m, 1H), 3.85 (s, 3H), 3.76 (d, J = 12.2 Hz, 2H), 3.29-3.24 (m, 1H), 2.98-2.85 (m, 2H), 2.76-2.65 (m, 2H), 2.64-2.54 (m, 2H), 2.32-2.08 (m, 4H), 2.08-1.84 (m, 6H), 1.84-1.62 (m, 3H), 1.27-1.21 (m, 3H).

### Example 168 Preparation of Compound H168

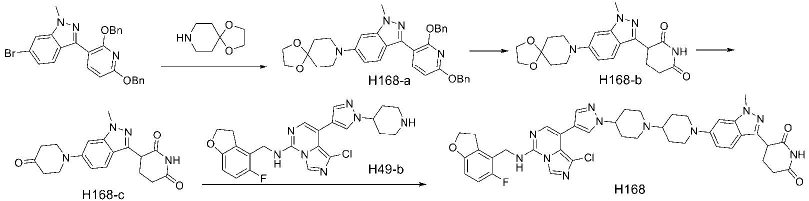

Step 1: 3-(2,6-Bis(benzyloxy)pyridin-3-yl)-6-bromo-1-methyl-1H-indazole (1 g, 2.00 mmol) and 1,4-dioxa-8-azaspiro[4.5]decane (858.44 mg, 6.00 mmol) were dissolved in 1,4-dioxane (25 mL). Under argon protection, Pd₂(dba)₃ (183.00 mg, 199.85 µmol), X-Phos (190.54 mg, 399.69 µmol), and Cs₂CO₃ (1.63 g, 5.00 mmol) were added, and the temperature was raised to 100°C and stirred for 8 h. After the reaction was complete, the reaction solution was concentrated, water and ethyl acetate were added, extraction was performed with ethyl acetate (70 mL×3), the combined organic phases were washed with brine (70 mL), dried over anhydrous sodium sulfate, concentrated, and purified by CombiFlash (20 g, 0-80% EA/PE) to give H168-a (1.03 g, light yellow oil), yield: 91.60%. MS m/z (ESI): 563.3 [M+H]⁺.

Step 2: H168-a (1.03 g, 1.83 mmol) and Pd/C (500 mg) were dissolved in MeOH (30 mL). The mixture was purged with hydrogen three times, and reacted under a hydrogen balloon at 45°C for 8 hours. The starting material was completely reacted. The reaction solution was filtered, and the filtrate was concentrated to give product H168-b (650 mg, light yellow solid), yield: 92.36%. MS m/z (ESI): 385.2 [M+H]⁺.

Step 3: H168-b (650 mg, 1.69 mmol) was dissolved in THF (10 mL). HCl (4 mol/L solution in 1,4-dioxane, 10 mL) was then added, and the mixture was stirred at room temperature for 4 hours. The starting material was completely reacted. The mixture was neutralized with saturated aqueous sodium bicarbonate solution, extracted with dichloromethane (50 mL×3). The combined organic phases were dried over anhydrous sodium sulfate and concentrated to give H168-c (270 mg, light yellow solid, crude), yield: 46.91%. MS m/z (ESI): 341.1 [M+H]⁺.

Step 4: H49-b (200 mg, 427.42 µmol) and H168-c (189.13 mg, 555.64 µmol) were dissolved in DMSO (4 mL) and EtOH (1.3 mL). NaBH₃CN (214.88 mg, 3.42 mmol) and CH₃COOH (51.33 mg, 854.84 µmol) were then added, and the mixture was stirred under microwave at 100°C for 1 hour. After the reaction was complete, the reaction solution was purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H168 (29.64 mg), yield: 8.32%. MS m/z (ESI): 792.3 [M+H]⁺. ¹H NMR (400 MHz, DMSO-d₆) δ 10.83 (s, 1H), 8.62 (s, 1H), 8.28 (s, 1H), 7.93 (s, 1H), 7.57 (s, 1H), 7.47 (d, J = 8.9 Hz, 1H), 7.15 (s, 1H), 6.92 (t, J = 9.5 Hz, 2H), 6.83 (s, 1H), 6.67 (dd, J = 8.5, 3.8 Hz, 1H), 4.68 (d, J = 4.4 Hz, 2H), 4.52 (t, J = 8.7 Hz, 2H), 4.24 (dd, J = 8.9, 4.9 Hz, 1H), 4.14 (t, J = 11.7 Hz, 1H), 3.88 (s, 3H), 3.84 (d, J = 11.7 Hz, 2H), 3.26 (d, J = 8.6 Hz, 2H), 3.00 (d, J = 10.5 Hz, 2H), 2.73 (t, J = 11.2 Hz, 2H), 2.60 (dd, J = 10.0, 7.6 Hz, 2H), 2.45 (s, 1H), 2.29 (dt, J = 14.5, 9.0 Hz, 3H), 2.20 - 2.10 (m, 1H), 2.04 (d, J = 12.0 Hz, 2H), 1.99 - 1.89 (m, 2H), 1.85 (d, J = 11.7 Hz, 2H), 1.59 (dd, J = 23.4, 13.3 Hz, 2H).

### Example 169 Preparation of Compound H169

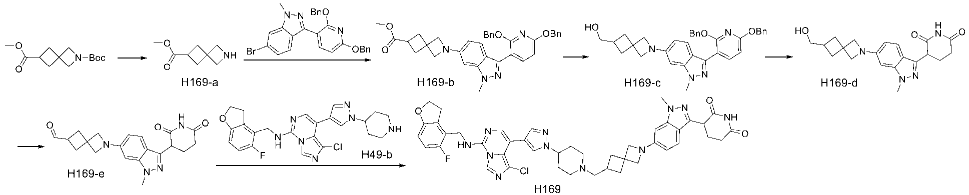

Step 1: Di-tert-butyl 2-azaspiro[3.3]heptane-2,6-dicarboxylate (2 g, 7.83 mmol) was dissolved in DCM (10 mL) and TFA (5 mL) and reacted at room temperature for 3 h. After the reaction was complete, the reaction solution was concentrated to give H169-a (crude). MS m/z (ESI): 156.1 [M+H]⁺.

Step 2: H169-a (516.91 mg, 3.33 mmol) and 3-(2,6-bis(benzyloxy)pyridin-3-yl)-6-bromo-1-methyl-1H-indazole (2 g, 4.00 mmol) were dissolved in 1,4-dioxane (20 mL). Under argon protection, Pd₂(dba)₃ (305.00 mg, 333.08 µmol), X-Phos (317.57 mg, 666.15 µmol), and Cs₂CO₃ (2.17 g, 6.66 mmol) were added, and the temperature was raised to 100°C and stirred overnight. After the reaction was complete, the reaction solution was concentrated and purified by CombiFlash (20 g, 0-30% EA/PE) to give H169-b (1.3 g, yellow solid), yield: 67.92%. MS m/z (ESI): 575.3 [M+H]⁺.

Step 3: H169-b (451.55 mg, 785.76 µmol) was dissolved in THF (10 mL). Lithium aluminum hydride (89.47 mg, 2.36 mmol) was added dropwise under a dry ice bath, and the mixture was reacted at -78°C for 2 h. After the reaction was complete, saturated aqueous ammonium chloride solution was added to the reaction solution, extraction was performed twice with ethyl acetate, the combined organic phases were concentrated, and purified by combi-flash (DCM:MeOH = 10:1) to give H169-c (400 mg, yellow solid), yield: 93.12%. MS m/z (ESI): 547.3 [M+H]⁺.

Step 4: H169-c (400 mg, 731.72 µmol) was dissolved in EtOH (6 mL). Pd/C (88.87 mg, 731.72 µmol) was added, the mixture was purged with hydrogen three times, and reacted under a hydrogen balloon for 16 h. After the reaction was complete, the reaction solution was concentrated to give H169-d (crude). MS m/z (ESI): 369.2 [M+H]⁺.

Step 5: H169-d (100 mg, 271.42 µmol) and 2-iodoxybenzoic acid (149.61 mg, 618.22 µmol) were dissolved in EA (5 mL) and reacted at 50°C overnight. After the reaction was complete, the reaction solution was concentrated to give H169-e (crude). MS m/z (ESI): 367.2 [M+H]⁺.

Step 6: H49-b (34 mg, 72.78 µmol) was dissolved in DCM (5 mL). H169-e (40 mg, 109.87 µmol) was added and stirred at room temperature for 2 h. NaBH(OAc)₃ (65.43 mg, 308.7 µmol) was added, and the mixture was stirred at room temperature overnight. After the reaction was complete, the reaction solution was concentrated and preliminarily purified by CombiFlash (4 g, 0-12% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H169 (7.71 mg, purity 99.7%), yield: 12.91%. MS m/z (ESI): 818.3 [M+H]⁺. ¹H NMR (400 MHz, DMSO-d₆) δ 10.80 (s, 1H), 8.59 (s, 1H), 8.27 (s, 1H), 7.91 (s, 1H), 7.55 (s, 1H), 7.43 (d, J = 8.7 Hz, 1H), 7.13 (d, J = 1.3 Hz, 1H), 6.95 - 6.83 (m, 1H), 6.66 (dd, J = 8.6, 3.8 Hz, 1H), 6.36 - 6.21 (m, 2H), 4.66 (d, J = 4.9 Hz, 2H), 4.50 (t, J = 8.7 Hz, 2H), 4.20 (dd, J = 9.0, 5.1 Hz, 1H), 4.12 (s, 1H), 3.81 (t, J = 25.1 Hz, 8H), 3.25 (t, J = 8.6 Hz, 2H), 2.94 - 2.84 (m, 2H), 2.58 (d, J = 5.7 Hz, 2H), 2.33 (d, J = 32.2 Hz, 6H), 2.14 - 2.05 (m, 2H), 2.00 - 1.85 (m, 6H).

### Example 170 Preparation of Compound H170

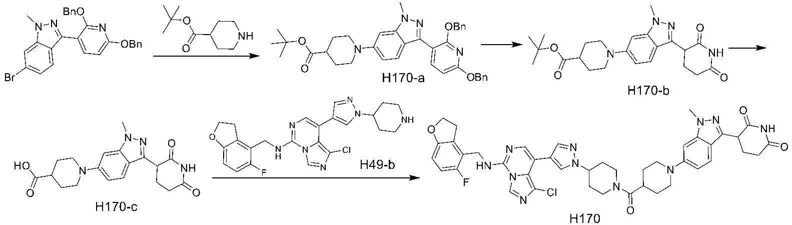

Steps 1-3: Referring to the preparation method of Example 168, following the above synthetic route, H170-c was prepared. MS m/z (ESI): 371.1 [M+H]⁺.

Step 4: H49-b (50 mg, 106.85 µmol) and H170-c (51.45 mg, 138.91 µmol) were dissolved in DMF (5 mL). TEA (32.44 mg, 320.56 µmol, 44.71 µL) and HATU (80.63 mg, 213.71 µmol) were added, and the mixture was stirred at room temperature for 1 hour. After the reaction was complete, the reaction solution was concentrated, ethyl acetate and saturated aqueous sodium bicarbonate solution were added, extraction was performed three times with ethyl acetate, the combined organic phases were dried over anhydrous sodium sulfate, concentrated, and purified by CombiFlash (4 g, 0-11% MeOH/DCM), then further purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H170 (59.76 mg, purity 98.95%), yield: 67.46%. MS m/z (ESI): 820.3 [M+H]⁺. ¹H NMR (400 MHz, DMSO-d₆) δ 10.81 (s, 1H), 8.61 (s, 1H), 8.27 (t, J = 5.2 Hz, 1H), 7.96 (d, J = 0.8 Hz, 1H), 7.58 (d, J = 0.8 Hz, 1H), 7.46 (d, J = 9.2 Hz, 1H), 7.15 (s, 1H), 6.96 - 6.87 (m, 2H), 6.82 (d, J = 2.0 Hz, 1H), 6.66 (dd, J = 8.8, 4.0 Hz, 1H), 4.67 (d, J = 4.8 Hz, 2H), 4.50 (q, J = 7.6, 6.4 Hz, 4H), 4.23 (dd, J = 9.2, 5.2 Hz, 1H), 4.13 (d, J = 13.2 Hz, 1H), 3.86 (s, 3H), 3.80 (d, J = 12.0 Hz, 2H), 3.25 (d, J = 8.8 Hz, 3H), 2.90-2.70 (m, 4H), 2.65 - 2.53 (m, 2H), 2.34 - 2.22 (m, 1H), 2.20 - 2.02 (m, 3H), 1.89 (d, J = 11.6 Hz, 1H), 1.73 (s, 5H).

### Example 171 Preparation of Compound H171

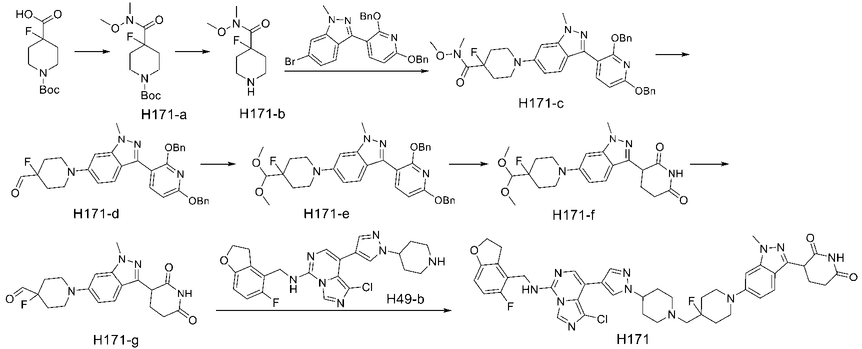

Step 1: Under ice bath cooling, HATU (8.39 g, 22.24 mmol) was added to a solution of 1-(tert-butoxycarbonyl)-4-fluoropiperidine-4-carboxylic acid (5 g, 20.22 mmol), dimethylhydroxylamine hydrochloride (2.96 g, 30.33 mmol), TEA (6.14 g, 60.66 mmol, 8.46 mL), and DCM (49.15 mL). After addition, the mixture was reacted at room temperature for 1 hour. After the reaction was complete, the reaction solution was poured into 10% aqueous citric acid solution, separated, the organic layer was washed with saturated brine, concentrated, and purified by silica gel column chromatography (dichloromethane = 100%) to give H171-a (5.6 g, colorless liquid), yield: 95.39%. MS m/z (ESI): 235.1 [M+H-56]⁺.

Step 2: At room temperature, TFA (10 mL) was added to a solution of H171-a (5.5 g, 18.94 mmol) in DCM (15 mL). After addition, the mixture was reacted at room temperature for 1 hour. After the reaction was complete, the reaction solution was concentrated to give H171-b (3.5 g, colorless liquid, crude), yield: 97.13%. MS m/z (ESI): 191.1 [M+H]⁺.

Step 3: 3-(2,6-Bis(benzyloxy)pyridin-3-yl)-6-bromo-1-methyl-1H-indazole (0.9 g, 1.80 mmol) and H171-b (1.03 g, 5.40 mmol) were dissolved in 1,4-dioxane (30 mL) and DMSO (3 mL). Under argon protection, Pd₂(dba)₃ (164.70 mg, 179.86 µmol), X-Phos (171.49 mg, 359.72 µmol), and Cs₂CO₃ (2.34 g, 7.19 mmol) were added, and the temperature was raised to 100°C and stirred overnight. After the reaction was complete, the reaction solution was filtered, and the filtrate was concentrated and purified by CombiFlash (PE:EA = 50%:50%) to give H171-c (1 g, brown oil), yield: 91.19%. MS m/z (ESI): 610.3 [M+H]⁺.

Step 4: Under argon protection at -78°C, DIBAL-H (1 mol/L, 10.66 mL) was added to a solution of H171-c (650 mg, 1.07 mmol) in THF (20 mL). After addition, the mixture was reacted at -78°C for 2 hours. After the reaction was complete, water (0.43 mL), sodium hydroxide solution (15 wt%, 0.43 mL), and water (1 mL) were added dropwise to the reaction solution. After stirring at room temperature for 0.5 hour, anhydrous magnesium sulfate was added for drying for 0.5 hour, filtered, and the filtrate was concentrated to give H171-d (450 mg, yellow oil), yield: 76.66%. MS m/z (ESI): 569.3 [M+H+18]⁺.

Step 5: H171-d (380 mg, 690.13 µmol), trimethyl orthoformate (366.18 mg, 3.45 mmol), TsOH (11.88 mg, 69.01 µmol), MeOH (221.13 mg, 6.90 mmol), and toluene (20 mL) were mixed and reacted at 110°C for 48 hours. After the reaction was complete, saturated aqueous sodium bicarbonate solution was added to adjust the pH to 8-9, then the mixture was concentrated to give H171-e (360 mg, yellow oil, crude), yield: 87.42%. MS m/z (ESI): 597.3 [M+H]⁺.

Step 6: Under a hydrogen atmosphere, H171-e (340 mg, 569.81 µmol), palladium on carbon (60.64 mg, 56.98 µmol, 10% purity), EtOH (4 mL), and THF (4 mL) were mixed and heated to 50°C for reaction for 16 hours. After the reaction was complete, the reaction solution was filtered, and the filtrate was concentrated to give H171-f (220 mg, gray oil, crude), yield: 92.27%. MS m/z (ESI): 419.2 [M+H]⁺.

Step 7: At room temperature, H171-f (210 mg, 501.84 µmol), TFA (6 mL), and DCM (6 mL) were mixed and stirred for 1 hour, then heated to 75°C and reacted for 4 hours. After the reaction was complete, the reaction solution was concentrated, dissolved in organic solvent (DCM:MeOH = 10:1), then saturated aqueous sodium bicarbonate solution was added to adjust the pH to 7-8, separated, the organic phase was dried over anhydrous sodium sulfate, and concentrated to give H171-g (160 mg, yellow oil, purity 38%), yield: 32.53%. MS m/z (ESI): 391.2 [M+H+18]⁺.

Step 8: H49-b (30 mg, 64.11 µmol), H171-g (75.40 mg, 76.94 µmol), DCM (3 mL), and AcOH (0.01 mL) were mixed and heated to 40°C for reaction for 1 hour. NaBH(OAc)₃ (40.76 mg, 192.34 µmol) was then added, and the mixture was reacted at room temperature for 2 hours. After the reaction was complete, water was added to quench the reaction, the mixture was concentrated, and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H171 (2.12 mg, white solid), yield: 4.01%. MS m/z (ESI): 824.3 [M+H]⁺. ¹H NMR (400 MHz, DMSO-d₆) δ 10.82 (s, 1H), 8.60 (s, 1H), 8.27 (t, J = 5.2 Hz, 1H), 7.92 (s, 1H), 7.56 (s, 1H), 7.47 (d, J = 8.8 Hz, 1H), 7.14 (s, 1H), 6.94 - 6.86 (m, 3H), 6.66 (dd, J = 8.8, 3.8 Hz, 1H), 4.66 (d, J = 4.8 Hz, 2H), 4.51 (t, J = 8.8 Hz, 2H), 4.23 (dd, J = 9.2, 5.2 Hz, 1H), 4.17 - 4.10 (m, 1H), 3.87 (s, 3H), 3.56 (d, J = 12.0 Hz, 2H), 3.24 (d, J = 8.8 Hz, 2H), 3.10 - 2.97 (m, 4H), 2.59 (d, J = 8.4 Hz, 2H), 2.54 (s, 1H), 2.33 - 2.23 (m, 3H), 2.17-2.09 (m, 1H), 2.01 - 1.87 (m, 6H), 1.86 - 1.73 (m, 2H), 1.21 (s, 1H).

### Example 172 Preparation of Compound H172

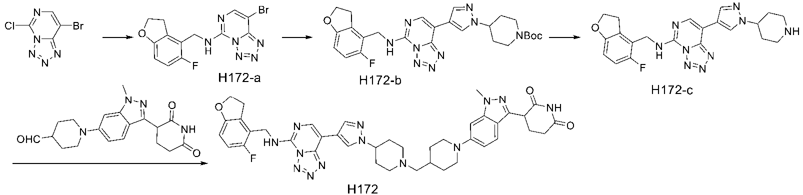

Step 1: 8-Bromo-5-chlorotetrazolo[1,5-c]pyrimidine (464 mg, 2 mmol), (5-fluoro-2,3-dihydrobenzofuran-4-yl)methanamine (334 mg, 2 mmol), and triethylamine (1 mL) were added to 1,4-dioxane (3 mL) and heated to 80°C for reaction for 30 minutes. After the reaction was complete, the reaction solution was purified by silica gel column chromatography (30-100% PE/EA) to give H172-a (546 mg), yield: 75%. MS m/z (ESI): 365 [M+H]⁺.

Steps 2-4: Referring to the preparation method of Example 49, following the above synthetic route, H172 was prepared. The purification conditions for preparative HPLC were: waters-sunfire-10 µm-19×150 mm column (mobile phase: 28%-38% (v/v) acetonitrile and aqueous formic acid). MS m/z (ESI): 774 [M+H]⁺. ¹H NMR (400 MHz, DMSO-d₆) δ 10.81 (s, 1H), 8.44 (s, 1H), 8.35 (s, 1H), 8.14 (s, 1H), 7.45 (d, J = 8.9 Hz, 1H), 6.87 (t, J = 9.3 Hz, 2H), 6.80 (s, 1H), 6.63 (dd, J = 8.6, 3.8 Hz, 1H), 4.72 (s, 2H), 4.48 (t, J = 8.7 Hz, 3H), 4.22 (dd, J = 9.3, 5.0 Hz, 2H), 3.86 (s, 3H), 3.76 (d, J = 12.0 Hz, 2H), 2.71 (t, J = 11.8 Hz, 3H), 2.59 (q, J = 6.3, 5.4 Hz, 2H), 2.34 - 2.17 (m, 3H), 2.06 - 1.98 (m, 5H), 1.81 (d, J = 12.7 Hz, 2H), 1.40 (s, 2H), 1.14 - 1.27 (m, 6H).

### Example 173 Preparation of Compound H173

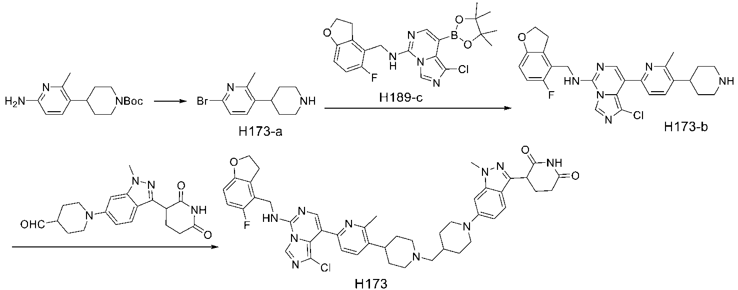

Step 1: tert-Butyl 6-amino-2-methyl-3',6'-dihydro-[3,4'-bipyridine]-1'(2'H)-carboxylate (1011 mg, 3.5 mmol) was dissolved in THF (5 mL), cooled to 0°C, and aqueous HBr solution (48 wt%, 10 mL) was added. After maintaining the temperature for 30 minutes, 1 ml of bromine solution was added dropwise. After the addition was complete, the mixture was stirred at room temperature overnight. After the reaction was complete, water (20 mL) was added to the reaction solution, then extraction was performed with ethyl acetate (30 mL×3). The combined organic phases were concentrated and purified by silica gel column chromatography (PE/EA 30-100%) to give H173-a (302 mg), yield: 28.6%. MS m/z (ESI): 255 [M+H]⁺.

Step 2: H173-a (255 mg, 1 mmol), H189-c (266 mg, 0.6 mmol), K₂CO₃ (41 mg, 3 mmol), and Pd(dppf)Cl₂ (146 mg, 0.2 mmol) were added to dioxane (5 mL) for dissolution, then water (1 mL) was added. The mixture was purged with nitrogen three times, then heated to 100°C and stirred for 5 hours. After the reaction was complete, the reaction solution was purified by silica gel column chromatography (30-100% PE/EA) to give H173-b (98 mg), yield: 33%. MS m/z (ESI): 493 [M+H]⁺.

Step 3: H173-b (98 mg, 0.2 mmol) and 1-(3-(2,6-dioxopiperidin-3-yl)-1-methyl-1H-indazol-6-yl)piperidine-4-carbaldehyde (53 mg, 0.15 mmol) were added to dichloromethane (2 mL). Two drops of DMSO were added, followed by sodium triacetoxyborohydride (15 mg). The resulting mixture was heated under microwave at 35°C for 45 minutes. After the reaction was complete, the reaction solution was purified by preparative HPLC (waters-sunfire-10 µm-19×150 mm column, mobile phase: 28%-38% (v/v) acetonitrile and aqueous formic acid) to give H173 (8.6 mg), yield: 6.9%. MS m/z (ESI): 831.4 [M+H]⁺. ¹H NMR (400 MHz, DMSO-d₆) δ 10.85 (s, 1H), 8.68 (s, 1H), 8.49 (d, J = 5.2 Hz, 1H), 7.68 (d, J = 8.1 Hz, 1H), 7.48 (d, J = 8.9 Hz, 1H), 7.34 (d, J = 8.7 Hz, 2H), 7.02-6.88 (m, 2H), 6.83 (s, 1H), 6.70 (dd, J = 8.6, 3.8 Hz, 1H), 4.73 (d, J = 4.9 Hz, 2H), 4.54 (t, J = 8.7 Hz, 2H), 4.25 (dd, J = 9.3, 5.1 Hz, 1H), 3.89 (s, 3H), 3.79 (d, J = 11.9 Hz, 2H), 3.29 (t, J = 8.7 Hz, 2H), 3.00 (d, J = 10.1 Hz, 2H), 2.73 (t, J = 11.9 Hz, 4H), 2.60 (t, J = 6.0 Hz, 2H), 2.54 (s, 3H), 2.35-2.10 (m, 5H), 2.06 (d, J = 14.7 Hz, 2H), 1.90-1.61 (m, 7H).

### Example 174 Preparation of Compound H174

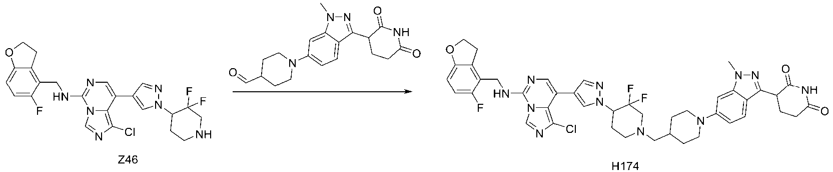

Compound H174 was prepared referring to the method of Example 71. MS m/z (ESI): 842.3 [M+H]⁺. ¹H NMR (400 MHz, DMSO-d₆) δ 10.83 (s, 1H), 8.64 (s, 1H), 8.33 (t, J = 4.9 Hz, 1H), 8.05 (s, 1H), 7.67 (s, 1H), 7.47 (d, J = 9.0 Hz, 1H), 7.20 (s, 1H), 6.92 (dd, J = 20.4, 10.0 Hz, 2H), 6.81 (s, 1H), 6.69 (dd, J = 8.6, 3.8 Hz, 1H), 4.91 - 4.82 (m, 1H), 4.69 (d, J = 4.6 Hz, 2H), 4.53 (t, J = 8.7 Hz, 2H), 4.24 (dd, J = 9.0, 5.1 Hz, 1H), 3.88 (s, 3H), 3.77 (d, J = 11.6 Hz, 2H), 3.28 (d, J = 8.8 Hz, 2H), 3.16 - 3.08 (m, 1H), 2.91 (dd, J = 24.5, 11.1 Hz, 2H), 2.73 (t, J = 12.0 Hz, 2H), 2.61 (dd, J = 11.1, 6.6 Hz, 2H), 2.47 - 2.26 (m, 4H), 2.25 - 2.06 (m, 3H), 1.89 - 1.70 (m, 3H), 1.27 (dd, J = 21.8, 9.8 Hz, 2H).

### Example 175 Preparation of Compound H175

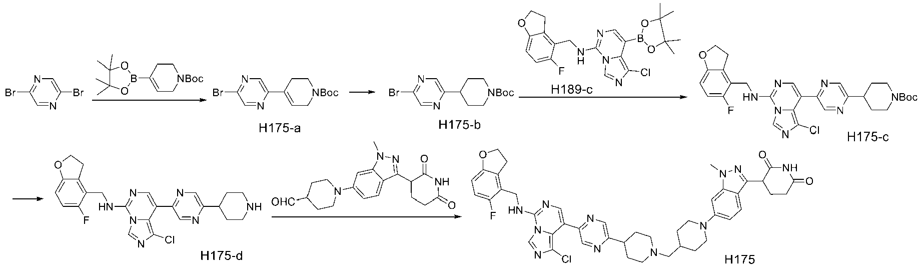

Step 1: 2,5-Dibromopyrazine (1180 mg, 5 mmol), tert-butyl 4-(4,4,5,5-tetramethyl-1,3,2-dioxaborolan-2-yl)-3,6-dihydropyridine-1(2H)-carboxylate (1545 mg, 5 mmol), K₂CO₃ (2070 mg, 15 mmol), and Pd(dppf)Cl₂ (365 mg, 0.5 mmol) were added to a mixed solvent of 1,4-dioxane (15 mL) and water (3 mL). After purging with nitrogen three times, the mixture was heated to 100°C and stirred for 5 hours. After the reaction was complete, the reaction solution was purified by silica gel column chromatography (30-100% PE/EA) to give H175-a (1190 mg). Yield: 75%. MS m/z (ESI): 340 [M+H]⁺.

Step 2: H175-a (1190 mg, 3.5 mmol) and PtO₂ (150 mg) were added to methanol (5 mL), purged with hydrogen, and stirred overnight. After the reaction was complete, the reaction solution was filtered, and the filtrate was concentrated to give H175-b (1193 mg). Yield: 100%. MS m/z (ESI): 342 [M+H]⁺.

Steps 3-5: Referring to the preparation method of Example 189, following the above synthetic route, H175 was prepared. The purification conditions for preparative HPLC were: waters-sunfire-10 µm-19×150 mm column (mobile phase: 28%-38% (v/v) acetonitrile and aqueous formic acid). MS m/z (ESI): 818 [M+H]⁺. ¹H NMR (400 MHz, DMSO-d₆) δ 10.81 (s, 1H), 8.71-8.66 (m, 2H), 8.66-8.54 (m, 2H), 7.51-7.36 (m, 2H), 7.00-6.83 (m, 2H), 6.80 (d, J = 2.0 Hz, 1H), 6.67 (dd, J = 8.7, 3.8 Hz, 1H), 4.72 (d, J = 4.6 Hz, 2H), 4.51 (t, J = 8.7 Hz, 2H), 4.22 (dd, J = 9.1, 5.1 Hz, 1H), 3.86 (s, 3H), 3.76 (d, J = 11.9 Hz, 2H), 2.97 (d, J = 10.6 Hz, 2H), 2.73 (q, J = 14.2, 11.6 Hz, 3H), 2.67-2.55 (m, 2H), 2.40-2.06 (m, 5H), 2.00 (q, J = 10.0, 8.3 Hz, 3H), 1.89-1.55 (m, 6H), 1.44 (d, J = 7.5 Hz, 1H), 1.32-1.14 (m, 2H).

### Example 176 Preparation of Compound H176

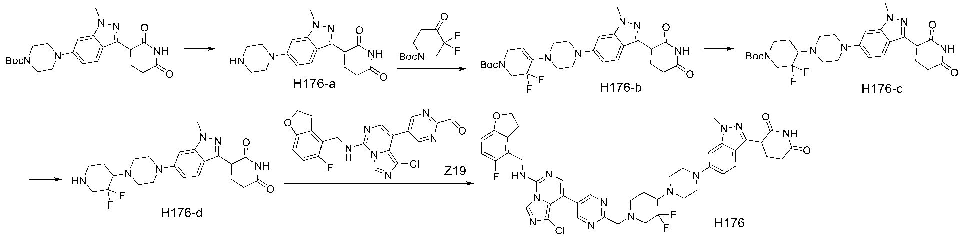

Step 1: tert-Butyl 4-[3-(2,6-dioxopiperidin-3-yl)-1-methylindazol-6-yl]piperazine-1-carboxylate (490 mg, 1.15 mmol) was dissolved in DCM (15 mL). TFA (4 mL) was added, and the reaction solution was stirred at room temperature for 4 hours. After the reaction was complete, the reaction solution was concentrated, neutralized to pH = 8 with saturated aqueous NaHCO₃ solution, extracted with DCM, the combined organic phases were dried over anhydrous sodium sulfate, and concentrated to give H176-a (280 mg, crude), yield: 74.62%. MS m/z (ESI): 328.2 [M+H]⁺.

Step 2: H176-a (280 mg, 855.27 µmol) and tert-butyl 3,3-difluoro-4-oxopiperidine-1-carboxylate (301.78 mg, 1.28 mmol) were dissolved in toluene (6 mL) and MeCN (3 mL). NaOAc (175.40 mg, 2.14 mmol) and HOAc (293.72 mg, 4.89 mmol) were added, and the reaction solution was stirred at 120°C for 20 hours. After the reaction was complete, the reaction solution was poured into water, neutralized to basic pH with saturated aqueous NaHCO₃ solution, extracted with DCM, the combined organic phases were dried over anhydrous sodium sulfate, and concentrated to give H176-b (467 mg, crude), yield: 99.89%. MS m/z (ESI): 545.3 [M+H]⁺.

Step 3: H176-b (467 mg, 857.52 µmol) was dissolved in DCE (6 mL) and MeOH (6 mL). AcOH (125.88 mg, 2.10 mmol) and NaBH₃CN (269.44 mg, 4.29 mmol) were added, and the reaction solution was stirred at room temperature overnight. After the reaction was complete, the reaction solution was poured into water, neutralized with saturated aqueous NaHCO₃ solution, extracted with DCM, the combined organic phases were dried over anhydrous sodium sulfate, and concentrated to give H176-c (369 mg, crude), yield: 78.72%. MS m/z (ESI): 547.3 [M+H]⁺.

Step 4: H176-c (369 mg, 675.07 µmol) was dissolved in DCM (10 mL). TFA (3 mL) was added, and the reaction solution was stirred at room temperature for 2 hours. After the reaction was complete, the reaction solution was concentrated, neutralized with saturated aqueous NaHCO₃ solution, extracted with DCM, the combined organic phases were dried over anhydrous sodium sulfate, and concentrated to give H176-d (283 mg, crude), yield: 93.89%. MS m/z (ESI): 447.2 [M+H]⁺.

Step 5: Intermediate Z19 (120 mg, 282.48 µmol) and H176-d (151.35 mg, 338.97 µmol) were dissolved in DCM (15 mL). NaBH(OAc)₃ (77.83 mg, 367.22 µmol) was added, and the reaction solution was stirred at room temperature overnight. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H176 (9.73 mg), yield: 3.99%. MS m/z (ESI): 855.3 [M+H]⁺. ¹H NMR (400 MHz, DMSO-d₆) δ 10.84 (s, 1H), 8.88 (s, 2H), 8.72 (s, 1H), 8.61 (t, J = 5.1 Hz, 1H), 7.49 (d, J = 9.0 Hz, 1H), 7.34 (s, 1H), 6.97-6.89 (m, 2H), 6.83 (s, 1H), 6.70 (dd, J = 8.7, 3.9 Hz, 1H), 4.73 (d, J = 4.8 Hz, 2H), 4.55 (t, J = 8.7 Hz, 2H), 4.25 (dd, J = 9.2, 5.2 Hz, 1H), 3.90 (d, J = 10.7 Hz, 4H), 3.33 (s, 2H), 3.23 - 3.14 (m, 4H), 3.04 (d, J = 9.8 Hz, 1H), 2.95 - 2.76 (m, 5H), 2.70 - 2.54 (m, 3H), 2.49 - 2.24 (m, 3H), 2.21 - 2.09 (m, 1H), 2.00 - 1.73 (m, 3H).

### Example 177 Preparation of Compound H177

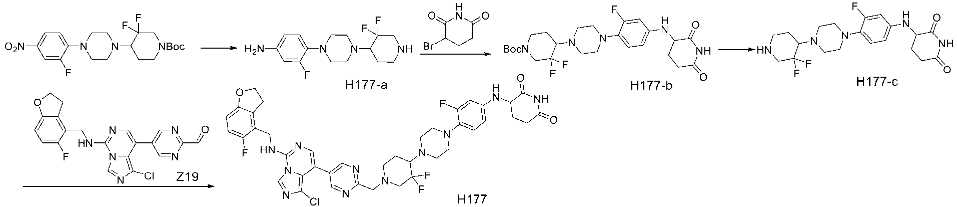

Step 1: tert-Butyl 3,3-difluoro-4-[4-(2-fluoro-4-nitrophenyl)piperazin-1-yl]piperidine-1-carboxylate (2.96 g, 6.66 mmol) was dissolved in EA (60 mL). Pd/C (0.3 g, 10% purity) was added, the mixture was purged with hydrogen three times, and reacted at room temperature overnight. After the reaction was complete, the reaction solution was filtered through Celite, and the filtrate was concentrated to give H177-a (2.76 g), yield: 100.00%. MS m/z (ESI): 315.3 [M+H]⁺.

Step 2: H177-a (1.4 g, 3.38 mmol) and 3-bromopiperidine-2,6-dione (1.95 g, 10.13 mmol) were dissolved in DMF (11 mL). NaHCO₃ (1.70 g, 20.27 mmol) was added, and the reaction solution was heated to 90°C in a sealed tube and stirred for 2 hours. After the reaction was complete, ethyl acetate (100 mL) was added to the reaction solution, the organic phase was washed with water (50 mL×3) and saturated brine (50 mL×1), dried over anhydrous sodium sulfate, concentrated, and purified by silica gel column chromatography (0-100% EA/PE) to give H177-b (1.42 g), yield: 79.99%. MS m/z (ESI): 526.3 [M+H]⁺.

Step 3: H177-b (1.47 g, 2.80 mmol) was dissolved in DCM (30 mL). TFA (6 mL) was added, and the mixture was stirred at room temperature for 2 h. After the reaction was complete, the reaction solution was concentrated, neutralized with saturated aqueous NaHCO₃ solution, extracted with DCM, the organic phase was dried over anhydrous sodium sulfate, and concentrated to give H177-c (983 mg, crude), yield: 82.59%. MS m/z (ESI): 426.2 [M+H]⁺.

Step 4: H177-c (50 mg, 117.52 µmol) and Intermediate Z19 (99.85 mg, 235.05 µmol) were dissolved in DCM (15 mL). NaBH(OAc)₃ (49.82 mg, 235.05 µmol) was added, and the reaction solution was stirred at room temperature overnight. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H177 (10.98 mg), yield: 10.59%. MS m/z (ESI): 834.3 [M+H]⁺. ¹H NMR (400 MHz, DMSO-d₆) δ 10.76 (s, 1H), 8.87 (d, J = 3.7 Hz, 2H), 8.71 (s, 1H), 8.60 (s, 1H), 7.34 (s, 1H), 6.97 - 6.90 (m, 1H), 6.82 (t, J = 9.3 Hz, 1H), 6.70 (dd, J = 8.6, 3.9 Hz, 1H), 6.50 (d, J = 15.1 Hz, 1H), 6.41 (d, J = 8.5 Hz, 1H), 5.78 (d, J = 7.5 Hz, 1H), 4.73 (d, J = 4.0 Hz, 2H), 4.55 (t, J = 8.7 Hz, 2H), 4.25 (dd, J = 13.9, 9.8 Hz, 1H), 3.94 - 3.85 (m, 2H), 3.33 (s, 2H), 3.22 - 3.15 (m, 1H), 3.03 (d, J = 11.8 Hz, 1H), 2.90 - 2.75 (m, 9H), 2.74 - 2.66 (m, 1H), 2.65 - 2.54 (m, 2H), 2.36 (dd, J = 17.8, 8.0 Hz, 1H), 2.12 - 2.05 (m, 1H), 1.88 - 1.76 (m, 3H).

### Example 178 Preparation of Compound H178

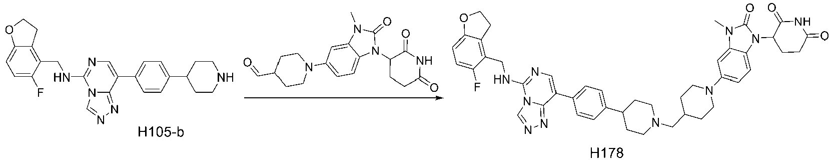

Step 1: H105-b (100 mg, 224.97 µmol) and 1-[1-(2,6-dioxopiperidin-3-yl)-3-methyl-2-oxobenzimidazol-5-yl]piperidine-4-carbaldehyde (166.66 mg, 449.94 µmol) were dissolved in DCM (10 mL). NaBH(OAc)₃ (143.04 mg, 674.91 µmol) was added with stirring at room temperature, and the mixture was stirred at room temperature overnight. After the reaction was complete, the reaction solution was concentrated and purified by preparative HPLC to give H178 (28.52 mg, purity 93.03%), yield: 14.76%. MS m/z (ESI): 799 [M+H]⁺. ¹H NMR (400 MHz, DMSO-d₆) δ 11.03 (s, 1H), 9.46 (s, 1H), 8.63 (t, J = 5.0 Hz, 1H), 7.99 (d, J = 8.5 Hz, 3H), 7.32 (d, J = 8.3 Hz, 2H), 7.00 - 6.85 (m, 2H), 6.80 (d, J = 2.0 Hz, 1H), 6.68 (dd, J = 8.7, 3.9 Hz, 1H), 6.61 (d, J = 6.6 Hz, 1H), 5.26 (dd, J = 12.8, 5.4 Hz, 1H), 4.70 (d, J = 4.7 Hz, 2H), 4.52 (t, J = 8.7 Hz, 2H), 3.57 (d, J = 11.9 Hz, 2H), 3.27 (d, J = 4.6 Hz, 5H), 2.90 (dd, J = 46.1, 13.8 Hz, 3H), 2.71 - 2.49 (m, 5H), 2.19 (d, J = 6.2 Hz, 2H), 1.97 (d, J = 10.5 Hz, 3H), 1.85 - 1.57 (m, 6H), 1.24 (d, J = 9.8 Hz, 2H).

### Example 179 Preparation of Compound H179

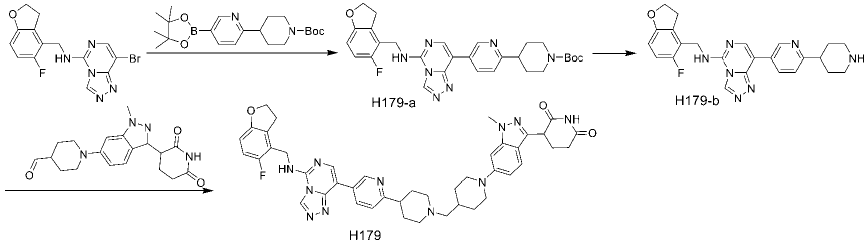

Referring to the preparation method of Example 49, following the above synthetic route, H179 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 784.4 [M+H]⁺. ¹H NMR (400 MHz, DMSO-d₆) δ 10.81 (s, 1H), 9.49 (s, 1H), 9.13 (d, J = 2.4 Hz, 1H), 8.38 (dd, J = 8.0, 2.4 Hz, 1H), 8.08 (s, 1H), 7.45 (d, J = 8.8 Hz, 1H), 7.37 (d, J = 8.4 Hz, 1H), 6.98 - 6.85 (m, 2H), 6.80 (s, 1H), 6.68 (dd, J = 8.8, 4.0 Hz, 1H), 4.71 (s, 2H), 4.52 (t, J = 8.8 Hz, 2H), 4.22 (dd, J = 9.2, 5.2 Hz, 1H), 3.86 (s, 3H), 3.76 (d, J = 12.0 Hz, 2H), 3.31 (s, 3H), 2.96 (d, J = 10.4 Hz, 2H), 2.76-2.64 (m, 3H), 2.63 - 2.54 (m, 2H), 2.36 - 2.24 (m, 1H), 2.22-2.09 (m, 3H), 2.04-1.96 (m, 2H), 1.91 - 1.62 (m, 7H), 1.30-1.17 (m, 2H).

### Example 180 Preparation of Compound H180

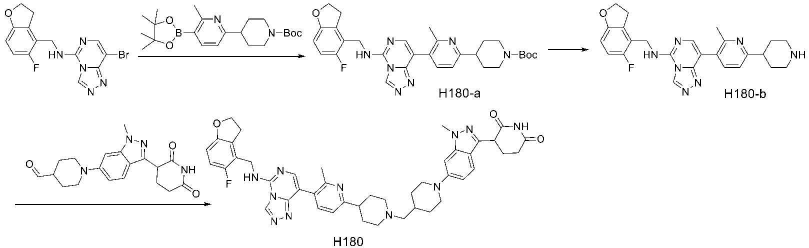

Referring to the preparation method of Example 49, following the above synthetic route, H180 was prepared. MS m/z (ESI): 798 [M+H]⁺. ¹H NMR (400 MHz, DMSO-d₆) δ 10.82 (s, 1H), 9.45 (s, 1H), 8.67 (s, 1H), 7.68 - 7.57 (m, 2H), 7.45 (d, J = 9.1 Hz, 1H), 7.18 (d, J = 8.0 Hz, 1H), 6.99 - 6.84 (m, 2H), 6.81 (s, 1H), 6.69 (dd, J = 8.5, 3.9 Hz, 1H), 4.69 (d, J = 4.4 Hz, 2H), 4.53 (t, J = 8.8 Hz, 2H), 4.22 (dd, J = 8.9, 4.9 Hz, 1H), 3.86 (s, 3H), 3.77 (d, J = 11.8 Hz, 2H), 3.32 (s, 1H), 2.96 (s, 2H), 2.78 - 2.54 (m, 5H), 2.35 (s, 3H), 2.32 - 1.62 (m, 12H), 1.31 (d, J = 34.9 Hz, 4H).

### Example 181 Preparation of Compound H181

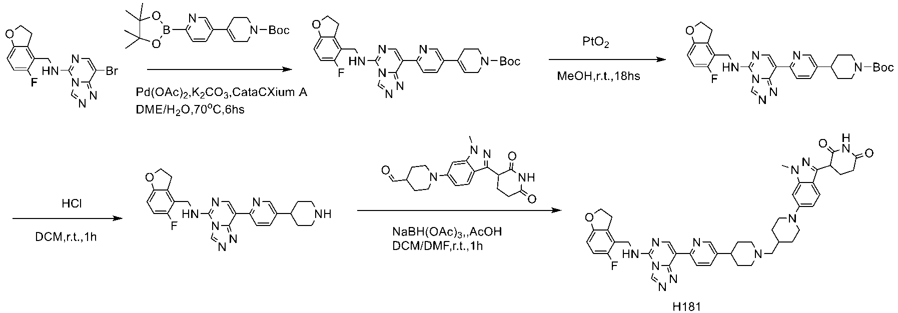

Referring to the preparation method of the above route, following the above synthetic route, H181 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 784.4 [M+H]⁺. ¹H NMR (400 MHz, DMSO-d₆) δ 10.82 (s, 1H), 9.51 (s, 1H), 8.86 (s, 1H), 8.71 (d, J = 7.8 Hz, 1H), 8.61 (s, 1H), 8.54 (s, 1H), 8.18 (s, 1H), 7.80 (d, J = 7.5 Hz, 1H), 7.46 (d, J = 8.9 Hz, 1H), 6.92 (dd, J = 21.4, 9.8 Hz, 2H), 6.81 (s, 1H), 6.71 (s, 1H), 4.74 (s, 2H), 4.53 (t, J = 8.6 Hz, 2H), 4.23 (m, 1H), 3.87 (s, 3H), 3.77 (m, 2H), 3.31 (t, J = 8.0 Hz, 4H), 2.99 (d, J = 8.0 Hz, 2H), 2.72 (d, J = 8.0 Hz, 2H), 2.64-2.55 (m, 2H), 2.36-2.19 (m, 3H), 2.20-2.10 (m, 1H), 2.03 (t, J = 8.0 Hz, 2H), 1.87-1.67 (m, 5H), 1.34-1.17 (m, 2H).

### Example 183 Preparation of Compound H183

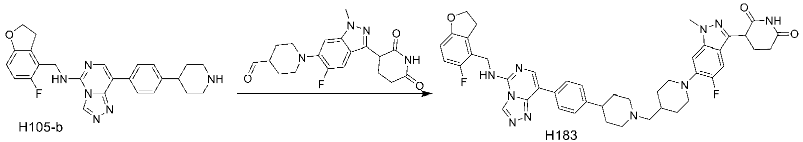

Referring to the preparation method of Example 109, following the above synthetic route, H183 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 802.4 [M+H]⁺. ¹H NMR (400 MHz, DMSO-d₆) δ 10.82 (s, 1H), 9.46 (s, 1H), 8.62 (s, 1H), 7.99 (d, J = 8.8 Hz, 3H), 7.41 (d, J = 12.4 Hz, 1H), 7.33 (d, J = 8.0 Hz, 2H), 7.07 (d, J = 7.2 Hz, 1H), 6.93 (t, J = 9.2 Hz, 1H), 6.68 (dd, J = 8.8, 4.0 Hz, 1H), 4.70 (d, J = 4.8 Hz, 2H), 4.52 (t, J = 8.8 Hz, 2H), 4.24 (dd, J = 9.6, 5.2 Hz, 1H), 3.91 (s, 3H), 3.41 (d, J = 11.2 Hz, 2H), 3.30 (s, 2H), 2.97 (d, J = 10.4 Hz, 2H), 2.67 (t, J = 12.0 Hz, 2H), 2.57 (t, J = 6.0 Hz, 2H), 2.31 (d, J = 8.8 Hz, 1H), 2.22 (s, 2H), 2.15-2.06 (m, 1H), 2.00 (s, 2H), 1.88-1.65 (m, 7H), 1.39 - 1.19 (m, 3H).

### Example 184 Preparation of Compound H184

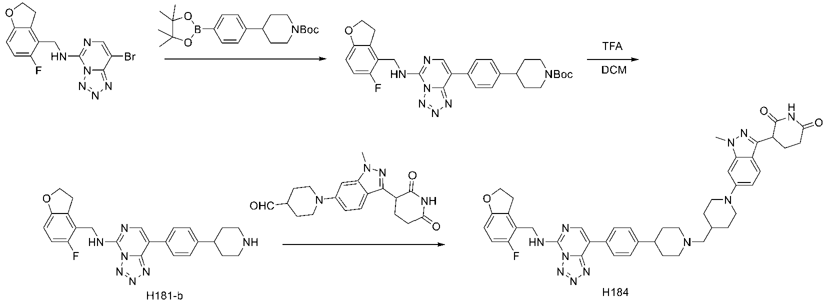

Following the above route and referring to the method of Example 71, H184 was prepared. MS m/z (ESI): 784 [M+H]⁺. ¹H NMR (400 MHz, DMSO-d₆) δ 10.82 (s, 1H), 9.50 (t, J = 5.6 Hz, 1H), 8.37 (s, 1H), 7.98 (d, J = 8.0 Hz, 2H), 7.45 (d, J = 9.0 Hz, 1H), 7.37 (d, J = 8.1 Hz, 2H), 6.88 (s, 2H), 6.80 (d, J = 1.9 Hz, 1H), 6.64 (dd, J = 8.7, 3.8 Hz, 1H), 4.75 (d, J = 5.4 Hz, 2H), 4.49 (t, J = 8.7 Hz, 2H), 4.22 (dd, J = 9.1, 5.1 Hz, 1H), 3.86 (s, 3H), 3.77 (d, J = 12.1 Hz, 2H), 2.96 (d, J = 10.7 Hz, 2H), 2.72 (t, J = 11.9 Hz, 2H), 2.58 (t, J = 5.3 Hz, 3H), 2.35 - 2.09 (m, 4H), 1.99 (s, 2H), 1.88 - 1.65 (m, 7H), 1.25 (q, J = 10.8, 9.6 Hz, 4H).

### Example 185 Preparation of Compound H185

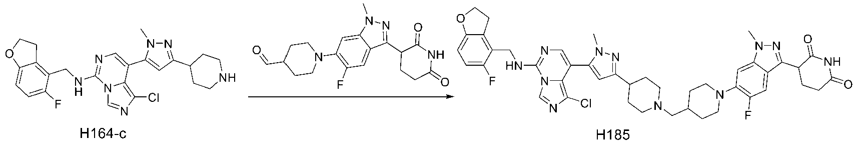

Referring to the preparation method of Example 109, following the above synthetic route, H185 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 838.3 [M+H]⁺. ¹H NMR (400 MHz, DMSO-d₆) δ 10.83 (s, 1H), 8.64 (s, 1H), 8.54 (t, J = 5.0 Hz, 1H), 7.41 (d, J = 12.6 Hz, 1H), 7.20 (s, 1H), 7.07 (d, J = 7.2 Hz, 1H), 6.95-6.90 (m, 1H), 6.69 (dd, J = 8.6, 4.0 Hz, 1H), 6.13 (s, 1H), 4.69 (d, J = 4.8 Hz, 2H), 4.53 (t, J = 8.8 Hz, 2H), 4.25 (dd, J = 9.6, 4.8 Hz, 1H), 3.92 (s, 3H), 3.53 (s, 3H), 3.41 (d, J = 11.6 Hz, 2H), 3.28 (d, J = 8.8 Hz, 2H), 2.89 (d, J = 8.8 Hz, 2H), 2.67 (t, J = 11.6 Hz, 2H), 2.61- 2.55 (m, 2H), 2.36-2.29 (m, 1H), 2.21 (d, J = 3.6 Hz, 2H), 2.15-2.08 (m, 1H), 2.03-1.95 (m, 2H), 1.85 (t, J = 14.6 Hz, 4H), 1.70-1.58 (m, 3H), 1.37-1.24 (m, 3H).

### Example 186 Preparation of Compound H186

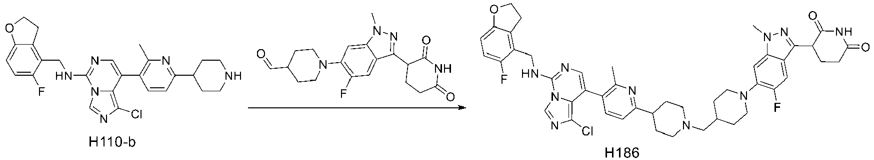

H110-b (100 mg, 202.85 µmol) and 1-(3-(2,6-dioxopiperidin-3-yl)-5-fluoro-1-methyl-1H-indazol-6-yl)piperidine-4-carbaldehyde (75.54 mg, 202.85 µmol) were dissolved in DCM (10 mL). NaBH(OAc)₃ (42.99 mg, 202.85 µmol) was added with stirring at room temperature, and the mixture was stirred at room temperature overnight. After the reaction was complete, the reaction solution was concentrated and purified by preparative HPLC to give H186 (12.13 mg, purity 94.05%), yield: 6.62%. MS m/z (ESI): 850 [M+H]⁺. ¹H NMR (400 MHz, DMSO-d₆) δ 10.82 (s, 1H), 8.63 (s, 1H), 8.37 (s, 1H), 7.44 (dd, J = 26.0, 10.2 Hz, 2H), 7.13 (d, J = 7.8 Hz, 1H), 7.07 (d, J = 8.6 Hz, 2H), 6.96 - 6.88 (m, 1H), 6.71 - 6.62 (m, 1H), 4.78 - 4.61 (m, 2H), 4.52 (t, J = 8.7 Hz, 2H), 4.28 - 4.19 (m, 1H), 3.92 (s, 3H), 3.41 (d, J = 10.0 Hz, 2H), 3.29 (s, 1H), 2.95 (s, 2H), 2.65 (dd, J = 30.4, 19.6 Hz, 5H), 2.40 - 1.56 (m, 17H), 1.32 (d, J = 10.2 Hz, 2H).

### Example 187 Preparation of Compound H187

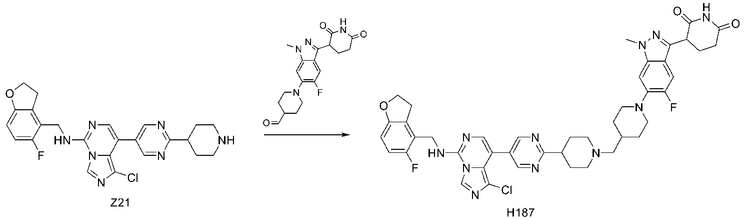

Compound H187 was prepared referring to the method of Example 71. MS m/z (ESI): 836.3 [M+H]⁺. ¹H NMR (400 MHz, DMSO-d₆) δ 10.81 (s, 1H), 8.80 (s, 2H), 8.68 (s, 1H), 8.55 (t, J = 5.2 Hz, 1H), 7.41 (d, J = 12.6 Hz, 1H), 7.29 (s, 1H), 7.06 (d, J = 7.1 Hz, 1H), 6.97-6.85 (m, 1H), 6.67 (dd, J = 8.7, 3.9 Hz, 1H), 4.70 (d, J = 4.9 Hz, 2H), 4.52 (t, J = 8.7 Hz, 2H), 4.24 (dd, J = 9.6, 5.1 Hz, 1H), 3.91 (s, 3H), 3.41 (d, J = 11.4 Hz, 2H), 3.26 (s, 1H), 2.95 (d, J = 10.4 Hz, 2H), 2.82 (d, J = 11.6 Hz, 1H), 2.72-2.53 (m, 4H), 2.39-2.17 (m, 3H), 2.15-1.93 (m, 5H), 1.89-1.66 (m, 5H), 1.41-1.14 (m, 3H).

### Example 188 Preparation of Compound H188

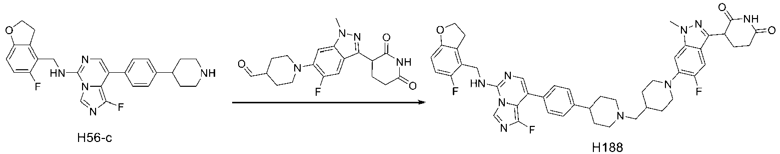

H56-c (60 mg, 130.0 µmol) was dissolved in DCM (3 mL). Compound 2 (62.94 mg, 169.0 µmol) was added, and the mixture was stirred at room temperature for 2 h. NaBH(OAc)₃ (82.66 mg, 390.03 µmol) was added, and the mixture was stirred at room temperature overnight. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 5%-95% acetonitrile change) to give H188 (13.39 mg, purity 98.9%), yield: 12.34%. MS m/z (ESI): 819.4 [M+H]⁺. ¹H NMR (400 MHz, DMSO-d₆) δ 10.82 (s, 1H), 8.36 (s, 1H), 8.30- 8.23 (m, 1H), 7.48-7.36 (m, 3H), 7.30 (d, J = 8.0 Hz, 2H), 7.16 (s, 1H), 7.07 (d, J = 7.1 Hz, 1H), 6.96- 6.87 (m, 1H), 6.67 (dd, J = 8.7, 3.8 Hz, 1H), 4.68 (d, J = 4.9 Hz, 2H), 4.52 (t, J = 8.7 Hz, 2H), 4.24 (dd, J = 9.6, 5.0 Hz, 1H), 3.92 (s, 3H), 3.41 (d, J = 11.3 Hz, 2H), 3.26 (s, 1H), 2.96 (d, J = 10.5 Hz, 2H), 2.75-2.51 (m, 5H), 2.39-2.17 (m, 3H), 2.15-1.95 (m, 3H), 1.92-1.58 (m, 8H), 1.37-1.27 (m, 2H).

### Example 189 Preparation of Compound H189

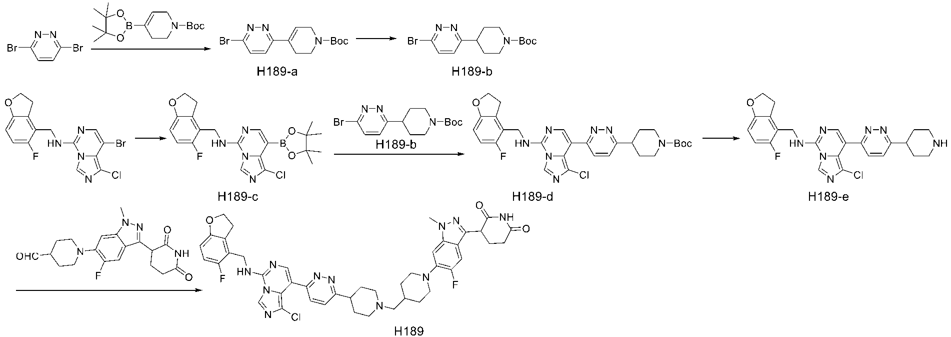

Step 1: tert-Butyl 4-(4,4,5,5-tetramethyl-1,3,2-dioxaborolan-2-yl)-3,6-dihydro-2H-pyridine-1-carboxylate (5.0 g, 16.17 mmol) and 3,6-dibromopyridazine (5.77 g, 24.26 mmol) were dissolved in 1,4-dioxane (75 mL) and water (7.5 mL). Under argon protection, Pd(dppf)Cl₂ (1.18 g, 1.62 mmol) and K₂CO₃ (4.47 g, 32.34 mmol) were added, and the temperature was raised to 95°C and stirred for 3 hours. After the reaction was complete, the reaction solution was concentrated and purified by silica gel column chromatography (EA:PE = 0-50%) to give H189-a (3.5 g, yellow solid), yield: 63.62%. MS m/z (ESI): 341 [M+H]⁺.

Step 2: H189-a (1.7 g, 5.00 mmol) was dissolved in methanol (20 mL) and tetrahydrofuran (20 mL). Chlorotris(triphenylphosphine)rhodium(I) (924.63 mg, 999.37 µmol) was added with stirring at room temperature, and the mixture was stirred at room temperature overnight under hydrogen protection. After the reaction was complete, the reaction solution was concentrated and purified by silica gel column chromatography (EA:PE = 0-50%) to give H189-b (1.2 g, yellow solid), yield: 70.17%. MS m/z (ESI): 343 [M+H]⁺.

Step 3: 8-Bromo-1-chloro-N-[(5-fluoro-2,3-dihydrobenzofuran-4-yl)methyl]imidazo[1,5-c] pyrimidin-5-amine (200 mg, 502.98 µmol) and 4,4,5,5-tetramethyl-2-(4,4,5,5-tetramethyl-1,3,2-dioxaborolan-2-yl)-1,3,2-dioxaborolane (383.18 mg, 1.51 mmol) were dissolved in 1,4-dioxane (5 mL). Under argon protection, Pd(dppf)Cl₂ (184.02 mg, 251.49 µmol) and KOAc (148.09 mg, 1.51 mmol) were added, and the temperature was raised to 110°C and stirred for 3 hours. After the reaction was complete, the reaction solution was concentrated and purified by silica gel column chromatography (EA:PE = 0-100%) to give H189-c (60 mg, yellow solid), yield: 26.82%. MS m/z (ESI): 445 [M+H]⁺.

Step 4: H189-c (100 mg, 224.87 µmol) and H189-b (115.44 mg, 337.31 µmol) were dissolved in dioxane (5 mL) and water (0.5 mL). Under argon protection, Pd(dppf)Cl₂ (164.54 mg, 224.87 µmol) and K₂CO₃ (31.08 mg, 224.87 µmol) were added sequentially, and the temperature was raised to 100°C and stirred for 3 hours. After the reaction was complete, the reaction solution was concentrated and purified by silica gel column chromatography (EA:PE = 0-100%) to give H189-d (30 mg, yellow solid), yield: 23.00%. MS m/z (ESI): 581 [M+H]⁺.

Step 5: H189-d (30 mg, 51.72 µmol) was dissolved in methanol (3 mL) and dichloromethane (5 mL). A solution of hydrogen chloride in dioxane (4.0 mol/L, 3 mL) was added with stirring at room temperature, and the mixture was stirred at room temperature for 2 hours. After the reaction was complete, the reaction solution was concentrated, basified with saturated aqueous sodium carbonate solution, extracted with ethyl acetate, the organic phase was dried over anhydrous sodium sulfate, and concentrated to give H189-e (20 mg, yellow solid), yield: 81%. MS m/z (ESI): 480 [M+H]⁺.

Step 6: H189-e (97.36 mg, 202.85 µmol) and 1-(3-(2,6-dioxopiperidin-3-yl)-5-fluoro-1-methyl-1H-indazol-6-yl)piperidine-4-carbaldehyde (75.54 mg, 202.85 µmol) were dissolved in dichloromethane (10 mL). NaBH(OAc)₃ (42.99 mg, 202.85 µmol) was added with stirring at room temperature, and the mixture was stirred at room temperature overnight. After the reaction was complete, the reaction solution was concentrated and purified by preparative HPLC to give H189 (8.78 mg, purity 100%), yield: 5.18%. MS m/z (ESI): 836.3 [M+H]⁺. ¹H NMR (400 MHz, DMSO-d₆) δ 10.83 (s, 1H), 8.70 (s, 1H), 8.62 (s, 1H), 7.74 (d, J = 8.7 Hz, 1H), 7.68 (d, J = 8.7 Hz, 1H), 7.48-7.36 (m, 2H), 7.07 (d, J = 6.9 Hz, 1H), 6.97-6.87 (m, 1H), 6.68 (dd, J = 8.5, 3.9 Hz, 1H), 4.73 (d, J = 4.9 Hz, 2H), 4.52 (t, J = 8.8 Hz, 2H), 4.24 (dd, J = 9.7, 5.1 Hz, 1H), 3.92 (s, 3H), 3.42 (d, J = 11.7 Hz, 2H), 3.27 (s, 1H), 3.10-2.80 (m, 3H), 2.67-2.50 (m, 4H), 2.39-2.18 (m, 3H), 1.60-3.13 (m, 10H), 1.40-1.15 (m, 3H).

### Example 190 Preparation of Compound H190

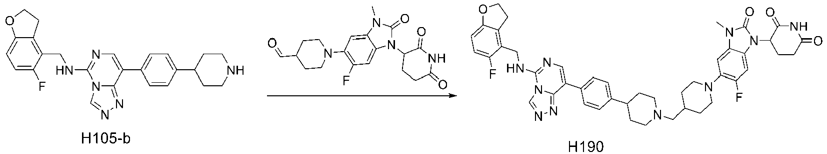

Referring to the preparation method of Example 109, following the above synthetic route, H190 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 817.4 [M+H]⁺. ¹H NMR (400 MHz, DMSO-d₆) δ 11.04 (s, 1H), 9.47 (s, 1H), 8.65 (t, J = 4.9 Hz, 1H), 8.15 (s, 1H), 8.04-7.91 (m, 3H), 7.32 (d, J = 8.4 Hz, 2H), 7.10 (d, J = 12.0 Hz, 1H), 6.92 (dd, J = 12.1, 8.7 Hz, 2H), 6.68 (dd, J = 8.6, 3.9 Hz, 1H), 5.28 (dd, J = 12.8, 5.4 Hz, 1H), 4.70 (d, J = 4.6 Hz, 2H), 4.52 (t, J = 8.7 Hz, 2H), 3.34-3.21 (m, 6H), 3.02 (d, J = 10.8 Hz, 2H), 2.89-2.78 (m, 1H), 2.77-2.51 (m, 5H), 2.29 (d, J = 6.7 Hz, 2H), 2.09 (t, J = 10.8 Hz, 2H), 2.00-1.90 (m, 1H), 1.75 (dt, J = 21.3, 10.2 Hz, 7H), 1.3-1.23 (m, 2H).

### Example 191 Preparation of Compound H191

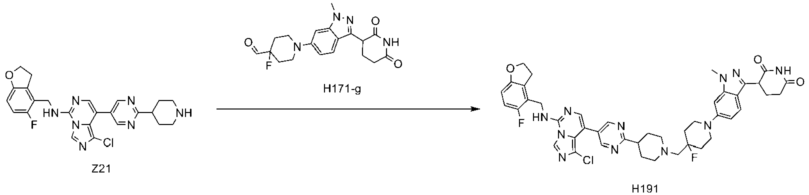

Z21 (40 mg, 83.34 µmol) and H171-g (31.04 mg, 83.34 µmol) were dissolved in 1,2-dichloroethane (10 mL) and tetraisopropyl titanate (47.38 mg, 166.69 µmol). The mixture was stirred in an oil bath at 60°C for 18 hours, then cooled to room temperature, and NaBH(OAc)₃ (52.99 mg, 250.03 µmol) was added. The reaction was stirred at room temperature for 2 hours. The reaction solution was then concentrated and purified by preparation (preparative column: 21.2×250 mm C18 column; system: 10 mM NH₄HCO₃/H₂O/acetonitrile; wavelength: 254/214 nm; gradient: 0%-60% acetonitrile change) to successfully give white solid product H191 (9.05 mg, 12.98% yield). MS m/z (ESI): 836.3 [M+H]⁺. ¹H NMR (400 MHz, DMSO-d₆) δ 10.82 (s, 1H), 8.80 (s, 2H), 8.69 (s, 1H), 8.60-8.53 (m, 1H), 7.48 (d, J = 8.9 Hz, 1H), 7.30 (s, 1H), 6.91 (dd, J = 14.2, 5.5 Hz, 3H), 6.68 (dd, J = 8.6, 3.8 Hz, 1H), 4.71 (d, J = 4.9 Hz, 2H), 4.53 (t, J = 8.7 Hz, 2H), 4.24 (dd, J = 9.2, 5.1 Hz, 1H), 3.88 (s, 3H), 3.57 (d, J = 12.5 Hz, 2H), 3.28 (d, J = 8.9 Hz, 4H), 3.13-2.97 (m, 4H), 2.83 (dd, J = 17.5, 8.3 Hz, 1H), 2.65-2.52 (m, 3H), 2.34-2.21 (m, 3H), 2.18-2.10 (m, 1H), 2.01-1.73 (m, 7H).

### Example 192 Preparation of Compound H192

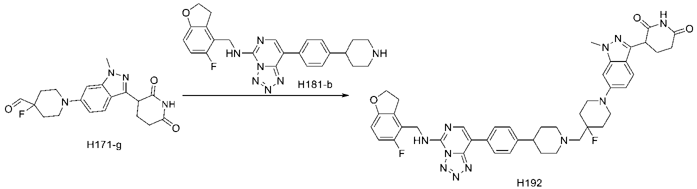

Compound H192 was prepared referring to the method of Example 71. MS m/z (ESI): 802.3 [M+H]⁺. ¹H NMR (400 MHz, DMSO-d₆) δ 10.84 (s, 1H), 9.52 (s, 1H), 8.40 (s, 1H), 8.00 (d, J = 8.3 Hz, 2H), 7.50 (d, J = 8.9 Hz, 1H), 7.39 (d, J = 8.4 Hz, 2H), 6.92 (dd, J = 20.3, 9.6 Hz, 3H), 6.67 (dd, J = 8.6, 3.9 Hz, 1H), 4.78 (s, 2H), 4.52 (t, J = 8.7 Hz, 2H), 4.26 (dd, J = 9.2, 5.2 Hz, 1H), 3.90 (s, 3H), 3.60 (d, J = 12.8 Hz, 2H), 3.34 (d, J = 8.8 Hz, 2H), 3.08 (dd, J = 23.6, 11.3 Hz, 4H), 2.69 - 2.52 (m, 4H), 2.38 - 2.08 (m, 5H), 2.03 - 1.87 (m, 3H), 1.84 - 1.67 (m, 5H).

### Example 193 Preparation of Compound H193

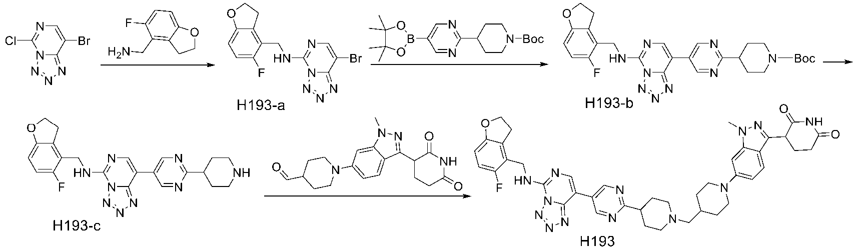

Step 1: 8-Bromo-5-chlorotetrazolo[1,5-c]pyrimidine (250 mg, 1.07 mmol) and (5-fluoro-2,3-dihydrobenzofuran-4-yl)methanamine (356.55 mg, 2.13 mmol) were dissolved in 1,4-dioxane (5 mL). Et₃N (539.52 mg, 5.33 mmol) was then added, and the mixture was stirred at 80°C for 0.5 hour. After the reaction was complete, the reaction solution was concentrated and purified by CombiFlash (8 g, 0-40% EA/PE) to give H192-a (355 mg, white solid), yield: 91.17%. MS m/z (ESI): 365.0 [M+H]⁺.

Steps 2-4: Referring to the preparation method of Example 49, following the above synthetic route, H193 was prepared. The purification conditions for preparative HPLC were: preparative column: 21.2×250 mm C18 column; system: 10 mM NH₄HCO₃/H₂O/acetonitrile; wavelength: 254/214 nm; gradient: 0%-60% acetonitrile change. MS m/z (ESI): 786.4 [M+H]⁺. ¹H NMR (400 MHz, DMSO-d₆) δ 10.82 (s, 1H), 9.77 (s, 1H), 9.36 (s, 2H), 8.57 (s, 1H), 8.18 (s, 1H), 7.45 (d, J = 9.0 Hz, 1H), 6.89 (dd, J = 13.1, 5.8 Hz, 2H), 6.80 (s, 1H), 6.65 (dd, J = 8.6, 3.8 Hz, 1H), 4.77 (s, 2H), 4.50 (t, J = 8.7 Hz, 2H), 4.22 (dd, J = 9.1, 5.1 Hz, 1H), 3.88 (d, J = 18.1 Hz, 3H), 3.77 (d, J = 12.4 Hz, 2H), 3.31 (s, 2H), 2.95 (d, J = 10.9 Hz, 2H), 2.85 (t, J = 11.5 Hz, 1H), 2.72 (t, J = 11.6 Hz, 2H), 2.64 - 2.54 (m, 2H), 2.33 - 2.24 (m, 1H), 2.21 (d, J = 6.8 Hz, 2H), 2.13 (dd, J = 13.1, 5.6 Hz, 1H), 2.04 (t, J = 10.8 Hz, 2H), 1.95 (d, J = 11.5 Hz, 2H), 1.88 - 1.77 (m, 3H), 1.73 (s, 1H), 1.25 (dd, J = 21.3, 11.0 Hz, 2H).

### Example 194 to Example 201

Compounds H194 to H201 were prepared according to by selecting the corresponding aldehyde and amine and following the method of Example 71.

| | |
|---|---|
| 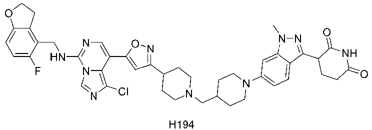 | MS m/z (ESI): 807.3[M+H]⁺. |
| | ¹H NMR (400 MHz, DMSO-*d*₆) δ 10.86 (s, 1H), 8.83 (s, 1H), 8.74 (s, 1H), 7.59 (s, 1H), 7.48 (d, *J =* 8.9 Hz, 1H), 6.94 (dd, *J =* 20.3, 10.5 Hz, 2H), 6.83 (s, 1H), 6.76 - 6.67 (m, 2H), 4.74 (s, 2H), 4.54 (t, *J =* 8.8 Hz, 2H), 4.25 (dd, *J* = 9.2, 5.0 Hz, 1H), 3.89 (s, 3H), 3.79 (d, *J =* 11.9 Hz, 2H), 3.29 (t, *J =* 9.0 Hz, 2H), 2.93 (d, *J =* 10.6 Hz, 2H), 2.73 (t, *J= 11.1* Hz, 3H), 2.62 (dd, *J =* 10.6, 6.0 Hz, 2H), 2.31 (dd, *J* = 10.1, 7.1 Hz, 1H), 2.22 - 2.17 (m, 2H), 2.03 (t, *J =* 10.1 Hz, 2H), 1.92 (d, *J =* 13.1 Hz, 2H), 1.82 (d, *J =* 13.3 Hz, 2H), 1.77 - 1.67 (m, 3H), 1.25 (dd, *J =* 22.9, 9.4 Hz, 3H). |
| 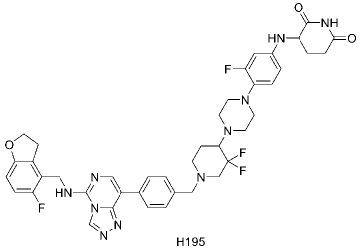 | MS m/z (ESI): 799[M+H]⁺. |
| | ¹H NMR (400 MHz, DMSO-d₆) δ 10.73 (s, 1H), 9.47 (s, 1H), 8.66 (t, *J =* 5.1 Hz, 1H), 8.14-7.97 (m, 3H), 7.37 (d, *J =* 8.1 Hz, 2H), 6.93 (dd, *J =* 10.3, 8.7 Hz, 1H), 6.79 (t, *J* = 9.3 Hz, 1H), 6.69 (dd, *J =* 8.7, 3.9 Hz, 1H), 6.52-6.34 (m, 2H), 4.71 (d, *J =* 4.9 Hz, 2H), 4.53 (t, *J =* 8.7 Hz, 2H), 4.28-4.14 (m, 1H), 3.59 (d, *J* = 4.6 Hz, 2H), 3.05 - 2.87 (m, 3H), 2.80 (s, 9H), 2.67 (d, *J =* 5.4 Hz, 1H), 2.56 (d, *J =* 4.4 Hz, 1H), 2.34-2.17 (m, 1H), 2.17-1.99 (m, 2H), 1.81 (dd, J = 23.0, 10.5 Hz, 3H), 1.22 (s, 2H). |
| 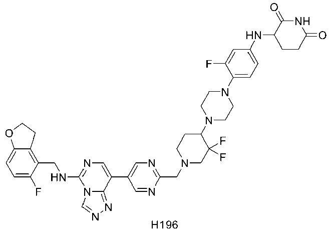 | MS m/z (ESI): 801.3[M+H]⁺. |
| 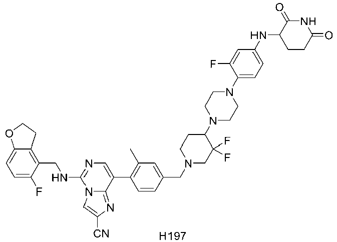 | MS m/z (ESI): 837.4[M+H]⁺. |
| | ¹H NMR (400 MHz, DMSO-d₆) δ 10.73 (s, 1H), 8.90 (s, 1H), 8.44 (s, 1H), 7.68 (s, 1H), 7.29 - 7.11 (m, 3H), 6.99 - 6.87 (m, 1H), 6.80 (t, *J =* 9.2 Hz, 1H), 6.69 (dd, *J =* 8.6, 3.7 Hz, 1H), 6.48 (d, *J =* 14.8 Hz, 1H), 6.39 (d, *J =* 8.6 Hz, 1H), 5.76 (d, *J =* 7.4 Hz, 1H), 4.70 (s, 2H), 4.53 (t, *J =* 8.7 Hz, 2H), 4.29 - 4.17 (m, 1H), 3.56 (s, 2H), 3.33 (s, 6H), 3.07 - 2.96 (m, 1H), 2.96 - 2.87 (m, 1H), 2.81 (s, 6H), 2.73 - 2.64 (m, 1H), 2.59 - 2.50 (m, 1H), 2.28 (dd, *J* = 29.4, 11.0 Hz, 1H), 2.13 (s, 3H), 2.06 (dd,*J* = 13.6, 5.3 Hz, 1H), 1.89 - 1.69 (m, 3H). |
| 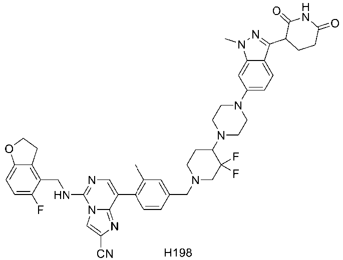 | MS m/z (ESI): 858.4[M+H]⁺. |
| | ¹H NMR (400 MHz, DMSO-*d*₆) δ 10.81 (s, 1H), 8.89 (s, 1H), 8.41 (t, *J =* 4.7 Hz, 1H), 8.12 (s, 1H), 7.68 (s, 1H), 7.47 (d, *J* = 9.0 Hz, 1H), 7.28-7.20 (m, 2H), 7.17 (d, *J =* 7.6 Hz, 1H), 6.98 - 6.84 (m, 2H), 6.81 (s, 1H), 6.69 (dd, *J* = 8.6, 3.8 Hz, 1H), 6.48 (s, 1H), 4.70 (d, *J =* 4.6 Hz, 2H), 4.53 (t, *J* = 8.7 Hz, 2H), 4.23 (dd, *J* = 9.0, 5.1 Hz, 1H), 3.86 (s, 3H), 3.57 (s, 2H), 3.42-3.30 (m, 3H), 3.19-3.15 (m, 3H), 3.04-2.99 (m, 1H), 2.97-2.78 (m, 5H), 2.65-2.54 (m, 2H), 2.37-2.21 (m, 2H), 2.20-2.08 (m, 4H), 1.83-1.73 (m, 2H). |
| 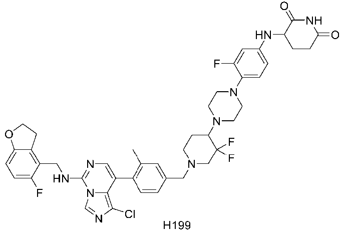 | MS m/z (ESI): 846.3[M+H]⁺. |
| | ¹H NMR (400 MHz, DMSO-d₆) δ 10.73 (s, 1H), 8.62 (s, 1H), 8.30 (dd, *J* = 13.8, 8.8 Hz, 1H), 7.18 (s, 1H), 7.16-7.09 (m, 2H), 7.04 (s, 1H), 6.97 - 6.87 (m, 1H), 6.84-6.75 (m, 1H), 6.68 (dd, *J* = 8.6, 3.9 Hz, 1H), 6.48 (dd, *J* = 15.0, 2.4 Hz, 1H), 6.39 (d, *J* = 8.7 Hz, 1H), 5.76 (d, *J* = 7.5 Hz, 1H), 4.68 (qd, *J* = 14.2, 4.6 Hz, 2H), 4.53 (t, *J* = 8.7 Hz, 2H), 4.27-4.18 (m, 1H), 3.56 (q, *J* = 13.3 Hz, 2H), 3.31 (d, *J* = 8.5 Hz, 5H), 3.02-2.87 (m, 2H), 2.82 (d, *J* = 12.3 Hz, 7H), 2.72-2.63 (m, 1H), 2.54 (dd, *J =* 13.3, 4.0 Hz, 1H), 2.25 (dd, *J =* 29.4, 11.8 Hz, 1H), 2.12 (s, 1H), 2.07 (d, *J =* 11.2 Hz, 3H), 1.89-1.70 (m, 3H). |
| 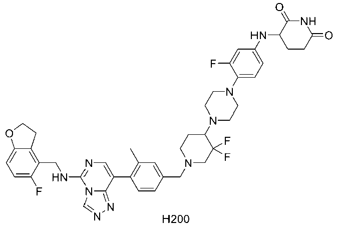 | MS m/z (ESI):813.3[M+H]⁺ |
| | ¹H NMR (400 MHz, DMSO-d₆) δ 10.74 (s, 1H), 9.44 (s, 1H), 8.59 (t, *J* = 5.2 Hz, 1H), 7.55 (s, 1H), 7.28 (d, *J=* 7.6 Hz, 1H), 7.22 (d, *J* = 1.6 Hz, 1H), 7.17 (dd, *J* = 7.6, 1.6 Hz, 1H), 6.94 (dd, *J =* 10.0, 8.4 Hz, 1H), 6.86-6.76 (m, 1H), 6.70 (dd, *J* = 8.4, 4.0 Hz, 1H), 6.48 (dd, *J =* 15.2, 2.8 Hz, 1H), 6.39 (dd, *J =* 8.8, 2.4 Hz, 1H), 5.76 (d, *J =* 7.6 Hz, 1H), 4.69 (d, *J =* 4.8 Hz, 2H), 4.54 (t, *J =* 8.8 Hz, 2H), 4.26-4.18 (m, 1H), 3.57 (s, 2H), 3.32 (d, *J=* 9.2 Hz, 2H), 3.00 (q, *J* = 7.2 Hz, 1H), 2.92 (d, *J* = 10.4 Hz, 1H), 2.88-2.75 (m, 9H), 2.73-2.65 (m, 1H), 2.58-2.51 (m, 1H), 2.35 - 2.21 (m, 1H), 2.18 (s, 3H), 2.15-2.01 (m, 2H), 1.87-1.74 (m, 3H). |
| 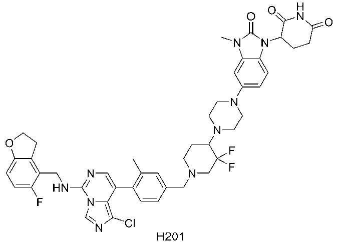 | MS m/z (ESI):442.3 [M/2+H]⁺. |
| | ¹H NMR (400 MHz, DMSO-d₆) δ 11.03 (s, 1H), 8.64 (s, 1H), 8.33 (t, *J* = 4.9 Hz, 1H), 7.17 (dd, *J* = 18.2, 8.9 Hz, 3H), 7.06 (s, 1H), 6.99 - 6.89 (m, 2H), 6.83 (d, *J =* 2.0 Hz, 1H), 6.71 (dd, *J =* 8.6, 3.9 Hz, 1H), 6.62 (dd, *J =* 8.7, 2.0 Hz, 1H), 5.29 (dd, *J* = 12.8, 5.3 Hz, 1H), 4.70 (qd, *J =* 14.3, 4.4 Hz, 2H), 4.55 (t, *J* = 8.7 Hz, 2H), 3.59 (q, *J =* 13.5 Hz, 2H), 3.33 (d, *J =* 13.2 Hz, 6H), 3.16 - 2.78 (m, 11H), 2.73-2.59 (m, 2H), 2.38 - 2.05 (m, 5H), 2.03 - 1.94 (m, 1H), 1.93 - 1.75 (m, 2H). |

### Example 202 Preparation of Compound H202

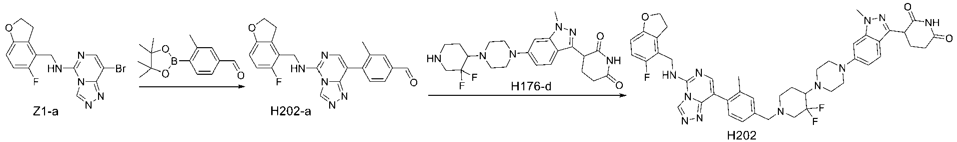

Step 1: Z1-a (140 mg, 384.43 µmol) and 3-methyl-4-(4,4,5,5-tetramethyl-1,3,2-dioxaborolan-2-yl)benzaldehyde (113.54 mg, 461.32 µmol) were dissolved in dioxane (10 mL) and water (1 mL). Pd(dppf)Cl₂ (28.13 mg, 38.44 µmol) and K₂CO₃ (106.26 mg, 768.87 µmol) were added, and the reaction solution was stirred at 100°C for 3 hours. After the reaction was complete, the reaction solution was poured into water, extracted with ethyl acetate (30 mL×3). The combined organic phases were dried over anhydrous sodium sulfate, concentrated, and purified by CombiFlash (0-100% EA/PE) to give H202-a (100 mg, yellow solid), yield: 64.48%. MS m/z (ESI): 404.1 [M+H]⁺.

Step 2: H202-a (25 mg, 61.97 µmol) and H176-d (27.67 mg, 61.97 µmol) were dissolved in DCM (10 mL). NaBH(OAc)₃ (26.27 mg, 123.94 µmol) was added, and the reaction solution was stirred at room temperature overnight. After the reaction was complete, the reaction solution was concentrated and purified by preparative HPLC (preparative column: 21.2×250 mm C18 column; system: 10 mM NH₄HCO₃/H₂O/acetonitrile; wavelength: 254/214 nm; gradient: 0%-60% acetonitrile change) to give H202 (11.63 mg), yield: 21.53%. MS m/z (ESI): 418.7 [M/2+H]⁺. ¹H NMR (400 MHz, DMSO-d₆) δ 10.83 (s, 1H), 9.46 (s, 1H), 8.61 (t, J = 5.0 Hz, 1H), 7.57 (s, 1H), 7.49 (d, J = 8.9 Hz, 1H), 7.33 - 7.17 (m, 3H), 7.00 - 6.88 (m, 2H), 6.83 (s, 1H), 6.72 (dd, J = 8.6, 3.9 Hz, 1H), 4.72 (d, J = 4.8 Hz, 2H), 4.56 (t, J = 8.7 Hz, 2H), 4.25 (dd, J = 9.1, 5.1 Hz, 1H), 3.89 (s, 3H), 3.60 (s, 2H), 3.35 (d, J = 8.7 Hz, 2H), 3.25-3.11 (m, 4H), 3.09-2.84 (m, 7H), 2.69-2.56 (m, 2H), 2.38-2.25 (m, 2H), 2.24-2.10 (m, 5H), 1.94-1.75 (m, 2H).

### Example 203 Preparation of Compound H203

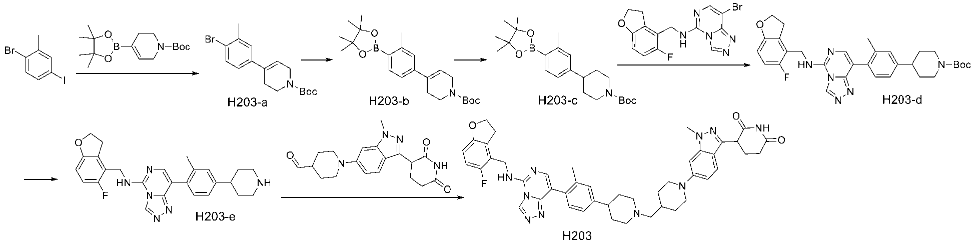

Step 1: 1-Bromo-4-iodo-2-methylbenzene (5 g, 16.84 mmol) and tert-butyl 4-(4,4,5,5-tetramethyl-1,3,2-dioxaborolan-2-yl)-3,6-dihydropyridine-1(2H)-carboxylate (5.21 g, 16.84 mmol) were dissolved in water (8 mL) and 1,4-dioxane (50 mL). Under argon protection, Pd(dppf)Cl₂ (1.85 g, 2.53 mmol) and K₂CO₃ (4.65 g, 33.68 mmol) were added. The temperature was raised to 80°C and stirred for 16 hours. After the reaction was complete, the reaction solution was concentrated and purified by combi-flash (PE:EA = 2:1) to give H203-a (5 g, yellow solid), yield: 84.29%. MS m/z (ESI): 299.2 [M-56+H]⁺.

Step 2: H203-a (3 g, 8.52 mmol) was dissolved in 1,4-dioxane (5 mL). 4,4,5,5-Tetramethyl-2-(4,4,5,5-tetramethyl-1,3,2-dioxaborolan-2-yl)-1,3,2-dioxaborolane (4.54 g, 17.88 mmol), Pd(dppf)Cl₂ (933.81 mg, 1.28 mmol), and potassium acetate (2.09 g, 21.29 mmol) were added. The mixture was purged with argon three times, heated to 95°C, and stirred overnight. After the reaction was complete, the reaction solution was filtered, and the filtrate was concentrated and purified by combiflash (PE:EA = 1:1) to give H203-b (3 g, yellow solid), yield: 88.21%. MS m/z (ESI): 344.2 [M-56+H]⁺.

Step 3: H203-b (3 g, 7.51 mmol) was dissolved in MeOH (40 mL). Palladium carbon (912.44 mg, 7.51 mmol) was added, the mixture was purged with hydrogen three times, and reacted at room temperature for 3 h. After the reaction was complete, the reaction solution was filtered, and the filtrate was concentrated to give H203-c (crude). MS m/z (ESI): 346.2 [M-56+H]⁺.

Steps 4-6: Referring to the preparation method of Example 105, following the above synthetic route, H203 was prepared. The purification conditions for preparative HPLC were: preparative column: 21.2×250 mm C18 column; system: 10 mM NH₄HCO₃/H₂O/acetonitrile; wavelength: 254/214 nm; gradient: 5%-95% acetonitrile change. MS m/z (ESI): 797.4 [M+H]⁺. ¹H NMR (400 MHz, DMSO-d₆) δ 10.81 (s, 1H), 9.42 (s, 1H), 8.56 (t, J = 5.2 Hz, 1H), 7.52 (s, 1H), 7.46 (d, J = 9.0 Hz, 1H), 7.26-7.16 (m, 2H), 7.12 (d, J = 7.8 Hz, 1H), 6.98-6.85 (m, 2H), 6.81 (s, 1H), 6.70 (dd, J = 8.7, 3.8 Hz, 1H), 4.69 (d, J = 4.8 Hz, 2H), 4.53 (t, J = 8.7 Hz, 2H), 4.22 (dd, J = 9.0, 5.1 Hz, 1H), 3.86 (s, 3H), 3.77 (d, J = 12.1 Hz, 2H), 2.97 (s, 2H), 2.73 (t, J = 11.9 Hz, 2H), 2.63-2.56 (m, 2H), 2.15 (s, 8H), 1.99 (s, 2H), 1.78 (dd, J = 33.2, 17.5 Hz, 8H), 1.33-1.18 (m, 3H).

### Example 204 to Example 221

Compounds H204 to H221 were prepared using Z54, Z55, H136-d, Z56, H136-d, Z57, Z58, H157-c, H157-c, H105-b, H167-b, Z59, Z61, Z51, Z62, Z63, Z64, Z65 as starting materials, respectively, with the corresponding aldehydes, following the method of Example 71.

| Compound number and structure | MS and/or ¹H NMR |
|---|---|
| 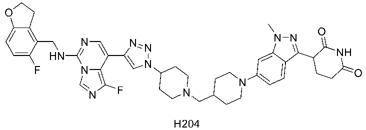 | MS m/z (ESI): 791 [M+H]⁺. |
| 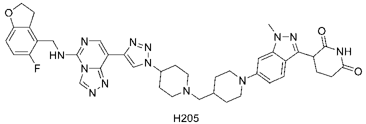 | MS m/z (ESI): 774.4 [M+H]⁺. |
| | ¹H NMR (400 MHz, DMSO-*d*₆) δ10.82 (s, 1H), 9.50 (s, 1H), 8.73 (t, *J =* 5.0 Hz, 1H), 8.68 (s, 1H), 8.35 (s, 1H), 7.46 (d, *J =* 9.0 Hz, 1H), 6.99 - 6.85 (m, 2H), 6.82 (s, 1H), 6.70 (dd, *J =* 8.6, 3.8 Hz, 1H), 4.72 (d, *J =* 4.6 Hz, 2H), 4.61 (s, 1H), 4.53 (t, *J =* 8.7 Hz, 2H), 4.23 (dd, *J* = 9.1, 5.2 Hz, 1H), 3.87 (s, 3H), 3.78 (d, *J* = 12.0 Hz, 2H), 3.32 (s, 1H), 3.28 (s, 2H), 3.04-2.90 (m, 2H), 2.73 (t, *J* = 11.5 Hz, 2H), 2.66-2.54 (m, 2H), 2.34-2.20 (m, 3H), 2.19-1.99 (m, 6H), 1.83 (d, *J =* 10.7 Hz, 2H), 1.72 (s, 1H), 1.30-1.21 (m, 2H). |
| 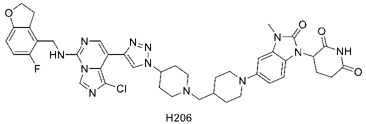 | MS m/z (ESI): 823.3[M+H]⁺. |
| | ¹H NMR (400 MHz, DMSO-d₆) δ 11.04 (s, 1H), 8.66 (s, 1H), 8.46 (t, *J* = 5.0 Hz, 1H), 8.36 (s, 1H), 7.36 (s, 1H), 6.96-6.86 (m, 2H), 6.81 (d, *J =* 2.0 Hz, 1H), 6.68 (dd, *J =* 8.7, 3.9 Hz, 1H), 6.62 (dd, *J =* 8.7, 1.9 Hz, 1H), 5.27 (dd, *J =* 12.8, 5.3 Hz, 1H), 4.71 (d, *J =* 4.8 Hz, 2H), 4.52 (t, *J* = 8.7 Hz, 3H), 3.57 (d, *J =* 11.9 Hz, 2H), 3.30 - 3.23 (m, 5H), 2.96 (d, *J* = 10.0 Hz, 2H), 2.88 (dd,*J* = 17.5, 12.2 Hz, 1H), 2.65 (dt, *J* = 24.8, 11.2 Hz, 4H), 2.22 (d, *J* = 7.0 Hz, 2H), 2.17-1.89 (m, 7H), 1.80 (d, *J* = 12.2 Hz, 2H), 1.64 (s, 1H), 1.33-1.16 (m, 2H). |
| 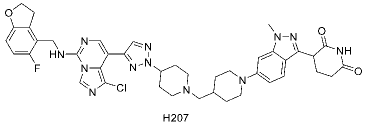 | MS m/z (ESI):807.3[M+H]⁺. |
| | ¹H NMR (400 MHz, DMSO-*d*₆) δ 10.83 (s, 1H), 8.68 (s, 1H), 8.53 (t, *J =* 4.9 Hz, 1H), 7.92 (s, 1H), 7.47 (d, *J =* 8.9 Hz, 1H), 7.38 (s, 1H), 6.93 (dd, *J =* 19.0, 9.3 Hz, 2H), 6.82 (s, 1H), 6.70 (dd, *J =* 8.6, 3.8 Hz, 1H), 4.73 (d, *J* = 4.8 Hz, 2H), 4.54 (t, *J =* 8.7 Hz, 3H), 4.24 (dd, *J* = 9.0, 5.1 Hz, 1H), 3.88 (s, 3H), 3.78 (d, *J =* 12.0 Hz, 2H), 3.28 (d, *J =* 8.8 Hz, 2H), 2.93 (d, *J =* 9.5 Hz, 2H), 2.74 (t, *J* = 11.4 Hz, 2H), 2.61 (dt, *J* = 15.2, 7.7 Hz, 2H), 2.33-2.21 (m, 3H), 2.19 -2.03 (m, 7H), 1.82 (d, *J =* 12.0 Hz, 2H), 1.71 (dt, *J* = 23.4, 11.8 Hz, 1H), 1.25 (dt, *J* = 12.7, 6.5 Hz, 2H). |
| 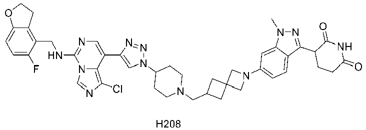 | MS m/z (ESI): 410.3[M/2+H]⁺. |
| | ¹H NMR (400 MHz, DMSO-*d*₆) δ 10.80 (s, 1H), 8.65 (s, 1H), 8.46 (t, J = 5.2 Hz, 1H), 8.35 (s, 1H), 7.44 (d, J = 8.6 Hz, 1H), 7.35 (s, 1H), 6.92 (dd, J = 10.3, 8.6 Hz, 1H), 6.68 (dd, J = 8.6, 3.9 Hz, 1H), 6.38-6.23 (m, 2H), 4.71 (d, J = 5.0 Hz, 2H), 4.52 (t, J = 8.7 Hz, 3H), 4.21 (dd, J = 9.1, 5.1 Hz, 1H), 3.91-3.75 (m, 7H), 3.26 (d, J = 8.7 Hz, 2H), 2.92 (d, J = 10.8 Hz, 2H), 2.59 (q, J = 5.7 Hz, 2H), 2.44 -2.22 (m, 6H), 2.18-1.96 (m, 7H), 1.89 (t, J = 6.7 Hz, 2H). |
| 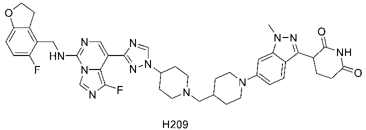 | MS m/z (ESI): 791.4 [M+H]⁺. |
| | ¹H NMR (400 MHz, DMSO-*d*₆) δ 10.83 (s, 1H), 8.59 (s, 1H), 8.45 (t, *J =* 5.1 Hz, 1H), 8.39 (s, 1H), 7.70 (s, 1H), 7.46 (d, *J =* 9.0 Hz, 1H), 6.98-6.85 (m, 2H), 6.81 (d, J = 1.9 Hz, 1H), 6.68 (dd, *J =* 8.6, 3.9 Hz, 1H), 4.71 (d, *J =* 4.9 Hz, 2H), 4.52 (t, *J =* 8.7 Hz, 2H), 4.35-4.19 (m, 2H), 3.87 (s, 3H), 3.77 (d, *J =* 12.1 Hz, 2H), 2.94 (d, *J* = 9.9 Hz, 3H), 2.72 (t, *J =* 11.9 Hz, 2H), 2.60 (q, *J =* 6.5, 5.6 Hz, 2H), 2.33-2.25 (m, 1H), 2.21 (d, *J =* 7.0 Hz, 2H), 2.19-2.16 (m, 8H), 1.81 (d, *J =* 12.7 Hz, 2H), 1.71 (s, 1H), 1.34-1.17 (m, 2H). |
| 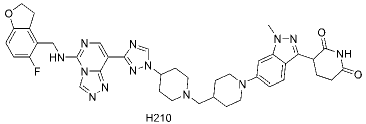 | MS m/z (ESI): 774.4[M+H]⁺ |
| 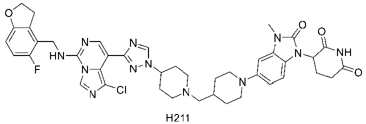 | MS m/z (ESI): 823[M+H]⁺. |
| | ¹H NMR (400 MHz, DMSO-*d*₆) δ 11.04 (s, 1H), 8.66 (s, 1H), 8.61 (s, 1H), 8.52 (s, 1H), 7.49 (s, 1H), 6.98-6.87 (m, 2H), 6.80 (d, *J* = 2.1 Hz, 1H), 6.68 (dd, *J* = 8.6, 3.9 Hz, 1H), 6.61 (dd, *J =* 8.6, 2.1 Hz, 1H), 5.27 (dd, *J* = 12.8, 5.3 Hz, 1H), 4.72 (d, *J* = 4.6 Hz, 2H), 4.52 (t, *J* = 8.8 Hz, 2H), 4.30 (s, 1H), 3.57 (d, *J* = 11.5 Hz, 2H), 3.30-3.22 (m, 5H), 2.91 (t, *J* = 14.4 Hz, 3H), 2.71-2.53 (m, 4H), 2.20 (d, *J* = 6.9 Hz, 2H), 2.11-1.90 (m, 7H), 1.79 (d, *J =* 11.0 Hz, 2H), 1.63 (s, 1H), 1.24 (d, *J =* 12.4 Hz, 2H). |
| 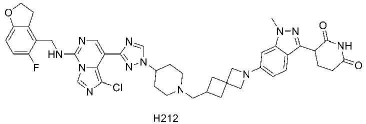 | MS m/z (ESI): 410.3[M/2+H]⁺_{∘} |
| | ¹H NMR (400 MHz, DMSO-*d*₆) δ 10.82 (s, 1H), 8.65 (s, 1H), 8.60 (s, 1H), 8.52 (t, *J =* 5.2 Hz, 1H), 7.49 (s, 1H), 7.44 (d, *J =* 8.7 Hz, 1H), 6.98-6.88 (m, 1H), 6.68 (dd, *J =* 8.7, 3.9 Hz, 1H), 6.32 (dd, *J =* 8.8, 1.8 Hz, 1H), 6.28 (s, 1H), 4.72 (d, *J =* 4.9 Hz, 2H), 4.52 (t, *J =* 8.7 Hz, 2H), 4.23 (ddd, *J =* 14.2, 9.6, 5.1 Hz, 2H), 3.82 (t, *J =* 25.0 Hz, 8H), 2.89 (d, *J =* 10.9 Hz, 2H), 2.58 (t, *J =* 6.0 Hz, 2H), 2.38 (s, 3H), 2.30 (s, 3H), 2.12-1.95 (m, 8H), 1.88 (t, *J =* 6.6 Hz, 2H). |
| 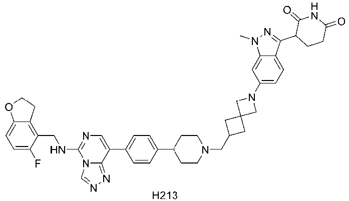 | MS m/z (ESI): 795.4[M+H]⁺. |
| | ¹H NMR (400 MHz, DMSO-*d*₆) δ 10.82 (s, 1H), 9.48 (s, 1H), 8.65 (t, *J =* 5.1 Hz, 1H), 8.00 (d, *J* = 8.0 Hz, 3H), 7.45 (d, *J =* 8.7 Hz, 1H), 7.33 (d, *J =* 8.2 Hz, 2H), 6.98-6.91 (m, 1H), 6.70 (dd, *J =* 8.7, 3.9 Hz, 1H), 6.32 (dd, *J =* 8.6, 1.8 Hz, 1H), 6.28 (d, *J =* 1.8 Hz, 1H), 4.71 (d, *J=* 4.9 Hz, 2H), 4.53 (t, *J =* 8.7 Hz, 2H), 4.22 (dd, *J =* 9.0, 5.1 Hz, 1H), 3.89 (s, 2H), 3.82 (s, 3H), 3.77 (s, 2H), 3.29-3.25 (m, 3H), 2.99-2.98 (m, 2H), 2.63-2.56 (m, 2H), 2.45-2.20 (m, 6H), 2.18-1.85 (m, 5H), 1.80-1.60 (m, 4H). |
| | |
| 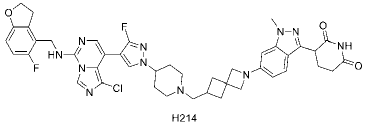 | MS m/z (ESI): 418.8[M/2+H] ⁺_{∘} |
| | ¹H NMR (400 MHz, DMSO-*d*₆) δ 10.82 (s, 1H), 8.61 (s, 1H), 7.44 (d, *J* = 8.6 Hz, 1H), 7.12 (s, 1H), 6.68 (dd, *J =* 8.7, 3.8 Hz, 2H), 6.51 (s, 1H), 6.32 (d, J = 9.6 Hz, 1H), 6.28 (s, 1H), 4.67 (d, *J =* 5.0 Hz, 3H), 4.53 (d, *J* = 8.6 Hz, 3H), 3.82 (t, *J =* 25.1 Hz, 8H), 2.88 (s, 5H), 2.65 (d, *J* = 2.0 Hz, 2H), 2.37 (s, 3H), 2.31 (s, 3H), 2.01-1.94 (m, 4H), 1.88 (d, *J =* 12.9 Hz, 4H). |
| 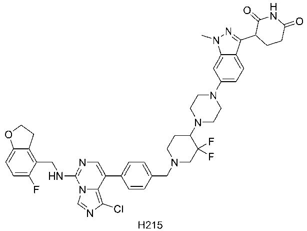 | MS m/z (ESI): 853.3[M+H]⁺_{∘} |
| | ¹H NMR (400 MHz, DMSO-*d*₆) δ 10.86 (s, 1H), 8.68 (s, 1H), 8.40 (t, *J =* 5.0 Hz, 1H), 7.49 (d, *J =* 9.0 Hz, 1H), 7.37 (q, *J =* 8.2 Hz, 4H), 7.18 (s, 1H), 6.93 (dd, *J =* 19.2, 9.2 Hz, 2H), 6.83 (s, 1H), 6.70 (dd, J = 8.6, 3.9 Hz, 1H), 4.71 (d, *J =* 4.9 Hz, 2H), 4.54 (t, *J* = 8.7 Hz, 2H), 4.25 (dd, *J =* 9.2, 5.1 Hz, 1H), 3.88 (s, 3H), 3.69 -3.55 (m, 2H), 3.30 (d, *J* = 8.7 Hz, 2H), 3.25-3.14 (m, 4H), 3.07-2.97 (m, 1H), 2.97-2.77 (m, 6H), 2.69-2.53 (m, 2H), 2.38-2.23 (m, 2H), 2.20-2.10 (m, 2H), 1.92-1.72 (m, 2H). |
| 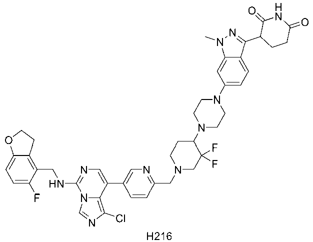 | MS m/z (ESI): 854.3[M+H]⁺_{∘} |
| | ¹H NMR (400 MHz, DMSO-*d*₆) δ 10.86 (s, 1H), 8.70 (s, 1H), 8.56 (d, *J =* 2.0 Hz, 1H), 8.52 (t, *J =* 5.3 Hz, 1H), 7.85 (dd, *J* = 8.0, 2.3 Hz, 1H), 7.49 (dd, *J* = 8.5, 3.4 Hz, 2H), 7.25 (s, 1H), 6.93 (dd, *J* = 19.1, 9.0 Hz, 2H), 6.84 (s, 1H), 6.70 (dd, *J =* 8.6, 3.9 Hz, 1H), 4.72 (d, *J =* 4.3 Hz, 2H), 4.54 (t, *J =* 8.7 Hz, 2H), 4.25 (dd, *J =* 9.2, 5.0 Hz, 1H), 3.88 (s, 3H), 3.76 (s, 2H), 3.30 (d, *J* = 8.9 Hz, 2H), 3.19 (t*, J =* 4.4 Hz, 4H), 3.12-3.02 (m, 1H), 3.01-2.80 (m, 6H), 2.67 -2.54 (m, 2H), 2.48-2.37 (m, 1H), 2.30 (ddd, *J =* 25.9, 15.1, 9.0 Hz, 2H), 2.15 (dt, *J* = 19.4, 5.9 Hz, 1H), 1.93-1.74 (m, 2H). |
| 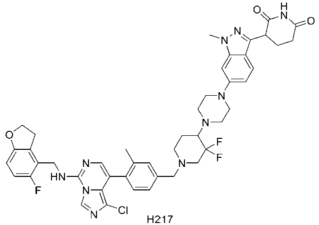 | MS m/z (ESI): 867.3[M+H]⁺ |
| | ¹H NMR (400 MHz, DMSO-*d*₆) δ 10.86 (s, 1H), 8.64 (s, 1H), 8.35 (t, *J =* 5.2 Hz, 1H), 7.49 (d, *J* = 9.2 Hz, 1H), 7.21 (s, 1H), 7.16 (d, *J* = 2.0 Hz, 2H), 7.06 (s, 1H), 6.99-6.89 (m, 2H), 6.83 (s, 1H), 6.71 (dd, *J =* 8.6, 3.8 Hz, 1H), 4.77-4.63 (m, 2H), 4.55 (t, *J* = 8.8 Hz, 2H), 4.25 (dd, *J* = 9.2, 5.2 Hz, 1H), 3.89 (s, 3H), 3.65-3.54 (m, 2H), 3.30 (s, 2H), 3.18 (d, *J =* 5.2 Hz, 4H), 2.90 (d, *J =* 5.6 Hz, 7H), 2.66 -2.54 (m, 2H), 3.04-2.84 (m, 2H), 2.20-2.11 (m, 2H), 2.08 (s, 3H), 1.88-1.76 (m, 2H). |
| 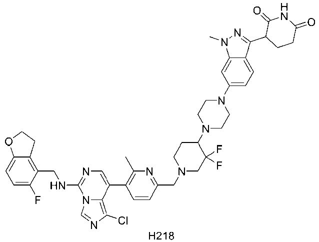 | MS m/z (ESI):434.8[M/2+H]⁺ |
| | ¹H NMR (400 MHz, DMSO-*d*₆) δ 10.86 (s, 1H), 8.66 (s, 1H), 8.45 (s, 1H), 7.60 (d, *J* = 8.0 Hz, 1H), 7.49 (d, *J* = 8.8 Hz, 1H), 7.33 (d*, J* = 7.6 Hz, 1H), 7.13 (d,*J* = 3.2 Hz, 1H), 7.01-6.89 (m, 2H), 6.84 (s, 1H), 6.71 (dd, *J =* 8.4, 3.6 Hz, 1H), 4.71 (dd, *J =* 14.4, 4.4 Hz, 2H), 4.55 (t*, J =* 8.8 Hz, 2H), 4.26 (dd, *J* = 9.2, 5.2 Hz, 1H), 3.89 (s, 3H), 3.80 - 3.66 (m, 2H), 3.31 (s, 1H), 3.19 (d, *J* = 5.2 Hz, 4H), 3.06 (s, 1H), 2.96-2.86 (m, 6H), 2.68-2.55 (m, 2H), 2.45-2.38 (m, 1H), 2.27 (s, 6H), 2.19-2.10 (m, 1H), 1.92-1.77 (m, 2H). |
| 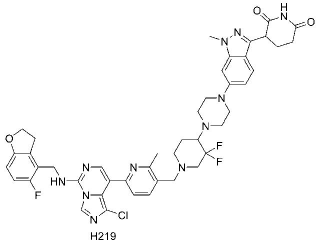 | MS m/z (ESI): 868.3[M+H]⁺_{∘} |
| | ¹H NMR (400 MHz, DMSO-*d*₆) δ 10.87 (s, 1H), 8.69 (s, 1H), 8.52 (t*, J =* 5.1 Hz, 1H), 7.67 (d, *J* = 7.9 Hz, 1H), 7.50 (d, *J* = 8.9 Hz, 1H), 7.43-7.31 (m, 2H), 7.06-6.89 (m, 2H), 6.84 (d, *J* = 1.9 Hz, 1H), 6.70 (dd, *J =* 8.7, 3.8 Hz, 1H), 4.74 (d, *J =* 4.2 Hz, 2H), 4.55 (t, *J =* 8.7 Hz, 2H), 4.26 (dd, *J* = 9.2, 5.1 Hz, 1H), 3.89 (s, 3H), 3.61 (s, 2H), 3.30 (t, *J =* 8.7 Hz, 2H), 3.19 (t, *J* = 5.0 Hz, 4H), 3.04-2.84 (m, 7H), 2.62 (dt, *J* = 11.9, 5.7 Hz, 2H), 2.53 (s, 3H), 2.47-2.23 (m, 3H), 2.20-2.11 (m, 1H), 1.83 (s, 2H). |
| 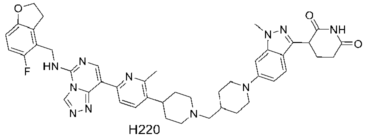 | MS m/z (ESI): 399.8[M/2+H]⁺ |
| 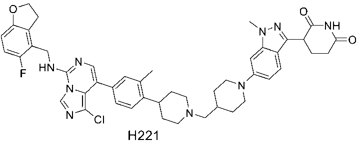 | MS m/z (ESI): 415.8[M/2+H] ⁺_{∘} |
| | ¹H NMR (400 MHz, DMSO-*d*₆) δ 10.86 (s, 1H), 8.67 (s, 1H), 8.37 (t, *J =* 5.0 Hz, 1H), 7.48 (d, *J =* 8.9 Hz, 1H), 7.27 (d, *J* = 8.0 Hz, 1H), 7.20 (d, *J* = 7.4 Hz, 2H), 7.14 (s, 1H), 6.93 (dd, *J =* 18.0, 8.6 Hz, 2H), 6.83 (s, 1H), 6.70 (dd, *J =* 8.6, 3.9 Hz, 1H), 4.70 (d, *J =* 5.0 Hz, 2H), 4.54 (t*, J =* 8.7 Hz, 2H), 4.25 (dd, *J* = 9.2, 5.2 Hz, 1H), 3.89 (s, 3H), 3.79 (d, *J* = 12.0 Hz, 2H), 3.30 (t, *J* = 8.9 Hz, 2H), 3.00 (d, *J =* 10.9 Hz, 2H), 2.80-2.66 (m, 3H), 2.60 (td, *J =* 13.6, 6.9 Hz, 2H), 2.34 (s, 3H), 2.27-2.12 (m, 3H), 2.09-1.95 (m, 2H), 1.84 (d, *J* = 10.4 Hz, 2H), 1.79-1.57 (m, 5H), 1.37-1.16 (m, 3H). |

### Biological Test

### Test Example 1. Test of compound on EED protein degradation

Materials and reagents are shown in Table 1:

**Table 1 Materials and reagents**

| Name | Supplier | Catalog Number |
|---|---|---|
| 96-well microplate, white bottom transparent flat bottom | Costar | 3903 |
| Phosphate buffered saline | Gibco | 10010 |
| Poly-D-lysine solution | Gibco | A38904-01 |
| RPMI-1640 medium | Gibco | 61870-127 |
| Fetal bovine serum | Gibco | 10099-141C |
| Dimethyl sulfoxide | Sigma | D5879 |
| Glutaraldehyde solution | Sigma | G6257 |
| Triton X-100 solution | Sigma | 93443 |
| Tris buffered saline-Tween (tablets) | Medicago | 09-7510-100 |
| Bovine serum albumin | Sigma | B2064 |
| EED rabbit monoclonal antibody | Cell Signal | 85322s |
| Anti-rabbit IgG, HRP-linked antibody | Cell Signal | 7074s |
| TMB substrate | Sigma | T4444 |
| Sulfuric acid | SCRC | 10021618 |
| Constant temperature incubator | Thermo | FORMA seriesII |
| Cell counter | CounterStar | IC1000 |
| Microplate reader | Tecan | Infinite Pro 3000 |
| Centrifuge | Eppendorf | 5810R |
| Horizontal shaker | Kylin-Bell | TS-200 |

In this experiment, In-cell western (ICW) method was used to quantitatively analyze the EED protein expression level in wsuDLCL-2 cells to detect the degradation activity of the compounds on the target protein. The specific steps were as follows:
1. wsuDLCL-2 cells were purchased from Nanjing Cobioer (Catalog No. CBP60073), subcultured and maintained in logarithmic growth state using RPMI-1640 complete medium (containing 10% v/v fetal bovine serum) according to the supplier's recommended conditions. The number of passages of cells used in the test did not exceed 20.
2. One day before cell seeding, a 96-well plate was coated with 100 µL of coating buffer (phosphate buffered saline containing 20 µg/mL poly-D-lysine) at room temperature for 2 hours. The coating solution was discarded, and the plate was washed once with 300 µL of phosphate buffered saline, which was then discarded. The plate was allowed to air dry naturally in a clean bench, wrapped around the edges with Parafilm to prevent moisture, and stored in a 4°C refrigerator until use.
3. Before cell seeding, the cells were collected in a centrifuge tube, centrifuged at 250 ref for 3 minutes, and the culture medium was discarded. The cells were resuspended in fresh complete medium, and 20 µL of the cell suspension was taken to calculate the cell concentration using a Countstar cell counter. After adjusting the density, cells were seeded into the prepared coated plate at 20,000 cells/90 µL and pre-incubated in a 37°C, 5% CO₂ incubator for 4 hours.
4. The negative and positive control groups were treated with 10 µL of control solution (non-complete medium containing 2.5% v/v DMSO, prepared by adding 2.5% v/v DMSO to RPMI-1640 medium). Compound gradient dilutions were prepared using non-complete medium, and 10 µL of the dilutions were added to the corresponding culture wells. The final concentration of DMSO in the test was 0.25% v/v. Subsequently, the plate was incubated in a 37°C, 5% CO₂ incubator for 48 hours (for EED detection).
5. Cells were fixed by directly adding 10 µL of fixative solution (phosphate buffered saline containing 5% v/v glutaraldehyde) to the cells and incubating at room temperature for 30 minutes. The fixative solution was discarded. Subsequently, cells were permeabilized with 100 µL of permeabilization solution (phosphate buffered saline containing 0.1% v/v Triton X-100) at room temperature for 30 minutes, and the permeabilization solution was discarded.
6. A sufficient amount of antibody dilution buffer (Tris buffered saline-Tween solution containing 1% w/v bovine serum albumin) was prepared and stored in a 4°C refrigerator until use.
7. Primary antibody dilution (EED antibody diluted at 1:500 with antibody dilution buffer) was prepared. 100 µL of antibody dilution buffer without antibody was added to the negative control group. 100 µL of the primary antibody dilution was added to the cells in the other groups, and the plate was incubated overnight at 4°C.
8. The primary antibody dilution was discarded. The plate was washed 3 times with 300 µL of Tris Buffered Saline-Tween solution, and the wash solution was discarded. Secondary antibody dilution (HRP-conjugated antibody diluted at 1:500 with antibody dilution buffer) was prepared, and 100 µL was added to the cells. The plate was incubated at room temperature for 2 hours.
9. The secondary antibody dilution was discarded. The plate was washed 3 times with 300 µL of Tris Buffered Saline-Tween solution, and the wash solution was discarded. 100 µL of TMB substrate solution was added, and the plate was incubated at room temperature in the dark for 15 minutes. Then, stop solution (2 M sulfuric acid) was added to terminate the reaction. The OD₄₅₀ₙₘ value was then read on a plate reader, and the results were used to analyze the target protein degradation ratio and compound activity.
10. The signal value of the negative control group was set as Bottom, and the signal value of the positive control group was set as Top. The compound concentration value (logarithm) was the X-axis, and the OD₄₅₀ₙₘ value at the corresponding concentration was the Y-axis, with HillSlope as the slope factor. A four-parameter logistic model (4PL logistic model) was used for fitting, and the calculation method was as follows: $Y = Bottom + \left(Top-Bottom\right) / \left(1+{10}^{∧}\left(\left({LogDC}_{50}-X\right)\times HillSlope\right)\right)$

After fitting, the DC₅₀ value of the compound was calculated, and the target protein degradation activity of the compound was compared based on the DC₅₀ value.

The test results show that the example compounds of the present application have excellent degradation effects on the EED protein, with half-maximal degradation concentration DC₅₀ values less than 1 µM. The DC₅₀ values of some example compounds are less than 500 nM, and the DC₅₀ values of some example compounds are less than 100 nM. The maximum degradation rate Dmax of the example compounds of the present application on the EED protein is greater than 50%. The test results are shown in Table 2:

**Table 2 Degradation activity of compounds on EED protein in wsuDLCL-2 cells**

| Compound number | EED DC₅₀ (nM) |
|---|---|
| H2 | <100 |
| H7 | <100 |
| H27 | <100 |

### Test Example 2. Test of compound on proliferation inhibition of tumor cells (wsuDLCL-2 cells)

Materials and reagents are shown in Table 3 below:

**Table 3 Materials and reagents**

| Name | Supplier | Catalog Number |
|---|---|---|
| 96-well microplate, white bottom transparent flat bottom | Costar | 3903 |
| Phosphate buffered saline | Gibco | 10010 |
| RPMI-1640 medium | Gibco | 61870-127 |
| Fetal bovine serum | Gibco | 10099-141C |
| Dimethyl sulfoxide | Sigma | D5879 |
| Cell Titer Glo | Promega | G7573 |
| Constant temperature incubator | Thermo | FORMA seriesII |
| Cell counter | CounterStar | IC1000 |
| Microplate reader | PerkinElmer | VictorX5 |
| Centrifuge | Eppendorf | 5810R |

In this experiment, the Cell Titer-Glo reagent was used to quantitatively analyze the proliferation activity of wsuDLCL-2 cells to detect the inhibitory ability of the compounds on tumor cell proliferation. The specific steps were as follows:
1. wsuDLCL-2 cells were purchased from Nanjing Cobioer (Catalog Nos. CBP60073 and CBP60629), subcultured and maintained in logarithmic growth state using RPMI-1640 complete medium (containing 10% v/v fetal bovine serum) according to the supplier's recommended conditions. The number of passages of cells used in the test did not exceed 20.
2. Before cell seeding, cells were collected in a centrifuge tube, centrifuged at 250 rcf for 3 minutes, and the culture medium was discarded. The cells were resuspended in fresh complete medium, and 20 µL of the cell suspension was taken to calculate the cell concentration using a Countstar cell counter. After adjusting the density, cells were seeded into a 96-well plate at 3,000 cells/180 µL and pre-incubated in a 37°C, 5% CO₂ incubator for 4 hours.
3. The negative and positive control groups were treated with 20 µL of control solution (non-complete medium containing 2.5% v/v DMSO). Compound gradient dilutions were prepared using non-complete medium, and 20 µL of the dilutions were added to the corresponding culture wells. The final concentration of DMSO in the test was 0.25% v/v. Subsequently, the plate was incubated in a 37°C, 5% CO₂ incubator for 144 hours.
4. 150 µL of Cell Titer-Glo (CTG) reagent was added directly to the cell culture, and the plate was incubated at room temperature in the dark for 30 minutes. The luminescence value was then read on a plate reader, and the results were used to analyze the cell proliferation ratio and compound activity.
5. The signal value of the negative control group was set as Bottom, and the signal value of the positive control group was set as Top. The compound concentration value (logarithm) was the X-axis, and the luminescence reading at the corresponding concentration was the Y-axis, with HillSlope as the slope factor. A four-parameter logistic model (4PL logistic model) was used for fitting, and the calculation method was as follows:
$Y = Bottom + \left(Top-Bottom\right) / \left(1+{10}^{∧}\left(\left({LogIC}_{50}-X\right)\times HillSlope\right)\right)$

After fitting, the IC₅₀ value of the compound was calculated, and the proliferation inhibitory activity of the compound was compared based on the IC₅₀ value.

The test results show that the example compounds of the present application have excellent proliferation inhibitory activity on tumor cells, such as wsuDLCL-2 cells, with IC₅₀ values less than 500 nM, and the IC₅₀ values of some compounds are less than 100 nM. The test results are shown in Table 4:

**Table 4 Proliferation inhibitory activity of compounds on wsuDLCL-2 cells**

| Compound number | CTG IC₅₀ (nM) | Compound number | CTG IC₅₀ (nM) | Compound number | CTG IC₅₀ (nM) |
|---|---|---|---|---|---|
| H1 | 336 | H2 | 24 | H4 | 141 |
| H6 | 335 | H7 | 14 | H10 | 272 |
| H16 | 94 | H27 | <100 | | |

### Test Example 3. Test of compound on proliferation inhibition of tumor cells (Pfeiffer cells)

Materials and reagents are shown in Table 5 below:

**Table 5 Materials and reagents**

| Name | Supplier | Catalog Number |
|---|---|---|
| 384-well microplate, white bottom transparent flat bottom | Costar | 3765 |
| Phosphate buffered saline | Gibco | 10010 |
| RPMI-1640medium | Gibco | 61870-127 |
| Fetal bovine serum | Gibco | 10099-141C |
| Dimethyl sulfoxide | Sigma | D5879 |
| Cell Titer Glo | Promega | G7573 |
| Constant temperature incubator | Thermo | FORMA series II |
| Cell counter | CounterStar | IC1000 |
| Microplate reader | PerkinElmer | VictorX5 |
| Centrifuge | Eppendorf | 5810R |

In this experiment, Cell Titer-Glo reagent was used to quantitatively analyze the proliferation activity of Pfeiffer cells to detect the inhibitory ability of the compounds on tumor cell proliferation. The specific steps were as follows:
1. Pfeiffer cells were purchased from Nanjing Cobioer (purchased from ATCC, Catalog No. #CRL2632), subcultured and maintained in logarithmic growth state using RPMI-1640 complete medium (containing 10% v/v fetal bovine serum) according to the supplier's recommended conditions. The number of passages of cells used in the test after resuscitation did not exceed 20.
2. Before cell seeding, cells were collected in a centrifuge tube, centrifuged at 250 rcf for 3 minutes, and the culture medium was discarded. The cells were resuspended in fresh complete medium, and 20 µL of the cell suspension was taken to calculate the cell concentration using a Countstar cell counter. After adjusting the density, cells were seeded into a 384-well plate at 2,000 cells/45 µL and pre-incubated in a 37°C, 5% CO₂ incubator for 4 hours.
3. The negative and positive control groups were treated with 5 µL of control solution (non-complete medium containing 1% v/v DMSO). Compound gradient dilutions were prepared using non-complete medium, and 5 µL of the dilutions were added to the corresponding culture wells. The final concentration of DMSO in the test was 0.1% v/v. Subsequently, the plate was incubated in a 37°C, 5% CO₂ incubator for 144 hours.
4. 40 µL of Cell Titer-Glo reagent was added directly to the cell culture, and the plate was incubated at room temperature in the dark for 30 minutes. The luminescence value was then read on a plate reader, and the results were used to analyze the cell proliferation ratio and compound activity.
5. The signal value of the negative control group was set as Bottom, and the signal value of the positive control group was set as Top. The compound concentration value (logarithm) was the X-axis, and the luminescence reading at the corresponding concentration was the Y-axis, with HillSlope as the slope factor. A four-parameter logistic model (4PL logistic model) was used for fitting, and the calculation method was as follows:Y = Bottom + (Top - Bottom) / (1 + 10^((Log IC₅₀ - X) × HillSlope))

After fitting, the IC₅₀ value of the compound was calculated, and the proliferation inhibitory activity of the compound was compared based on the IC₅₀ value.

The test results show that the example compounds of the present application have excellent proliferation inhibitory activity on tumor cells, such as Pfeiffer cells, with IC₅₀ values less than 500 nM. The IC₅₀ values of some compounds are less than 100 nM, the IC₅₀ values of some compounds are less than 50 nM, the IC₅₀ values of some compounds are less than 10 nM, the IC₅₀ values of some compounds are less than 5 nM, and the IC₅₀ values of some compounds are less than 1 nM. The IC₅₀ values of some compounds are less than 0.1 nM. The test results are shown in Table 6:

**Table 6 Proliferation inhibitory activity of example compounds on Pfeiffer cells**

| Compound number | CTG IC₅₀ (nM) | Compound number | CTG IC₅₀ (nM) | Compound number | CTG IC₅₀ (nM) |
|---|---|---|---|---|---|
| H2 | 170 | H110 | 0.5 | H170 | 1.3 |
| H7 | 218 | H111 | <0.1 | H171 | 7 |
| H26 | 164 | H112 | 0.7 | H172 | 8 |
| H27 | 30 | H113 | 0.4 | H173 | 0.2 |
| H33 | 164 | H114 | 0.6 | H175 | 1 |
| H37 | 211 | H115 | 4 | H176 | 0.8 |
| H38 | 176 | H116 | 2.2 | H177 | <0.03 |
| H39 | 240 | H117 | 9 | H178 | 2 |
| H43 | 45 | H118 | 1.7 | H179 | 0.9 |
| H44 | 10 | H119 | 5 | H180 | 0.7 |
| H45 | 7 | H120 | 6 | H181 | <0.03 |
| H46 | 43 | H121 | 0.7 | H183 | 0.9 |
| H48 | 58 | H122 | 0.9 | H184 | 1.7 |
| H49 | 15 | H123 | 0.4 | H185 | <0.03 |
| H51 | 49 | H124 | 4 | H186 | 2 |
| H53 | 9 | H125 | 7 | H187 | 21 |
| H54 | 65 | H129 | 3 | H188 | 4 |
| H56 | 56 | H130 | 1.4 | H189 | <0.03 |
| H57 | 9 | H131 | 1.3 | H190 | 0.5 |
| H58 | 43 | H132 | 1.5 | H191 | 0.35 |
| H59 | 188 | H133 | 7 | H192 | 12 |
| H60 | 0.8 | H134 | 7 | H193 | 1.1 |
| H61 | <0.1 | H135 | 4 | H194 | 0.12 |
| H62 | <0.1 | H136 | 0.2 | H195 | 30 |
| H63 | 0.2 | H137 | 1 | H197 | 109 |
| H64 | 58 | H138 | 25 | H200 | 84 |
| H65 | 5 | H139 | 20 | H201 | 70 |
| H66 | 38 | H140 | 13 | H202 | 5 |
| H67 | 60 | H141 | 20 | H203 | 1 |
| H68 | 17 | H144 | 0.3 | H205 | 0.06 |
| H69 | 53 | H145 | 8 | H206 | 0.10 |
| H70 | 8 | H146 | 11 | H207 | 0.27 |
| H71 | 0.1 | H147 | 1.5 | H208 | 0.07 |
| H72 | 0.7 | H148 | 1 | H209 | 0.11 |
| H73 | <0.1 | H149 | 0.5 | H211 | 0.15 |
| H78 | 163 | H150 | 8 | H212 | 0.17 |
| H80 | 191 | H151 | 0.4 | H213 | 0.07 |
| H93 | 13 | H152 | 0.4 | H215 | 5.83 |
| H94 | 28 | H153 | 5 | H216 | 1.15 |
| H95 | 2.7 | H154 | 136 | H217 | 7.29 |
| H96 | 29 | H157 | <0.03 | H218 | 2.18 |
| H97 | 0.7 | H159 | 0.9 | H219 | 2.48 |
| H98 | 15 | H160 | 0.1 | H221 | 0.76 |
| H99 | 0.5 | H161 | 0.9 | D2 | 391 |
| H100 | 0.5 | H162 | 5 | | |
| H102 | 3.8 | H163 | 1 | | |
| H103 | 4.2 | H164 | 2 | | |
| H104 | 5 | H165 | 22 | | |
| H105 | 0.2 | H166 | 0.2 | | |
| H106 | 1.6 | H167 | 2 | | |
| H107 | 1 | H168 | 0.8 | | |
| H109 | 0.5 | H169 | 1.4 | | |

Among them, Compound D2 is 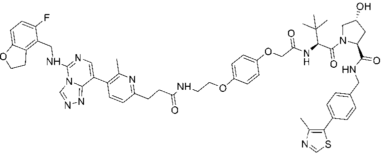 ( CAS: 2639882-72-5), which can be prepared by referring to the prior art or obtained commercially.

### Test Example 4. Test on EED Protein degradation in Karpas-422 Cells (ICW Method)

Reagents and materials are shown in Table 7:

**Table 7 Reagents and materials**

| Name | Supplier | Catalog Number | Name | Supplier | Catalog Number |
|---|---|---|---|---|---|
| 384-well microplate, white bottom transparent, flat bottom | Costar | 3765 | Anti-rabbit IgG, HRP-linked antibody | Cell Signal | 7074s |
| Phosphate buffered saline | Gibco | 10010 | TMB substrate | Sigma | T4444 |
| Poly-D-lysine solution | Gibco | A38904-01 | Sulfuric acid | SCRC | 1002161 8 |
| RPMI-1640 medium | Gibco | 61870-127 | Constant temperature incubator | Thermo | FORMA series II |
| Fetal bovine serum | Gibco | 10099-141C | Cell counter | CounterStar | IC1000 |
| Dimethyl sulfoxide (DMSO) | Sigma | D5879 | Microplate reader | Tecan | Infinite Pro 3000 |
| Glutaraldehyde solution | Sigma | G6257 | Centrifuge | Eppendorf | 5810R |
| Triton X-100 solution | Sigma | 93443 | Constant temperature incubation shaker | Shanghai Huxi | HW-400 TG |
| Hoechst 33342 | Thermo | H3570 | Bovine serum albumin | Sigma | B2064 |
| Tris buffered saline-Tween (tablets) | Medicago | 09-7510-100 | EED rabbit monoclonal antibody | Cell Signal | 85322s |

In this experiment, In-cell western (ICW) method was used to quantitatively analyze the EED protein expression level in Karpas-422 cells to detect the degradation activity of the compounds on the target protein. The specific steps were as follows:
1. Karpas-422 cells were purchased from Nanjing Cobioer (Catalog No. CBP60629), subcultured and maintained in logarithmic growth state using RPMI-1640 complete medium (containing 10% v/v fetal bovine serum) according to the supplier's recommended conditions. The number of passages of cells used in the test did not exceed 20.
2. One day before cell seeding, a 384-well plate was coated with 30 µL of coating buffer (phosphate buffered saline containing 30 µg/mL poly-D-lysine) at room temperature for 2 hours. The coating solution was then discarded, and the plate was washed once with 90 µL of phosphate buffered saline, which was then discarded. The plate was allowed to air dry naturally in a clean bench, wrapped around the edges with Parafilm to prevent moisture, and stored in a 4°C refrigerator until use.
3. Before cell seeding, cells were collected in a centrifuge tube, centrifuged at 250 rcf for 3 minutes, and the culture medium was discarded. The cells were resuspended in fresh complete medium, and 20 µL of the cell suspension was taken to calculate the cell concentration using a Countstar cell counter. After adjusting the density, cells were seeded into the prepared coated plate at 15,000 cells/45 µL and pre-incubated in a 37°C, 5% CO₂ incubator for 4 hours.
4. The negative and positive control groups were treated with 5 µL of control solution (serum-free medium containing 1% v/v DMSO). Compound gradient dilutions were prepared using the control solution, and 5 µL of the dilutions were added to the corresponding culture wells. The final concentration of DMSO in the test was 0.1% v/v. Subsequently, the plate was incubated in a 37°C, 5% CO₂ incubator for 18 hours.
5. The culture plate was removed, and cells were fixed by directly adding 10 µL of fixative solution (phosphate buffered saline containing 3% v/v glutaraldehyde) to the cells and incubating at room temperature for 30 minutes. The liquid was discarded. Subsequently, cells were treated with 30 µL of permeabilization staining solution (phosphate buffered saline containing 0.1% v/v Triton X-100 and 20 µg/mL Hoechst 33342) at room temperature for 30 minutes. The permeabilization solution was discarded, and the fluorescence value (excitation at 360 nm, emission at 460 nm) was read on a microplate reader.
6. 50 µL of repair solution (50 mM citric acid buffer) was added to all wells, and the plate was incubated at 70°C for 2 hours on a constant temperature incubation shaker. The microplate was then removed, equilibrated to room temperature, and the liquid was discarded.
7. A sufficient amount of antibody dilution buffer (Tris Buffered Saline-Tween solution containing 1% w/v bovine serum albumin) was prepared and stored in a 4°C refrigerator until use.
8. Primary antibody dilution (EED antibody diluted 1:1000 with antibody dilution buffer) was prepared. 30 µL of antibody dilution buffer without antibody was added to the negative control group. 30 µL of the primary antibody dilution was added to the cells in the other groups, and the plate was incubated overnight at 4°C.
9. The primary antibody dilution was discarded. The plate was washed 3 times with 90 µL of Tris Buffered Saline-Tween solution, and the wash solution was discarded. Secondary antibody dilution (HRP-linked antibody diluted 1:500 with antibody dilution buffer) was prepared, and 30 µL was added to the cells. The plate was incubated at room temperature for 2 hours.
10. The secondary antibody dilution was discarded. The plate was washed 3 times with 90 µL of Tris Buffered Saline-Tween solution, and the wash solution was discarded. 30 µL of TMB substrate solution was added, and the plate was incubated at room temperature in the dark for 15 minutes. Then, 30 µL of stop solution (2 M sulfuric acid) was added to terminate the reaction. The OD₄₅₀ₙₘ value was then read on a plate reader, and the results were used to analyze the target protein degradation ratio and compound activity.
11. The signal value of the negative control group was set as Bottom, and the signal value of the positive control group was set as Top. The compound concentration value (logarithm) was the X-axis, and the OD₄₅₀ₙₘ value at the corresponding concentration was the Y-axis. A four-parameter logistic model (4PL logistic model) was used for fitting, and the calculation method was as follows:Y = Bottom + (Top - Bottom) / (1 + 10^((LogDC₅₀ - X) × HillSlope)). After fitting, the DC₅₀ value of the compound was calculated, and the target protein degradation activity of the compound was compared based on the DC₅₀ value.

The test results show that the compounds of the present application have excellent EED protein degradation activity, especially better EED protein degradation activity compared to Compound D2. The specific results are shown in Table 8:

**Table 8 Degradation activity of compounds on EED protein in Karpas-422 cells**

| Compound number | DC₅₀ (nM) | Dₘₐₓ (%) | Compound number | DC₅₀ (nM) | Dₘₐₓ (%) |
|---|---|---|---|---|---|
| H2 | 4 | 80 | H131 | 2 | 63 |
| H4 | 21 | 73 | H132 | 2 | 78 |
| H7 | 5 | 81 | H133 | 1.5 | 72 |
| H10 | ND | 65 | H134 | 1.5 | 71 |
| H20 | 50 | 57 | H135 | 1.8 | 65 |
| H24 | 23 | 72 | H136 | 0.7 | 69 |
| H25 | 20 | 77 | H137 | 9 | 55 |
| H26 | 11 | 81 | H138 | 6 | 54 |
| H27 | 3 | 81 | H139 | 3 | 64 |
| H29 | ND | 51 | H140 | 6 | 62 |
| H32 | ND | 26 | H141 | 8 | 56 |
| H33 | 11 | 81 | H143 | ND | 38 |
| H36 | ND | 39 | H144 | 3 | 68 |
| H37 | 37 | 73 | H145 | ND | 47 |
| H39 | ND | 31 | H146 | ND | 48 |
| H40 | 148 | 68 | H147 | 45 | 61 |
| H41 | ND | 22 | H148 | 29 | 59 |
| H43 | 28 | 63 | H149 | 10 | 64 |
| H44 | 3 | 73 | H150 | ND | 41 |
| H45 | 6 | 75 | H151 | 1 | 67 |
| H46 | 23 | 76 | H152 | 9 | 55 |
| H47 | ND | 46 | H153 | ND | 37 |
| H49 | 27 | 51 | H154 | ND | 33 |
| H51 | ND | 32 | H155 | ND | 29 |
| H52 | ND | 25 | H157 | 10 | 62 |
| H53 | 16 | 55 | H158 | ND | 38 |
| H54 | ND | 36 | H159 | 3 | 68 |
| H55 | ND | 40 | H160 | 4 | 71 |
| H56 | ND | 29 | H161 | 3 | 71 |
| H57 | 25 | 59 | H162 | <0.01 | 75 |
| H58 | ND | 33 | H163 | 0.6 | 69 |
| H60 | 6 | 59 | H164 | 0.3 | 59 |
| H61 | 4 | 55 | H165 | ND | 49 |
| H62 | 4 | 66 | H166 | 1.8 | 68 |
| H63 | 3 | 55 | H167 | 4 | 62 |
| H64 | 20 | 66 | H168 | 7 | 54 |
| H65 | 4 | 74 | H169 | 1 | 57 |
| H66 | 19 | 76 | H170 | ND | 48 |
| H67 | 22 | 78 | H171 | 28 | 51 |
| H68 | 7 | 77 | H172 | 18 | 52 |
| H69 | 23 | 81 | H173 | 0.4 | 67 |
| H70 | 2 | 86 | H174 | ND | 43 |
| H71 | 15 | 67 | H175 | 1.6 | 67 |
| H72 | 0.7 | 57 | H176 | 4.1 | 66 |
| H73 | 3.3 | 67 | H177 | 1.1 | 65 |
| H87 | ND | 35 | H178 | 3 | 70 |
| H88 | ND | 21 | H179 | 3 | 67 |
| H89 | ND | 32 | H180 | <0.01 | 72 |
| H90 | ND | 35 | H181 | 5 | 66 |
| H92 | ND | 21 | H183 | 3 | 63 |
| H93 | 5 | 62 | H184 | 3 | 66 |
| H94 | 15 | 61 | H185 | 16 | 56 |
| H95 | 3 | 57 | H186 | 3 | 56 |
| H96 | ND | 36 | H187 | ND | 39 |
| H97 | 0.3 | 61 | H188 | 7 | 64 |
| H98 | ND | 41 | H189 | 3 | 66 |
| H99 | 0.3 | 70 | H190 | 2.5 | 71 |
| H100 | 0.8 | 65 | H191 | 4.8 | 69 |
| H102 | 22 | 52 | H192 | 5.8 | 65 |
| H103 | ND | 50 | H193 | 2.8 | 68 |
| H104 | 20 | 58 | H194 | 1.5 | 64 |
| H105 | 0.2 | 74 | H195 | ND | 30 |
| H106 | 2.5 | 65 | H197 | ND | 44 |
| H107 | 1.1 | 65 | H198 | ND | 41 |
| H108 | ND | 43 | H199 | ND | 27 |
| H109 | 0.9 | 70 | H200 | ND | 23 |
| H110 | 0.1 | 63 | H201 | ND | 43 |
| H111 | 0.6 | 73 | H202 | ND | 47 |
| H112 | 0.4 | 66 | H203 | 1.1 | 70 |
| H113 | 0.4 | 65 | H204 | ND | 41 |
| H114 | 1.4 | 61 | H205 | 7.6 | 58 |
| H115 | 1 | 74 | H206 | 3.4 | 62 |
| H116 | 4.3 | 61 | H207 | 3.3 | 66 |
| H117 | 9.1 | 56 | H208 | 1.1 | 56 |
| H118 | 5.1 | 58 | H209 | 27 | 54 |
| H119 | 1.4 | 67 | H211 | 3.1 | 56 |
| H120 | 2.5 | 63 | H212 | ND | 51 |
| H121 | 0.5 | 71 | H213 | 1.5 | 59 |
| H122 | 33 | 64 | H214 | ND | 27 |
| H123 | 0.6 | 65 | H215 | ND | 47 |
| H124 | 4 | 62 | H216 | 17 | 53 |
| H125 | ND | 48 | H217 | ND | 45 |
| H126 | 96 | 54 | H218 | ND | 48 |
| H127 | ND | 38 | H219 | 35 | 51 |
| H128 | ND | 23 | H221 | 5 | 62 |
| H129 | 3 | 66 | D2 | 74 | 76 |
| H130 | 2.8 | 64 | | | |

| | | | | | |
|---|---|---|---|---|---|
| Note: ND indicates not detected. | | | | | |

Among them, Compound D2 is 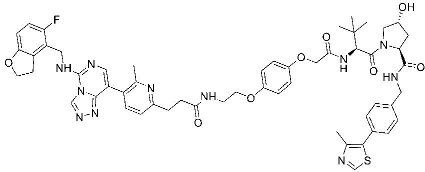 (CAS: 2639882-72-5), which can be prepared by referring to the prior art or obtained commercially.

### Test Example 5. In vivo pharmacokinetic study in mice

LC/MS/MS method was used to determine the drug concentrations in mouse plasma at different time points after intravenous injection and oral gavage administration of the compounds of the present application. The pharmacokinetic behavior of the compounds of the present application in mice was studied to evaluate their pharmacokinetic characteristics.

| **Reagent** | **Company** | **Catalog Number** |
|---|---|---|
| HP-β-CD | Sigma | 102616102 |
| Citric Acid | Qurchem | XW00779291 |
| PEG 400 | Sigma | 102637684 |
| DMSO | Sigma | 102626022 |
| Methanol | Fisher | F23NBD201 |
| Acetonitrile | Fisher | F22M9L201 |
| Tween 80 | Sigma | BCBJ8859V |
| Saline | SHIMEN | 2308222002 |
| FA | SCR | 80065518 |

1. Experimental protocol:
Test animals: Healthy adult male ICR mice, weighing 25-40 g, 6 mice. All mice had free access to food and water. Supplied by Beijing Vital River Laboratory Animal Technology Co., Ltd. Minimum of 3 days adaptation period, SPF area room, indoor temperature maintained at 20-26°C, indoor relative humidity maintained at 40-70%. Illuminated with fluorescent lamps, 12-hour light (08:00-20:00) and 12-hour dark, 2-5 mice per cage (same administration group). Unlimited access to feed (irradiated sterilized, Shaanxi Qinle Pharmaceutical Chemical Co., Ltd., China) and water (tap water purified by ultrapure water filtration system).

| Group | Compound | Number of animals | Route of administration | Dose (mg/kg) | Formulation concentration (mg/mL) | Administration volume (mL/kg) | Vehicle |
|---|---|---|---|---|---|---|---|
| 1 | Test Compound | 4 | IV | 2 | 0.5 | 4 | 10%DMSO/40% PEG400/5%Twe en 80/45% saline |
| 2 | Test Compound | 4 | PO | 10 | 1 | 10 | 10%HP-β-CD and 50 mM Citrate in water (pH 4.0) |

2. Animal experiment formulation:
2.1. 10% DMSO / 40% PEG400 / 5% Tween 80 / 45% saline
   2.1.1 Vehicle formulation preparation: N/A
   2.1.2 Administration formulation preparation:
      Intravenous injection group: an appropriate amount of API powder was accurately weighed, added to 10% of the final volume of DMSO, and shaken and sonicated to ensure complete dissolution. After dissolution, 40% of the formulation volume of PEG400 and 5% of the formulation volume of Tween 80 were added, followed by vortexing or sonication. Finally, 45% of the formulation volume of saline was added to obtain a clear, transparent solution at a concentration of 0.5 mg/mL.
2.2 10% HP-β-CD and 50 mM Citrate buffer in water (pH 4.0)
   2.2.1 Vehicle formulation preparation:
      50 mM Citrate buffer solution (pH 4.0): 1.92 g of anhydrous citric acid was weighed and added to 100 mL of purified water, stirred to dissolve, yielding a 100 mM citric acid solution. Separately, 2.94 g of sodium citrate was weighed and added to 100 mL of water, yielding a 100 mM sodium citrate solution. 33 mL of the 100 mM citric acid solution was taken, mixed with 17 mL of the 100 mM sodium citrate solution, mixed well, and diluted to 100 mL to obtain 50 mM citrate buffer at pH 4.0.
      10% HP-β-CD (Hydroxypropyl-β-cyclodextrin) and 50 mM Citrate buffer (pH 4.0) solution: 10 g of hydroxypropyl-β-cyclodextrin was accurately weighed and added to 100 mL of 50 mM Citrate buffer (pH 4.0) aqueous solution, and sonicated to ensure complete dissolution.
   2.2.2 Administration formulation preparation:
      Oral gavage group: an appropriate amount of API powder was accurately weighed, added to the final volume of 10% HP-β-CD and 50 mM Citrate in water (pH 4.0) solution, and shaken and sonicated to ensure complete dissolution. The mixture was then disrupted using a cell disruptor for 1 minute to obtain a clear, transparent or suspended solution at a concentration of 1 mg/mL.

3. Administration method: before administration, animals meeting the experimental requirements were selected, weighed, and marked. ICR mice were administered 2 mg/kg via tail vein injection and 10 mg/kg via oral gavage.
4. Sample Collection: before sampling, mice were restrained. Blood was collected from each dosed mouse via the submandibular vein at predetermined time points (IV administration: 0.083h, 0.25h, 0.5h, 1h, 2h, 4h, 7h, 24h, 30h, and 48h post-dose, totaling 10 time points; Oral gavage administration: 0.083h, 0.25h, 0.5h, 1h, 2h, 4h, 7h, 24h, 30h, and 48h post-dose, totaling 10 time points), approximately 80 µL of blood was collected each time. The blood was transferred to a 1.5 mL tube pre-filled with K₂EDTA, and centrifuged (Eppendorf, Centrifuge 5804R) for 3 min (10000 rpm, 4°C) to separate plasma. The entire process was completed within 15 minutes after blood collection. All samples were stored in a -20°C freezer (Panasonic) until sample analysis.
5. Sample Analysis and Calculation: the drug concentration was determined using LC/MS/MS (SCIEX, TRIPLE QUAD 6500+), and pharmacokinetic parameters such as maximum drug concentration (Cₘₐₓ), time to peak concentration (Tₘₐₓ), drug elimination half-life (T_{1/2}), drug exposure (AUC_{0-inf}), and bioavailability (F) were calculated using Data Analysie System software (Shanghai Bojia Medical Technology Co., Ltd., Version 3.0). The pharmacokinetic parameters of some example compounds of the present application in mice under the same dose and administration route are shown in Table 9:

**Table 9 Pharmacokinetic parameters of compounds in mice**

| Compound Number | Maximum drug concentration Cₘₐₓ (ng/mL) | Time to peak concentration Tₘₐₓ (h) | Drug elimination half-life T_{1/2} (h) | Drug exposure AUC_{0-inf} (hr*ng/mL) | Oral bioavailability F (%) |
|---|---|---|---|---|---|
| H53 | 1364 | 7.0 | 10.2 | 34773 | 61.1 |
| H60 | 785 | 5.0 | 12.7 | 19505 | 55.8 |
| H61 | 23 | 2.0 | 3.8 | 119 | 1.4 |
| H63 | 385 | 3.3 | 5.4 | 5058 | 21.5 |
| H105 | 445 | 7.0 | 12.3 | 11332 | 34.2 |
| H110 | 613 | 6.0 | 13.9 | 14055 | 43.3 |
| H111 | 1131 | 4.0 | 5.1 | 16610 | 82.9 |
| H114 | 6 | 2.3 | 4.5 | 44 | 0.2 |
| H115 | 491 | 7.0 | 13.4 | 13748 | 46.6 |
| H121 | 1250 | 5.0 | 6.8 | 18218 | 23.7 |
| H122 | 1031 | 4.0 | 4.2 | 13186 | 33.8 |
| H123 | 1310 | 4.0 | 6.0 | 16675 | 61.4 |
| H131 | 265 | 7.0 | 7.0 | 3963 | 15.8 |
| H136 | 917 | 6.0 | 4.6 | 13339 | 39.2 |
| H144 | 721 | 6.0 | 5.9 | 11637 | 40.9 |
| H147 | 269 | 4.3 | 4.0 | 3236 | 18.4 |
| H148 | 548 | 7.0 | 5.7 | 8559 | 24.0 |
| H149 | 1150 | 7.0 | 4.4 | 17646 | 65.1 |
| H151 | 909 | 7.0 | 4.0 | 13258 | 38.8 |
| H157 | 1370 | 5.3 | 4.5 | 19631 | 71.6 |
| H159 | 814 | 3.3 | 4.8 | 10730 | 65.1 |
| H160 | 492 | 5.0 | 6.6 | 8204 | 57.0 |
| H166 | 90.5 | 4.0 | 10.88 | 2992 | 11.49 |
| H167 | 819 | 6.0 | 6.9 | 14508 | 41.0 |
| H169 | 1360 | 5.0 | 5.6 | 20215 | 35.8 |
| H173 | 1667 | 7.0 | 11.7 | 39355 | 63.9 |
| H175 | 808 | 6.0 | 5.1 | 12456 | 51.4 |
| H176 | 2543 | 6.0 | 4.8 | 36662 | 65.4 |
| H177 | 1553 | 1.3 | 4.2 | 17788 | 73.6 |
| H180 | 20 | 2.7 | 6.6 | 203 | 1.3 |
| H185 | 534 | 6.0 | 5.8 | 8401 | 34.8 |
| H189 | 664 | 3.5 | 6.6 | 8307 | 24.5 |
| D2 | 21 | 0.4 | 1.1 | 40 | 1.7 |
| D3 | 0.00 | 0.00 | 0.00 | 0.00 | 0.00 |

Among them, Compound D3 is 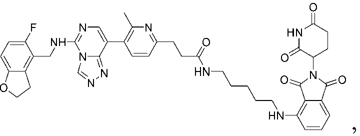 which can be prepared by referring to the prior art or obtained commercially.

As can be seen from Table 9, compared with existing compounds, the compounds of the present application have superior *in vivo* drug exposure and higher oral bioavailability.

**Test Example 6.** Test for the growth effect on subcutaneous xenograft tumor model of human B-cell non-Hodgkin lymphoma Karpas-422 cell line in BALB/c nude mice

Materials and reagents are shown in Table 10

**Table 10 Materials and reagents**

| **Reagent** | **Company** | **Catalog Number** |
|---|---|---|
| RPMI-1640Medium | GIBCO | 61870-036 |
| Fetal bovine serum (FBS) | GIBCO | 10099-141C |
| Trypsin-EDTA | GIBCO | 25200-072 |
| PBS | Corning cellgro | 21-040-CVR |
| Martigel | Corning cellgro | 354234 |
| PEG 300 | Sigma | 102601449 |
| HP-β-CD | Sigma | 102616102 |
| Citric acid | Qurchem | XW00779291 |
| PEG 400 | Sigma | 102637684 |
| DMSO | Sigma | 102626022 |

| | | |
|---|---|---|
| Instruments and equipment are shown in Table 11 | | |

**Table 11 Instruments and equipment**

| **Instrument** | **Company** | **Model** |
|---|---|---|
| Centrifuge | Thermo Fisher Scientific | Thermo Fisher legend MACH 1.6/ |
| Inverted microscope | Nikon | TS100 |
| Biosafety cabinet (cell culture) | Thermo Fisher Scientific | 1379 |
| CO₂ incubator | Thermo Fisher Scientific | Thermo HERA cell 150 |
| Water bath | Shanghai Jinghong | DK-8B |
| Liquid nitrogen tank | Thermo Fisher Scientific | Thermo LOCATOR 4 |
| Pipettor (electric) (cell culture) | Thermo Fisher Scientific | S1 Pipet Filler |
| Pipette (1 ml) (cell culture) | Eppendorf | L20989H |
| Pipette (100 µl) (cell culture) | Eppendorf | L38752H |
| Pipette (20 µl) (cell culture) | Eppendorf | N22990H |
| Anesthesia machine | RWD Life Science | R500IE |
| Biosafety cabinet (cell inoculation) | Thermo Fisher Scientific | Thermo MSC- Advantage |
| Biosafety cabinet (Dispensing) | Thermo Fisher Scientific | Thermo MSC- Advantage |
| Balance (animal body weight) | Mettler Toledo | PL602E/02 |
| Vernier caliper | Mitutoyo | 500-151-30 CD-15AX |

### 1. Animals

Animal species and strain: BALB/c nude mice
Administration history: no history of administration
Sex, age, weight: female, 6-8 weeks, body weight 18-25 grams
Breeder/supplier: Shanghai Model Organisms Center, Inc.
Acclimation period: no less than 3-7 days
Room: SPF area room
Indoor temperature: 20-26°C
Indoor relative humidity: 40-70%
Lighting: fluorescent lamp illumination, 12-hour light (08:00-20:00) and 12-hour dark
Animal housing: 2-6 animals per cage (same administration group)
Food: unlimited access to feed (irradiated sterilized, Shaanxi Qinle Pharmaceutical Chemical Co., Ltd., China)
Water: unlimited access to drinking water (tap water purified by ultrapure water filtration system)
Female BALB/c nude mice were purchased from the supplier and used to select animals with suitable body weight range and tumor size for enrollment. All animals were specific pathogen-free, approximately 6-8 weeks old upon arrival at the laboratory.

### 2. Experimental procedure

### 2.1 Cell culture

The human B-cell non-Hodgkin lymphoma Karpas-422 cell line used in this experiment was cultured in RPMI-1640 medium supplemented with 10% FBS in a 37°C incubator with 5% CO₂. Within ten passages of continuous culture, 100 µL of PBS containing 1 × 10⁷ Karpas-422 cells was mixed with an equal volume of Matrigel, and the mixture was subcutaneously inoculated into the right side of the mouse near the armpit, with an inoculation volume of approximately 200 µL. Before inoculation, mice were anesthetized with 3-4% isoflurane.

### 2.2 Animal grouping and administration regimen

When the tumors grew to an average size of approximately 70-150 mm³, tumor-bearing mice were randomly assigned to groups based on tumor volume and body weight, with 8 mice per group. The day of grouping and administration was defined as Day 0. As shown in Table 9, Compound D2 had low oral bioavailability (1.7%) and low *in vivo* exposure (40 hr*ng/mL) in mice, making it unsuitable for efficacy studies and was used as a positive compound for efficacy studies. According to the results disclosed in the reference (J. Med. Chem. 2021, 64, 14540-14556), Compound D1 (CAS: 2585648-55-9) was selected as the positive compound for this experiment.

The grouping and administration regimen are shown in Table 12. The control group was administered intraperitoneally with 70% aqueous solution containing 5% glucose + 30% aqueous solution containing 15% HP-β-CD.

**Table 12. Grouping and Administration Regimen**

| Group | Test Article | N | Dose (mg/kg) | Administration Regimen |
|---|---|---|---|---|
| G1 | Control group | 8 | NA | i.p. (intraperitoneal injection), Q2D (once every two days) |
| G2 | Compound D1 | 8 | 50 | p.o. (oral), QD (once daily) |
| G3 | Compound H105 | 8 | 10 | p.o. (oral), QD (once daily) |

| | | | | |
|---|---|---|---|---|
| Note: N: number of animals per group. Administration volume: the administration volume for animals was adjusted according to 10 µL/g body weight. NA indicates no administration. | | | | |

### 2.3 Preparation of test articles

The preparation and storage of test articles are shown in Table 13:

**Table 13 Preparation and storage scheme for test articles**

| Test Article | Preparation method | Concentration after preparation (mg/mL) | Storage conditions | Preparation frequency |
|---|---|---|---|---|
| Compound D 1 50 mg/kg | Weigh 12.5 mg powder + 2.5 mL (PEF 300) → G2 | G2: 5 | 2 - 8°C | Freshly prepared |
| Compound H105 10 mg/kg | Weigh 2.5 mg powder + 2.5 mL (pH=4.0 buffer solution containing 10% HP-β-CD in 50 mM citric acid) → G3 | G3: 1 | 2 - 8°C | Freshly prepared |

### 2.4 Evaluation method

Cage-side observation: The appearance and behavior of each mouse were observed daily, continuously from the start of administration until the end of the experiment. All abnormal appearance, morphology, and behavioral activities were recorded in the laboratory clinical observation log.

Tumor volume: For the subcutaneous xenograft model, tumor volumes were measured twice weekly after grouping. The tumor volume (V) was calculated as follows: V = (length × width²) / 2. The relative tumor volume (RTV) for each nude mouse was calculated as: RTV = Vₜ / V₀, where Vₜ is the measured volume at each time point, and V₀ is the volume at the start of treatment.

Animal body weight: After grouping, mouse body weight was measured and recorded twice weekly.

Animal status observation: If animals developed diarrhea, lethargy, etc., it was recorded. 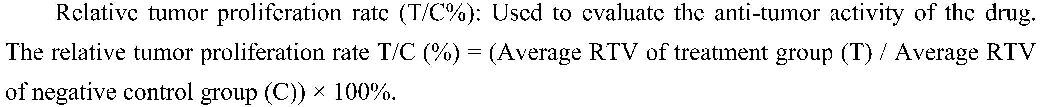 Relative tumor proliferation rate (T/C%): Used to evaluate the anti-tumor activity of the drug. The relative tumor proliferation rate T/C (%) = (Average RTV of treatment group (T) / Average RTV of negative control group (C)) × 100%. 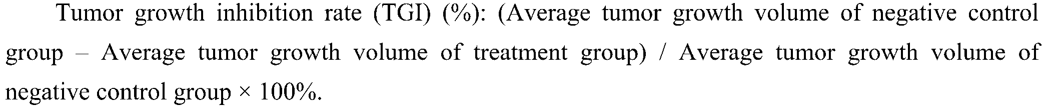 Tumor growth inhibition rate (TGI) (%): (Average tumor growth volume of negative control group - Average tumor growth volume of treatment group) / Average tumor growth volume of negative control group × 100%.

The negative control group was the control group, and the treatment groups were the groups administered D1 or H105. Results are presented as mean ± S.E.M. Comparisons between two groups were performed using Dunnett's multiple comparison test. A p-value < 0.05 was considered statistically significant.

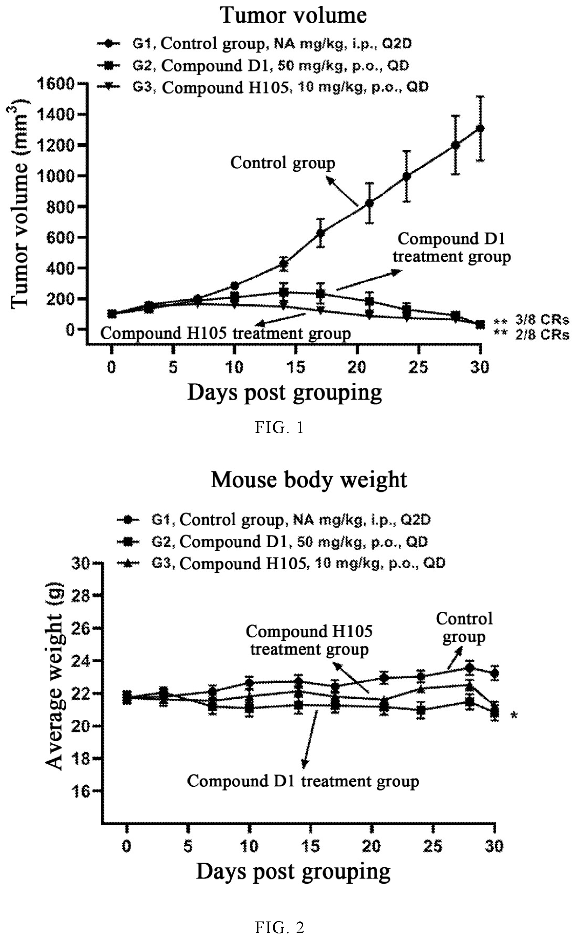

The test results are shown in Figures 1-2 and Table 14. Figure 1 shows the *in vivo* anti-tumor activity results of Compound D1 and Compound H105, with *P<0.05, P<0.01, and *P<0.001 indicating significant differences from G1; CRs indicate complete tumor regression. Figure 2 shows the effect of Compound D1 and Compound H105 on mouse body weight, with *P<0.05, P<0.01, and *P<0.001 indicating significant differences from G1. Table 14 shows the experimental therapeutic effect of the test compounds on the human B-cell non-Hodgkin lymphoma Karpas-422 xenograft in nude mice.

**Table 14 Experimental therapeutic effect of test compounds on human B-cell non-Hodgkin lymphoma Karpas-422 xenograft in nude mice**

| Compound | Group | Dose (mg/kg) | Average body weight (g) | | Tumor volume | | RTV | T/C (%) | TGI (%) |
|---|---|---|---|---|---|---|---|---|---|
| | | | D0 | D30 | D0 | D30 | | | |
| Control Group | G1 | / | 21.8 | 23.2 | 74 | 1307 | 16.99 | / | / |
| Compound D1 | G2 | 50 | 21.8 | 20.8 | 74 | 30 | 0.43 | 2.53 | 103.56 |
| Compound H105 | G3 | 10 | 21.7 | 21.2 | 74 | 31 | 0.46 | 2.69 | 103.43 |

| | | | | | | | | | |
|---|---|---|---|---|---|---|---|---|---|
| Note: TGI: tumor growth (volume) inhibition rate. | | | | | | | | | |

From Table 14 above and Figure 1, it can be seen that the compounds of the present disclosure have a strong *in vivo* anti-tumor activity. Compound D1 at a dose of 50 mg/kg could effectively inhibit tumor growth, and Compound H105 at a dose of 10 mg/kg could effectively inhibit tumor growth. It can be seen from Figure 2 that there was no significant fluctuation in the body weight of the tested mice, indicating that the compounds of the present disclosure have no obvious toxicity to the mice themselves while effectively inhibiting tumors.

Among them, Compound D1 is 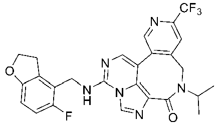 (CAS: 2585648-55-9), which can be prepared by referring to the prior art or obtained commercially.

## Claims

1. A compound of formula (I), or a pharmaceutically acceptable salt thereof, or a stereoisomer thereof:
POI-(L)ₙ₀-ULM (I),
wherein, POI is a ligand that binds to an EED protein;
L is a linker connecting POI and ULM;
ULM is a group that binds to an E3 ligase; and
n0 is 0 or 1.

2. The compound of formula (I) according to claim 1, or a pharmaceutically acceptable salt thereof, or a stereoisomer thereof, wherein POI is a structure of formula (A-1) or an isomer thereof: 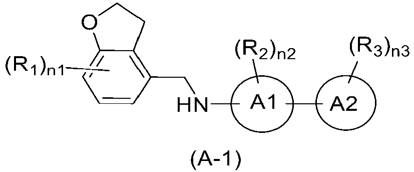 wherein,
ring A1 is a 5- to 20-membered heterocycloalkyl ring (preferably a 5- to 6-membered heterocycloalkyl ring, preferably a 15- to 20-membered heterocycloalkyl ring, more preferably a 15- to 18-membered heterocycloalkyl ring, further preferably an 18-membered heterocycloalkyl ring) or a 5-to 10-membered heteroaryl ring;
ring A2 is absent, or is a 3- to 15-membered heterocycloalkyl ring, a C₃₋₁₀ cycloalkyl ring, a 5- to 15-membered heteroaryl ring (preferably a 5- to 12-membered heteroaryl ring, more preferably a 5- to 10-membered heteroaryl ring, further preferably a 5- to 6-membered heteroaryl ring) or a C₆₋₁₀ aryl ring (preferably a benzene ring or a naphthalene ring);
(R₁)ₙ₁ represents substitution of hydrogen on 2,3-dihydrobenzofuran ring by n₁ R₁, n1 is 0, 1 or 2, each R₁ is identical or different, and each R₁ is independently selected from the group consisting of hydrogen, deuterium, halogen (preferably fluorine, chlorine, or bromine), oxo, C₁₋₈ alkyl (preferably C₁₋₆ alkyl, more preferably C₁₋₃ alkyl), C₁₋₈ haloalkyl (preferably C₁₋₆ haloalkyl, more preferably C₁₋₃ haloalkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy), -COC₁₋₈ alkyl (preferably -COC₁₋₆ alkyl, more preferably -COC₁₋₃ alkyl), -OCOC₁₋₈ alkyl (preferably -OCOC₁₋₆ alkyl, more preferably -OCOC₁₋₃ alkyl), -COOC₁₋₈ alkyl (preferably -COOC₁₋₆ alkyl, more preferably -COOC₁₋₃ alkyl), -CONH₂, -CONHC₁₋₈ alkyl (preferably -CONHC₁₋₆ alkyl, more preferably -CONHC₁₋₃ alkyl), -CON(C₁₋₈ alkyl)₂ (preferably -CON(C₁₋₆ alkyl)₂, more preferably -CON(C₁₋₃ alkyl)₂), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), -OSO₂C₁₋₈ alkyl (preferably -OSO₂C₁₋₆ alkyl, more preferably -OSO₂C₁₋₃ alkyl), -SO₂NH₂, -SO₂NHC₁₋₈ alkyl (preferably -SO₂NHC₁₋₆ alkyl, more preferably -SO₂NHC₁₋₃ alkyl), -SO₂N(C₁₋₈ alkyl)₂ (preferably -SO₂N(C₁₋₆ alkyl)₂, more preferably -SO₂N(C₁₋₃ alkyl)₂), C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl); and/or two R₁ and carbon atom linked to the two R₁ together form a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring) or a 3- to 15-membered heterocycloalkyl ring (preferably a 3- to 10-membered heterocycloalkyl ring, more preferably a 3- to 8-membered heterocycloalkyl ring, further preferably a 3- to 6-membered heterocycloalkyl ring); the C₁₋₈ alkyl, C₁₋₈ haloalkyl, C₁₋₈ alkoxy, C₁₋₈ haloalkoxy, -COC₁₋₈ alkyl, -COOC₁₋₈ alkyl, -OCOC₁₋₈ alkyl, -CONH₂, -CONHC₁₋₈ alkyl, -CON(C₁₋₈ alkyl)₂, -SOC₁₋₈ alkyl, -SO₂C₁₋₈ alkyl, -OSO₂C₁₋₈ alkyl, -SO₂NH₂, -SO₂NHC₁₋₈ alkyl, -SO₂N(C₁₋₈ alkyl)₂, C₃₋₈ cycloalkyl, 3- to 12-membered heterocycloalkyl, 5- to 10-membered heteroaryl, C₆₋₁₄ aryl, C₃₋₁₅ cycloalkyl ring and 3- to 15-membered heterocycloalkyl ring are unsubstituted or substituted with 1, 2 or 3 substituents selected from the group consisting of halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl) and 5- to 10-membered heteroaryl (preferably 5- to 6-membered heteroaryl);
(R₂)ₙ₂ represents substitution of hydrogen on ring A1 by n2 R₂, n2 is 0, 1 or 2, each R₂ is identical or different, and each R₂ is independently selected from the group consisting of X₁, hydrogen, deuterium, halogen, cyano, carboxyl, hydroxyl, amino, oxo, C₁₋₈ alkyl (preferably C₁₋₆ alkyl, more preferably C₁₋₃ alkyl), C₁₋₈ haloalkyl (preferably C₁₋₆ haloalkyl, more preferably C₁₋₃ haloalkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy), -NHC₁₋₈ alkyl (preferably -NHC₁₋₆ alkyl, more preferably -NHC₁₋₃ alkyl), -N(C₁₋₈ alkyl)₂ (preferably -N(C₁₋₆ alkyl)₂, more preferably -N(C₁₋₃ alkyl)₂), -COC₁₋₈ alkyl (preferably -COC₁₋₆ alkyl, more preferably -COC₁₋₃ alkyl), -OCOC₁₋₈ alkyl (preferably -OCOC₁₋₆ alkyl, more preferably -OCOC₁₋₃ alkyl), -COOC₁₋₈ alkyl (preferably -COOC₁₋₆ alkyl, more preferably -COOC₁₋₃ alkyl), -CONH₂, -CONHC₁₋₈ alkyl (preferably -CONHC₁₋₆ alkyl, more preferably -CONHC₁₋₃ alkyl), -CON(C₁₋₈ alkyl)₂ (preferably -CON(C₁₋₆ alkyl)₂, more preferably -CON(C₁₋₃ alkyl)₂), -PO(C₁₋₈ alkyl)₂ (preferably -PO(C₁₋₆ alkyl)₂, more preferably -PO(C₁₋₃ alkyl)₂), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), -OSO₂C₁₋₈ alkyl (preferably -OSO₂C₁₋₆ alkyl, more preferably -OSO₂C₁₋₃ alkyl), -SC₁₋₆ alkyl, -SO₂NH₂, -SO₂NHC₁₋₈ alkyl (preferably -SO₂NHC₁₋₆ alkyl, more preferably -SO₂NHC₁₋₃ alkyl), -SO₂N(C₁₋₈ alkyl)₂ (preferably -SO₂N(C₁₋₆ alkyl)₂, more preferably -SO₂N(C₁₋₃ alkyl)₂), C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl); the C₁₋₈ alkyl, C₁₋₈ haloalkyl, C₁₋₈ alkoxy, C₁₋₈ haloalkoxy, -NHC₁₋₈ alkyl, -N(C₁₋₈ alkyl)₂, -COC₁₋₈ alkyl, -OCOC₁₋₈ alkyl, -COOC₁₋₈ alkyl, -CONH₂, -CONHC₁₋₈ alkyl, -CON(C₁₋₈ alkyl)₂, -PO(C₁₋₈ alkyl)₂, -SOC₁₋₈ alkyl, -SO₂C₁₋₈ alkyl, -OSO₂C₁₋₈ alkyl, -SO₂NH₂, -SO₂NHC₁₋₈ alkyl, -SO₂N(C₁₋₈ alkyl)₂, C₃₋₈ cycloalkyl, 3- to 12-membered heterocycloalkyl, 5- to 10-membered heteroaryl and C₆₋₁₄ aryl are unsubstituted or substituted with 1, 2 or 3 substituents selected from the group consisting of halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, NHC₁₋₆ alkyl, -N(C₁₋₆ alkyl)₂, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -PO(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl);
or R₁, R₂ and carbon atom linked to R₁ and R₂ together form a 6- to 15-membered heterocycloalkyl ring; the 6- to 15-membered heterocycloalkyl ring is unsubstituted or substituted with 1, 2, 3 or 4 substituents selected from the group consisting of halogen, cyano, hydroxyl, carboxyl, nitro, formyl, sulfo, C₁₋₆ alkyl, C₁₋₆ haloalkyl, C₁₋₆ alkoxy, C₁₋₆ haloalkoxy, -NHC₁₋₆ alkyl, -N(C₁₋₆ alkyl)₂, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -PO(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₈ cycloalkyl (preferably C₃₋₆ cycloalkyl), 3- to 12-membered heterocycloalkyl (preferably 4- to 8-membered heterocycloalkyl, more preferably 4- to 6-membered heterocycloalkyl), 5- to 10-membered heteroaryl (preferably 5- to 6-membered heteroaryl) and C₆₋₁₄ aryl (preferably C₆₋₁₂ aryl);
(R₃)ₙ₃ represents substitution of hydrogen on ring A2 by n3 R₃, n3 is 0, 1, 2 or 3, each R₃ is identical or different, and each R₃ is independently selected from the group consisting of X₁, hydrogen, deuterium, cyano, carboxyl, nitro, formyl, sulfo, halogen, oxo, C₁₋₁₀ alkyl (preferably C₁₋₈ alkyl, more preferably C₁₋₆ alkyl, further preferably C₁₋₃ alkyl), C₁₋₁₀ haloalkyl (preferably C₁₋₈ haloalkyl, more preferably C₁₋₆ haloalkyl, further preferably C₁₋₃ haloalkyl), C₁₋₁₀ alkoxy (preferably C₁₋₈ alkoxy, more preferably C₁₋₆ alkoxy, further preferably C₁₋₃ alkoxy), C₁₋₁₀ haloalkoxy (preferably C₁₋₈ haloalkoxy, more preferably C₁₋₆ haloalkoxy, further preferably C₁₋₃ haloalkoxy), -COC₁₋₁₀ alkyl (preferably -COC₁₋₈ alkyl, more preferably -COC₁₋₆ alkyl, further preferably -COC₁₋₃ alkyl), -OCOC₁₋₁₀ alkyl (preferably -OCOC₁₋₈ alkyl, more preferably -OCOC₁₋₆ alkyl, further preferably -OCOC₁₋₃ alkyl), -COOC₁₋₁₀ alkyl (preferably -COOC₁₋₈ alkyl, more preferably -COOC₁₋₆ alkyl, further preferably -COOC₁₋₃ alkyl), -CONH₂, -CONHC₁₋₁₀ alkyl (preferably -CONHC₁₋₈ alkyl, more preferably -CONHC₁₋₆ alkyl, further preferably -CONHC₁₋₃ alkyl), -CON(C₁₋₁₀ alkyl)₂ (preferably -CON(C₁₋₈ alkyl)₂, more preferably -CON(C₁₋₆ alkyl)₂, further preferably -CON(C₁₋₃ alkyl)₂), -SOC₁₋₁₀ alkyl (preferably -SOC₁₋₈ alkyl, more preferably -SOC₁₋₆ alkyl, further preferably -SOC₁₋₃ alkyl), -SO₂C₁₋₁₀ alkyl (preferably -SO₂C₁₋₈ alkyl, more preferably -SO₂C₁₋₆ alkyl, further preferably -SO₂C₁₋₃ alkyl), -OSO₂C₁₋₁₀ alkyl (preferably -OSO₂C₁₋₈ alkyl, more preferably -OSO₂C₁₋₆ alkyl, further preferably -OSO₂C₁₋₃ alkyl), -SO₂NH₂, -SO₂NHC₁₋₁₀ alkyl (preferably -SO₂NHC₁₋₈ alkyl, more preferably -SO₂NHC₁₋₆ alkyl, further preferably -SO₂NHC₁₋₃ alkyl), -SO₂N(C₁₋₁₀ alkyl)₂ (preferably -SO₂N(C₁₋₈ alkyl)₂, more preferably -SO₂N(C₁₋₆ alkyl)₂, further preferably -SO₂N(C₁₋₃ alkyl)₂), C₃₋₈ cycloalkyl (preferably C₃₋₆ cycloalkyl), 3- to 15-membered heterocycloalkyl (preferably 4- to 12-membered heterocycloalkyl, more preferably 4- to 8-membered heterocycloalkyl, further preferably 4- to 6-membered heterocycloalkyl), 5- to 10-membered heteroaryl (preferably 5- to 6-membered heteroaryl) and C₆₋₁₄ aryl; the C₁₋₁₀ alkyl, C₁₋₁₀ haloalkyl, C₁₋₁₀ alkoxy, C₁₋₁₀ haloalkoxy, -COC₁₋₁₀ alkyl, -OCOC₁₋₁₀ alkyl, -COOC₁₋₁₀ alkyl, -CONH₂, -CONHC₁₋₁₀ alkyl, -CON(C₁₋₁₀ alkyl)₂, -SOC₁₋₁₀ alkyl, -SO₂C₁₋₁₀ alkyl, -OSO₂C₁₋₁₀ alkyl, -SO₂NH₂, -SO₂NHC₁₋₁₀ alkyl, -SO₂N(C₁₋₁₀ alkyl)₂, C₃₋₈ cycloalkyl, 3- to 15-membered heterocycloalkyl, 5- to 10-membered heteroaryl and C₆₋₁₄ aryl are unsubstituted or substituted with 1, 2 or 3 substituents selected from the group consisting of cyano, hydroxyl, carboxyl, nitro, formyl, sulfo, halogen, C₁₋₆ alkyl, C₁₋₆ haloalkyl, C₁₋₆ alkoxy, C₁₋₆ haloalkoxy, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, C₃₋₆ cycloalkyl, 3- to 15-membered heterocycloalkyl (preferably 4- to 12-membered heterocycloalkyl, more preferably 4- to 8-membered heterocycloalkyl, further preferably 4- to 6-membered heterocycloalkyl), 5- to 10-membered heteroaryl (preferably 5- to 6-membered heteroaryl), phenyl and naphthyl;
X₁ in R₂ and R₃ is a point of attachment of POI to L or ULM, and at least one of R₂ and R₃ is X₁.

3. The compound of formula (I) according to claim 2, or a pharmaceutically acceptable salt thereof, or a stereoisomer thereof, wherein ring A1 is an 8- to 10-membered heteroaryl ring, a 5- to 6-membered heterocycloalkyl ring or an 18-membered heterocycloalkyl ring;
preferably, ring A1 is a 9-membered heteroaryl ring or a 10-membered heteroaryl ring;
preferably, ring A1 is a 9-membered nitrogen-containing heteroaryl ring or a 10-membered nitrogen-containing heteroaryl ring;
preferably, ring A1 is selected from the group consisting of an indolizine ring, a pyrazolopyridine ring, an imidazopyridine ring, a triazolopyridine ring, a tetrazolopyridine ring, a pyrrolopyridazine ring, a pyrazolopyridazine ring, an imidazopyridazine ring, a triazolopyridazine ring, a tetrazolopyridazine ring, a pyrrolopyrimidine ring, a pyrazolopyrimidine ring, an imidazopyrimidine ring, a triazolopyrimidine ring, a tetrazolopyrimidine ring, a pyrrolopyrazine ring, a pyrazolopyrazine ring, an imidazopyrazine ring, a triazolopyrazine ring, a pyridopyridine ring, a pyridopyrazine ring, a pyridopyridazine ring, a pyridotriazine ring, a pyridopyrimidine ring and a tetrazolopyrazine ring;
preferably, ring A1 is selected from the group consisting of [1,2,4]triazolo[4,3-c]pyrimidine, imidazo[1,5-c]pyrimidine, imidazo[1,2-c]pyrimidine, [1,2,4]triazolo[4,3-a]pyridine and pyrido[3,4-d]pyridazine;
preferably, ring A1 is selected from the group consisting of pyrimidin-4(3H)-one, 1,6-dihydropyrimidine, pyridin-2(1H)-one, 1,2-dihydropyridine and 3,8-diazabicyclo[3.2.1]octane;
preferably, ring A1 is a partially unsaturated 18-membered heterocycloalkyl ring;
preferably, ring A1 is selected from the group consisting of 2,3-dihydro-7'H-spiro[indene-1,8'-pyrido[3,4-d]pyridazin]-7'-one and 2,3-dihydro-7'H-spiro[indene-1,8'-pyrido[4,3-c]pyridazin]-7'-one.

4. The compound of formula (I) according to claim 2, or a pharmaceutically acceptable salt thereof, or a stereoisomer thereof, wherein 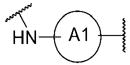 is selected from the group consisting of the following structures or isomers thereof: 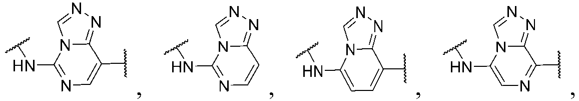 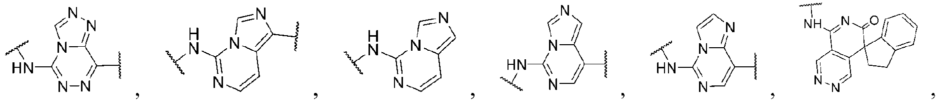 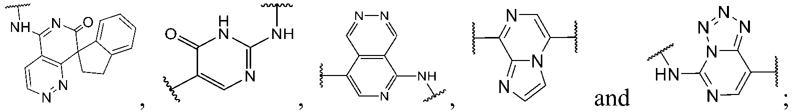
preferably, 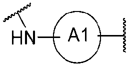 is selected from the group consisting of the following structures or isomers thereof: 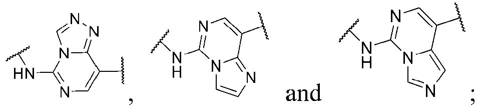
preferably, 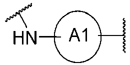 is selected from the group consisting of the following structures or isomers thereof: 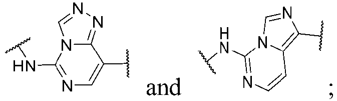
preferably, 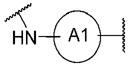 is selected from the group consisting of the following structures or isomers thereof:

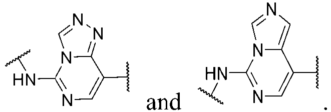

5. The compound of formula (I) according to claim 2, or a pharmaceutically acceptable salt thereof, or a stereoisomer thereof, wherein POI is a structure of formula (A-2-1) or an isomer thereof: 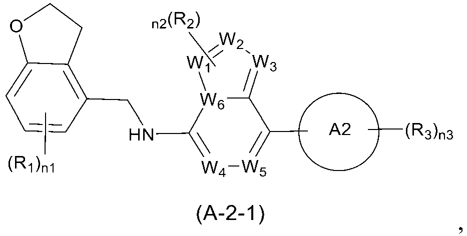
wherein, W₁, W₂, W₃, W₄, W₅ and W₆ are each independently CH or N, ring A2, R₁, n1, R₂, n2, R₃ and n3 are as defined in claim 2, and at least one of R₂ and R₃ is X₁;
preferably, POI is a structure of formula (A-2-2) or an isomer thereof: 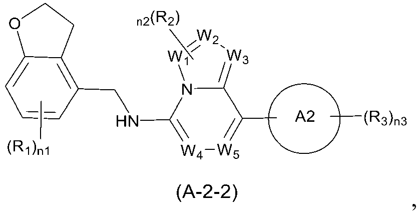
wherein, W₁, W₂, W₃, W₄ and W₅ are each independently CH or N; ring A2, R₁, n1, R₂, n2, R₃ and n3 are as defined in claim 2, and at least one of R₂ and R₃ is X₁.

6. The compound of formula (I) according to claim 2, or a pharmaceutically acceptable salt thereof, or a stereoisomer thereof, wherein POI is selected from the group consisting of the following structures or isomers thereof: 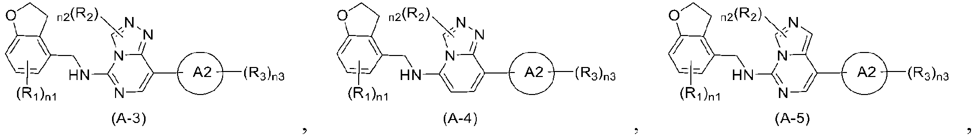 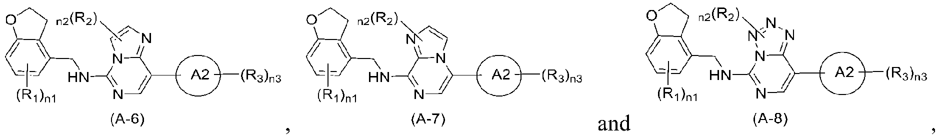 wherein, ring A2, R₁, n1, R₂, n2, R₃ and n3 are as defined in claim 2, and at least one of R₂ and R₃ is X₁.

7. The compound of formula (I) according to claim 2, or a pharmaceutically acceptable salt thereof, or a stereoisomer thereof, wherein ring A2 is absent or is selected from the group consisting of a 4- to 8-membered heterocycloalkyl ring, a C₄₋₈ cycloalkyl ring, a 5- to 10-membered heteroaryl ring, a 10- to 15-membered heterocycloalkyl ring and a benzene ring;
preferably, ring A2 is absent or is selected from the group consisting of a 4- to 8-membered heterocycloalkyl ring, a saturated C₄₋₈ cycloalkyl ring, a 5- to 10-membered heteroaryl ring, a partially unsaturated 10- to 15-membered heterocycloalkyl ring and a benzene ring;
preferably, ring A2 is absent, or is selected from the group consisting of a cyclobutane ring, a cyclopentane ring, a cyclohexane ring, an azetidine ring, a tetrahydropyrrole ring, a pyrazolidine ring, an imidazolidine ring, a piperidine ring, a hexahydropyridazine ring, a hexahydropyrimidine ring, a piperazine ring, a benzene ring, a pyrazole ring, an oxazole ring, an imidazole ring, a triazole ring, a tetrazole ring, a pyridine ring, a pyrimidine ring, a pyrazine ring, a pyridazine ring, an indolizine ring, pyrazolopyridine, imidazopyridine, triazolopyridine, tetrazolopyridine, pyrrolopyridazine, pyrazolopyridazine, imidazopyridazine, triazolopyridazine, tetrazolopyridazine, pyrrolopyrimidine, pyrazolopyrimidine, imidazopyrimidine, triazolopyrimidine, tetrazolopyrimidine, pyrrolopyrazine, pyrazolopyrazine, imidazopyrazine, triazolopyrazine, tetrazolopyrazine, pyridopyridine, pyridopyrazine, pyridopyridazine, pyridotriazine, pyridopyrimidine, 3,8-diazabicyclo[3.2.1]octane and 2,3-dihydrospiro[indene-1,4'-piperidine];
preferably, ring A2 is absent or is selected from the group consisting of a saturated 6-membered heterocycloalkyl ring, a partially unsaturated 12-membered heterocycloalkyl ring, a cyclohexane ring, a 5- to 6-membered heteroaryl ring, a 9-membered heteroaryl ring and a benzene ring;
preferably, ring A2 is absent or is selected from the group consisting of a cyclohexane ring, a piperidine ring, a piperazine ring, a benzene ring, a pyridine ring, a pyrazole ring, a triazolopyridine ring and 2,3-dihydrospiro[indene-1,4'-piperidine];
preferably, ring A2 is selected from the group consisting of a benzene ring, a pyridine ring and a pyrazole ring.

8. The compound of formula (I) according to claim 2, or a pharmaceutically acceptable salt thereof, or a stereoisomer thereof, wherein 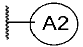 is selected from the group consisting of the following structures or isomers thereof: 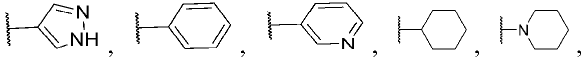 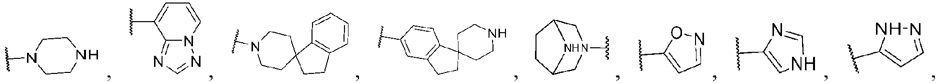 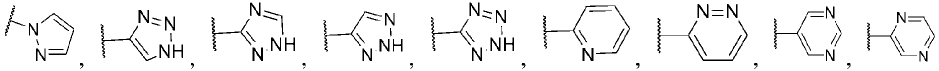 and 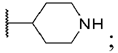
preferably, 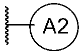 is selected from the group consisting of the following structures: 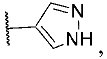 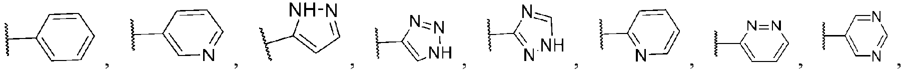 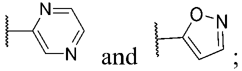
preferably, 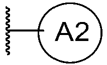
is 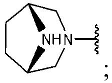
preferably, 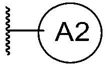 is selected from the group consisting of the following structures or isomers thereof: 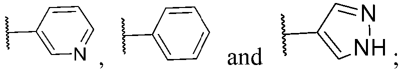
preferably, 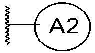 is selected from the group consisting of the following structures or isomers thereof:

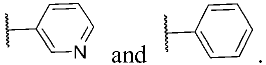

9. The compound of formula (I) according to claim 2, or a pharmaceutically acceptable salt thereof, or a stereoisomer thereof, wherein R₁ is selected from the group consisting of hydrogen, deuterium, fluorine, chlorine, bromine, iodine, methyl, ethyl, propyl, isopropyl, monofluoromethyl, difluoromethyl, trifluoromethyl, monofluoroethyl, difluoroethyl, trifluoroethyl, methoxy, ethoxy, propoxy, isopropoxy, monofluoromethoxy, difluoromethoxy, trifluoromethoxy, monofluoroethoxy, difluoroethoxy, trifluoroethoxy, cyclopropyl, cyclobutyl, cyclopentyl, cyclohexyl, cyclohexenyl, pyrrolyl, pyrazolyl, pyridinyl, phenyl, pyrimidinyl, -CH₂-cyclopropyl, -CH₂-tetrahydropyrrolyl, -CH₂-pyrrolyl, -CH₂-phenyl, -CH₂-pyridinyl, -CH₂-cyclohexenyl, -CH₂-azetidine, -CH₂-piperidine, -CH₂-piperazine, tetrahydro-2H-pyran and -CH₂-(tetrahydro-2H-pyran); or two R₁ and carbon atom linked to the two R₁ together form a cyclopropyl ring, a cyclobutyl ring, a cyclopentyl ring, a cyclohexyl ring, a cyclobutene ring, a cyclopentene ring, a cyclohexene ring, an azetidine ring, a tetrahydropyrrole ring, a piperidine ring or a piperazine ring;
preferably, R₁ is selected from the group consisting of hydrogen, deuterium, fluorine, chlorine, bromine, iodine, -CH₃, -CH₂CH₃, -CH(CH₃)₂, -CH₂CH₂CH₃, -CH₂F, -CHF₂, -CF₃, -OCH₃, -OCH₂CH₃, -OCH₂CH₂CH₃, -OCH(CH₃)₂, -OCH₂F, -OCHF₂, -OCF₃, -CH₂CH₂F, -CH₂CHF₂, -CH₂CF₃, -OCH₂CH₂F, -OCH₂CHF₂ and -OCH₂CF₃;
preferably, two R₁ and carbon atom linked to the two R₁ together form a cyclopropyl ring;
preferably, R₁ is fluorine.

10. The compound of formula (I) according to claim 2, or a pharmaceutically acceptable salt thereof, or a stereoisomer thereof, wherein 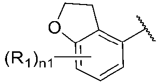 is 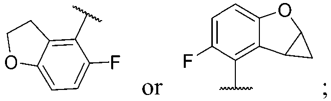 preferably, 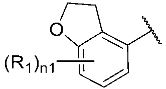 is 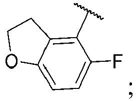 preferably, 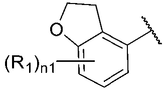 is

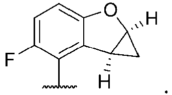

11. The compound of formula (I) according to claim 2, or a pharmaceutically acceptable salt thereof, or a stereoisomer thereof, wherein R₂ is selected from the group consisting of X₁, hydrogen, deuterium, halogen, cyano, carboxyl, hydroxyl, amino, oxo, -NHC₁₋₆ alkyl, -N(C₁₋₆ alkyl)₂, -CONH₂, -CONHC₁₋₆ alkyl, -CON(C₁₋₆ alkyl)₂, -COC₁₋₆ alkyl, -COOC₁₋₆ alkyl, -OCOC₁₋₆ alkyl, -PO(C₁₋₆ alkyl)₂, -SOC₁₋₆ alkyl, -SO₂C₁₋₆ alkyl, -OSO₂C₁₋₆ alkyl, -SC₁₋₆ alkyl, C₁₋₆ alkyl, C₁₋₆ alkoxy, C₁₋₆ haloalkyl and C₁₋₆ haloalkoxy;
preferably, R₂ is selected from the group consisting of X₁, hydrogen, deuterium, halogen, cyano, carboxyl, hydroxyl, amino, oxo, -NHC₁₋₆ alkyl, -N(C₁₋₆ alkyl)₂, -CONH₂, -CONHC₁₋₆ alkyl, -CON(C₁₋₆ alkyl)₂, -COC₁₋₃ alkyl, -COOC₁₋₃ alkyl, -OCOC₁₋₃ alkyl, -PO(C₁₋₃ alkyl)₂, -SOC₁₋₃ alkyl, -SO₂C₁₋₃ alkyl, -OSO₂C₁₋₃ alkyl, -SC₁₋₃ alkyl, C₁₋₃ alkyl, C₁₋₃ alkoxy, C₁₋₃ haloalkyl and C₁₋₃ haloalkoxy;
preferably, R₂ is selected from the group consisting of X₁, hydrogen, deuterium, halogen, cyano, carboxyl, hydroxyl, amino, oxo, -NHCH₃, -N(CH₃)₂, -CONH₂, -CONHCH₃, -CON(CH₃)₂, -COCH₃, -COC(CH₃)₃, -COOCH₃, -COOCH₂CH₃, -COOC(CH₃)₃, -OCOCH₃, -OCOCH₂CH₃, -OCOC(CH₃)₃, -PO(CH₃)₂, -PO(CH₂CH₃)₂, -SOCH₃, -SOCH₂CH₃, -SOC(CH₃)₃, -SO₂CH₃, -SO₂CH₂CH₃, -SO₂C(CH₃)₃, -OSO₂CH₃, -OSO₂CH₂CH₃, -OSO₂C(CH₃)₃, -SCH₃, -SCH₂CH₃, methyl, ethyl, propyl, isopropyl, tert-butyl, methoxy, ethoxy, propoxy, isopropoxy, tert-butoxy, monofluoromethyl, difluoromethyl, trifluoromethyl, monofluoroethyl, difluoroethyl, trifluoroethyl, monofluoropropyl, difluoropropyl, trifluoropropyl, monofluoroisopropyl, difluoroisopropyl, trifluoroisopropyl, monofluoro-tert-butyl, difluoro-tert-butyl, trifluoro-tert-butyl, monofluoromethoxy, difluoromethoxy, trifluoromethoxy, monofluoroethoxy, difluoroethoxy, trifluoroethoxy, monofluoropropoxy, difluoropropoxy, trifluoropropoxy, monofluoroisopropoxy, difluoroisopropoxy, trifluoroisopropoxy, monofluoro-tert-butoxy, difluoro-tert-butoxy and trifluoro-tert-butoxy;
preferably, R₂ is selected from the group consisting of cyano, fluorine, chlorine, bromine, iodine, -CH₃, -CF₃, -SO₂CH₃ and hydrogen;
preferably, R₂ is selected from the group consisting of cyano, fluorine, chlorine, bromine, iodine, -SO₂CH₃ and hydrogen;
preferably, R₂ is selected from the group consisting of X₁, hydrogen, halogen, cyano, -SO₂C₁₋₆ alkyl and -OSO₂C₁₋₆ alkyl;
preferably, R₂ is selected from the group consisting of X₁, hydrogen, halogen, cyano, -SO₂C₁₋₃ alkyl and -OSO₂C₁₋₃ alkyl;
preferably, R₂ is selected from the group consisting of X₁, hydrogen, fluorine, chlorine, bromine, iodine, cyano, -SO₂CH₃ and -OSO₂CH₃.

12. The compound of formula (I) according to claim 2, or a pharmaceutically acceptable salt thereof, or a stereoisomer thereof, wherein 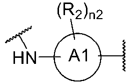 is selected from the group consisting of the following structures or isomers thereof: 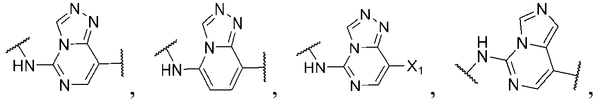 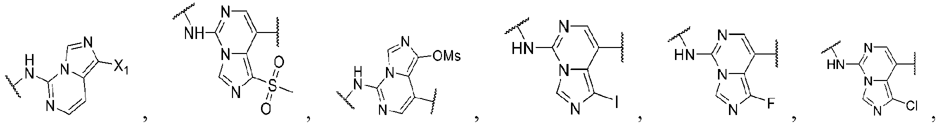 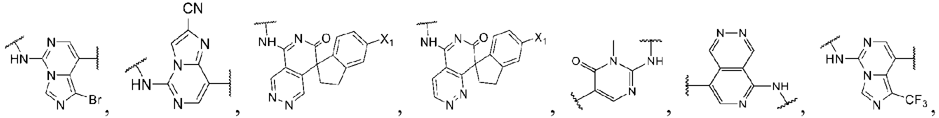 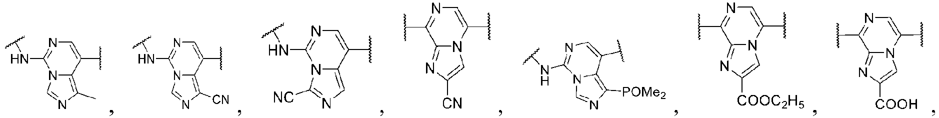 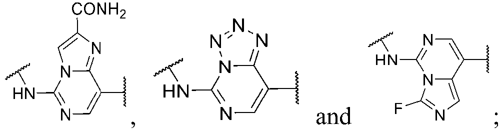 wherein X₁ is the point of attachment to L or ULM;
preferably, 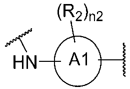 is selected from the group consisting of the following structures or isomers thereof: 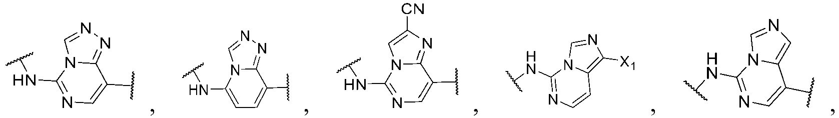 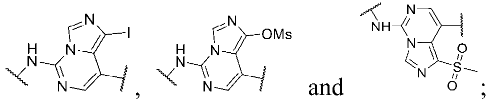 wherein X₁ is the point of attachment to L or ULM;
preferably, 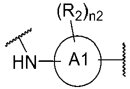 is selected from the group consisting of the following structures or isomers thereof: 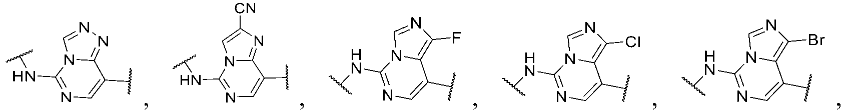 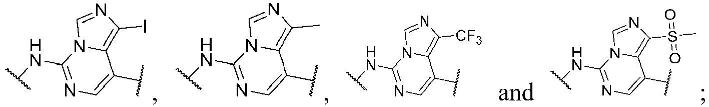
preferably, 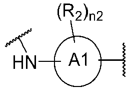 is selected from the group consisting of the following structures or isomers thereof: 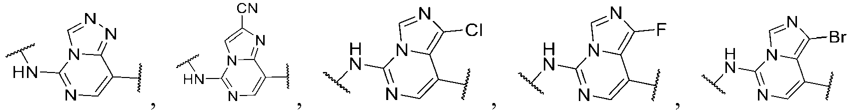 and 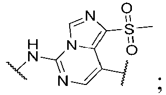
preferably, 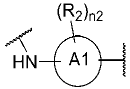 is selected from the group consisting of the following structures or isomers thereof:

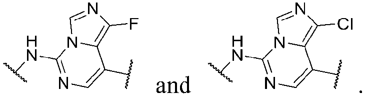

13. The compound of formula (I) according to claim 2, or a pharmaceutically acceptable salt thereof, or a stereoisomer thereof, wherein R₁, R₂ and carbon atom linked to R₁ and R₂ together form a 6- to 15-membered heterocycloalkyl ring, the 6- to 15-membered heterocycloalkyl ring is selected from the group consisting of 5,8-dihydro-4H-1,4-oxazocine, 4,5,8,9-tetrahydro-1,4-oxazepine, 5,8,9,10-tetrahydro-4H-1,4-oxazonine, and 1-oxa-4-azacycloundeca-2,6-diene; the 6- to 15-membered heterocycloalkyl ring is unsubstituted or substituted with 1, 2, 3 or 4 substituents selected from the group consisting of fluorine, chlorine, bromine, iodine, cyano, hydroxyl, carboxyl, nitro, formyl, sulfo, methyl, ethyl, isopropyl, tert-butyl, monofluoromethyl, difluoromethyl, trifluoromethyl, monofluoroethyl, difluoroethyl, trifluoroethyl, methoxy, ethoxy, isopropoxy, tert-butoxy, monofluoromethoxy, difluoromethoxy, trifluoromethoxy, monofluoroethoxy, difluoroethoxy, trifluoroethoxy, -COCH₃, -COCH₂CH₃, -COCH(CH₃)₂, -COC(CH₃)₃, -OCOCH₃, -OCOCH₂CH₃, -OCOCH(CH₃)₂, -OCOC(CH₃)₃, -CONH₂, -CONHCH₃, -CONHCH₂CH₃, -CONHCH(CH₃)₂, -CONHC(CH₃)₃, -CON(CH₃)₂, -CON(CH₂CH₃)₂, -SOCH₃, -SO₂CH₃, -SO₂CH₂CH₃, -SO₂CH(CH₃)₂, -SO₂C(CH₃)₃, -OSO₂CH₃, -OSO₂CH₂CH₃, -OSO₂CH(CH₃)₂, -OSO₂C(CH₃)₃, -SO₂NH₂, -SO₂NHCH₃, -SO₂NHCH₂CH₃, -SO₂NHCH(CH₃)₂, -SO₂NHC(CH₃)₃, -SO₂N(CH₃)₂, -SO₂N(CH₂CH₃)₂, cyclopropyl, cyclobutyl, cyclopentyl, cyclohexyl, cyclohexenyl, azetidinyl, tetrahydropyrrolyl, piperidinyl, piperazinyl, pyrrolyl, pyrazolyl, pyridinyl, phenyl and pyrimidinyl;
preferably, R₁, R₂ and carbon atom linked to R₁ and R₂ together form (2Z,6Z)-4,5,8,9-tetrahydro-1,4-oxazepine, (2E,6Z)-4,5,8,9-tetrahydro-1,4-oxazepine, (2E,6E)-4,5,8,9-tetrahydro-1,4-oxazepine, (2Z,6E)-4,5,8,9-tetrahydro-1,4-oxazepine, (2Z,6Z)-5,8-dihydro-4H-1,4-oxazocine, (2E,6Z)-5,8-dihydro-4H-1,4-oxazocine, (2Z,6E)-5,8-dihydro-4H-1,4-oxazocine, (2E,6E)-5,8-dihydro-4H-1,4-oxazocine, (2Z,6Z)-5,8,9,10-tetrahydro-4H-1,4-oxazonine, (2E,6Z)-5,8,9,10-tetrahydro-4H-1,4-oxazonine, (2Z,6E)-5,8,9,10-tetrahydro-4H-1,4-oxazonine, (2E,6E)-5,8,9,10-tetrahydro-4H-1,4-oxazonine, (2Z,6Z)-1-oxa-4-azacycloundeca-2,6-diene, (2E,6Z)-1-oxa-4-azacycloundeca-2,6-diene, (2Z,6E)-1-oxa-4-azacycloundeca-2,6-diene, or (2E,6E)-1-oxa-4-azacycloundeca-2,6-diene;
preferably, R₁, R₂ and carbon atom linked to R₁ and R₂ together form (2Z,6Z)-4,5,8,9-tetrahydro-1,4-oxazepine, (2Z,6Z)-5,8-dihydro-4H-1,4-oxazocine, (2Z,6Z)-5,8,9,10-tetrahydro-4H-1,4-oxazonine, or (2Z,6Z)-1-oxa-4-azacycloundeca-2,6-diene.

14. The compound of formula (I) according to claim 2, or a pharmaceutically acceptable salt thereof, or a stereoisomer thereof, wherein 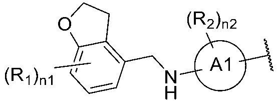 is selected from the group consisting of 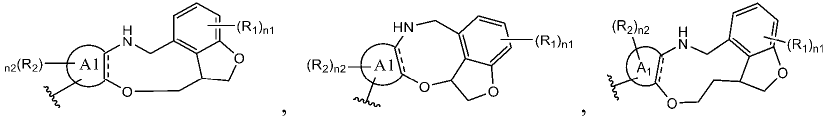 and 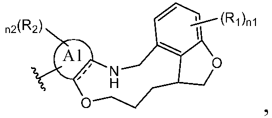 wherein -̅-̅-̅-̅-̅-̅ represents 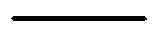 (single bond) or 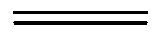 (double bond); n1 is 0 or 1, and n2 is 0 or 1;
preferably, 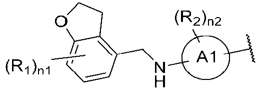 is selected from the group consisting of 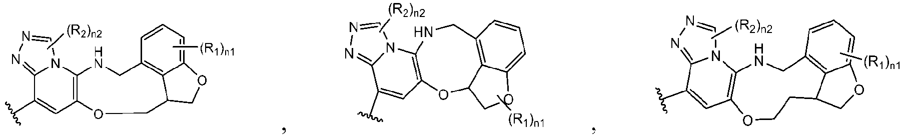 and 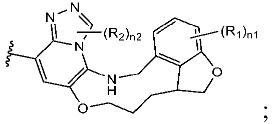 wherein n1 is 0 or 1, and n2 is 0 or 1;
preferably, 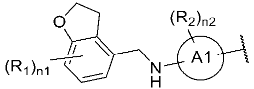 is selected from the group consisting of 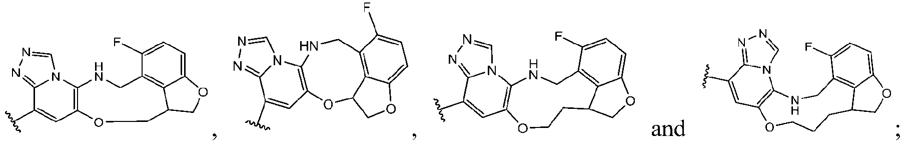 wherein n1 is 0 or 1, and n2 is 0 or 1;
preferably, 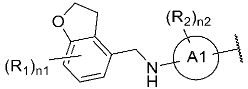 is selected from the group consisting of 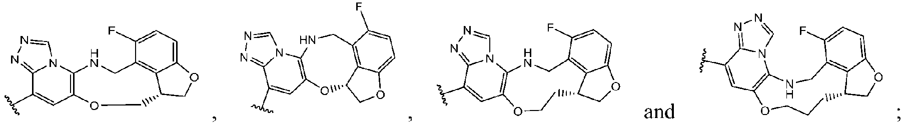 wherein n1 is 0 or 1, and n2 is 0 or 1.

15. The compound of formula (I) according to claim 2, or a pharmaceutically acceptable salt thereof, or a stereoisomer thereof, wherein R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl; the C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl are unsubstituted or substituted with 1, 2, or 3 substituents selected from the group consisting of cyano, hydroxyl, carboxyl, nitro, formyl, sulfo, halogen, C₁₋₃ alkyl, C₁₋₃ haloalkyl, C₁₋₃ alkoxy, C₁₋₃ haloalkoxy, -COC₁₋₃ alkyl, -COOC₁₋₃ alkyl, -CONH₂, -CONHC₁₋₃ alkyl, -CON(C₁₋₃ alkyl)₂, -SOC₁₋₃ alkyl, -SO₂C₁₋₃ alkyl, -OSO₂C₁₋₃ alkyl, -SO₂NH₂, -SO₂NHC₁₋₃ alkyl, -SO₂N(C₁₋₃ alkyl)₂, C₃₋₆ cycloalkyl, 4- to 6-membered heterocycloalkyl, 5- to 6-membered heteroaryl and phenyl;
preferably, R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl; the C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl are unsubstituted or substituted with 1, 2, or 3 substituents selected from the group consisting of cyano, hydroxyl, carboxyl, nitro, formyl, sulfo, halogen, methyl, trifluoromethyl, methoxy, trifluoromethoxy, -COCH₃, -OCOCH₃, -COOCH₃, -CONH₂, -CONHCH₃, -CON(CH₃)₂, -SOCH₃, -SO₂CH₃, -SO₂NH₂, -SO₂NHCH₃, -SO₂N(CH₃)₂, cyclopropyl, cyclobutyl, cyclopentyl, cyclohexyl, cyclobutenyl, cyclopentenyl, cyclohexenyl, azetidinyl, tetrahydropyrrolyl, piperidinyl, piperazinyl, pyrrolyl, pyrazolyl, imidazolyl, triazolyl, pyridinyl, pyrimidinyl, pyridazinyl and phenyl;
preferably, R₃ is selected from the group consisting of X₁, halogen, C₁₋₆ alkyl, C₁₋₆ alkoxy, C₁₋₆ haloalkyl, C₁₋₆ haloalkoxy, C₃₋₈ cycloalkyl, -COC₁₋₆ alkyl, -OCOC₁₋₆ alkyl, -COOC₁₋₆ alkyl, -CONH₂, -CONHC₁₋₆ alkyl, -CON(C₁₋₆ alkyl)₂, -SOC₁₋₆ alkyl, -SO₂C₁₋₆ alkyl, -OSO₂C₁₋₆ alkyl, -SO₂NH₂, -SO₂NHC₁₋₆ alkyl, -SO₂N(C₁₋₆ alkyl)₂, 4- to 8-membered heterocycloalkyl, 5- to 6-membered heteroaryl and phenyl;
preferably, R₃ is selected from the group consisting of X₁, fluorine, chlorine, bromine, iodine, methyl, ethyl, n-propyl, isopropyl, tert-butyl, methoxy, ethoxy, isopropoxy, tert-butoxy, monofluoromethyl, difluoromethyl, trifluoromethyl, monofluoroethyl, difluoroethyl, trifluoroethyl, monofluoromethoxy, difluoromethoxy, trifluoromethoxy, monofluoroethoxy, difluoroethoxy, trifluoroethoxy, -COCH₃, -COCH₂CH₃, -COC(CH₃)₃, -OCOCH₃, -OCOCH₂CH₃, -OCOC(CH₃)₃, -COOCH₃, -COOCH₂CH₃, -COOC(CH₃)₃, -CONH₂, -CONHCH₃, -CON(CH₃)₂, -SOCH₃, -SO₂CH₃, -SO₂C(CH₃)₃, -OSO₂CH₃, -OSO₂C(CH₃)₃, -SO₂NH₂, -SO₂NHCH₃, -SO₂N(CH₃)₂, cyclopropyl, cyclobutyl, cyclobutenyl, cyclopentyl, cyclopentenyl, cyclohexyl, cyclohexenyl, azetidinyl, piperazinyl, piperidinyl, 1,2,3,6-tetrahydropyridinyl, pyrrolyl, pyrazolyl, imidazolyl, pyridinyl, pyrimidinyl and phenyl;
preferably, R₃ is selected from the group consisting of X₁, C₁₋₆ alkyl, C₃₋₈ cycloalkyl, C₁₋₆ haloalkyl and -COC₁₋₆ alkyl;
preferably, R₃ is selected from the group consisting of X₁, C₁₋₃ alkyl, C₃₋₆ cycloalkyl, C₁₋₃ haloalkyl and -COC₁₋₃ alkyl;
preferably, R₃ is selected from the group consisting of X₁, methyl, cyclopropyl, trifluoromethyl and -COCH₃;
preferably, R₃ is selected from the group consisting of X₁, fluorine and methyl.

16. The compound of formula (I) according to claim 2, or a pharmaceutically acceptable salt thereof, or a stereoisomer thereof, wherein 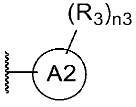 is selected from the group consisting of the following structures or isomers thereof: 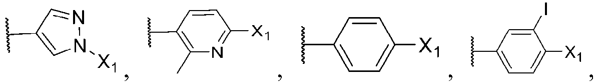 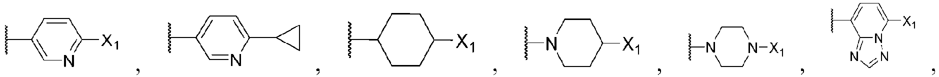 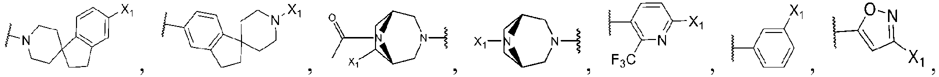 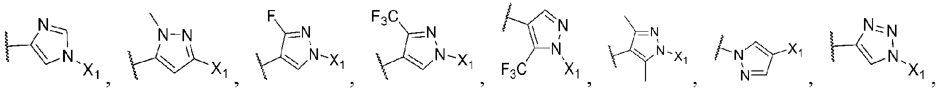 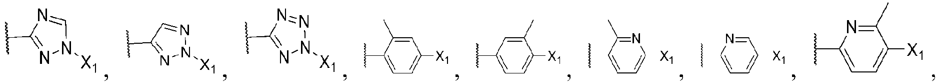 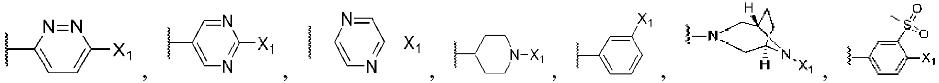 and 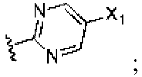 X₁ is the point of attachment to L or ULM;
preferably, 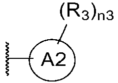 is selected from the group consisting of the following structures or isomers thereof: 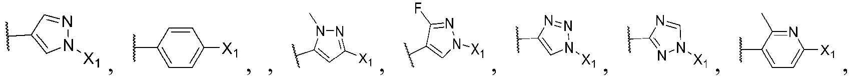 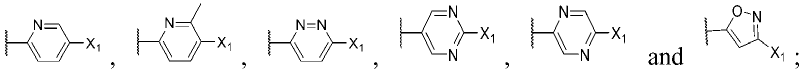 X₁ is the point of attachment to L or ULM;
preferably, 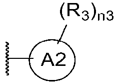 is selected from the group consisting of the following structures or isomers thereof: 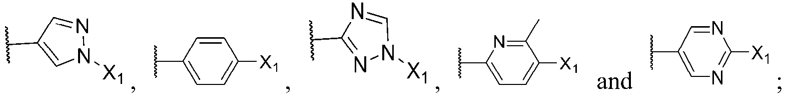 X₁ is the point of attachment to L or ULM;
preferably, 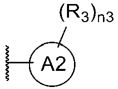 is selected from the group consisting of the following structures or isomers thereof: 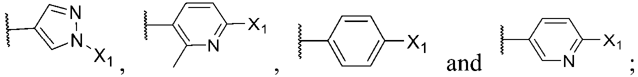 X₁ is the point of attachment to L or ULM;
preferably, 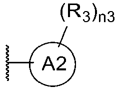 is selected from the group consisting of the following structures or isomers thereof: 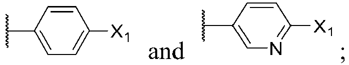 X₁ is the point of attachment to L or ULM.

17. The compound of formula (I) according to claim 2, or a pharmaceutically acceptable salt thereof, or a stereoisomer thereof, wherein POI is selected from the group consisting of the following structures or isomers thereof: 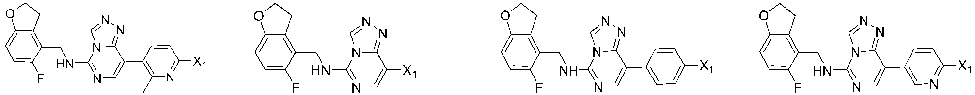 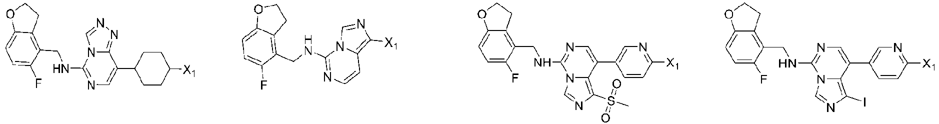 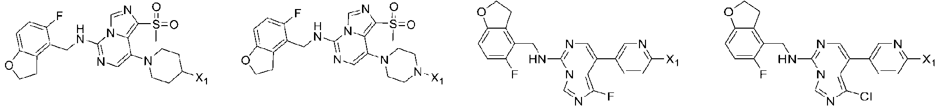 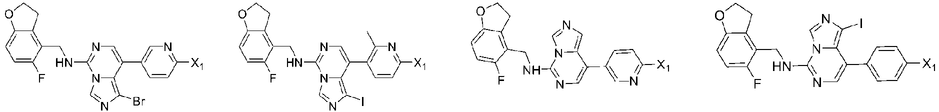 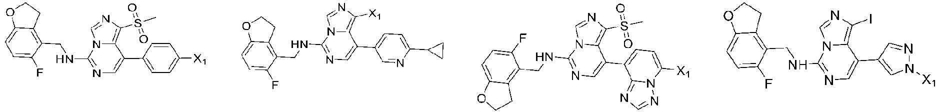 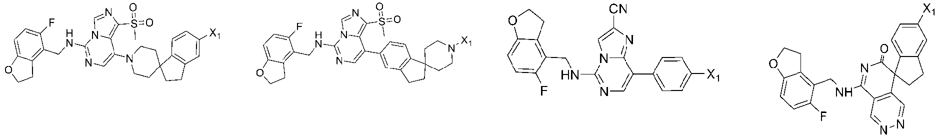 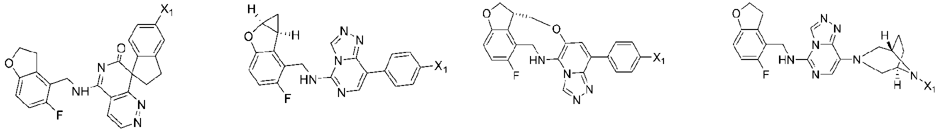 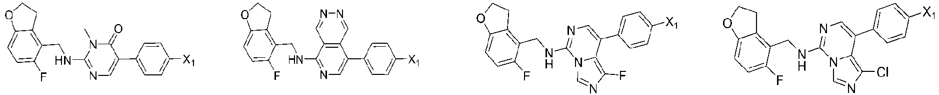 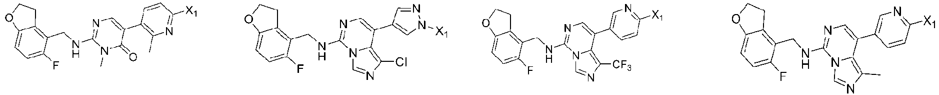 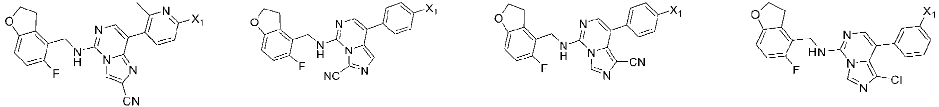 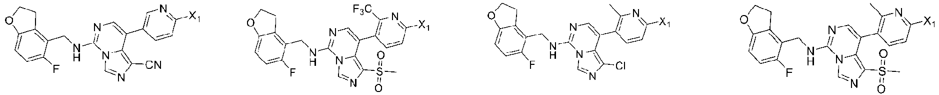 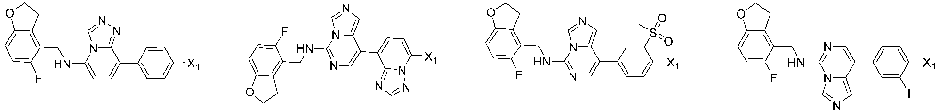 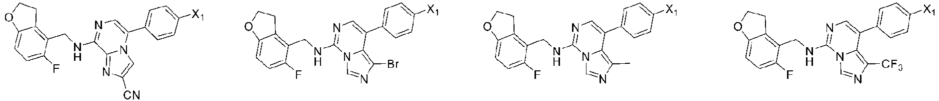 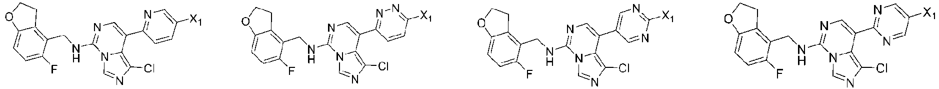 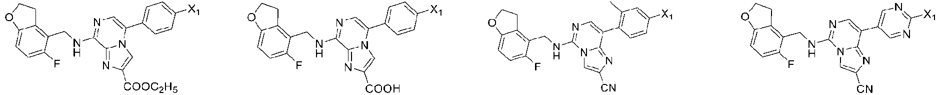 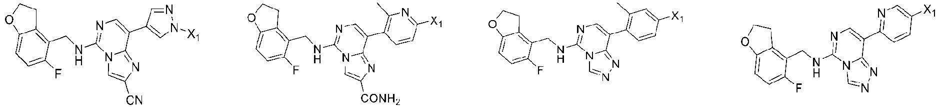 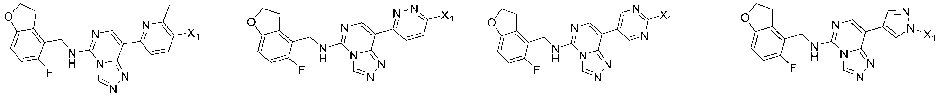 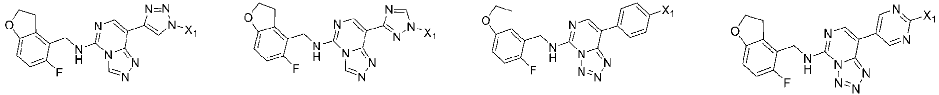 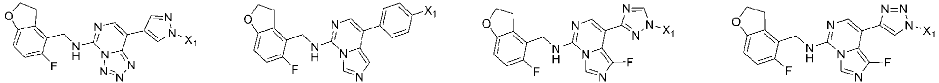 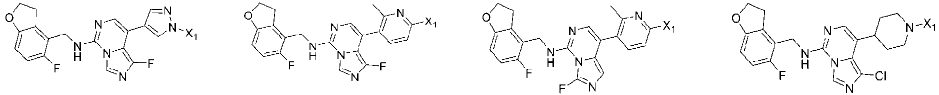 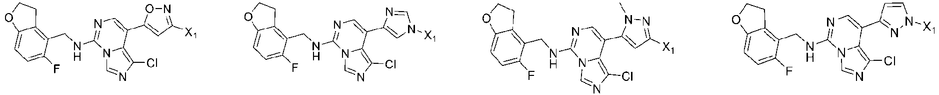 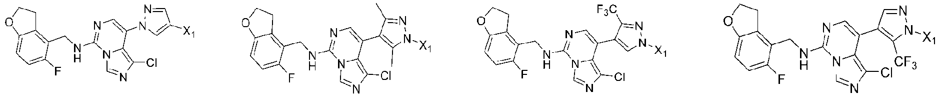 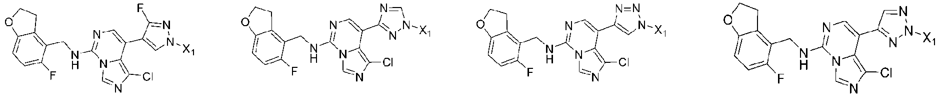 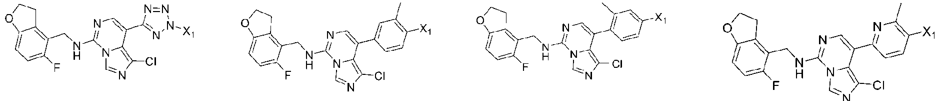 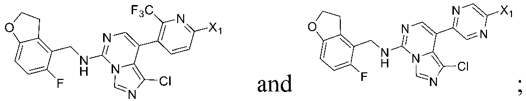
wherein X₁ is the point of attachment to L or ULM;
preferably, POI is selected from the group consisting of the following structures or isomers thereof: 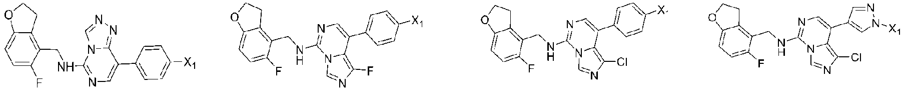 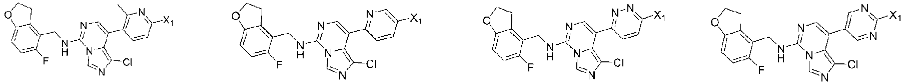 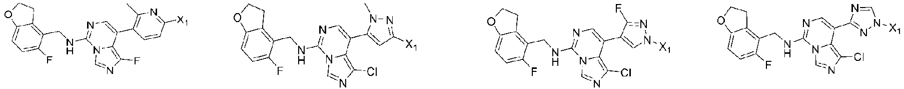 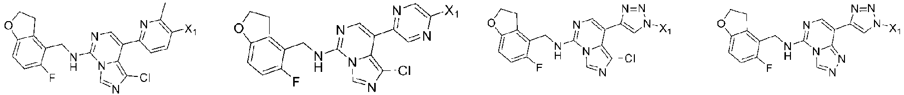 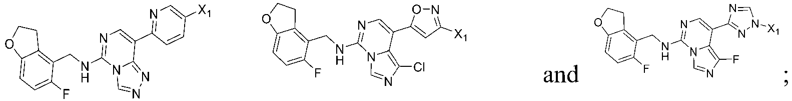
preferably, POI is selected from the group consisting of the following structures or isomers thereof: 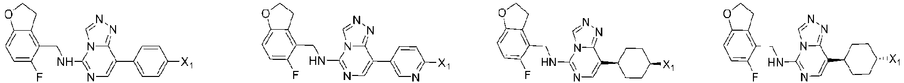 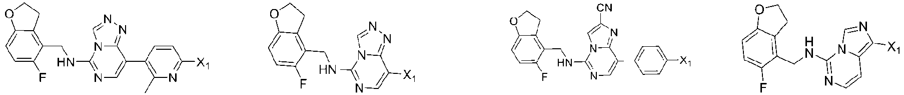 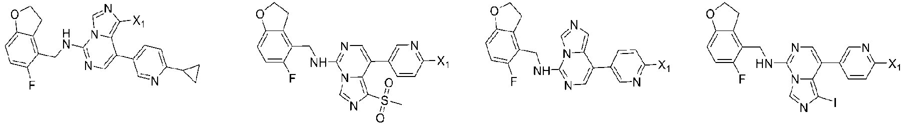 and 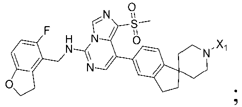 wherein X₁ is the point of attachment to L or ULM;
preferably, POI is selected from the group consisting of the following structures or isomers thereof: 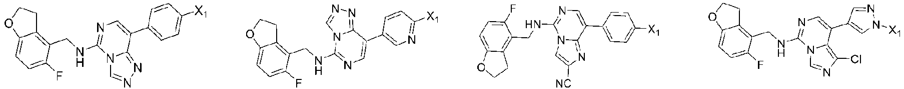 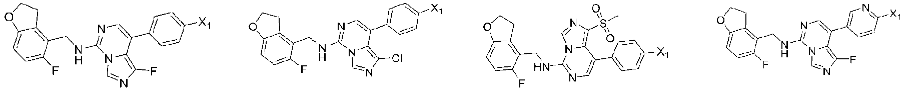 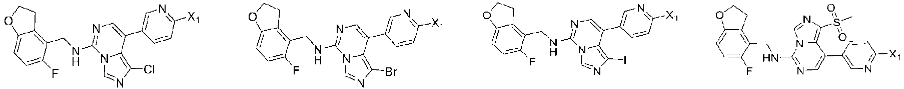 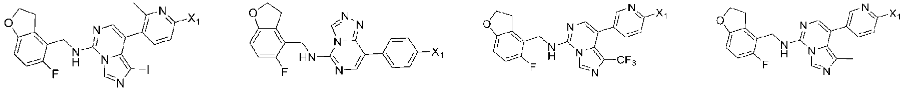 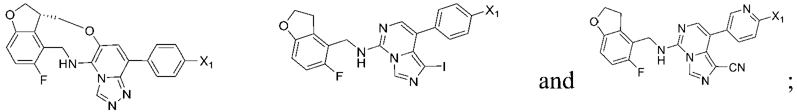
wherein X₁ is the point of attachment to L or ULM;
preferably, POI is selected from the group consisting of the following structures or isomers thereof: 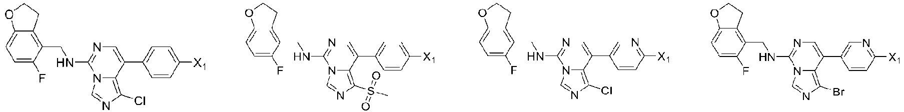 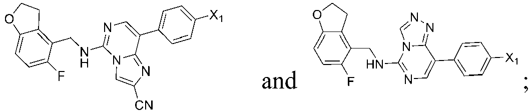
wherein X₁ is the point of attachment to L or ULM.

18. The compound of formula (I) according to claim 1, or a pharmaceutically acceptable salt thereof, or a stereoisomer thereof, wherein L is a structure of formula (L-1) or an isomer thereof,
-(Lₐ)ₘ₁- (L-1),
wherein m1 is independently 0, 1, 2, 3, 4, 5, 6, 7, 8, 9 or 10;
each Lₐ at each occurrence is independently selected from the group consisting of a chemical bond, -C(O)-, -C(O)NR_{L1}-, -NR_{L1}-, -O-, -S-, C₁₋₁₀ alkylene (preferably C₁₋₈ alkylene, more preferably C₁₋₆ alkylene, further preferably C₁₋₃ alkylene), C₁₋₁₀ alkyleneoxy (preferably C₁₋₈ alkyleneoxy, more preferably C₁₋₆ alkyleneoxy, further preferably C₁₋₃ alkyleneoxy), C₂₋₁₀ alkenylene (preferably C₂₋₈ alkenylene, more preferably C₂₋₆ alkenylene, further preferably C₂₋₄ alkenylene), C₂₋₁₀ alkynylene (preferably C₂₋₈ alkynylene, more preferably C₂₋₆ alkynylene, further preferably C₂₋₄ alkynylene), a C₃₋₁₅ cycloalkylene ring (preferably a C₃₋₁₀ cycloalkylene ring, more preferably a C₃₋₈ cycloalkylene ring, further preferably a C₃₋₆ cycloalkylene ring), a 3- to 15-membered heterocycloalkylene ring (preferably a 4- to 12-membered heterocycloalkylene ring, more preferably a 4- to 10-membered heterocycloalkylene ring, further preferably a 4- to 8-membered heterocycloalkylene ring, further preferably a 4- to 6-membered heterocycloalkylene ring), a 5- to 15-membered heteroarylene ring (preferably a 5- to 14-membered heteroarylene ring, more preferably a 5- to 12-membered heteroarylene ring, further preferably a 5- to 10-membered heteroarylene ring, further preferably a 5-to 6-membered heteroarylene ring) and a C₆₋₁₄ arylene ring (preferably a phenylene ring or a naphthylene ring); the C₁₋₁₀ alkylene, C₁₋₁₀ alkyleneoxy, C₂₋₁₀ alkenylene, C₂₋₁₀ alkynylene, C₃₋₁₅ cycloalkylene ring, 3- to 15-membered heterocycloalkylene ring, 5- to 15-membered heteroarylene ring and C₆₋₁₄ arylene ring are unsubstituted or substituted with 1, 2, 3 or 4 R_{L2}, each R_{L2} is independently selected from the group consisting of deuterium, halogen (preferably fluorine, chlorine or bromine), hydroxyl, cyano, amino, carboxyl, formyl, oxo, sulfo, C₁₋₆ alkyl, C₁₋₆ alkoxy, C₁₋₆ haloalkyl, C₁₋₆ haloalkoxy, hydroxy-substituted C₁₋₆ alkyl, cyano-substituted C₁₋₆ alkyl, amino-substituted C₁₋₆ alkyl, C₁₋₆ alkoxy C₁₋₆ alkyl, C₃₋₆ cycloalkyl, C₃₋₆ cycloalkyl C₁₋₆ alkyl, 3- to 6-membered heterocycloalkyl, 3- to 6-membered heterocycloalkyl C₁₋₆ alkyl, 5- to 6-membered heteroaryl, 5- to 6-membered heteroaryl C₁₋₆ alkyl, phenyl, phenyl C₁₋₆ alkyl, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO2C₁₋₆ alkyl, -SO₂NH₂, -SO₂NHC₁₋₆ alkyl and -SO₂N(C₁₋₆ alkyl)₂;
each R_{L1} at each occurrence is independently selected from the group consisting of hydrogen, deuterium, C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), C₁₋₈ haloalkoxy (preferably C₁₋₆ haloalkoxy, more preferably C₁₋₃ haloalkoxy) and C₁₋₈ haloalkyl (preferably C₁₋₆ haloalkyl, more preferably C₁₋₃ haloalkyl).

19. The compound of formula (I) according to claim 18, or a pharmaceutically acceptable salt thereof, or a stereoisomer thereof, wherein each R_{L1} at each occurrence is independently selected from the group consisting of hydrogen, methyl, ethyl, isopropyl, tert-butyl, methoxy, ethoxy, isopropoxy, tert-butoxy, trifluoromethyl, difluoromethyl, monofluoromethyl, trifluoroethyl, difluoroethyl, monofluoroethyl, trifluoromethoxy, difluoromethoxy, monofluoromethoxy, trifluoroethoxy, difluoroethoxy and monofluoroethoxy;
preferably, each R_{L1} at each occurrence is independently selected from the group consisting of hydrogen, methyl, ethyl, difluoromethyl and monofluoromethyl;
preferably, R_{L1} is hydrogen.

20. The compound of formula (I) according to claim 18, or a pharmaceutically acceptable salt thereof, or a stereoisomer thereof, wherein each R_{L2} is independently selected from the group consisting of deuterium, halogen (preferably fluorine, chlorine or bromine), hydroxyl, cyano, amino, carboxyl, hydroxymethyl, hydroxyethyl, methyl, ethyl, difluoromethyl, monofluoromethyl, trifluoromethyl, monofluoroethyl, difluoroethyl, trifluoroethyl, cyclopropyl, cyclobutyl, cyclopentyl, cyclohexyl, cyclohexenyl, cyclopentenyl, tetrahydropyrrolyl, tetrahydrofuranyl, phenyl, pyrrolyl, furanyl, thienyl, thiazolyl, oxazolyl, pyrazolyl, imidazolyl, triazolyl, tetrazolyl, pyridyl, pyrazinyl, pyrimidinyl, pyridazinyl, triazinyl, -CH₂-cyclopropyl, -CH₂-cyclobutyl, -CH₂-cyclopentyl, -CH₂-cyclohexyl, -CH₂-cyclohexenyl, -CH₂-cyclopentenyl, -CH₂-tetrahydropyrrolyl, -CH₂-tetrahydrofuranyl, -CH₂-phenyl, -CH₂-pyrrolyl, -CH₂-triazolyl, -CH₂-tetrazolyl, -CH₂-pyridyl, -CH₂-pyrazinyl, -CH₂-triazinyl, methoxy, ethoxy, difluoromethoxy, monofluoromethoxy, trifluoromethoxy, acetyl, acetamido and sulfonamido;
preferably, each R_{L2} is independently selected from the group consisting of deuterium, -F, -Cl, -Br, -OH, -CN, -CHO, -COOH, -NH₂, -CH₂OH, -CH₂CH₂OH, -CH₃, -CH₂CH₃, -CH₂F, -CHF₂, -CF₃, -CH₂CH₂F, -CH₂CHF₂, -CH₂CF₃, -OCH₃, -OCH₂CH₃, -OCH₂F, -OCHF₂, -OCF₃, -OCH₂CH₂F, -OCH₂CHF₂, -OCH₂CF₃, -COCH₃, -CH₂-cyclopropyl, cyclopropyl, -CONH₂, -COOCH₃, -OCOCH₃, -CONHCH₃, -CON(CH₃)₂, -SOCH₃, -SO₂CH₃, -SO₂NH₂, -SO₂NHCH₃ and -SO₂N(CH₃)₂;
preferably, each R_{L2} is independently selected from the group consisting of deuterium, -F, -Cl, -Br, -OH, -CN, -COOH, -NH₂, -CH₂OH, -CH₂CH₂OH, -CH₃, -CH₂CH₃, -CH₂F, -CHF₂, -CF₃, -CH₂CH₂F, -CH₂CHF₂, -CH₂CF₃, -OCH₃, -OCH₂CH₃, -OCH₂F, -OCHF₂, -OCF₃, -OCH₂CH₂F, -OCH₂CHF₂, -OCH₂CF₃, -COCH₃, -CH₂-cyclopropyl, cyclopropyl and -CONH₂;
preferably, each R_{L2} is independently selected from the group consisting of fluorine, chlorine, bromine, hydroxyl and hydroxymethyl.

21. The compound of formula (I) according to claim 18, or a pharmaceutically acceptable salt thereof, or a stereoisomer thereof, wherein each Lₐ is independently selected from the group consisting of the following structures or isomers thereof: -C(O)-, -C(O)NH-, -O-, -S-, -NH-, C₁₋₁₀ alkylene, C₁₋₁₀ alkyleneoxy, a C₃₋₁₂ cycloalkylene ring, a 4- to 12-membered heterocycloalkylene ring, a 5- to 6-membered heteroarylene ring and a phenylene ring; the C₃₋₁₂ cycloalkylene ring, 4- to 12-membered heterocycloalkylene ring, 5- to 6-membered heteroarylene ring and phenylene ring are unsubstituted or substituted with 1, 2, 3 or 4 R_{L2}, and R_{L2} is selected from the group consisting of fluorine, hydroxyl and hydroxymethyl;
preferably, each Lₐ is independently selected from the group consisting of the following structures or isomers thereof: -O-, -S-, -NH-, -C(O)-, -C(O)NH-, -(CH₂)ₘ₂-, -O(CH₂)ₘ₂-, -(CH₂)ₘ₂O-, and a divalent form of a cyclic group selected from the group consisting of: a cyclopropane ring, a cyclobutane ring, a bicyclopentane ring, a cyclopentane ring, a cyclohexane ring, an azetidine ring, a tetrahydropyrrole ring, a piperidine ring, a piperazine ring, a hydroxy-substituted piperidine ring, a hydroxy-substituted piperazine ring, a hydroxymethyl-substituted piperidine ring, a hydroxymethyl-substituted piperazine ring, 3,3,5,5-tetramethylpiperidine, 3,3-difluoropiperidine, a 2-azaspiro[3.3]heptane ring, a 6-azaspiro[3.4]octane ring, a 7-azaspiro[3.5]nonane ring, a 2,6-diazaspiro[3.3]heptane ring, a 2,6-diazaspiro[3.4]octane ring, a 2,7-diazaspiro[3.5]nonane ring, a 2-azaspiro[3.5]nonane ring, a spiro[3.3]heptane ring, a spiro[3.4]octane ring, a spiro[3.5]nonane ring, 3,9-diazaspiro[5.5]undecane, a benzene ring, a pyridine ring, a pyrimidine ring, a pyrazine ring, a pyridazine ring, a triazine ring, a thiophene ring, a furan ring, a pyrrole ring, a thiazole ring, an oxazole ring, a pyrazole ring, an imidazole ring and a triazole ring; wherein each m2 at each occurrence is independently 1, 2, 3, 4, 5, 6, 7, 8, 9 or 10;
preferably, each Lₐ is independently selected from the group consisting of the following structures or isomers thereof: -(CH₂)-, -(CH₂)₂-, -(CH₂)₅-, -C(O)-, -(CH₂)₃-, -C(O)NH-, -(CH₂)₈-, -O-, -NH-, 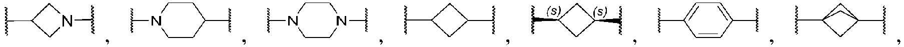 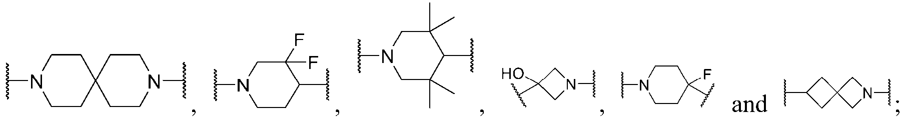
preferably, each Lₐ is independently selected from the group consisting of the following structures or isomers thereof: -(CH₂)-,

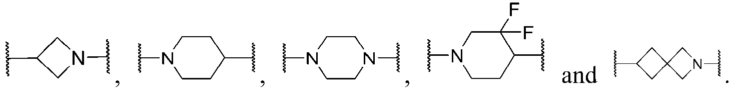

22. The compound of formula (I) according to claim 18, or a pharmaceutically acceptable salt thereof, or a stereoisomer thereof, wherein L is selected from the group consisting of the following structures or isomers thereof: -(CH₂)ₘ₃-, -C(O)-(CH₂)ₘ₃-, -C(O)NH-(CH₂)ₘ₃-, -C(O)NH-(CH₂)ₘ₃-O-, -Cy₀-, -NH-Cy₀-, -Cy₀-NH-(CH₂)ₘ₃-, -Cy₀-(CH₂)ₘ₃-NH-, -Cy₀-(CH₂)ₘ₃-O-, -C(O)NH-Cy₀-, -(CH₂)ₘ₃-C(O)NH-(CH₂)ₘ₃-O-Cy₀-O-(CH₂)ₘ₃-, -Cy₀-Cy₀-, -(CH₂)ₘ₃-Cy₀-O-Cyo-O-(CH₂)ₘ₃-, -Cy₀-C(O)NH-Cy₀-, -Cy₀-(CH₂)ₘ₃-Cy₀-, -Cy₀-O-Cy₀-, -Cy₀-C(O)-Cy₀-, -(CH₂)ₘ₃-Cy₀-Cy₀ and -C(O)NH-(CH₂)ₘ₃-Cy₀-(CH₂)ₘ₃-Cy₀; wherein each m3 at each occurrence is independently 0, 1, 2, 3, 4, 5, 6, 7, 8, 9 or 10; each Cy₀ at each occurrence is independently selected from the group consisting of a C₃₋₁₂ cycloalkyl ring (preferably a C₃₋₁₀ cycloalkyl ring, more preferably a C₃₋₈ cycloalkyl ring, further preferably a C₃₋₆ cycloalkyl ring), a 3- to 12-membered heterocycloalkyl ring (preferably a 3- to 10-membered heterocycloalkyl ring, more preferably a 3- to 8-membered heterocycloalkyl ring, further preferably a 3- to 6-membered heterocycloalkyl ring), a 5- to 6-membered heteroaryl ring and a benzene ring; the C₃₋₁₂ cycloalkyl ring, 3- to 12-membered heterocycloalkyl ring, 5- to 6-membered heteroaryl ring, benzene ring are unsubstituted or substituted with 1, 2, 3 or 4 R_{L2}, each R_{L2} at each occurrence is independently selected from the group consisting of deuterium, halogen, hydroxyl, cyano, amino, carboxyl, C₁₋₃ alkyl, C₁₋₃ alkoxy, C₁₋₃ haloalkyl, C₁₋₃ haloalkoxy, hydroxy-substituted C₁₋₃ alkyl, cyano-substituted C₁₋₃ alkyl, amino-substituted C₁₋₃ alkyl, C₁₋₃ alkoxy C₁₋₃ alkyl, C₃₋₆ cycloalkyl, C₃₋₆ cycloalkyl C₁₋₃ alkyl, -COC₁₋₃ alkyl, -COOC₁₋₃ alkyl, -OCOC₁₋₃ alkyl, -CONH₂, -CONHC₁₋₃ alkyl and -CON(C₁₋₃ alkyl)₂;
preferably, each Cy₀ is independently selected from the group consisting of a cyclopropane ring, a cyclobutane ring, a cyclopentane ring, a bicyclopentane ring, a cyclohexane ring, an azetidine ring, a hydroxy-substituted azetidine ring, a tetrahydropyrrole ring, a piperidine ring, a hydroxy-substituted piperidine ring, a hydroxymethyl-substituted piperidine ring, a 4-fluoropiperidine ring, 3,3-difluoropiperidine, 3,3,5,5-tetramethylpiperidine, a piperazine ring, a 2-azaspiro[3.3]heptane ring, a 6-azaspiro[3.4]octane ring, a 7-azaspiro[3.5]nonane ring, a 2,6-diazaspiro[3.3]heptane ring, a 2,6-diazaspiro[3.4]octane ring, a 2,7-diazaspiro[3.5]nonane ring, a 2-azaspiro[3.5]nonane ring, a spiro[3.3]heptane ring, a spiro[3.4]octane ring, a spiro[3.5]nonane ring, 3,9-diazaspiro[5.5]undecane, a benzene ring, a pyridine ring, a pyrimidine ring, a pyrazine ring, a pyridazine ring, a triazine ring, a thiophene ring, a furan ring, a pyrrole ring, a thiazole ring, an oxazole ring, a pyrazole ring, an imidazole ring and a triazole ring;
preferably, each Cy₀ is independently selected from the group consisting of a cyclobutane ring, a bicyclopentane ring, a benzene ring, an azetidine ring, a piperidine ring, a piperazine ring and 3,9-diazaspiro[5.5]undecane;
preferably, each Cy₀ is independently selected from the group consisting of a cyclobutane ring, an azetidine ring, a benzene ring and a piperidine ring;
preferably, each Cy₀ is independently selected from the group consisting of the following structures or isomers thereof: 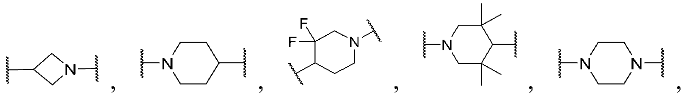 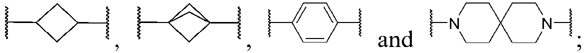
preferably, each Cy₀ is independently selected from the group consisting of the following structures or isomers thereof:

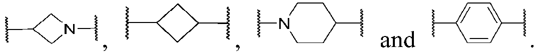

23. The compound of formula (I) according to claim 18, or a pharmaceutically acceptable salt thereof, or a stereoisomer thereof, wherein L is selected from the group consisting of the following structures or isomers thereof: 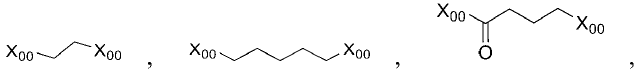 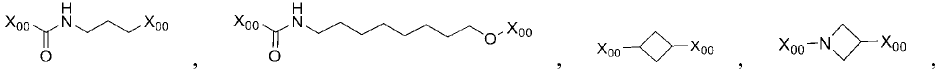 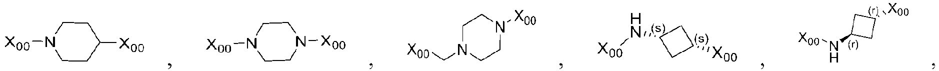 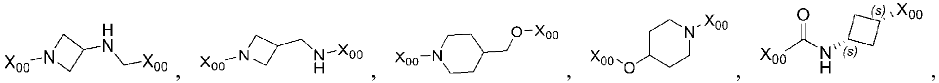 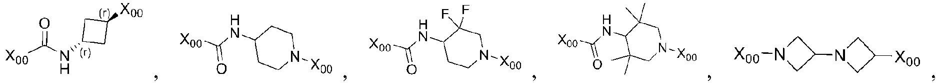 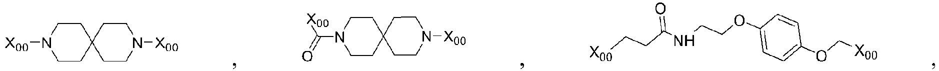 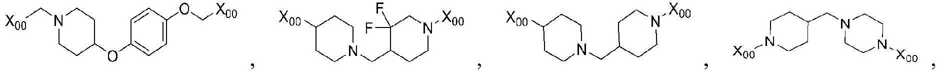 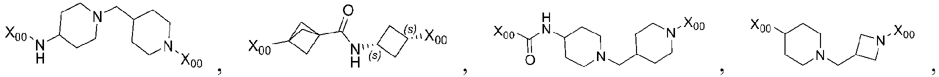 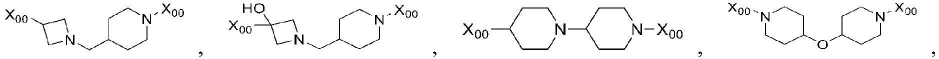 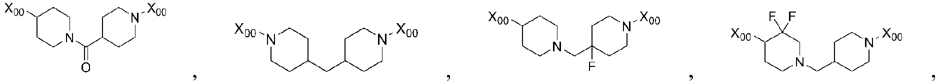 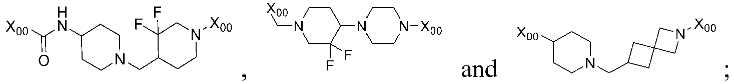 wherein X₀₀ is a point of attachment of L to ULM or POI;
preferably, L is selected from the group consisting of the following structures or isomers thereof: 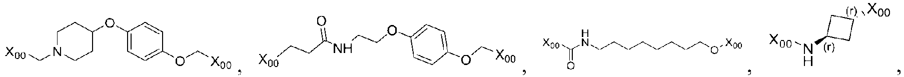 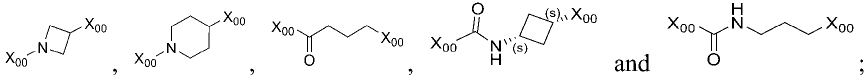 wherein X₀₀ is the point of attachment of L to ULM or POI;
preferably, L is selected from the group consisting of the following structures or isomers thereof: 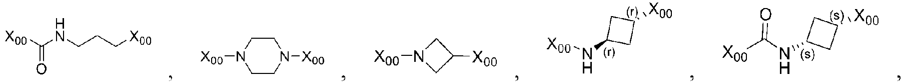 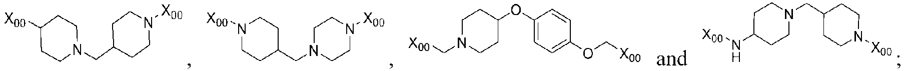 wherein X₀₀ is the point of attachment of L to ULM or POI;
preferably, L is selected from the group consisting of the following structures or isomers thereof: 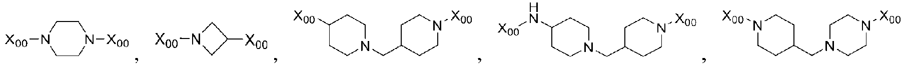 and 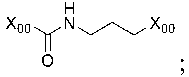 wherein X₀₀ is the point of attachment of L to ULM or POI;
preferably, L is selected from the group consisting of the following structures or isomers thereof: 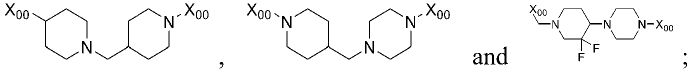 wherein X₀₀ is the point of attachment of L to ULM or POI;
preferably, L is selected from the group consisting of the following structures or isomers thereof: 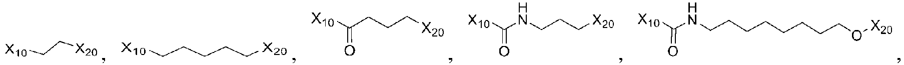 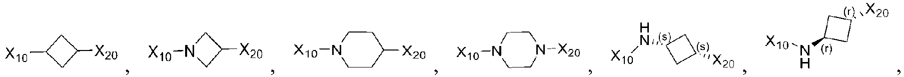 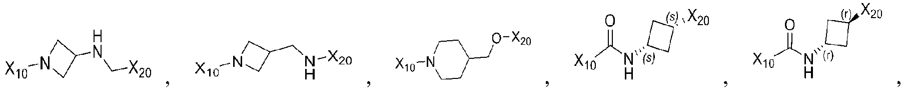 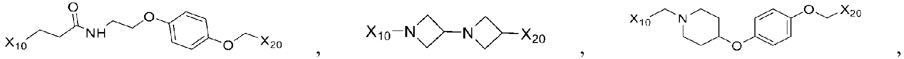 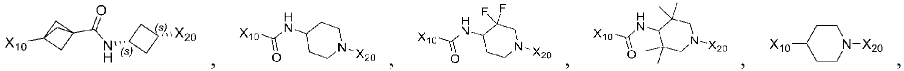 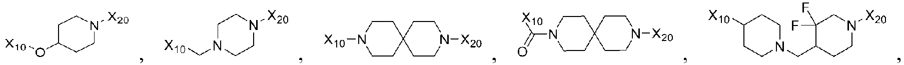 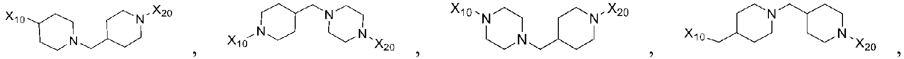 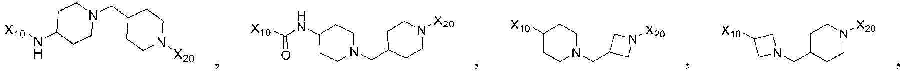 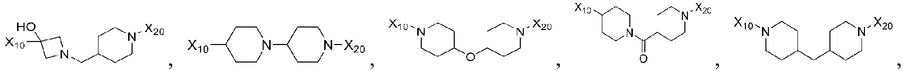 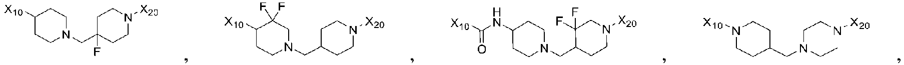 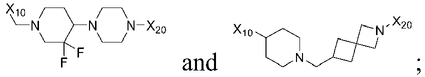 wherein X₁₀ is a point of attachment of L to POI, and X₂₀ is a point of attachment of L to ULM;
preferably, L is selected from the group consisting of the following structures or isomers thereof: 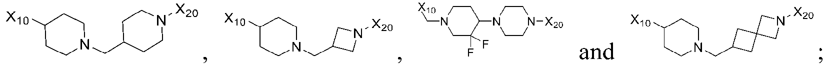 wherein X₁₀ is the point of attachment of L to POI, and X₂₀ is the point of attachment of L to ULM;
preferably, L is selected from the group consisting of the following structures or isomers thereof: 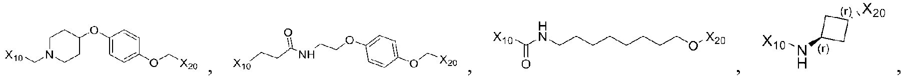 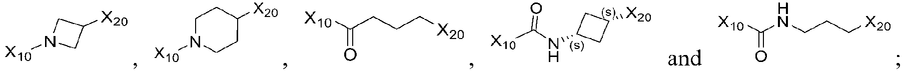 wherein X₁₀ is the point of attachment of L to POI, and X₂₀ is the point of attachment of L to ULM;
preferably, L is selected from the group consisting of the following structures or isomers thereof: 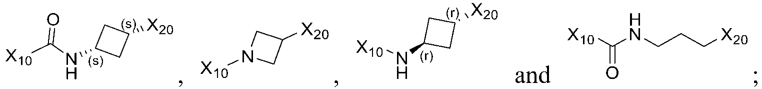 wherein X₁₀ is the point of attachment of L to POI, and X₂₀ is the point of attachment of L to ULM;
preferably, L is selected from the group consisting of the following structures or isomers thereof: 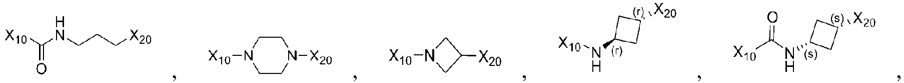 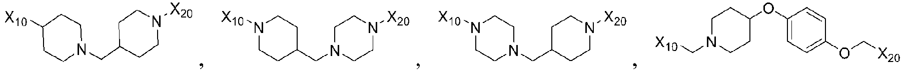 and 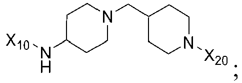 wherein X₁₀ is the point of attachment of L to POI, and X₂₀ is the point of attachment of L to ULM;
preferably, L is selected from the group consisting of the following structures or isomers thereof: 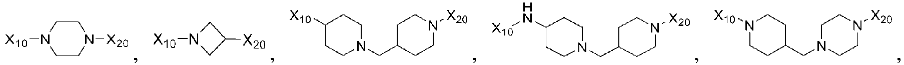 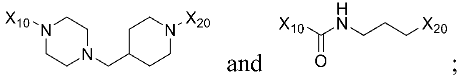 wherein X₁₀ is the point of attachment of L to POI, and X₂₀ is the point of attachment of L to ULM;
preferably, L is selected from the group consisting of the following structures or isomers thereof: 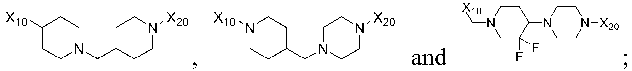 wherein X₁₀ is the point of attachment of L to POI, and X₂₀ is the point of attachment of L to ULM.

24. The compound of formula (I) according to claim 1, or a pharmaceutically acceptable salt thereof, or a stereoisomer thereof, wherein ULM is a compound of formula (U-1) or an isomer thereof: 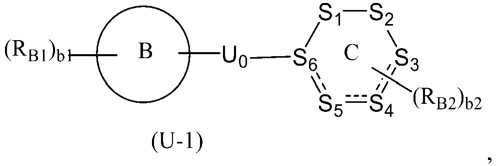
wherein, -̅ -̅ -̅ -̅ -̅ -̅ represents 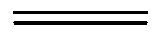 (double bond) or 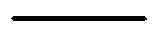 (single bond);
U₀ is a chemical bond, -N(R_{U0})-, -CON(R_{U0})-, -CH₂- or -(CH₂)₂-;
each R_{U0} at each occurrence is independently hydrogen or C₁₋₃ alkyl;
ring B is absent or is selected from the group consisting of a 5- to 15-membered heteroaryl ring (preferably a 6- to 12-membered heteroaryl ring, more preferably a 6- to 10-membered heteroaryl ring), a 3- to 15-membered heterocycloalkyl ring (preferably a 5- to 12-membered heterocycloalkyl ring, more preferably a 5- to 10-membered heterocycloalkyl ring), a C₃₋₁₅ cycloalkyl ring and a C₆₋₁₀ aryl ring (preferably a benzene ring);
S₁, S₃ and S₅ are each independently selected from the group consisting of a chemical bond, -O-, -NH-, -N-, -CH₂-, -CH-, -C(O)-, -C(O)O-, -C(O)S-, -CH₂C(O)-, -CH₂C(S)-, -C(S)-, -CONH-, -CH=N-, -N=N-, -CH=CH-, -SO- and -SO₂-;
S₂ and S₄ are each independently selected from the group consisting of -N-, -NH-, -CH- and -CH₂-;
S₆ is selected from the group consisting of C, -CH- and N;
(R_{B1})_{b1} represents substitution of hydrogen on ring B by b1 R_{B1}, b1 is 0, 1, 2 or 3, each R_{B1} is identical or different, and each R_{B1} is independently selected from the group consisting of X₂, deuterium, halogen (preferably fluorine, chlorine or bromine), cyano, carboxyl, hydroxyl, nitro, -NRₐ₁R_{b1}, C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), -SC₁₋₈ alkyl (preferably -SC₁₋₆ alkyl, more preferably -SC₁₋₃ alkyl), -SOC₁₋₈ alkyl (preferably -SOC₁₋₆ alkyl, more preferably -SOC₁₋₃ alkyl), -SO₂C₁₋₈ alkyl (preferably -SO₂C₁₋₆ alkyl, more preferably -SO₂C₁₋₃ alkyl), C₁₋₈ haloalkyl (preferably C₁₋₆ haloalkyl, more preferably C₁₋₃ haloalkyl), C₁₋₈ haloalkoxy (preferably C₁₋₆ haloalkoxy, more preferably C₁₋₃ haloalkoxy), amino-substituted C₁₋₈ alkyl (preferably amino-substituted C₁₋₆ alkyl, more preferably amino-substituted C₁₋₃ alkyl), cyano-substituted C₁₋₈ alkyl (preferably cyano-substituted C₁₋₆ alkyl, more preferably cyano-substituted C₁₋₃ alkyl), hydroxy-substituted C₁₋₈ alkyl (preferably hydroxy-substituted C₁₋₆ alkyl, more preferably hydroxy-substituted C₁₋₃ alkyl), carboxy-substituted C₁₋₈ alkyl (preferably carboxy-substituted C₁₋₆ alkyl, more preferably carboxy-substituted C₁₋₃ alkyl), -COC₁₋₈ alkyl (preferably -COC₁₋₆ alkyl, more preferably -COC₁₋₃ alkyl), -COOC₁₋₈ alkyl-CONRₐ₂R_{b2} (preferably -COOC₁₋₆ alkyl-CONRₐ₂R_{b2}, more preferably -COOC₁₋₃ alkyl-CONRₐ₂R_{b2}), -SO₂NRₐ₂R_{b2}, a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring), a 3- to 15-membered heterocycloalkyl ring (preferably a 4- to 12-membered heterocycloalkyl ring, more preferably a 4- to 10-membered heterocycloalkyl ring, further preferably a 4- to 8-membered heterocycloalkyl ring, further preferably a 4- to 6-membered heterocycloalkyl ring), a 5- to 10-membered heteroaryl ring (preferably a 5- to 6-membered heteroaryl ring) and a C₆₋₁₀ aryl ring (preferably a benzene ring or naphthalene ring); or two adjacent R_{B1} and carbon atom linked to the two adjacent R_{B1} together form a C₃₋₁₅ cycloalkyl ring (preferably a C₃₋₁₀ cycloalkyl ring, more preferably a C₃₋₈ cycloalkyl ring, further preferably a C₃₋₆ cycloalkyl ring), a 3-to 15-membered heterocycloalkyl ring (preferably a 4- to 12-membered heterocycloalkyl ring, more preferably a 4- to 10-membered heterocycloalkyl ring, further preferably a 4- to 8-membered heterocycloalkyl ring, further preferably a 4- to 6-membered heterocycloalkyl ring), a 5- to 6-membered heteroaryl ring or a benzene ring; the C₃₋₁₅ cycloalkyl ring, 3- to 15-membered heterocycloalkyl ring, 5- to 6-membered heteroaryl ring or benzene ring is unsubstituted or substituted with 1, 2, 3 or 4 substituents selected from the group consisting of: X₂, deuterium, halogen (preferably fluorine, chlorine or bromine), cyano, carboxyl, hydroxyl, nitro, -NRₐ₁R_{b1}, C₁₋₆ alkyl, C₁₋₆ alkoxy, -SC₁₋₆ alkyl, -SOC₁₋₆ alkyl, -SO₂C₁₋₆ alkyl, C₁₋₆ haloalkyl, C₁₋₆ haloalkoxy, -COC₁₋₆ alkyl, -COOC₁₋₆ alkyl, -CONRₐ₂R_{b2} and -SO₂NRₐ₂R_{b2};
(R_{B2})_{b2} represents substitution of hydrogen on ring C by b2 R_{B2}, b2 is 0, 1, 2, 3 or 4, each R_{B2} is identical or different, and each R_{B2} is independently selected from the group consisting of X₂, deuterium, halogen (preferably fluorine, chlorine or bromine), cyano, carboxyl, hydroxyl, -NRa1R_{b1}, C₁₋₆ alkyl, C₁₋₆ alkoxy, C₁₋₆ haloalkyl, C₁₋₆ haloalkoxy, -COC₁₋₆ alkyl, -COOC₁₋₆ alkyl, -OCOC₁₋₆ alkyl, -CONRₐ₂R_{b2}, -OC(O)C₁₋₆ alkyl-substituted C₁₋₆ alkyl and -COOC₁₋₆ alkyl-substituted C₁₋₆ alkyl;
Rₐ₁, R_{b1}, Rₐ₂ and R_{b2} are each independently selected from the group consisting of hydrogen, C₁₋₆ alkyl, C₁₋₆ haloalkyl, hydroxy-substituted C₁₋₆ alkyl, cyano-substituted C₁₋₆ alkyl, carboxy-substituted C₁₋₆ alkyl, amino-substituted C₁₋₆ alkyl, -COC₁₋₆ alkyl and -COOC₁₋₆ alkyl;
X₂ in R_{B1} and R_{B2} is a point of attachment of ULM to L or POI, and at least one of R_{B1} and R_{B2} is X₂.

25. The compound of formula (I) according to claim 24, or a pharmaceutically acceptable salt thereof, or a stereoisomer thereof, wherein R_{B1} and R_{B2} are not both X₂.

26. The compound of formula (I) according to claim 24, or a pharmaceutically acceptable salt thereof, or a stereoisomer thereof, wherein ring B is absent;
or the 5- to 15-membered heteroaryl ring in ring B is selected from the group consisting of a pyrrole ring, a furan ring, a thiophene ring, a pyrazole ring, an imidazole ring, a triazole ring, a tetrazole ring, an oxazole ring, a thiazole ring, an oxadiazole ring, a thiadiazole ring, a pyridine ring, a pyridazine ring, a pyrimidine ring, a pyrazine ring, a triazine ring, a benzopyrrole ring, a benzofuran ring, a benzothiophene ring, a benzopyrazole ring, a benzimidazole ring, a benzothiazole ring, a benzoxazole ring, a pyridopyrrole ring, a pyridofuran ring, a pyridothiophene ring, a pyridopyrazole ring, a pyridoimidazole ring, a pyridothiazole ring, a pyridooxazole ring, a pyrimidopyrrole ring, a pyridazopyrrole ring, a pyrazinopyrrole ring, a pyrimidopyrazole ring, a pyridazopyrazole ring, a pyrazinopyrazole ring, a pyrimidoimidazole ring, a pyridazoimidazole ring, a pyrazinoimidazole ring, a quinoline ring, an isoquinoline ring and a 9H-pyrido[2,3-b]indole ring;
preferably, the 5- to 15-membered heteroaryl ring in ring B is a benzopyrazole ring;
or the 3- to 15-membered heterocycloalkyl ring in ring B is 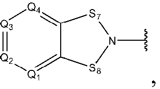
wherein Q₁, Q₂, Q₃, Q₄ are each independently selected from the group consisting of -CH-, N and N-O;
S₇ and S₈ are each independently selected from the group consisting of a chemical bond, -O-, -NH-, -CH₂-, -C(O)-, -C(O)O-, -C(O)S-, -CH₂C(O)-, -CH₂C(S)-, -C(S)-, -CONH-, -CH=N-, -N=N-, -CH=CH-, -SO- and -SO₂-;
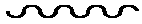 represents a covalent connection to U₀;
preferably, Q₁, Q₂, Q₃ and Q₄ are each independently -CH-;
preferably, S₇ and S₈ are each independently selected from the group consisting of -CH₂- and -C(O)-;
preferably, S₇ is -CH₂-, and S₈ is -C(O)-;
preferably, S₇ is -C(O)-, and S₈ is -C(O)-;
preferably, 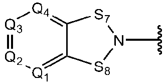 is selected from the group consisting of the following structures or isomers thereof: 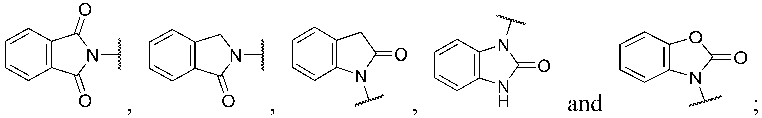 wherein 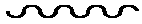 represents a covalent connection to U₀;
or the 3- to 15-membered heterocycloalkyl ring in ring B is 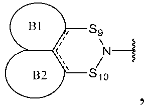 wherein ring B1 and ring B2 are each independently selected from the group consisting of a C₄₋₈ cycloalkyl ring, a 4- to 8-membered heterocycloalkyl ring, a 5- to 6-membered heteroaryl ring and a benzene ring; S₉ and S₁₀ are each independently selected from the group consisting of a bond, -CH₂- and -C(O)-; 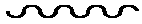 represents a covalent connection to U₀;
preferably, 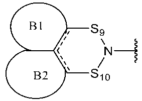 is selected from the group consisting of 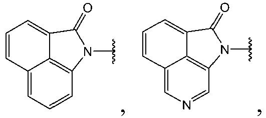 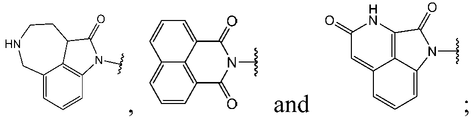 wherein 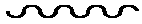 represents a covalent connection to U₀; or the 3- to 15-membered heterocycloalkyl ring in ring B is 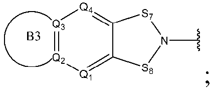 wherein ring B3 is a 3- to 7-membered heterocycloalkyl ring, and 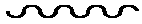 represents the covalent connection to U₀;
preferably, ring B3 is a 5- to 7-membered nitrogen-containing heterocycloalkyl ring;
preferably, ring B3 is a partially unsaturated 5- to 7-membered nitrogen-containing heterocycloalkyl ring;
preferably, ring B3 is selected from the group consisting of 2,5-dihydro-1H-pyrrole, 2,3-dihydro-1H-pyrrole, 1,2,3,6-tetrahydropyridine, 1,2,3,4-tetrahydropyridine, 2,3,6,7-tetrahydro-1H-azepine, 2,3,4,7-tetrahydro-1H-azepine and 2,3,4,5-tetrahydro-1H-azepine;
preferably, 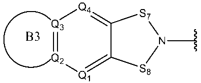 is selected from the group consisting of 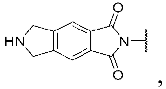 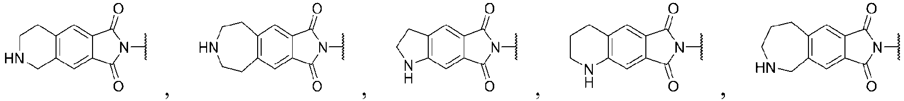 and 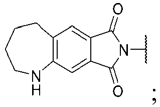 wherein 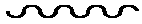 represents the covalent connection to U₀.

27. The compound of formula (I) according to claim 24, or a pharmaceutically acceptable salt thereof, or a stereoisomer thereof, wherein ring B is selected from the group consisting of: a benzene ring, 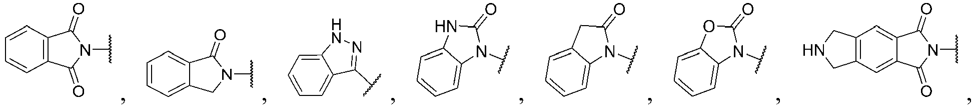 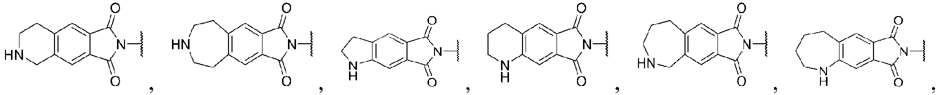 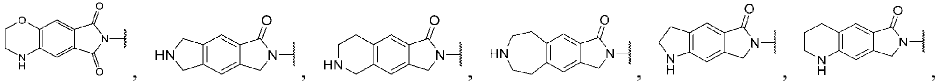 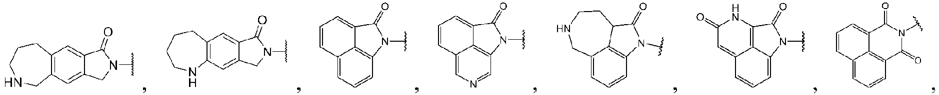 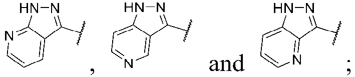 wherein 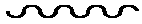 represents a covalent connection to U₀;
preferably, ring B is selected from the group consisting of: 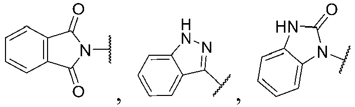 and 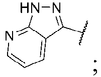 wherein 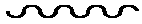 represents a covalent connection to U₀;
preferably, ring B is selected from the group consisting of: 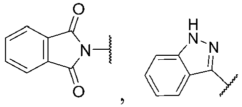 and 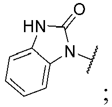 wherein 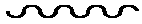 represents the covalent connection to U₀.

28. The compound of formula (I) according to claim 24, or a pharmaceutically acceptable salt thereof, or a stereoisomer thereof, wherein each R_{B1} is independently X₂, deuterium, fluorine, chlorine, bromine, cyano, carboxyl, hydroxyl, nitro, -NH₂, -N(CH₃)₂, -NHCH₃, -NHCOCH₃, methyl, ethyl, propyl, isopropyl, methoxy, ethoxy, isopropoxy, trifluoromethyl, difluoromethyl, monofluoromethyl, trifluoromethoxy, difluoromethoxy, monofluoromethoxy, -SCH₃, -SOCH₃, -SO₂CH₃, -CH₂NH₂, -(CH₂)₂NH₂, -(CH₂)₃NH₂, -CH₂CN, -(CH₂)₂CN, -(CH₂)₃CN, -CH₂OH, -(CH₂)₂OH, -(CH₂)₃OH, -CH₂COOH, -(CH₂)₂COOH, -(CH₂)₃COOH, -COCH₃, -COCH₂CH₃, -COOCH₃, -COOCH₂CH₃, -CONH₂ or -SO₂NH₂; or two adjacent R_{B1} and carbon atom linked to the two adjacent R_{B1} together form a cyclobutyl ring, a cyclopentyl ring, a cyclohexyl ring, a cycloheptyl ring, a cyclopentene ring, a cyclohexene ring, a cycloheptene ring, a tetrahydropyrrole ring, a tetrahydrofuran ring, a tetrahydrothiophene ring, a piperidine ring, a pyrazine ring, a 1,2,3,4-tetrahydropyridine ring, a 1,2,3,4-tetrahydropyran ring, a 3,4-dihydro-2H-1,4-oxazine ring, a 2,3,4,5-tetrahydro-1H-azepine ring, a pyrrole ring, a pyrazole ring, an oxazole ring, a thiazole ring, a pyran ring, a pyridine ring, a pyridazine ring, a pyrimidine ring or a benzene ring; the cyclobutyl ring, cyclopentyl ring, cyclohexyl ring, cycloheptyl ring, cyclopentene ring, cyclohexene ring, cycloheptene ring, tetrahydropyrrole ring, tetrahydrofuran ring, tetrahydrothiophene ring, piperidine ring, pyrazine ring, 1,2,3,4-tetrahydropyridine ring, 1,2,3,4-tetrahydropyran ring, 3,4-dihydro-2H-1,4-oxazine ring, 2,3,4,5-tetrahydro-1H-azepine ring, pyrrole ring, pyrazole ring, oxazole ring, thiazole ring, pyran ring, pyridine ring, pyridazine ring, pyrimidine ring, or benzene ring is unsubstituted or substituted with 1, 2, 3 or 4 substituents selected from the group consisting of: X₂, deuterium, fluorine, chlorine, bromine, cyano, carboxyl, hydroxyl, nitro, amino, methyl, trifluoromethyl, methoxy, trifluoromethoxy, -SCH₃, -SOCH₃, -SO₂CH₃, -COCH₃, -COOCH₃, -CONH₂ and -SO₂NH₂;
preferably, R_{B1} is selected from the group consisting of X₂, fluorine, chlorine, hydroxyl, methyl, trifluoromethyl and methoxy.

29. The compound of formula (I) according to claim 24, or a pharmaceutically acceptable salt thereof, or a stereoisomer thereof, wherein 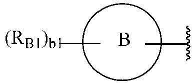 is selected from the group consisting of: 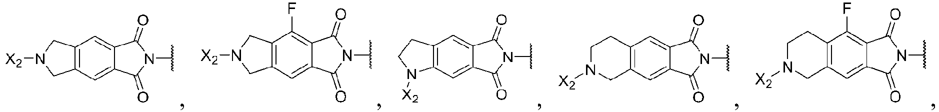 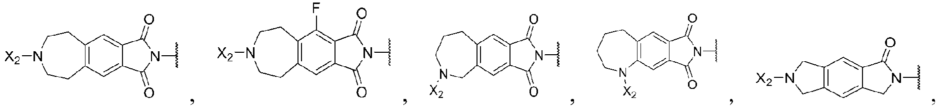 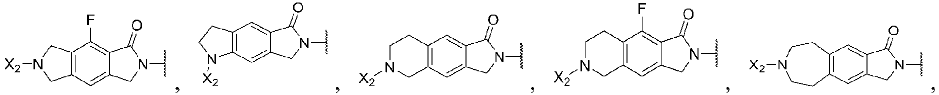 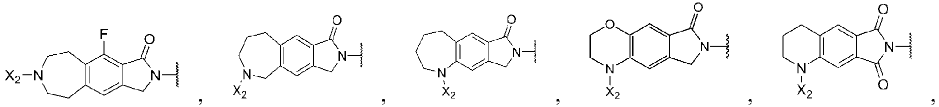 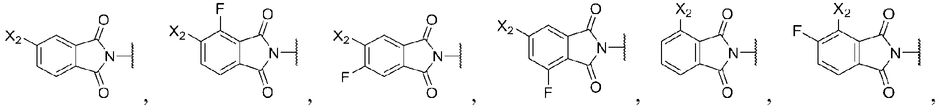 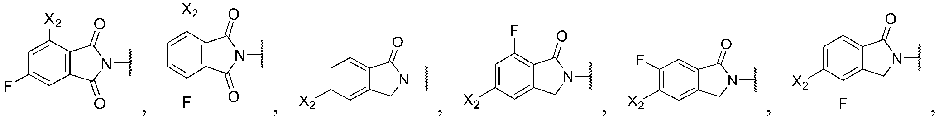 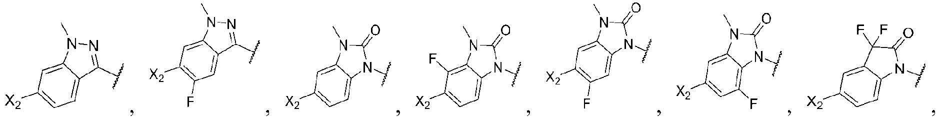 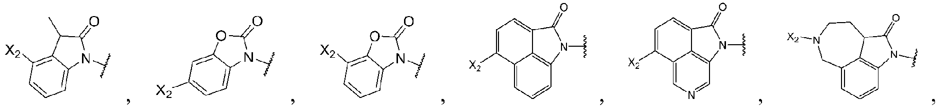 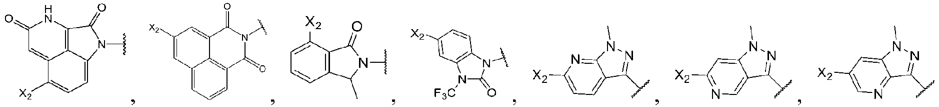 and 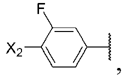 wherein X₂ is the point of attachment of ULM to L or POI, and 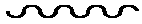 represents a covalent connection to U₀;
preferably, 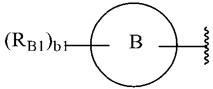 is selected from the group consisting of: 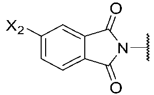 and 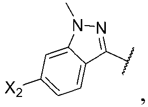 wherein X₂ is the point of attachment of ULM to L or POI, and 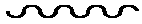 represents the covalent connection to U₀.

30. The compound of formula (I) according to claim 24, or a pharmaceutically acceptable salt thereof, or a stereoisomer thereof, wherein U₀ is a chemical bond, -NH-, -CONH- or -CH₂-.

31. The compound of formula (I) according to claim 24, or a pharmaceutically acceptable salt thereof, or a stereoisomer thereof, wherein S₁ and S₃ are -C(O)-, S₂ is -NH-, S₄ and S₃ are -CH₂-.

32. The compound of formula (I) according to claim 24, or a pharmaceutically acceptable salt thereof, or a stereoisomer thereof, wherein S₆ is CH or N.

33. The compound of formula (I) according to claim 24, or a pharmaceutically acceptable salt thereof, or a stereoisomer thereof, wherein 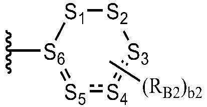 is selected from the group consisting of the following structure or an isomer thereof: 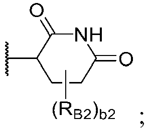 wherein 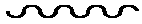 represents a covalent connection to U₀;
preferably, 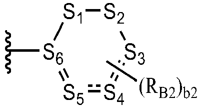 is selected from the group consisting of the following structures or isomers thereof: 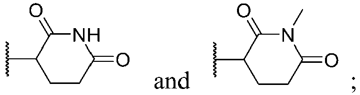 wherein 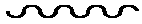 represents a covalent connection to U₀.

34. The compound of formula (I) according to claim 24, or a pharmaceutically acceptable salt thereof, or a stereoisomer thereof, wherein ULM is selected from the group consisting of the following structures or isomers thereof: 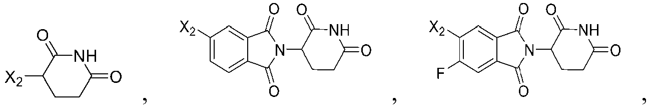 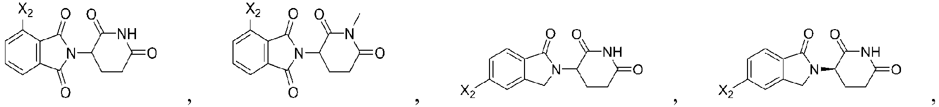 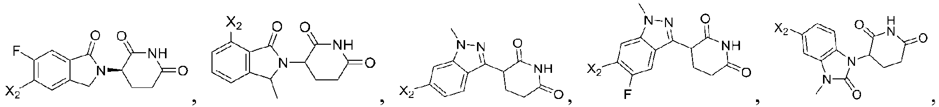 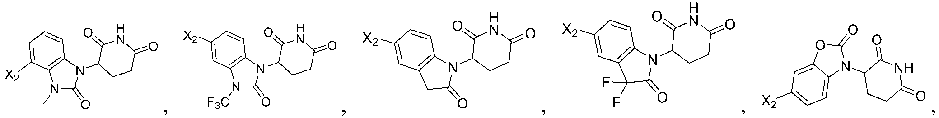 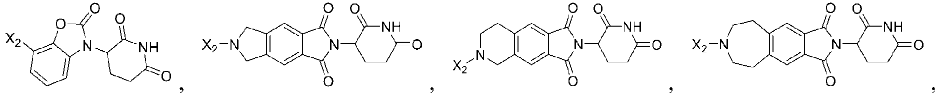 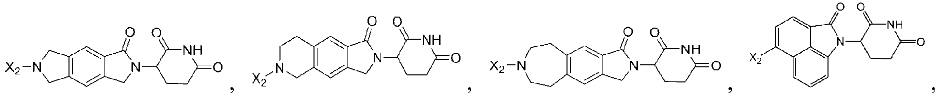 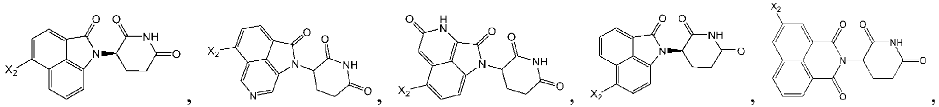 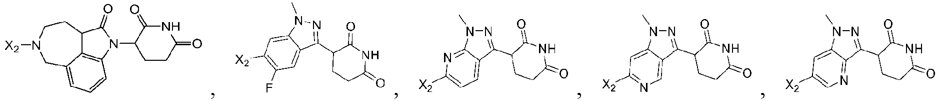 and 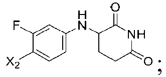 wherein X₂ is the point of attachment of ULM to L or POI;
preferably, ULM is selected from the group consisting of the following structure or an isomer thereof: 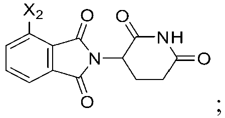 wherein X₂ is the point of attachment of ULM to L or POI;
preferably, ULM is selected from the group consisting of the following structures or isomers thereof: 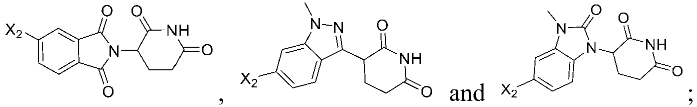 wherein X₂ is the point of attachment of ULM to L or POI.

35. The compound of formula (I) according to claim 1, or a pharmaceutically acceptable salt thereof, or a stereoisomer thereof, wherein ULM is a structure of formula (U-2) or an isomer thereof: 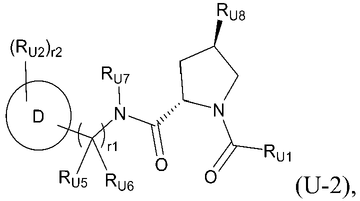 wherein,
r1 is 0, 1 or 2;
ring D is selected from the group consisting of a benzene ring, a 5- to 6-membered heteroaryl ring, a C₃₋₁₀ cycloalkyl ring (preferably a C₃₋₈ cycloalkyl ring, more preferably a C₃₋₆ cycloalkyl ring) and a 3- to 10-membered heterocycloalkyl ring (preferably a 3- to 8-membered heterocycloalkyl ring, more preferably a 3- to 6-membered heterocycloalkyl ring);
(R_{U2})ᵣ₂ represents substitution of hydrogen on ring D by r2 R_{U2}, r2 is 0, 1, 2 or 3, each R_{U2} is identical or different, and each R_{U2} is independently selected from the group consisting of X₂, hydrogen, deuterium, halogen (preferably fluorine, chlorine), nitro, cyano, carboxyl, hydroxyl, C₁₋₆ alkyl, C₁₋₆ alkoxy, C₁₋₆ alkoxy C₁₋₆ alkyl, hydroxy-substituted C₁₋₆ alkyl, C₁₋₆ haloalkyl, C₁₋₆ haloalkoxy, -NRₐ₃R_{b3}, -COC₁₋₆ alkyl, -COOC₁₋₆ alkyl, -OCOC₁₋₆ alkyl, -CONH₂, -CONHC₁₋₆ alkyl, -CON(C₁₋₆ alkyl)₂, -SOC₁₋₆ alkyl, -SO2C₁₋₆ alkyl, -SC₁₋₆ alkyl, 5- to 6-membered heteroaryl and phenyl; the 5- to 6-membered heteroaryl and phenyl are unsubstituted or substituted with 1, 2 or 3 substituents selected from the group consisting of: hydrogen, deuterium, halogen, cyano, carboxyl, hydroxyl, C₁₋₆ alkyl (preferably C₁₋₃ alkyl), hydroxy-substituted C₁₋₆ alkyl (preferably hydroxy-substituted C₁₋₃ alkyl), C₁₋₆ alkoxy C₁₋₆ alkyl (preferably C₁₋₃ alkoxy C₁₋₃ alkyl), C₁₋₆ alkoxy (preferably C₁₋₃ alkoxy), C₁₋₆ haloalkyl (preferably C₁₋₃ haloalkyl), C₁₋₆ haloalkoxy (preferably C₁₋₃ haloalkoxy), -NH₂, -NHCOC₁₋₆ alkyl (preferably -NHCOC₁₋₃ alkyl), -COC₁₋₆ alkyl (preferably -COC₁₋₃ alkyl), -COOC₁₋₆ alkyl (preferably -COOC₁₋₃ alkyl), -OCOC₁₋₆ alkyl (preferably -OCOC₁₋₃ alkyl), -CONH₂, -NHCONH₂, -CONHC₁₋₆ alkyl (preferably -CONHC₁₋₃ alkyl), -NHCONHC₁₋₆ alkyl (preferably -NHCONHC₁₋₃ alkyl), -SOC₁₋₆ alkyl (preferably -SOC₁₋₃ alkyl), -SO₂C₁₋₆ alkyl (preferably -SO2C₁₋₃ alkyl) and -SC₁₋₆ alkyl (preferably -SC₁₋₃ alkyl);
R_{U1} is -C(R_{U3}R_{U4})-U₁;
U₁ is selected from the group consisting of the following structures or isomers thereof: X₂, -NHCO-X₂, -NHCOCH₃, 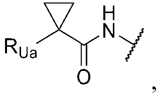 a 5- to 6-membered heteroaryl ring, 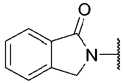 and 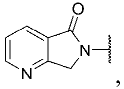 the 5- to 6-membered heteroaryl ring, 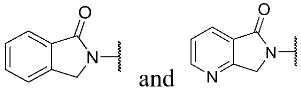 are unsubstituted or substituted with 1, 2 or 3 substituents selected from the group consisting of: X₂, halogen, hydroxyl, cyano, amino, carboxyl, C₁₋₆ alkyl (preferably methyl, ethyl, isopropyl), C₁₋₆ alkoxy (preferably methoxy, ethoxy, isopropoxy), C₁₋₆ haloalkyl (preferably trifluoromethyl), C₁₋₆ haloalkoxy (preferably trifluoromethoxy), -COC₁₋₆ alkyl (preferably -COCH₃), -COOC₁₋₆ alkyl (preferably -COOCH₃), -CONH₂, -CONHC₁₋₆ alkyl (preferably -CONHCH₃), -CON(C₁₋₆ alkyl)₂ (preferably -CON(CH₃)₂) and hydroxy-substituted C₁₋₆ alkyl (preferably -CH₂OH);
R_{Ua} is selected from the group consisting of hydrogen, halogen (preferably fluorine, chlorine, or bromine), cyano, hydroxyl, carboxyl, amino, C₁₋₆ alkyl, C₁₋₆ alkoxy, C₁₋₆ haloalkyl, C₁₋₆ haloalkoxy, -COC₁₋₆ alkyl, -NHCOC₁₋₆ alkyl, -N(C₁₋₆ alkyl)COC₁₋₆ alkyl, -NHC₁₋₆ alkyl and -N(C₁₋₆ alkyl)₂;
preferably, R_{Ua} is selected from the group consisting of fluorine, cyano, methyl, ethyl, trifluoromethyl and trifluoromethoxy;
preferably, R_{Ua} is selected from the group consisting of fluorine and cyano;
R_{U3} and R_{U4} are each independently selected from the group consisting of hydrogen, deuterium, halogen, cyano, carboxyl, hydroxyl, C₁₋₆ alkyl (preferably C₁₋₃ alkyl), C₁₋₆ alkoxy (preferably C₁₋₃ alkoxy), C₁₋₆ haloalkyl (preferably C₁₋₃ alkoxy), C₁₋₆ haloalkoxy (preferably C₁₋₃ haloalkoxy), and -SC₁₋₆ alkyl (preferably -SC₁₋₃ alkyl); or R_{U3}, R_{U4}, and carbon atom linked to R_{U3} and R_{U4} together form a C₃₋₇ cycloalkyl (preferably C₃₋₆ cycloalkyl) or a 3- to 7-membered heterocycloalkyl (preferably a 4- to 6-membered heterocycloalkyl); the C₁₋₆ alkyl, C₁₋₆ alkoxy, C₁₋₆ haloalkyl, C₁₋₆ haloalkoxy, -SC₁₋₆ alkyl, C₃₋₇ cycloalkyl and 3- to 7-membered heterocycloalkyl are unsubstituted or substituted with 1, 2 or 3 substituents selected from the group consisting of: X₂, halogen (preferably fluorine, chlorine or bromine), cyano, carboxyl and hydroxyl;
R_{U5} and R_{U6} are each independently selected from the group consisting of X₂, hydrogen, deuterium, halogen, amino, cyano, carboxyl, hydroxyl, C₁₋₆ alkyl (preferably C₁₋₃ alkyl), C₁₋₆ alkoxy (preferably C₁₋₃ alkoxy), C₁₋₆ haloalkyl (preferably C₁₋₃ haloalkyl), C₁₋₆ haloalkoxy (C₁₋₃ haloalkoxy), -SC₁₋₆ alkyl (preferably -SC₁₋₃ alkyl), CONHC₁₋₆ alkyl-substituted C₁₋₆ alkyl, CON(C₁₋₆ alkyl)₂-substituted C₁₋₆ alkyl, carboxy-substituted C₁₋₆ alkyl and COOC₁₋₆ alkyl-substituted C₁₋₆ alkyl;
R_{U7} is selected from the group consisting of hydrogen, C₁₋₆ alkyl, and C₁₋₆ haloalkyl; or R_{U7}, nitrogen atom linked to R_{U7}, R_{U6} and carbon atom linked to R_{U6} together form a 3- to 8-membered heterocycloalkyl ring or a 5- to 6-membered heteroaryl ring;
R_{U8} is selected from the group consisting of hydroxyl, amino, -NH(C₁₋₆ alkyl), -N(C₁₋₆ alkyl)₂, C₁₋₆ alkoxy, C₁₋₆ haloalkoxy and -OCOC₁₋₆ alkyl;
Rₐ₃ and R_{b3} are each independently selected from the group consisting of hydrogen, C₁₋₆ alkyl (preferably C₁₋₃ alkyl), C₁₋₆ alkoxy (preferably C₁₋₃ alkoxy), -SC₁₋₆ alkyl (preferably -SC₁₋₃ alkyl), C₁₋₆ haloalkyl (preferably C₁₋₃ haloalkyl), C₁₋₆ haloalkoxy (preferably C₁₋₃ haloalkoxy), -COC₁₋₆ alkyl (preferably -COC₁₋₃ alkyl), -CONH₂, -CONHC₁₋₆ alkyl (preferably -CONHC₁₋₃ alkyl), -CON(C₁₋₆ alkyl)₂ (preferably -CON(C₁₋₃ alkyl)₂), 5- to 6-membered heteroaryl and phenyl; wherein the 5- to 6-membered heteroaryl and phenyl are each independently unsubstituted or substituted with 1, 2 or 3 substituents selected from the group consisting of: hydrogen, C₁₋₆ alkyl (preferably C₁₋₃ alkyl), C₁₋₆ alkoxy (preferably C₁₋₃ alkoxy), -SC₁₋₆ alkyl (preferably -SC₁₋₃ alkyl), C₁₋₆ haloalkyl (preferably C₁₋₃ haloalkyl), C₁₋₆ haloalkoxy (preferably C₁₋₃ haloalkoxy), -COC₁₋₆ alkyl (preferably -COC₁₋₃ alkyl), -CONH₂, -CONHC₁₋₆ alkyl (preferably -CONHC₁₋₃ alkyl) and -CON(C₁₋₆ alkyl)₂ (preferably -CON(C₁₋₃ alkyl)₂);
preferably, Rₐ₃ and R_{b3} are each independently selected from the group consisting of hydrogen, 5-to 6-membered heteroaryl, and phenyl; the 5- to 6-membered heteroaryl is thiazolyl, oxazolyl, pyrazolyl, imidazolyl, thienyl, furanyl, pyrrolyl, triazolyl or tetrazolyl; the 5- to 6-membered heteroaryl and phenyl are unsubstituted or substituted with 1 or 2 substituents selected from the group consisting of: hydrogen, methyl, ethyl, isopropyl, trifluoromethyl, trifluoromethoxy, -COCH₃ and -CONH₂;
X₂ in R_{U2}, R_{U1}, R_{U5} and R_{U6} is a point of attachment of ULM to L or POI, and at least one of R_{U1}, R_{U5}, R_{U6} and R_{U2} is X₂.

36. The compound of formula (I) according to claim 35, or a pharmaceutically acceptable salt thereof, or a stereoisomer thereof, wherein ring D is selected from the group consisting of a benzene ring, a 5- to 6-membered heteroaryl ring, a C₅₋₉ cycloalkyl ring and a 5- to 9-membered heterocycloalkyl ring;
preferably, ring D is selected from the group consisting of a benzene ring and 2,3-dihydro-1H-indene;
preferably, ring D is 2,3-dihydro-1H-indene;
preferably, ring D is selected from the group consisting of a benzene ring and a 5- to 6-membered heteroaryl ring;
preferably, ring D is selected from the group consisting of a benzene ring, a pyrrole ring, a thiophene ring, a furan ring, a pyrazole ring, an imidazole ring, a triazole ring, a thiazole ring, an oxazole ring, a pyridine ring, a pyrimidine ring, a pyrazine ring, a pyridazine ring, a cyclopropane ring, a cyclobutane ring, a cyclopentane ring, a cyclohexane ring, a piperidine ring, a piperazine ring and a tetrahydropyrrole ring;
preferably, ring D is selected from the group consisting of a benzene ring and a pyridine ring; preferably, ring D is a benzene ring.

37. The compound of formula (I) according to claim 35, or a pharmaceutically acceptable salt thereof, or a stereoisomer thereof, wherein r1 is 1, and ring D is a benzene ring.

38. The compound of formula (I) according to claim 35, or a pharmaceutically acceptable salt thereof, or a stereoisomer thereof, wherein R_{U2} is 5- to 6-membered heteroaryl or phenyl, the 5- to 6-membered heteroaryl or phenyl is unsubstituted or substituted with 1, 2 or 3 substituents selected from the group consisting of: deuterium, halogen (preferably fluorine, chlorine), cyano, carboxyl, hydroxyl, C₁₋₃ alkyl, C₁₋₃ alkoxy, C₁₋₃ haloalkyl, C₁₋₃ haloalkoxy, -NH₂, -NHCOC₁₋₃ alkyl, -COC₁₋₃ alkyl, -COOC₁₋₃ alkyl, -OCOC₁₋₃ alkyl, -CONH₂, -NHCONH₂, -CONHC₁₋₃ alkyl, -NHCONHC₁₋₃ alkyl, -SOC₁₋₃ alkyl, -SO₂C₁₋₃ alkyl and -SC₁₋₃ alkyl;
preferably, the 5- to 6-membered heteroaryl is selected from the group consisting of thiazolyl, oxazolyl, pyrazolyl, imidazolyl, pyrrolyl, pyridyl, pyrimidinyl, pyridazinyl, pyrazinyl, tetrazolyl and triazolyl;
preferably, the 5- to 6-membered heteroaryl is selected from the group consisting of 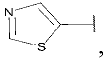 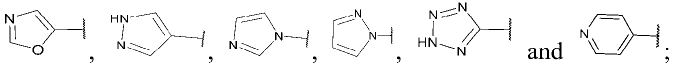
preferably, the 5- to 6-membered heteroaryl is 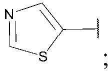
preferably, the 5- to 6-membered heteroaryl is selected from the group consisting of a thiazole ring, an oxazole ring, a pyrazole ring, an imidazole ring, a pyrrole ring, a pyridine ring, a pyrimidine ring, a pyridazine ring, a pyrazine ring, a tetrazole ring and a triazole ring;
preferably, R_{U2} is cyano.

39. The compound of formula (I) according to claim 35, or a pharmaceutically acceptable salt thereof, or a stereoisomer thereof, wherein R_{U2} is -NHRₐ₃, wherein Rₐ₃ is 5- to 6-membered heteroaryl or phenyl, the 5- to 6-membered heteroaryl is selected from the group consisting of thiazolyl, imidazolyl, pyrazolyl, oxazolyl, pyridyl and pyrimidinyl; the 5- to 6-membered heteroaryl or phenyl is unsubstituted or substituted with 1, 2 or 3 substituents selected from the group consisting of: C₁₋₃ alkoxy, C₁₋₃ haloalkoxy, C₁₋₃ alkyl, C₁₋₃ haloalkyl, -SC₁₋₃ alkyl and -OCOC₁₋₃ alkyl;
preferably, R_{U2} is -NHRₐ₃, wherein Rₐ₃ is thiazolyl; the thiazolyl is substituted with 1, 2 or 3 substituents selected from the group consisting of: methyl, ethyl, propyl, isopropyl, methoxy, ethoxy, propoxy, isopropoxy, monofluoromethyl, difluoromethyl, trifluoromethyl, monofluoroethyl, difluoroethyl, trifluoroethyl, monofluoromethoxy, difluoromethoxy, trifluoromethoxy, monofluoroethoxy, difluoroethoxy and trifluoroethoxy;
preferably, R_{U2} is -NHRₐ₃, wherein Rₐ₃ is thiazolyl; the thiazolyl is substituted with 1, 2, or 3 substituents selected from the group consisting of: methyl, ethyl, propyl and isopropyl.

40. The compound of formula (I) according to claim 35, or a pharmaceutically acceptable salt thereof, or a stereoisomer thereof, wherein R_{U2} is selected from the group consisting of the following structures: cyano,

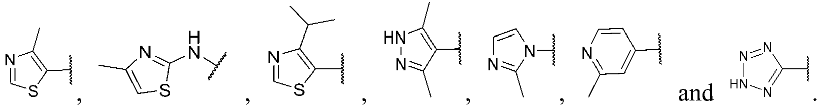

41. The compound of formula (I) according to claim 35, or a pharmaceutically acceptable salt thereof, or a stereoisomer thereof, wherein r2 is 1, and R_{U2} is

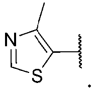

42. The compound of formula (I) according to claim 35, or a pharmaceutically acceptable salt thereof, or a stereoisomer thereof, wherein r2 is 2, and R_{U2} are X₂ and 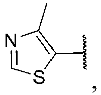 respectively.

43. The compound of formula (I) according to claim 35, or a pharmaceutically acceptable salt thereof, or a stereoisomer thereof, wherein 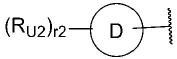 is selected from the group consisting of the following structures or isomers thereof: 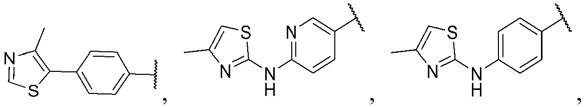 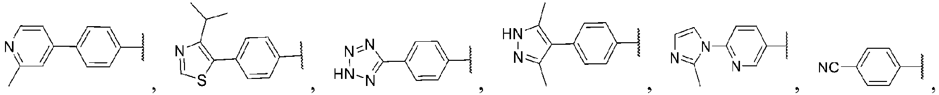 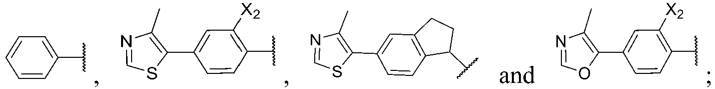 wherein X₂ is the point of attachment of ULM to L or POI;
preferably, 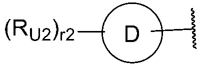 is selected from the group consisting of the following structure or an isomer thereof:

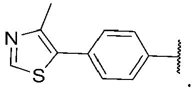

44. The compound of formula (I) according to claim 35, or a pharmaceutically acceptable salt thereof, or a stereoisomer thereof, wherein one of R_{U1}, R_{U5}, R_{U6} and R_{U2} is X₂.

45. The compound of formula (I) according to claim 35, or a pharmaceutically acceptable salt thereof, or a stereoisomer thereof, wherein U₁ is selected from the group consisting of X₂, -NHCO-X₂, -NHCOCH₃, 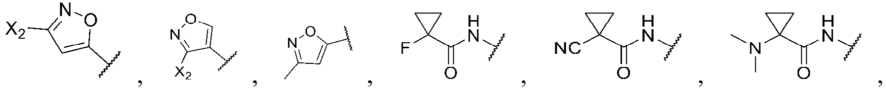 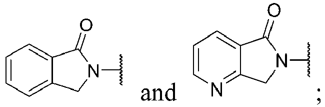 wherein X₂ is the point of attachment of ULM to L or POI;
preferably, U1 is selected from the group consisting of -NHCO-X₂ and 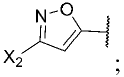 wherein X₂ is the point of attachment of ULM to L or POI.

46. The compound of formula (I) according to claim 35, or a pharmaceutically acceptable salt thereof, or a stereoisomer thereof, wherein R_{U3} and R_{U4} are each independently hydrogen, C₁₋₆ alkyl, C₁₋₆ haloalkyl, C₁₋₆ alkoxy or C₁₋₆ haloalkoxy; or R_{U3}, R_{U4} and carbon atom linked to R_{U3} and R_{U4} form a C₃₋₆ cycloalkyl ring;
preferably, R_{U3} and R_{U4} are each independently hydrogen, -CH₃, -CH₂CH₃, -CH(CH₃)₂, -C(CH₃)₃, -CF₃, -CHF₂, -CH₂F, -OCH₃, -OCH(CH₃)₂, -OC(CH₃)₃, -OCF₃, -OCHF₂, -OCH₂F, fluoro-substituted isopropyl or fluoro-substituted tert-butyl; or R_{U3}, R_{U4} and carbon atom linked to R_{U3} and R_{U4} form a cyclopropyl ring, a cyclobutyl ring, a cyclopentyl ring or a cyclohexyl ring;
preferably, R_{U3} and R_{U4} are each independently hydrogen, -CH₃, -CH₂CH₃, -CH₂CH₂CH₃, -CH(CH₃)₂ or -C(CH₃)₃; or R_{U3}, R_{U4} and carbon atom linked to R_{U3} and R_{U4} form a cyclopropyl ring;
preferably, R_{U3} and R_{U4} are each independently hydrogen, -CH(CH₃)₂ or -C(CH₃)₃.

47. The compound of formula (I) according to claim 35, or a pharmaceutically acceptable salt thereof, or a stereoisomer thereof, wherein R_{U1} is selected from the group consisting of the following structures or isomers thereof: 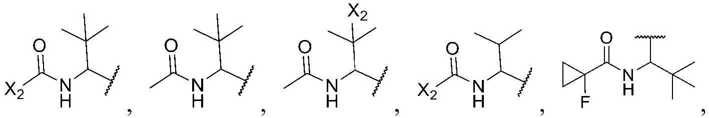 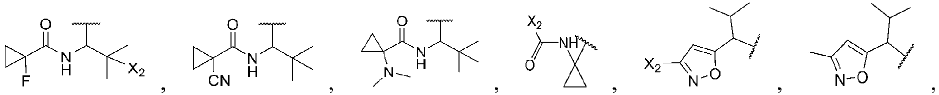 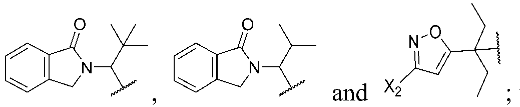 wherein X₂ is the point of attachment of ULM to L or POI;
preferably, R_{U1} is selected from the group consisting of the following structures: 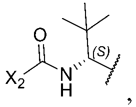 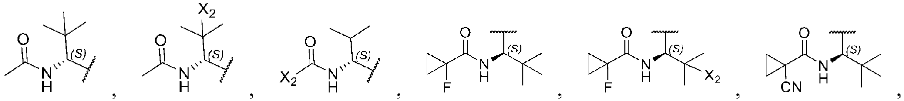 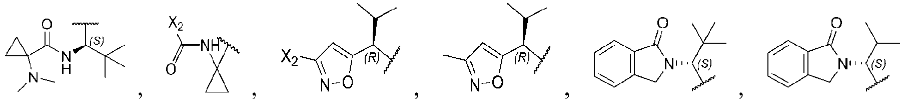 and 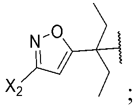 wherein X₂ is the point of attachment of ULM to L or POI;
preferably, R_{U1} is selected from the group consisting of the following structure: 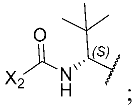 wherein X₂ is the point of attachment of ULM to L or POI.

48. The compound of formula (I) according to claim 35, or a pharmaceutically acceptable salt thereof, or a stereoisomer thereof, wherein R_{U5} and R_{U6} are each independently X₂, hydrogen, deuterium, halogen, hydroxyl, carboxyl, cyano, amino, C₁₋₃ alkyl, C₁₋₃ alkoxy, C₁₋₃ haloalkyl, or C₁₋₃ haloalkoxy; or R_{U5} and carbon atom on ring D together form a C₃₋₆ cycloalkyl ring; the C₃₋₆ cycloalkyl ring is unsubstituted or substituted with 1, 2, 3, or 4 substituents selected from the group consisting of: deuterium, halogen (preferably fluorine, chlorine, or bromine), cyano, carboxyl, hydroxyl, nitro, formyl, sulfo, -NH₂, -NH(C₁₋₆ alkyl), -N(C₁₋₆ alkyl)₂, C₁₋₆ alkyl, C₁₋₆ alkoxy, -SC₁₋₆ alkyl, -SOC₁₋₆ alkyl, -SO2C₁₋₆ alkyl, C₁₋₆ haloalkyl, C₁₋₆ haloalkoxy, amino-substituted C₁₋₆ alkyl, cyano-substituted C₁₋₆ alkyl, hydroxy-substituted C₁₋₆ alkyl, carboxy-substituted C₁₋₆ alkyl, -COC₁₋₆ alkyl, -CO₂NH₂, -CO₂NH(C₁₋₆ alkyl), -CO₂N(C₁₋₆ alkyl)₂, -SO₂NH₂, -SO₂NH(C₁₋₆ alkyl), -SO₂N(C₁₋₆ alkyl)₂, a C₃₋₈ cycloalkyl ring, a 4- to 10-membered heterocycloalkyl ring, a 5- to 6-membered heteroaryl ring and a benzene ring;
preferably, R_{U5} and R_{U6} are each independently hydrogen, C₁₋₃ alkyl, or C₁₋₃ haloalkyl;
preferably, R_{U5} and R_{U6} are each independently hydrogen, -CH₃, -OCH₃, -CF₃, -OCF₃, -CHF₂, -CH₂F, -OCHF₂ or -OCH₂F;
preferably, R_{U5} and R_{U6} are each independently hydrogen or -CH₃.

49. The compound of formula (I) according to claim 35, or a pharmaceutically acceptable salt thereof, or a stereoisomer thereof, wherein R_{U7} is selected from the group consisting of hydrogen, methyl, ethyl, monofluoromethyl, difluoromethyl and trifluoromethyl;
preferably, R_{U7} is hydrogen.

50. The compound of formula (I) according to claim 35, or a pharmaceutically acceptable salt thereof, or a stereoisomer thereof, wherein R_{U7}, nitrogen atom linked to R_{U7}, R_{U6}, and carbon atom linked to R_{U6} together form a partially unsaturated 3- to 8-membered heterocycloalkyl ring, a 5- to 6-membered heteroaryl ring or a benzene ring;
preferably, R_{U7}, nitrogen atom linked to R_{U7}, R_{U6} and carbon atom linked to R_{U6} together form a tetrahydropyrrole ring, a pyrazolidine ring, an imidazolidine ring, a piperazine ring, a piperidine ring, a 2,3-dihydro-1H-pyrrole ring, a 2,3-dihydro-1H-pyrazole ring, a pyrrole ring, a pyrazole ring, an imidazole ring, a pyridine ring, a pyrimidine ring or a benzene ring;
preferably, R_{U7}, nitrogen atom linked to R_{U7}, R_{U6} and the carbon atom linked to R_{U6} together form a 4- to 6-membered heterocycloalkyl ring;
preferably, R_{U7}, nitrogen atom linked to R_{U7}, R_{U6} and carbon atom linked to R_{U6} together form a 5-membered heterocycloalkyl ring;
preferably, R_{U7}, nitrogen atom linked to R_{U7}, R_{U6} and carbon atom linked to R_{U6} together form a tetrahydropyrrole ring.

51. The compound of formula (I) according to claim 35, or a pharmaceutically acceptable salt thereof, or a stereoisomer thereof, wherein 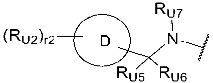 is selected from the group consisting of the following structures or isomers thereof: 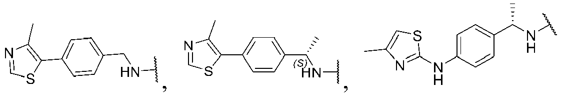 , 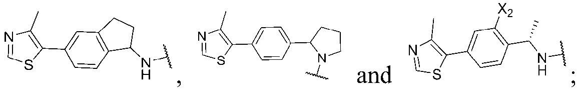 wherein X₂ is the point of attachment of ULM to L or POI;
preferably, 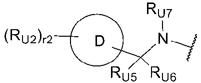 is selected from the group consisting of the following structures or isomers thereof:

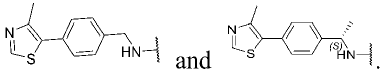

52. The compound of formula (I) according to claim 35, or a pharmaceutically acceptable salt thereof, or a stereoisomer thereof, wherein R_{U8} is selected from the group consisting of hydroxyl, amino, -NH(C₁₋₃ alkyl), -N(C₁₋₃ alkyl)₂, C₁₋₃ alkoxy, C₁₋₃ fluoroalkoxy and -OCOC₁₋₃ alkyl;
preferably, R_{U8} is selected from the group consisting of hydroxyl, amino, -NH(CH₃), -N(CH₃)₂, -OCH₃, -OCF₃, and -OCOCH₃;
preferably, R_{U8} is hydroxyl.

53. The compound of formula (I) according to claim 35, or a pharmaceutically acceptable salt thereof, or a stereoisomer thereof, wherein ULM is selected from the group consisting of the following structures or isomers thereof: 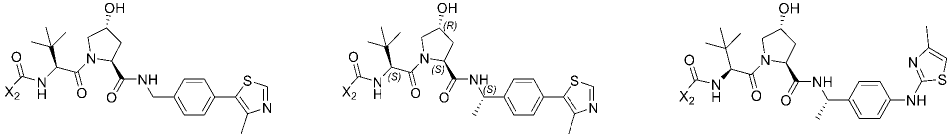 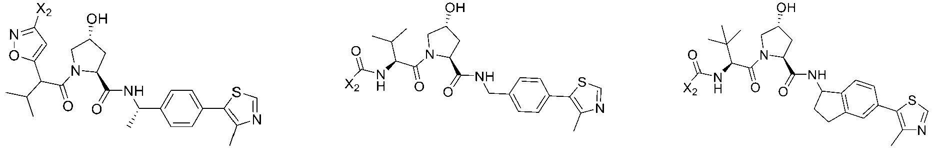 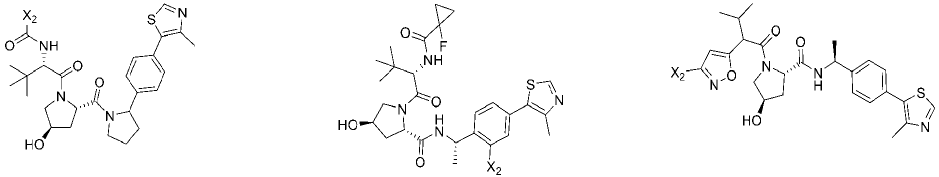 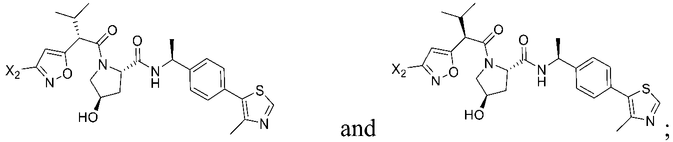
wherein X₂ is the point of attachment of ULM to L or POI;
preferably, ULM is selected from the group consisting of the following structures or isomers thereof: 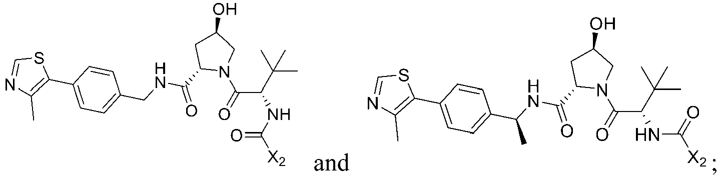 wherein X₂ is the point of attachment of ULM to L or POI;
preferably, ULM is selected from the group consisting of the following structures or isomers thereof: 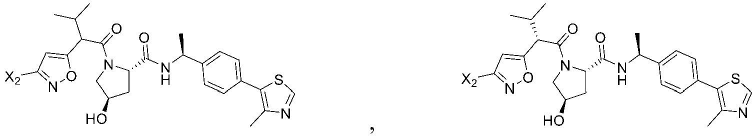 and 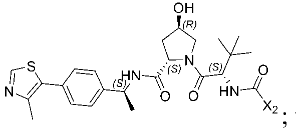 wherein X₂ is the point of attachment of ULM to L or POI.

54. The compound of formula (I) according to claim 1, or a pharmaceutically acceptable salt thereof, or a stereoisomer thereof, wherein the compound of formula (I) is a structure of formula (I-A), 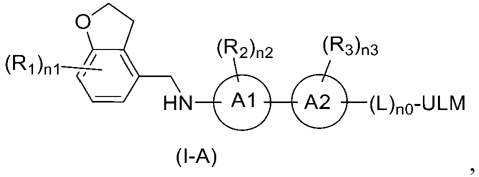 wherein ring A1, ring A2, R₁, n1, R₂, n2, R₃ and n3 are as defined in claim 2, L, n0 and ULM are as defined in claim 1, and both R₂ and R₃ are not X₁.

55. The compound of formula (I) according to claim 1, or a pharmaceutically acceptable salt thereof, or a stereoisomer thereof, wherein the compound of formula (I) is a structure of formula (I-B), 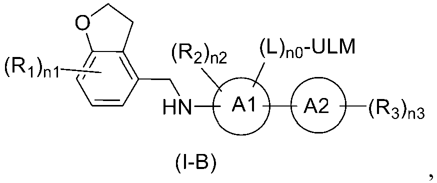 wherein ring A1, ring A2, R₁, n1, R₂, n2, R₃ and n3 are as defined in claim 2, L, n0 and ULM are as defined in claim 1, and both R₂ and R₃ are not X₁.

56. The compound of formula (I) according to claim 1, or a pharmaceutically acceptable salt thereof, or a stereoisomer thereof, wherein the compound of formula (I) is selected from the group consisting of the following structures or isomers thereof: 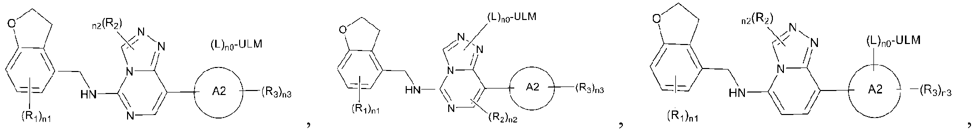 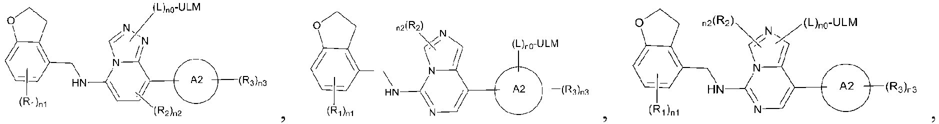 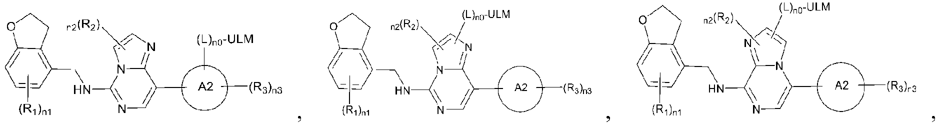 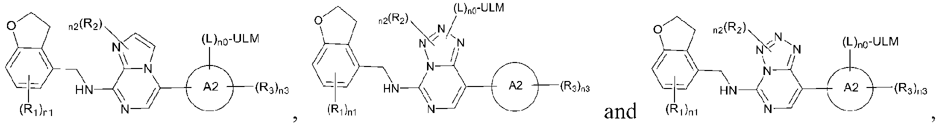 wherein ring A2, R₁, n1, R₂, n2, R₃ and n3 are as defined in claim 2, L, n0 and ULM are as defined in claim 1, and both R₂ and R₃ are not X₁.

57. The compound of formula (I) according to claim 1, or a pharmaceutically acceptable salt thereof, or a stereoisomer thereof, wherein the compound of formula (I) is selected from the group consisting of the following structures or isomers thereof: 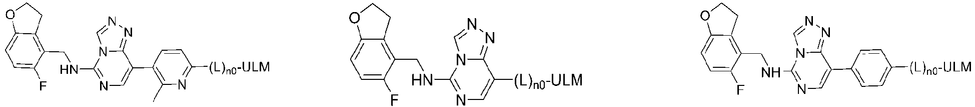 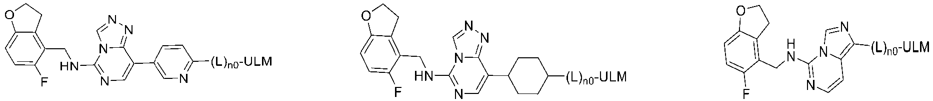 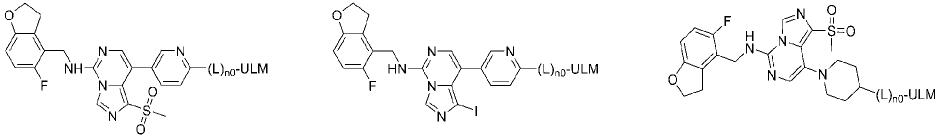 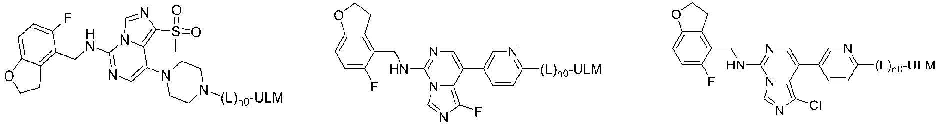 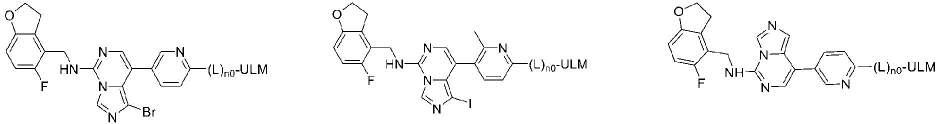 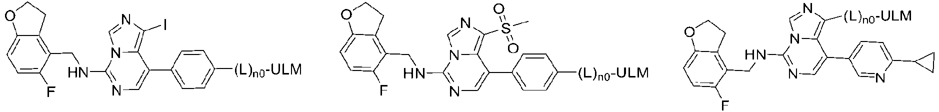 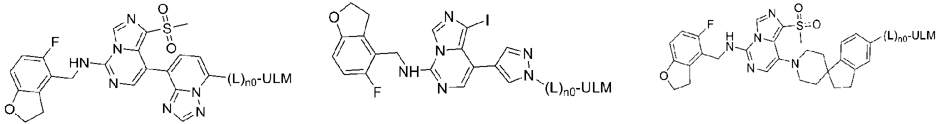 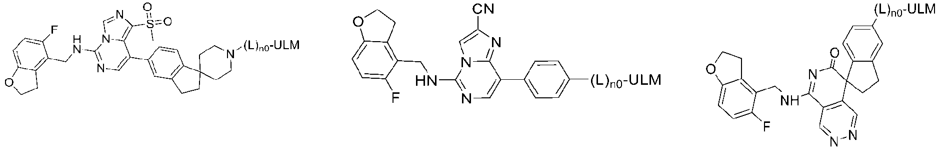 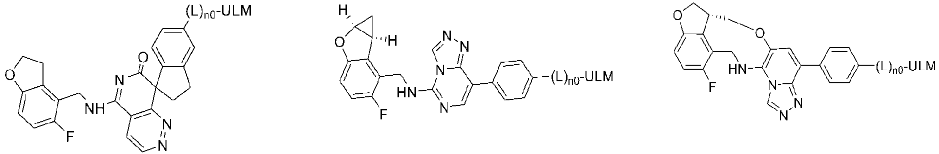 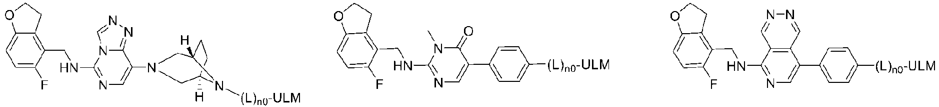 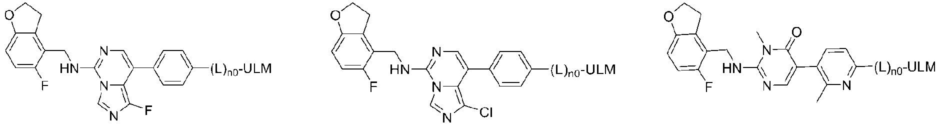 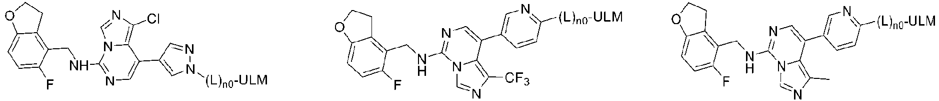 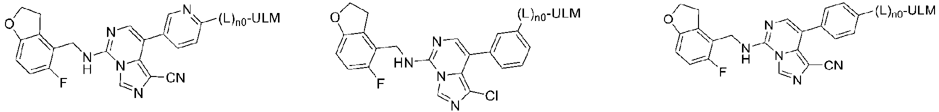 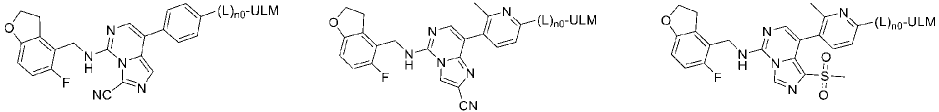 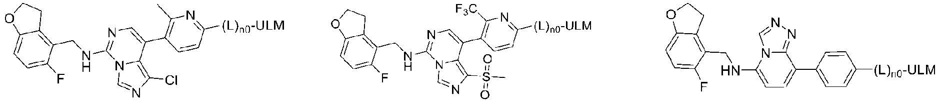 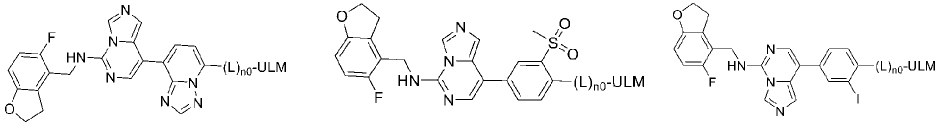 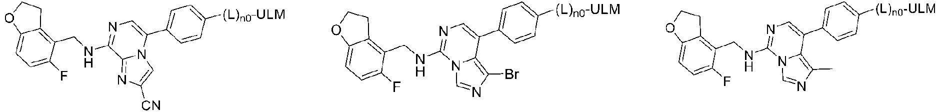 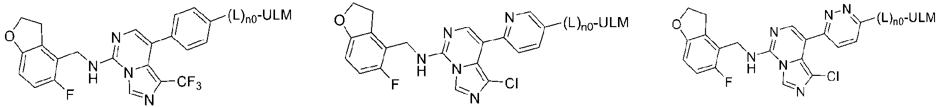 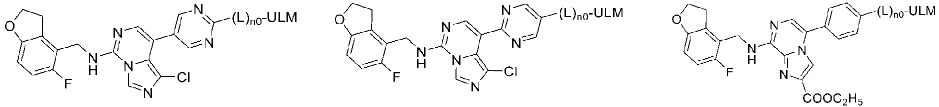 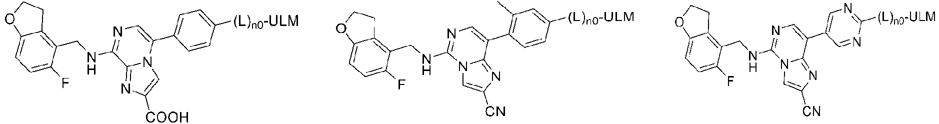 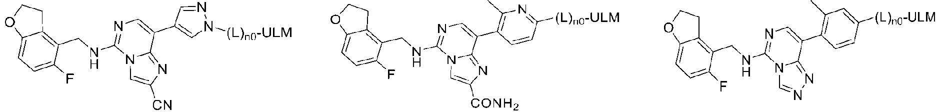 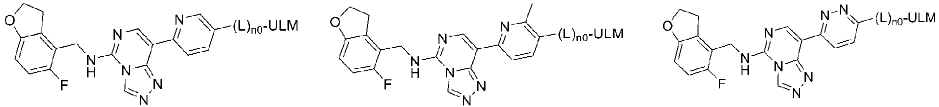 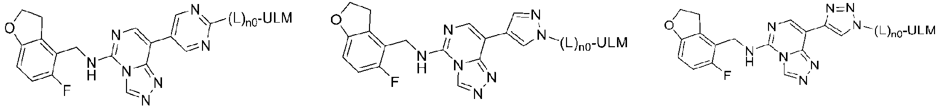 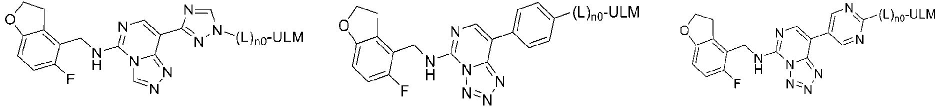 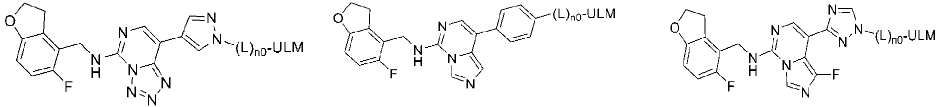 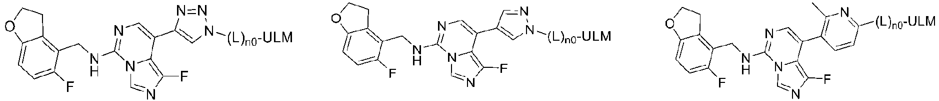 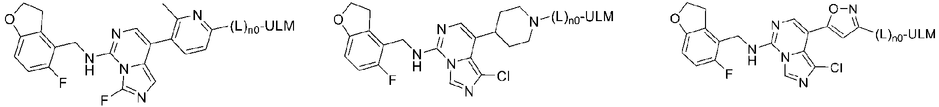 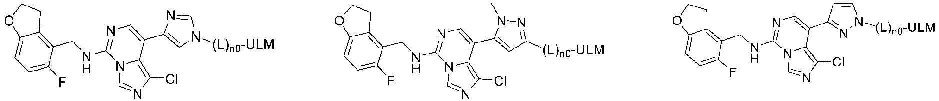 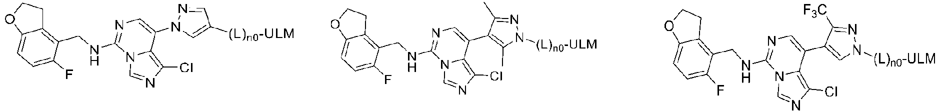 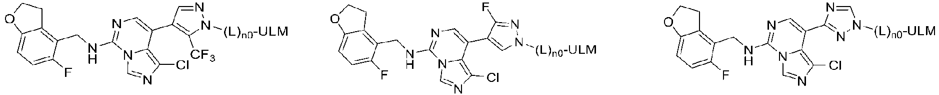 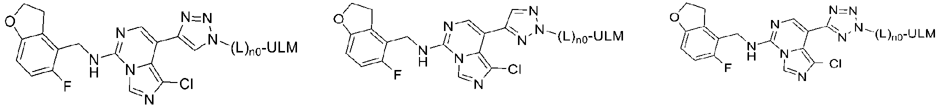 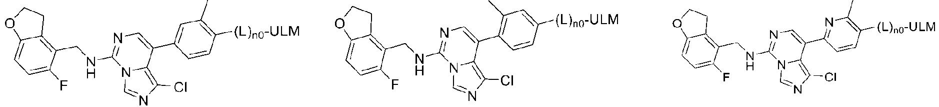 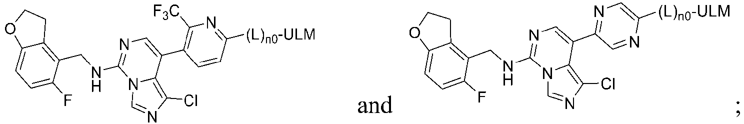
preferably, the compound of formula (I) is selected from the group consisting of the following structures or isomers thereof: 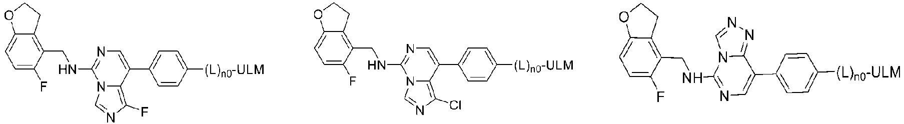 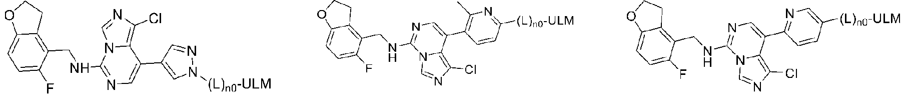 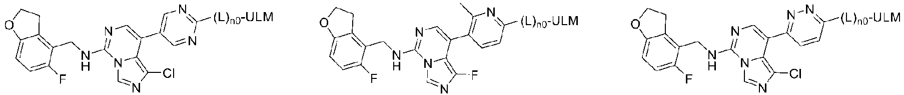 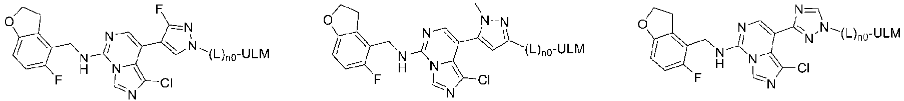 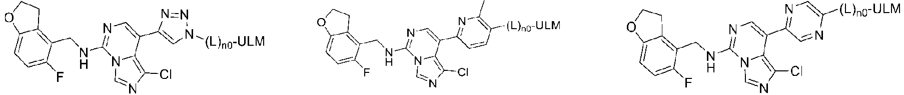 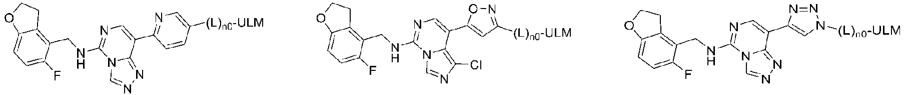 and 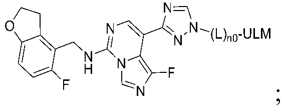
preferably, the compound of formula (I) is selected from the group consisting of the following structures or isomers thereof: 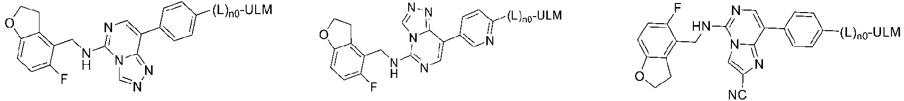 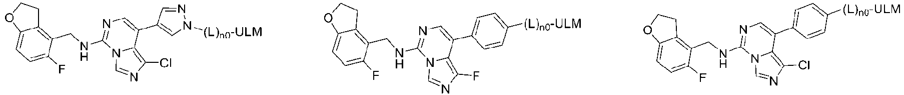 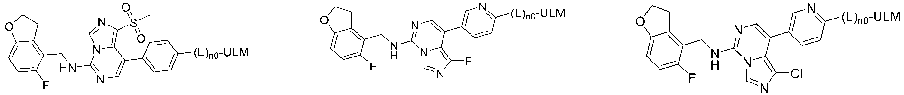 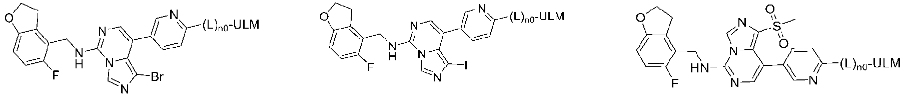 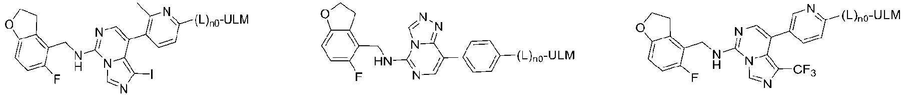 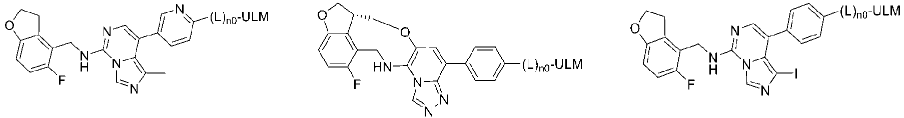 and

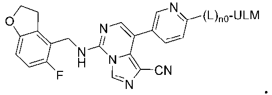

58. The compound of formula (I) according to claim 1, or a pharmaceutically acceptable salt thereof, or a stereoisomer thereof, wherein the compound of formula (I) is a compound selected from the group consisting of Table A or a stereoisomer thereof.

59. The compound of formula (I) according to claim 1, or a pharmaceutically acceptable salt thereof, or a stereoisomer thereof, wherein the compound of formula (I) is a compound selected from the group consisting of Table B or a stereoisomer thereof.

60. A pharmaceutical composition, comprising the compound of formula (I) according to any one of claims 1 to 59, or a pharmaceutically acceptable salt thereof, or a stereoisomer thereof; and a pharmaceutically acceptable carrier.

61. Use of the compound of formula (I) according to any one of claims 1 to 59, or a pharmaceutically acceptable salt thereof, or a stereoisomer thereof, or the pharmaceutical composition according to claim 60, in the manufacture of a medicament for preventing and/or treating an EED-mediated disease;
preferably, the EED-mediated disease is a tumor or an autoimmune disease;
preferably, the EED-mediated disease is cancer;
preferably, the cancer is selected from the group consisting of multiple myeloma, leukemia, non-small cell lung cancer, colon cancer, central nervous system cancer, melanoma, ovarian cancer, renal cancer, prostate cancer and breast cancer.

62. A method for preventing and/or treating an EED-mediated disease, comprising administering a therapeutically effective amount of the compound of formula (I) according to any one of claims 1 to 59, or a pharmaceutically acceptable salt thereof, or a stereoisomer thereof, or the pharmaceutical composition according to claim 60 to a subject;
preferably, the EED-mediated disease is a tumor or an autoimmune disease;
preferably, the EED-mediated disease is cancer;
preferably, the cancer is selected from the group consisting of multiple myeloma, leukemia, non-small cell lung cancer, colon cancer, central nervous system cancer, melanoma, ovarian cancer, renal cancer, prostate cancer and breast cancer.
